# EUROPÄISCHE PATENTSCHRIFT

(11) **EP 2 999 338 B1**
(45) Veröffentlichungstag und Bekanntmachung des Hinweises auf die Patenterteilung: **12.07.2017**
(21) Anmeldenummer: 14726944.3
(22) Anmeldetag: 23.05.2014
(51) Int. Cl.: A01N 43/56, A01N 43/647, A01N 43/653, A01N 43/58, A01N 43/66, A01N 43/713, A01N 43/76, A01N 43/78, A01N 43/82, A01N 43/90, C07D 401/06, C07D 401/14, C07D 403/12, C07D 413/14, C07D 401/12, C07D 417/14, C07D 471/04, C07D 487/04, C07D 513/04

(54) **BEKANNTE UND NEUE HETEROCYCLISCHE VERBINDUNGEN ALS SCHÄDLINGSBEKÄMPFUNGSMITTEL**
KNOWN AND NOVEL HETEROCYCLIC COMPOUNDS AS PESTICIDES
COMPOSÉS HÉTÉROCYCLIQUES CONNUES ET NOUVEAUX COMME PESTICIDES

(30) Priorität: 23.05.2013 EP 13168871
(43) Veröffentlichungstag der Anmeldung: 30.03.2016
(73) Patentinhaber: Bayer CropScience Aktiengesellschaft, 40789 Monheim am Rhein (DE)
(72) Erfinder: KÜBBELER, Susanne, 40589 Düsseldorf (DE); HEIL, Markus, 42799 Leichlingen (DE); COQUERON, Pierre-Yves, 69009 Lyon (FR); JANSEN, Johannes-Rudolf, 40789 Monheim (DE); FISCHER, Reiner, 40789 Monheim (DE); ANDREE, Roland, 40764 Langenfeld (DE); BOLLENBACH-WAHL, Birgit, 55413 Weiler/Bingen (DE); FRANKEN, Eva-Maria, 51381 Leverkusen (DE); ILG, Kerstin, 50670 Köln (DE); VOERSTE, Arnd, 50674 Köln (DE)
(74) Vertreter: BIP Patents
(86) Internationale Anmeldenummer: PCT/EP2014/060595
(87) Internationale Veröffentlichungsnummer: WO 2014/187928

(56) Entgegenhaltungen:
- WO-A1-03/097604
- WO-A1-2005/074686
- WO-A2-2008/014905

## Beschreibung

Die vorliegende Anmeldung betrifft die Verwendung bekannter und neuer heterocyclischer Verbindungen zur Bekämpfung von tierischen Schädlingen, neue heterocyclische Verbindungen, Verfahren zu Ihrer Herstellung und ihre Verwendung zur Bekämpfung von tierischen Schädlingen.

In WO 2011/049987 A2, WO 2011/106114 A1, WO 2009/025793 A2, US 2008/0103182 A1, WO 2007/055941 A2, WO 2006/114313 A1, US 2009/0163545 A1 und FR 2836915 A1 werden heterocyclische Verbindungen offenbart, für die pharmazeutische Verwendungen beschrieben werden. In US 2511231 und JP 63218665 A werden heterocyclische Verbindungen für die Verwendung in der Fotografie offenbart. In der Literatur sind bestimmte substituierte Pyridine als Insektizide beschrieben, siehe beispielsweise WO 2003/097604 A1. In WO 2005/074686 A1 und WO 2008/014905 A2 werden Carboxamide für die Bekämpfung von unerwünschten Mikroorganismen offenbart.

Die heterocyclischen Verbindungen der Formeln (G1), (G2) und (G4) sind bekannt. Eine Verwendung für diese Verbindungen wurde nicht angegeben.

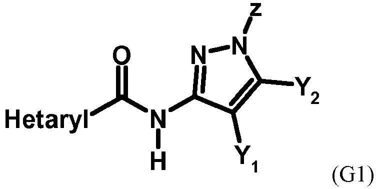

| **Nr.** | **CAS Nummer** | **Hetaryl** | **Y₁** | **Y₂** | **Z** |
|---|---|---|---|---|---|
| G1-1 | 1394594-59-2 | 6-Cyanopyridin-3-yl | 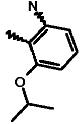 | | Methyl |
| G1-2 | 1388443-85-3 | 5-Brompyridin-3-yl | H | H | 3-Amino-3-oxopropyl |
| G1-3 | 1386349-79-6 | 5-Chlor-6-isopropoxypyridin-3-yl | H | H | Methyl |
| G1-4 | 1386201-75-7 | 5-Chlor-6-(2,2,2-trifluorethoxy)-pyridin-3-yl | H | H | Benzyl |
| G1-5 | 1378162-64-1 | 2-Ethylpyrimidin-5-yl | 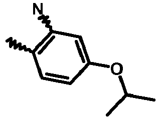 | | 2,2,2-Trifluorethyl |
| G1-6 | 1378144-60-5 | 2-Methylpyrimidin-5-yl | 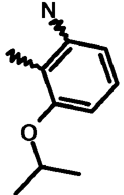 | | 4-Fluorphenyl |
| G1-7 | 1364005-12-8 | Pyridin-3-yl | H | H | Cyanomethyl |
| G1-8 | 1343727-78-5 | 6-Fluorpyridin-3-yl | H | H | Methyl |
| G1-9 | 1331064-54-0 | 6-Methylpyridin-3-yl | H | H | Methyl |
| G1-10 | 1322107-71-0 | 5-Chlor-6-(2,2,2-trifluorethoxy)-pyridin-3-yl | H | H | Phenyl |
| G1-11 | 1311484-14-6 | 6-Methylpyridin-3-yl | H | H | Quinolin-8-ylmethyl |
| G1-12 | 1301300-24-2 | 6-(2,2,2-Trifluorethoxy)pyridin-3-yl | H | H | Quinolin-8-ylmethyl |
| G1-13 | 1299334-31-8 | 6-(2,2,2-Trifluorethoxy)-pyridin-3-yl | H | H | Pyridin-4-ylmethyl |
| G1-14 | 1298991-86-2 | 5-Brompyridin-3-yl | H | H | 2-(Pyridin-2-yl)ethyl |
| G1-15 | 1293710-76-5 | 5-Chlor-6-isopropoxypyridin-3-yl | H | H | Pyridin-2-ylmethyl |
| G1-16 | 1293710-71-0 | 5-Chlor-6-(2,2,2-trifluorethoxy)pyridin-3-yl | H | H | Pyridin-3-ylmethyl |
| G1-17 | 1293302-73-4 | Pyridin-3-yl | 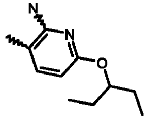 | | Methyl |
| G1-18 | 1288605-17-3 | 6-Chlorpyridin-3-yl | H | H | Quinolin-8-ylmethyl |
| G1-19 | 1252297-97-4 | 6-(2,2,2-Trifluorethoxy)pyridin-3-yl | H | H | Pyridin-2-ylmethyl |
| G1-20 | 1193169-23-1 | 6-Chlorpyridin-3-yl | H | Methyl | Phenyl |
| G1-21 | 1193168-56-7 | 6-Chlorpyridin-3-yl | H | H | 4-Chlorphenyl |
| G1-22 | 1193166-26-5 | 6-Chlorpyridin-3-yl | H | Ethyl | phenyl |
| G1-23 | 1192992-75-8 | 6-Chlorpyridin-3-yl | H | Ethyl | 4-Cyanophenyl |
| G1-24 | 1192974-12-1 | 6-Chlorpyridin-3-yl | H | Methyl | 3-Chlorphenyl |
| G1-25 | 1192974-02-9 | 6-Chlorpyridin-3-yl | H | Methyl | 4-Chlorphenyl |
| G1-26 | 1192967-85-3 | 6-Chlorpyridin-3-yl | H | H | 2,4-Difluorphenyl |
| G1-27 | 1189895-78-0 | 6-Chlorpyridin-3-yl | H | Ethyl | 4-Chlorphenyl |
| G1-28 | 1189688-16-1 | 6-Chlorpyridin-3-yl | H | Ethyl | 2,4-Difluorphenyl |
| G1-29 | 1189644-39-0 | 6-Chlorpyridin-3-yl | H | Ethyl | 3-Methylphenyl |
| G1-30 | 1189486-79-0 | 6-Chlorpyridin-3-yl | H | Methyl | 4-Isopropylphenyl |
| G1-31 | 1189418-28-7 | 6-Chlorpyridin-3-yl | H | H | 4-Isopropylphenyl |
| G1-32 | 1185159-42-5 | 6-Chlorpyridin-3-yl | H | Methyl | 3,4-Dichlorphenyl |
| G1-33 | 1185131-97-8 | 6-Chlorpyridin-3-yl | H | H | phenyl |
| G1-34 | 1185105-88-7 | 6-Chlorpyridin-3-yl | H | H | 3-Chlorphenyl |
| G1-35 | 1185047-56-6 | 6-Chlorpyridin-3-yl | H | Ethyl | 3,4-Dichlorphenyl |
| G1-36 | 1184979-44-9 | 6-Chlorpyridin-3-yl | H | H | 4-Methoxyphenyl |
| G1-37 | 1184968-09-9 | 6-Chlorpyridin-3-yl | H | Methyl | 3-Methylphenyl |
| G1-38 | 1179419-36-3 | Pyridin-3-yl | Brom | H | 2-Fluorbenzyl |
| G1-39 | 1172876-67-3 | 6-Chlorpyridin-3-yl | H | Ethyl | 3-Chlorphenyl |
| G1-40 | 1172816-42-0 | Pyridin-3-yl | H | H | 2,4-Difluorphenyl |
| G1-41 | 1052597-55-3 | 5-Brompyridin-3-yl | H | H | methyl |
| G1-42 | 1 O 18036-37-7 | Pyridin-3-yl | H | H | 2-Fluorbenzyl |
| G1-43 | 1018035-68-1 | Pyridin-3-yl | H | H | 4-Fluorbenzyl |
| G1-44 | 1018034-07-5 | Pyridin-3-yl | H | H | Methyl |
| G1-45 | 1018020-80-8 | Pyridin-3-yl | H | H | 3-Fluorbenzyl |
| G1-46 | 1017985-14-6 | Pyridin-3-yl | H | H | ethyl |
| G1-47 | 1017984-07-4 | Pyridin-3-yl | H | H | 4-Methylbenzyl |
| G1-48 | 1017981-75-7 | Pyridin-3-yl | Brom | H | 3-Methylbenzyl |
| G1-49 | 1017980-53-8 | Pyridin-3-yl | Brom | H | 4-Methylbenzyl |
| G1-50 | 1017978-97-0 | Pyridin-3-yl | Brom | H | 4-Fluorbenzyl |
| G1-51 | 1017586-40-1 | Pyridin-3-yl | H | H | 2,5-Dichlorbenzyl |
| G1-52 | 1017534-13-2 | Pyridin-3-yl | H | H | 2-Methylbenzyl |
| G1-53 | 1017532-86-3 | Pyridin-3-yl | H | H | 2-Chlorbenzyl |
| G1-54 | 1006331-96-9 | Pyridin-3-yl | H | Methyl | benzyl |
| G1-55 | 1005882-11-0 | 6-Chlorpyridin-3-yl | H | H | 4-Chlorbenzyl |
| G1-56 | 1001841-69-5 | Pyridin-3-yl | Cyano | Ethylsulfanyl | Phenyl |
| G1-57 | 1001841-28-6 | Pyridin-3-yl | Cyano | Methylsulfanyl | Phenyl |
| G1-58 | 957513-83-6 | Pyridin-3-yl | H | Methyl | 3-Methylbenzyl |
| G1-59 | 957501-08-5 | Pyridin-3-yl | H | H | 4-Chlorbenzyl |
| G1-60 | 957336-71-9 | Pyridin-3-yl | H | H | 2-Ethoxy-2-oxoethyl |
| G1-61 | 957277-00-8 | Pyridin-3-yl | H | Methyl | 4-Fluorbenzyl |
| G1-62 | 957007-89-5 | 6-Chlorpyridin-3-yl | H | H | Methyl |
| G1-63 | 957007-68-0 | 6-(2,2,2-Trifluorethoxy)pyridin-3-yl | H | H | Methyl |
| G1-64 | 956962-14-4 | Pyridin-3-yl | H | H | 1-Naphthyhnethyl |
| G1-65 | 956763-30-7 | Pyridin-3-yl | H | H | 2,4-Dichlorbenzyl |
| G1-66 | 956627-03-5 | Pyridin-3-yl | H | Methyl | 4-Methoxy-4-oxobutyl |
| G1-67 | 956536-22-4 | 6-Methoxypyridin-3-yl | H | H | Methyl |
| G1-68 | 956334-28-4 | 5,6-Dichlorpyridin-3-yl | H | H | Methyl |
| G1-69 | 955963-35-6 | Pyridin-3-yl | H | H | Benzyl |
| G1-70 | 895950-13-7 | Pyridin-3-yl | Chlor | H | 2-Chlor-6-fluorbenzyl |
| G1-71 | 1401567-88-1 | Pyridin-3-yl | 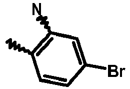 | | H |
| G1-72 | 1398113-90-0 | 6-Methylpyridin-3-yl | H | Cyclohexylmethyl | H |
| G1-73 | 1390185-62-2 | 5-Brompyridin-3-yl | Methyl | H | H |
| G1-74 | 1373059-58-5 | 5-Brompyridin-3-yl | H | Cyclopentyl | H |
| G1-75 | 1355894-01-7 | 6-(2,2,3,3-Tetrafluorpropoxy)-pyridin-3-yl | H | H | H |
| G1-76 | 1346686-04-1 | 6-Isopropoxypyridin-3-yl | H | Cyclohexylmethyl | H |
| G1-77 | 1342817-43-9 | 6-Methoxypyridin-3-yl | H | Cyclohexylmethyl | H |
| G1-78 | 1291744-04-1 | 6-(Trifluormethyl)pyridin-3-yl | H | H | H |
| G1-79 | 1282798-05-3 | 5-Brompyridin-3-yl | 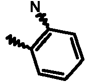 | | H |
| G1-80 | 1275713-61-5 | 6-Chlorpyridin-3-yl | 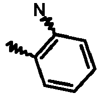 | | H |
| G1-81 | 1275068-38-6 | 6-Methylpyridin-3-yl | 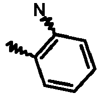 | | H |
| G1-82 | 1270883-15-2 | Pyridin-3-yl | 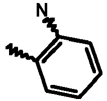 | | H |
| G1-83 | 1249829-04-6 | 6-Chlorpyridin-3-yl | H | H | H |
| G1-84 | 1249637-58-8 | 6-Methoxypyridin-3-yl | H | H | H |
| G1-85 | 1248794-65-1 | Pyridin-3-yl | H | H | H |
| G1-86 | 1248082-67-8 | 6-Methylpyridin-3-yl | H | H | H |
| G1-87 | 1248016-37-6 | 5,6-Dichlorpyridin-3-yl | H | H | H |
| G1-88 | 1247118-66-6 | 5-Brompyridin-3-yl | H | H | H |
| G1-89 | 1241269-40-8 | 5-Chlor-6-isopropoxypyridin-3-yl | H | H | H |
| G1-90 | 1042684-17-2 | Pyridin-3-yl | Cyano | Ethylsulfanyl | H |
| G1-91 | 942662-36-4 | Pyridin-3-yl | Cyano | Methylsulfa-nyl | H |
| G1-92 | 313371-16-3 | 5-Brompyridin-3-yl | H | OH | Phenyl |
| G1-93 | 957010-63-8 | Pyridin-3-yl | H | H | 3-Methylbenzyl |
| G1-94 | 1436033-05-4 | Pyridin-3-yl | H | H | 1-Phenylethyl |
| G1-95 | 1445574-69-5 | 5-Iodpyridin-3-yl | H | Methyl | Methyl |
| G1-96 | 1458185-22-2 | 6-Methoxypyridin-3-yl | 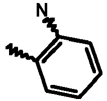 | | H |
| G1-97 | 1458685-50-1 | 5,6-Dichlorpyridin-3-yl | 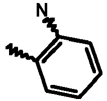 | | H |
| G1-98 | 1479443-93-0 | 5-Methylpyridin-3-yl | 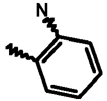 | | H |
| G1-99 | 1486844-11-4 | 5-Methylpyridin-3-yl | H | H | H |
| G1-100 | 1486514-55-9 | Pyridin-3-yl | H | 1-Methylbutyl | H |
| G1-101 | 1509673-22-6 | 6-Brompyridin-3-yl | H | H | Methyl |
| G1-102 | 1512341-63-7 | 6-Brompyridin-3-yl | H | H | H |
| G1-103 | 1522750-95-3 | 6-Brompyridin-3-yl | 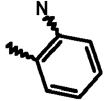 | | H |

Bei den Verbindungen G1-1, G1-5, G1-6, G1-17, G1-71, G1-79 bis G1-82 und G1-96 bis G1-98 und G1-103 sind in den Spalten Y₁ und Y₂ auch die mit Y₁ und Y₂ verbundenen C-Atome und das mit Z verknüpfte N-Atom dargestellt. Entsprechendes gilt für alle Tabellen in dieser Patentanmeldung, in denen Y₁ und Y₂ gemeinsam vorkommen.

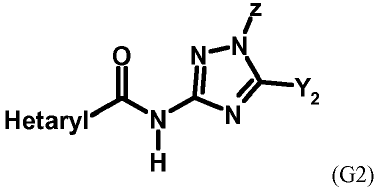

| **Nr.** | **CAS Nummer** | **Hetaryl** | **Y₂** | **Z** |
|---|---|---|---|---|
| G2-1 | 1390344-07-6 | 6-Methylpyridin-3-yl | H | (Benzylamino)-2-oxoethyl |
| G2-2 | 1311690-79-5 | 6-Methylpyridin-3-yl | H | (Azepan-1-yl)-2-oxoethyl |
| G2-3 | 1301090-70-9 | 5-Chlor-6-(2,2,2-trifluorethoxy)pyridin-3 -yl | H | Pyridin-2-ylmethyl |
| G2-4 | 1299805-79-0 | 6-Methylpyridin-3-yl | H | 4-Brombenzyl |
| G2-5 | 1295730-21-0 | 6-(2,2,2-Trifluorethoxy)pyridin-3-yl | H | Pyridin-2-ylmethyl |
| G2-6 | 1288475-13-7 | 5-Brompyridin-3-yl | H | Pyridin-2-ylmethyl |
| G2-7 | 1287863-15-3 | 5-Brompyridin-3-yl | H | 4-Brombenzyl |
| G2-8 | 1279624-84-8 | 6-(2,2,2-Trifluorethoxy)pyridin-3-yl | H | 4-Brombenzyl |
| G2-9 | 1252459-54-3 | 6-Methoxypyridin-3-yl | H | Benzyl |
| G2-10 | 1241202-60-7 | Pyridin-3-yl | H | Benzyl |
| G2-11 | 1240880-06-1 | 5-Chlor-6-(2,2,2-trifluorethoxy)pyridin-3 -yl | H | Pyridin-3-ylmethyl |
| G2-12 | 1146924-12-0 | Pyridin-3-yl | H | 3-Chlorbenzyl |
| G2-13 | 1389504-29-3 | 5-Chlor-6-(2,2,2-trifluorethoxy)pyridin-3 -yl | Trifluormethyl | H |
| G2-14 | 1388561-95-2 | 5-Chlor-6-(2,2,2-trifluorethoxy)pyridin-3-yl | H | H |
| G2-15 | 1301165-37-6 | 6-(2,2,2-Trifluorethoxy)pyridin-3-yl | Piperidin-1-yl | H |
| G2-16 | 1279629-65-0 | 5-Chlor-6-isopropoxypyridin-3-yl | Methylsulfonyl | H |
| G2-17 | 1258777-77-3 | 5-Chlor-6-isopropoxypyridin-3-yl | Isopropyl | H |
| G2-18 | 1252429-89-2 | Pyridin-3-yl | Isopropyl | H |
| G2-19 | 1249755-35-8 | 6-Methylpyridin-3-yl | H | H |
| G2-20 | 1248113-40-7 | 5,6-Dichlorpyridin-3-yl | H | H |
| G2-21 | 1241493-74-2 | 5-Chlor-6-isopropoxypyridin-3-yl | Trifluormethyl | H |
| G2-22 | 1210286-20-6 | 6-(Trifluormethyl)pyridin-3-yl | H | H |
| G2-23 | 1209076-53-8 | 6-(2,2,2-Trifluorethoxy)pyridin-3-yl | H | H |
| G2-24 | 1099921-76-2 | 6-Methylpyridin-3-yl | Trifluormethyl | H |
| G2-25 | 1099895-46-1 | 1-Oxidopyridin-3-yl | Trifluormethyl | H |
| G2-26 | 1099290-03-5 | 5-Brompyridin-3-yl | Trifluormethyl | H |
| G2-27 | 1087922-52-8 | 1-Oxidopyridin-3-yl | H | H |
| G2-28 | 1087874-75-6 | 6-Chlorpyridin-3-yl | H | H |
| G2-29 | 1050833-67-4 | 6-Methoxypyridin-3-yl | H | H |
| G2-30 | 946667-19-2 | Pyridin-3-yl | Amino | H |
| G2-31 | 886126-65-4 | Pyridin-3-yl | Carboxy | H |
| G2-32 | 500867-55-0 | Pyridin-3-yl | Methylsulfanyl | H |
| G2-33 | 448240-88-8 | 5-Brompyridin-3-yl | H | H |
| G2-34 | 21051-18-3 | Pyridin-3-yl | H | H |

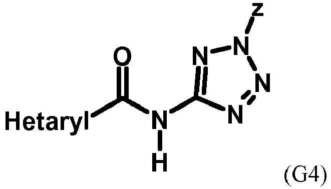

| **Nr.** | **CAS Nummer** | **Hetaryl** | **Z** |
|---|---|---|---|
| G4-1 | 925582-52-1 | Pyridin-3-yl | Vinyl |
| G4-2 | 925573-37-1 | 5-Brompyridin-3-yl | Allyl |
| G4-3 | 925167-93-7 | Pyridin-3-yl | Allyl |
| G4-4 | 925047-93-4 | 5-Brompyridin-3-yl | Vinyl |
| G4-5 | 865099-19-0 | Pyridin-3-yl | Butyl |
| G4-6 | 638147-30-5 | Pyridin-3-yl | Propyl |
| G4-7 | 585557-66-0 | Pyridin-3-yl | Methyl |
| G4-8 | 1355508-65-4 | 6-(2,2,3,3-Tetrafluorpropoxy)pyridin-3-yl | H |
| G4-9 | 1333542-52-1 | 6-(2,2,2-Trifluorethoxy)pyridin-3-yl | H |
| G4-10 | 1285996-08-8 | 5-Brompyridin-3-yl | H |
| G4-11 | 87884-47-7 | Pyridin-3-yl | H |
| G4-12 | 1505086-24-7 | 6-Chlorpyridin-3-yl | Methyl |
| G4-13 | 1500601-31-9 | 6-Brompyridin-3-yl | Methyl |
| G4-14 | 1515071-97-2 | 5,6-Dichlorpyridin-3-yl | Methyl |
| G4-15 | 1523210-31-2 | 5-Brompyridin-3-yl | Methyl |
| G4-16 | 1533839-68-7 | 6-Fluorpyridin-3-yl | Methyl |

Moderne Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch die Verwendung von Verbindungen der Formel (I) 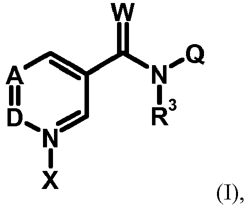 worin
A für N oder CR¹ steht,
D für N oder CR² steht, wobei A und D nicht gleichzeitig für N stehen,
R¹ für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
R² für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
X für ein freies Elektronenpaar oder Sauerstoff steht,
W für Sauerstoff, Schwefel oder NR⁴ steht,
R³ für einen Rest aus der Reihe Wasserstoff, Cyano, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl, C₁-C₆- Alkylcarbonyloxy, C₁-C₆-Alkoxycarbonyloxy, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-C₁-C₆-alkylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
R⁴ für einen Rest aus der Reihe Wasserstoff, Cyano, Nitro, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₆-Alkylsulfonyl, C₁-C₆-Halogenalkylsulfonyl, Aryl, Hetaryl, Aryl-C₁-C₆-alkyl, Hetaryl-C₁-C₆-alkyl, Arylcarbonyl, Hetarylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
Q für einen der Reste Q-1 bis Q-4 steht, 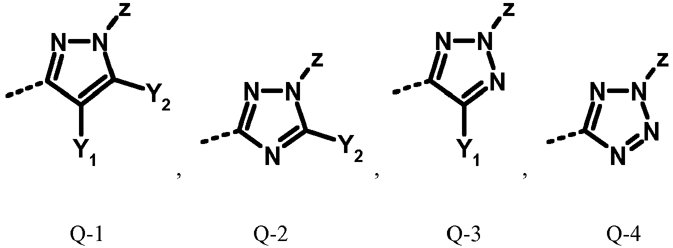 worin die gestrichelte Linie die Bindung zum Stickstoff in der Gruppe NR³ bedeutet,
Y₁ für einen Rest aus der Reihe Wasserstoff, Halogen, Cyano, Nitro, Amino, C₁-C₆-Alkylamino, Di-(C₁-C₆)-alkylamino, Hydroxy, COOH, C₁-C₆-Alkyl, C₃-C₆-Cycloalkyl, C₁-C₆-Halogenalkyl, C₁-C₆-Alkoxy, C₁-C₆- Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Y₂ für einen Rest aus der Reihe Wasserstoff, Halogen, Cyano, Nitro, Amino, C₁-C₆-Alkylamino, Di-(C₁-C₆)-alkylamino, Hydroxy, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆- Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht, oder
im Falle von Q = Q-1 Y₁ und Y₂ zusammen mit den Kohlenstoffatomen, an die sie gebunden sind, einen Phenylring oder einen 5- bis 7-gliedrigen heteroaromatischen Ring ausbilden, welcher gegebenenfalls durch einen oder mehrere, gleiche oder verschiedene Substituenten M¹ substituiert ist,
M¹ für Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl und C₁-C₆-Alkoxy-C₁-C₆-alkyl steht,
Z für einen Rest aus der Reihe Wasserstoff, Cyano, Nitro, L¹(=G)C*, L¹O(=G)C* L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₈-Alkyl, C₃-C₈-Cycloalkyl, C₂-C₈-Alkenyl, C₅-C₈-Cycloalkenyl, C₃-C₈-Alkinyl, für gegebenenfalls einfach oder mehrfach, gleich oder verschieden in der - C₁-C₈-alkyl-Gruppe durch M⁶ substituiertes L⁵C(=E)L²N(=G)C-C₁-C₈-alkyl*, L⁵(=O)ₙSL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl* steht,
M² für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆-Alkyl, C₃-C₆-Cycloalkyl, C₂-C₆-Alkenyl, C₅-C₆-Cycloalkenyl, C₃-C₆-Alkinyl, C₁-C₆-Alkoxy steht,
M³ für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C^{*} steht,
G und E unabhängig voneinander für Sauerstoff oder Schwefel stehen,
n für 1 oder 2 steht,
L¹ und L⁵ unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁴ für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹ steht,
L² für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl steht,
L³ und L⁴ unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, Cs-C₆-Cycloalkyl-C₁-C₆-alkyl, C₂-C₆-Alkenyl, C₃-C₆-Alkinyl stehen,
M⁵ für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹ steht,
M⁶ für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₅-C₆-Cycloalkenyl, C₃-C₆-Alkinyl, C₁-C₆-Alkoxy-C₁-C₆-alkyl, C₁-C₆-Halogenalkoxy-C₁-C₆-alkyl steht,
L⁹ und L¹⁰ unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₆-Alkenyl, C₂-C₆-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl stehen,
und von deren Salzen und N-Oxiden zur Bekämpfung von tierischen Schädlingen.

In den genannten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₂-C₆-Alkenyl, C₃-C₆-Alkinyl, C₃-C₆-Cycloalkyl, C₁-C₆-Halogenalkyl, Cyano-C₁-C₆-alkyl, C₂-C₆-Halogenalkenyl, C₃-C₆-Halogenalkinyl, C₁-C₆-Alkoxy, C₂-C₆-Alkenyloxy, C₃-C₆-Alkinyloxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkylcarbonylamino, CO₂H SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert. Der Stern (*) in den Restedefinitionen markiert die Stelle, mit der der jeweilige Rest mit dem übrigen Teil der Verbindung der Formel (I) verbunden ist.

Beispielhaft seien an den Resten der Formeln L¹(=G)C, L¹O(=G)C L³L⁴N(=G)C, L⁵(=E)CL²N(=G)C (entspricht L⁵C(=E)L²N(=G)C), L⁵(=O)ₙSL²N(=G)C, L⁵O(=E)CL²N(=G)C (entspricht L⁵OC(=E)L²N(=G)C), L¹L⁵N(=E)CL²N(=G)C), (entspricht L¹L⁵NC(=E)L²N(=G)C), L¹(=O)ₙS, L³L⁴N(=O)ₙS und L³O(=O)ₙS die zugehörigen Strukturen dargestellt: 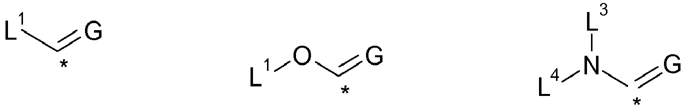 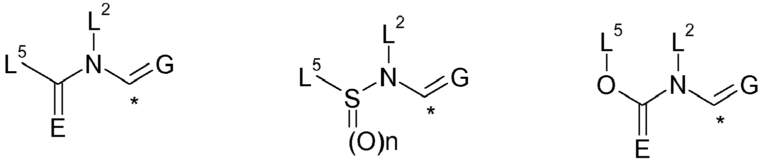 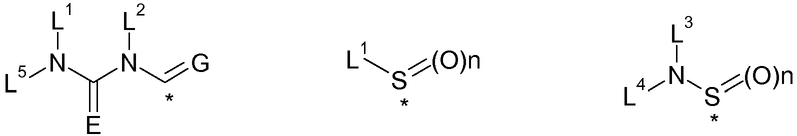 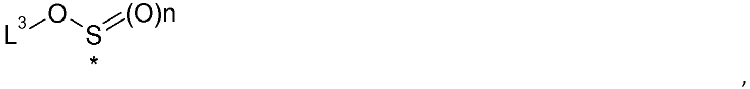 worin der Stern (*) jeweils das Atom kennzeichnet, über welches der jeweilige Rest an den übrigen Teil des Moleküls gebunden ist.

Die Verbindungen der Formel (I) können gegebenenfalls auch in Abhängigkeit von der Art der Substituenten als Tautomere und/oder Stereoisomere, d.h. als geometrische und/oder als optische Isomere oder entsprechende Isomerengemische in unterschiedlicher Zusammensetzung vorliegen. Die Erfindung betrifft sowohl die Verwendung der reinen Isomere als auch die der Isomerengemische, auch wenn hier im Allgemeinen nur von Verbindungen der Formel (I) die Rede ist.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calcium- oder Magnesiumsalze, Ammoniumsalze, Salze mit organischen Basen sowie mit organischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Sulfate, oder Phosphate, Salze mit organischen Carbonsäuren oder organischen Sulfonsäuren, beispielsweise Formiate, Acetate, Trifluoracetate, Maleate, Tartrate, Methansulfonate, Benzolsulfonate oder para-Toluolsulfonate, Salze mit Aminosäuren, beispielsweise Arginate, Aspartate oder Glutamate und Ähnliches zu nennen.

Die erfindungsgemäßen Verbindungen können auch als Metallkomplexe vorliegen, wie sie für andere Amide beispielsweise in DE 2221647 A beschrieben sind.

Bevorzugte Substituenten bzw. Bereiche der in den Verbindungen der Formel (I) aufgeführten Reste werden im Folgenden erläutert. Ihre Kombination bildet den Vorzugsbereich (1). 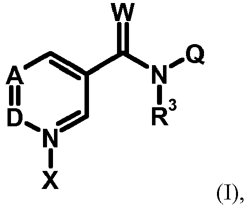
A steht für N oder CR¹.
D steht für N oder CR², wobei A und D nicht gleichzeitig für N stehen.
R¹ steht für einen Rest aus der Reihe Wasserstoff, Halogen, Cyano, Nitro, C₁-C₃-Alkyl, C₁-C₃-Halogenalkyl, C₁-C₃-Alkoxy, C₁-C₃-Halogenalkoxy.
R² steht für einen Rest aus der Reihe Wasserstoff, Halogen, Cyano, Nitro, C₁-C₃-Alkyl, C₁-C₃-Halogenalkyl, C₁-C₃-Alkoxy, C₁-C₃-Halogenalkoxy.
X steht für ein freies Elektronenpaar oder Sauerstoff.
W steht für Sauerstoff, Schwefel oder NR⁴.
R³ steht für einen Rest aus der Reihe Wasserstoff, Cyano, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₃-C₆-Cycloalkyl-carbonyl, C₃-C₆-Cycloalkyl-C₁-C₂-alkylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₃-Alkylsulfonyl, C₁-C₃-Halogenalkylsulfonyl, C₁-C₃-Alkylaminocarbonyl, Di-(C₁-C₃)-alkylaminocarbonyl, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-C₁-C₆-alkylcarbonyl gegebenenfalls einfach oder mehrfach, gleich oder unterschiedlich durch Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind.
R⁴ steht für einen Rest aus der Reihe Wasserstoff, Cyano, Nitro, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogen-cycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₃-Alkylsulfonyl, C₁-C₃-Halogenalkylsulfonyl, Aryl, Hetaryl, Aryl-C₁-C₆-alkyl, Hetaryl-C₁-C₆-alkyl, Arylcarbonyl, Hetarylcarbonyl, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, C₁-C₄-Alkyl, C₁-C₄-Halogenalkyl, C₁-C₃-Alkoxy, C₁-C₃-Halogenalkoxy, C₁-C₆-Alkoxy-C₁-C₂-alkyl substituiert sind.
Q steht für einen der Reste Q-1 bis Q-4, 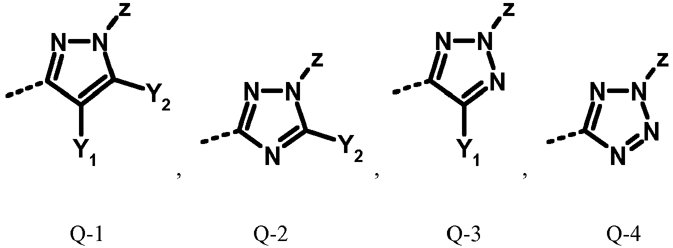 worin die gestrichelte Linie die Bindung zum Stickstoff in der Gruppe NR³ bedeutet.
Y₁ steht für einen Rest aus der Reihe Wasserstoff, Halogen, Cyano, Amino, C₁-C₃-Alkylamino, Di-(C₁-C₃)-alkylamino, Hydroxy, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₂-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, SH, C₁-C₆-Alkylthio, C₁-C₆-Alkylcarbonyl, C₃-C₆-Cycloalkylcarbonyl, C₂-C₄-Alkenylcarbonyl, C₁-C₆-Halogenalkylcarbonyl, C₁-C₃-Alkoxycarbonyl, C₁-C₆-Alkylsulfonyl, C₁-C₃-Halogenalkylsulfonyl, C₁-C₃-Alkylaminocarbonyl, Di-(C₁-C₃)-alkylaminocarbonyl.
Y₂ steht für einen Rest aus der Reihe Wasserstoff, Halogen, Cyano, Amino, C₁-C₃-Alkylamino, Di-(C₁-C₃)-alkylamino, Hydroxy, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₂-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₂-alkyl, SH, C₁-C₆-Alkylthio, C₁-C₆-Alkylcarbonyl, C₃-C₆-Cycloalkylcarbonyl, C₂-C₄-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₃-Alkylaminocarbonyl, Di-(C₁-C₃)-alkylamino-carbonyl, oder
im Falle von Q = Q-1 Y₁ und Y₂ zusammen mit den Kohlenstoffatomen, an die sie gebunden sind, einen Phenylring oder einen 5- bis 7-gliedrigen heteroaromatischen Ring ausbilden, welcher gegebenenfalls durch einen oder mehrere, gleiche oder verschiedene Substituenten M¹ substituiert ist.
M¹ steht für Halogen, Cyano, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl und C₁-C₆-Alkoxy-C₁-C₂-alkyl.
Z steht für einen R est aus der Reihe Wasserstoff, Cyano, L¹(=G)C*, L¹⁰(=G)C*, L³L⁴N(=G)C*, L⁵(=E)CL²N(=G)C_{*}, L⁵(=O)ₙSL²N(=G)C*, L⁵O(=E)CL²N(=G)C*, L¹L⁵N(=E)CL²N(=G)C*, L¹(=O)ₙS*, L³L4^{N}(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₆-alkyl-Gruppe durch M⁶ substituiertes L⁵C(=E)L²N(=G)C-C₁-C₆-alkyl*, L⁵(=O)ₙSL²N(=G)C-C₁-C₆-alkyl*, L⁵OC(=E)L²N(=G)C-C₁-C₆-alkyl*, L¹L⁵NC(=E)L²N(=G)C-C₁-C₆-alkyl*, L¹(=O)ₙS-C₁-C₆-alkyl^{*}, L³O(=O)ₙS-C₁-C₆-alkyl*, L³L⁴N(=O)ₙS-C₁-C₆-alkyl*.
M² steht für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆-Alkyl, C₃-C₆-Cycloalkyl, C₂-C₆-Alkenyl, C₃-C₆-Alkinyl, C₁-C₆-Alkoxy.
M³ steht für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C*.
G und E stehen unabhängig voneinander für Sauerstoff oder Schwefel.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
M⁴ steht für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹.
L² steht für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
M⁵ steht für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹.
M⁶ steht für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₃-C₆-Alkinyl, C₁-C₆-Alkoxy-C₁-C₃-alkyl, C₁-C₆-Halogenalkoxy-C₁-C₃-alkyl.
L⁹ und L¹⁰ stehen unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₄-Alkenyl, C₂-C₄-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆- Alkylcarbonyl, C₂-C₄-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₄-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl.

In den genannten bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₃-C₆-Cycloalkyl, C₁-C₆-Halogenalkyl, Cyano-C₁-C₆-alkyl, C₁-C₆-Alkoxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkylcarbonylamino, CO₂H, SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert.

Besonders bevorzugte Substituenten bzw. Bereiche der in den Verbindungen der Formel (I) aufgeführten Reste werden im Folgenden erläutert. Ihre Kombination bildet den Vorzugsbereich (2). 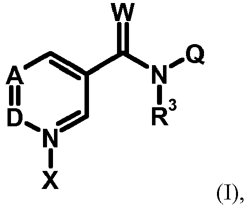
A steht für N oder CR¹.
D steht für N oder CR², wobei A und D nicht gleichzeitig für N stehen.
R¹ steht für einen Rest aus der Reihe Wasserstoff, Halogen, Cyano, Methyl, Halogenmethyl, Methoxy, Halogenmethoxy.
R² steht für einen Rest aus der Reihe Wasserstoff, Halogen, Cyano, Methyl, Halogenmethyl, Methoxy, Halogenmethoxy.
X steht für ein freies Elektronenpaar oder Sauerstoff.
W steht für Sauerstoff, Schwefel oder NR⁴.
R³ steht für einen Rest aus der Reihe Wasserstoff, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₃-C₆-Cycloalkylcarbonyl, C₁-C₆-Alkoxy-C₁-C₆-alkylcarbonyl, C₁-C₆-Alkoxycarbonyl, C₁-C₆-Halogenalkoxycarbonyl, Aryl, Hetaryl, Arylcarbonyl, Hetarylcarbonyl, wobei die Substituenten Aryl, Hetaryl, Arylcarbonyl und Hetarylcarbonyl gegebenenfalls einfach oder mehrfach, gleich oder unterschiedlich durch Halogen, Cyano, C₁-C₃-Alkyl, C₁-C₃-Halogenalkyl, C₁-C₃-Alkoxy, C₁-C₃-Halogenalkoxy, C₁-C₃-Alkoxy-C₁-C₂-alkyl substituiert sind.
R⁴ steht für einen Rest aus der Reihe Wasserstoff, Cyano, Nitro, C₁-C₃-Alkyl, C₃-C₆-Cycloalkyl, C₁-C₃-Halogenalkyl, C₃-C₆-Halogencycloalkyl, C₃-C₆-Cycloalkyl-C₁-C₃-alkyl, C₃-C₆-Halogen-cycloalkyl-C₁-C₃-alkyl, Cyano-C₁-C₃-alkyl, C₁-C₃-Alkoxy, C₁-C₃-Halogenalkoxy, C₁-C₃-Alkoxy-C₁-C₃-alkyl, C₁-C₃-Halogenalkoxy-C₁-C₃-alkyl, C₁-C₄-Alkylcarbonyl, C₁-C₄-Halogenalkylcarbonyl, C₁-C₃-Alkoxycarbonyl, C₁-C₃-Alkylcarbonyloxy.
Q steht für einen der Reste Q-1 bis Q-3, 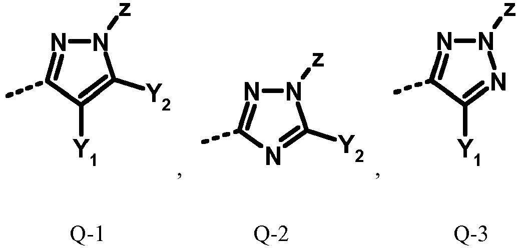 worin die gestrichelte Linie die Bindung zum Stickstoff in der Gruppe NR³ bedeutet.
Y₁ steht für einen Rest aus der Reihe Wasserstoff, Halogen, Cyano, Hydroxy, C₁-C₆-Alkyl, C₃-C₆-Cycloalkyl, C₁-C₆-Halogenalkyl, C₁-C₆-Alko_{X}y, C₁-C₆-Halogenalkoxy, C₁-C₆-Alkoxy-C₁-C₃-alkyl, C₁-C₆-Halogenalkoxy-C₁-C₃-alkyl, C₁-C₆-Alkoxy-C₁-C₃-alkyloxy, Cyano-C₁-C₃-alkyl, C₃-C₆-Cycloalkyl-C₁-C₃-alkyl, SH, C₁-C₃-Alkylthio, C₁-C₄-Alkylcarbonyl, C₁-C₄-Halogenalkylcarbonyl, C₁-C₃-Alkylsulfonyl.
Y₂ steht für einen Rest aus der Reihe Wasserstoff, Halogen, Cyano, Hydroxy, C₁-C₆-Alkyl, C₃-C₆-Cycloalkyl, C₁-C₆-Halogenalkyl, C₁-C₆-Alkoxy, C₁-C₆-Halogenalkoxy, C₁-C₆-Alkoxy-C₁-C₄-alkyl, C₁-C₆-Halogenalkoxy-C₁-C₄-alkyl, C₁-C₆-Alkoxy-C₁-C₄-alkyloxy, Cyano-C₁-C₃-alkyl, C₃-C₆-Cycloalkyl-C₁-C₂-alkyl, SH, C₁-C₃-Alkylthio, C₁-C₄-Alkylcarbonyl, C₁-C₄-Halogenalkylcarbonyl, C₁-C₄-Alkoxycarbonyl, C₁-C₃-Alkylsulfonyl, C₁-C₃-Halogenalkylsulfonyl, oder
im Falle von Q = Q-1 Y₁ und Y₂ zusammen mit den Kohlenstoffatomen, an die sie gebunden sind, einen Phenylring oder einen 5- bis 7-gliedrigen heteroaromatischen Ring ausbilden, welcher gegebenenfalls durch einen oder mehrere, gleiche oder verschiedene Substituenten M¹ substituiert ist.
M¹ steht für Halogen, Cyano, C₁-C₄-Alkyl, C₁-C₄-Halogenalkyl, C₁-C₄-Alkoxy und C₁-C₄-Halogenalkoxy.
Z steht für einen Rest aus der Reihe Wasserstoff, L¹(=G)C*, L¹O(=G)C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₄-alkyl-Gruppe durch M⁶ substituiertes L⁵C(=E)L²N(=G)C-C₁-C₄-alkyl*, L⁵(=O)ₙSL²N(=G)C-C₁-C₄-alkyl*, L⁵OC(=E)L²N(=G)C-C₁-C₄-alkyl*, L¹L⁵NC(=E)L²N(=G)C-C₁-C₄-alkyl*, L¹(=O)ₙS-C₁-C₄-alkyl*, L³O(=O)ₙS-C₁-C₄-alkyl*, L³L⁴N(=O)ₙS-C₁-C₄-alkyl*.
M² steht für Halogen, Cyano, NL⁹L¹⁰, OL⁹, SL⁹, L¹(=G)C*, L³O(=G)C*, L³L⁴N(=G)C*, Heterocyclyl, Aryl, Hetaryl, C₁-C₄-Alkyl, C₁-C₄-Halogenalkyl, C₁-C₄-Cyanoalkyl, C₃-C₆-Cycloalkyl, C₃-C₆-Halogencycloalkyl.
G und E stehen unabhängig voneinander für Sauerstoff oder Schwefel.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl, C₂-C₆-Alkenyl, C₃-C₆-Alkinyl.
M⁴ steht für Halogen, Cyano, C₁-C₃-Halogenalkyl, C₁-C₃-Alkoxy, C₁-C₃-Halogenalkoxy.
L² steht für Wasserstoff, Aryl, Hetaryl, gegebenenfalls einfach oder mehrfach, gleich oder verschieden durch M⁵ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁵ steht für Halogen, Cyano, C₁-C₆-Halogenalkyl, C₁-C₆-Alkoxy, C₁-C₆-Halogenalkoxy, C₁-C₄-Alkoxycarbonyl.
M⁶ steht für Halogen, Cyano, NL⁹L¹⁰, OL⁹, Aryl, Hetaryl, C₁-C₃-Halogenalkyl, C₃-C₄-Cycloalkyl, C₃-C₄-Halogencycloalkyl, C₃-C₄-Cycloalkyl-C₁-C₃-alkyl, C₃-C₄-Halogencycloalkyl-C₁-C₃-alkyl.
L⁹ und L¹⁰ stehen unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl, Cyano-C₁-C₆-alkyl, C₁-C₆-Alkoxy-C₁-C₄-alkyl, C₁-C₆-Halogenalkoxy-C₁-C₄-alkyl, C₁-C₆-Alkylcarbonyl, C₁-C₆-Halogenalkylcarbonyl, C₃-C₆-Cycloalkylcarbonyl, C₃-C₆-Cycloalkyl-C₁-C₄-alkylcarbonyl, C₃-C₆-Halogencycloalkylcarbonyl, C₁-C₆-Alkoxycarbonyl, C₁-C₆-Alkoxy-C₁-C₄-alkylcarbonyl, C₁-C₆-Halogenalkoxycarbonyl, C₃-C₆-Cycloalkoxycarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylamino-carbonyl, Aryl, Hetaryl, Heterocyclyl, Aryl-C₁-C₆-alkyl, Hetaryl-C₁-C₆-alkyl.

In den genannten besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₆-Alkyl, C₃-C₆-Cycloalkyl, C₁-C₆-Halogenalkyl, Cyano-C₁-C₄-alkyl, C₁-C₆-Alkoxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₁-C₄-Alkylcarbonylamino, C₁-C₄-Halogenalkylcarbonylamino, C₁-C₆-Cycloalkylcarbonylamino, C₁-C₄-Alkylcarbonyl, C₁-C₄-Halogenalkylcarbonyl, C₃-C₆-Cycloalky-lcarbonyl, C₃-C₆-Cycloalkyl-C₁-C₂-alkylcarbonyl, C₃-C₆-Halogencycloalkylcarbonyl, C₁-C₆-Alkoxycarbonyl, C₁-C₆-Alkoxy-C₁-C₂-alkylcarbonyl, C₁-C₆-Halogenalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkyl-carbonyloxy, C₁-C₄-Halogenalkylcarbonyloxy, C₁-C₄-Alkoxycarbonyloxy, C₁-C₄-Halogenalkoxy-carbonyloxy, Aryl, Hetaryl, Aryloxy, Hetaryloxy substituiert.

Ganz besonders bevorzugte Substituenten bzw. Bereiche der in den Verbindungen der Formel (I) aufgeführten Reste werden im Folgenden erläutert. Ihre Kombination bildet den Vorzugsbereich (3). 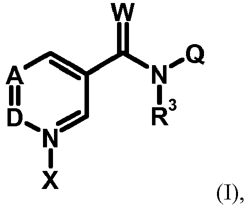
A steht für CR¹.
D steht für CR².
R¹ steht für Wasserstoff oder Fluor.
R² steht für Wasserstoff.
X steht für ein freies Elektronenpaar oder Sauerstoff.
W steht für Sauerstoff oder Schwefel.
R³ steht für einen Rest aus der Reihe Wasserstoff, C₁-C₄-Alkyl, C₃-C₄-Cycloalkyl, C₁-C₄-Halogenalkyl, C₃-C₄-Cycloalkyl-C₁-C₂-alkyl, C₁-C₃-Alkoxy-C₁-C₂-alkyl, C₁-C₄-Alkylcarbonyl, C₁-C₄-Halogenalkylcarbonyl, C₃-C₄-Cycloalkylcarbonyl, C₁-C₃-Alkoxycarbonyl, Aryl, Hetaryl, Arylcarbonyl, Hetarylcarbonyl, Aryl-C₁-C₃-alkyl, Hetaryl-C₁-C₃-alkyl, wobei die Substituenten Aryl, Hetaryl, Aryl-C₁-C₃-alkyl, Hetaryl-C₁-C₃-alkyl, Arylcarbonyl und Hetarylcarbonyl gegebenenfalls einfach oder mehrfach durch Halogen substituiert sind.
Q steht für Q-1, 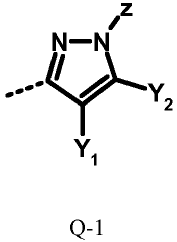 worin die gestrichelte Linie die Bindung zum Stickstoff in der Gruppe NR³ bedeutet.
Y₁ steht für einen Rest aus der Reihe Wasserstoff, Halogen, Cyano, C₁-C₄-Alkyl, C₁-C₄-Halogenalkyl, C₁-C₃-Alkoxy, C₁-C₃-Halogenalkoxy.
Y₂ steht für einen Rest aus der Reihe Wasserstoff, Halogen, Cyano, C₁-C₄-Alkyl, C₃-C₅-Cycloalkyl, C₁-C₄-Halogenalkyl, C₁-C₄-Alkoxy, C₁-C₄-Halogenalkoxy, C₁-C₃-Alkoxy-C₁-C₄-alkyl, C₁-C₃-Halogenalkoxy-C₁-C₄-alkyl, C₁-C₃-Alkoxy-C₁-C₄-alkyloxy, C₁-C₄-Alkoxycarbonyl, oder
im Falle von Q = Q-1 Y₁ und Y₂ zusammen mit den Kohlenstoffatomen, an die sie gebunden sind, einen Phenylring oder einen 5- bis 7-gliedrigen heteroaromatischen Ring ausbilden, welcher gegebenenfalls durch einen oder mehrere, gleiche oder verschiedene Substituenten M¹ substituiert ist.
M¹ steht für Halogen, Cyano, C₁-C₃-Alkyl, C₁-C₃-Halogenalkyl, C₁-C₂-Alkoxy und C₁-C₂-Halogenalkoxy.
Z steht für einen Rest aus der Reihe Wasserstoff, L¹(=G)C*, L¹O(=G)C* L³L⁴N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₄-alkyl-Gruppe durch M⁶ substituiertes L⁵(=O)ₙSL²N(=G)C-C₁-C₄-alkyl*, L¹(=O)ₙS-C₁-C₄-alkyl*.
M² steht für Halogen, Cyano, NL⁹L¹⁰, OL⁹, SL⁹, L¹(=G)C*, L³O(=G)C*, L³L⁴N(=G)C*, Heterocyclyl, Aryl, Hetaryl, C₁-C₄-Alkyl, C₁-C₄-Halogenalkyl, C₁-C₄-Cyanoalkyl, C₃-C₄-Cycloalkyl, C₃-C₄-Halogencycloalkyl.
G steht für Sauerstoff.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₄-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁴ steht für Halogen, Cyano, C₁-C₃-Halogenalkyl, C₁-C₃-Alkoxy, C₁-C₃-Halogenalkoxy.
L² steht für Wasserstoff, gegebenenfalls einfach oder mehrfach, gleich oder verschieden durch M⁵ substituiertes C₁-C₄-Alkyl, C₃-C₆-Cycloalkyl, C₃-C₆-Cycloalkyl-C₁-C₄-alkyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₄-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁵ steht für Halogen, Cyano, C₁-C₃-Halogenalkyl, C₁-C₃-Alkoxy, C₁-C₄-Alkoxycarbonyl.
M⁶ steht für Halogen, Cyano, C₁-C₃-Halogenalkyl.
L⁹ und L¹⁰ stehen unabhängig voneinander für Wasserstoff, C₁-C₄-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl, Cyano-C₁-C₄-alkyl, C₁-C₄-Alkoxy-C₁-C₄-alkyl, C₁-C₄-Halogenalkoxy-C₁-C₄-alkyl, C₁-C₄-Alkylcarbonyl, C₁-C₄-Halogenalkylcarbonyl, C₃-C₆-Cycloalkylcarbonyl, C₃-C₆-Cycloalkyl-C₁-C₄-alkylcarbonyl, C₃-C₆-Halogencycloalkylcarbonyl, C₁-C₄-Alkoxycarbonyl, C₁-C₄-Alkoxy-C₁-C₄-alkylcarbonyl, C₁-C₄-Halogenalkoxycarbonyl, C₁-C₄-Alkylaminocarbonyl, Di-(C₁-C₄)-alkylamino-carbonyl, C₃-C₆-Cycloalkylaminocarbonyl, C₃-C₆-Cycloalkyl-C₁-C₄-alkylaminocarbonyl, Aryl, Hetaryl, Heterocyclyl, Aryl-C₁-C₃-alkyl, Hetaryl-C₁-C₃-alkyl.

In den genannten ganz besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₄-Alkyl, C₃-C₆-Cycloalkyl, C₁-C₄-Halogenalkyl, C₁-C₄-Alkoxy, C₁-C₄-Halogenalkoxy, C₃-C₆-Halogencycloalkyl, C₁-C₄-Alkoxy-C₁-C₄-alkyl, C₁-C₄-Halogenalkoxy-C₁-C₄-alkyl, C₁-C₄-Alkoxycarbonyl, C₁-C₄-Halogenalkoxycarbonyl, C₁-C₃-Alkylthio, C₁-C₃-Halogenalkylthio, C₁-C₃-Alkylsulfonyl, C₁-C₃-Halogenalkylsulfonyl, C₁-C₃-Alkylaminosulfonyl, C₁-C₃-Halogenalkyl-aminosulfonyl, Di-(C₁-C₃)-alkylaminosulfonyl, C₁-C₃-Alkylaminocarbonyl, Di-(C₁-C₃)-alkylamino-carbonyl, Aryl, Hetaryl, Aryloxy, Hetaryloxy substituiert.

Ausdrücklich ganz besonders bevorzugte Substituenten bzw. Bereiche der in den Verbindungen der Formel (I) aufgeführten Reste werden im Folgenden erläutert. Ihre Kombination bildet den Vorzugsbereich (4). 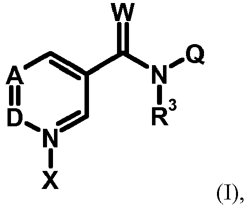
A steht für C_{R}¹.
D steht für CR².
R¹ steht für Wasserstoff oder Fluor.
_{R}² steht für Wasserstoff.
X steht für ein freies Elektronenpaar.
W steht für Sauerstoff.
R³ steht für einen Rest aus der Reihe Wasserstoff, C₁-C₄-Alkyl, C₁-C₄-Halogenalkyl, C₃-C₄-Cycloalkyl-C₁-C₂-alkyl, C₁-C₄-Alkylcarbonyl, Arylcarbonyl, Hetarylcarbonyl, wobei die Substituenten Arylcarbonyl und Hetarylcarbonyl gegebenenfalls einfach oder mehrfach durch Halogen substituiert sind.
Q steht für Q-1, 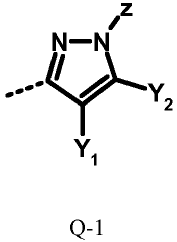 worin die gestrichelte Linie die Bindung zum Stickstoff in der Gruppe NR³ bedeutet.
Y₁ steht für einen Rest aus der Reihe Wasserstoff, Halogen, Cyano, Methyl, Ethyl, *n*-Propyl, *iso*Propyl, Trifluormethyl, Difluormethyl, Methoxy, Ethoxy, Difluormethoxy, Trifluormethoxy.
Y₂ steht für einen Rest aus der Reihe Wasserstoff, Halogen, Cyano, C₁-C₄-Alkyl, C₃-C₅-Cycloalkyl, C₁-C₃-Halogenalkyl, C₁-C₃-Alkoxy, C₁-C₃-Halogenalkoxy, C₁-C₃-Alkoxy-C₁-C₂-alkyl, C₁-C₃-Halogenalkoxy-C₁-C₂-alkyl, C₁-C₃-Alkoxy-C₁-C₂-alkyloxy, C₁-C₃-Alkoxycarbonyl.
Z steht für einen Rest aus der Reihe Wasserstoff, L¹(=G)C*, L¹O(=G)C*, L³L⁴N(=G)C*, L¹(=O)ₙS*, L³L⁴N(=O)ₙS*, Aryl, Hetaryl, C₃-C₆-Cycloalkyl, C₃-C₆-Halogencycloalkyl, C₂-C₆-Alkenyl, C₃-C₆-Alkinyl, C₂-C₆-Halogenalkenyl, C₃-C₆-Halogenalkinyl, für gegebenenfalls einfach oder mehrfach, gleich oder verschieden durch M² substituiertes C₁-C₆-Alkyl, für gegebenenfalls einfach oder mehrfach, gleich oder verschieden in der -C₁-C₄-alkyl-Gruppe durch M⁶ substituiertes L⁵(=O)ₙSL²N(=G)C-C₁-C₄-alkyl*, L¹(=O)ₙS-C₁-C₄-alkyl*.
M² steht für Halogen, Cyano, Heterocyclyl, Aryl, Hetaryl, C₃-C₄-Cycloalkyl, C₃-C₄-Halogencycloalkyl, NL⁹L¹⁰, OL⁹, SL⁹, L¹(=G)C*, L³O(=G)C*, L³L⁴N(=G)C*.
G steht für Sauerstoff.
n steht für 1 oder 2.
L¹ und L⁵ stehen unabhängig voneinander für Aryl, Hetaryl, C₁-C₄-Alkyl, C₁-C₄-Halogenalkyl, C₁-C₄-Cyanoalkyl, C₁-C₃-Alkoxy-C₁-C₄-alkyl, C₁-C₃-Halogenalkoxy-C₁-C₄-alkyl, Aryl-C₁-C₄-alkyl, Aryl-C₁-C₄-halogenalkyl, Aryl-C₁-C₄-cyanoalkyl, Hetaryl-C₁-C₄-alkyl, Hetaryl-C₁-C₄-halogenalkyl, Hetaryl-C₁-C₄-cyanoalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl.
L² steht für Wasserstoff oder C₁-C₄-Alkyl.
L³ und L⁴ stehen unabhängig voneinander für Wasserstoff, Aryl, Hetaryl, C₁-C₄-Alkyl, C₁-C₄-Halogenalkyl, C₁-C₄-Cyanoalkyl, C₁-C₃-Alkoxy-C₁-C₄-alkyl, Aryl-C₁-C₄-alkyl, Aryl-C₁-C₄-halogenalkyl, Aryl-C₁-C₄-cyanoalkyl, Hetaryl-C₁-C₄-alkyl, Hetaryl-C₁-C₄-halogenalkyl, Hetaryl-C₁-C₄-cyanoalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl.
M⁶ steht für Halogen, Cyano, C₁-C₃-Halogenalkyl.
L⁹ und L¹⁰ stehen unabhängig voneinander für Wasserstoff, C₁-C₄-Alkyl, C₁-C₄-Halogenalkyl, C₃-C₆-Cycloalkyl, C₃-C₆-Cycloalkyl-C₁-C₄-alkyl, Cyano-C₁-C₄-alkyl, C₁-C₄-Alkylcarbonyl, C₁-C₄-Halogenalkylcarbonyl, C₃-C₆-Cycloalkyl-C₁-C₄-alkylcarbonyl, C₁-C₄-Alkoxycarbonyl, C₁-C₄-Alkylaminocarbonyl, Di-(C₁-C₄)-alkylaminocarbonyl, C₃-C₆-Cycloalkylaminocarbonyl, C₃-C₆-Cycloalkyl-C₁-C₄-alkylaminocarbonyl, Aryl, Hetaryl, Aryl-C₁-C₃-alkyl, Hetaryl-C₁-C₃-alkyl.

In den genannten ausdrücklich ganz besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₃-Alkyl, C₁-C₃-Halogenalkyl, C₁-C₂-Alkoxy, C₁-C₂-Halogenalkoxy, C₁-C₃-Alkoxycarbonyl, C₁-C₃-Halogenalkoxycarbonyl, C₁-C₂-Alkylthio, C₁-C₂-Halogenalkylthio, C₁-C₂-Alkylsulfonyl, C₁-C₂-Halogenalkylsulfonyl, C₁-C₃-Alkylaminosulfonyl, C₁-C₃-Alkylaminocarbonyl, Aryl, Hetaryl, Aryloxy, Hetaryloxy substituiert.

Eine bevorzugte Ausführungsform der Erfindung betrifft die Verwendung von Verbindungen der Formel (I), in denen die Substituenten bzw. Bereiche der aufgeführten Reste die folgenden Bedeutungen haben. Ihre Kombination bildet den Vorzugsbereich (5). 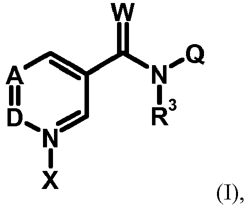
A steht für N oder CR¹.
D steht für N oder CR², wobei A und D nicht gleichzeitig für N stehen.
R¹ steht für Wasserstoff, Chlor, Brom oder Fluor.
R² steht für Wasserstoff, Methyl, Methoxy oder Chlor.
X steht für ein freies Elektronenpaar oder Sauerstoff.
W steht für Sauerstoff oder Schwefel.
R³ steht für einen Rest aus der Reihe Wasserstoff, C₁-C₃-Alkyl, C₁-C₃-Halogenalkyl, C₃-C₄-Alkinyl, Cyano-C₁-C₂-alkyl, Arylcarbonyl und Hetaryl-C₁-C₂-alkyl, wobei die Substituenten Arylcarbonyl und Hetaryl-C₁-C₂-alkyl gegebenenfalls einfach oder mehrfach, gleich oder unterschiedlich durch Halogen substituiert sind.
Q steht für Q-1, 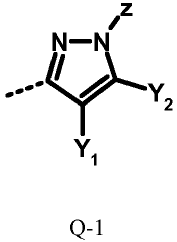 worin die gestrichelte Linie die Bindung zum Stickstoff in der Gruppe NR³ bedeutet.
Y₁ steht für einen Rest aus der Reihe Wasserstoff, Halogen, C₁-C₃-Alkyl, Cyano und C₁-C₂-Alkoxycarbonyl,
Y₂ steht für einen Rest aus der Reihe Wasserstoff, C₁-C₃-Alkyl, C₃-C₅-Cycloalkyl, C₁-C₂-Halogenalkyl, Halogen, Cyano, C₁-C₂-Halogenalkoxy, C₁-C₂-Alkoxycarbonyl, C₁-C₂-Alkylthio und C₁-C₂-Alkylsulfinyl, oder
Y₁ und Y₂ bilden zusammen mit den Kohlenstoffatomen, an die sie gebunden sind, einen Phenylring oder einen 6-gliedrigen heteroaromatischen Ring, welche gegebenenfalls einfach oder mehrfach durch Methyl substituiert sind.
Z steht für einen Rest aus der Reihe Wasserstoff, C₁-C₆-Alkyl, C₁-C₄-Halogenalkyl, C₃-C₇-Cycloalkyl, C₂-C₆-Alkenyl, C₂-C₄-Halogenalkenyl, C₃-C₅-Alkinyl, Cyano-C₁-C₄-alkyl, C₃-C₅-Cycloalkyl-C₁-C₃-alkyl, C₃-C₅-Halogencycloalkyl-C₁-C₂-alkyl, Aryl, Hetaryl, Aryl-C₁-C₂-alkyl, Hetaryl-C₁-C₂-alkyl, Hetaryl-C₁-C₃-halogenalkyl, Heterocyclyl-C₁-C₂-alkyl, C₁-C₆-Alkylaminocarbonyl, C₃-C₆-Cycloalkylaminocarbonyl, C₁-C₃-Halogenalkylaminocarbonyl, C₃-C₅-Cycloalkyl-C₁-C₂-alkylamino-carbonyl, C₁-C₂-Alkoxy-C₁-C₄-alkylaminocarbonyl, Di-(C₁-C₃-Alkoxy)-C₁-C₄-alkylaminocarbonyl, C₁-C₂-Halogenalkoxy-C₁-C₃-alkylaminocarbonyl, Di-(C₁-C₃)-alkylaminocarbonyl, Di-(C₁-C₂-Alkoxy-C₁-C₄-alkyl)-aminocarbonyl, Aryl-C₁-C₄-alkylaminocarbonyl, Hetaryl-C₁-C₄-alkylaminocarbonyl, Heterocyclyl-C₁-C₃-alkylaminocarbonyl, Aryloxy-C₁-C₃-alkylaminocarbonyl, C₁-C₂-Alkylsulfonyl-(C₁-C₂-alkylamino)carbonyl, Arylsulfonyl-(C₁-C₂-alkylamino)carbonyl, C₁-C₄-Alkylaminosulfonyl, Di-(C₁-C₂)-alkylaminosulfonyl, C₁-C₃-Alkylsulfonyl, Hetarylsulfonyl, Heterocyclylsulfonyl, C₁-C₅-Alkoxycarbonyl, C₁-C₃-Halogenalkoxycarbonyl, Aryloxycarbonyl, C₁-C₅-Alkylcarbonyl, C₃-C₅-Cycloalkylcarbonyl, Arylcarbonyl, Hetarylcarbonyl, Heterocyclylcarbonyl, C₁-C₂-Alkoxy-C₁-C₃-alkyl, C₁-C₃-Alkylthio-C₁-C₃-alkyl, C₁-C₃-Alkylsulfinyl-C₁-C₃-alkyl, C₁-C₃-Alkylsulfonyl-C₁-C₃-alkyl, Hetarylthio-C₁-C₂-alkyl, Hetarylsulfinyl-C₁-C₂-alkyl, Hetarylsulfonyl-C₁-C₂-alkyl, C₁-C₂-Alkylthio-C₁-C₃-alkylcarbonyl, C₁-C₂-Alkylsulfonyl-C₁-C₃-alkylcarbonyl, C₁-C₂-Alkylthio-C₁-C₃-alkylamino-carbonyl, C₁-C₂-Alkylsulfinyl-C₁-C₃-alkylaminocarbonyl, C₁-C₂-Alkylsulfonyl-C₁-C₃-alkylamino-carbonyl, C₁-C₂-Alkoxy-C₁-C₂-alkylcarbonyl, C₃-C₅-Halogencycloalkylcarbonyl-C₁-C₂-alkyl, C₁-C₃-Alkoxycarbonyl-C₁-C₄-alkyl, C₁-C₂-Alkoxycarbonyloxy-C₁-C₃-alkyl, C₁-C₃-Alkylaminocarbonyl-C₁-C₂-alkyl, Hydroxycarbonyl-C₁-C₂-alkyl, C₁-C₂-Alkoxycarbonyl-C₁-C₄-alkylaminocarbonyl-C₁-C₂-alkyl, C₁-C₃-Alkoxycarbonyl-di-(C₁-C₂)-alkylammocarbonyl-C₁-C₂-alkyl, Hetaryl-C₂-C₃-halogenalkenyl und Aryl-C₁-C₂-alkylcarbonyl-C₁-C₂-alkyl,
wobei die Substituenten Aryl, Hetaryl, Aryl-C₁-C₂-alkyl, Hetaryl-C₁-C₂-alkyl, Hetaryl-C₁-C₃-halogenalkyl, Aryl-C₁-C₄-alkylaminocarbonyl, Hetaryl-C₁-C₄-alkylaminocarbonyl, Aryloxy-C₁-C₃-alkylaminocarbonyl, Arylsulfonyl-(C₁-C₂-alkylamino)carbonyl, Hetarylsulfonyl, Aryloxycarbonyl, Arylcarbonyl, Hetarylcarbonyl, Hetarylthio-C₁-C₂-alkyl, Hetarylsulfinyl-C₁-C₂-alkyl, Hetarylsulfonyl-C₁-C₂-alkyl, Hetaryl-C₂-C₃-halogenalkenyl und Aryl-C₁-C₂-alkylcarbonyl-C₁-C₂-alkyl gegebenenfalls einfach oder mehrfach, gleich oder unterschiedlich durch Halogen, Cyano, C₁-C₅-Alkyl, C₁-C₂-Halogenalkyl, Di-(C₁-C₂)-alkylaminocarbonyl, C₁-C₃-Alkoxycarbonyl, Phenyl, C₁-C₂-Alkoxy, C₁-C₂-Halogenalkoxy, C₁-C₃-Halogenalkylthio oder COOH substituiert sind,
und wobei die Substituenten Heterocyclyl-C₁-C₂-alkyl, Heterocyclyl-C₁-C₃-alkylaminocarbonyl, Heterocyclylsulfonyl und Heterocyclylcarbonyl gegebenenfalls einfach oder mehrfach, gleich oder unterschiedlich durch C₁-C₃-Alkyl, C₃-C₅-Cycloalkyl oder C₁-C₂-Alkoxy substituiert sind.

In den bevorzugten Definitionen (Vorzugsbereich (1)) ist, sofern nichts anderes angegeben ist,
Halogen ausgewählt aus der Reihe Fluor, Chlor, Brom und Iod, bevorzugt wiederum aus der Reihe Fluor, Chlor, Brom und Iod,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, Triazolyl, Oxazolyl, Isoxazolyl, Thiazolyl, Isothiazolyl, Oxadiazolyl, Thiadiazolyl, Tetrazolyl, Pyridyl, Pyrimidinyl, Pyridazinyl, Pyrazinyl, Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Indolizinyl, Imidazopyridinyl und Benzthiadiazol.

Heterocyclyl steht für einen gesättigten oder ungesättigten, nicht heteroaromatischen Rest, der mindestens einen 3- bis 7-gliedrigen heterocyclischen Ring (=carbocyclischer Ring, in dem mindestens ein C-Atom durch ein Heteroatom ersetzt ist, vorzugsweise durch ein Heteroatom aus der Gruppe N, O, S) enthält, dabei kann dieser unsubstituiert oder substituiert sein, wobei die Bindungsstelle an einem Ringatom lokalisiert ist. Heterocyclyl ist bevorzugt ausgewählt aus der Reihe Aziridinyl, Azirenyl, Oxiranyl, Oxirenyl, Thiiranyl, Thiirenyl, Azetidinyl, Dihydroazetyl, Oxetanyl, Oxetyl, Thietanyl, Thietyl, Dioxetanyl, Dithietanyl, Oxathietanyl, Pyrrolidinyl, Dihydropyrrolyl, Piperidinyl, Tetrahydropyridinyl, Dihydropyridinyl, Azepanyl, Tetrahydrofuranyl, Dihydrofuranyl, Tetrahydropyranyl, Dihydropyranyl, Pyranyl, Oxepanyl, Tetrahydrothiophenyl, Dihydrothiophenyl, Tetrahydrothiopyranyl, Dihydrothiopyranyl, Thiepanyl, Dioxolanyl, Dioxanyl, Dioxepanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl. Handelt es sich um einen teilweise oder vollständig gesättigten Stickstoff-haltigen Heterocyclus, so kann dieser sowohl über Kohlenstoff als auch über den Stickstoff mit dem Rest des Moleküls verknüpft sein. Wenn in obigen Definitionen in Ringen Schwefel vorkommt, dann kann der Schwefel gegebenenfalls auch als SO oder SO₂ vorliegen. Gegebenenfalls kann der Heterocyclus einfach oder mehrfach durch Sauerstoff oder Schwefel substituiert sein, was die Bildung von C=O bzw. C=S Gruppen zur Folge hat.

In einer bevorzugten Ausführungsform gelten diese Definitionen auch für den Vorzugsbereich (5).

In den besonders bevorzugten Definitionen (Vorzugsbereich (2)) ist, sofern nichts anderes angegeben ist,
Halogen ausgewählt aus der Reihe Fluor, Chlor, Brom und Iod,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, Triazolyl, Oxazolyl, Isoxazolyl, Thiazolyl, Isothiazolyl, Oxadiazolyl, Thiadiazolyl, Tetrazolyl, Pyridyl, Pyrimidinyl, Pyridazinyl, Pyrazinyl, Imidazopyridinyl und Benzthiadiazol.

Heterocyclyl ist ausgewählt aus der Reihe Aziridinyl, Oxiranyl, Thiiranyl, Azetidinyl, Oxetanyl, Thietanyl, Pyrrolidinyl, Dihydropyrrolyl, Piperidinyl, Tetrahydropyridinyl, Dihydropyridinyl, Azepanyl, Tetrahydrofuranyl, Dihydrofuranyl, Tetrahydropyranyl, Dihydropyranyl, Pyranyl, Oxepanyl, Tetrahydrothiophenyl, Dihydrothiophenyl, Tetrahydrothiopyranyl, Dihydrothiopyranyl, Thiepanyl, Dioxolanyl, Dioxanyl, Dioxepanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (5).

In den ganz besonders bevorzugten Definitionen (Vorzugsbereich (3)) ist, sofern nichts anderes angegeben ist,
Halogen ausgewählt aus der Reihe Fluor, Chlor, Brom und Iod,

Aryl (auch als Teil einer größeren Einheit, wie beispielsweise Arylalkyl) ausgewählt aus der Reihe Phenyl, Naphthyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Pyrazolyl, Imidazolyl, Triazolyl, Thiazolyl, Isothiazolyl, Thiadiazolyl, Tetrazolyl, Pyridyl, Pyrimidinyl, Pyridazinyl, Pyrazinyl, Imidazopyridinyl und Benzthiadiazol.

Heterocyclyl ist ausgewählt aus der Reihe Aziridinyl, Oxiranyl, Thiiranyl, Azetidinyl, Oxetanyl, Thietanyl, Pyrrolidinyl, Piperidinyl, Tetrahydrofuranyl, Tetrahydropyranyl, Tetrahydrothiophenyl, Tetrahydrothiopyranyl, Dioxolanyl, Dioxanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (5).

In den ausdrücklich ganz besonders bevorzugten Definitionen (Vorzugsbereich (4)) ist, sofern nichts anderes angegeben ist,
Halogen ausgewählt aus der Reihe Fluor, Chlor und Brom,
Aryl (auch als Teil einer größeren Einheit, wie beispielsweise Arylalkyl) gleich Phenyl,

Hetaryl (gleichbedeutend mit Heteroaryl, auch als Teil einer größeren Einheit, wie beispielsweise Hetarylalkyl) ausgewählt aus der Reihe Pyrazolyl, Imidazolyl, Triazolyl, Thiazolyl, Isothiazolyl, Thiadiazolyl, Pyridyl, Pyrimidinyl, Pyridazinyl, Pyrazinyl, Imidazopyridinyl und Benzthiadiazol.

Heterocyclyl ist ausgewählt aus der Reihe Tetrahydrofuranyl, Tetrahydropyranyl, Tetrahydrothiophenyl, Tetrahydrothiopyranyl, Dioxolanyl, Dioxanyl, Dithiolanyl, Dithianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (5).

Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod.

Gesättigte oder ungesättigte Kohlenwasserstoffreste wie z.B. Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt (Vorzugsbereich (1).

Erfindungsgemäß besonders bevorzugt verwendet werden Verbindungen der Formel (I), in welchen eine Kombination der vorstehend als besonders bevorzugt aufgeführten Bedeutungen vorliegt (Vorzugsbereich (2).

Erfindungsgemäß ganz besonders bevorzugt verwendet werden Verbindungen der Formel (I), in welchen eine Kombination der vorstehend als ganz besonders bevorzugt aufgeführten Bedeutungen vorliegt (Vorzugsbereich (3).

Erfindungsgemäß ausdrücklich ganz besonders bevorzugt verwendet werden Verbindungen der Formel (I), in welchen eine Kombination der vorstehend als ausdrücklich ganz besonders bevorzugt aufgeführten Bedeutungen vorliegt (Vorzugsbereich (4).

Die Erfindung betrifft auch neue Verbindungen der Formel (I) 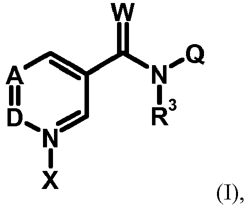 worin
A für N oder CR¹ steht,
D für N oder CR² steht, wobei A und D nicht gleichzeitig für N stehen,
R¹ für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
R² für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
X für ein freies Elektronenpaar oder Sauerstoff steht,
W für Sauerstoff, Schwefel oder NR⁴ steht,
R³ für einen Rest aus der Reihe Wasserstoff, Cyano, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl, C₁-C₆-Alkylcarbonyloxy, C₁-C₆-Alkoxycarbonyloxy, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-C₁-C₆-alkylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
R⁴ für einen Rest aus der Reihe Wasserstoff, Cyano, Nitro, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₆-Alkylsulfonyl, C₁-C₆-Halogenalkylsulfonyl, Aryl, Hetaryl, Aryl-C₁-C₆-alkyl, Hetaryl-C₁-C₆-alkyl, Arylcarbonyl, Hetarylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
Q für einen der Reste Q-1 bis Q-4 steht, 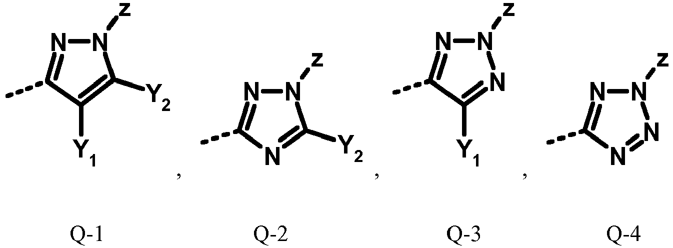 worin die gestrichelte Linie die Bindung zum Stickstoff in der Gruppe NR³ bedeutet,
Y₁ für einen Rest aus der Reihe Wasserstoff, Halogen, Cyano, Nitro, C₁-C₆-Alkylamino, Di-(C₁-C₆)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Y₂ für einen Rest aus der Reihe Wasserstoff, Halogen, Cyano, Nitro, C₁-C₆-Alkylamino, Di-(C₁-C₆)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Z für einen Rest aus der Reihe Wasserstoff, Cyano, Nitro, L¹(=G)C*, L¹O(=G)C* L³L⁴N(=G)C*, L⁵(=E)CL²N(=G)C*, L⁵S(=O)ₙL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₈-Alkyl, C₃-C₈-Cycloalkyl, C₂-C₈-Alkenyl, C₅-C₈-Cycloalkenyl, C₃-C₈-Alkinyl, für gegebenenfalls einfach oder mehrfach, gleich oder verschieden in der - C₁-C₈-alkyl-Gruppe durch M⁶ substituiertes L⁵C(=E)L²N(=G)C-C₁-C₈-alkyl*, L⁵S(=O)ₙL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl* steht,
mit der Maßgabe, dass, wenn Q für Q-4 steht, Z nicht gleichzeitig für Wasserstoff oder unsubstituiertes C₁-C₈-Alkyl steht, und mit der Maßgabe, dass Z nicht für Wasserstoff steht, wenn W für NOH steht, und mit der Maßgabe, dass, wenn Q für Q-2 steht, Z nicht gleichzeitig für Wasserstoff steht,
M² für Halogen, Cyano, Nitro, NL⁹L¹⁰ OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆-Alkyl, C₃-C₆-Cycloalkyl, C₂-C₆-Alkenyl, C₅-C₆-Cycloalkenyl, C₃-C₆-Alkinyl, C₁-C₆-Alkoxy steht,
M³ für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C* steht,
G und E unabhängig voneinander für Sauerstoff oder Schwefel stehen,
n für 1 oder 2 steht,
L¹ und L⁵ unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁴ für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹ steht,
L² für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl steht,
L³ und L⁴ unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁵ für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹ steht,
M⁶ für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₅-C₆-Cycloalkenyl, C₃-C₆-Alkinyl, C₁-C₆-Alkoxy-C₁-C₆-alkyl, C₁-C₆-Halogenalkoxy-C₁-C₆-alkyl steht,
L⁹ und L¹⁰ unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₆-Alkenyl, C₂-C₆-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl stehen,
wobei, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₂-C₆-Alkenyl, C₃-C₆-Alkinyl, C₃-C₆-Cycloalkyl, C₁-C₆- Halogenalkyl, Cyano-C₁-C₆-alkyl, C₂-C₆-Halogenalkenyl, C₃-C₆-Halogenalkinyl, C₁-C₆-Alkoxy, C₂-C₆-Alkenyloxy, C₃-C₆-Alkinyloxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkylcarbonylamino, CO₂H SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert sind,
mit der Maßgabe, dass, wenn Y₂ für Methyl und Z für Phenyl-C₁-C₆-alkyl steht, der Phenylring in der ortho-Position nicht durch Alkoxy und nicht durch Arylalkoxy substituiert ist, mit der Maßgabe, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und mit der Maßgabe, dass Verbindungen der Formeln (G1), (G2) und (G4) ausgenommen sind.

Bevorzugte Substituenten bzw. Bereiche der in den neuen Verbindungen der Formel (I) aufgeführten Reste werden im Folgenden erläutert (Vorzugsbereich (6). 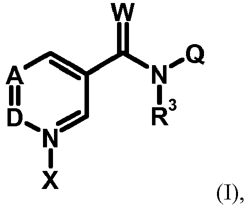
A steht für N oder CR¹.
D steht für N oder CR², wobei A und D nicht gleichzeitig für N stehen.
R¹ steht für einen Rest aus der Reihe Wasserstoff, Halogen, Cyano, Nitro, C₁-C₃-Alkyl, C₁-C₃-Halogenalkyl, C₁-C₃-Alkoxy, C₁-C₃-Halogenalkoxy.
R² steht für einen Rest aus der Reihe Wasserstoff, Halogen, Cyano, Nitro, C₁-C₃-Alkyl, C₁-C₃-Halogenalkyl, C₁-C₃-Alkoxy, C₁-C₃-Halogenalkoxy.
X steht für ein freies Elektronenpaar oder Sauerstoff.
W steht für Sauerstoff, Schwefel oder NR⁴.
R³ steht für einen Rest aus der Reihe Wasserstoff, Cyano, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-₁-C₆-alkyl, C₁-C₆-Alkylcarbonyl, C₁-C₆-Halogenalkylcarbonyl, C₃-C₆-Cycloalkylcarbonyl, C₃-C₆-Cycloalkyl-C₁-C₂-alkylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₃-Alkylsulfonyl, C₁-C₃-Halogenalkylsulfonyl, C₁-C₃-Alkylaminocarbonyl, Di-(C₁-C₃)-alkylaminocarbonyl, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-₁-C₆-alkylcarbonyl gegebenenfalls einfach oder mehrfach, gleich oder unterschiedlich durch Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind.
R⁴ steht für einen Rest aus der Reihe Wasserstoff, Cyano, Nitro, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₃-Alkylsulfonyl, C₁-C₃-Halogenalkylsulfonyl, Aryl, Hetaryl, Aryl-C₁-C₆-alkyl, Hetaryl-C₁-C₆-alkyl, Arylcarbonyl, Hetarylcarbonyl, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, C₁-C₄-Alkyl, C₁-C₄-Halogenalkyl, C₁-C₃-Alkoxy, C₁-C₃-Halogenalkoxy, C₁-C₆-Alkoxy-C₁-C₂-alkyl substituiert sind.
Q steht für einen der Reste Q-1 bis Q-4, 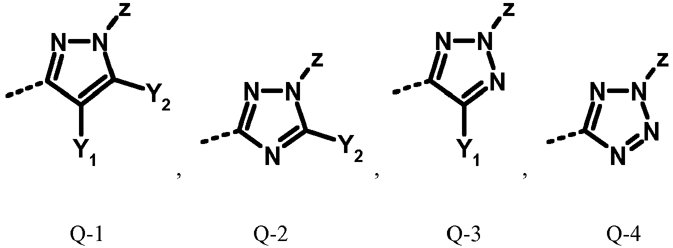 worin die gestrichelte Linie die Bindung zum Stickstoff in der Gruppe NR³ bedeutet.
Y₁ steht für einen Rest aus der Reihe Wasserstoff, Halogen, Cyano, C₁-C₃-Alkylamino, Di-(C₁-C₃)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₂-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₁-C₆-Alkylthio, C₁-C₆-Alkylcarbonyl, C₃-C₆-Cycloalkylcarbonyl, C₂-C₄-Alkenylcarbonyl, C₁-C₆-Halogenalkylcarbonyl, C₁-C₃-Alkoxycarbonyl, C₁-C₆-Alkylsulfonyl, C₁-C₃-Halogenalkylsulfonyl, C₁-C₃-Alkylaminocarbonyl, Di-(C₁-C₃)-alkylaminocarbonyl.
Y₂ steht für einen Rest aus der Reihe Wasserstoff, Halogen, Cyano, C₁-C₃-Alkylamino, Di-(C₁-C₃)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₂-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₂-alkyl, SH, C₁-C₆-Alkylthio, C₁-C₆-Alkylcarbonyl, C₃-C₆-Cycloalkylcarbonyl, C₂-C₄-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₃-Alkylaminocarbonyl, Di-(C₁-C₃)-alkylaminocarbonyl.
Z steht für einen Rest aus der Reihe Wasserstoff, Cyano, L¹(=G)C*, L¹O(=G)C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₆-alkyl-Gruppe durch M⁶ substituiertes L⁵C(=E)L²N(=G)C-C₁-C₆-alkyl*, L⁵(=O)ₙSL²N(=G)C-C₁-C₆-alkyl*, L⁵OC(=E)L²N(=G)C-C₁-C₆-alkyl*, L¹L⁵NC(=E)L²N(=G)C-C₁-C₆-alkyl*, L¹(=O)ₙS-C₁-C₆-alkyl*, L³O(=O)ₙS-C₁-C₆-alkyl*, L³L⁴N(=O)ₙS-C₁-C₆-alkyl*,
mit der Maßgabe, dass, wenn Q für Q-4 steht, Z nicht gleichzeitig für Wasserstoff oder unsubstituiertes C₁-C₈-Alkyl steht, und mit der Maßgabe, dass, wenn Q für Q-2 steht, Z nicht gleichzeitig für Wasserstoff steht.
M² steht für Halogen, Cyano, Nitro, NL⁹L¹⁰ OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆- Alkyl, C₃-C₆-Cycloalkyl, C₂-C₆-Alkenyl, C₃-C₆-Alkinyl, C₁-C₆-Alkoxy.
M³ steht für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C*.
G und E stehen unabhängig voneinander für Sauerstoff oder Schwefel.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
M⁴ steht für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹.
L² steht für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl.
M⁵ steht für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹.
M⁶ steht für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₃-C₆-Alkinyl, C₁-C₆-Alkoxy-C₁-C₃-alkyl, C₁-C₆-Halogenalkoxy-C₁-C₃-alkyl.
L⁹ und L¹⁰ stehen unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₄-Alkenyl, C₂-C₄-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆-Alkylcarbonyl, C₂-C₄-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₄-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl.

Es gilt hierbei, dass, wenn Y₂ für Methyl und Z für Phenyl-C₁-C₆-alkyl steht, der Phenylring in der ortho-Position nicht durch Alkoxy und nicht durch Arylalkoxy substituiert ist, ferner, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und schließlich, dass Verbindungen der Formeln (G1), (G2) und (G4) ausgenommen sind.

In den genannten bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₃-C₆-Cycloalkyl, C₁-C₆-Halogenalkyl, Cyano-C₁-C₆-alkyl, C₁-C₆-Alkoxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkyl-carbonylamino, CO₂H, SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert,

Besonders bevorzugte Substituenten bzw. Bereiche der in den neuen Verbindungen der Formel (I) aufgeführten Reste werden im Folgenden erläutert (Vorzugsbereich (7). 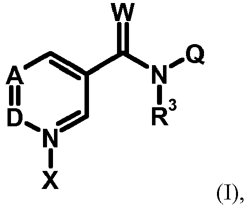
A steht für N oder CR¹.
D steht für N oder CR², wobei A und D nicht gleichzeitig für N stehen.
R¹ steht für einen Rest aus der Reihe Wasserstoff, Halogen, Cyano, Methyl, Halogenmethyl, Methoxy, Halogenmethoxy.
R² steht für einen Rest aus der Reihe Wasserstoff, Halogen, Cyano, Methyl, Halogenmethyl, Methoxy, Halogenmethoxy.
X steht für ein freies Elektronenpaar oder Sauerstoff.
W steht für Sauerstoff, Schwefel oder NR⁴.
R³ steht für einen Rest aus der Reihe Wasserstoff, C₁-C₆-Alkyl, C₃-C₆-Cycloalkyl, C₁-C₆-Halogenalkyl, C₃-C₆-Halogencycloalkyl, C₃-C₆-Cycloalkyl-C₁-C₆-alkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₃-C₆-Cycloalkylcarbonyl, C₁-C₆-Alkoxy-C₁-C₆-alkylcarbonyl, C₁-C₆-Alkoxycarbonyl, C₁-C₆-Halogenalkoxycarbonyl, Aryl, Hetaryl, Arylcarbonyl, Hetarylcarbonyl, wobei die Substituenten Aryl, Hetaryl, Arylcarbonyl und Hetarylcarbonyl gegebenenfalls einfach oder mehrfach, gleich oder unterschiedlich durch Halogen, Cyano, C₁-C₃-Alkyl, C₁-C₃-Halogenalkyl, C₁-C₃-Alkoxy, C₁-C₃-Halogenalkoxy, C₁-C₃-Alkoxy-C₁-C₂-alkyl substituiert sind.
R⁴ steht für einen Rest aus der Reihe Wasserstoff, Cyano, Nitro, C₁-C₃-Alkyl, C₃-C₆-Cycloalkyl, C₁-C₃-Halogenalkyl, C₃-C₆-Halogencycloalkyl, C₃-C₆-Cycloalkyl-C₁-C₃-alkyl, C₃-C₆-Halogencycloalkyl-C₁-C₃-alkyl, Cyano-C₁-C₃-alkyl, C₁-C₃-Alkoxy, C₁-C₃-Halogenalkoxy, C₁-C₃-Alkoxy-C₁-C₃-alkyl, C₁-C₃-Halogenalkoxy-C₁-C₃-alkyl, C₁-C₄-Alkylcarbonyl, C₁-C₄-Halogenalkylcarbonyl, C₁-C₃-Alkoxycarbonyl, C₁-C₃-Alkylcarbonyloxy.
Q steht für einen der Reste Q-1 bis Q-3, 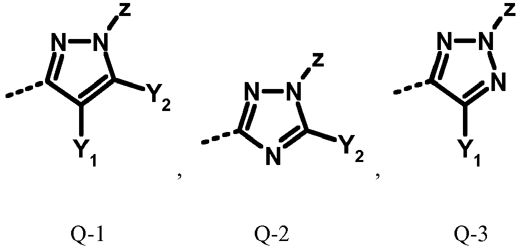 worin die gestrichelte Linie die Bindung zum Stickstoff in der Gruppe NR³ bedeutet.
Y₁ steht für einen Rest aus der Reihe Wasserstoff, Halogen, Cyano, C₁-C₆-Alkyl, C₃-C₆-Cycloalkyl, C₁-C₆-Halogenalkyl, C₁-C₆-Alkoxy, C₁-C₆-Halogenalkoxy, C₁-C₆-Alkoxy-C₁-C₃-alkyl, C₁-C₆-Halogenalkoxy-C₁-C₃-alkyl, C₁-C₆-Alkoxy-C₁-C₃-alkyloxy, Cyano-C₁-C₃-alkyl, C₃-C₆-Cycloalkyl-C₁-C₃-alkyl, C₁-C₃-Alkylthio, C₁-C₄-Alkylcarbonyl, C₁-C₄-Halogenalkylcarbonyl, C₁-C₃-Alkylsulfonyl.
Y₂ steht für einen Rest aus der Reihe Wasserstoff, Halogen, Cyano, C₁-C₆-Alkyl, C₃-C₆-Cycloalkyl, C₁-C₆-Halogenalkyl, C₁-C₆-Alkoxy, C₁-C₆-Halogenalkoxy, C₁-C₆-Alkoxy-C₁-C₄-alkyl, C₁-C₆-Halogenalkoxy-C₁-C₄-alkyl, C₁-C₆-Alkoxy-C₁-C₄-alkyloxy, Cyano-C₁-C₃-alkyl, C₃-C₆-Cycloalkyl-C₁-C₂-alkyl, SH, C₁-C₃-Alkylthio, C₁-C₄-Alkylcarbonyl, C₁-C₄-Halogenalkylcarbonyl, C₁-C₄-Alkoxycarbonyl, C₁-C₃-Alkylsulfonyl, C₁-C₃-Halogenalkylsulfonyl.
Z steht für einen Rest aus der Reihe Wasserstoff, L¹(=G)C*, L¹O(=G)C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₄-alkyl-Gruppe durch M⁶ substituiertes L⁵C(=E)L²N(=G)C-C₁-C₄-alkyl*, L⁵(=O)ₙSL²N(=G)C-C₁-C₄-alkyl*, L⁵OC(=E)L²N(=G)C-C₁-C₄-alkyl*, L¹L⁵NC(=E)L²N(=G)C-C₁-C₄-alkyl*, L¹(=O)ₙS-C₁-C₄-alkyl*, L³O(=O)ₙS-C₁-C₄-alkyl*, L³L⁴N(=O)ₙS-C₁-C₄-alkyl*,
mit der Maßgabe, dass, wenn Q für Q-2 steht, Z nicht gleichzeitig für Wasserstoff steht.
M² steht für Halogen, Cyano, NL⁹L¹⁰, OL⁹, SL⁹, L¹(=G)C*, L³O(=G)C*, L³L⁴N(=G)C*, Heterocyclyl, Aryl, Hetaryl, C₁-C₄-Alkyl, C₁-C₄-Halogenalkyl, C₁-C₄-Cyanoalkyl, C₃-C₆-Cycloalkyl, C₃-C₆-Halogencycloalkyl.
G und E stehen unabhängig voneinander für Sauerstoff oder Schwefel.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl, C₂-C₆-Alkenyl, C₃-C₆-Alkinyl.
M⁴ steht für Halogen, Cyano, C₁-C₃-Halogenalkyl, C₁-C₃-Alkoxy, C₁-C₃-Halogenalkoxy.
L² steht für Wasserstoff, Aryl, Hetaryl, gegebenenfalls einfach oder mehrfach, gleich oder verschieden durch M⁵ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁵ steht für Halogen, Cyano, C₁-C₆-Halogenalkyl, C₁-C₆-Alkoxy, C₁-C₆-Halogenalkoxy, C₁-C₄-Alkoxycarbonyl.
M⁶ steht für Halogen, Cyano, NL⁹L¹⁰, OL⁹, Aryl, Hetaryl, C₁-C₃-Halogenalkyl, C₃-C₄-Cycloalkyl, C₃-C₄-Halogencycloalkyl, C₃-C₄-Cycloalkyl-C₁-C₃-alkyl, C₃-C₄-Halogencycloalkyl-C₁-C₃-alkyl.
L⁹ und L¹⁰ stehen unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl, Cyano-C₁-C₆-alkyl, C₁-C₆-Alkoxy-C₁-C₄-alkyl, C₁-C₆-Halogenalkoxy-C₁-C₄-alkyl, C₁-C₆-Alkylcarbonyl, C₁-C₆-Halogenalkylcarbonyl, C₃-C₆-Cycloalkylcarbonyl, C₃-C₆-Cycloalkyl-C₁-C₄-alkylcarbonyl, C₃-C₆-Halogencycloalkylcarbonyl, C₁-C₆-Alkoxycarbonyl, C₁-C₆- Alkoxy-C₁-C₄-alkylcarbonyl, C₁-C₆-Halogenalkoxycarbonyl, C₃-C₆-Cycloalkoxycarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl.

Es gilt hierbei, dass, wenn Y₂ für Methyl und Z für Phenyl-C₁-C₆-alkyl steht, der Phenylring in der ortho-Position nicht durch Alkoxy und nicht durch Arylalkoxy substituiert ist, ferner, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und schließlich, dass Verbindungen der Formeln (G1) und (G2) ausgenommen sind.

In den genannten besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₆-Alkyl, C₃-C₆-Cycloalkyl, C₁-C₆-Halogenalkyl, Cyano-C₁-C₄-alkyl, C₁-C₆-Alkoxy, C₁-C₆- Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₁-C₄-Alkylcarbonylamino, C₁-C₄-Halogenalkylcarbonylamino, C₁-C₆-Cycloalkylcarbonylamino, C₁-C₄-Alkylcarbonyl, C₁-C₄-Halogenalkylcarbonyl, C₃-C₆-Cycloalkylcarbonyl, C₃-C₆-Cycloalkyl-C₁-C₂-alkylcarbonyl, C₃-C₆-Halogencycloalkylcarbonyl, C₁-C₆-Alkoxycarbonyl, C₁-C₆-Alkoxy-C₁-C₂-alkylcarbonyl, C₁-C₆-Halogenalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₄-Halogenalkylcarbonyloxy, C₁-C₄-Alkoxycarbonyloxy, C₁-C₄-Halogenalkoxycarbonyloxy, Aryl, Hetaryl, Aryloxy, Hetaryloxy substituiert.

Ganz besonders bevorzugte Substituenten bzw. Bereiche der in den neuen Verbindungen der Formel (I) aufgeführten Reste werden im Folgenden erläutert (Vorzugsbereich (8). 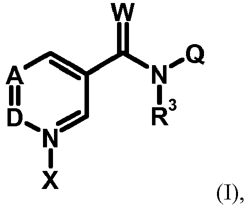
A steht für CR¹.
D steht für CR².
R¹ steht für Wasserstoff oder Fluor.
R² steht für Wasserstoff.
X steht für ein freies Elektronenpaar oder Sauerstoff.
W steht für Sauerstoff oder Schwefel.
R³ steht für einen Rest aus der Reihe Wasserstoff, C₁-C₄-Alkyl, C₃-C₄-Cycloalkyl, C₁-C₄-Halogenalkyl, C₃-C₄-Cycloalkyl-C₁-C₂-alkyl, C₁-C₃-Alkoxy-C₁-C₂-alkyl, C₁-C₄-Alkylcarbonyl, C₁-C₄-Halogenalkylcarbonyl, C₃-C₄-Cycloalkylcarbonyl, C₁-C₃-Alkoxycarbonyl, Aryl, Hetaryl, Arylcarbonyl, Hetarylcarbonyl, Aryl-C₁-C₃-alkyl, Hetaryl-C₁-C₃-alkyl, wobei die Substituenten Aryl, Hetaryl, Aryl-C₁-C₃-alkyl, Hetaryl-C₁-C₃-alkyl, Arylcarbonyl und Hetarylcarbonyl gegebenenfalls einfach oder mehrfach durch Halogen substituiert sind.
Q steht für Q-1, 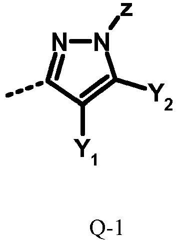 worin die gestrichelte Linie die Bindung zum Stickstoff in der Gruppe NR³ bedeutet.
Y₁ steht für einen Rest aus der Reihe Wasserstoff, Halogen, Cyano, C₁-C₄-Alkyl, C₁-C₄-Halogenalkyl, C₁-C₃-Alkoxy, C₁-C₃-Halogenalkoxy.
Y₂ steht für einen Rest aus der Reihe Wasserstoff, Halogen, Cyano, C₁-C₄-Alkyl, C₃-C₅-Cycloalkyl, C₁-C₄-Halogenalkyl, C₁-C₄-Alkoxy, C₁-C₄-Halogenalkoxy, C₁-C₃-Alkoxy-C₁-C₄-alkyl, C₁-C₃-Halogenalkoxy-C₁-C₄-alkyl, C₁-C₃-Alkoxy-C₁-C₄-alkyloxy, C₁-C₄-Alkoxycarbonyl.
Z steht für einen Rest aus der Reihe Wasserstoff, L¹(=G)C*, L¹O(=G)C*, L³L⁴N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₆-Alkyl, C₃-C₆-Cycloalkyl, C₂-C₆-Alkenyl, C₃-C₆-Alkinyl, für gegebenenfalls einfach oder mehrfach, gleich oder verschieden in der -C₁-C₄-alkyl-Gruppe durch M⁶ substituiertes L⁵(=O)ₙSL²N(=G)C-C₁-C₄-alkyl*, L¹(=O)ₙS-C₁-C₄-alkyl*.
M² steht für Halogen, Cyano, NL⁹L¹⁰, OL⁹, SL⁹, L¹(=G)C*, L³O(=G)C*, L³L⁴N(=G)C*, Heterocyclyl, Aryl, Hetaryl, C₁-C₄-Alkyl, C₁-C₄-Halogenalkyl, C₁-C₄-Cyanoalkyl, C₃-C₄-Cycloalkyl, C₃-C₄-Halogencycloalkyl.
G steht für Sauerstoff.
n steht für 1 oder 2.
L¹ und L⁵ stehen unabhängig voneinander für Heterocyclyl, Aryl, Hetaryl, gegebenenfalls einfach oder mehrfach, gleich oder verschieden durch M⁴ substituiertes C₁-C₄-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁴ steht für Halogen, Cyano, C₁-C₃-Halogenalkyl, C₁-C₃-Alkoxy, C₁-C₃-Halogenalkoxy.
L² steht für Wasserstoff, gegebenenfalls einfach oder mehrfach, gleich oder verschieden durch M⁵ substituiertes C₁-C₄-Alkyl, C₃-C₆-Cycloalkyl, C₃-C₆-Cycloalkyl-C₁-C₄-alkyl.
L³ und L⁴ stehen unabhängig voneinander für Wasserstoff, Heterocyclyl, Aryl, Hetaryl, gegebenenfalls einfach oder mehrfach, gleich oder verschieden durch M⁵ substituiertes C₁-C₄-Alkyl, Aryl-C₁-C₄-alkyl, Hetaryl-C₁-C₄-alkyl, C₃-C₆-Cycloalkyl, C₃-C₆-Cycloalkyl-C₁-C₄-alkyl.
M⁵ steht für Halogen, Cyano, C₁-C₃-Halogenalkyl, C₁-C₃-Alkoxy, C₁-C₄-Alkoxycarbonyl.
M⁶ steht für Halogen, Cyano, C₁-C₃-Halogenalkyl.
L⁹ und L¹⁰ stehen unabhängig voneinander für Wasserstoff, C₁-C₄-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl, Cyano-C₁-C₄-alkyl, C₁-C₄-Alkoxy-C₁-C₄-alkyl, C₁-C₄-Halogenalkoxy-C₁-C₄-alkyl, C₁-C₄-Alkylcarbonyl, C₁-C₄-Halogenalkylcarbonyl, C₃-C₆-Cycloalkylcarbonyl, C₃-C₆-Cycloalkyl-C₁-C₄-alkylcarbonyl, C₃-C₆-Halogencycloalkylcarbonyl, C₁-C₄-Alkoxycarbonyl, C₁-C₄-Alkoxy-C₁-C₄-alkylcarbonyl, C₁-C₄-Halogenalkoxycarbonyl, C₁-C₄-Alkylaminocarbonyl, Di-(C₁-C₄)-alkylaminocarbonyl, C₃-C₆-Cycloalkylaminocarbonyl, C₃-C₆-Cycloalkyl-C₁-C₄-alkylaminocarbonyl, Aryl, Hetaryl, Heterocyclyl, Aryl-C₁-C₃-alkyl, Hetaryl-C₁-C₃-alkyl.

Es gilt hierbei, dass, wenn Y₂ für Methyl und Z für Phenyl-C₁-C₆-alkyl steht, der Phenylring in der ortho-Position nicht durch Alkoxy und nicht durch Arylalkoxy substituiert ist, ferner, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und schließlich, dass Verbindungen der Formeln (G1) ausgenommen sind.

In den genannten ganz besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₄-Alkyl, C₃-C₆-Cycloalkyl, C₁-C₄-Halogenalkyl, C₁-C₄-Alkoxy, C₁-C₄-Halogenalkoxy, C₃-C₆-Halogencycloalkyl, C₁-C₄-Alkoxy-C₁-C₄-alkyl, C₁-C₄-Halogenalkoxy-C₁-C₄-alkyl, C₁-C₄-Alkoxycarbonyl, C₁-C₄-Halogenalkoxycarbonyl, C₁-C₃-Alkylthio, C₁-C₃-Halogenalkylthio, C₁-C₃-Alkylsulfonyl, C₁-C₃-Halogenalkylsulfonyl, C₁-C₃-Alkylaminosulfonyl, C₁-C₃-Halogenalkylaminosulfonyl, Di-(C₁-C₃)-alkylaminosulfonyl, C₁-C₃-Alkylaminocarbonyl, Di-(C₁-C₃)-alkylaminocarbonyl, Aryl, Hetaryl, Aryloxy, Hetaryloxy substituiert.

Ausdrücklich ganz besonders bevorzugte Substituenten bzw. Bereiche der in den neuen Verbindungen der Formel (I) aufgeführten Reste werden im Folgenden erläutert (Vorzugsbereich (9). 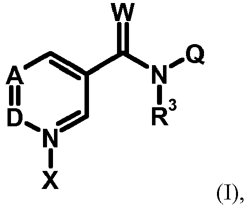
A steht für CR¹.
D steht für CR².
R¹ steht für Wasserstoff oder Fluor.
R² steht für Wasserstoff.
X steht für ein freies Elektronenpaar.
W steht für Sauerstoff.
R³ steht für einen Rest aus der Reihe Wasserstoff, C₁-C₄-Alkyl, C₁-C₄-Halogenalkyl, C₃-C₄-Cycloalkyl-C₁-C₂-alkyl, C₁-C₄-Alkylcarbonyl, Arylcarbonyl, Hetarylcarbonyl, wobei die Substituenten Arylcarbonyl und Hetarylcarbonyl gegebenenfalls einfach oder mehrfach durch Halogen substituiert sind.
Q steht für Q-1, 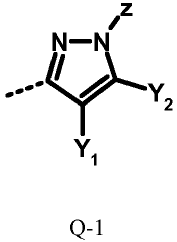 worin die gestrichelte Linie die Bindung zum Stickstoff in der Gruppe NR³ bedeutet.
Y₁ steht für einen Rest aus der Reihe Wasserstoff, Halogen, Cyano, Methyl, Ethyl, *n*-Propyl, *iso*Propyl, Trifluormethyl, Difluormethyl, Methoxy, Ethoxy, Difluormethoxy, Trifluormethoxy.
Y₂ steht für einen Rest aus der Reihe Wasserstoff, Halogen, Cyano, C₁-C₄-Alkyl, C₃-C₅-Cycloalkyl, C₁-C₃-Halogenalkyl, C₁-C₃-Alkoxy, C₁-C₃-Halogenalkoxy, C₁-C₃-Alkoxy-C₁-C₂-alkyl, C₁-C₃-Halogenalkoxy-C₁-C₂-alkyl, C₁-C₃-Alkoxy-C₁-C₂-alkyloxy, C₁-C₃-Alkoxycarbonyl.
Z steht für einen Rest aus der Reihe Wasserstoff, L¹(=G)C*, L¹O(=G)C*, L³L⁴N(=G)C*, L¹(=O)ₙS*, L³L⁴N(=O)ₙS*, Aryl, Hetaryl, C₃-C₆-Cycloalkyl, C₃-C₆-Halogencycloalkyl, C₂-C₆-Alkenyl, C₃-C₆-Alkinyl, C₂-C₆-Halogenalkenyl, C₃-C₆-Halogenalkinyl, für gegebenenfalls einfach oder mehrfach, gleich oder verschieden durch M² substituiertes C₁-C₆-Alkyl, für gegebenenfalls einfach oder mehrfach, gleich oder verschieden in der -C₁-C₄-alkyl-Gruppe durch M⁶ substituiertes L⁵(=O)ₙSL²N(=G)C-C₁-C₄-alkyl*, L¹(=O)ₙS-C₁-C₄-alkyl*.
M² steht für Halogen, Cyano, Heterocyclyl, Aryl, Hetaryl, C₃-C₄-Cycloalkyl, C₃-C₄-Halogencycloalkyl, NL⁹L¹⁰, OL⁹, SL⁹, L¹(=G)C*, L³O(=G)C*, L³L⁴N(=G)C*.
G steht für Sauerstoff.
n steht für 1 oder 2.
L¹ und L⁵ stehen unabhängig voneinander für Aryl, Hetaryl, C₁-C₄-Alkyl, C₁-C₄-Halogenalkyl, C₁-C₄-Cyanoalkyl, C₁-C₃-Alkoxy-C₁-C₄-alkyl, C₁-C₃-Halogenalkoxy-C₁-C₄-alkyl, Aryl-C₁-C₄-alkyl, Aryl-C₁-C₄-halogenalkyl, Aryl-C₁-C₄-cyanoalkyl, Hetaryl-C₁-C₄-alkyl, Hetaryl-C₁-C₄-halogenalkyl, Hetaryl-C₁-C₄-cyanoalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl.
L² steht für Wasserstoff oder C₁-C₄-Alkyl.
L³ und L⁴ stehen unabhängig voneinander für Wasserstoff, Aryl, Hetaryl, C₁-C₄-Alkyl, C₁-C₄-Halogenalkyl, C₁-C₄-Cyanoalkyl, C₁-C₃-Alkoxy-C₁-C₄-alkyl, Aryl-C₁-C₄-alkyl, Aryl-C₁-C₄-halogenalkyl, Aryl-C₁-C₄-cyanoalkyl, Hetaryl-C₁-C₄-alkyl, Hetaryl-C₁-C₄-halogenalkyl, Hetaryl-C₁-C₄-cyanoalkyl, C₃-C₆-Cycloalkyl, C₃-C₆-Halogencycloalkyl, C₃-C₆-Cycloalkyl-C₁-C₄-alkyl, C₃-C₆-Halogencycloalkyl-C₁-C₄-alkyl.
M⁶ steht für Halogen, Cyano, C₁-C₃-Halogenalkyl.
L⁹ und L¹⁰ stehen unabhängig voneinander für Wasserstoff, C₁-C₄-Alkyl, C₁-C₄-Halogenalkyl, C₃-C₆-Cycloalkyl, C₃-C₆-Cycloalkyl-C₁-C₄-alkyl, Cyano-C₁-C₄-alkyl, C₁-C₄-Alkylcarbonyl, C₁-C₄-Halogenalkylcarbonyl, C₃-C₆-Cycloalkyl-C₁-C₄-alkylcarbonyl, C₁-C₄-Alkoxycarbonyl, C₁-C₄-Alkylaminocarbonyl, Di-(C₁-C₄)-alkylaminocarbonyl, C₃-C₆-Cycloalkylaminocarbonyl, C₃-C₆-Cycloalkyl-C₁-C₄-alkylaminocarbonyl, Aryl, Hetaryl, Aryl-C₁-C₃-alkyl, Hetaryl-C₁-C₃-alkyl.

Es gilt hierbei, dass, wenn Y₂ für Methyl und Z für Phenyl-C₁-C₆-alkyl steht, der Phenylring in der ortho-Position nicht durch Alkoxy und nicht durch Arylalkoxy substituiert ist, ferner, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und schließlich, dass Verbindungen der Formeln (G1) ausgenommen sind.

In den genannten ausdrücklich ganz besonders bevorzugten Substituenten bzw. Bereichen sind, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, C₁-C₃-Alkyl, C₁-C₃-Halogenalkyl, C₁-C₂-Alkoxy, C₁-C₂-Halogenalkoxy, C₁-C₃-Alkoxycarbonyl, C₁-C₃-Halogenalkoxycarbonyl, C₁-C₂-Alkylthio, C₁-C₂-Halogenalkylthio, C₁-C₂-Alkylsulfonyl, C₁-C₂-Halogenalkylsulfonyl, C₁-C₂-Alkylaminosulfonyl, C₁-C₃-Alkylaminocarbonyl, Aryl, Hetaryl, Aryloxy, Hetaryloxy substituiert.

Eine bevorzugte Ausführungsform der Erfindung betrifft Verbindungen der Formel (I), in denen die Substituenten bzw. Bereiche der aufgeführten Reste die folgenden Bedeutungen haben. Ihre Kombination bildet den Vorzugsbereich (10). 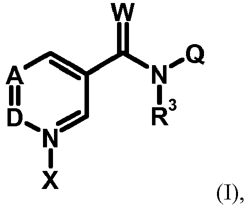
A steht für N oder CR¹.
D steht für N oder CR², wobei A und D nicht gleichzeitig für N stehen.
R¹ steht für Wasserstoff, Chlor, Brom oder Fluor.
R² steht für Wasserstoff, Methyl, Methoxy oder Chlor.
X steht für ein freies Elektronenpaar oder Sauerstoff.
W steht für Sauerstoff oder Schwefel.
R³ steht für einen Rest aus der Reihe Wasserstoff, C₁-C₃-Alkyl, C₁-C₃-Halogenalkyl, C₃-C₄-Alkinyl, Cyano-C₁-C₂-alkyl, Arylcarbonyl und Hetaryl-C₁-C₂-alkyl, wobei die Substituenten Arylcarbonyl und Hetaryl-C₁-C₂-alkyl gegebenenfalls einfach oder mehrfach, gleich oder unterschiedlich durch Halogen substituiert sind.
Q steht für Q-1, 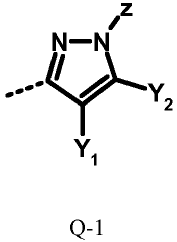 worin die gestrichelte Linie die Bindung zum Stickstoff in der Gruppe NR³ bedeutet.
Y₁ steht für einen Rest aus der Reihe Wasserstoff, Halogen, C₁-C₃-Alkyl, Cyano und C₁-C₂-Alkoxycarbonyl,
Y₂ steht für einen Rest aus der Reihe Wasserstoff, C₁-C₃-Alkyl, C₃-C₅-Cycloalkyl, C₁-C₂-Halogenalkyl, Halogen, Cyano, C₁-C₂-Halogenalkoxy, C₁-C₂-Alkoxycarbonyl, C₁-C₂-Alkylthio und C₁-C₂-Alkylsulfinyl, oder
Y₁ und Y₂ bilden zusammen mit den Kohlenstoffatomen, an die sie gebunden sind, einen Phenylring oder einen 6-gliedrigen heteroaromatischen Ring aus, welche gegebenenfalls einfach oder mehrfach durch Methyl substituiert sind.
Z steht für einen Rest aus der Reihe Wasserstoff, C₁-C₆-Alkyl, C₁-C₄-Halogenalkyl, C₃-C₇-Cycloalkyl, C₂-C₆-Alkenyl, C₂-C₄-Halogenalkenyl, C₃-C₅-Alkinyl, Cyano-C₁-C₄-alkyl, C₃-C₅-Cycloalkyl-C₁-C₃-alkyl, C₃-C₅-Halogencycloalkyl-C₁-C₂-alkyl, Aryl, Hetaryl, Aryl-C₁-C₂-alkyl, Hetaryl-C₁-C₂-alkyl, Hetaryl-C₁-C₃-halogenalkyl, Heterocyclyl-C₁-C₂-alkyl, C₁-C₆-Alkylaminocarbonyl, C₃-C₆-Cycloalkylaminocarbonyl, C₁-C₃-Halogenalkylaminocarbonyl, C₃-C₅-Cycloalkyl-C₁-C₂-alkylaminocarbonyl, C₁-C₂-Alkoxy-C₁-C₄-alkylaminocarbonyl, Di-(C₁-C₃-Alkoxy)-C₁-C₄-alkylamino-carbonyl, C₁-C₂-Halogenalkoxy-C₁-C₃-alkylaminocarbonyl, Di-(C₁-C₃)-alkylaminocarbonyl, Di-(C₁-C₂-Alkoxy-C₁-C₄-alkyl)-aminocarbonyl, Aryl-C₁-C₄-alkylaminocarbonyl, Hetaryl-C₁-C₄-alkylamino-carbonyl, Heterocyclyl-C₁-C₃-alkylaminocarbonyl, Aryloxy-C₁-C₃-alkylaminocarbonyl, C₁-C₂-Alkylsulfonyl-(C₁-C₂-alkylamino)carbonyl, Arylsulfonyl-(C₁-C₂-alkylamino)carbonyl, C₁-C₄-Alkylaminosulfonyl, Di-(C₁-C₂)-alkylaminosulfonyl, C₁-C₃-Alkylsulfonyl, Hetarylsulfonyl, Heterocyclylsulfonyl, C₁-C₅-Alkoxycarbonyl, C₁-C₃-Halogenalkoxycarbonyl, Aryloxycarbonyl, C₁-C₅-Alkylcarbonyl, C₃-C₅-Cycloalkylcarbonyl, Arylcarbonyl, Hetarylcarbonyl, Heterocyclylcarbonyl, C₁-C₂-Alkoxy-C₁-C₃-alkyl, C₁-C₃-Alkylthio-C₁-C₃-alkyl, C₁-C₃-Alkylsulfinyl-C₁-C₃-alkyl, C₁-C₃-Alkylsulfonyl-C₁-C₃-alkyl, Hetarylthio-C₁-C₂-alkyl, Hetarylsulfinyl-C₁-C₂-alkyl, Hetarylsulfonyl-C₁-C₂-alkyl, C₁-C₂- Alkylthio-C₁-C₃-alkylcarbonyl, C₁-C₂- Alkylsulfonyl-C₁-C₃-alkylcarbonyl, C₁-C₂-Alkylthio-C₁-C₃-alkylaminocarbonyl, C₁-C₂-Alkylsulfinyl-C₁-C₃-alkylaminocarbonyl, C₁-C₂-Alkylsulfonyl-C₁-C₃-alkylaminocarbonyl, C₁-C₂-Alkoxy-C₁-C₂-alkylcarbonyl, C₃-C₅-Halogencycloalkylcarbonyl-C₁-C₂-alkyl, C₁-C₃-Alkoxycarbonyl-C₁-C₄-alkyl, C₁-C₂-Alkoxycarbonyloxy-C₁-C₃-alkyl, C₁-C₃-Alkylaminocarbonyl-C₁-C₂-alkyl, Hydroxycarbonyl-C₁-C₂-alkyl, C₁-C₂-Alkoxycarbonyl-C₁-C₄-alkylaminocarbonyl-C₁-C₂-alkyl, C₁-C₃-Alkoxycarbonyl-di-(C₁-C₂)-alkylaminocarbonyl-C₁-C₂-alkyl, Hetaryl-C₂-C₃-halogenalkenyl und Aryl-C₁-C₂-alkylcarbonyl-C₁-C₂-alkyl,
wobei die Substituenten Aryl, Hetaryl, Aryl-C₁-C₂-alkyl, Hetaryl-C₁-C₂-alkyl, Hetaryl-C₁-C₃-halogenalkyl, Aryl-C₁-C₄-alkylaminocarbonyl, Hetaryl-C₁-C₄-alkylaminocarbonyl, Aryloxy-C₁-C₃-alkylaminocarbonyl, Arylsulfonyl-(C₁-C₂-alkylamino)carbonyl, Hetarylsulfonyl, Aryloxycarbonyl, Arylcarbonyl, Hetarylcarbonyl, Hetarylthio-C₁-C₂-alkyl, Hetarylsulfinyl-C₁-C₂-alkyl, Hetarylsulfonyl-C₁-C₂-alkyl, Hetaryl-C₂-C₃-halogenalkenyl und Aryl-C₁-C₂-alkylcarbonyl-C₁-C₂-alkyl gegebenenfalls einfach oder mehrfach, gleich oder unterschiedlich durch Halogen, Cyano, C₁-C₅-Alkyl, C₁-C₂-Halogenalkyl, Di-(C₁-C₂)-alkylaminocarbonyl, C₁-C₃-Alkoxycarbonyl, Phenyl, C₁-C₂-Alkoxy, C₁-C₂-Halogenalkoxy, C₁-C₃-Halogenalkylthio oder COOH substituiert sind,
und wobei die Substituenten Heterocyclyl-C₁-C₂-alkyl, Heterocyclyl-C₁-C₃-alkylaminocarbonyl, Heterocyclylsulfonyl und Heterocyclylcarbonyl gegebenenfalls einfach oder mehrfach, gleich oder unterschiedlich durch C₁-C₃-Alkyl, C₃-C₅-Cycloalkyl oder C₁-C₂-Alkoxy substituiert sind,
und mit der Maßgabe, dass, wenn Y₂ für Methyl und Z für Aryl-C₁-C₂-alkyl steht, der Phenylring in der ortho-Position nicht durch Alkoxy und nicht durch Arylalkoxy substituiert ist, und mit der Maßgabe, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht und R³, Y₁ und Y₂ für Wasserstoff stehen, und mit der Maßgabe, dass Verbindungen der Formel (G1) ausgenommen sind.

In den bevorzugten Definitionen (Vorzugsbereich (6)) ist, sofern nichts anderes angegeben ist, Halogen ausgewählt aus der Reihe Fluor, Chlor, Brom und Iod, bevorzugt wiederum aus der Reihe Fluor, Chlor, Brom und Iod,

Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, Triazolyl, Oxazolyl, Isoxazolyl, Thiazolyl, Isothiazolyl, Oxadiazolyl, Thiadiazolyl, Tetrazolyl, Pyridyl, Pyrimidinyl, Pyridazinyl, Pyrazinyl, Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Indolizinyl, Imidazopyridinyl und Benzthiadiazol.

Heterocyclyl steht für einen gesättigten oder ungesättigten, nicht heteroaromatischen Rest, der mindestens einen 3- bis 7-gliedrigen heterocyclischen Ring (=carbocyclischer Ring, in dem mindestens ein C-Atom durch ein Heteroatom ersetzt ist, vorzugsweise durch ein Heteroatom aus der Gruppe N, O, S) enthält, dabei kann dieser unsubstituiert oder substituiert sein, wobei die Bindungsstelle an einem Ringatom lokalisiert ist. Heterocyclyl ist bevorzugt ausgewählt aus der Reihe Aziridinyl, Azirenyl, Oxiranyl, Oxirenyl, Thiiranyl, Thiirenyl, Azetidinyl, Dihydroazetyl, Oxetanyl, Oxetyl, Thietanyl, Thietyl, Dioxetanyl, Dithietanyl, Oxathietanyl, Pyrrolidinyl, Dihydropyrrolyl, Piperidinyl, Tetrahydropyridinyl, Dihydropyridinyl, Azepanyl, Tetrahydrofuranyl, Dihydrofuranyl, Tetrahydropyranyl, Dihydropyranyl, Pyranyl, Oxepanyl, Tetrahydrothiophenyl, Dihydrothiophenyl, Tetrahydrothiopyranyl, Dihydrothiopyranyl, Thiepanyl, Dioxolanyl, Dioxanyl, Dioxepanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl. Handelt es sich um einen teilweise oder vollständig gesättigten Stickstoff-haltigen Heterocyclus, so kann dieser sowohl über Kohlenstoff als auch über den Stickstoff mit dem Rest des Moleküls verknüpft sein. Wenn in obigen Definitionen in Ringen Schwefel vorkommt, dann kann der Schwefel gegebenenfalls auch als SO oder SO₂ vorliegen. Gegebenenfalls kann der Heterocyclus einfach oder mehrfach durch C=O oder C=S Gruppen unterbrochen sein.

In einer bevorzugten Ausführungsform gelten diese Definitionen auch für den Vorzugsbereich (10).

In den besonders bevorzugten Definitionen (Vorzugsbereich (7)) ist, sofern nichts anderes angegeben ist,
Halogen ausgewählt aus der Reihe Fluor, Chlor, Brom und Iod,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, Triazolyl, Oxazolyl, Isoxazolyl, Thiazolyl, Isothiazolyl, Oxadiazolyl, Thiadiazolyl, Tetrazolyl, Pyridyl, Pyrimidinyl, Pyridazinyl, Pyrazinyl, Imidazopyridinyl und Benzthiadiazol.

Heterocyclyl ist ausgewählt aus der Reihe Aziridinyl, Oxiranyl, Thiiranyl, Azetidinyl, Oxetanyl, Thietanyl, Pyrrolidinyl, Dihydropyrrolyl, Piperidinyl, Tetrahydropyridinyl, Dihydropyridinyl, Azepanyl, Tetrahydrofuranyl, Dihydrofuranyl, Tetrahydropyranyl, Dihydropyranyl, Pyranyl, Oxepanyl, Tetrahydrothiophenyl, Dihydrothiophenyl, Tetrahydrothiopyranyl, Dihydrothiopyranyl, Thiepanyl, Dioxolanyl, Dioxanyl, Dioxepanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (10).

In den ganz besonders bevorzugten Definitionen (Vorzugsbereich (8)) ist, sofern nichts anderes angegeben ist,
Halogen ausgewählt aus der Reihe Fluor, Chlor, Brom und Iod,
Aryl (auch als Teil einer größeren Einheit, wie beispielsweise Arylalkyl) ausgewählt aus der Reihe Phenyl, Naphthyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Pyrazolyl, Imidazolyl, Triazolyl, Thiazolyl, Isothiazolyl, Thiadiazolyl, Tetrazolyl, Pyridyl, Pyrimidinyl, Pyridazinyl, Pyrazinyl, Imidazopyridinyl und Benzthiadiazol.

Heterocyclyl ist ausgewählt aus der Reihe Aziridinyl, Oxiranyl, Thiiranyl, Azetidinyl, Oxetanyl, Thietanyl, Pyrrolidinyl, Piperidinyl, Tetrahydrofuranyl, Tetrahydropyranyl, Tetrahydrothiophenyl, Tetrahydrothiopyranyl, Dioxolanyl, Dioxanyl, Dithiolanyl, Dithianyl, Oxathiolanyl, Oxathianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (10).

In den ausdrücklich ganz besonders bevorzugten Definitionen (Vorzugsbereich (9)) ist, sofern nichts anderes angegeben ist, Halogen ausgewählt aus der Reihe Fluor, Chlor und Brom,
Aryl (auch als Teil einer größeren Einheit, wie beispielsweise Arylalkyl) gleich Phenyl,

Hetaryl (gleichbedeutend mit Heteroaryl, auch als Teil einer größeren Einheit, wie beispielsweise Hetarylalkyl) ausgewählt aus der Reihe Pyrazolyl, Imidazolyl, Triazolyl, Thiazolyl, Isothiazolyl, Thiadiazolyl, Pyridyl, Pyrimidinyl, Pyridazinyl, Pyrazinyl, Imidazopyridinyl und Benzthiadiazol.

Heterocyclyl ist ausgewählt aus der Reihe Tetrahydrofuranyl, Tetrahydropyranyl, Tetrahydrothiophenyl, Tetrahydrothiopyranyl, Dioxolanyl, Dioxanyl, Dithiolanyl, Dithianyl, Pyronyl, Triazolonyl, Triazolidindionyl, Tetrahydropyrimidinonyl, Thiadiazolopyrimidinonyl und 2-(N-Cyanoimino)thiazolidinyl.

In einer bevorzugten Ausführungsform gelten diese Definitionen auch für den Vorzugsbereich (10).

Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod.

Gesättigte oder ungesättigte Kohlenwasserstoffreste wie z.B. Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind neue Verbindungen der Formel (I), in welchen eine Kombination der vorstehend als bevorzugt aufgeführten Bedeutungen vorliegt (Vorzugsbereich (6)).

Erfindungsgemäß besonders bevorzugt sind neue Verbindungen der Formel (I), in welchen eine Kombination der vorstehend als besonders bevorzugt aufgeführten Bedeutungen vorliegt (Vorzugsbereich (7)).

Erfindungsgemäß ganz besonders bevorzugt sind neue Verbindungen der Formel (I), in welchen eine Kombination der vorstehend als ganz besonders bevorzugt aufgeführten Bedeutungen vorliegt (Vorzugsbereich (8)).

Erfindungsgemäß ausdrücklich ganz besonders bevorzugt sind neue Verbindungen der Formel (I), in welchen eine Kombination der vorstehend als ausdrücklich ganz besonders bevorzugt aufgeführten Bedeutungen vorliegt (Vorzugsbereich (9)).

Weitere bevorzugte Ausführungsformen der Erfindung betreffen Verbindungen, wie sie durch die Vorzugsbereiche (1) bis (10) definiert sind, in welchen W für Sauerstoff steht.

Weitere bevorzugte Ausführungsformen der Erfindung betreffen Verbindungen, wie sie durch die Vorzugsbereiche (1) bis (10) definiert sind, in welchen W für Schwefel steht.

Weitere bevorzugte Ausführungsformen der Erfindung betreffen Verbindungen, wie sie durch die Vorzugsbereiche (1) bis (10) definiert sind, in welchen W für NR⁴ steht.

Weitere bevorzugte Ausführungsformen der Erfindung betreffen Verbindungen, wie sie durch die Vorzugsbereiche (1) bis (10) definiert sind, in welchen X für ein Elektronenpaar steht.

Weitere bevorzugte Ausführungsformen der Erfindung betreffen Verbindungen, wie sie durch die Vorzugsbereiche (1) bis (10) definiert sind, in welchen W für Sauerstoff steht, X für ein Elektronenpaar steht und Y₁ und Y₂ gemeinsam mit den Atomen, an die sie gebunden sind, keinen Ring bilden.

Weitere bevorzugte Ausführungsformen der Erfindung betreffen Verbindungen, wie sie durch die Vorzugsbereiche (1) bis (10) definiert sind, in welchen W für Sauerstoff steht, X für ein Elektronenpaar steht, Y₁ und Y₂ gemeinsam mit den Atomen, an die sie gebunden sind, keinen Ring bilden und A und D jeweils für C-H oder C-Halogen stehen.

Weitere bevorzugte Ausführungsformen der Erfindung betreffen Verbindungen, wie sie durch die Vorzugsbereiche (1) bis (10) definiert sind, in welchen W für Sauerstoff steht, X für ein Elektronenpaar steht, Y₁ und Y₂ gemeinsam mit den Atomen, an die sie gebunden sind, einen Ring (bevorzugt Phenyl oder gegebenenfalls durch Methyl substituiertes Pyridyl) bilden und A und D jeweils für C-H oder C-Halogen stehen.

Weitere bevorzugte Ausführungsformen der Erfindung betreffen Verbindungen, wie sie durch die Vorzugsbereiche (1) bis (10) definiert sind, in welchen W für Sauerstoff steht, X für ein Elektronenpaar steht, Y₁ und Y₂ gemeinsam mit den Atomen, an die sie gebunden sind, keinen Ring bilden und A und D jeweils für C-H stehen.

Weitere bevorzugte Ausführungsformen der Erfindung betreffen Verbindungen, wie sie durch die Vorzugsbereiche (1) bis (10) definiert sind, in welchen W für Sauerstoff steht, X für ein Elektronenpaar steht, Y₁ und Y₂ gemeinsam mit den Atomen, an die sie gebunden sind, keinen Ring bilden und A und D jeweils für C-Halogen stehen.

Die neuen Verbindungen der Formel (I) können nach den im Folgenden beschriebenen Verfahren hergestellt werden.

Die Erfindung betrifft auch Verfahren zur Herstellung der neuen Verbindungen der Formel (I), in denen W für O steht, 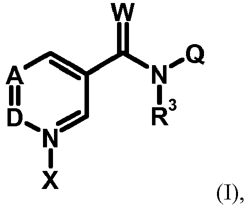 wobei A, D, X, R³ und Q die oben beschriebenen Bedeutungen haben,
durch Umsetzung von Aminen der Formeln (II-1) bis (II-4) 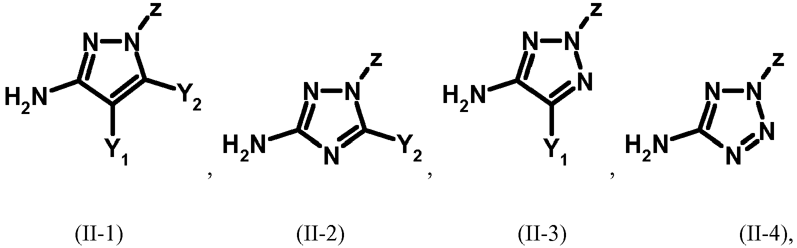 worin Z, Y₁ und Y₂ die oben beschriebene Beudeutung haben,
mit Carbonsäuren oder Carbonsäurederivaten der Formel (III), 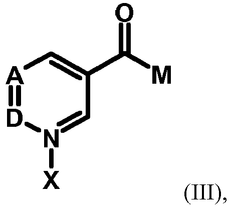 worin
- M: für Halogen, Hydroxy, Alkoxy, Alkylsulfanyl, Acyloxy, Sulfonyloxy, Azid oder für N-Heterocyclyl (z.B. Imidazolyl) steht,
- A: die oben angegebenen Bedeutungen hat,
- D: die oben angegebenen Bedeutungen hat und
- X: die oben angegebenen Bedeutungen hat.

Das Verfahren ist in Syntheseschema 1 dargestellt.

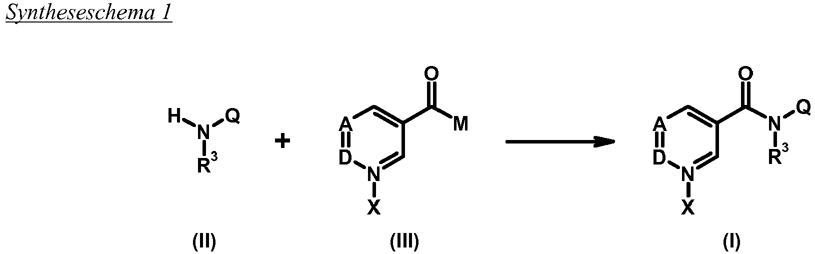

Hierbei können Verbindungen der Formel (III) bereits aktiviert sein oder *in situ* aktiviert werden. Beispielsweise können Verbindungen der Formel (III) als Säurehalogenide (z.B. M = Chlor) eingesetzt werden. Die Umsetzung wird dann vorteilhaft in Gegenwart einer Base, wie z.B. Triethylamin, Pyridin oder Natriumhydroxid, oder eines anderen Reagenz, wie z.B. Silbercyanid, durchgeführt. Es können aber auch Carbonsäuren (M = OH) in Gegenwart von Kupplungsreagenzien wie z.B. Dicyclohexylcarbodiimid und Additiven wie z.B. 1-Hydroxy-1H-benzotriazol eingesetzt werden (W. König, R. Geiger, Chem. Ber. 1970, 103, 788). Verwendbar sind ferner Kupplungsreagenzien wie 1-Ethyl-3-(3-dimethylaminopropyl)carbodiimid, 1,1'-Carbonyldiimidazol, O-(Benzotriazol-1-yl)-N,N,N',N'-tetramethyluroniumhexafluorphosphat, O-(7-Azabenzotriazol-1-yl)-N,N,N',N'-tetramethyluroniumhexafluorphosphat, Propanphosphonsäure-cyclo-anhydrid und ähnliche Verbindungen. Als Kupplungsreagenzien zur Durchführung des Herstellungsverfahrens sind grundsätzlich alle Verbindungen geeignet, die die Bildung einer Amidbindung ermöglichen (vgl. z.B. E. Valeur, M. Bradley, Chem. Soc. Rev. 2009, 38, 606; S.-Y. Han, Y.-A. Kim, Tetrahedron 2004, 60, 2447). Verwendbar sind ferner Additive wie 1-Hydroxy-7-azabenzotriazol, 4-(Dimethylamino)-pyridin, Diisopropylethylamin und ähnliche Verbindungen. Weiterhin können auch symmetrische oder gemischte Anhydride zur Darstellung von Verbindungen der Formel (I) verwendet werden (G. W. Anderson, J. E. Zimmerman, F. M. Calahan, J. Am. Chem. Soc. 1967, 89, 5012). Dabei können verschiedene Chlorameisensäureester zum Einsatz kommen, wie z.B. Chlorameisensäureisobutylester und Chlorameisensäure-*sec*-butylester. Ebenfalls können beispielsweise Isovalerylchlorid und Pivaloylchlorid verwendet werden.

Verbindungen der Formel (I), in denen W für O und X für ein freies Elektronenpaar steht und R³ verschieden von Wasserstoff ist, können ebenfalls nach Syntheseschema 2 aus Verbindungen der Formel (I), in denen R³ für Wasserstoff steht, durch Alkylierung gewonnen werden. Dabei kommen geeignete Alkylierungsmittel zum Einsatz, wie z.B. Alkylhalogenide oder Acylhalogenide, in der Gegenwart von bestimmten Basen, wie z.B. Diisopropylethylamin, Triethylamin, Pyridin oder Caesiumcarbonat.

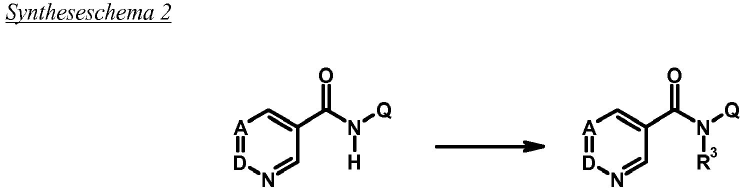

Verbindungen der Formel (I), in denen Z für O (Sauerstoffatom) und X für ein freies Elektronenpaar steht, können nach Syntheseschema 3 mit einem Schwefelungsreagenz, wie z.B. Diphosphorpentasulfid oder Lawessons Reagenz (vgl. z.B. C. P. Dell in Comprehensive Organic Functional Group Transformations, Vol. 5, Hrsg.: A. R. Katrizky, O. Meth-Cohn, C. W. Rees, Pergamon, Oxford, 1995, S. 565-628; M. Jesberger, T. P. Davis, L. Barner, Synthesis 2003, 13, 1929), zu Verbindungen der Formel (I), in denen Z für S (Schwefelatom) steht, umgesetzt werden.

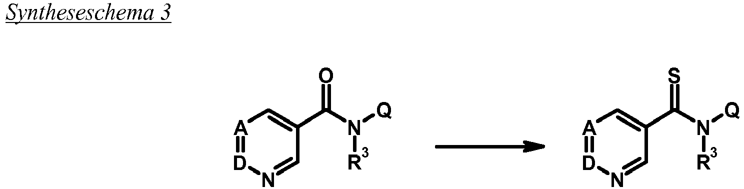

Verbindungen der Formel (I), in denen Z für S und X für ein freies Elektronenpaar steht, können nach Syntheseschema 4 zu Verbindungen der Formel (I), in denen Z für NR⁴ steht, umgesetzt werden durch Reaktion mit geeigneten Aminen (V. A. Glushkov, L. V. Anikina, Yu. B. Vikharev, E. V. Feshina, Yu. V. Shklyaev, Pharmaceutical Chemistry Journal 2005, 39, 533). Alternativ können Verbindungen der Formel (I), in denen Z für O und X für ein freies Elektronenpaar steht, nach Syntheseschema 5 aktiviert werden, z.B. über Chlorierung mit Thionylchlorid, Phosphoroxychlorid oder Phosphorpentachlorid, und dann mit geeigneten Aminen zu Verbindungen der Formel (I), in denen Z für NR⁴ steht, überführt werden.

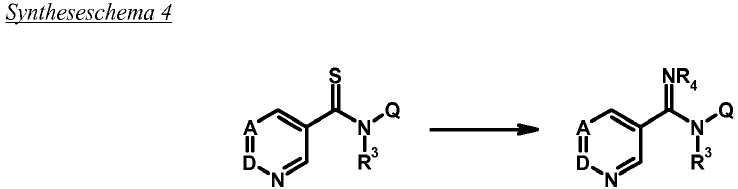

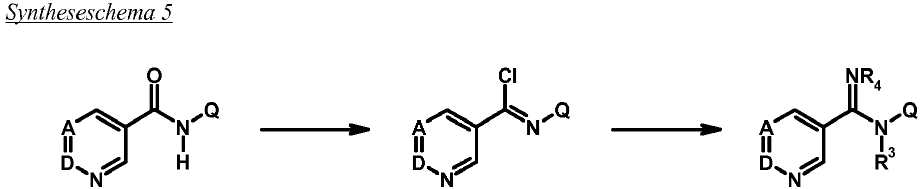

Die für die Herstellung der erfindungsgemäßen Verbindungen der Formel (I) benötigten Pyridin-3-carbonsäuren, Pyrimidin-5-carbonsäuren und Pyridazin-4-carbonsäuren der Formel (III) sind kommerziell erhältlich oder können anhand literaturbekannter Verfahren synthetisiert werden.

Gewisse Amine der Formel (II-1) 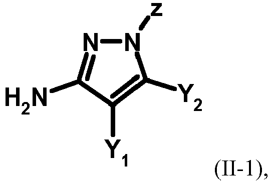 sind kommerziell erhältlich, literaturbekannt oder können anhand literaturbekannter Verfahren synthetisiert werden. Diese seien exemplarisch im Folgenden genannt.

| **Amine der Formel (II-1)** | **Bemerkung** | **Amine der Formel (II-1)** | **Bemerkung** |
|---|---|---|---|
| 1-(2,2-Difluorethyl)-5-methyl-1H-pyrazol-3-amin | kommerziell erhältlich | 1-(2,4-Difluorphenyl)-1H-pyrazol-3-amin | kommerziell erhältlich |
| 1,5-Dimethyl-1H-pyrazol-3-amin | kommerziell erhältlich | 1-(2,4-Dichlorbenzyl)-1H-pyrazol-3-amin | kommerziell erhältlich |
| 1-(Pyrimidin-2-ylmethyl)-1H-pyrazol-3-amin | kommerziell erhältlich | 1-(2,5-Difluorbenzyl)-1H-pyrazol-3-amin | kommerziell erhältlich |
| 1-(2,2,2-Trifluorethyl)-1H-pyrazol-3-amin | kommerziell erhältlich | 2-[(3-Amino-1H-pyrazol-1-yl)methyl]benzonitril | kommerziell erhältlich |
| 1-Isobutyl-5-methyl-1H-pyrazol-3-amin | kommerziell erhältlich | 1-[2-(Trifluormethyl)benzyl]-1H-pyrazol-3-amin | kommerziell erhältlich |
| 1-(2,2-Difluorethyl)-1H-pyrazol-3-amin | kommerziell erhältlich | 1-(2-Fluorbenzyl)-1H-pyrazol-3-amin | kommerziell erhältlich |
| N,1,5-Trimethyl-1H-pyrazol-3-amin | kommerziell erhältlich | 1-Benzyl-1H-pyrazol-3-amin | kommerziell erhältlich |
| 5-Methyl-1H-pyrazol-3-amin | kommerziell erhältlich | 1-(2,4-Difluorphenyl)-5-ethyl-1H-pyrazol-3-amin | kommerziell erhältlich |
| tert-Butyl-3-amino-5-methyl-1H-pyrazol-1-carboxylat | kommerziell erhältlich | 5-Methyl-1-[3-(trifluormethyl)benzyl]-1H-pyrazol-3-amin | kommerziell erhältlich |
| 5-Methyl-1-(pyrimidin-2-yl)-1H-pyrazol-3-amin | kommerziell erhältlich | 1-(3,4-Dichlorphenyl)-5-methyl-1H-pyrazol-3-amin | kommerziell erhältlich |
| Ethyl-(3-amino-5-methyl-1H-pyrazol-1-yl)acetat | kommerziell erhältlich | 5-(Difluormethoxy)-1-methyl-1H-pyrazol-3-amin | kommerziell erhältlich |
| 1-(Pyridin-2-ylmethyl)-1H-pyrazol-3-amin | kommerziell erhältlich | 1-(3,4-Dichlorphenyl)-5-ethyl-1H-pyrazol-3-amin | kommerziell erhältlich |
| 1-(3-Fluorpyridin-2-yl)-1H-pyrazol-3-amin | kommerziell erhältlich | 5-Ethyl-1-(3-methylphenyl)-1H-pyrazol-3-amin | kommerziell erhältlich |
| 1-Benzyl-5-ethyl-1H-pyrazol-3-amin | kommerziell erhältlich | 1-(3-Chlorphenyl)-5-ethyl-1H-pyrazol-3-amin | kommerziell erhältlich |
| 1-Ethyl-5-methyl-1H-pyrazol-3-amin | kommerziell erhältlich | 4-(3-Amino-5-ethyl-1H-pyrazol-1-yl)benzonitril | kommerziell erhältlich |
| 1-[3-Chlor-5-(trifluormethyl)pyridin-2-yl]-5-methyl-1H-pyrazol-3-amin | kommerziell erhältlich | 1-(4-Chlorphenyl)-5-ethyl-1H-pyrazol-3-amin | kommerziell erhältlich |
| 5-Methyl-1-[5-(trifluormethyl)pyridin-2-yl]-1H-pyrazol-3-amin | kommerziell erhältlich | 5-Ethyl-1-phenyl-1H-pyrazol-3-amin | kommerziell erhältlich |
| 1-Benzyl-5-methyl-1H-pyrazol-3-amin | kommerziell erhältlich | 5-Methyl-1-(3-methylphenyl)-1H-pyrazol-3-amin | kommerziell erhältlich |
| 1-(3-Chlorpyridin-2-yl)-1H-pyrazol-3-amin | kommerziell erhältlich | 1-(3-Chlorphenyl)-5-methyl-1H-pyrazol-3-amin | kommerziell erhältlich |
| 1-Isopropyl-5-methyl-1H-pyrazol-3-amin | kommerziell erhältlich | 1-(4-Chlorphenyl)-5-methyl-1H-pyrazol-3-amin | kommerziell erhältlich |
| 5-Methyl-1-(3-methylbenzyl)-1H-pyrazol-3-amin | kommerziell erhältlich | 5-Methyl-1-phenyl-1H-pyrazol-3-amin | kommerziell erhältlich |
| 1-(Pyridin-2-yl)-1H-pyrazol-3-amin | kommerziell erhältlich | (3-Amino-5-methyl-1H-pyrazol-1-yl)essigsäure | kommerziell erhältlich |
| 1-(3-Fluorbenzyl)-5-methyl-1H-pyrazol-3-amin | kommerziell erhältlich | 4-Chlor-1-(2-chlor-6-fluorbenzyl)-1H-pyrazol-3-amin | kommerziell erhältlich |
| 1-(4-Fluorbenzyl)-5-methyl-1H-pyrazol-3-amin | kommerziell erhältlich | 2-(3-Amino-1H-pyrazol-1-yl)benzonitril | kommerziell erhältlich |
| 1-[3-Chlor-5-(trifluormethyl)pyridin-2-yl]-1H-pyrazol-3-amin | kommerziell erhältlich | 1-(3-Chlorphenyl)-1H-pyrazol-3-amin | kommerziell erhältlich |
| 1-(2-Fluorbenzyl)-5-methyl-1H-pyrazol-3-amin | kommerziell erhältlich | 1-(4-Methoxyphenyl)-1H-pyrazol-3-amin | kommerziell erhältlich |
| 5-Methoxy-1-methyl-1H-pyrazol-3-amin | kommerziell erhältlich | 1-Phenyl-1H-pyrazol-3-amin | kommerziell erhältlich |
| Methyl-4-(3-amino-5-methyl-1H-pyrazol-1-yl)butanoat | kommerziell erhältlich | Methyl-3-amino-1-methyl-1H-pyrazol-5-carboxylat | kommerziell erhältlich |
| 1-(2,6-Difluorbenzyl)-1H-pyrazol-3-amin | kommerziell erhältlich | 1-(4,6-Dimethylpyrimidin-2-yl)-5-methyl-1H-pyrazol-3-amin | kommerziell erhältlich |
| Ethyl-(3-amino-1H-pyrazol-1-yl)acetat | kommerziell erhältlich | 2-(3-Amino-5-methyl-1H-pyrazol-1-yl)-N-methylacetamid | kommerziell erhältlich |
| 1-(3-Fluorbenzyl)-1H-pyrazol-3-amin | kommerziell erhältlich | 5-Methyl-1-(2,2,2-trifluorethyl)-1H-pyrazol-3-amin | kommerziell erhältlich |
| 1-[3-(Trifluormethyl)benzyl]-1H-pyrazol-3-amin | kommerziell erhältlich | 4-Methyl-1-(pyrimidin-2-ylmethyl)-1H-pyrazol-3-amin | kommerziell erhältlich |
| 1-(3-Methylbenzyl)-1H-pyrazol-3-amin | kommerziell erhältlich | 4-Chlor-1-(pyrimidin-2-ylmethyl)-1H-pyrazol-3-amin | kommerziell erhältlich |
| 1-(3-Methoxybenzyl)-1H-pyrazol-3-amin | kommerziell erhältlich | 5-Methyl-1-(pyridin-2-ylmethyl)-1H-pyrazol-3-amin | WO 2009/46802 |
| 1-(2,4-Difluorbenzyl)-1H-pyrazol-3-amin | kommerziell erhältlich | (3-Amino-5-methyl-1H-pyrazol-1-yl)acetonitril | keine Referenz |
| 1-(4-tert-Butylbenzyl)-1H-pyrazol-3-amin | kommerziell erhältlich | Methyl-3-[(3-amino-1H-pyrazol-1-yl)methyl]benzoat | US 2008/21032 |
| 1-(4-Methoxybenzyl)-1H-pyrazol-3-amin | kommerziell erhältlich | Ethyl-4-[(3-amino-1H-pyrazol-1-yl)methyl]benzoat | WO 2011095997 |
| 1-(2,5-Dimethylbenzyl)-1H-pyrazol-3-amin | kommerziell erhältlich | 1-Methyl-1H-pyrazolo[3,4-b]pyridin-3-amin | kommerziell erhältlich |
| 5-Methyl-1-(tetrahydrofuran-2-ylmethyl)-1H-pyrazol-3-amin | kommerziell erhältlich | 5-Methyl-1-[(4-methyl-1H-pyrazol-1-yl)methyl]-1H-pyrazol-3-amin | kommerziell erhältlich |
| 1-(Pyrimidin-2-ylmethyl)-1H-indazol-3-amin | kommerziell erhältlich | 1-tert-Butyl-5-methyl-1H-pyrazol-3-amin | kommerziell erhältlich |
| 3-(3-Amino-5-methyl-1H-pyrazol-1-yl)benzonitril | kommerziell erhältlich | 5-Methyl-1-(1H-pyrazol-1-ylmethyl)-1H-pyrazol-3-amin | kommerziell erhältlich |

Neue 1H-Pyrazol-3-amine der Formel (II-1) lassen sich allgemein gemäß Syntheseschema 6 herstellen. Die Erfindung betrifft demnach auch neue Verbindungen der Formel (II-1), die als Intermediate zur Herstellung von Verbindungen der Formel (I) dienen.

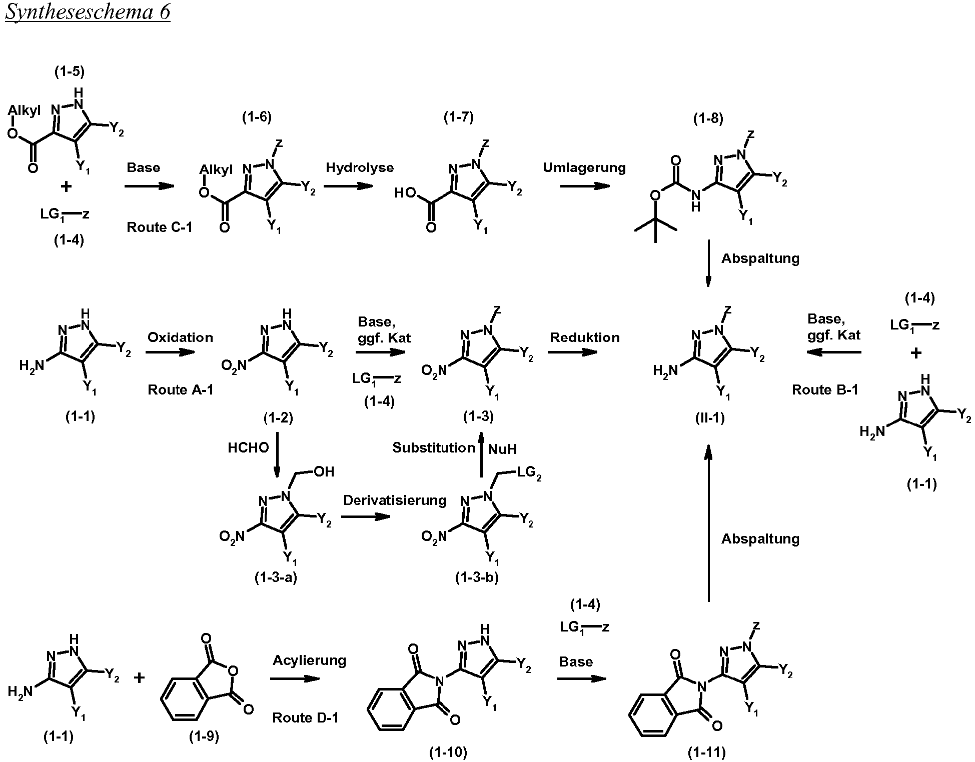

Gemäß Route A-1 lassen sich 1H-Pyrazol-3-amine der Formel (1-1) in die korrespondierenden 3-Nitro-1H-pyrazole der Formel (1-2) überführen mit Hilfe eines geeigneten Oxidationsmittels (z.B. Kaliumperoxymonosulfat oder Wasserstoffperoxid (vgl. M. D. Coburn, J. Het. Chem. 1970, 7, 455)). Anschließend ist die Umsetzung mit Verbindungen der Formel (1-4), die über eine geeignete Gruppe LG1 verfügen, möglich, was zum Beispiel in Anwesenheit einer Base (z.B. Kaliumcarbonat für z.B. LG1 = Chlor (vgl. WO 2008/156757), Pyridin für z.B. LG1 = Cl (vgl. C. Nyffenegger et al, Synlett 2009, 8, 1318)) oder Natriumhydrid für z.B. LG1 = Chlor oder Brom (vgl. US 2008/0021032)) und/oder eines Katalysators (z.B. CuI/Proline für z.B. LG1 = Brom (vgl. WO 2012/006475)) erfolgen kann. Alternativ können die Verbindungen der Formel (1-2) mit Formaldehyd zu (1-3-a) umgesetzt werden, wobei über die Hydroxygruppe anschließende Einführung einer Abgangsgruppe möglich ist, z.B. mit Thionylchlorid für LG2 = Cl (vgl. S. Julia, Heterocycles 1986, 24, 2233). Substanzen der Formel (1-3-b) erlauben durch Reaktion mit jeglicher Art Nucleophil NuH, was zum Beispiel in Anwesenheit einer Base wie Kaliumcarbonat für z.B. LG1 = Chlor (vgl. X. Wen, Bioorg. Med. Chem. Lett. 2007, 17, 5777) oder Natriumhydrid für z.B. LG1 = Chlor (vgl. R. Wang, Eur. J. Org. Chem. 2007, 4, 655) erfolgen kann, Zugang zu zahlreichen Verbindungen der Formel (1-3). Darauf folgende Reduktion von Verbindungen der Formel (1-3) mit einem angemessenen Reduktionsmittel (z.B. Pd/C und Wasserstoff (vgl. US 2008/0021032), Zinkstaub und Ammoniumchlorid (vgl. US 2012/0010235), Zinn(IV)-chlorid und Ammoniumchlorid (vgl. WO 2011/140488) oder Zinn(II)-chlorid (vgl. US 2010/0310493)) ermöglicht Verbindungen der Formel (II-1) herzustellen.

In einer alternativen Route B-1 können 1H-Pyrazol-3-amine der Formel (1-1) mit Verbindungen der Formel (1-4) umgesetzt werden, die über eine geeignete Gruppe LG1 verfügen. Dies kann zum Beispiel in Anwesenheit einer Base (z.B. Kalium-tert-butoxid für z.B. LG1 = Chlor (vgl. WO 2009/012482), Cäsiumcarbonat für z.B. LG1 = Chlor (vgl. WO 2007/056155) oder Triethylamin für z.B. LG1 = *tert-*Butyloxycarbonyloxy (vgl. T. Honma et al, J. Med. Chem. 2001, 44, 4628)) oder/und eines Kupplungsreagenzes (z.B. 1-Ethyl-3-(3-dimethylaminopropyl)carbodiimid (E. Valeur, M. Bradley, Chem. Soc. Rev. 2009, 38, 606; S.-Y. Han, Y.-A. Kim, Tetrahedron 2004, 60, 2447)) und/oder eines Katalysators (z.B. CuI/N,N-Dimethylethan-1,2-diamin für z.B. LG1 = Iod (vgl. WO 2008/153042) oder Pd2(dba)3/Xantphos für z.B. LG1 = Chlor (vgl. Z. Shen, Y. Hong, X. He, W. Mo, B. Hu, N. Sun, X. Hu, Org. Lett. 2010, 12, 552)) erfolgen und so die Bildung von Verbindungen der Formel (II-1) ermöglichen.

Gemäß Alternativroute C-1 können 1H-Pyrazol-3-carbonsäurealkylester der Formel (1-5) mit Verbindungen der Formel (1-4), die über eine geeignete Gruppe LG1 verfügen, nach den in Route A-1 und B-1 beschriebenen Methoden umgesetzt werden. Anschließend kann der Ester hydrolysiert werden, z.B. durch geeignete Basen (z.B. Natriumhydroxid (vgl. WO 2011/063501) oder Lithiumhydroxid (vgl. D. J. Jeon et al, Bull. Korean Chem. Soc. 1998, 19, 725)) oder Säuren (z.B. Schwefelsäure (vgl. WO 2008/064265)) und die resultierenden Verbindungen der Formel (1-7) einer Umlagerung unterzogen werden. Diese kann in Anwesenheit bestimmter Reagenzien erfolgen, z.B. Diphenylphosphorazidat/Triethylamin/tert-Butanol (vgl. WO 2006/114313). Die folgende Abspaltung der *tert-*Butyloxycarbonylgruppe unter sauren Bedingungen (z.B. verdünnte Salzsäure (vgl. WO 2006/114313) oder Trifluoressigsäure (vgl. WO 2007/129052)) liefert Zugang zu Verbindungen der Formel (II-1).

Über Route D-1 können 1H-Pyrazol-3-amine der Formel (1-1) über eine Sequenz aus Bildung der N-substituierten Phthalimide der Formel (1-10) durch Umsetzung mit Phthalsäureanhydrid (vgl. WO 2008/074832, WO 2009/106209), Folgereaktion der Produkte mit Verbindungen der Formel (1-4), die über eine geeignete Gruppe LG1 verfügen, nach den in Route A-1 und B-1 beschriebenen Methoden, und Spaltung der Phthalimide mittels geeigneter Reagenzien (z.B. Hydrazinhydrat (vgl. WO 2009/074677) oder 2-Aminoethanol (vgl. M. H. Norman, D. J. Minick, G. C. Rigdon, J. Med. Chem. 1996, 39, 149)), in Verbindungen der Formel (II-1) überführt werden.

Gewisse Amine der Formel (II-2) 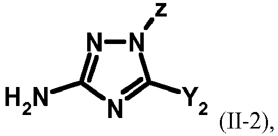 sind kommerziell erhältlich, literaturbekannt oder können anhand literaturbekannter Verfahren synthetisiert werden. Als Beispiele seien genannt:
1H-1,2,4-Triazol-3-amin (kommerziell erhältlich),
1-Phenyl-1H-1,2,4-triazol-3,5-diamin (kommerziell erhältlich),
5-Isopropyl-1H-1,2,4-triazol-3-amin (kommerziell erhältlich),
5-Methyl-1H-1,2,4-triazol-3-amin (kommerziell erhältlich),
5-Methoxy-1-methyl-1H-1,2,4-triazol-3-amin (kommerziell erhältlich),
1-(Methoxymethyl)-1H-1,2,4-triazol-3-amin (kommerziell erhältlich),
1-Isopropyl-5-methyl-1H-1,2,4-triazol-3-amin (kommerziell erhältlich),
1-(2-Methylbenzyl)-1H-1,2,4-triazol-3-amin (kommerziell erhältlich),
5-Methoxy-1-phenyl-1H-1,2,4-triazol-3-amin (kommerziell erhältlich),
1-(4,6-Dimethylpyrimidin-2-yl)-1H-1,2,4-triazol-3-amin (kommerziell erhältlich),
5-Chlor-1-phenyl-1H-1,2,4-triazol-3-amin (kommerziell erhältlich),
1-(4,5,6-Trimethylpyrimidin-2-yl)-1H-1,2,4-triazol-3-amin (kommerziell erhältlich),
5-(Methylsulfanyl)-1-phenyl-1H-1,2,4-triazol-3-amin (kommerziell erhältlich),
1-(3-Chlorbenzyl)-1H-1,2,4-triazol-3-amin (kommerziell erhältlich),
1-(3-Brompyridin-2-yl)-1H-1,2,4-triazol-3-amin (kommerziell erhältlich),
(3-Amino-1H-1,2,4-triazol-1-yl)(2-chlorphenyl)methanon (HU 39969 A2)
3-Amino-N-benzyl-1H-1,2,4-triazol-1-carboxamid (S. B. Naik, V. Joshi, Indian J. Het. Chem. 2002, 12, 109).

Gewisse Amine der Formel (II-3) 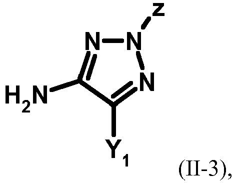 sind kommerziell erhältlich, literaturbekannt oder können anhand literaturbekannter Verfahren synthetisiert werden. Als Beispiele seien genannt:
Methyl-5-amino-2H-1,2,3-triazol-4-carboxylat (kommerziell erhältlich),
2-Propyl-2H-1,2,3-triazol-4-amin (kommerziell erhältlich),
5-Amino-2-isopropyl-2H-1,2,3-triazol-4-carbonitril (kommerziell erhältlich),
4-(4-Amino-2H-1,2,3-triazol-2-yl)butanonitril (kommerziell erhältlich),
2-{[2-(Methoxymethyl)-1,3-thiazol-5-yl]methyl}-2H-1,2,3-triazol-4-amin (WO 2012/077049 A1),
1-[5-Amino-2-(2-chlorphenyl)-2H-1,2,3-triazol-4-yl]propan-1-on (S. I. Aziz, H. F. Anwar,D. H. Fleita, M. H. Elnagdi, J. Het. Chem. 2007, 44, 725).

Gewisse Amine der Formel (II-4) 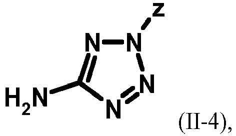 sind kommerziell erhältlich, literaturbekannt oder können anhand literaturbekannter Verfahren synthetisiert werden. Als Beispiele seien genannt:
2H-Tetrazol-5-amin (kommerziell erhältlich),
2-Ethyl-2H-tetrazol-5-amin (kommerziell erhältlich),
2-Allyl-2H-tetrazol-5-amin (kommerziell erhältlich),
3-(5-Amino-2H-tetrazol-2-yl)propanonitril (kommerziell erhältlich),
1-(5-Amino-2H-tetrazol-2-yl)aceton (kommerziell erhältlich),
Methyl-(5-amino-2H-tetrazol-2-yl)acetat (kommerziell erhältlich),
5-Amino-N-methyl-2H-tetrazol-2-carbothioamid (kommerziell erhältlich),
2-Phenyl-2H-tetrazol-5-amin (kommerziell erhältlich),
Ethyl-(5-amino-2H-tetrazol-2-yl)acetat (kommerziell erhältlich),
2-Benzyl-2H-tetrazol-5-amin (kommerziell erhältlich),
2-(5-Amino-2H-tetrazol-2-yl)-N-(1-naphthylmethyl)acetamid (kommerziell erhältlich),
2-(5-Amino-2H-tetrazol-2-yl)-N-butylacetamid (kommerziell erhältlich),
2-[(3,5-Dimethyl-1,2-oxazol-4-yl)methyl]-2H-tetrazol-5-amin (US 2010/0254916),
2-(5-Amino-2H-tetrazol-2-yl)-1-(2-chlorphenyl)ethylcarbamat (WO 2006/112685),
2-(3,4-Dichlorbenzyl)-2H-tetrazol-5-amin (W. Holzer, B. Brandstatter, C. Jager, M. Kaun, T. Langer, W. D. Bowen, Scientia Pharmaceutica 2004, 72, 197),
2-(Cyclohexylmethyl)-2H-tetrazol-5-amin (G. Quelever, S. Burlet, C. Garino, N. Pietrancosta, Y. Laras, J.-L. Kraus, J. Combinatorial Chem. 2004, 6, 695),
5-[(5-Amino-2H-tetrazol-2-yl)methyl]-4-(hydroxymethyl)-2-methylpyridin-3-ol (WO 2002/004421).

Im Allgemeinen können Verbindungen der Formel (I) und (II) nach den oben beschriebenen Verfahren hergestellt werden. Falls einzelne Verbindungen nicht nach den oben beschriebenen Verfahren hergestellt werden können, ist die Synthese durch Derivatisierung anderer Verbindungen der Formeln (I) und (II) möglich oder durch individuelle Modifikationen der beschriebenen Verfahren. Zum Beispiel kann es Vorteile haben gewisse Verbindungen der Formeln (I) oder (II) aus anderen Verbindungen der Formeln (I) und (II) herzustellen z.B. durch Hydrolyse, Verersterung, Amidbildung, Reduktion, Veretherung, Oxidation, Olefinierung, Halogenierung, Acylierung, Alkylierung und dergleichen.

Die erfindungsgemäßen Verfahren zur Herstellung der neuen Verbindungen der Formel (I) werden vorzugsweise unter Verwendung eines Verdünnungsmittels durchgeführt. Als Verdünnungsmittel zur Durchführung der erfindungsgemäßen Verfahren kommen neben Wasser alle inerten Lösungsmittel in Frage. Als Beispiele sind zu nennen: Halogenkohlenwasserstoffe (z.B. Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, Anisol, Phenetol, Cyclohexylmethylether, Dimethylether, Diethylether, Diproplether, Diisopropylether, Di-*n*-butylether, Düsobutylether, Diisoamylether, Ethylenglycoldimethylether, Tetrahydrofuran, 1,4-Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, *o-*Nitrotoluol), Nitrile (wie z.B. Acetonitril, Propionitril, Butyronitril, Isobutyronitril, Benzonitril, *m-*Chlorbenzonitril), Tetrahydrothiophendioxid, Dimethylsulfoxid, Tetramethylensulfoxid, Dipropylsulfoxid, Benzylmethylsulfoxid, Düsobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Octan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeintervalles von 70°C bis 190°C, Cyclohexan, Methylcyclohexan, Petrolether, Ligroin, Benzol, Toluol, Xylol, Ester (z.B. Methyl-, Ethyl-, Butyl-, Isobutylacetat, Dimethyl-, Dibutyl-, Ethylencarbonat), Amide (z.B. Hexamethylenphosphorsäuretriamid, Formamid, N-Methylformamid, N,N-Dimethylformamid, N,N-Dipropylformamid, N,N-Dibutylformamid, N-Methylpyrrolidin, N-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formylpiperidin, N,N'-Diformylpiperazin) und Ketone (z.B. Aceton, Acetophenon, Methylethylketon, Methylbutylketon).

Selbstverständlich kann man das erfindungsgemäße Verfahren auch in Gemischen der genannten Lösungs- und Verdünnungsmittel durchführen.

Die Reaktionstemperaturen können bei der Durchführung des erfindungsgemäßen Verfahrens in einem größeren Bereich variiert werden. Im Allgemeinen arbeitet man bei Temperaturen zwischen -30 °C und +150 °C, vorzugsweise zwischen -10 °C und +120 °C.

Das erfindungsgemäße Verfahren wird im Allgemeinen unter Normaldruck durchgeführt. Es ist jedoch auch möglich, das erfindungsgemäße Verfahren unter erhöhtem oder vermindertem Druck - im Allgemeinen bei absoluten Drücken zwischen 0,1 bar und 15 bar - durchzuführen.

Zur Durchführung des erfindungsgemäßen Verfahrens werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels, gegebenenfalls auch unter einer Schutzgasatmosphäre (z.B. unter Stickstoff, Argon oder Helium) durchgeführt und das Reaktionsgemisch wird im Allgemeinen mehrere Stunden bei der erforderlichen Temperatur gerührt. Die Aufarbeitung wird nach üblichen Methoden durchgeführt (vgl. Herstellungsbeispiele).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)-octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyltoluidin, N,N-Dimethyl-*p*-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, 2-Picolin, 3-Picolin, Pyrimidin, Acridin, N,N,N',N'-Tetramethylendiamin, N,N,N',N'-Tetraethylendiamin, Chinoxalin, N-Propyldiisopropylamin, N-Ethyl-diisopropylamin, N,N'-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoffsäuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)chlorid, Bortrifluorid oder sein Etherat, Titan(IV)chlorid, Zinn(IV)chlorid) und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure) eingesetzt werden.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apüfolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropylguar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al, Pesticide Science 1997, 51, 131) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl als Saatgutanwendungen als auch in Vor-, Tank- oder Fertigmischungen verwendet werden.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Als Mischpartner geeignete Insektizide / Akarizide / Nematizide sind:
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin; oder
   Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. Bacillus thuringiensis Subspezies israelensis, Bacillus thuringiensis Subspezies aizawai, Bacillus thuringiensis Subspezies kurstaki, Bacillus thuringiensis Subspezies tenebrionis und B.t. Pflanzenproteine: Cry1Ab, Cry1Ac, Cry1Fa, Cry1A.105, Cry2Ab, Vip3A, mCry3A, Cry3Ab, Cry3Bb, Cry34 Ab1/35Ab1; oder
   Bacillus sphaericus.
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise
   Diamide, z.B. Chlorantraniliprole, Cyantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ4-sulfanyliden}cyanamid (bekannt aus WO2007/149134) und seine Diastereomere {[(1R)-1-(6-Chlorpyridin-3-yl)ethyl](methyl)oxido-λ4-sulfanyliden}cyanamid (A) und {[(1S)-1-(6-Chlorpyridin-3-yl)ethyl](methyl)oxido-λ4-sulfanyliden}cyanamid (B) (ebenfalls bekannt aus WO2007/149134) sowie Diastereomere [(R)-Methyl(oxido){(1R)-1-[6-(trifluormethyl)pyridin-3-yl]ethyl}-λ4-sulfanyliden]cyanamid (A1) und [(S)-Methyl(oxido){(1S)-1-[6-(trifluormethyl)pyridin-3-yl]ethyl}-λ4-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ4-sulfanyliden]cyanamid (B1) und [(S)-Methyl(oxido){(1R)-1-[6-(trifluormethyl)pyridin-3-yl]ethyl}-λ4-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911) 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), Afidopyropen (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), Pyflubumide (bekannt aus WO2002/096882), Methyl-2-[2-({[3-brom-1-(3-chlorpyndin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233), Heptafluthrin, Pyriminostrobin, Flufenoxystrobin und 3-Chlor-N2-(2-cyanpropan-2-yl)-N1-[4-(1,1,1,2,3,3,3-heptafluorpropan-2-yl)-2-methylphenyl]phthalamid (bekannt aus WO2012/034472).

Als Mischpartner geeignete Fungizide sind:
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1 H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2- {2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb,, (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)immo][6-(difluormethoxy)-2,3-difluorphenyl]methyl} -2-phenylacetamid, (15.85) N-{(Z)-[(Cyclopropylmethoxy)immo][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Diftuormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,SR)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl} -N-ethyl-N-methylimidoformamid, (15.155) N'- {5-Brom-6-[(cis-4-isopropylcyclohexyl)oxy]-2-methylpyridin-3-yl} -N-ethyl-N-methylimidoformamid, (15.156) N'- {5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus*, insbesondere *B. cereus* Stamm CNCM I-1562 oder *Bacillus firmus*, Stamm I-1582 (Accession number CNCM I-1582) oder *Bacillus pumilus*, insbesondere Stamm GB34 (Accession No. ATCC 700814) und Stamm QST2808 (Accession No. NRRL B-30087), oder *Bacillus subtilis*, insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis*, insbesondere *B. thuringiensis* subspecies *israelensis* (serotype H-14), Stamm AM65-52 (Accession No. ATCC 1276), oder *B. thuringiensis subsp. aizawai*, insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana*, insbesondere Stamm ATCC 74040, *Coniothyrium minitans*, insbesondere Stamm CON/M/91-8 (Accession No. DSM-9660), *Lecanicillium spp*., insbesondere Stamm HRO LEC 12, *Lecanicillium lecanii*, (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus* (neu: *Isaria fumosorosea)*, insbesondere Stamm IFPC 200613, oder Stamm Apopka 97 (Accesion No. ATCC 20874), *Paecilomyces lilacinus*, insbesondere *P. lilacinus* Stamm 251 (AGAL 89/030550), *Talaromyces flavus*, insbesondere Stamm V117b, *Trichoderma atroviride*, insbesondere Stamm SC1 (Accession Number CBS 122089), *Trichoderma harzianum*, insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp*., *Azorhizobium caulinodans*, *Azospirillum spp*., *Azotobacter spp*., *Bradyrhizobium spp*., *Burkholderia spp*., insbesondere *Burkholderia cepacia* (ehemals bekannt als *Pseudomonas cepacia*), *Gigaspora spp*., oder *Gigaspora monosporum*, *Glomus spp*., *Laccaria spp*., *Lactobacillus buchneri*, *Paraglomus spp*., *Pisolithus tinctorus*, *Pseudomonas spp*., *Rhizobium spp*., insbesondere *Rhizobium trifolii*, *Rhizopogon spp*., *Scleroderma spp*., *Suillus spp*., *Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decane (CAS 71526-07-3), 2,2,5-Trimethyl-3-(dichloracetyl)-1,3-oxazolidine (CAS 52836-31-4).

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Die Behandlung der Pflanzen und Pflanzenteile mit den erfindungsgemäßen bzw. erfindungsgemäß zu verwendenden Wirkstoffen, Wirkstoffkombinationen bzw. Mitteln erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)-Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. die Wirkstoffe, Wirkstoffkombinationen bzw. Mittel werden auf das Blattwerk aufgebracht, wobei die Behandlungsfrequenz und die Aufwandmenge auf den Befallsdruck des jeweiligen Schädlings abgestimmt sein kann.

Bei systemisch wirksamen Verbindungen gelangen die Wirkstoffe, Wirkstoffkombinationen bzw. Mittel über das Wurzelwerk in die Pflanzen. Die Behandlung der Pflanzen erfolgt dann durch Einwirkung der Wirkstoffe, Wirkstoffkombinationen bzw. Mittel auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Wirkstoffe, Wirkstoffkombinationen bzw. Mittel getränkt, oder durch die Bodenapplikation, d.h. die erfindungsgemäßen Wirkstoffe, Wirkstoffkombinationen bzw. Mittel werden in fester Form, (z.B. in Form eines Granulats) in den Standort der Pflanzen eingebracht. Bei Wasserreiskulturen kann das auch durch Zudosieren der Erfindung in einer festen Anwendungsform (z.B. als Granulat) in ein überflutetes Reisfeld sein.

Die Bekämpfung von Tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen bzw. erfindungsgemäß zu verwendenden Wirkstoff behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang mit einem Wirkstoff der Formel (I) und Mischungspartner behandelt wird. Es umfasst auch ein Verfahren, in dem das Saatgut zu unterschiedlichen Zeiten mit einem Wirkstoff der Formel (I) und Mischungspartner behandelt wird.

Die Erfindung bezieht sich ebenfalls auf die Verwendung der erfindungsgemäßen Wirkstoffe bzw. einer ihn enthaltenden Wirkstoffkombination zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor Tierischen Schädlingen mit einem erfindungsgemäßen Wirkstoff bzw. einer ihn enthaltenden Wirkstoffkombination behandelt wurde. Die Erfindung bezieht sich auch auf Saatgut, welches zur gleichen Zeit mit einem Wirkstoff der Formel (I) und Mischungspartner behandelt wurde. Die Erfindung bezieht sich weiterhin auf Saatgut, welches zu unterschiedlichen Zeiten mit einem Wirkstoff der Formel (I) und Mischungspartner behandelt wurde. Bei Saatgut, welches zu unterschiedlichen Zeiten mit einem Wirkstoff der Formel (I) und Mischungspartner behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel (I) und Mischungspartner enthalten, durch eine Zwischenschicht getrennt sein. Die Erfindung bezieht sich auch auf Saatgut, bei dem ein Wirkstoff der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit dem erfindungsgemäßen Wirkstoff bzw. einer ihn enthaltenden Wirkstoffkombination einem Filmcoating-Verfahren unterzogen wird, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit dem erfindungsgemäßen Wirkstoff bzw. einer ihn enthaltenden Wirkstoffkombination Keimung und Auflauf des behandelten Saatguts gefördert werden können.

Ebenso ist es als vorteilhaft anzusehen, dass erfindungsgemäße Wirkstoffe bzw. ihn enthaltende Wirkstoffkombinationen insbesondere auch bei transgenem Saatgut eingesetzt werden können.

Zu nennen ist auch, dass erfindungsgemäße Wirkstoffe in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einem erfindungsgemäßen Wirkstoff bzw. einer Wirkstoffkombination eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird Wirkstoff der Formel (I) allein (bzw. als Wirkstoffkombination) oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe/Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe/Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Lösungsmittel Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S.401).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt des Wirkstoffs in den Formulierungen und nach dem Saatgut. Die Aufwandmengen bei Wirkstoffen/Wirkstoffkombinationen liegen im Allgemeinen zwischen 0,001 und 50 g pro Kilogramm Saatgut, vorzugsweise zwischen 0,01 und 15 g pro Kilogramm Saatgut.

Bevorzugt sind Pflanzen aus der Gruppe der Nutzpflanzen, Zierpflanzen, Rasenarten, allgemein genutzte Bäume, die in öffentlichen und privaten Bereichen als Zierpflanzen Verwendungen finden, und Forstbestand. Der Forstbestand umfasst Bäume für die Herstellung von Holz, Zellstoff, Papier und Produkten, die aus Teilen der Bäume hergestellt werden.

Der Begriff Nutzpflanzen, wie hier verwendet, bezeichnet Kulturpflanzen, die als Pflanzen für die Gewinnung von Nahrungsmitteln, Futtermitteln, Treibstoffen oder für technische Zwecke eingesetzt werden.

Zu den Nutzpflanzen zählen z.B. folgende Pflanzenarten: Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen. Diese Aufzählung stellt keine Limitierung dar.

Als besonders geeignete Zielkulturen sind folgende Pflanzen anzusehen: Bamwolle, Aubergine, Turf, Kernobst, Steinobst, Beerenobst, Mais, Weizen, Gerste, Gurke, Tabak, Reben, Reis, Getreide, Birne, Bohnen, Sojabohnen, Raps, Tomate, Paprika, Melonen, Kohl, Kartoffel und Apfel.

Als Bäume seien beispielhaft genannt: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Als bevorzugte Bäume können genannt werden: Aus der Baumart Aesculus: A. hippocastanum, A. pariflora, A. carnea; aus der Baumart Platanus: P. aceriflora, P. occidentalis, P. racemosa; aus der Baumart Picea: P. abies; aus der Baumart Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; aus der Baumart Eucalyptus: E. grandis, E. globulus, E. camadentis, E. nitens, E. obliqua, E. regnans, E. pilularus. Als besonders bevorzugte Bäume können genannt werden: Aus der Baumart Pinus: P. radiate, P. ponderosa, P. contorta, P. sylvestre, P. strobes; aus der Baumart Eucalyptus: E. grandis, E. globulus, E. camadentis. Als ganz besonders bevorzugte Bäume können genannt werden: Rosskastanie, Platanengewächse, Linde, Ahornbaum.

Die vorliegende Erfindung kann auch an beliebigen Rasenarten ("turfgrasses") durchgeführt werden, einschließlich "cool season turfgrasses" und "warm season turfgrasses". Beispiele für Rasenarten für die kalte Jahreszeit sind Blaugräser ("blue grasses"; *Poa spp*.), wie "Kentucky bluegrass" (*Poa pratensis* L.), "rough bluegrass" *(Poa trivialis* L.), "Canada bluegrass" *(Poa compressa* L.), "annual bluegrass" *(Poa annua* L.), "upland bluegrass" (Poa *glaucantha Gaudin),* "wood bluegrass" *(Poa nemoralis* L.) und "bulbous bluegrass" (*Poa bulbosa* L.); Straussgräser ("Bentgrass", *Agrostis spp*.), wie "creeping bentgrass" *(Agrostis palustris* Huds.), "colonial bentgrass" *(Agrostis tenuis* Sibth.), "velvet bentgrass" *(Agrostis canina* L.), "South German Mixed Bentgrass" *(Agrostis spp.* einschließlich *Agrostis tenius* Sibth., *Agrostis canina* L., und *Agrostis palustris* Huds.), und "redtop" *(Agrostis alba* L.); Schwingel ("Fescues", *Festucu spp*.), wie "red fescue" *(Festuca rubra* L. spp. *rubra*), "creeping fescue" *(Festuca rubra* L.), "chewings fescue" (*Festuca rubra commutata* Gaud.), "sheep fescue" *(Festuca ovina* L.), "hard fescue" *(Festuca longifolia* Thuill.), "hair fescue" *(Festucu capillata* Lam.), "tall fescue" *(Festuca arundinacea* Schreb.) und "meadow fescue" *(Festuca elanor* L.); Lolch ("ryegrasses", *Lolium* spp.), wie "annual ryegrass" (*Lolium multiflorum* Lam.), "perennial ryegrass" *(Lolium perenne* L.) und "italian ryegrass" *(Lolium multiflorum* Lam.); und Weizengräser ("wheatgrasses", *Agropyron* spp..), wie "fairway wheatgrass" *(Agropyron cristatum* (L.) Gaertn.), "crested wheatgrass" (*Agropyron desertorum* (Fisch.) Schult.) und "western wheatgrass" *(Agropyron smithii* Rydb.). Beispiele für weitere "cool season turfgrasses" sind "beachgrass" *(Ammophila breviligulata* Fern.), "smooth bromegrass" *(Bromus inermis* Leyss.), Schilf ("cattails") wie "Timothy" *(Phleum pratense* L.), "sand cattail" (*Phleum subulatum* L.), "orchardgrass" *(Dactylis glomerata* L.), "weeping alkaligrass" (*Puccinellia distans* (L.) Parl.) und "crested dog's-tail" *(Cynosurus cristatus* L.). Beispiele für "warm season turfgrasses" sind "Bermudagrass" (*Cynodon spp.* L. C. Rich), "zoysiagrass" *(Zoysia spp.* Willd.), "St. Augustine grass" (*Stenotaphrum secundatum* Walt Kuntze), "centipedegrass" *(Eremochloa ophiuroides* Munro Hack.), "carpetgrass" *(Axonopus affinis* Chase), "Bahia grass" *(Paspalum notatum* Flugge), "Kikuyugrass" *(Pennisetum clandestinum* Hochst. ex Chiov.), "buffalo grass" *(Buchloe dactyloids* (Nutt.) Engelm.), "Blue gramma" *(Bouteloua gracilis* (H.B.K.) Lag. ex Griffiths), "seashore paspalum" (*Paspalum vaginatum* Swartz) und "sideoats grama" *(Bouteloua curtipendula* (Michx. Torr.). "Cool season turfgrasses" sind für die erfindungsgemäße Verwendung im Allgemeinen bevorzugt. Besonders bevorzugt sind Blaugras, Straussgras und "redtop", Schwingel und Lolch. Straussgras ist insbesondere bevorzugt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid-tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid-resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.; aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella bumetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

Die Wirkstoffe der Formel (I) und ihre Zusammensetzungen eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Wirkstoffe oder Zusammensetzungen allein oder in Kombination mit anderen Wirk- und/oder Hilfsstoffen verwendet. Bevorzugt werden sie in Haushaltsinsektizid-Produkten verwendet. Die erfindungsgemäßen Wirkstoffe sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Wirkstoffe der Formel (I) und sie enthaltende Zusammensetzungen eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer erfindungsgemäßen Ausführungsform enthalten die erfindungsgemäßen Zusammensetzungen oder Mittel noch mindestens ein weiteres Insektizid und/oder mindestens ein Fungizid.

In einer weiteren Ausführungsform ist diese erfindungsgemäße Zusammensetzung eine anwendungsfertige (ready-to-use) Zusammensetzung, d.h. sie kann ohne weitere Änderungen auf das entsprechende Material aufgebracht werden. Als weitere Insektizide oder als Fungizide kommen die oben genannten in Frage.

Überraschenderweise wurde auch gefunden, dass die erfindungsgemäßen Wirkstoffe und Zusammensetzungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die erfindungsgemäßen Wirkstoffe und Zusammensetzungen allein oder in Kombinationen mit anderen Wirkstoffen als Antifouling-Mittel eingesetzt werden.

Die folgenden Herstellungs- und Verwendungsbeispiele illustrieren die Erfindung, ohne sie zu beschränken.

### Herstellunssbeispiele

### Synthesebeispiel 1

### 6-Chlor-N-(1-{[3-chlor-5-(trifluormethyl)pyridin-2-yl]methyl}-1H-pyrazol-3-yl)nicotinamid (Verbindung I-1-118 in Tabelle 1)

### Stufe 1: 3-Chlor-2-[(3-nitro-1H-pyrazol-1-yl)methyl]-5-(trifluormethyl)pyridin (gemäß Route A-1)

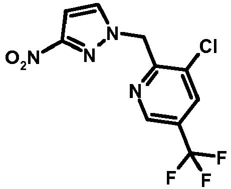

Eine Lösung von 3-Nitro-1H-pyrazol (1,60 g) in 40 mL Acetonitril wurde mit Kaliumcarbonat (3,91 g) und 3-Chlor-2-(chlormethyl)-5-(trifluormethyl)pyridin (4,88 g) versetzt und 12 h bei 60°C gerührt. Es wurde mit Wasser versetzt, der resultierende Niederschlag abgesaugt und im Vakuum getrocknet. Das Rohprodukt wurde an Kieselgel mit Cyclohexan/Ethylacetat als Laufmittel säulenchromatographisch gereinigt. Es wurden 3,50 g der Titelverbindung erhalten. HPLC-MS: logP = 2,67; Masse (m/z): 307,1 (M+H)⁺; 1H-NMR (DMSO-D₆) 5.88 (s, 2H), 7.12 - 7.13 (m, 1H), 8.15 - 8.16 (m, 1H), 8.57 (s, 1H), 8.89 (s, 1H).

### Stufe 2: 1-{[3-Chlor-5-(trifluormethyl)pyridin-2-yl]methyl}-1H-pyrazol-3-amin

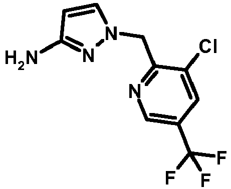

Eine Lösung von 3-Chlor-2-[(3-nitro-1H-pyrazol-1-yl)methyl]-5-(trifluormethyl)pyridin (3,40 g) in Ethanol wurde mit SnCl₂ (8,30 g) versetzt und 1 h unter Rückfluss erhitzt. Die Reaktionslösung wurde im Vakuum eingeengt, der Rückstand mit Eiswasser aufgenommen, mit 10%iger NaOH-Lösung auf pH 9 gebracht und mit Ethylacetat extrahiert. Die organische Phase wurde über Magnesiumsulfat getrocknet, filtriert und im Vakuum eingeengt. Es wurden 2,95 g der Titelverbindung erhalten. HPLC-MS: logP = 1,36; Masse (m/z): 277,1 (M+H)⁺; 1H-NMR (DMSO-D₆) 4.56 (br. s, 2H), 5.35 (s, 2H), 5.42 (s, 1H), 7.45 (s, 1H), 8.46 (s, 1H), 8.89 (s, 1H).

### Stufe 3: 6-Chlor-N-(1-{[3-chlor-5-(trifluormethyl)pyridin-2-yl]methyl}-1H-pyrazol-3-yl)nicotinamid

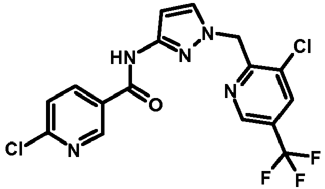

Eine Lösung von 6-Chlornicotinsäure (56 mg) in 2mL 1,4-Dioxan wurde mit 1-Ethyl-3-(3-dimethylaminopropyl)carbodümid-hydrochlorid (83 mg) versetzt und 30 Minuten bei Raumtemperatur gerührt. Anschließend wurde 1-}[3-Chlor-5-(trifluormethyl)pyridin-2-yl]methyl}-1H-pyrazol-3-amin (100 mg) zugefügt und die resultierende Reaktionsmischung 18 h bei Raumtemperatur gerührt. Es wurde im Vakuum eingeengt, der Rückstand mit Wasser versetzt, mit Ethylacetat extrahiert, die organische Phase über Magnesiumsulfat getrocknet, filtriert und im Vakuum eingeengt. Es wurden 92 mg der Titelverbindung erhalten. HPLC-MS: logP = 2,64; Masse (m/z): 416,1 (M)⁺; 1H-NMR (DMSO-D₆) 5.63 (s, 2H), 6.69 (s, 1H), 7.63 - 7.65 (m, 1H), 7.86 (s, 1H), 8.31 - 8.34 (m, 1H), 8.52 (s, 1H), 8.92 (s, 2H), 11.18 (s, 1H).

### Synthesebeispiel 2

### Methyl-3-{[(5-fluorpyridin-3-yl)carbonyl]amino}-1-methyl-1H-pyrazol-5-carboxylat (Verbindung I-1-134 in Tabelle 1)

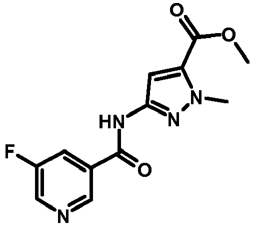

Eine Lösung von 5-Fluornicotinsäure (174 mg) in 10 mL Dichlormethan wurde mit 1-Ethyl-3-(3-dimethylaminopropyl)carbodiimid-hydrochlorid (237 mg), 1-Hydroxy-1H-benzotriazol (167 mg), Düsopropylethylamin (0,26 mL) und 4-(Dimethylamino)-pyridin (56 mg) versetzt und 5 Minuten bei Raumtemperatur gerührt. Anschließend wurde Methyl-3-amino-1-methyl-1H-pyrazol-5-carboxylat (120 mg) zugefügt und die resultierende Reaktionsmischung 48 h bei Raumtemperatur gerührt. Es wurde mit 3 mL Wasser gewaschen, die organische Phase wurde über Natriumsulfat getrocknet, filtriert und im Vakuum eingeengt. Das Rohprodukt wurde an Kieselgel mit Cyclohexan/Ethylacetat (4:6) als Laufmittel säulenchromatographisch gereinigt. Es wurden 175 mg der Titelverbindung erhalten. HPLC-MS: logP = 1,5; Masse (m/z): 279,1 (M+H)⁺; 1H-NMR (DMSO-D₆) 3.86 (s, 3H), 4.06 (s, 3H), 7.19 (s, 1H), 8.23 - 8.26 (m, 1H), 8.78 - 8.79 (m, 1H), 9.01 (s, 1H), 11.43 (s, 1H).

### Synthesebeispiel 3

### tert-Butyl-5-methyl-3-[(pyridin-3-ylcarbonyl)amino]-1H-pyrazol-1-carboxylat (Verbindung I-1-18 in Tabelle 1)

### Stufe 1: tert-Butyl-3-amino-5-methyl-1H-pyrazol-1-carboxylat (gemäß Route B-1)

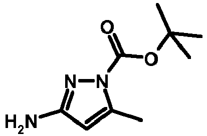

5-Methyl-1H-pyrazol-3-amin (1,00 g) wurde in Dichlormethan (50 mL) vorgelegt, mit Triethylamin (1,58 mL) und Di-*tert*-butyldicarbonat (2,47 g) versetzt und die resultierende Lösung für 3 h bei Raumtemperatur gerührt. Das Reaktionsgemisch wurde anschließend mit ges. NaCl-Lösung gewaschen, die organische Phase über Natriumsulfat getrocknet, filtriert und im Vakuum eingeengt. Das Rohprodukt wurde an Kieselgel mit Cyclohexan/Ethylacetat (3:7) als Laufmittel säulenchromatographisch gereinigt. Es wurden 1,48 g der Titelverbindung erhalten. HPLC-MS: logP = 1,23; 1H-NMR (MeOD) 1.61 (s, 9H), 2.10 (s, 3H), 5.24 (s, 1H).

### Stufe 2: tert-Butyl-5-methyl-3-[(pyridin-3-ylcarbonyl)amino]-1H-pyrazol-1-carboxylat

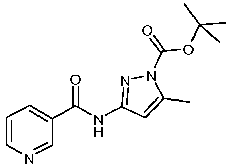

Eine Lösung von *tert*-Butyl-3-amino-5-methyl-1H-pyrazol-1-carboxylat (11,1 g) in Dichlormethan (740 mL) wurde mit Triethylamin (15,69 mL) und Nicotinoylchloridhydrochlorid (10,018 g) versetzt und 48 h bei Raumtemperatur gerührt. Das Reaktionsgemisch wurde anschließend mit 200 mL ges. NaCl-Lösung gewaschen, die organische Phase über Natriumsulfat getrocknet, filtriert und im Vakuum eingeengt. Das Rohprodukt wurde an Kieselgel mit Dichlormethan/Ethylacetat (7:3) als Laufmittel säulenchromatographisch gereinigt. Es wurden 3,98 g der Titelverbindung erhalten. HPLC-MS: logP = 2,04; Masse (m/z): 303,1 (M+H)⁺; 1H-NMR (DMSO-D₆) 1.55 (s, 9H), 2.21 (s, 3H), 6.60 (s, 1H), 7.61 - 7.65 (m, 1H), 8.24 - 8.27 (m, 1H), 8.81 - 8.83 (m, 1H), 9.08 (s, 1H), 10.80 (s, 1H).

### Synthesebeispiel 4

### N-(5-Methyl-1H-pyrazol-3-yl)nicotinamid (Verbindung I-1-17 in Tabelle 1)

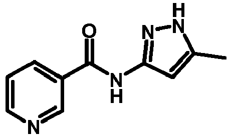

Eine Lösung von *tert*-Butyl-5-methyl-3-[(pyridin-3-ylcarbonyl)amino]-1H-pyrazol-1-carboxylat (300 mg) in Dichlormethan (10 mL) wurde mit Trifluoressigsäure (2,29 mL) versetzt und 2 h bei Raumtemperatur gerührt. Das Reaktionsgemisch wurde anschließend im Vakuum eingeengt und lieferte 200 mg der Titelverbindung. HPLC-MS: logP = 0,3; Masse (m/z): 203,1 (M+H)⁺; 1H-NMR (DMSO-D₆) 2.27 (s, 3H), 6.44 (s, 1H), 7.94 - 7.98 (m, 1H), 8.80 - 8.82 (m, 1H), 8.95 - 8.97 (m, 1H), 9.33 (s, 1H), 11.52 (s, 1H).

### Synthesebeispiel 5

### N-[5-(Difluormethoxy)-1-methyl-1H-pyrazol-3-yl]-5-fluornicotinamid (Verbindung I-1-112 in Tabelle 1)

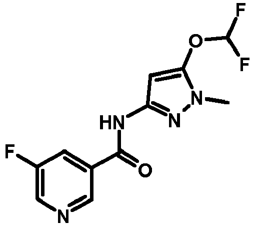

Eine Lösung von 5-Fluornicotinsäure (141 mg) in 2 mL Tetrahydrofuran wurde mit 1-Ethyl-3-(3-dimethylaminopropyl)carbodiimid-hydrochlorid (230 mg) versetzt und 30 Minuten bei Raumtemperatur gerührt. Anschließend wurde eine Lösung von 5-(Difluormethoxy)-1-methyl-1H-pyrazol-3-amin (163 mg) in 0,5 mL Tetrahydrofuran zugefügt und die resultierende Reaktionsmischung 12 h bei Raumtemperatur gerührt. Es wurde im Vakuum eingeengt und das Rohprodukt an Kieselgel mit Dichlormethan/*iso*Propanol als Laufmittel säulenchromatographisch gereinigt. Es wurden 180 mg der Titelverbindung erhalten. HPLC-MS: logP = 1,56; Masse (m/z): 287,0 (M+H)⁺; 1H-NMR (DMSO-D₆) 3.63 (s, 3H), 6.45 (s, 1H), 7.33 (t, 1H), 8.20 - 8.24 (m, 1H), 8.76 - 8.77 (m, 1H), 8.98 - 8.99 (m, 1H), 11.23 (s, 1H).

### Synthesebeispiel 6

### N-[1-(2-Chlorbenzoyl)-5-methyl-1H-pyrazol-3-yl]nicotinamid (Verbindung I-1-122 in Tabelle 1)

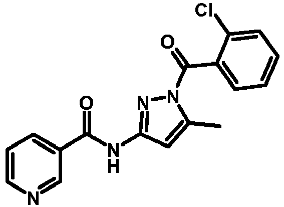

Eine Lösung von N-(5-Methyl-1H-pyrazol-3-yl)nicotinamid (0,1 g) in Dichlormethan (5 mL) wurde mit Triethylamin (0,07 mL) und 2-Chlorbenzoylchlorid (0,087 g) versetzt und 48 h bei Raumtemperatur gerührt. Das Reaktionsgemisch wurde anschließend mit Wasser gewaschen, die organische Phase wurde über Natriumsulfat getrocknet, filtriert und im Vakuum eingeengt. Das Rohprodukt wurde an Kieselgel mit Cyclohexan/Ethylacetat als Laufmittel säulenchromatographisch gereinigt. Es wurden 11 mg der Titelverbindung erhalten. HPLC-MS: logP = 2,27; Masse (m/z): 341,0 (M+H)⁺; 1H-NMR (DMSO-D₆) 2.68 (s, 3H), 7.01 (s, 1H), 7.45 - 7.59 (m, 4H), 7.64 - 7.66 (m, 1H), 8.26 - 8.29 (m, 1H), 8.70 - 8.72 (m, 1H), 9.05 (s, 1H), 11.41 (s, 1H). 13C-NMR (DMF-D₇) 15.2, 107.0, 124.9, 128.5, 130.9, 131.0, 132.0, 133.2, 136.9, 137.1, 145.9, 150.8, 152.3, 154.2, 166.0, 168.4.

### Synthesebeispiel 7

### N-(1-Benzyl-5-methyl-1H-pyrazol-3-yl)pyridazin-4-carboxamid (Verbindung I-1-79 in Tabelle 1)

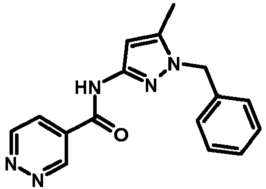

Eine Lösung von Pyridazin-4-carbonsäure (63 mg) in 2 mL 1,4-Dioxan wurde mit 1-Ethyl-3-(3-dimethylaminopropyl)carbodiimid-hydrochlorid (117 mg) versetzt und 60 Minuten bei Raumtemperatur gerührt. Anschließend wurde eine Lösung von 1-Benzyl-5-methyl-1H-pyrazol-3-amin (100 mg) in 0,5 mL 1,4-Dioxan zugefügt und die resultierende Reaktionsmischung 24 h bei Raumtemperatur und 12 h bei 50°C gerührt. Es wurde im Vakuum eingeengt, der Rückstand mit Wasser versetzt, mit Ethylacetat extrahiert, die organische Phase über Natriumsulfat getrocknet, filtriert und im Vakuum eingeengt. Der Rückstand wurde an einer präparativen HPLC mit Wasser/Acetonitril als Laufmittel chromatographiert. Es wurden 63 mg der Titelverbindung erhalten. HPLC-MS: logP = 1,67; Masse (m/z): 294,1 (M+H)⁺; 1H-NMR (DMSO-D₆) 2.26 (s, 3H), 5.23 (s, 2H), 6.52 (s, 1H), 7.14 - 7.36 (m, 5H), 8.08 - 8.10 (m, 1H), 9.39 - 9.41 (m, 1H), 9.61 (s, 1H), 11.17 (s, 1H).

### Synthesebeispiel 8

### N-[1-(Cyclopropylcarbonyl)-5-methyl-1H-pyrazol-3-yl]nicotinamid (Verbindung I-1-121 in Tabelle 1)

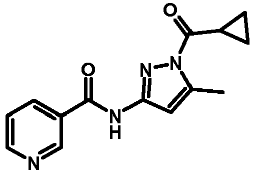

Eine Lösung von N-(5-Methyl-1H-pyrazol-3-yl)nicotinamid (0,1 g) in Dichlormethan (5 mL) wurde mit Triethylamin (0,07 mL) und Cyclopropancarbonylchlorid (0,052 g) versetzt und 48 h bei Raumtemperatur gerührt. Das Reaktionsgemisch wurde anschließend mit Wasser gewaschen, die organische Phase wurde über Natriumsulfat getrocknet, filtriert und im Vakuum eingeengt. Das Rohprodukt wurde an Kieselgel mit Cyclohexan/Ethylacetat als Laufmittel säulenchromatographisch gereinigt. Es wurden 21 mg der Titelverbindung erhalten. HPLC-MS: logP = 1,65; Masse (m/z): 271,1 (M+H)⁺; 1H-NMR (DMF-D₇) 1.09 - 1.17 (m, 4H), 2.58 (s, 3H), 3.06 - 3.10 (m, 1H), 6.96 (s, 1H), 7.59 - 7.62 (m, 1H), 8.50 - 8.52 (m, 1H), 8.81 - 8.82 (m, 1H), 9.32 (s, 1H), 11.47 (s, 1H).

### Synthesebeispiel 9

### N-[5-Brom-1-(pyrimidin-2-ylmethyl)-1H-pyrazol-3-yl]nicotinamid (Verbindung I-1-140 in Tabelle 1)

### Stufe 1: 2-(5-Brom-1H-pyrazol-3-yl)-1H-isoindol-1,3(2H)-dion (gemäß Route D-1)

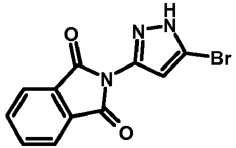

Eine Lösung von 5-Brom-1H-pyrazol-3-amin (1,00 g) in 50 mL 1,4-Dioxan wurde mit 2g Molekularsieb (2 Å) und 2-Benzofuran-1,3-dion (1,28 g) versetzt und über Nacht unter Rückfluss erhitzt. Anschließend wurde das Reaktionsgemisch filtriert und das Filtrat im Vakuum eingeengt. Das Rohprodukt wurde mit *tert*-Butyl-methylether verrührt und der resultierende Feststoff im Vakuum getrocknet. Es wurden 1,68 g der Titelverbindung erhalten. HPLC-MS: logP = 1,83; 1H-NMR (DMSO-D₆) 6.56 (s, 1H), 7.94 - 8.01 (m, 4H), 13.62 - 13.88 (m, 1H).

### Stufe 2: 2-[5-Brom-1-(pyrimidin-2-ylmethyl)-1H-pyrazol-3-yl]-1H-isoindol-1,3(2H)-dion

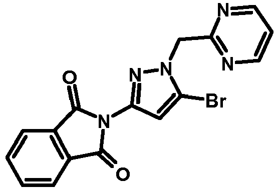

Eine Lösung von 2-(5-Brom-1H-pyrazol-3-yl)-1H-isoindol-1,3(2H)-dion (1,60 g) in 30 mL Acetonitril wurde mit 2,27 g Kaliumcarbonat und 994 mg 2-(Chlormethyl)pyrimidinhydrochlorid versetzt und 3 h unter Rückfluss erhitzt. Die resultierende Reaktionsmischung wurde filtriert und das Filtrat im Vakuum eingeengt. Es verblieben 2,00 g des Rohprodukts, das ohne weitere Reinigung für den nächsten Schritt eingesetzt wurde. HPLC-MS: logP = 2,02; Masse (m/z): 385,0 (M+H)⁺.

### Stufe 3: 5-Brom-1-(pyrimidin-2-ylmethyl)-1H-pyrazol-3-amin

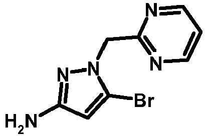

Ein Gemisch aus 2-[5-Brom-1-(pyrimidin-2-ylmethyl)-1H-pyrazol-3-yl]-1H-isoindol-1,3(2H)-dion (2,00 g) und 4 mL 2-Aminoethanol wurde für 2 h auf 100°C erhitzt. Die resultierende Reaktionsmischung wurde im Vakuum eingeengt, der Rückstand mit Ethylacetat aufgenommen, mit 10 mL 10%iger Natriumhydroxid-Lösung versetzt und die Mischung 1 h bei Raumtemperatur gerührt. Die organische Phase wurde über Natriumsulfat getrocknet, filtriert und im Vakuum eingeengt. Das Rohprodukt wurde an Kieselgel mit Ethylacetat/Ethanol (9:1) als Laufmittel säulenchromatographisch gereinigt. Es wurden 810 mg 3-Brom-1-(pyrimidin-2-ylmethyl)-1H-pyrazol-5-amin sowie 110 mg der Titelverbindung erhalten. HPLC-MS: logP = 0,56; Masse (m/z): 255,0 (M+H)⁺; 1H-NMR (DMSO-D₆) 4.79 (br. s, 2H), 5.26 (s, 2H), 5.61 (s, 1H), 7.41 - 7.43 (m, 1H), 8.75 - 8.77 (m, 2H).

### Stufe 4: N-[5-Brom-1-(pyrimidin-2-ylmethyl)-1H-pyrazol-3-yl]nicotinamid

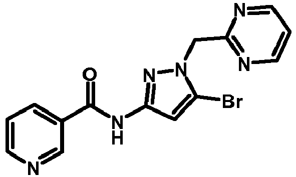

Eine Lösung von Nicotinsäure (83 mg) in 10 mL Dichlormethan wurde mit 1-Ethyl-3-(3-dimethylaminopropyl)carbodümid-hydrochlorid (130 mg), 1-Hydroxy-1H-benzotriazol (91 mg), Di*is*opropylethylamin (0,15 mL) und 4-(Dimethylamino)-pyridin (31 mg) versetzt und 5 Minuten bei Raumtemperatur gerührt. Anschließend wurde 5-Brom-1-(pyrimidin-2-ylmethyl)-1H-pyrazol-3-amin (108 mg) zugefügt und die resultierende Reaktionsmischung 48 h bei Raumtemperatur gerührt. Es wurde mit 3 mL Wasser versetzt, die organische Phase wurde über Natriumsulfat getrocknet, filtriert und im Vakuum eingeengt. Das Rohprodukt wurde an Kieselgel mit Ethylacetat/Ethanol (4:1) als Laufmittel säulenchromatographisch gereinigt. Es wurden 99 mg der Titelverbindung erhalten. HPLC-MS: logP = 1,1; Masse (m/z): 360,9 (M+H)⁺; 1H-NMR (DMSO-D₆) 5.54 (s, 2H), 6.85 (s, 1H), 7.45 - 7.47 (m, 1H), 7.50 - 7.53 (m, 1H), 8.30 - 8.33 (m, 1H), 8.72 - 8.73 (m, 1H), 8.79 - 8.81 (m, 2H), 9.10 - 9.11 (m, 1H), 11.26 (s, 1H).

### Synthesebeispiel 10

### N-(1-Benzyl-5-ethyl-1H-pyrazol-3-yl)nicotinamid (Verbindung I-1-35 in Tabelle 1)

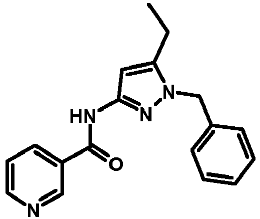

Eine Lösung von Nicotinsäure (61 mg) in 2 mL 1,4-Dioxan wurde mit 1-Ethyl-3-(3-dimethylaminopropyl)carbodiimid-hydrochlorid (114 mg) versetzt und 30 Minuten bei Raumtemperatur gerührt. Anschließend wurde eine Lösung von 1-Benzyl-5-ethyl-1H-pyrazol-3-amin (100 mg) in 0,5 mL 1,4-Dioxan zugefügt und die resultierende Reaktionsmischung 24 h bei Raumtemperatur gerührt. Es wurde im Vakuum eingeengt, der Rückstand mit Wasser versetzt, mit Ethylacetat extrahiert, die organische Phase über Natriumsulfat getrocknet, filtriert und im Vakuum eingeengt. Das Rohprodukt wurde an Kieselgel mit Cyclohexan/Ethylacetat als Laufmittel säulenchromatographisch gereinigt. Es wurden 86 mg der Titelverbindung erhalten. HPLC-MS: logP = 2,03; Masse (m/z): 307,1 (M+H)⁺; 1H-NMR (DMSO-D₆) 1.17 (t, 3H), 2.62 (q, 2H), 5.23 (s, 2H), 6.54 (s, 1H), 7.13 - 7.35 (m, 5H), 7.46 - 7.50 (m, 1H), 8.29 - 8.32 (m, 1H), 8.69 - 8.70 (m, 1H), 9.11 (s, 1H), 10.81 (s, 1H).

### Synthesebeispiel 11

### N-[4-Methyl-1-(pyrimidin-2-ylmethyl)-1H-pyrazol-3-yl]nicotinamid (Verbindung I-1-136 in Tabelle 1)

### Stufe 1: 2-(4-Methyl-1H pyrazol-3-yl)-1H-isoindol-1,3(2H)-dion (gemäß Route D-1)

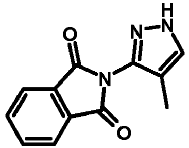

Eine Lösung von 4-Methyl-1H-pyrazol-3-amin (1,00 g) in 50 mL 1,4-Dioxan wurde mit 2g Molekularsieb (2Å) und 2-Benzofuran-1,3-dion (2,13 g) versetzt und über Nacht unter Rückfluss erhitzt. Anschließend wurde das Reaktionsgemisch filtriert und das Filtrat im Vakuum eingeengt. Das Rohprodukt wurde mit *tert*-Butyl-methylether verrührt und der resultierende Feststoff im Vakuum getrocknet. Es wurden 2,30 g der Titelverbindung erhalten. HPLC-MS: logP = 1,34; Masse (m/z): 228,1 (M+H)⁺; 1H-NMR (DMSO-D₆) 1.87 (s, 3H), 7.68 (s, 1H), 7.92 - 8.02 (m, 4H), 12.89 (s, 1H).

### Stufe 2: 2-[4-Methyl-1-(pyrimidin-2-ylmethyl)-1H-pyrazol-3-yl]-1H-isoindol-1,3(2H)-dion

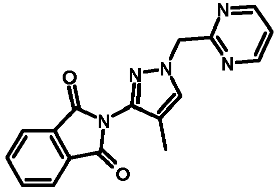

Eine Lösung von 2-(4-Methyl-1H-pyrazol-3-yl)-1H-isoindol-1,3(2H)-dion (2,30 g) in 50 mL Acetonitril wurde mit 4,19 g Kaliumcarbonat und 1,83 g 2-(Chlormethyl)pyrimidinhydrochlorid versetzt und 5 h unter Rückfluss erhitzt. Es wurde erneut 1,39 g Kaliumcarbonat und 1,66 g 2-(Chlormethyl)pyrimidinhydrochlorid zugesetzt und für weitere 3 h unter Rückfluss erhitzt. Die resultierende Reaktionsmischung wurde filtriert und das Filtrat im Vakuum eingeengt. Es verblieben 3,50 g des Rohprodukts, das ohne weitere Reinigung für den nächsten Schritt eingesetzt wurde. HPLC-MS: logP = 1,58; Masse (m/z): 320,1 (M+H)⁺.

### Stufe 3: 4-Methyl-1-(pyrimidin-2-ylmethyl)-1H-pyrazol-3-amin

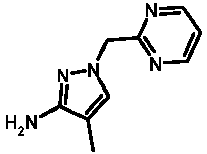

Ein Gemisch aus 2-[4-Methyl-1-(pyrimidin-2-ylmethyl)-1H-pyrazol-3-yl]-1H-isoindol-1,3(2H)-dion (3,50 g) und 6 mL 2-Aminoethanol wurde für 2 h auf 100°C erhitzt. Die resultierende Reaktionsmischung wurde im Vakuum eingeengt, der Rückstand mit Ethylacetat aufgenommen, mit 15 mL 10%iger Natriumhydroxid-Lösung versetzt und die Mischung 1 h bei Raumtemperatur gerührt.

Die organische Phase wurde über Natriumsulfat getrocknet, filtriert und im Vakuum eingeengt. Das Rohprodukt wurde an Kieselgel mit Ethylacetat/Ethanol (4:1) als Laufmittel säulenchromatographisch gereinigt. Es wurden 220 mg 4-Methyl-1-(pyrimidin-2-ylmethyl)-1H-pyrazol-5-amin sowie 250 mg der Titelverbindung erhalten. HPLC-MS: logP = -0,22; Masse (m/z): 190,1 (M+H)⁺; 1H-NMR (DMSO-D₆) 1.81 (s, 3H), 4.32 (br. s, 2H), 5.14 (s, 2H), 7.24 (s, 1H), 7.38 - 7.41 (m, 1H), 8.74 - 8.75 (m, 2H).

### Stufe 4: N-[4-Methyl-1-(pyrimidin-2-ylmethyl)-1H-pyrazol-3-yl]nicotinamid

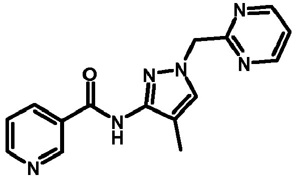

Eine Lösung von Nicotinsäure (145 mg) in 12 mL Dichlormethan wurde mit 1-Ethyl-3-(3-dimethylaminopropyl)carbodiimid-hydrochlorid (226 mg), 1-Hydroxy-1H-benzotriazol (159 mg), Di*is*opropylethylamin (0,25 mL) und 4-(Dimethylamino)-pyridin (54 mg) versetzt und 5 Minuten bei Raumtemperatur gerührt. Anschließend wurde 4-Methyl-1-(pyrimidin-2-ylmethyl)-1H-pyrazol-3-amin (140 mg) zugefügt und die resultierende Reaktionsmischung 18 h bei Raumtemperatur gerührt. Es wurde mit 10 mL Dichlormethan verdünnt und mit 10 mL Wasser versetzt, die organische Phase wurde über Natriumsulfat getrocknet, filtriert und im Vakuum eingeengt. Das Rohprodukt wurde an Kieselgel mit Ethylacetat/Ethanol (4:1) als Laufmittel säulenchromatographisch gereinigt. Es wurden 93 mg der Titelverbindung erhalten. HPLC-MS: logP = 0,44; Masse (m/z): 295,1 (M+H)⁺; 1H-NMR (DMSO-D₆) 1.91 (s, 3H), 5.43 (s, 2H), 7.43 - 7.46 (m, 1H), 7.51 - 7.54 (m, 1H), 7.66 (s, 1H), 8.28 - 8.31 (m, 1H), 8.72 - 8.74 (m, 1H), 8.79 - 8.80 (m, 2H), 9.10 (s, 1H), 10.39 (s, 1H).

### Synthesebeispiel 12

### 5-Fluor-N-[5-methyl-1-(pyrimidin-2-ylmethyl)-1H-pyrazol-3-yl]nicotinamid (Verbindung I-1-7 in Tabelle 1)

### Stufe 1: Methyl-5-methyl-1-(pyrimidin-2-ylmethyl)-1H-pyrazol-3-carboxylat (gemäß Route C-1)

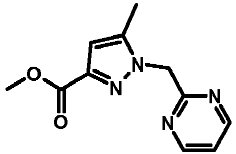

Eine Lösung von Methyl-5-methyl-1H-pyrazol-3-carboxylat (4,60 g) in 90 mL N,N-Dimethylformamid wurde mit Kaliumcarbonat (10,88 g) versetzt und 15 Minuten bei Raumtemperatur gerührt.

Anschließend wurde 2-(Chlormethyl)pyrimidinhydrochlorid (6,49 g) zugefügt, die Reaktionsmischung für 18h unter Rückfluss erhitzt, auf 300 mL Wasser gegossen und mit Ethylacetat extrahiert. Die organische Phase wurde über Natriumsulfat getrocknet, filtriert und im Vakuum eingeengt. Das Rohprodukt wurde an Kieselgel mit Cyclohexan/Ethylacetat (1:9) als Laufmittel säulenchromatographisch gereinigt. Es wurden 0,96 g Methyl-3-methyl-1-(pyrimidin-2-ylmethyl)-1H-pyrazol-5-carboxylat sowie 3,85 g der Titelverbindung erhalten. HPLC-MS: logP = 0,98; Masse (m/z): 233,0 (M+H)⁺; 1H-NMR (DMSO-D₆) 2.27 (s, 3H), 3.75 (s, 3H), 5.59. (s, 2H), 6.59 (s, 1H), 7.44 - 7.46 (m, 1H), 8.77 - 8.78 (m, 2H).

### Stufe 2: 5-Methyl-1-(pyrimidin-2-ylmethyl)-1H-pyrazol-3-carbonsäure

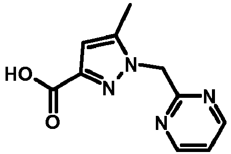

Eine Lösung von Methyl-5-methyl-1-(pyrimidin-2-ylmethyl)-1H-pyrazol-3-carboxylat (3,80 g) in 70 mL 1,4-Dioxan wurde tropfenweise mit einer Lösung von Natriumhydroxid (0,85 g) in 15 mL Wasser versetzt und 12 h bei Raumtemperatur gerührt. Die resultierende Reaktionsmischung wurde im Vakuum eingeengt, der Rückstand mit 30 mL Wasser aufgenommen, mit 2 N Salzsäure angesäuert, der resultierende Feststoff abgesaugt und im Vakuum getrocknet. Es wurden 3,30 g der Titelverbindung erhalten. HPLC-MS: logP = 0,45; Masse (m/z): 219,0 (M+H)⁺; 1H-NMR (DMSO-D₆) 2.26 (s, 3H), 5.57 (s, 2H), 6.52 (s, 1H), 7.44 - 7.47 (m, 1H), 8.78 - 8.79 (m, 2H), 12.51 (s, 1H).

### Stufe 3: tert-Butyl-[5-methyl-1-(pyrimidin-2-ylmethyl)-1H-pyrazol-3-yl]carbamat

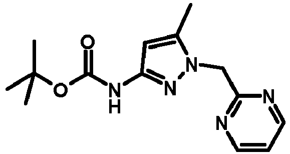

Eine Lösung von 5-Methyl-1-(pyrimidin-2-ylmethyl)-1H-pyrazol-3-carbonsäure (0,50 g) in 2-Methylpropan-2-ol (5,09 g) wurde mit Triethylamin (0,696 g) versetzt, auf 80°C erwärmt und tropfenweise mit Diphenylphosphorazidat (0,694 g) versetzt. Die Reaktionsmischung wurde 3 h bei 80°C und 12 h bei Raumtemperatur gerührt, anschließend mit ges. NaCl-Lösung versetzt und zweimal mit Ethylacetat extrahiert. Die vereinigten organischen Phasen wurden über Natriumsulfat getrocknet, filtriert und im Vakuum eingeengt. Das Rohprodukt wurde an Kieselgel mit Cyclohexan/Ethylacetat als Laufmittel säulenchromatographisch gereinigt. Es wurden 97 mg der Titelverbindung erhalten. HPLC-MS: logP = 1,75; Masse (m/z): 290,1 (M+H)⁺; 1H-NMR (DMSO-D₆) 1.41 (s, 9H), 2.23 (s, 3H), 5.31 (s, 2H), 6.11 (br. s, 1H), 7.41 - 7.43 (m, 1H), 8.75 - 8.76 (m, 2H), 9.37 (br. s, 1H).

### Stufe 4: 5-Methyl-1-(pyrimidin-2-ylmethyl)-1H-pyrazol-3-amin

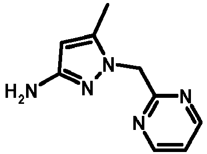

Ein Gemisch aus tert-Butyl-[5-methyl-1-(pyrimidin-2-ylmethyl)-1H-pyrazol-3-yl]carbamat (37 mg) 4 N HCl-Lösung in 1,4-Dioxan (4 mL) wurde 12 h bei Raumtemperatur gerührt und anschließend im Vakuum eingeengt. Der Rückstand wurde mit ges. NaHCO₃-Lösung versetzt und mit Dichlormethan extrahiert. Die organische Phase wurde über Natriumsulfat getrocknet, filtriert und im Vakuum eingeengt. Es verblieben 33 mg des Rohprodukts, das ohne weitere Reinigung für den nächsten Schritt eingesetzt wurde. HPLC-MS: logP < -0.23; Masse (m/z): 190,1 (M+H)⁺; 1H-NMR (DMSO-D₆) 2.16 (s, 3H), 4.37 (s, 2H), 5.15 (s, 2H), 5.25 (s, 1H), 7.38 - 7.41 (m, 1H), 8.74 - 8.75 (m, 2H).

### Stufe 5: 5-Fluor-N-[5-methyl-1-(pyrimidin-2-ylmethyl)-1H-pyrazol-3-yl]nicotinamid

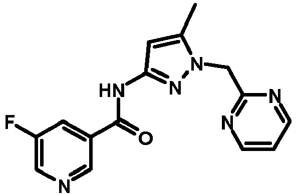

Eine Lösung von 5-Fluornicotinsäure (112 mg) in 3 mL 1,4-Dioxan wurde mit 1-Ethyl-3-(3-dimethylaminopropyl)carbodiimid-hydrochlorid (183 mg) versetzt und 30 Minuten bei Raumtemperatur gerührt. Anschließend wurde eine Lösung von 5-Methyl-1-(pyrimidin-2-ylmethyl)-1H-pyrazol-3-amin (166 mg) in 2 mL 1,4-Dioxan zugefügt und die resultierende Reaktionsmischung 18 h bei Raumtemperatur gerührt. Es wurde im Vakuum eingeengt, der Rückstand mit Wasser versetzt, mit Ethylacetat extrahiert, die organische Phase wurde über Natriumsulfat getrocknet, filtriert und im Vakuum eingeengt. Es wurden 207 mg der Titelverbindung erhalten. HPLC-MS: logP = 1,11; Masse (m/z): 313,1 (M+H)⁺; 1H-NMR (DMSO-D₆) 2.32 (s, 3H), 5.42 (s, 2H), 6.52 (s, 1H), 7.42 - 7.45 (m, 1H), 8.19 - 8.22 (m, 1H), 8.73 - 8.74 (m, 1H), 8.77 - 8.78 (m, 2H), 8.97 (s, 1H), 11.06 (s, 1H).

### Synthesebeispiel 13

### N-[5-Methyl-1-(pyrimidin-2-yl)-1H-pyrazol-3-yl]nicotinamid (Verbindung I-1-23 in Tabelle 1)

### Stufe 1: 2-(5-Methyl-3-nitro-1H pyrazol-1-yl)pyrimidin (gemäß Route A-1)

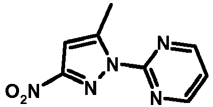

Eine Lösung von 5-Methyl-3-nitro-1H-pyrazol (450 mg) in 30 mL Acetonitril wurde mit Kaliumcarbonat (733 mg) und 2-Chlorpyrimidin (608 mg) versetzt und 12 h bei 90°C gerührt. Es wurde im Vakuum eingeengt, der Rückstand mit Wasser versetzt, mit Ethylacetat extrahiert, die organische Phase über Natriumsulfat getrocknet, filtriert und im Vakuum eingeengt. Das Rohprodukt wurde mit *n-*Hexan verrührt, abgesaugt und im Vakuum getrocknet. Es wurden 539 mg der Titelverbindung erhalten. HPLC-MS: logP = 1,41; Masse (m/z): 206,1 (M+H)⁺; 1H-NMR (DMSO-D₆) 2.65 (s, 3H), 7.07 (s, 1H), 7.65 - 7.68 (m, 1H), 8.99 - 9.00 (m, 2H).

### Stufe 2: 5-Methyl-1-(pyrimidin-2-yl)-1H-pyrazol-3-amin

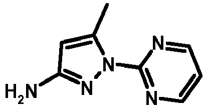

Eine Lösung von 2-(5-Methyl-3-nitro-1H-pyrazol-1-yl)pyrimidin (539 mg) in Ethanol wurde mit Pd/C (10%, 53 mg) versetzt und bei 1 bar und Raumtemperatur 18 h unter Wasserstoffatmosphäre hydriert. Die Reaktionslösung wurde anschließend über ein Kieselgelpad filtriert und im Vakuum eingeengt. Es wurden 419 mg der Titelverbindung erhalten. HPLC-MS: logP = 0,5; Masse (m/z): 176,1 (M+H)⁺; 1H-NMR (DMSO-D₆) 2.52 (s, 3H), 4.87 (br. s, 2H), 5.65 (s, 1H), 7.19 - 7.21 (m, 1H), 8.68 - 8.70 (m, 2H).

### Stufe 3: N-[5-Methyl-1-(pyrimidin-2-yl)-1H-pyrazol-3-yl]nicotinamid

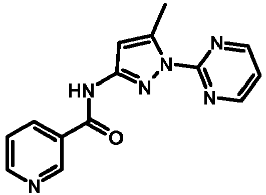

Eine Lösung von Nicotinsäure (140 mg) in 2 mL 1,4-Dioxan wurde mit 1-Ethyl-3-(3-dimethylaminopropyl)carbodiimid-hydrochlorid (262 mg) versetzt und 30 Minuten bei Raumtemperatur gerührt. Anschließend wurde eine Lösung von 5-Methyl-1-(pyrimidin-2-yl)-1H-pyrazol-3-amin (200 mg) in 0,5 mL 1,4-Dioxan zugefügt und die resultierende Reaktionsmischung 72 h bei Raumtemperatur gerührt. Es wurde im Vakuum eingeengt, der Rückstand mit Wasser versetzt, mit Ethylacetat extrahiert, die organische Phase über Natriumsulfat getrocknet, filtriert und im Vakuum eingeengt. Das Rohprodukt wurde mit Acetonitril im Ultraschallbad behandelt, der Niederschlag abgesaugt und im Vakuum getrocknet. Es wurden 163 mg der Titelverbindung erhalten. HPLC-MS: logP = 1,14; Masse (m/z): 281,1 (M+H)⁺; 1H-NMR (DMSO-D₆) 2.66 (s, 3H), 6.83 (s, 1H), 7.41 - 7.43 (m, 1H), 7.49 - 7.53 (m, 1H), 8.35 - 8.38 (m, 1H), 8.72 - 8.74 (m, 1H), 8.84 - 8.85 (m, 2H), 9.17 (s, 1H), 11.26 (s, 1H).

### Synthesebeispiel 14

### N-Methyl-N-[5-methyl-1-(pyrimidin-2-yl)-1H-pyrazol-3-yl]nicotinamid (Verbindung I-1-25 in Tabelle 1)

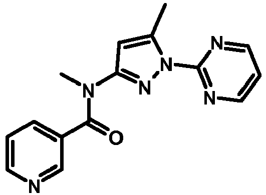

Eine Lösung von N-[5-Methyl-1-(pyrimidin-2-yl)-1H-pyrazol-3-yl]nicotinamid (154 mg) in 3 mL Tetrahydrofuran wurde bei 0°C mit Natriumhydrid (36 mg) versetzt und 1 h bei 0°C gerührt. Anschließend wurde Iodmethan (155 mg) zugetropft und 12 h bei Raumtemperatur gerührt. Es wurde mit Ethylacetat verdünnt, mit Wasser gewaschen, die wässrige Phase mit Ethylacetat extrahiert, die vereinigten organischen Phasen über Natriumsulfat getrocknet, filtriert und im Vakuum eingeengt. Der Rückstand wurde an einer präparativen HPLC mit Wasser/Acetonitril als Laufmittel chromatographiert (Gradient = 40 min von 10 % Acetonitril in Wasser auf 100 % Acetonitril). Es wurden 16 mg der Titelverbindung erhalten. HPLC-MS: logP = 1,02; Masse (m/z): 295,1 (M+H)⁺; 1H-NMR (DMSO-D₆) 2.53 (s, 3H), 3.43 (s, 3H), 6.11 (br. s, 1H), 7.30 - 7.33 (m, 2H), 7.79 - 7.82 (m, 1H), 8.56 - 8.67 (m, 2H), 8.76 - 8.77 (m, 2H).

### Synthesebeispiel 15

### N-[5-Methyl-2-(pyridin-2-yl)-2H-1,2,3-triazol-4-yl]nicotinamid (Verbindung I-3-1 in Tabelle 3)

### Stufe 1-6: 5-Methyl-2-(pyridin-2-yl)-2H-1,2,3-triazol-4-amin

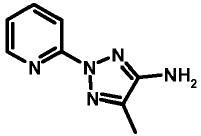

Ein Gemisch aus 0.1 mol 2-Hydrazinopyridin und 0.1 mol Ethyl-(2Z)-2-(hydroxyimino)-3-oxobutanoat in 100 mL Propan-2-ol wurde 1 h bei Raumtemperatur gerührt. Anschließend wurde filtriert, das Filtrat mit 100 mL Wasser versetzt und die Mischung über Nacht stehen gelassen. Der ausgefallene Feststoff wurde abgesaugt und getrocknet (60 % Ausbeute). Das gewonnene Ethyl-(2Z)-2-(hydroxyimino)-3-[2-(pyridin-2-yl)hydrazino]butanoat wurde mit einem Überschuss an Essigsäureanhydrid für 1 h auf 120°C erwärmt. Es wurde im Vakuum eingeengt und das resultierende Rohprodukt ohne weitere Aufreinigung verwendet (60 % Ausbeute). Erhaltenes Ethyl-5-methyl-2-(pyridin-2-yl)-2H-1,2,3-triazol-4-carboxylat wurde zusammen mit Hydrazinhydrat in Ethanol 4 h unter Rückfluss erhitzt. Das Reaktionsgemisch wurde abgekühlt und der resultierende Feststoff abgesaugt, mit Wasser gewaschen und getrocknet. 5-Methyl-2-(pyridin-2-yl)-2H-1,2,3-triazol-4-carbohydrazid wurde mit 75 % Ausbeute erhalten. Das Hydrazid wurde anschließend in Essigsäure suspendiert, bei 10°C mit einer wässrigen Lösung von Natriumnitrit versetzt und das Gemisch einige Zeit gerührt. Der resultierende Feststoff wurde abgesaugt, mit Wasser gewaschen und im Vakuum getrocknet (65 % Ausbeute). Das so gewonnene 5-Methyl-2-(pyridin-2-yl)-2H-1,2,3-triazol-4-carbonylazid wurde anschließend 2 h in Propan-2-ol unter Rückfluss erhitzt bis keine Gasentwicklung mehr zu vernehmen war. Es wurde im Vakuum eingeengt und das resultierende Rohprodukt ohne weitere Aufreinigung in der folgenden Stufe verwendet (90 % Ausbeute). Das so gewonnene Isopropyl-[5-methyl-2-(pyridin-2-yl)-2H-1,2,3-triazol-4-yl]carbamat wurde für 10 h in einem Gemisch aus Essigsäure und konz. Salzsäure erwärmt. Es wurde im Vakuum eingeengt, das resultierende Rohprodukt mit wässriger Natriumcarbonat-Lösung behandelt und der Feststoff abgesaugt sowie mit Wasser gewaschen. Umkristallisation aus Hexan lieferte 5-Methyl-2-(pyridin-2-yl)-2H-1,2,3-triazol-4-amin in 60 % Ausbeute. HPLC-MS: logP = 0,74; Masse (m/z): 176,1 (M+H)⁺; 1H-NMR (DMSO-D₆) 2.20 (s, 3H), 5.50 (br. s, 2H), 7.20 - 7.23 (m, 1H), 7.67 - 7.69 (m, 1H), 7.85 - 7.89 (m, 1H), 8.37 - 8.39 (m, 1H).

### Stufe 7: N-[5-Methyl-2-(pyridin-2-yl)-2H-1,2,3-triazol-4-yl]nicotinamid

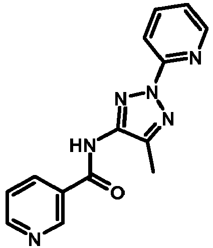

Eine Lösung von Nicotinsäure (70 mg) in 12 mL Dichlormethan wurde mit 1-Ethyl-3-(3-dimethylaminopropyl)carbodiimid-hydrochlorid (330 mg) und 4-(Dimethylamino)-pyridin (209 mg) versetzt und 15 Minuten bei Raumtemperatur gerührt. Anschließend wurde 5-Methyl-2-(pyridin-2-yl)-2H-1,2,3-triazol-4-amin (100 mg) zugefügt und die resultierende Reaktionsmischung 18 h bei Raumtemperatur gerührt. Es wurde mit wässriger Natriumdihydrogenphosphat-Lösung versetzt, die Phasen getrennt, die wässrige Phase mit Dichlormethan extrahiert, über Natriumsulfat getrocknet und im Vakuum eingeengt. Der Rückstand wurde an einer präparativen HPLC mit Wasser/Acetonitril als Laufmittel chromatographiert. Es wurden 70 mg der Titelverbindung erhalten. HPLC-MS: logP = 1,08; Masse (m/z): 281,1 (M+H)⁺; 1H-NMR (DMSO-D₆) 2.32 (s, 3H), 7.44- 7.47 (m, 1H), 7.58 - 7.62 (m, 1H), 7.93 - 7.95 (m, 1H), 8.03 - 8.07 (m, 1H), 8.36 - 8.38 (m, 1H), 8.54 - 8.55 (m, 1H), 8.80 - 8.81 (m, 1H), 9.18 (s, 1H), 11.11 (s, 1H).

### Synthesebeispiel 16

### N-(2-{2-Oxo-2-[(1-phenylethyl)amino]ethyl}-2H-tetrazol-5-yl)nicotinamid (Verbindung I-4-6 in Tabelle 4)

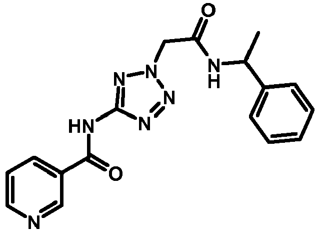

Eine Lösung von 2-(5-Amino-2H-tetrazol-2-yl)-N-(1-phenylethyl)acetamid (250 mg) in 4 mL Acetonitril wurde mit Pyridin (0,2 mL) und Nicotinoylchloridhydrochlorid (180 mg) versetzt. Anschließend wurde das Reaktionsgemisch für 1h in der Mikrowelle (CEM Discover-S, 300W) auf 120°C erhitzt, eingeengt und der Rückstand mit 10 mL Wasser verrührt. Der resultierende Niederschlag wurde abgesaugt, mit 5 mL Methyl-*tert*-butylether/Cyclohexan gewaschen und im Vakuum getrocknet. Es wurden 350 mg der Titelverbindung erhalten. HPLC-MS: logP = 1,34; Masse (m/z): 352,2 (M+H)⁺; 1H-NMR (399.95MHz, DMSO-D₆): 11.716 (5.7); 9.156 (4.0); 9.151 (4.1); 9.030 (2.2); 9.011 (2.3); 8.880 (2.7); 8.868 (2.7); 8.821 (2.7); 8.818 (2.9); 8.809 (2.9); 8.806 (2.9); 8.480 (0.6); 8.460 (1.3); 8.441 (0.7); 8.397 (1.5); 8.392 (2.2); 8.388 (1.6); 8.377 (1.7); 8.372 (2.4); 8.368 (1.6); 7.973 (1.7); 7.957 (2.1); 7.954 (2.1); 7.938 (1.5); 7.639 (2.0); 7.627 (2.1); 7.619 (2.0); 7.607 (2.0); 7.361 (16.0); 7.354 (8.6); 7.347 (8.2); 7.331 (1.8); 7.286 (0.5); 7.274 (1.2); 7.267 (1.6); 7.260 (1.7); 7.253 (1.9); 7.245 (1.4); 7.239 (1.0); 7.232 (0.6); 5.495 (12.6); 5.146 (0.4); 4.973 (0.4); 4.955 (1.6); 4.937 (2.4); 4.918 (1.7); 4.901 (0.5); 4.137 (0.3); 4.130 (0.3); 4.085 (0.4); 4.062 (0.4); 4.027 (0.4); 4.007 (0.4); 3.988 (0.4); 3.918 (0.4); 3.908 (0.4); 3.862 (0.3); 3.843 (0.3); 3.837 (0.3); 3.820 (0.3); 3.793 (0.4); 2.507 (35.7); 2.503 (45.5); 2.499 (36.0); 2.330 (0.3); 1.425 (9.9); 1.407 (10.0); 1.379 (0.6); 0.000 (28.5).

### Synthesebeispiel 17

### N-{2-[5-(Trifluormethyl)pyridin-2-yl]-2H-1,2,3-triazol-4-yl}nicotinamid (Verbindung I-3-14 in Tabelle 3)

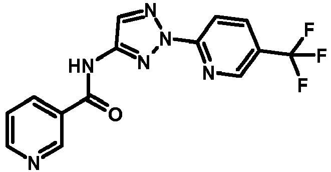

Eine Lösung von 2-[5-(Trifluormethyl)pyridin-2-yl]-2H-1,2,3-triazol-4-amin (217 mg) in Dichlormethan (6 mL) wurde mit Silbercyanid (317 mg) und Nicotinoylchloridhydrochlorid (169 mg) versetzt und die resultierende Mischung sechs Tage bei Raumtemperatur gerührt. Filtration der Reaktionsmischung über Kieselgur, wobei mit Acetonitril und Methanol nachgewaschen wurde, sowie Chromatographie an einer präparativen HPLC mit Wasser/Acetonitril als Laufmittel lieferten 31 mg der Titelverbindung. HPLC-MS: logP = 1,91; Masse (m/z): 335,0 (M+H)⁺; 1H-NMR (DMSO-D₆) 7.58 - 7.61 (m, 1H), 8.17 - 8.19 (m, 1H), 8.40 - 8.49 (m, 2H), 8.58 (s, 1H), 8.79 - 8.81 (m, 1H), 8.99 (s, 1H), 9.21 - 9.22 (m, 1H), 12.08 (s, 1H).

### Synthesebeispiel 18

### N-[1-(2-Chlorbenzyl)-1H-1,2,4-triazol-3-yl]nicotinamid (Verbindung I-2-7 in Tabelle 2)

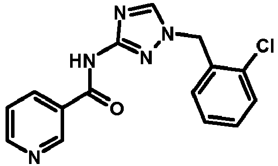

Eine Lösung von 1-(2-Chlorbenzyl)-1H-1,2,4-triazol-3-amin (202 mg) in Dichlormethan (6 mL) wurde mit Silbercyanid (324 mg) und Nicotinoylchloridhydrochlorid (172 mg) versetzt und die resultierende Mischung vier Tage bei Raumtemperatur gerührt. Filtration der Reaktionsmischung sowie Chromatographie an einer präparativen HPLC mit Wasser/Acetonitril als Laufmittel lieferten 82 mg der Titelverbindung. HPLC-MS: logP = 1,22; Masse (m/z): 314,0 (M)⁺; 1H-NMR (DMSO-D₆) 5.50 (s, 2H), 7.28 - 7.30 (m, 1H), 7.35 - 7.42 (m, 2H), 7.49 - 7.54 (m, 2H), 8.25 - 8.27 (m, 1H), 8.62 (s, 1H), 8.73 - 8.75 (m, 1H), 9.06 - 9.07 (m, 1H), 10.99 (s, 1H).

### Synthesebeispiel 19

### N-{1-[(Ethylsulfanyl)methyl]-5-methyl-1H-pyrazol-3-yl}nicotinamid (Verbindung I-1-242 in Tabelle 1)

### Stufe 1: 1-[(Ethylsulfanyl)methyl}-5-methyl-3-nitro-1H-pyrazol (gemäß Route A-1)

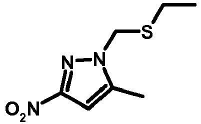

Eine Lösung von 1-(Chlormethyl)-5-methyl-3-nitro-1H-pyrazol (2,00 g) in 50 mL Acetonitril wurde mit Natriumethanthiolat (1,10 g) versetzt und 12 Stunden bei 90°C gerührt. Es wurde erneut Natriumethanthiolat (0,5 g) zugefügt und weitere 8 Stunden bei 90°C gerührt. Das Reaktionsgemisch wurde im Vakuum eingeengt und an Kieselgel mit Cyclohexan/Ethylacetat (2:1) als Laufmittel säulenchromatographisch gereinigt. Es wurden 1,00 g der Titelverbindung erhalten. HPLC-MS: logP = 2,21; Masse (m/z): 202,1 (M+H)⁺; 1H-NMR (DMSO-D₆) 1.14 - 1.18 (t, 3H), 2.39 (s, 3H), 2.58 - 2.63 (q, 2H), 5.41 (s, 2H), 6.91 (s, 1H).

### Stufe 2: 1-[(Ethylsulfanyl)methyl]-5-methyl-1H-pyrazol-3-amin

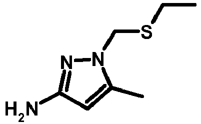

Eine Lösung von 1-[(Ethylsulfanyl)methyl]-5-methyl-3-nitro-1H-pyrazol (980 mg) in Methanol (60 mL) wurde mit Pd/C (10%, 100 mg) versetzt und bei 10 bar und Raumtemperatur 18 h unter Wasserstoffatmosphäre hydriert. Die Reaktionslösung wurde anschließend über Celite filtriert und im Vakuum eingeengt. Es wurden 830 mg der Titelverbindung erhalten. HPLC-MS: logP = 0,63; Masse (m/z): 172,0 (M+H)⁺; 1H-NMR (DMSO-D₆) 1.14 - 1.17 (t, 3H), 2.14 (s, 3H), 2.56 - 2.61 (q, 2H), 4.50 (br. s, 2H), 4.95 (s, 2H), 5.28 (s, 1H).

### Stufe 3: N-{1-[(Ethylsulfanyl)methyl]-5-methyl-1H-pyrazol-3-yl}nicotinamid

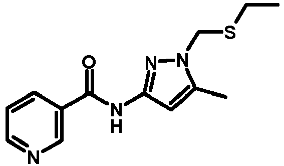

Eine Lösung von 1-[(Ethylsulfanyl)methyl]-5-methyl-1H-pyrazol-3-amin (500 mg) in Dichlormethan (40 mL) wurde mit Silbercyanid (977 mg) und Nicotinoylchloridhydrochlorid (519 mg) versetzt und die resultierende Mischung zwei Tage bei Raumtemperatur gerührt. Filtration der Reaktionsmischung sowie säulenchromatographische Reinigung des Rohproduktes an Kieselgel mit Ethylacetat/Ethanol (98:2) als Laufmittel lieferte 580 mg der Titelverbindung. HPLC-MS: logP = 1,49; Masse (m/z): 277,1 (M+H)⁺; 1H-NMR (DMSO-D₆) 1.16 - 1.19 (t, 3H), 2.32 (s, 3H), 2.59 - 2.65 (q, 2H), 5.19 (s, 2H), 6.53 (s, 1H), 7.49 - 7.53 (m, 1H), 8.30 - 8.33 (m, 1H), 8.71 - 8.72 (m, 1H), 9.10 - 9.11 (m, 1H), 11.05 (s, 1H).

### Synthesebeispiel 20

### N-{1-[(Ethylsulfonyl)methyl]-5-methyl-1H-pyrazol-3-yl}nicotinamid (Verbindung I-1-246 in Tabelle 1)

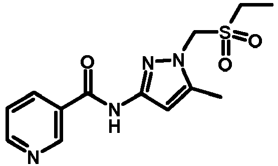

Eine Lösung von N-{1-[(Ethylsulfanyl)methyl]-5-methyl-1H-pyrazol-3-yl}nicotinamid (425 mg) in 15 mL Dichlormethan wurde mit meta-Chlorperbenzoesäure (77%ig, 448 mg) versetzt und die resultierende Mischung über Nacht bei Raumtemperatur gerührt. Das Rohprodukt wurde an Kieselgel mit Ethylacetat/Ethanol (6:4) als Laufmittel säulenchromatographisch gereinigt. Es wurden 105 mg der Titelverbindung sowie 300 mg N-{1-[(Ethylsulfinyl)methyl]-5-methyl-1H-pyrazol-3-yl}nicotinamid erhalten. HPLC-MS: logP = 0,83; Masse (m/z): 309,1 (M+H)⁺; 1H-NMR (DMSO-D₆) 1.24 - 1.28 (t, 3H), 2.36 (s, 3H), 3.16 - 3.22 (q, 2H), 5.60 (s, 2H), 6.60 (s, 1H), 7.50 - 7.54 (m, 1H), 8.30 - 8.32 (m, 1H), 8.72 - 8.74 (m, 1H), 9.10 (s, 1H), 11.12 (s, 1H).

Weitere neue 1H-Pyrazol-3-amine der Formel (II-1) wurden gemäß oder in Analogie zu den oben beschriebenen Synthesebeispielen erhalten.

### Verbindungen der Formel (II-1)

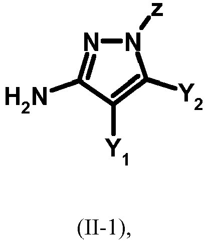

worin Y₁, Y₂ und Z die in der folgenden Tabelle angegebene Bedeutung aufweisen.

| Verbindung Nr. | **Y₁** | **Y₂** | **Z** | **log P (acid)¹** |
|---|---|---|---|---|
| II-1-1 | H | Cyclopropyl | (6-Chlorpyridin-3 -yl)methyl | 1,21 |
| II-1-2 | H | Cyclopropyl | Benzyl | 1,36 |
| II-1-3 | H | H | (6-Chlorpyridin-3 -yl)methyl | 0,56 |
| II-1-4 | H | Cyclopropyl | Prop-2-yn-1-yl | 0,63 |
| II-1-5 | H | Methyl | (6-Chlorpyridin-3 -yl)methyl | 0,77 |
| II-1-6 | H | Methyl | Pyrazin-2-yl | 0,8 |
| II-1-7 | H | H | 1,3-Difluorpropan-2-yl | -0,09 |
| II-1-8 | H | H | [3-Chlor-5-(trifluormethyl)pyridin-2-yl]methyl | 1,36 |
| II-1-9 | H | Methyl | (4,6-Dimethylpyrimidin-2-yl)methyl | 0,42 |
| II-1-10 | H | Methyl | 1,3-Thiazol-2-ylmethyl | 0,19 |
| II-1-11 | H | Methyl | 3-(Trifluormethoxy)benzyl | 1,39 |
| II-1-12 | H | Methyl | (4,6-Dimethoxypyrimidin-2-yl)methyl | 0,81 |
| II-1-13 | H | Methyl | 2-Chlorethyl | 0,13 |
| II-1-14 | 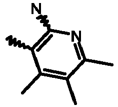 | | Methyl | -0,12 |
| II-1-16 | H | Methyl | (4-Methylpyrimidin-2-yl)methyl | 0,02 |
| II-1-17 | H | Ethyl | Prop-2-yn-1-yl | 0,46 |
| II-1-18 | H | Methyl | Pyridin-4-ylmethyl | 0,45 |
| II-1-19 | H | Ethyl | (6-Chlorpyridin-3 -yl)methyl | 1,11 |
| II-1-20 | H | Methyl | Prop-2-yn-1-yl | -0,15 |
| II-1-23 | H | Methyl | Pyrimidin-2-ylmethyl | <-0,23 |
| II-1-24 | H | Methyl | (3-Chlorpyridin-2-yl)methyl | 0,6 |
| II-1-25 | H | Methyl | (3-Cyanopyridin-2-yl)methyl | 0,21 |
| II-1-26 | Brom | Difluormethoxy | Methyl | - |
| II-1-27 | H | Methyl | Difluormethyl | 0,47 |
| II-1-28 | H | Methyl | Pyridin-3-ylmethyl | <-0,23 |
| II-1-30 | H | Brom | Pyrimidin-2-ylmethyl | 0,56 |
| II-1-31 | H | Methyl | 3-Chlorpyridin-2-yl | 0,79 |
| II-1-33 | H | Methyl | Ethoxymethyl | 0,15 |
| II-1-34 | Brom | Methyl | Pyrimidin-2-ylmethyl | 0,92 |
| II-1-35 | H | CF₃ | Pyrimidin-2-ylmethyl | 1,06 |
| II-1-36 | H | Cl | Pyrimidin-2-ylmethyl | 0,49 |
| II-1-37 | H | CH₃ | 2-Cyanopropan-2-yl | 0,64 |
| II-1-38 | H | CH₃ | (3-Methyl-1H-pyrazol-1-yl)methyl | 0,55 |
| II-1-39 | H | Methyl | (5-Methyl-1H-pyrazol-1-yl)methyl | 0,52 |
| II-1-40 | H | CHF₂ | Methyl | 0,22 |
| II-1-41 | H | Methyl | [5-(Trifluormethyl)-1H-pyrazol-1-yl]methyl | 1,26 |
| II-1-42 | H | Methyl | [3-(Trifluormethyl)-1H-pyrazol-1-yl]methyl | 1,51 |
| II-1-43 | H | Methyl | 1H-1,2,4-Triazol-1-ylmethyl | -0,23 |
| II-1-44 | H | Methyl | [(2E)-2-(Cyanoimino)-1,3-thiazolidin-3-yl]methyl | 0,44 |
| II-1-45 | H | Methyl | 1H-1,2,3-Triazol-1-ylmethyl | <-0,23 |
| II-1-46 | H | Methyl | (3,4-Dimethyl-5-oxo-4,5-dihydro-1H-1,2,4-triazol-1-yl)methyl | <-0,23 |
| II-1-47 | H | Methyl | (4-Cyclopropyl-3-methoxy-5-oxo-4,5-dihydro-1H-1,2,4-triazol-1-yl)methyl | 0,72 |
| II-1-48 | H | Methyl | (2,4-Dimethyl-3,5-dioxo-1,2,4-triazolidin-1-yl)methyl | 0,03 |
| II-1-49 | H | Methyl | (4-Cyclopropyl-3-methyl-5-oxo-4,5-dihydro-1H-1,2,4-triazol-1-yl)methyl | 0,43 |
| II-1-50 | H | Methyl | (3,4-Dicyclopropyl-5-oxo-4,5-dihydro-1H-1,2,4-triazol-1-yl)methyl | 0,98 |
| II-1-51 | H | Methyl | (Pyrimidin-2-ylsulfanyl)methyl | 0,49 |
| II-1-52 | H | Methyl | [(Isopropylideneamino)oxy]methyl | 0,57 |
| II-1-53 | H | Methyl | (2,2-Dichlorcyclopropyl)methyl | 1,12 |
| II-1-54 | H | Methyl | (1,3-Dimethyl-5-oxo-1,5-dihydro-4H-1,2,4-triazol-4-yl)methyl | <-0,23 |
| II-1-56 | H | Methyl | 3,3-Dichlorprop-2-en-1-yl | 1,13 |
| II-1-57 | H | Methyl | (2-Oxotetrahydropyrimidin-1(2H)-yl)methyl | <-0,23 |
| II-1-58 | H | Methyl | 3-(2-Chlorphenyl)-2-oxopropyl | 1,39 |
| II-1-59 | H | Methyl | 3-Methylbut-2-en-1-yl | 0,73 |
| II-1-60 | H | Methyl | (Methylsulfanyl)methyl | 0,14 |
| II-1-61 | H | Methyl | (Ethylsulfanyl)methyl | 0,63 |
| II-1-62 | H | CN | Pyrimidin-2-ylmethyl | 0,20 |
| II-1-63 | H | Methyl | (1-Methyl-1H-1,2,4-triazol-5-yl)methyl | <-0,23 |
| II-1-64 | H | Methyl | 1-Cyanoethyl | 0,02 |

**NMR Daten ausgewählter Beispiele²⁾**

| |
|---|
| Verbindung Nr. II-1-1, Lösungsmittel: [DMSO], Spektrometer: 399.95MHz |
| 8.668 (0.5); 8.623 (0.4); 7.942 (0.3); 7.852 (0.4); 7.832 (0.4); 7.639 (0.4); 7.626 (0.4); 7.370 (0.4); 7.354 (0.4); 5.901 (0.5); 5.876 (0.4); 5.807 (1.1); 5.772 (0.4); 5.559 (0.7); 5.501 (2.0); 5.448 (0.6); 5.422 (0.4); 5.353 (0.8); 3.904 (4.0); 3.646 (0.4); 3.508 (0.4); 3.167 (16.0); 2.512 (24.5); 2.508 (48.8); 2.503 (63.9); 2.499 (47.6); 2.494 (24.4); 2.334 (0.3); 2.330 (0.4); 2.325 (0.4); 2.052 (0.4); 2.039 (0.6); 2.027 (0.4); 2.018 (0.5); 2.006 (0.4); 1.993 (0.4); 1.003 (0.5); 0.996 (0.6); 0.986 (1.3); 0.980 (1.4); 0.970 (1.1); 0.965 (1.5); 0.959 (1.7); 0.949 (1.1); 0.935 (0.9); 0.873 (0.3); 0.678 (0.8); 0.668 (1.3); 0.663 (1.3); 0.656 (1.3); 0.650 (1.3); 0.639 (0.8); 0.622 (0.9); 0.615 (0.9); 0.000 (2.2) |
| Verbindung Nr. II-1-2, Lösungsmittel: [DMSO], Spektrometer: 399.95MHz |
| 8.111 (0.5); 8.107 (0.5); 7.375 (0.8); 7.368 (0.9); 7.352 (2.1); 7.331 (4.7); 7.327 (5.4); 7.323 (4.3); 7.320 (4.6); 7.310 (10.0); 7.307 (6.5); 7.291 (7.9); 7.266 (2.7); 7.254 (5.5); 7.242 (3.1); 7.236 (5.3); 7.221 (1.8); 7.218 (2.0); 7.203 (1.0); 7.197 (1.0); 7.161 (1.8); 7.157 (1.9); 7.137 (7.3); 7.133 (7.8); 7.116 (6.0); 7.080 (0.5); 7.066 (0.5); 7.049 (0.4); 6.139 (0.8); 6.114 (0.4); 5.389 (0.5); 5.362 (0.5); 5.350 (0.4); 5.294 (1.6); 5.280 (1.3); 5.275 (1.4); 5.223 (0.4); 5.211 (0.5); 5.168 (0.6); 5.110 (16.0); 5.080 (11.4); 4.796 (0.6); 4.564 (0.9); 4.281 (0.8); 4.266 (0.7); 3.903 (5.8); 3.329 (134.7); 3.174 (0.5); 3.163 (0.5); 2.680 (0.5); 2.675 (0.8); 2.671 (1.0); 2.666 (0.6); 2.511 (96.4); 2.506 (149.7); 2.502 (175.8); 2.497 (122.2); 2.492 (57.6); 2.337 (1.0); 2.333 (1.4); 2.328 (4.0); 2.205 (1.4); 1.935 (0.4); 1.912 (0.4); 1.899 (0.3); 1.891 (0.4); 1.884 (0.3); 1.878 (0.5); 1.864 (0.5); 1.856 (0.4); 1.845 (0.4); 1.765 (1.0); 1.752 (1.7); 1.744 (2.0); 1.740 (1.5); 1.732 (3.0); 1.723 (1.4); 1.719 (1.8); 1.711 (1.6); 1.698 (0.8); 1.298 (0.4); 1.258 (0.6); 1.236 (0.7); 1.037 (0.3); 1.002 (0.3); 0.996 (0.4); 0.985 (0.6); 0.981 (0.6); 0.970 (0.7); 0.964 (0.9); 0.955 (0.7); 0.949 (0.8); 0.943 (0.7); 0.934 (0.7); 0.928 (0.6); 0.920 (0.9); 0.910 (1.3); 0.903 (1.6); 0.897 (1.4); 0.889 (1.4); 0.884 (1.4); 0.853 (3.5); 0.842 (6.9); 0.837 (7.4); 0.832 (4.5); 0.827 (4.6); 0.821 (6.7); 0.816 (6.1); 0.806 (2.7); 0.789 (0.7); 0.779 (0.5); 0.769 (0.6); 0.754 (0.4); 0.717 (1.1); 0.710 (0.9); 0.696 (1.2); 0.691 (0.9); 0.684 (0.8); 0.678 (0.6); 0.671 (0.8); 0.664 (0.5); 0.657 (0.6); 0.651 (0.7); 0.645 (0.5); 0.628 (0.5); 0.610 (0.5); 0.597 (0.7); 0.587 (0.9); 0.582 (0.9); 0.574 (0.9); 0.569 (1.0); 0.558 (0.8); 0.547 (0.7); 0.541 (0.6); 0.534 (0.5); 0.510 (3.4); 0.500 (7.1); 0.497 (5.3); 0.494 (6.9); 0.487 (6.4); 0.482 (5.9); 0.472 (2.1); 0.455 (0.5); 0.447 (0.5); 0.442 (0.5); 0.436 (0.4); 0.008 (0.4); 0.000 (3.9) |
| Verbindung Nr. II-1-3, Lösungsmittel: [DMSO], Spektrometer: 399.95MHz |
| 8.723 (1.0); 8.646 (2.1); 8.202 (1.5); 8.183 (1.1); 7.993 (1.7); 7.987 (1.9); 7.924 (3.5); 7.889 (1.7); 7.882 (1.7); 7.862 (2.0); 7.785 (0.8); 7.615 (1.0); 7.553 (0.4); 7.532 (0.4); 7.313 (1.8); 7.293 (1.5); 7.197 (0.4); 7.069 (0.3); 6.310 (0.7); 6.305 (0.8); 6.283 (2.1); 6.268 (1.6); 6.175 (1.2); 6.148 (1.2); 5.441 (2.6); 5.415 (3.2); 5.363 (0.7); 5.339 (0.6); 5.288 (3.2); 5.253 (3.0); 3.903 (12.5); 3.507 (5.8); 3.489 (5.7); 3.476 (5.8); 3.463 (5.6); 3.388 (4.4); 3.378 (4.2); 3.371 (4.2); 3.365 (4.0); 3.354 (3.8); 3.167 (16.0); 2.676 (0.8); 2.672 (1.3); 2.667 (0.9); 2.512 (102.1); 2.507 (217.5); 2.503 (295.7); 2.498 (225.8); 2.494 (120.7); 2.334 (2.2); 2.329 (2.7); 2.325 (2.3); 1.954 (0.5); 1.557 (0.4); 1.470 (0.3); 1.450 (0.3); 1.434 (0.4); 1.336 (0.5); 1.298 (1.5); 1.258 (1.9); 1.249 (0.6); 1.235 (0.7); 1.215 (0.4); 0.891 (0.4); 0.873 (0.7); 0.855 (0.4); 0.000 (9.8); -0.009 (0.5) |
| Verbindung Nr. II-1-4, Lösungsmittel: [DMSO], Spektrometer: 399.95MHz |
| 8.124 (0.4); 8.121 (0.4); 6.438 (0.4); 6.120 (0.7); 6.103 (0.7); 5.058 (11.8); 5.031 (0.5); 4.975 (0.5); 4.938 (0.8); 4.933 (1.0); 4.929 (0.4); 4.916 (1.0); 4.912 (1.0); 4.719 (15.7); 4.715 (16.0); 4.675 (0.5); 4.516 (0.3); 4.489 (0.6); 4.485 (0.8); 4.471 (6.3); 3.384 (0.3); 3.367 (0.5); 3.322 (197.8); 3.266 (3.7); 3.262 (8.0); 3.258 (3.8); 3.230 (0.4); 3.224 (0.4); 3.220 (0.5); 3.216 (0.4); 2.616 (0.7); 2.613 (1.0); 2.610 (0.7); 2.522 (2.0); 2.519 (2.6); 2.516 (2.9); 2.507 (56.4); 2.504 (120.3); 2.501 (165.7); 2.498 (121.5); 2.495 (57.7); 2.388 (0.9); 2.385 (1.2); 2.382 (0.9); 1.947 (2.5); 1.895 (0.4); 1.884 (1.3); 1.788 (0.8); 1.780 (1.5); 1.774 (1.6); 1.766 (2.9); 1.758 (1.7); 1.752 (1.6); 1.744 (0.9); 1.336 (0.5); 1.298 (0.6); 1.258 (0.8); 1.235 (0.9); 1.225 (0.7); 1.040 (0.3); 1.033 (0.5); 1.029 (0.5); 1.019 (0.6); 1.015 (0.6); 1.008 (0.4); 0.976 (0.4); 0.969 (0.8); 0.965 (0.8); 0.959 (0.8); 0.955 (1.0); 0.952 (0.8); 0.945 (0.7); 0.941 (0.6); 0.925 (0.4); 0.901 (2.6); 0.894 (6.4); 0.890 (6.6); 0.887 (3.2); 0.884 (3.2); 0.880 (6.6); 0.876 (6.6); 0.870 (2.8); 0.865 (1.1); 0.861 (1.1); 0.857 (1.0); 0.853 (0.9); 0.846 (0.7); 0.742 (0.4); 0.636 (0.6); 0.624 (0.7); 0.616 (0.7); 0.608 (0.6); 0.604 (0.6); 0.571 (0.3); 0.562 (0.4); 0.546 (2.7); 0.539 (6.6); 0.536 (6.8); 0.531 (6.5); 0.527 (6.7); 0.520 (2.3); 0.498 (0.5); 0.451 (0.4); 0.442 (0.4); 0.005 (0.4); 0.000 (9.2) |
| Verbindung Nr. II-1-5, Lösungsmittel: [DMSO], Spektrometer: 399.95MHz |
| 8.572 (0.5); 8.553 (0.3); 7.704 (0.3); 7.683 (0.4); 7.662 (0.4); 7.655 (0.4); 7.641 (0.3); 7.545 (0.4); 7.525 (0.5); 7.519 (0.5); 7.498 (0.5); 7.488 (0.4); 7.310 (0.7); 7.192 (0.6); 7.184 (0.6); 7.064 (0.6); 5.953 (0.5); 5.347 (0.5); 5.317 (1.2); 5.289 (1.0); 5.273 (0.4); 5.240 (0.4); 5.169 (0.6); 4.876 (0.3); 3.903 (4.4); 3.421 (2.5); 3.414 (2.5); 3.388 (2.3); 3.372 (2.1); 3.339 (1.7); 3.167 (16.0); 2.676 (0.4); 2.672 (0.5); 2.667 (0.4); 2.525 (0.7); 2.520 (1.4); 2.511 (35.6); 2.507 (74.0); 2.502 (98.6); 2.498 (72.3); 2.493 (36.0); 2.338 (0.6); 2.334 (0.8); 2.329 (1.0); 2.325 (0.8); 2.320 (0.5); 2.303 (3.5); 2.295 (2.5); 2.273 (2.3); 2.266 (2.5); 2.223 (1.0); 2.207 (0.9); 2.198 (0.5); 2.187 (0.5); 2.169 (0.4); 2.141 (0.4); 2.105 (0.4); 1.298 (0.4); 1.258 (0.5); 0.000 (3.5) |
| Verbindung Nr. II-1-6, Lösungsmittel: [DMSO], Spektrometer: 399.95MHz |
| 9.304 (0.7); 9.301 (0.6); 9.086 (0.3); 8.941 (13.8); 8.939 (13.5); 8.737 (0.6); 8.730 (0.7); 8.657 (0.5); 8.654 (0.5); 8.651 (0.5); 8.647 (0.4); 8.522 (0.4); 8.515 (0.6); 8.510 (0.5); 8.507 (0.4); 8.372 (7.3); 8.366 (12.1); 8.363 (11.5); 8.349 (15.6); 8.343 (9.0); 6.791 (0.7); 6.789 (0.7); 5.669 (16.0); 5.279 (2.5); 3.904 (10.1); 3.508 (0.4); 3.333 (178.9); 3.169 (5.4); 2.703 (2.9); 2.676 (1.7); 2.672 (2.2); 2.667 (1.8); 2.662 (1.3); 2.656 (1.6); 2.646 (0.6); 2.635 (1.3); 2.624 (0.4); 2.615 (0.4); 2.609 (0.4); 2.586 (0.6); 2.573 (1.5); 2.543 (59.9); 2.525 (6.3); 2.512 (116.2); 2.507 (229.4); 2.503 (298.4); 2.498 (213.5); 2.494 (101.9); 2.380 (0.5); 2.338 (0.7); 2.334 (1.4); 2.329 (1.9); 2.325 (1.3); 2.320 (0.6); 1.298 (0.7); 1.259 (1.1); 1.249 (0.6); 1.234 (2.0); 0.854 (0.6); 0.843 (0.4); 0.836 (0.5); 0.813 (0.4); 0.008 (0.4); 0.000 (10.2); -0.009 (0.3) |
| Verbindung Nr. II-1-7, Lösungsmittel: [DMSO], Spektrometer: 399.95MHz |
| 7.314 (13.7); 7.308 (13.7); 7.297 (0.4); 5.561 (16.0); 5.555 (15.8); 4.817 (3.2); 4.800 (4.0); 4.793 (7.7); 4.775 (9.7); 4.761 (8.3); 4.749 (10.2); 4.737 (3.5); 4.725 (3.9); 4.697 (2.9); 4.679 (4.6); 4.673 (6.8); 4.656 (9.7); 4.643 (8.0); 4.634 (5.6); 4.631 (10.5); 4.623 (2.5); 4.619 (3.7); 4.611 (2.5); 4.607 (5.4); 4.601 (0.9); 4.591 (2.2); 4.580 (2.6); 4.574 (2.6); 4.568 (1.3); 4.563 (3.8); 4.557 (1.3); 4.551 (2.0); 4.545 (1.8); 4.537 (1.5); 4.533 (1.5); 4.525 (0.8); 4.520 (2.0); 4.514 (0.7); 4.508 (0.9); 4.503 (0.7); 4.491 (0.4); 4.068 (0.6); 4.050 (0.6); 4.032 (0.4); 3.919 (2.0); 3.911 (1.9); 2.535 (0.4); 2.508 (0.3); 2.229 (0.3); 2.129 (0.5); 1.971 (1.9); 1.962 (1.1); 1.956 (1.7); 1.950 (7.9); 1.944 (13.9); 1.938 (18.3); 1.932 (12.5); 1.925 (6.4); 1.474 (1.4); 1.440 (1.6); 1.221 (0.5); 1.203 (1.0); 1.185 (0.5); 1.145 (0.6); 0.008 (1.3); 0.000 (32.6); -0.009 (1.0) |
| Verbindung Nr. II-1-8, Lösungsmittel: [DMSO], Spektrometer: 399.95MHz |
| 8.899 (4.9); 8.896 (4.8); 8.472 (4.9); 8.469 (4.7); 7.464 (7.7); 7.459 (7.5); 5.425 (8.8); 5.420 (8.7); 5.357 (16.0); 4.568 (6.4); 3.332 (2.3); 2.512 (4.8); 2.507 (9.5); 2.503 (12.3); 2.498 (8.8); 2.494 (4.2); 0.000 (2.1) |
| Verbindung Nr. II-1-9, Lösungsmittel: [DMSO], Spektrometer: 399.95MHz |
| 7.130 (2.0); 5.243 (2.1); 5.030 (5.0); 4.360 (2.2); 3.325 (3.7); 2.519 (0.4); 2.510 (3.0); 2.506 (5.9); 2.501 (7.8); 2.497 (5.6); 2.492 (2.7); 2.382 (0.4); 2.362 (16.0); 2.179 (7.4); 0.000 (5.5) |
| Verbindung Nr. II-1-10, Lösungsmittel: [DMSO], Spektrometer: 399.95MHz |
| 7.771 (0.7); 7.764 (0.8); 7.731 (4.5); 7.693 (0.7); 7.686 (0.7); 7.647 (4.1); 7.221 (0.7); 7.093 (0.7); 6.965 (0.7); 5.508 (0.9); 5.485 (0.3); 5.447 (0.4); 5.389 (0.4); 5.359 (0.8); 5.321 (5.8); 5.294 (10.3); 4.793 (0.7); 3.903 (5.5); 3.329 (128.7); 3.208 (0.6); 3.168 (1.0); 2.675 (1.0); 2.671 (1.3); 2.666 (0.9); 2.523 (2.8); 2.506 (186.6); 2.501 (232.8); 2.497 (171.3); 2.360 (0.4); 2.328 (1.9); 2.324 (1.5); 2.309 (0.9); 2.277 (1.6); 2.154 (16.0); 2.101 (0.8); 2.066 (1.0); 2.038 (0.7); 1.990 (0.3); 1.926 (0.5); 1.820 (0.3); 1.258 (0.3); 1.235 (0.5); 0.000 (6.1) |
| Verbindung Nr. II-1-11, Lösungsmittel: [DMSO], Spektrometer: 399.95MHz |
| 7.478 (0.4); 7.471 (1.8); 7.451 (3.7); 7.431 (2.9); 7.411 (0.9); 7.390 (0.6); 7.317 (0.4); 7.299 (0.9); 7.280 (1.0); 7.250 (2.0); 7.225 (2.0); 7.210 (1.4); 7.206 (1.5); 7.189 (0.9); 7.141 (2.5); 7.121 (2.2); 7.107 (0.4); 7.082 (3.3); 7.060 (0.6); 7.050 (0.4); 7.016 (0.7); 6.441 (0.4); 5.360 (0.4); 5.349 (0.4); 5.323 (0.7); 5.310 (0.7); 5.295 (0.7); 5.271 (0.7); 5.268 (0.6); 5.260 (0.6); 5.249 (0.6); 5.243 (0.5); 5.234 (0.5); 5.188 (0.3); 5.175 (0.4); 5.140 (2.5); 5.113 (1.9); 5.075 (8.0); 4.011 (0.8); 3.995 (0.3); 3.440 (0.3); 3.426 (0.4); 3.393 (0.6); 3.378 (0.9); 3.324 (943.5); 3.268 (0.9); 3.241 (0.4); 2.710 (0.5); 2.692 (0.5); 2.675 (8.2); 2.670 (11.2); 2.666 (8.1); 2.589 (0.7); 2.572 (0.9); 2.540 (7.9); 2.510 (699.4); 2.505 (1346.2); 2.501 (1747.0); 2.497 (1277.8); 2.492 (632.1); 2.371 (0.9); 2.332 (8.7); 2.328 (11.6); 2.323 (8.6); 2.301 (0.7); 2.281 (0.8); 2.261 (0.5); 2.248 (0.5); 2.245 (0.4); 2.242 (0.4); 2.227 (0.5); 2.195 (0.5); 2.186 (0.5); 2.179 (0.4); 2.146 (0.4); 2.132 (0.4); 2.109 (0.4); 2.097 (0.4); 2.091 (0.5); 2.075 (0.3); 2.062 (0.3); 2.047 (0.4); 2.027 (0.4); 2.006 (0.8); 1.975 (16.0); 1.919 (3.2); 1.882 (0.8); 1.848 (0.3); 1.829 (1.0); 1.814 (0.3); 1.760 (0.8); 1.734 (0.3); 1.667 (0.5); 1.559 (2.4); 1.505 (0.3); 1.459 (0.5); 1.440 (0.4); 1.433 (0.4); 1.297 (1.3); 1.258 (2.6); 1.236 (9.3); 1.147 (0.3); 1.131 (0.3); 0.869 (0.8); 0.853 (2.0); 0.835 (0.9); 0.008 (1.5); 0.000 (32.7); -0.008 (1.4) |
| Verbindung Nr. II-1-12, Lösungsmittel: [DMSO], Spektrometer: 399.95MHz |
| 6.117 (2.6); 5.281 (1.6); 4.961 (3.7); 3.903 (0.7); 3.810 (16.0); 3.331 (26.7); 2.519 (0.4); 2.511 (6.9); 2.506 (14.1); 2.502 (18.6); 2.497 (13.4); 2.493 (6.4); 2.217 (5.7); 0.000 (0.6) |
| Verbindung Nr. II-1-13, Lösungsmittel: [DMSO], Spektrometer: 399.95MHz |
| 5.983 (0.7); 5.873 (2.3); 5.455 (0.7); 5.433 (1.3); 5.400 (1.4); 5.384 (2.0); 5.372 (1.5); 5.340 (1.7); 4.368 (2.9); 4.359 (5.6); 4.350 (3.4); 4.186 (2.9); 4.177 (3.8); 4.167 (2.9); 4.157 (3.8); 4.147 (6.1); 4.137 (4.1); 4.068 (4.1); 3.974 (5.2); 3.964 (7.6); 3.955 (5.0); 3.916 (4.2); 3.907 (6.4); 3.897 (4.8); 3.425 (4.5); 3.374 (3.8); 3.296 (1.5); 2.617 (1.2); 2.614 (1.9); 2.610 (1.3); 2.523 (0.9); 2.520 (1.3); 2.517 (0.6); 2.508 (106.3); 2.505 (238.7); 2.502 (338.9); 2.499 (251.3); 2.496 (121.8); 2.389 (2.1); 2.386 (2.7); 2.383 (2.1); 2.301 (16.0); 2.205 (7.5); 2.192 (2.6); 2.182 (12.1); 2.169 (6.7); 2.116 (7.9); 2.088 (5.8); 2.085 (4.3); 2.000 (2.0); 1.967 (1.5); 1.909 (2.6); 1.235 (0.9); 0.000 (5.4) |
| Verbindung Nr. II-1-14, Lösungsmittel: [DMSO], Spektrometer: 399.95MHz |
| 5.711 (3.1); 3.742 (16.0); 3.672 (0.8); 3.323 (53.0); 2.522 (0.4); 2.519 (0.4); 2.516 (0.4); 2.507 (11.3); 2.504 (24.8); 2.501 (34.1); 2.498 (24.9); 2.495 (11.7); 2.474 (0.7); 2.459 (11.4); 2.387 (14.2); 2.171 (0.5); 2.117 (10.9); 0.000 (1.0) |
| Verbindung Nr. II-1-16, Lösungsmittel: [DMSO], Spektrometer: 399.95MHz |
| 8.573 (3.3); 8.560 (3.3); 7.271 (2.3); 7.258 (2.2); 5.756 (0.8); 5.251 (3.8); 5.089 (9.3); 4.364 (3.8); 3.325 (1.9); 2.519 (0.4); 2.510 (4.7); 2.506 (9.7); 2.501 (12.8); 2.497 (9.2); 2.492 (4.3); 2.429 (16.0); 2.166 (13.2); 2.165 (13.8); 0.000 (2.7) |
| Verbindung Nr. II-1-17, Lösungsmittel: [DMSO], Spektrometer: 399.95MHz |
| 5.263 (6.6); 5.033 (0.5); 5.029 (0.5); 4.620 (9.7); 4.615 (9.9); 4.486 (3.7); 3.338 (194.5); 3.304 (0.3); 3.266 (2.2); 3.261 (4.6); 3.257 (2.1); 2.835 (0.4); 2.822 (0.4); 2.701 (0.4); 2.625 (0.5); 2.613 (0.6); 2.523 (2.2); 2.517 (1.1); 2.508 (12.7); 2.505 (23.2); 2.502 (31.3); 2.499 (24.4); 2.497 (14.1); 2.485 (1.9); 2.409 (0.4); 1.314 (0.3); 1.298 (0.4); 1.258 (0.6); 1.255 (0.5); 1.242 (1.0); 1.237 (0.6); 1.230 (0.5); 1.210 (0.5); 1.197 (1.0); 1.193 (0.4); 1.184 (0.6); 1.149 (7.7); 1.137 (16.0); 1.124 (7.5); 0.000 (1.3) |
| Verbindung Nr. II-1-18, Lösungsmittel: [DMSO], Spektrometer: 399.95MHz |
| 8.740 (0.3); 8.731 (0.5); 8.716 (0.4); 8.684 (0.3); 8.540 (0.7); 8.525 (0.8); 8.499 (2.4); 8.485 (2.5); 7.891 (0.4); 7.876 (0.4); 7.481 (0.5); 7.466 (0.5); 7.131 (0.5); 7.117 (0.4); 7.087 (0.3); 7.076 (0.4); 7.061 (0.4); 7.014 (3.1); 7.000 (3.0); 6.959 (0.3); 6.588 (0.4); 5.861 (0.4); 5.489 (0.4); 5.322 (4.3); 5.310 (0.8); 5.190 (0.8); 5.042 (8.1); 3.903 (5.6); 3.329 (101.5); 3.174 (0.9); 3.162 (0.8); 2.675 (0.5); 2.671 (0.7); 2.666 (0.5); 2.541 (0.3); 2.524 (1.8); 2.511 (46.8); 2.506 (93.4); 2.502 (122.4); 2.497 (88.3); 2.493 (42.7); 2.333 (0.6); 2.329 (0.8); 2.324 (0.6); 2.275 (0.8); 2.261 (1.4); 2.199 (0.4); 2.080 (16.0); 2.065 (0.7); 2.056 (0.4); 2.008 (0.4); 1.994 (0.4); 1.973 (0.4); 1.835 (2.1); 1.235 (0.3); 0.000 (3.9) |
| Verbindung Nr. II-1-19, Lösungsmittel: [DMSO], Spektrometer: 399.95MHz |
| 8.631 (0.9); 8.553 (0.6); 8.073 (0.5); 7.877 (0.3); 7.784 (0.5); 7.601 (0.5); 7.360 (0.4); 6.124 (0.5); 6.101 (0.5); 6.024 (0.9); 5.984 (0.4); 5.436 (0.8); 5.383 (2.1); 5.333 (0.6); 5.305 (0.6); 5.241 (0.9); 3.903 (5.8); 3.463 (6.3); 3.168 (16.0); 2.992 (0.5); 2.714 (0.6); 2.695 (1.7); 2.676 (2.7); 2.668 (1.6); 2.656 (1.9); 2.648 (1.2); 2.637 (1.3); 2.508 (80.9); 2.503 (107.0); 2.499 (81.9); 2.422 (0.7); 2.330 (0.9); 2.279 (0.5); 2.232 (0.3); 1.298 (0.5); 1.258 (0.6); 1.230 (1.0); 1.211 (1.8); 1.192 (1.9); 1.178 (2.7); 1.173 (2.9); 1.159 (4.7); 1.152 (3.3); 1.141 (2.9); 0.000 (1.6) |
| Verbindung Nr. II-1-20, Lösungsmittel: [DMSO], Spektrometer: 399.95MHz |
| 8.131 (0.4); 6.323 (0.5); 5.253 (4.0); 5.169 (0.4); 4.841 (0.9); 4.834 (1.0); 4.811 (0.3); 4.618 (7.0); 4.612 (7.6); 4.476 (1.1); 3.903 (9.1); 3.327 (214.3); 3.278 (3.2); 3.272 (5.3); 3.265 (3.1); 3.174 (1.0); 3.161 (1.0); 2.675 (0.6); 2.671 (0.9); 2.666 (0.6); 2.524 (1.2); 2.510 (62.4); 2.506 (128.7); 2.501 (171.3); 2.497 (126.4); 2.492 (63.7); 2.422 (2.1); 2.397 (1.0); 2.388 (1.1); 2.365 (1.2); 2.333 (1.4); 2.328 (1.6); 2.324 (1.3); 2.302 (0.9); 2.281 (0.9); 2.258 (2.4); 2.246 (1.6); 2.210 (0.8); 2.180 (0.8); 2.159 (1.0); 2.131 (16.0); 2.104 (1.2); 1.958 (1.3); 1.298 (0.4); 1.258 (0.5); 1.235 (0.7); 0.873 (0.4); 0.855 (0.4); 0.000 (5.5) |
| Verbindung Nr. II-1-23, Lösungsmittel: [DMSO], Spektrometer: 399.95MHz |
| 8.755 (7.5); 8.743 (7.5); 7.413 (1.7); 7.401 (3.3); 7.389 (1.6); 5.259 (4.4); 5.153 (10.9); 4.442 (0.3); 4.373 (4.3); 4.258 (0.6); 3.326 (2.7); 3.132 (0.5); 3.117 (0.3); 2.524 (0.5); 2.510 (7.6); 2.506 (14.4); 2.501 (18.4); 2.497 (12.9); 2.492 (6.0); 2.161 (16.0); 2.084 (1.3); 1.073 (0.4); 1.055 (0.8); 1.038 (0.4); 0.000 (5.0) |
| Verbindung Nr. II-1-24, Lösungsmittel: [DMSO], Spektrometer: 399.95MHz |
| 8.458 (0.3); 8.452 (2.1); 8.449 (2.3); 8.441 (2.2); 8.437 (2.1); 8.177 (1.4); 7.923 (2.1); 7.920 (2.1); 7.903 (2.3); 7.900 (2.2); 7.372 (2.0); 7.360 (2.0); 7.351 (1.8); 7.340 (1.8); 6.333 (0.4); 5.756 (2.6); 5.380 (0.9); 5.241 (4.4); 5.240 (4.4); 5.181 (11.0); 2.519 (0.5); 2.510 (7.3); 2.506 (14.9); 2.501 (19.6); 2.497 (14.0); 2.492 (6.6); 2.324 (0.4); 2.278 (1.3); 2.244 (0.4); 2.183 (16.0); 2.041 (0.4); 0.008 (0.5); 0.000 (13.9); - 0.009 (0.4) |
| Verbindung II-1-25, Lösungsmittel: [DMSO], Spektrometer: 399.95MHz |
| 8.770 (2.2); 8.765 (2.3); 8.757 (2.1); 8.753 (2.1); 8.320 (2.0); 8.316 (2.1); 8.301 (2.2); 8.296 (2.1); 8.157 (1.3); 7.543 (2.0); 7.531 (2.0); 7.524 (1.9); 7.512 (1.8); 5.755 (0.5); 5.440 (0.5); 5.249 (4.5); 5.248 (4.6); 5.221 (11.1); 2.510 (6.6); 2.506 (13.4); 2.501 (17.6); 2.497 (12.5); 2.492 (5.8); 2.386 (0.4); 2.344 (0.8); 2.306 (0.5); 2.250 (16.0); 2.097 (0.5); 0.008 (0.4); 0.000 (11.0); -0.009 (0.3) |
| Verbindung Nr. II-1-27, Lösungsmittel: [DMSO], Spektrometer: 399.95MHz |
| 12.048 (0.3); 10.357 (1.1); 8.608 (0.9); 8.589 (0.8); 8.114 (2.9); 8.111 (2.8); 6.243 (1.6); 5.740 (1.5); 5.400 (3.7); 3.326 (2.4); 2.616 (0.5); 2.613 (0.6); 2.610 (0.5); 2.522 (1.1); 2.519 (1.4); 2.516 (1.6); 2.507 (34.2); 2.504 (71.8); 2.501 (98.4); 2.498 (72.1); 2.495 (34.0); 2.389 (0.4); 2.386 (0.6); 2.383 (0.4); 2.184 (13.9); 2.171 (6.4); 2.130 (16.0); 1.235 (1.0); 0.854 (0.3); 0.000 (4.1) |
| Verbindung II-1-28: 1H-NMR (DMSO-D₆) 1.90 - 1.93 (m, 3H), 5.02 (s, 2H), 5.37 (s, 1H), 7.24 - 7.27 (m, 1H), 7.43 - 7.46 (m, 1H), 8.36 - 8.37 (m, 1H), 8.44 - 8.45 (m, 1H). |
| Verbindung Nr. II-1-30, Lösungsmittel: [DMSO], Spektrometer: 399.95MHz |
| 8.943 (0.4); 8.811 (0.6); 8.799 (0.7); 8.782 (0.4); 8.771 (12.0); 8.758 (12.0); 7.436 (2.7); 7.424 (5.1); 7.412 (2.5); 6.782 (0.7); 5.612 (12.1); 5.602 (0.7); 5.262 (16.0); 4.795 (7.0); 4.636 (0.7); 4.622 (0.3); 4.490 (0.3); 3.395 (0.4); 3.380 (0.9); 3.366 (0.9); 3.351 (0.4); 3.323 (25.0); 3.089 (0.8); 3.074 (0.8); 2.671 (0.3); 2.524 (1.0); 2.511 (20.4); 2.506 (41.6); 2.502 (54.9); 2.497 (39.1); 2.492 (18.5); 2.328 (0.4); 1.790 (4.2); 0.008 (1.7); 0.000 (47.4); -0.009 (1.6) |
| Verbindung II-1-31: 1H-NMR (CD₃CN) 1.91 (s, 3H), 5.59 (s, 1H), 7.37 - 7.40 (m, 1H), 7.94 - 7.97 (m, 1H), 8.43 - 8.44 (m, 1H). |
| Verbindung Nr. II-1-33, Lösungsmittel: [DMSO], Spektrometer: 399.95MHz |
| 7.785 (0.4); 7.780 (0.5); 7.706 (0.5); 7.701 (0.5); 6.558 (0.4); 5.682 (0.6); 5.569 (1.4); 5.079 (1.5); 4.976 (0.7); 4.628 (1.3); 4.609 (3.0); 4.598 (2.1); 4.590 (2.0); 4.580 (2.1); 4.488 (0.8); 4.454 (0.6); 3.816 (0.4); 3.457 (3.3); 3.445 (6.4); 3.434 (6.9); 3.422 (4.8); 3.412 (4.3); 3.400 (5.1); 3.377 (7.4); 3.335 (10.1); 3.175 (3.9); 3.141 (1.9); 3.134 (2.8); 3.128 (2.1); 3.091 (1.4); 2.752 (0.8); 2.616 (1.2); 2.613 (1.7); 2.610 (1.2); 2.540 (0.6); 2.522 (3.3); 2.519 (4.3); 2.516 (4.5); 2.507 (88.3); 2.504 (190.7); 2.501 (263.2); 2.498 (192.3); 2.495 (90.7); 2.388 (1.5); 2.385 (2.0); 2.382 (1.6); 2.338 (2.1); 2.293 (0.8); 2.243 (1.4); 2.222 (1.0); 2.195 (1.9); 2.166 (1.5); 2.154 (1.4); 2.131 (1.8); 2.097 (2.6); 2.086 (3.0); 2.080 (3.2); 2.069 (2.5); 2.059 (4.0); 2.051 (3.6); 2.049 (5.4); 2.034 (2.3); 2.024 (4.2); 2.003 (2.5); 1.981 (14.1); 1.955 (4.7); 1.949 (3.1); 1.939 (2.4); 1.926 (2.5); 1.921 (2.1); 1.915 (1.9); 1.908 (3.0); 1.870 (1.8); 1.866 (1.8); 1.856 (1.3); 1.108 (0.5); 1.096 (0.4); 1.083 (0.4); 1.067 (8.3); 1.055 (16.0); 1.049 (1.3); 1.043 (8.4); 1.037 (2.0); 1.026 (1.2); 0.986 (0.5); 0.005 (0.4); 0.000 (11.4); -0.006 (0.4) |
| Verbindung Nr. II-1-34, Lösungsmittel: [DMSO], Spektrometer: 399.95MHz |
| 8.796 (0.4); 8.783 (0.5); 8.769 (6.7); 8.767 (5.8); 8.757 (6.8); 8.755 (5.7); 8.173 (0.8); 7.428 (1.8); 7.416 (3.3); 7.405 (1.6); 5.512 (0.4); 5.345 (2.9); 5.286 (7.2); 5.264 (6.5); 4.619 (0.8); 3.373 (0.4); 3.168 (1.1); 2.524 (0.7); 2.510 (14.0); 2.506 (27.6); 2.501 (35.7); 2.497 (25.2); 2.492 (11.8); 2.244 (0.6); 2.174 (14.7); 2.074 (1.7); 1.946 (16.0); 1.790 (0.9); 0.000 (7.3) |
| Verbindung Nr. II-1-35: 1H-NMR (DMSO-D₆) 4.99 (s, 2H), 5.36 (s, 2H), 5.98 (s, 1H), 7.41 - 7.44 (t, 1H), 8.75 - 8.77 (d, 2H). |
| VerbindungNr. II-1-36: ¹H-NMR(400,0 MHz, CD3CN): 8,700(7,7);8,688(7,7);7,315(2,1);7,302(3,9);7,2 90(1,9);5,645(8,4);5,317(0,6);5,281(16,0);3,963(2,1);3,544(0,4);3,526(0,4);3,274(4,5);2,176(7,1);2,114( 0,5);2,107(0,5);2,101(0,4);2,080(0,3);1,964(1,7);1,952(14,0);1,946(24,2);1,940(31,2);1,934(21,5);1,928( 11,0);1,131(0,4);1,114(0,9);1,096(0,4);0,000(0,7) |
| Verbindung Nr. II-1-37: ¹H-NMR(400,0 MHz, d₆-DMSO): 5,404(2,1);4,684(2,1);3,328(9,7);2,505(5,8); 2,501(7,6);2,497(5,7);2,324(8,2);1,855(0,5);1,835(16,0);0,006(0,4);0,000(8,4);-0,008(0,4) |
| Verbindung Nr. II-1-38: ¹H-NMR(600,1 MHz, d₆-DMSO): 7,619(2,8);7,615(2,8);6,010(2,7);6,006(2,7); 5,891(10,7);5,258(4,0);4,568(4,5);3,328(10,0);2,504(2,2);2,501(4,6);2,498(6,3);2,495(4,5);2,493(2,1);2, 283(14,4);2,120(16,0) |
| Verbindung Nr. II-1-39: ¹H-NMR(400,0 MHz, d₆-DMSO): 7,311(3,3);7,308(3,2);6,000(3,2);5,932(11,7) ;5,241(4,5);4,565(5,2);4,046(1,2);3,326(16,9);2,505(18,6);2,501(23,3);2,496(17,0);2,448(15,9);2,322(16,0);0,000(8,2);-0,008(0,4) |
| Verbindung Nr. II-1-40: ¹H-NMR(400,0 MHz, d₆-DMSO): 7,242(1,7);7,107(3,5);6,973(1,7);5,696(3,7); 4,751(3,7);3,620(16,0);3,331(17,7);2,511(4,1);2,507(8,3);2,502(11,0);2,498(8,0);2,494(4,0);0,008(0,4);0 ,000(11,1);-0,009(0,5) |
| Verbindung Nr. II-1-41: ¹H-NMR(400,0 MHz, d₆-DMSO): 7,697(2,8);7,694(2,8);6,908(3,0);6,907(3,0); 6,905(3,0);6,167(0,3);6,105(10,5);5,296(4,7);4,635(5,3);3,327(13,0);2,505(16,0);2,501(20,6);2,497(15,7 );2,329(16,0);2,297(0,6);1,054(0,4);0,008(1,0);0,000(19,8);-0,008(1,4) |
| Verbindung Nr. II-1-42: ¹H-NMR(400,0 MHz, d₆-DMSO): 7,991(2,5);7,988(2,5);6,730(2,8);6,724(2,8); 6,132(10,5);5,307(4,7);4,677(5,4);3,332(14,4);2,506(13,9);2,502(17,9);2,498(13,4);2,291(16,0);0,008(0, 8);0,000(17,3);-0,008(0,8) |
| Verbindung Nr. II-1-43: ¹H-NMR(400,0 MHz, d₆-DMSO): 8,611(6,2);7,973(5,7);6,130(11,5);5,285(4,5) ;4,654(5,0);3,331(9,1);2,510(6,0);2,506(11,9);2,501(15,4);2,497(11,0);2,493(5,2);2,314(16,0);0,000(5,4) |
| Verbindung Nr. II-1-44: ¹H-NMR(400,0 MHz, d₆-DMSO): 8,316(0,7);5,333(10,8);5,296(4,7);4,699(5,3) ;3,910(2,5);3,892(4,7);3,872(2,9);3,472(3,0);3,453(4,8);3,434(2,5);3,329(10,2);2,506(11,4);2,502(14,7); 2,497(10,7);2,180(16,0);0,000(4,6) |
| Verbindung Nr. II-1-45: ¹H-NMR(400,0 MHz, d₆-DMSO): 8,317(0,7);8,095(4,5);8,093(4,6);7,722(4,4); 7,720(4,3);6,342(11,8);5,309(4,4);4,685(4,7);3,330(7,6);2,510(6,5);2,506(13,0);2,501(16,9);2,497(12,2); 2,493(5,9);2,306(16,0);2,142(0,4);1,055(0,5);0,008(1,0);0,000(23,5);-0,009(0,9) |
| Verbindung Nr. II-1-46: ¹H-NMR(400,0 MHz, d₆-DMSO): 5,525(8,7);5,232(3,3);5,231(3,3);4,539(4,0); 3,326(14,3);3,084(16,0);2,510(7,0);2,506(13,8);2,501(18,0);2,497(13,1);2,492(6,3);2,267(11,5);2,121(1 5,7);0,000(2,7) |
| Verbindung Nr. II-1-47: ¹H-NMR(400,0 MHz, d₆-DMSO): 8,316(0,5);5,458(8,0);5,244(3,5);4,556(4,2); 4,103(0,9);4,090(0,9);3,875(1,0);3,863(16,0);3,857(6,0);3,327(29,7);3,175(4,3);3,162(3,7);2,691(0,6);2, 684(0,7);2,676(1,4);2,667(0,8);2,659(0,8);2,649(0,4);2,644(0,4);2,524(0,4);2,510(10,3);2,506(20,4);2,50 2(26,8);2,497(20,0);2,260(11,7);0,858(1,5);0,853(2,3);0,849(1,8);0,836(5,5);0,829(4,5);0,821(4,0);0,809 (0,4);0,000(3,6) |
| Verbindung Nr. II-1-48: ¹H-NMR(400,0 MHz, d₆-DMSO): 5,755(5,0);5,475(8,4);5,272(3,6);4,628(4,1); 3,325(6,4);3,103(16,0);2,895(15,4);2,506(9,0);2,502(11,5);2,497(8,3);2,199(12,2);0,000(5,8) |
| Verbindung Nr. II-1-49: ¹H-NMR(400,0 MHz, d₆-DMSO): 8,316(1,3);5,756(0,4);5,479(8,6);5,230(3,5); 4,542(4,1);3,325(16,4);3,301(0,4);2,778(0,6);2,775(0,6);2,773(0,5);2,771(0,6);2,764(1,3);2,756(0,6);2,7 52(0,6);2,748(0,6);2,737(0,4);2,510(6,8);2,506(13,6);2,501(17,9);2,497(12,9);2,492(6,2);2,256(12,1);2,1 75(16,0);2,170(3,9);0,902(1,4);0,897(2,6);0,886(3,5);0,879(6,4);0,865(1,3);0,858(0,4);0,008(0,3);0,000( 9,8);-0,009(0,3) |
| Verbindung Nr. II-1-50: ¹H-NMR(400,0 MHz, d₆-DMSO): 11,156(0,7);8,316(1,5);5,756(0,9);5,500(0,4) ;5,495(0,5);5,453(11,1);5,225(4,7);4,552(5,8);3,326(56,4);3,302(0,6);3,110(0,8);2,873(0,4);2,862(0,8);2 ,855(1,0);2,846(1,7);2,836(0,9);2,830(1,0);2,819(0,9);2,811(0,6);2,803(1,1);2,792(0,5);2,786(0,4);2,524 (0,6);2,511(14,1);2,506(28,5);2,502(37,7);2,497(27,3);2,493(13,0);2,278(0,6);2,266(0,8);2,261(0,7);2,24 8(16,0);1,961(0,4);1,948(1,0);1,940(1,0);1,935(1,0);1,928(2,1);1,920(0,8);1,915(1,8);1,907(1,2);1,902(0 ,7);1,894(0,9);0,956(2,1);0,951(3,2);0,946(3,6);0,934(8,5);0,929(7,1);0,926(8,1);0,922(7,5);0,915(6,5);0 ,907(4,8);0,898(1,9);0,892(1,7);0,885(2,0);0,877(0,8);0,805(1,3);0,796(3,2);0,790(4,5);0,784(4,4);0,778 (4,4);0,772(1,9);0,767(1,0);0,760(0,6);0,008(0,6);0,000(19,8);-0,009(0,7) |
| Verbindung Nr. II-1-51: ¹H-NMR(400,0 MHz, d₆-DMSO): 8,689(7,7);8,677(7,8);7,278(2,2);7,265(4,2); 7,253(2,1);5,680(12,9);5,261(4,5);4,628(5,1);3,329(19,2);2,507(16,5);2,502(21,4);2,498(15,7);2,479(0,4 );2,200(16,0);0,008(0,6);0,000(16,0);-0,009(0,6) |
| Verbindung Nr. II-1-52: ¹H-NMR(601,6 MHz, d₆-DMSO): 5,506(9,7);5,274(2,8);5,273(2,9);4,546(2,4); 3,342(2,9);2,508(2,4);2,505(5,5);2,502(7,7);2,499(5,7);2,496(2,6);2,219(0,3);2,202(10,3);2,201(10,8);1, 789(0,5);1,780(15,4);1,759(0,4);1,742(16,0);1,731(0,4);0,005(0,4);0,000(13,4);-0,006(0,4) |
| Verbindung Nr. II-1-53: ¹H-NMR(400,0 MHz, d₆-DMSO): 5,235(4,5);4,492(1,8);4,058(1,3);4,040(1,4); 4,021(1,8);4,003(1,8);3,836(1,9);3,820(1,9);3,799(1,4);3,783(1,4);3,326(9,3);2,510(5,2);2,506(10,3);2,5 01(13,4);2,497(9,6);2,493(4,6);2,185(0,4);2,165(0,9);2,158(0,6);2,147(1,1);2,133(16,0);2,123(1,1);2,104 (0,5);1,799(1,8);1,780(2,0);1,772(1,6);1,753(1,7);1,501(1,8);1,482(2,8);1,463(1,5);0,000(6,7) |
| Verbindung Nr. II-1-54: ¹H-NMR(400,0 MHz, d₆-DMSO): 5,520(0,4);5,480(8,8);5,233(3,4);4,633(3,8); 3,327(17,8);3,227(16,0);3,186(1,2);3,175(1,2);3,162(1,1);2,510(6,5);2,506(13,1);2,502(17,3);2,497(12,6);2,493(6,2);2,394(0,9);2,379(15,1);2,312(0,8);2,300(12,1);2,035(1,2);1,055(0,5);0,008(0,8);0,000(21,9) ;-0,009(0,9) |
| Verbindung Nr. II-1-55: ¹H-NMR(601,6MHz, d₆-DMSO): 8,321(4,4);5,641(0,4);5,325(10,1);5,248(3,7) ;4,480(2,3);3,344(1,8);2,508(4,7);2,505(10,2);2,502(14,0);2,499(10,1);2,497(4,7);2,228(0,5);2,215(13,8) ;2,076(0,3);2,041(0,7);1,595(16,0);0,005(0,5);0,000(19,3);-0,006(0,7) |
| Verbindung Nr. II-1-56: ¹H-NMR(400,0 MHz, d₆-DMSO): 6,234(1,7);6,218(3,8);6,201(1,9);5,240(4,4); 5,239(4,2);4,521(8,4);4,505(7,8);3,325(14,3);2,510(8,2);2,506(15,4);2,502(19,6);2,497(14,2);2,422(0,4); 2,117(16,0);0,008(1,3);0,000(25,1);-0,009(1,1) |
| Verbindung Nr. II-1-57: ¹H-NMR(600,1 MHz, d₆-DMSO): 6,395(1,5);5,226(4,7);5,186(11,7);4,467(5,1) ;3,334(24,0);3,309(2,1);3,300(3,1);3,290(2,2);3,173(0,7);3,165(0,7);3,130(0,4);3,074(1,2);3,070(1,4);3, 064(2,1);3,061(2,1);3,055(1,5);3,051(1,2);2,507(2,4);2,504(5,3);2,501(7,3);2,498(5,3);2,495(2,4);2,182( 16,0);2,181(15,5);1,753(0,6);1,743(1,5);1,734(2,3);1,724(1,4);1,714(0,5) |
| Verbindung Nr. II-1-58: ¹H-NMR(400,0 MHz, d₆-DMSO): 7,445(1,0);7,441(0,9);7,435(1,6);7,427(1,7); 7,421(1,3);7,416(0,6);7,309(2,1);7,302(8,3);7,298(3,8);7,294(5,5);7,279(1,4);5,288(4,5);4,830(10,7);4,4 78(4,0);3,871(10,4);3,329(107,8);3,174(1,5);3,162(1,5);2,675(0,3);2,671(0,5);2,666(0,4);2,524(0,9);2,51 9(1,4);2,511(26,8);2,506(56,7);2,502(77,0);2,497(57,4);2,493(28,9);2,333(0,4);2,329(0,5);2,324(0,4);2,1 38(0,4);2,099(0,6);2,042(0,7);2,028(16,0);1,397(0,5);0,000(0,4) |
| Verbindung Nr. II-1-59: ¹H-NMR(400,0 MHz, d₆-DMSO): 5,188(4,9);5,187(4,7);5,181(0,7);5,177(1,1); 5,174(1,3);5,170(1,0);5,167(0,5);5,164(0,4);5,160(0,6);5,157(0,7);5,154(0,5);4,351(4,4);4,333(3,1);3,32 8(8,6);2,510(4,5);2,506(9,0);2,501(11,7);2,497(8,4);2,492(3,9);2,072(16,0);2,070(15,0);1,693(9,7);1,673 (8,6);1,671(8,4);0,008(0,7);0,000(19,0);-0,009(0,6) |
| Verbindung Nr. II-1-60: ¹H-NMR(400,0 MHz, d₆-DMSO): 5,288(3,1);4,913(9,4);4,516(1,8);3,329(8,6); 2,510(3,2);2,506(6,4);2,501(8,5);2,497(6,2);2,493(3,1);2,139(11,4);2,138(11,5);2,099(16,0);0,000(6,7) |
| Verbindung Nr. II-1-61: ¹H-NMR(400,0 MHz, d₆-DMSO): 5,285(4,4);4,952(11,5);4,509(5,1);3,326(15, 5);2,616(1,5);2,597(4,6);2,579(4,8);2,560(1,7);2,502(18,6);2,155(0,6);2,140(16,0);1,178(4,9);1,160(9,6) ;1,142(4,8);0,000(9,9) |
| Verbindung Nr. II-1-62: ¹H-NMR(400,0 MHz, d₆-DMSO): 8,859(1,8);8,847(1,8);8,807(0,7);8,801(10,9) ;8,788(11,2);8,781(0,7);8,773(1,2);8,768(0,5);7,505(0,5);7,493(1,0);7,480(0,7);7,475(2,6);7,463(4,9);7, 451(2,5);7,446(0,7);6,312(0,4);6,223(10,8);5,861(1,0);5,504(0,5);5,426(16,0);5,409(0,6);4,808(4,3);2,52 5(0,4);2,520(0,7);2,511(13,9);2,507(29,0);2,502(38,7);2,498(28,7);2,493(14,5);0,008(1,1);0,000(40,3);-0,009(1,8) |
| Verbindung Nr. II-1-63: ¹H-NMR(400,0 MHz, d₆-DMSO): 7,821(4,3);5,248(3,8);5,165(9,1);4,505(4,1); 3,828(16,0);3,328(6,1);2,505(8,7);2,501(11,4);2,497(9,0);2,196(12,9);0,008(0,9);0,000(19,4) |
| Verbindung Nr. II-1-64: ¹H-NMR(400,0 MHz, d₆-DMSO): 5,755(0,4);5,512(0,8);5,495(2,7);5,478(2,7); 5,460(0,8);5,308(4,3);4,758(4,2);3,325(8,5);2,519(0,3);2,510(5,1);2,506(10,4);2,502(13,7);2,497(9,7);2, 492(4,6);2,142(16,0); 1,629(10,2); 1,611 (10,0);0,008(0,6);0,000(17,4);-0,009(0,6) |

Die in den Tabellen 1 bis 4 beschriebenen Verbindungen der Formeln (I-1), (I-2), (I-3) und (I-4) sind ebenfalls bevorzugte Verbindungen, die gemäß oder in Analogie zu den oben beschriebenen Synthesebeispielen erhalten wurden. In den Tabellen steht Me für Methyl und Et für Ethyl.

**Tabelle 1**

| | | | | | | | | | |
|---|---|---|---|---|---|---|---|---|---|
| Verbindungen der Formel (I-1) | | | | | | | | | |
| 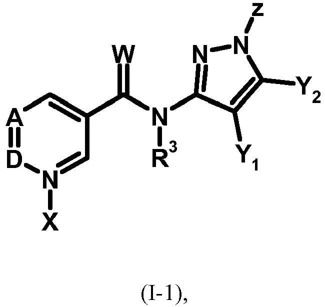 | | | | | | | | | |
| worin X, wenn nichts anderes angegeben ist, für ein freies Elektronenpaar steht und A, D, W, R³, Y₁, Y₂ und Z die in Tabelle 1 angegebene Bedeutung aufweisen. | | | | | | | | | |

| **Verbindung Nr.** | **X** | **D** | **A** | **R³** | **W** | **Y₁** | **Y₂** | **Z** | **log P (acid)¹** |
|---|---|---|---|---|---|---|---|---|---|
| I-1-1 | | C-H | C-H | H | O | H | Methyl | Pyrimidin-2-ylmethyl | 0,77 |
| I-1-10 | | C-H | C-H | H | O | H | H | 2,2,2-Trifluorethyl | 1,43 |
| I-1-100 | | C-Cl | C-Cl | H | O | H | Methyl | Benzyl | 3,18 |
| I-1-101 | | C-H | C-Br | H | O | H | Methyl | 2-Fluorbenzyl | 2,69 |
| I-1-102 | | C-Me | C-H | H | O | H | Methyl | 2-Fluorbenzyl | 1,73 |
| I-1-103 | | C-Cl | C-Cl | H | O | H | Methyl | 2-Fluorbenzyl | 3,28 |
| I-1-104 | | C-OMe | C-H | H | O | H | Methyl | Benzyl | 2,43 |
| I-1-105 | | C-OMe | C-H | H | O | H | Methyl | 2-Fluorbenzyl | 2,52 |
| I-1-106 | | C-Cl | C-H | H | O | H | Methyl | 3-Fluorbenzyl | 2,6 |
| I-1-107 | | C-H | C-Br | H | O | H | Methyl | 3-Fluorbenzyl | 2,71 |
| I-1-108 | | C-Me | C-H | H | O | H | Methyl | 3-Fluorbenzyl | 1,79 |
| I-1-109 | | C-Me | C-H | H | O | H | Methyl | 4-Fluorbenzyl | 1,76 |
| I-1-11 | | C-H | C-H | H | O | H | Methyl | Cyanomethyl | 0,7 |
| I-1-110 | | C-OMe | C-H | H | O | H | Methyl | 4-Fluorbenzyl | 2,47 |
| I-1-111 | | C-H | C-H | H | O | H | Difluormethoxy | Methyl | 0,89 |
| I-1-112 | | C-H | C-F | H | O | H | Difluormethoxy | Methyl | 1,56 |
| I-1-113 | | C-H | C-F | H | O | Brom | Difluormethoxy | Methyl | 1,67 |
| I-1-114 | | C-H | C-F | H | O | H | Methyl | Carboxymethyl | 0,87 |
| I-1-115 | | C-H | C-H | H | O | H | Methyl | Carboxymethyl | 0,09 |
| I-1-116 | | C-H | C-H | H | O | H | H | [3-Chlor-5-(trifluormethyl)pyridin-2-yl]methyl | 1,86 |
| I-1-117 | | C-H | C-H | H | O | H | H | 2-Cyanophenyl | 1,42 |
| I-1-118 | | C-Cl | C-H | H | O | H | H | [3-Chlor-5-(trifluormethyl)pyridin-2-yl]methyl | 2,64 |
| I-1-119 | | C-H | C-H | H | O | 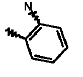 | | Methyl | 1,13 |
| I-1-12 | | C-H | C-H | H | O | H | Methyl | Isobutyl | 1,59 |
| I-1-120 | | C-H | C-H | H | O | H | Methyl | Acetyl | 1,18 |
| I-1-121 | | C-H | C-H | H | O | H | Methyl | Cyclopropylcarbonyl | 1,65 |
| I-1-122 | | C-H | C-H | H | O | H | Methyl | 2-Chlorbenzoyl | 2,27 |
| I-1-123 | | C-H | C-H | H | O | Methy l | Methyl | Pyrimidin-2-ylmethyl | 0,76 |
| I-1-124 | | C-H | C-H | H | O | H | Methyl | 2-(Methylamino)-2-oxoethyl | 0,29 |
| I-1-125 | | C-H | C-H | H | O | Brom | Methyl | Pyrimidin-2-ylmethyl | 0,98 |
| I-1-126 | | C-H | C-H | H | O | H | Methyl | Methoxycarbonyl | 1,08 |
| I-1-127 | | C-H | C-H | H | O | H | Methyl | 3-Chlorbenzoyl | 1,92 |
| I-1-128 | O | C-H | C-H | H | O | H | Methyl | Pyrimidin-2-ylmethyl | 0,55 |
| I-1-129 | | C-H | C-H | H | O | H | Methyl | Propionyl | 1,56 |
| I-1-13 | | C-H | C-H | H | O | H | H | 2,2-Difluorethyl | 0,74 |
| I-1-130 | | C-H | C-H | H | O | H | Methyl | Isobutyryl | 1,95 |
| I-1-131 | | C-H | C-H | H | O | H | Methyl | Butyryl | 1,94 |
| I-1-132 | | C-H | C-F | H | O | H | Methyl | 4,6-Dimethylpyrimidin-2-yl | 2,04 |
| I-1-133 | | C-H | C-H | H | O | H | Methyl | 2,2,2-Trifluorethyl | 1,32 |
| I-1-134 | | C-H | C-F | H | O | H | Methoxy - carbonyl | methyl | 1,5 |
| I-1-135 | | C-H | C-F | H | O | H | Methyl | 2,2,2-Trifluorethyl | 1,79 |
| I-1-136 | | C-H | C-H | H | O | Methy l | H | Pyrimidin-2-ylmethyl | 0,44 |
| I-1-137 | | C-H | C-H | H | O | Chlor | H | Pyrimidin-2-ylmethyl | 0,58 |
| I-1-138 | | C-H | C-H | H | O | H | Methoxy | Methyl | 1,05 |
| | | | | | | | carbonyl | | |
| I-1-139 | | C-H | C-H | H | O | H | Methyl | 4,6-Dimethylpyrimidin-2-yl | 1,6 |
| I-1-14 | | C-H | C-H | H | O | H | Methyl | 1,3-Thiazol-2-ylmethyl | 1,05 |
| I-1-140 | | C-H | C-H | H | O | H | Brom | Pyrimidin-2-ylmethyl | 1,1 |
| I-1-15 | | C-H | C-H | H | O | H | Methyl | Pyridin-3-ylmethyl | 0,12 |
| I-1-16 | | C-H | C-H | Methy 1 | O | H | Methyl | Methyl | 0,65 |
| I-1-17 | | C-H | C-H | H | O | H | Methyl | H | 0,3 |
| I-1-18 | | C-H | C-H | H | O | H | Methyl | tert-Butoxycarbonyl | 2,04 |
| I-1-19 | | C-H | C-H | H | O | H | Methyl | Prop-2-yn-1-yl | 0,93 |
| I-1-2 | | C-H | C-H | H | O | H | Methyl | Ethoxymethyl | 1,1 |
| I-1-20 | | C-H | C-H | H | O | H | Methyl | (3-Cyanopyridin-2-yl)methyl | 1,08 |
| I-1-21 | | C-H | C-H | H | O | H | methyl | (6-Chlorpyridin-3-yl)methyl | 1,42 |
| I-1-22 | | C-H | C-H | 3-Chlorbenzoyl | O | H | Methyl | 3-Chlorbenzoyl | 3,98 |
| I-1-23 | | C-H | C-H | H | O | H | Methyl | Pyrimidin-2-yl | 1,16 |
| I-1-24 | | C-H | C-H | H | O | H | Methyl | 2-Ethoxy-2-oxoethyl | 0,89 |
| I-1-25 | | C-H | C-H | Methy 1 | O | H | Methyl | Pyrimidin-2-yl | 1,02 |
| I-1-26 | | C-H | C-H | H | O | H | H | Pyridin-2-ylmethyl | 0,57 |
| I-1-27 | | C-H | C-H | H | O | H | H | (6-Chlorpyridin-3-yl)methyl | 1,22 |
| I-1-28 | | C-H | C-H | H | O | H | Methyl | Pyridin-3-ylcarbonyl | 1,57 |
| I-1-29 | | C-H | C-H | H | O | H | Cyclopropyl | (6-Chlorpyridin-3-yl)methyl | 1,77 |
| I-1-3 | | C-H | C-H | H | O | H | Methyl | Methyl | 0,63 |
| I-1-30 | | C-H | C-H | H | O | H | H | 3-Fluorpyridin-2-yl | 1,15 |
| I-1-31 | | C-H | C-H | H | O | H | Methyl | (4-Methylpyrimidin-2-yl)methyl | 0,96 |
| I-1-32 | | C-H | C-H | H | O | H | Methyl | 2-[(1-Methoxy-2-methyl-1-oxopropan-2-yl)amino]-2-oxoethyl | 0,72 |
| I-1-33 | | C-H | C-H | H | O | H | Methyl | 2-Chlorethyl | 1,16 |
| I-1-34 | | C-H | C-H | H | O | H | Methyl | 3-Chlorpyridin-2-yl | 1,35 |
| I-1-35 | | C-H | C-H | H | O | H | Ethyl | Benzyl | 2,03 |
| I-1-36 | | C-H | C-H | H | O | H | Methyl | (4,6-Dimethylpyrimidin-2-yl)methyl | 1,2 |
| I-1-37 | | C-H | C-H | H | O | H | Methyl | (3-Chlorpyridin-2-yl)methyl | 1,42 |
| I-1-38 | | C-H | C-H | H | O | H | Methyl | Ethyl | 0,93 |
| I-1-39 | | C-H | C-H | H | O | 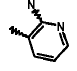 | | Methyl | 0,78 |
| I-1-4 | | C-H | C-H | H | O | H | Methyl | 2,2-Difluorethyl | 1,05 |
| I-1-40 | | C-H | C-H | H | O | H | Ethyl | Prop-2-yn-1-yl | 1,31 |
| I-1-41 | | C-H | C-F | H | O | H | Methyl | 2-[(1-Methoxy-2-methyl-1-oxopropan-2-yl)amino]-2-oxoethyl | 1,33 |
| I-1-42 | | C-H | C-H | 4-Chlorb enzoyl | O | H | Methyl | 4-Chlorbenzoyl | 4,18 |
| I-1-43 | | C-H | C-H | H | O | H | Cyclopropyl | Prop-2-yn-1-yl | 1,41 |
| I-1-44 | | C-H | C-H | H | O | H | Methyl | 3-Chlor-5-(trifluormethyl)pyridin-2-yl | 2,2 |
| I-1-45 | | C-H | C-H | H | O | H | Methyl | 5-(Trifluormethyl)pyridin-2-yl | 2,64 |
| I-1-46 | | C-H | C-H | H | O | H | Methyl | Benzyl | 1,79 |
| I-1-47 | | C-H | C-H | H | O | Brom | Difluormethoxy | Methyl | 1,13 |
| I-1-48 | | C-H | C-H | H | O | H | H | 3-Chlorpyridin-2-yl | 1,25 |
| I-1-49 | | C-H | C-H | H | O | H | Methyl | Isopropyl | 1,4 |
| I-1-5 | | C-H | C-H | H | O | H | Methyl | 2-Fluorethyl | 0,84 |
| I-1-50 | | C-H | C-H | H | O | H | Methyl | 3-Methylbenzyl | |
| I-1-51 | | C-H | C-H | H | O | H | Methyl | Pyridin-4-ylmethyl | 0,13 |
| I-1-52 | | C-H | C-H | H | O | H | H | Pyridin-2-yl | 1,32 |
| I-1-53 | | C-H | C-H | H | O | H | Methyl | (4,6-Dimethoxypyrimidin-2-yl)methyl | 1,59 |
| I-1-54 | | C-H | C-H | H | O | H | H | Prop-2-yn-1-yl | 0,69 |
| I-1-55 | | C-H | C-H | H | O | H | Methyl | Pyrazin-2-yl | 1,27 |
| I-1-56 | | C-H | C-H | H | O | H | Cyclopropyl | Benzyl | 2,1 |
| I-1-57 | | C-H | C-F | H | O | 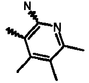 | | Methyl | 1,6 |
| I-1-58 | | C-H | C-H | H | O | H | Ethyl | (6-Chlorpyridin-3-yl)methyl | 1,67 |
| I-1-59 | | C-H | C-F | H | O | H | Methyl | 2-Ethoxy-2-oxoethyl | 1,53 |
| I-1-6 | | C-H | C-H | H | O | H | Methyl | Pyridin-2-ylmethyl | 0,88 |
| I-1-60 | | C-H | C-H | H | O | H | Methyl | 3-Fluorbenzyl | 1,9 |
| I-1-61 | | C-H | C-F | H | O | H | Methyl | 2-[(2-Ethoxy-2-oxoethyl)(methyl)amino]-2-oxoethyl | 1,35 |
| I-1-62 | | C-H | C-H | H | O | H | Methyl | 4-Fluorbenzyl | 1,88 |
| I-1-63 | | C-H | C-H | H | O | H | H | 3-Chlor-5-(trifluormethyl)pyridin-2-yl | 2,18 |
| I-1-64 | | C-H | C-H | H | O | H | Methyl | 2-Fluorbenzyl | 1,82 |
| I-1-65 | | C-H | C-H | H | O | H | H | 1,3-Difluorpropan-2-yl | 0,84 |
| I-1-66 | | C-H | N | H | O | H | Methyl | Benzyl | 1,76 |
| I-1-67 | | C-H | C-H | H | O | H | Methyl | 4-Methoxy-4-oxobutyl | |
| I-1-68 | | C-H | C-H | H | O | H | H | 2,6-Difluorbenzyl | 1,55 |
| I-1-69 | | C-H | C-H | H | O | H | H | 2-Ethoxy-2-oxoethyl | |
| I-1-7 | | C-H | C-F | H | O | H | Methyl | Pyrimidin-2-ylmethyl | 1,11 |
| I-1-70 | | C-H | C-H | H | O | H | H | 3-Fluorbenzyl | 1,59 |
| I-1-71 | | C-H | C-H | H | O | H | H | 3-(Trifluormethyl)benzyl | 2,07 |
| I-1-72 | | C-H | C-H | H | O | H | H | 3-Methylbenzyl | 1,83 |
| I-1-73 | | C-H | C-H | H | O | H | H | 3-(Trifluormethoxy)benzyl | 2,20 |
| I-1-74 | | C-H | C-H | H | O | H | H | 3-Methoxybenzyl | 1,57 |
| I-1-75 | | C-H | C-H | H | O | H | H | 4-tert-Butylbenzyl | 2,68 |
| I-1-76 | | C-H | C-H | H | O | H | H | 2,4-Difluorbenzyl | 1,68 |
| I-1-77 | | C-H | C-H | H | O | H | H | 3-(Methoxycarbonyl)benzyl | 1,57 |
| I-1-78 | | C-H | C-H | H | O | H | H | 4-Methoxybenzyl | 1,55 |
| I-1-79 | | N | C-H | H | O | H | Methyl | Benzyl | 1,67 |
| I-1-8 | | C-H | C-H | H | O | H | Methyl | Difluormethyl | 1,19 |
| I-1-80 | | C-Me | C-H | H | O | H | Methyl | Benzyl | 1,71 |
| I-1-81 | | C-H | C-H | H | O | H | H | 2,5-Dimethylbenzyl | 2,11 |
| I-1-82 | | C-H | C-H | H | O | H | H | 2,4-Dichlorbenzyl | |
| I-1-83 | | C-H | C-Br | H | O | H | Methyl | 4-Fluorbenzyl | 2,67 |
| I-1-84 | | C-H | C-H | H | O | H | H | Benzyl | 1,52 |
| I-1-85 | | C-H | C-H | H | O | H | H | 2,4-Difluorphenyl | |
| I-1-86 | | C-Cl | C-H | H | O | H | H | 4-tert-Butylbenzyl | 3,54 |
| I-1-87 | | C-Cl | C-H | H | O | H | H | 3-(Trifluormethyl)benzyl | 2,82 |
| I-1-88 | | C-H | C-H | H | O | H | H | 2-Fluorbenzyl | 1,58 |
| I-1-89 | | C-H | C-H | H | O | H | H | 2-Cyanobenzyl | 1,37 |
| I-1-9 | | C-H | C-H | H | O | H | H | Pyrimidin-2-ylmethyl | 0,42 |
| I-1-90 | | C-H | C-H | H | O | H | H | 2,5-Difluorbenzyl | 1,65 |
| I-1-91 | | C-H | C-H | H | O | H | H | 2-(Trifluormethyl)benzyl | 2,09 |
| I-1-92 | | C-H | C-H | H | O | H | H | 4-(Ethoxycarbonyl)benzyl | 1,85 |
| I-1-93 | | C-Cl | C-H | H | O | H | Methyl | 2-Fluorbenzyl | 2,59 |
| I-1-94 | | C-OMe | C-H | H | O | H | Methyl | 3-Fluorbenzyl | 2,51 |
| I-1-95 | | C-Cl | C-H | H | O | H | Methyl | 4-Fluorbenzyl | 2,57 |
| I-1-96 | | C-Cl | C-Cl | H | O | H | Methyl | 3-Fluorbenzyl | 3,26 |
| I-1-97 | | C-Cl | C-Cl | H | O | H | Methyl | 4-Fluorbenzyl | 3,23 |
| I-1-98 | | C-Cl | C-H | H | O | H | Methyl | Benzyl | 2,48 |
| I-1-99 | | C-H | C-Br | H | O | H | Methyl | Benzyl | 2,58 |
| I-1-141 | | C-H | C-H | H | O | CN | SOMe | Me | 0.06 |
| I-1-142 | | C-H | C-H | H | O | H | Me | [(2E)-2-(Cyanoimino)-1,3-thiazolidin-3-yl]methyl | 1.08 |
| I-1-143 | | C-H | C-H | H | O | H | Me | (3,4-Dimethyl-5-oxo-4,5-dihydro-1H-1,2,4-triazol-1-yl)methyl | 0.72 |
| I-1-144 | | C-H | C-H | H | O | H | Me | (4-Cyclopropyl-3-methoxy-5-oxo-4,5-dihydro-1H-1,2,4-triazol-1-yl)methyl | 1.27 |
| I-1-145 | | C-H | C-H | H | O | H | Me | (2,4-Dimethyl-3,5-dioxo-1,2,4-triazolidin-1-yl)methyl | 0.82 |
| I-1-146 | | C-H | C-H | H | O | H | Me | (4-Cyclopropyl-3-methyl-5-oxo-4,5-dihydro-1H-1,2,4-triazol-1-yl)methyl | 1.05 |
| I-1-147 | | C-H | C-H | H | O | H | Me | (3,4-Dicyclopropyl-5-oxo-4,5-dihydro-1H-1,2,4-triazol-1-yl)methyl | 1.45 |
| I-1-148 | | C-H | C-H | H | O | H | Me | (1,3-Dimethyl-5-oxo-1,5-dihydro-4H-1,2,4-triazol-4-yl)methyl | 0.74 |
| I-1-149 | | C-H | C-H | H | O | H | Me | (2-Oxotetrahydropyrimidin-1(2H)-yl)methyl | 0.62 |
| I-1-150 | | N | C-H | H | O | H | Me | (3,4-Dicyclopropyl-5-oxo-4,5-dihydro-1H-1,2,4-triazol-1-yl)methyl | 1.33 |
| I-1-151 | | N | C-H | H | O | H | Me | (4-Cyclopropyl-3-methyl-5-oxo-4,5-dihydro-1H-1,2,4-triazol-1-yl)methyl | 0.92 |
| I-1-152 | | N | C-H | H | O | H | Me | (4-Cyclopropyl-3-methoxy-5-oxo-4,5-dihydro-1H-1,2,4-triazol-1-yl)methyl | 1.17 |
| I-1-153 | | C-H | C-H | H | O | H | Me | (1,4-Dimethyl-5-oxo-4,5-dihydro-1H-1,2,4-triazol-3-yl)methyl | 0.62 |
| I-1-154 | | C-H | C-H | H | O | H | Me | (4-Methyl-2-oxo-2H-pyran-6-yl)methyl | |
| I-1-155 | | C-H | C-H | H | O | H | Me | [6-(Trifluormethyl)imidazo[1, 2-a]pyridin-2-yl]methyl | 1.54 |
| I-1-156 | | C-H | C-H | H | O | H | Me | [5-Carboxy-1-(3-chlorpyridin-2-yl)-1H-pyrazol-3-yl]methyl | 1.18 |
| I-1-157 | | C-H | C-H | H | O | H | Me | 2,3-Dihydro-1,4-benzodioxin-2-ylmethyl | 1.99 |
| I-1-158 | | C-H | C-H | H | O | H | Me | 5,6,7,8-Tetrahydronaphthalen-2-ylmethyl | 2.73 |
| I-1-159 | | C-H | C-H | H | O | H | Me | (6-Fluorimidazo[1,2-a]pyridin-2-yl)methyl | 0.66 |
| I-1-160 | | C-H | C-H | H | O | H | Me | (6-Chlorimidazo[1,2-a]pyridin-2-yl)methyl | 1.03 |
| I-1-161 | | C-H | C-H | H | O | H | Me | Pyrrolidin-1-ylcarbonyl | 1.29 |
| I-1-162 | | C-H | C-H | H | O | H | Me | (4-Methylpiperidin-1-yl)sulfonyl | 2.51 |
| I-1-163 | | C-H | C-H | H | O | H | Me | (2-Methoxyethyl)carbamoyl | 1.22 |
| I-1-164 | | C-H | C-H | H | O | H | Me | [(2,2-Dimethyl-1,3-dioxolan-4-yl)methyl]carbamoyl | 1.56 |
| I-1-165 | | C-H | C-H | H | O | H | Me | [3-(Methylsulfanyl)propyl]car bamoyl | 1.7 |
| I-1-166 | | C-H | C-H | H | O | H | Me | (1-Methoxypropan-2-yl)carbamoyl | 1.44 |
| I-1-167 | | C-H | C-H | H | O | H | Me | [2-(Methylsulfanyl)ethyl]carb amoyl | 1.55 |
| I-1-168 | | C-H | C-H | H | O | H | Me | (2-Phenoxyethyl)carbamoyl | 2.18 |
| I-1-169 | | C-H | C-H | H | O | H | Me | Bis(2-methoxyethyl)carbamoyl | 1.91 |
| I-1-170 | | C-H | C-H | H | O | H | Me | (2,2-Diethoxyethyl)carbamoyl | 1.88 |
| I-1-171 | | C-H | C-H | H | O | H | Me | [2-(2,6-Difluorphenoxy)ethyl]carb amoyl | 2.21 |
| I-1-172 | | C-H | C-H | H | O | H | Me | [2-(Trifluormethoxy)ethyl]car bamoyl | 1.91 |
| I-1-173 | | C-H | C-H | H | O | H | Me | 2-Methoxyethyl | 0.79 |
| I-1-174 | | C-H | C-H | H | O | H | Me | Cyclopropylmethyl | 1.31 |
| I-1-175 | | C-H | C-H | H | O | H | Me | CH₂OCH₃ | 0.71 |
| I-1-176 | | C-H | C-H | H | O | H | Me | Tetrahydrofuran-2-ylmethyl | 1.06 |
| I-1-177 | | C-H | C-H | Me | O | H | Me | Cyclopropylmethyl | 1.27 |
| I-1-178 | | C-H | C-H | Me | O | H | Me | CH₂OCH₃ | 0.7 |
| I-1-179 | | C-H | C-H | Me | O | H | Me | 2-Methoxyethyl | 0.79 |
| I-1-180 | | C-H | C-H | H | O | H | Me | 1,3-Dioxolan-2-ylmethyl | 0.81 |
| I-1-181 | | C-H | C-H | Me | O | H | Me | Tetrahydrofuran-2-ylmethyl | 1.05 |
| I-1-182 | | C-H | C-H | H | O | H | Me | 2-(Methylsulfonyl)ethyl | 0.49 |
| I-1-183 | | C-H | C-H | H | O | H | Me | 2-(Methylsulfinyl)ethyl | 0.25 |
| I-1-184 | | C-H | C-H | H | O | H | Me | 2-(Methylsulfanyl)ethyl | 1.22 |
| I-1-185 | | C-H | C-H | H | O | H | Me | 2-Cyanoethyl | 0.57 |
| I-1-186 | | C-H | C-H | Me | O | H | Me | 2-(Methylsulfinyl)ethyl | 0.16 |
| I-1-187 | | C-H | C-H | Me | O | H | Me | 2-(Methylsulfanyl)ethyl | 1.17 |
| I-1-188 | | C-H | C-H | H | O | H | Me | 3,3,3-Trifluorpropyl | 1.45 |
| I-1-189 | | C-H | C-H | H | O | H | Me | 2-(Ethylsulfanyl)ethyl | 1.53 |
| I-1-190 | | C-H | C-H | H | O | H | Me | 2-(Ethylsulfinyl)ethyl | 0.46 |
| I-1-191 | | C-H | C-H | H | O | H | Me | 2-[(Methoxycarbonyl)oxy] et hyl | 0.98 |
| I-1-192 | | C-H | C-H | H | O | H | Me | (3-Methyl-1H-pyrazol-1-yl)methyl | 1.16 |
| I-1-193 | | C-H | C-H | H | O | H | Me | 4-Methylphenyl | 2.02 |
| I-1-194 | | C-H | C-H | H | O | CN | Me | Pyrimidin-2-ylmethyl | 0.63 |
| I-1-195 | | C-H | C-H | H | O | H | Me | (5-Chlorpyrimidin-2-yl)methyl | 1.23 |
| I-1-196 | | C-H | C-H | H | O | H | CF₃ | Pyrimidin-2-ylmethyl | 1.46 |
| I-1-197 | | C-H | C-H | H | O | H | Cl | Pyrimidin-2-ylmethyl | 1.04 |
| I-1-198 | | C-H | C-H | H | O | H | H | CH₃ | 0.22 |
| I-1-199 | | C-H | C-H | H | O | Me | H | CH₂CN | 0.37 |
| I-1-200 | | C-H | C-H | H | O | H | Me | 4-Methoxyphenyl | 1.34 |
| I-1-201 | | C-H | C-H | H | O | Cl | SMe | CH₃ | 1.25 |
| I-1-202 | | C-H | C-H | H | O | H | Me | 3-Fluorphenyl | 1.85 |
| I-1-203 | | C-H | C-H | H | O | H | Me | 4-Fluorphenyl | 1.79 |
| I-1-204 | | C-H | C-H | H | O | H | Me | Cyclohexyl | 2.02 |
| I-1-205 | | C-H | C-H | H | O | H | Me | s-C₄H₉ | 1.59 |
| I-1-206 | | C-H | C-H | H | O | H | Me | 3,4-Dichlorphenyl | 2.59 |
| I-1-207 | | C-H | C-H | H | O | H | Me | 2,4-Dichlorphenyl | 2.36 |
| I-1-208 | | C-H | C-H | H | O | H | Me | 3,5-Dimethylphenyl | 2.4 |
| I-1-209 | | C-H | C-H | H | O | H | Me | 4-Chlorphenyl | 2.09 |
| I-1-210 | | C-H | C-H | H | O | H | Me | Pentan-3-yl | 1.92 |
| I-1-211 | | C-H | C-H | H | O | F | Me | Pyrimidin-2-ylmethyl | 0.72 |
| I-1-212 | | C-H | C-H | H | O | Cl | Me | Pyrimidin-2-ylmethyl | 0.94 |
| I-1-213 | | C-H | C-H | H | O | H | Me | 2-Chlorphenyl | 1.86 |
| I-1-214 | | C-H | C-H | H | O | H | Me | 1-Cyanoethyl | 0.92 |
| I-1-215 | | C-H | C-H | H | O | 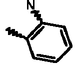 | | Pyrimidin-2-ylmethyl | 1.16 |
| I-1-216 | | C-H | C-F | H | O | 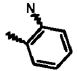 | | Pyrimidin-2-ylmethyl | 1.54 |
| I-1-217 | | C-H | C-H | H | O | H | Me | 3-Cyanophenyl | 1.59 |
| I-1-218 | | C-H | C-H | H | O | H | Me | 1-Cyclopropylethyl | 1.63 |
| I-1-219 | | C-H | C-H | H | O | H | Me | 2-Cyanopropan-2-yl | 1.31 |
| I-1-220 | | C-H | C-H | H | O | H | Me | (5-Methyl-1H-pyrazol-1-yl)methyl | 1.17 |
| I-1-221 | | C-H | C-H | H | O | H | CHF₂ | CH₃ | 1.04 |
| I-1-222 | | C-H | C-F | H | O | H | CHF₂ | CH₃ | 1.51 |
| I-1-223 | | C-H | C-H | H | O | H | Me | 1H-1,2,3-Triazol-1-ylmethyl | 0.65 |
| I-1-224 | | C-H | C-H | H | O | H | Me | 1H-1,2,4-Triazol-1-ylmethyl | 0.58 |
| I-1-225 | | C-H | C-H | H | O | H | Me | 1H-Pyrazol-1-ylmethyl | 0.94 |
| I-1-226 | | C-H | C-H | H | O | H | Me | (4-Methyl-1H-pyrazol-1-yl)methyl | 1.24 |
| I-1-227 | | C-H | C-H | H | O | H | Me | [5-(Trifluormethyl)-1H-pyrazol-1-yl]methyl | 1.72 |
| I-1-228 | | C-H | C-H | H | O | H | Me | [3-(Trifluormethyl)-1H-pyrazol-1-yl]methyl | 1.86 |
| I-1-229 | | C-H | C-H | H | O | H | Me | Cyclopentyl | 1.74 |
| I-1-230 | | C-H | C-H | H | O | H | Me | 2-Pyridyl | 2.19 |
| I-1-231 | | C-H | C-H | H | O | H | Me | SO₂N(CH₃)₂ | 1.52 |
| I-1-232 | | C-H | C-H | H | (2,4-Dichlorphe nyl)imino | H | Me | CH₃ | 1.77 |
| I-1-233 | | C-H | C-H | H | O | H | Me | CO₂Phenyl | 2 |
| I-1-234 | | C-H | C-H | H | O | H | Me | (Pyrimidin-2-ylsulfanyl)methyl | 1.25 |
| I-1-235 | | C-H | C-H | H | O | H | Me | (Pyrimidin-2-ylsulfinyl)methyl | 0.5 |
| I-1-236 | | C-H | C-H | H | O | H | Me | (Pyrimidin-2-ylsulfonyl)methyl | 0.74 |
| I-1-237 | | C-H | C-H | H | O | H | Me | (2,2-Dichlorcyclopropyl)methyl | 1.84 |
| I-1-238 | | C-H | C-H | H | O | H | Me | 3,3-Dichlorprop-2-en-1-yl | 1.83 |
| I-1-239 | | C-H | C-H | H | O | H | Me | 3-Methylbut-2-en-1-yl | 1.75 |
| I-1-240 | | N | C-H | H | O | H | Me | 1H-Pyrazol-1-ylmethyl | 0.91 |
| I-1-241 | | C-H | C-H | H | O | H | Me | (Methylsulfanyl)methyl | 1.17 |
| I-1-242 | | C-H | C-H | H | O | H | Me | (Ethylsulfanyl)methyl | 1.49 |
| I-1-243 | | C-H | C-H | H | O | H | Me | (Methylsulfinyl)methyl | 0.19 |
| I-1-244 | | C-H | C-H | H | O | H | Me | (Methylsulfonyl)methyl | 0.59 |
| I-1-245 | | C-H | C-H | H | O | H | Me | (Ethylsulfinyl)methyl | 0.45 |
| I-1-246 | | C-H | C-H | H | O | H | Me | (Ethylsulfonyl)methyl | 0.83 |
| I-1-247 | | C-H | C-H | H | O | H | Me | 3-(2-Chlorphenyl)-2-oxopropyl | 1.92 |
| I-1-248 | | N | C-H | H | O | H | Me | 4,6-Dimethylpyrimidin-2-yl | 1.53 |
| I-1-249 | | N | C-H | H | O | H | Me | 2-Pyridyl | 1.83 |
| I-1-250 | | N | C-H | H | O | H | Me | 2,2,2-Trifluorethyl | 1.2 |
| I-1-251 | | N | C-H | H | O | H | Me | (4,6-Dimethylpyrimidin-2-yl)methyl | 1.14 |
| I-1-252 | | N | C-H | H | O | H | Me | (4,6-Dimethoxypyrimidin-2-yl)methyl | 1.5 |
| I-1-253 | | N | C-H | H | O | H | Me | (4-Methylpyrimidin-2-yl)methyl | 0.88 |
| I-1-254 | | N | C-H | H | O | H | Me | (3-Chlorpyridin-2-yl)methyl | 1.33 |
| I-1-255 | | N | C-H | H | O | H | Me | [3-(Trifluormethyl)-1H-pyrazol-1-yl]methyl | 1.73 |
| I-1-256 | | N | C-H | H | O | H | Me | (4-Methyl-1H-pyrazol-1-yl)methyl | 1.1 |
| I-1-257 | | C-H | C-H | H | O | H | Me | Methylsulfamoyl | 0.55 |
| I-1-258 | | C-H | C-H | H | O | H | Me | Propylsulfamoyl | 1.07 |
| I-1-259 | | C-H | C-H | H | O | H | Me | (2,2,2-Trifluorethoxy)carbonyl | 1.79 |
| I-1-260 | | C-H | C-H | H | O | H | Me | 2-(1-Chlorcyclopropyl)-2-oxoethyl | 1.38 |
| I-1-261 | | C-H | C-H | H | O | H | Me | Methoxyacetyl | 1.17 |
| I-1-262 | | C-H | C-H | Cyano methyl | O | H | Me | Pyrimidin-2-ylmethyl | 0.84 |
| I-1-263 | | C-H | C-H | H | O | H | Me | Methyl(phenylsulfonyl)car bamoyl | 2.13 |
| I-1-264 | | C-H | C-H | Prop-2-yn-1-yl | O | H | Me | Pyrimidin-2-ylmethyl | 0.98 |
| I-1-265 | | C-H | C-H | 2,2-Difluo rethyl | O | H | Me | Pyrimidin-2-ylmethyl | 1.19 |
| I-1-266 | | C-H | C-H | H | O | H | Me | 3-(Methylsulfanyl)propanoyl | 1.87 |
| I-1-267 | | C-H | C-H | H | O | F | H | Pyrimidin-2-ylmethyl | 0.42 |
| I-1-268 | | C-H | C-H | H | O | H | Me | 3-(Methylsulfonyl)propanoyl | 1.04 |
| I-1-269 | | C-H | C-H | H | O | H | CN | Pyrimidin-2-ylmethyl | 1.0 |
| I-1-270 | | C-H | C-H | H | O | CN | SMe | CH₃ | 0.96 |
| I-1-271 | | C-H | C-H | H | O | H | H | 4-Methoxyphenyl | 1.6 |
| I-1-272 | | C-H | C-H | H | O | COO Et | H | Benzyl | 0.79 |
| I-1-273 | | C-H | C-H | H | O | H | H | 1,3-Thiazol-2-yl | 1.16 |
| I-1-274 | | C-H | C-H | H | O | H | Cl | Phenyl | 2.1 |
| I-1-275 | | C-H | C-H | H | O | Cl | OCHF₂ | CH₃ | 1.19 |
| I-1-276 | | C-H | C-Cl | H | O | H | Me | Pyrimidin-2-ylmethyl | 1.4 |
| I-1-277 | | C-H | C-H | H | O | H | Me | SO₂CH₃ | 1.03 |
| I-1-278 | | C-H | C-H | H | O | H | Me | Ethylsulfonyl | 1.29 |
| I-1-279 | | C-H | C-H | H | O | H | Me | Pyridin-2-ylsulfonyl | 1.51 |
| I-1-280 | | C-H | C-H | H | O | H | Me | Pyridin-3-ylsulfonyl | 1.45 |
| I-1-281 | | C-H | C-H | H | O | H | Me | (5-tert-Butyl-1,3,4-oxadiazol-2-yl)methyl | 1.44 |
| I-1-282 | | C-H | C-H | H | O | H | Me | (5-Methyl-1,2,4-oxadiazol-3-yl)methyl | 0.9 |
| I-1-283 | | C-H | C-H | H | O | H | Me | (5-Methyl-1,2-oxazol-3-yl)methyl | 1.19 |
| I-1-284 | | C-H | C-H | H | O | H | Me | (3-Methyl-1,2,4-oxadiazol-5-yl)methyl | 0.92 |
| I-1-285 | | C-H | C-H | H | O | H | Me | (5-tert-Butyl-1,2,4-oxadiazol-3-yl)methyl | 1.71 |
| I-1-286 | | C-H | C-H | H | O | H | Me | [5-(Dimethylcarbamoyl)-1,2,4-oxadiazol-3-yl]methyl | 1.03 |
| I-1-287 | | C-H | C-H | H | O | H | Me | Isopropylcarbamoyl | 1.46 |
| I-1-288 | | C-H | C-H | H | O | H | Me | Ethylcarbamoyl | 1.24 |
| I-1-289 | | C-H | C-H | H | O | H | Me | (Ethylsulfonyl)(methyl)car bamoyl | 1.5 |
| I-1-290 | | C-H | C-H | H | O | H | Me | Diethylcarbamoyl | 2.31 |
| I-1-291 | | C-H | C-H | H | O | H | Me | Dimethylcarbamoyl | 0.86 |
| I-1-292 | | C-H | C-H | H | O | H | Me | CONHCH₃ | 0.97 |
| I-1-293 | | C-H | C-H | H | O | H | Me | (1-Methyl-1H-imidazol-2-yl)methyl | -0.21 |
| I-1-294 | | C-H | C-H | H | O | H | Me | 2,2-Difluor-2-(5-fluorpyridin-2-yl)ethyl | 1.77 |
| I-1-295 | | C-H | C-H | H | O | H | Me | 1,3-Thiazol-4-ylmethyl | 0.95 |
| I-1-296 | | C-H | C-H | H | O | H | Me | (5-Methyl-1,3,4-thiadiazol-2-yl)methyl | 0.83 |
| I-1-297 | | C-H | C-H | H | O | H | Me | (5-Chlor-2-thienyl)methyl | 2.07 |
| I-1-298 | | C-H | C-H | H | O | H | Me | [3-(Dimethylcarbamoyl)-1,2-oxazol-5-yl]methyl | 1.09 |
| I-1-299 | | C-H | C-H | H | O | H | Me | (5-Phenyl-1,3,4-oxadiazol-2-yl)methyl | 1.57 |
| I-1-300 | | C-H | C-H | H | O | H | Me | (3-Phenyl-1,2,4-oxadiazol-5-yl)methyl | 2.01 |
| I-1-301 | | C-H | C-H | H | O | H | Me | (5,6-Dichlorpyridin-2-yl)methyl | 2.06 |
| I-1-302 | | C-H | C-H | H | O | H | Me | [2-(Trifluormethyl)-1,3-thiazol-4-yl]methyl | 1.93 |
| I-1-303 | | C-H | C-H | H | O | H | Me | (5-Methyl-2-phenyl-2H-1,2,3-triazol-4-yl)methyl | 2.33 |
| I-1-304 | | C-H | C-H | H | O | H | Me | (1-Phenyl-1H-1,2,4-triazol-5-yl)methyl | 1.32 |
| I-1-305 | | C-H | C-H | (3-Phenyl-1,2,4-oxadiazol-5-yl)methyl | O | H | Me | (3-Phenyl-1,2,4-oxadiazol-5-yl)methyl | 3.26 |
| I-1-306 | | C-H | C-H | H | O | H | Me | (Z)-2-(5-Chlorpyridin-2-yl)-2-fluorvinyl | 2.31 |
| I-1-307 | | C-H | C-H | H | O | H | Me | (1,1,1-Trifluorpropan-2-yl)carbamoyl | 1.89 |
| I-1-308 | | C-H | C-H | H | O | H | Me | [2-(Methylsulfinyl)ethyl]carbamoyl | 0.84 |
| I-1-309 | | C-H | C-H | H | O | H | Me | [2-(Methylsulfonyl)ethyl]carb amoyl | 0.97 |
| I-1-310 | | C-H | C-H | H | O | H | Me | (1,4-Dioxan-2-ylmethyl)carbamoyl | 1.2 |
| I-1-311 | | C-H | C-H | H | O | H | Me | (Tetrahydrofuran-2-ylmethyl)carbamoyl | 1.45 |
| I-1-312 | | C-H | C-H | H | O | H | Me | Cyclopropylcarbamoyl | 1.27 |
| I-1-313 | | C-H | C-H | H | O | H | Me | (Tetrahydro-2H-pyran-2-ylmethyl)carbamoyl | 1.78 |
| I-1-314 | | C-H | C-H | H | O | H | Me | Propylcarbamoyl | 1.56 |
| I-1-315 | | C-H | C-H | H | O | H | Me | Butylcarbamoyl | 1.92 |
| I-1-316 | | C-H | C-H | H | O | H | Me | (Cyclopropylmethyl)carba moyl | 1.65 |
| I-1-317 | | C-H | C-H | H | O | H | Me | sec-Butylcarbamoyl | 1.83 |
| I-1-318 | | C-H | C-H | H | O | H | Me | n-Pentan-2-ylcarbamoyl | 2.18 |
| I-1-319 | | C-H | C-H | H | O | H | Me | tert-Butylcarbamoyl | 1.99 |
| I-1-320 | | C-H | C-H | H | O | H | Me | Isobutylcarbamoyl | 1.89 |
| I-1-321 | | C-H | C-H | H | O | H | Me | (2,2-Dimethylpropyl)carbamoyl | 2.19 |
| I-1-322 | | C-H | C-H | H | O | H | Me | Benzylcarbamoyl | 1.97 |
| I-1-323 | | C-H | C-H | H | O | H | Me | (2-Chlorbenzyl)carbamoyl | 2.29 |
| I-1-324 | | C-H | C-H | H | O | H | Me | (3-Chlorbenzyl)carbamoyl | 2.39 |
| I-1-325 | | C-H | C-H | H | O | H | Me | (4-Chlorbenzyl)carbamoyl | 2.34 |
| I-1-326 | | C-H | C-H | H | O | H | Me | (2-Fluorbenzyl)carbamoyl | 2.04 |
| I-1-327 | | C-H | C-H | H | O | H | Me | (3-Fluorbenzyl)carbamoyl | 2.1 |
| I-1-328 | | C-H | C-H | H | O | H | Me | (4-Fluorbenzyl)carbamoyl | 2.12 |
| I-1-329 | | C-H | C-H | H | O | H | Me | (Pyridin-2-ylmethyl)carbamoyl | 0.88 |
| I-1-330 | | C-H | C-H | H | O | H | Me | (Pyridin-3-ylmethyl)carbamoyl | 0.64 |
| I-1-331 | | C-H | C-H | H | O | H | Me | (1-Phenylethyl)carbamoyl | 2.22 |
| I-1-332 | | C-H | C-H | H | O | H | Me | [1-(4-Fluorphenyl)ethyl]carbamo yl | 2.28 |
| I-1-333 | | C-H | C-H | H | O | H | Me | [1-(Pyrimidin-2-yl)ethyl]carbamoyl | 1.29 |
| I-1-334 | | C-H | C-H | H | O | H | Me | (Pyrimidin-2-ylmethyl)carbamoyl | 0.95 |
| I-1-335 | | C-H | C-H | H | O | H | Me | [2-(Pyrimidin-2-yl)propan-2-yl]carbamoyl | 1.67 |
| I-1-336 | | C-H | C-H | H | O | H | Me | (Pyrimidin-4-ylmethyl)carbamoyl | 1.05 |
| I-1-337 | | C-H | C-H | H | O | H | Me | 2-Fluor-4-methyl-5-[(2,2,2-trifluorethyl)sulfanyl]benzy l | 2.89 |
| I-1-338 | | C-H | C-H | H | O | H | Me | [(4,6-Dimethylpyrimidin-2-yl)methyl]carbamoyl | 1.43 |
| I-1-339 | | C-H | C-H | H | O | H | Me | 2,1,3-Benzothiadiazol-5-ylmethyl | 1.61 |
| I-1-340 | | C-H | C-H | H | O | H | Me | (5-Chlor-1,3-benzoxazol-2-yl)methyl | 2 |
| I-1-341 | | C-H | C-H | H | O | H | Me | Quinolin-2-ylmethyl | 1.66 |
| I-1-342 | | C-H | C-H | H | O | H | Me | (6-Chlor-1,3-benzoxazol-2-yl)methyl | 1.82 |
| I-1-343 | | C-H | C-H | 2,1,3-Benzothi adiazol-5-ylmethyl | O | H | Me | 2,1,3-Benzothiadiazol-5-ylmethyl | 2.57 |
| I-1-344 | | C-H | C-H | H | S | H | Me | Pyrimidin-2-ylmethyl | 1.24 |
| I-1-345 | | C-H | C-H | H | O | H | Me | Pyrimidin-2-ylcarbonyl | 1.11 |
| I-1-346 | | C-H | C-H | Me | O | H | Me | Pyrimidin-2-ylmethyl | 0.63 |
| I-1-347 | | C-H | C-H | H | O | H | Me | t-Butyl | 1.61 |
| I-1-348 | | C-H | C-F | H | O | H | Me | t-Butyl | 2.13 |
| I-1-349 | | C-H | C-H | H | O | H | Me | Phenyl | 1.7 |
| I-1-350 | | C-H | C-F | H | O | H | Me | Phenyl | 2.18 |
| I-1-351 | | C-H | C-H | H | O | H | Me | (6-Chlorimidazo[1,2-b]pyridazin-2-yl)methyl | 1.42 |
| I-1-352 | | C-H | C-H | H | O | H | Me | (2-Ethyl-5-oxo-5H-[1,3,4]thiadiazolo[3,2-a]pyrimidin-7-yl)methyl | 1.23 |
| I-1-353 | | C-H | C-H | H | O | H | Me | (5-Methyl-1,3-benzoxazol-2-yl)methyl | 1.9 |

**Tabelle 2**

| | | | | | | | |
|---|---|---|---|---|---|---|---|
| Verbindungen der Formel (I-2) | | | | | | | |
| 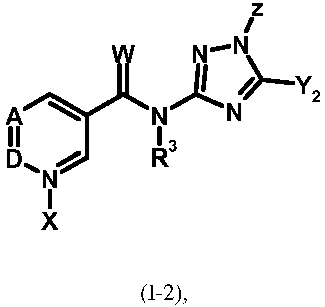 | | | | | | | |
| worin X für ein freies Elektronenpaar steht und A, D, W, R³, Y₂ und Z die in Tabelle 2 angegebene Bedeutung aufweisen. | | | | | | | |

| **Verbindung Nr.** | **D** | **A** | **R³** | **W** | **Y₂** | **Z** | **log P (acid)¹** |
|---|---|---|---|---|---|---|---|
| I-2-1 | C-H | C-H | H | O | Methyl | H | < -0.23 |
| I-2-2 | C-H | C-H | H | O | Methyl | Pyrimidin-2-ylmethyl | -0,05 |
| I-2-3 | C-H | C-H | H | O | H | 2,6-Dichlor-4-[(trifluormethyl)sulfonyl]phenyl | 2,02 |
| I-2-4 | C-H | C-H | H | O | H | 2-Methoxy-5-nitrobenzyl | 1,13 |
| I-2-5 | C-H | C-H | H | O | H | Phenyl | 0,98 |
| I-2-6 | C-H | C-H | H | O | Ethyl | Phenyl | 1,24 |
| I-2-7 | C-H | C-H | H | O | H | 2-Chlorbenzyl | 1,22 |
| I-2-8 | C-H | C-H | H | O | H | 2,6-Dichlorbenzyl | 1,39 |
| I-2-9 | C-H | C-H | H | O | H | Dimethylcarbamoyl | 0,47 |
| I-2-10 | C-H | C-H | H | O | H | 4,6-Diethoxy-1,3,5-triazin-2-yl | 1,41 |
| I-2-11 | C-H | C-H | H | O | H | 4-Methoxypyrimidin-2-yl | 0,70 |
| I-2-12 | C-H | C-H | H | O | SCH₃ | Ethyl | 0,71 |
| I-2-13 | C-H | C-H | H | O | H | 4-Chlorbenzyl | 1,36 |
| I-2-14 | C-H | C-H | H | O | H | Methyl | <-0,23 |
| I-2-15 | C-H | C-H | H | O | H | 3-Brompyridin-2-yl | 0,67 |
| I-2-16 | C-H | C-H | H | O | OCH₃ | Methyl | 0,00 |
| I-2-17 | C-H | C-H | H | O | Ethyl | Methyl | 0,01 |
| I-2-18 | C-H | C-H | H | O | H | CH2OMe | -0,23 |
| I-2-19 | C-H | C-H | H | O | H | 3-Chlorbenzyl | 1,33 |
| I-2-20 | C-H | C-H | H | O | Methyl | i-Propyl | 0,44 |
| I-2-21 | C-H | C-H | H | O | H | n-Propyl | 0,29 |
| I-2-22 | C-H | C-H | H | O | H | 4,6-Dimethylpyrimidin-2-yl | 0,86 |
| I-2-23 | C-H | C-H | H | O | H | 2-Methylbenzyl | 1,23 |
| I-2-24 | C-H | C-H | H | O | H | 4,6-Dimethoxypyrimidin-2-yl | 1,11 |
| I-2-25 | C-H | C-H | H | O | H | 4,5,6-Trimethylpyrimidin-2-yl | 1,07 |
| I-2-26 | C-H | C-H | H | O | SCH₃ | Methyl | 0,32 |
| I-2-27 | C-H | C-H | H | O | Cl | Phenyl | 1,58 |
| I-2-28 | C-H | C-H | H | O | OMe | Phenyl | 1,23 |

**Tabelle 3**

| | | | | | | | |
|---|---|---|---|---|---|---|---|
| Verbindungen der Formel (I-3) | | | | | | | |
| 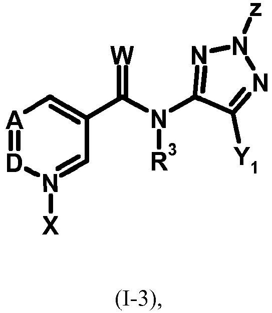 | | | | | | | |
| worin X für ein freies Elektronenpaar steht und A, D, W, R³, Y₁ und Z die in Tabelle 3 angegebene Bedeutung aufweisen. | | | | | | | |

| **Verbindung Nr.** | **D** | **A** | **R³** | **W** | **Y₁** | **Z** | **log P (acid)¹** |
|---|---|---|---|---|---|---|---|
| I-3-1 | C-H | C-H | H | O | Methyl | Pyridin-2-yl | 1,08 |
| I-3-2 | C-H | C-H | H | O | H | CH₃ | 0,38 |
| I-3-3 | C-H | C-H | H | O | Br | Phenyl | 2,09 |
| I-3-4 | C-H | C-H | H | O | H | 4-Chlorphenyl | 2,43 |
| I-3-5 | C-H | C-H | H | O | H | 3-Chlor-5-(trifluormethyl)pyridin-2-yl | 2,12 |
| I-3-6 | C-H | C-H | H | O | CF₃ | 3-Chlorpyridin-2-yl | 1,89 |
| I-3-7 | C-H | C-H | H | O | H | 3-Brompyridin-2-yl | 1,33 |
| I-3-8 | C-H | C-H | H | O | CF₃ | 2,4-Dichlorphenyl | 2,97 |
| I-3-9 | C-H | C-H | H | O | Me | 3-Chlorpyridin-2-yl | 1,24 |
| I-3-10 | C-H | C-H | H | O | H | i-Propyl | 1,09 |
| I-3-11 | C-H | C-H | H | O | Me | Phenyl | 1,82 |
| I-3-12 | C-H | C-H | H | O | CF₃ | Phenyl | 2,44 |
| I-3-13 | C-H | C-H | H | O | Cl | 2-Chlorphenyl | 2,04 |
| I-3-14 | C-H | C-H | H | O | H | 5-(Trifluormethyl)pyridin-2-yl | 1,91 |
| I-3-15 | C-H | C-H | H | O | H | 3,5-Dichlorpyridin-2-yl | 1,83 |
| I-3-16 | C-H | C-H | H | O | H | 3-Chlorpyridin-2-yl | 1,32 |
| I-3-17 | C-H | C-H | H | O | CH₃ | 3,5-Dichlorpyridin-2-yl | 1,74 |
| I-3-18 | C-H | C-H | H | O | CH₃ | 3-(Trifluormethyl)phenyl | 2,58 |
| I-3-19 | C-H | C-H | H | O | CH₃ | 2,4-Dichlorphenyl | 2,24 |
| I-3-20 | C-H | C-H | H | O | CF₃ | 4-Chlorphenyl | 2,93 |
| I-3-21 | C-H | C-H | H | O | Cl | 3-Chlorpyridin-2-yl | 1,51 |
| I-3-22 | C-H | C-H | H | O | Me | 2,5-Dichlorphenyl | 2,26 |
| I-3-23 | C-H | C-H | H | O | Me | 2-Chlorphenyl | 1,71 |
| I-3-24 | C-H | C-H | H | O | H | 2,6-Difluorphenyl | 1,67 |

**Tabelle 4**

| | | | | | | |
|---|---|---|---|---|---|---|
| Verbindungen der Formel (I-4) | | | | | | |
| 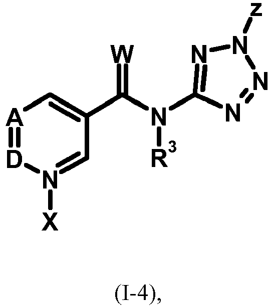 | | | | | | |
| worin X für ein freies Elektronenpaar steht und A, D, W, R³ und Z die in Tabelle 4 angegebene Bedeutung aufweisen. | | | | | | |

| **Verbindung Nr.** | **D** | **A** | **R³** | **W** | **Z** | **log P (acid)¹** |
|---|---|---|---|---|---|---|
| I-4-1 | C-H | C-H | H | O | Ethyl | - |
| I-4-2 | C-H | C-H | H | O | CH₃ | <-0,23 |
| I-4-3 | C-H | C-H | H | O | (6-Chlorpyridin-3-yl)methyl | 1,05 |
| I-4-4 | C-H | C-H | H | O | 4,6-Dimethoxypyrimidin-2-yl | 1,35 |
| I-4-5 | C-H | C-H | H | O | 2-Ethoxy-2-oxoethyl | 0,71 |
| I-4-6 | C-H | C-H | H | O | 2-Oxo-2-[(1-phenylethyl)amino]ethyl | 1,34 |
| I-4-7 | C-H | C-H | H | O | 2-[(1-Naphthylmethyl)amino]-2-oxoethyl | 1,71 |
| I-4-8 | C-H | C-H | H | O | Allyl | 0,60 |
| I-4-9 | C-H | C-H | H | O | n-Propyl | 0,76 |
| I-4-10 | C-H | C-H | H | O | Benzyl | 1,38 |
| I-4-11 | C-H | C-H | H | O | 2-(Butylamino)-2-oxoethyl | 0,97 |

| | | | | | | |
|---|---|---|---|---|---|---|
| ¹) Die Messung der logP Werte erfolgte gemäß EEC Directive 79/831 Annex V.A8 durch HPLC (High Performance Liquid Chromatography) an einer Phasenumkehrsäule (C 18). Temperatur: 55°C. | | | | | | |

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. Alternativ erfolgt die Bestimmung im sauren Bereich bei pH 2.3 mit 0,1% wässriger Phosphorsäure und Acetonitril als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

**NMR Daten ausgewählter Beispiele²⁾**

| |
|---|
| Verbindung Nr. I-1-25 (Synthesebeispiel 14): 1H-NMR (DMSO-D₆) 2.53 (s, 3H), 3.43 (s, 3H), 6.11 (br. s, 1H), 7.30 - 7.33 (m, 2H), 7.79 - 7.82 (m, 1H), 8.56 - 8.67 (m, 2H), 8.76 - 8.77 (m, 2H). |
| Verbindung Nr. I-1-131, Lösungsmittel: [CDC13], Spektrometer: 600.13MHz |
| 9,1353 (2,45); 9,1324 (2,42); 8,8222 (1,68); 8,82 (1,73); 8,8143 (1,76); 8,8122 (1,64); 8,412 (1,52); 8,2381 (1,24); 8,2351 (1,75); 8,2315 (1,22); 8,2249 (1,35); 8,2218 (1,82); 8,2183 (1,21); 7,4794 (1,48); 7,4713 (1,53); 7,4662 (1,52); 7,4582 (1,42); 7,2618 (20,87); 6,8688 (3,56); 3,018 (3,82); 3,0057 (6,42); 3 (0,56); 2,9932 (4,04); 2,6262 (16); 2,625 (15,51); 2,3081 (0,54); 2,0455 (0,33); 1,8123 (0,49); 1,7999 (2,18); 1,7876 (4,17); 1,7816 (0,43); 1,7752 (4,16); 1,7628 (2,19); 1,7505 (0,5); 1,6059 (2,23); 1,3326 (0,75); 1,2844 (1,01); 1,2714 (0,33); 1,2543 (1,84); 1,0586 (0,35); 1,0443 (6,17); 1,032 (12,22); 1,0196 (5,8); 0,8801 (0,34); 0,0697 (2,45); 0,0052 (0,65); - 0,0001 (14,55); -0,0056 (0,61) |
| Verbindung Nr. I-1-130, Lösungsmittel: [CDC13], Spektrometer: 600.13MHz |
| 9,1456 (1,87); 9,1426 (1,83); 8,8217 (1,31); 8,8192 (1,34); 8,8138 (1,36); 8,8112 (1,27); 8,5293 (1,07); 8,2486 (0,83); 8,2455 (1,16); 8,2421 (0,8); 8,2354 (0,88); 8,2323 (1,19); 8,2289 (0,78); 7,4796 (1); 7,4715 (1,04); 7,4664 (1,02); 7,4583 (0,96); 7,2645 (5,13); 6,8776 (2,33); 3,7868 (0,42); 3,7753 (1,08); 3,7638 (1,46); 3,7523 (1,1); 3,7408 (0,44); 2,6204 (10,39); 2,6192 (9,95); 2,0456 (0,35); 1,7042 (0,83); 1,2846 (0,5); 1,27 (15,75); 1,2585 (16); 1,2475 (0,39); 0,0708 (0,5); -0,0001 (3,64) |
| Verbindung Nr. I-1-129, Lösungsmittel: [CDC13], Spektrometer: 600.13MHz |
| 9,1449 (2,75); 8,8175 (1,92); 8,8108 (1,93); 8,5713 (1,88); 8,2413 (1,82); 8,2281 (1,87); 7,4778 (1,49); 7,4697 (1,62); 7,4648 (1,59); 7,4567 (1,4); 7,2653 (4,96); 6,8694 (3,8); 3,0639 (1,63); 3,0516 (4,88); 3,0393 (4,95); 3,027 (1,68); 2,6252 (16); 1,7875 (0,36); 1,2561 (5,49); 1,2438 (9,98); 1,2315 (4,96); 0,0711 (0,71); -0,0001 (3,52) |
| Verbindung Nr. I-1-121 (Synthesebeispiel 8), Lösungsmittel: [DMF], Spektrometer: 601.6MHz |
| 11,4702 (6,65); 9,3267 (10,56); 9,3255 (10,81); 9,3229 (10,95); 9,3218 (10,11); 8,8237 (8,74); 8,821 (9,21); 8,8157 (9,23); 8,813 (9,04); 8,5224 (5,53); 8,5195 (6,91); 8,5189 (6,42); 8,5158 (5,41); 8,5092 (5,83); 8,5062 (7); 8,5054 (6,87); 8,5025 (5,38); 8,3676 (1,02); 8,0268 (8,21); 7,6214 (6,33); 7,6201 (5,97); 7,6135 (6,21); 7,6122 (6,04); 7,6082 (6,32); 7,6069 (6,03); 7,6003 (6,05); 7,599 (5,65); 6,9658 (16); 6,9642 (15,53); 3,479 (10,85); 3,1044 (1,8); 3,0966 (4,03); 3,0912 (4,3); 3,0891 (2,81); 3,0834 (7,77); 3,0786 (2,36); 3,0757 (4,44); 3,0703 (4,28); 3,0626 (2,03); 2,9524 (7,84); 2,922 (4,07); 2,9189 (8,25); 2,9158 (11,65); 2,9126 (8,18); 2,9095 (3,83); 2,7852 (7,73); 2,7521 (4,55); 2,7489 (9,01); 2,7457 (13,1); 2,7425 (9,08); 2,7392 (4,48); 2,6904 (0,33); 2,633 (0,36); 2,6145 (0,34); 2,5838 (58,74); 2,5823 (59,79); 2,4736 (0,34); 2,4721 (0,33); 2,3282 (0,34); 2,0138 (0,88); 1,3793 (1,67); 1,2967 (2,36); 1,2765 (1,37); 1,1997 (0,51); 1,1701 (2,67); 1,1661 (3,39); 1,1627 (6,72); 1,1575 |
| (14,41); 1,1528 (6,67); 1,1494 (6,39); 1,1444 (12,15); 1,1392 (5,49); 1,1295 (2,51); 1,1146 (4,88); 1,1097 (12,56); 1,1074 (6,13); 1,1064 (6,7); 1,1045 (8,39); 1,102 (14,4); 1,097 (8,48); 1,0928 (2,95); 1,0899 (2,46); 0,0052 (0,47); -0,0002 (14,23); -0,0058 (0,44) |
| Verbindung Nr. I-1-126, Lösungsmittel: [DMSO], Spektrometer: 399.95MHz |
| 11,5431 (2,28); 10,8427 (0,56); 9,1475 (2,09); 9,1423 (2,07); 9,0744 (0,62); 9,0697 (0,61); 8,8372 (0,41); 8,8334 (0,43); 8,825 (0,43); 8,8205 (0,42); 8,7591 (1,48); 8,7551 (1,54); 8,7471 (1,52); 8,7432 (1,49); 8,369 (0,87); 8,3642 (1,24); 8,3591 (0,84); 8,349 (0,9); 8,3439 (1,3); 8,3393 (0,81); 8,3155 (0,55); 8,2626 (0,33); 8,2422 (0,37); 7,6516 (0,33); 7,6405 (0,32); 7,6329 (0,34); 7,6203 (0,32); 7,5566 (1,12); 7,5452 (1,14); 7,5364 (1,13); 7,5246 (1,06); 6,8599 (3,08); 6,6595 (1,36); 5,7555 (0,99); 3,9801 (4,65); 3,9407 (16); 3,3209 (39,7); 2,6751 (0,92); 2,6706 (1,27); 2,6659 (0,9); 2,5246 (15,38); 2,5101 (76,95); 2,506 (146,69); 2,5015 (188,94); 2,4971 (135,14); 2,493 (64,86); 2,3329 (0,85); 2,3282 (1,17); 2,3238 (0,88); 2,226 (4,09); 1,9884 (0,75); 1,2585 (0,45); 1,2341 (0,59); 1,1748 (0,41); 0,146 (0,44); 0,0079 (4,34); -0,0002 (99,88); -0,0085 (3,51); -0,1498 (0,47) |
| Verbindung Nr. I-1-127, Lösungsmittel: [DMSO], Spektrometer: 399.95MHz |
| 12,5148 (2,08); 8,9062 (3,17); 8,9023 (3,1); 8,7071 (2,27); 8,7031 (2,39); 8,695 (2,4); 8,691 (2,29); 8,3159 (1,1); 8,1252 (1,34); 8,1209 (1,85); 8,1154 (1,32); 8,1053 (1,51); 8,0998 (1,95); 8,0954 (1,37); 7,7921 (2,32); 7,7877 (3,77); 7,7834 (2,51); 7,7342 (1,61); 7,7317 (2,07); 7,7278 (1,51); 7,7149 (1,92); 7,7122 (2,35); 7,7085 (1,68); 7,6381 (1,45); 7,6356 (1,66); 7,6328 (1,51); 7,6302 (1,42); 7,6179 (1,97); 7,6154 (2,07); 7,6126 (2,06); 7,6102 (1,7); 7,5121 (1,77); 7,5103 (1,78); 7,4999 (4,1); 7,4923 (1,84); 7,4904 (1,8); 7,4801 (5,24); 7,4603 (1,66); 6,1543 (3,27); 6,151 (3,23); 4,0554 (0,67); 4,0376 (1,96); 4,0199 (1,96); 4,0021 (0,62); 3,3215 (206,98); 2,675 (2,46); 2,6705 (3,31); 2,666 (2,45); 2,5408 (2,59); 2,5237 (10,78); 2,5103 (199,81); 2,5059 (388,58); 2,5014 (498,01); 2,4969 (358,02); 2,4925 (170,77); 2,3328 (2,36); 2,3282 (3,13); 2,3237 (2,23); 2,2278 (0,78); 2,2141 (0,38); 2,1856 (16); 1,9886 (8,67); 1,298 (3,13); 1,2811 (0,53); 1,2586 (4,67); 1,2351 (5,43); 1,1926 (2,59); 1,1748 (4,92); 1,157 (2,46); 0,8842 (0,36); 0,8674 (0,66); 0,8537 (0,99); 0,8366 (0,55); 0,146 (2,54); 0,0215 (1,17); 0,008 (24,33); -0,0001 (613,66); -0,0084 (23,69); -0,1496 (2,55) |
| Verbindung Nr. I-1-8: Masse (m/z): 253,1 (M+H)⁺; 1H-NMR (CD₃CN) 2.44 (s, 3H), 6.74 (s, 1H), 7.08 - 7.38 (m, 1H), 7.44 - 7.47 (m, 1H), 8.20 - 8.23 (m, 1H), 8.72 - 8.73 (m, 1H), 9.06 (s, 1H), 9.13 (br. s, 1H). |
| Verbindung Nr. I-1-122 (Synthesebeispiel 6): HPLC-MS: logP = 2,27; Masse (m/z): 341,0 (M+H)⁺; 1H-NMR |
| (DMSO-D₆) 2.68 (s, 3H), 7.01 (s, 1H), 7.45 - 7.59 (m, 4H), 7.64 - 7.66 (m, 1H), 8.26 - 8.29 (m, 1H), 8.70 - 8.72 (m, 1H), 9.05 (s, 1H), 11.41 (s, 1H). 13C-NMR (DMF-D₇) 15.2, 107.0, 124.9, 128.5, 130.9, 131.0, 132.0, 133.2, 136.9, 137.1, 145.9, 150.8, 152.3, 154.2, 166.0, 168.4. |
| Verbindung Nr. I-1-28, Lösungsmittel: [DMSO], Spektrometer: 399.95MHz |
| 11,3961 (2,16); 11,2451 (2,58); 9,1109 (4,38); 9,1065 (4,28); 9,0984 (2,24); 9,097 (2,29); 9,0931 (2,2); 9,0748 (2,74); 9,0724 (2,68); 9,0694 (2,62); 8,8513 (1,88); 8,8473 (1,95); 8,8393 (2,03); 8,8352 (2,02); 8,8305 (1,95); 8,8263 (1,97); 8,8183 (1,98); 8,8141 (1,92); 8,7995 (1,11); 8,7952 (1,15); 8,788 (2,35); 8,7838 (2,31); 8,7761 (1,69); 8,772 (1,69); 8,7538 (1,65); 8,7497 (1,7); 8,7418 (1,7); 8,7377 (1,62); 8,3568 (1,14); 8,3517 (1,57); 8,3469 (1,08); 8,3363 (1,7); 8,3314 (2,71); 8,3269 (1,58); 8,3154 (1,07); 8,3098 (1,6); 8,3075 (1,66); 8,302 (2,3); 8,2973 (2,26); 8,292 (1,18); 8,2875 (1,46); 8,282 (2,62); 8,2776 (2,65); 8,2723 (1,07); 8,2645 (0,74); 8,2593 (0,95); 8,2546 (0,59); 7,6707 (1,3); 7,669 (1,3); 7,6586 (1,31); 7,657 (1,31); 7,6508 (1,28); 7,6491 (1,27); 7,6377 (2,39); 7,6255 (1,36); 7,6238 (1,35); 7,6178 (1,3); 7,616 (1,29); 7,6056 (1,41); 7,6029 (1,78); 7,6004 (1,24); 7,5899 (1,17); 7,5881 (1,16); 7,5823 (1,15); 7,5804 (1,15); 7,5701 (1,17); 7,5682 (1,21); 7,5652 (1,02); 7,5632 (0,96); 7,5528 (0,95); 7,5509 (1,02); 7,5453 (1,92); 7,5332 (1,81); 7,5272 (1,15); 7,5255 (1,09); 7,5151 (1,04); 7,5133 (1,01); 7,0048 (2,58); 7,0028 (2,47); 6,8275 (5,57); 4,0385 (0,63); 4,0207 (0,63); 3,3332 (5,42); 3,0885 (0,32); 2,6717 (9,36); 2,6699 (9,07); 2,6492 (0,41); 2,6275 (0,34); 2,5257 (1,12); 2,5125 (18,31); 2,5081 (35,47); 2,5035 (45,71); 2,499 (32,73); 2,4945 (15,61); 2,3303 (0,46); 2,2555 (16); 2,0746 (1,2); 2,0394 (0,92); 1,9898 (2,89); 1,2008 (1,03); 1,1931 (0,85); 1,1826 (2,11); 1,1753 (1,64); 1,1643 (1,03); 1,1576 (0,83); 0,008 (0,38); -0,0002 (10,56); -0,0085 (0,39) |
| Verbindung Nr. I-1-118 (Synthesebeispiel 1), Lösungsmittel: [DMSO], Spektrometer: 399.95MHz |
| 11,186 (6,01); 8,9279 (10,58); 8,923 (6,61); 8,5285 (5,54); 8,5241 (5,26); 8,349 (3,58); 8,3427 (3,46); 8,3281 (3,76); 8,3217 (3,7); 8,3131 (0,43); 7,8689 (7,75); 7,863 (7,69); 7,651 (6,34); 7,63 (5,99); 6,6992 (6,72); 6,6934 (6,66); 5,6307 (16); 3,3468 (382,22); 3,3421 (479,14); 2,9953 (0,52); 2,7123 (0,37); 2,6811 (0,43); 2,6768 (0,89); 2,6723 (1,23); 2,6678 (0,89); 2,6632 (0,41); 2,5425 (98,83); 2,5256 (3,55); 2,5207 (5,28); 2,5122 (69,51); 2,5077 (139,53); 2,5031 (185,38); 2,4985 (134,63); 2,4941 (63,84); 2,3687 (0,38); 2,3389 (0,42); 2,3344 (0,88); 2,3299 (1,22); 2,3253 (0,86); 2,321 (0,39); -0,0002 (2,89) |
| Verbindung Nr. I-1-116, Lösungsmittel: [DMSO], Spektrometer: 399.95MHz |
| 11,105 (5,35); 9,0903 (5,07); 9,086 (4,95); 8,9348 (5,34); 8,9323 (5,23); 8,7152 (4,17); 8,7112 (4,43); 8,7032 (4,37); 8,6991 (4,27); 8,5324 (5,51); 8,5287 (5,27); 8,3165 (0,7); 8,31 (2,11); 8,3051 (2,97); 8,3002 (2,08); 8,29 (2,22); 8,2849 (3,05); 8,2802 (2,02); 7,863 (7,71); 7,8572 (7,62); 7,5162 (2,9); 7,5145 (3,01); 7,5042 (2,87); 7,5024 (2,95); 7,4963 (2,82); 7,4945 (2,85); 7,4843 (2,68); 7,4825 (2,7); 6,7093 (6,47); 6,7035 (6,38); 5,6316 (16); 3,5679 (2,2); 3,3227 (88,64); 2,6798 (0,5); 2,6754 (1,04); 2,6707 (1,44); 2,6662 (1,04); 2,6615 (0,51); 2,5241 (5,38); 2,5192 (8,57); 2,5107 (83,22); 2,5063 (164,45); 2,5017 (214,4); 2,4971 (153,48); 2,4926 (72,98); 2,3375 |
| (0,49); 2,333 (1,04); 2,3284 (1,4); 2,3239 (1,02); 2,3194 (0,49); -0,0002 (5,74) |
| Verbindung Nr. I-1-128, Lösungsmittel: [DMSO], Spektrometer: 399.95MHz |
| 11,0842 (3,67); 8,7901 (6,31); 8,778 (6,38); 8,6469 (3,9); 8,3526 (2,19); 8,3363 (2,22); 7,8443 (2,13); 7,8243 (2,34); 7,5255 (1,69); 7,5069 (2,39); 7,4897 (1,47); 7,4537 (1,78); 7,4415 (3,26); 7,4294 (1,7); 6,5002 (4,82); 5,4266 (9,94); 3,3265 (12,95); 2,6717 (0,44); 2,5026 (64,24); 2,3131 (16); -0,0002 (3,96) |
| Verbindung Nr. I-1-68, Lösungsmittel: [DMSO], Spektrometer: 399.95MHz |
| 11,0958 (5,44); 9,0804 (5,51); 9,0761 (5,44); 8,7094 (4,18); 8,7054 (4,47); 8,6975 (4,4); 8,6934 (4,28); 8,3025 (2,24); 8,2978 (3,23); 8,2928 (2,26); 8,2826 (2,43); 8,2777 (3,37); 8,2729 (2,24); 7,7956 (6,29); 7,7901 (6,22); 7,506 (3,87); 7,4939 (3,41); 7,488 (4,55); 7,4859 (4,5); 7,4738 (3,67); 7,4679 (3,72); 7,4509 (1,85); 7,4469 (2,18); 7,4301 (0,95); 7,1832 (0,66); 7,1791 (0,91); 7,1688 (5,36); 7,1584 (1,31); 7,1489 (8,15); 7,1397 (1,32); 7,1288 (4,54); 7,118 (0,75); 6,6523 (6,87); 6,6466 (6,74); 5,3498 (16); 3,5684 (2,4); 3,3262 (12,24); 2,6716 (0,42); 2,5113 (28,02); 2,507 (52,81); 2,5026 (67,34); 2,498 (49,13); 2,4937 (24,41); 2,3338 (0,34); 2,3293 (0,44); 2,3248 (0,33); 0,0079 (0,38); -0,0002 (7,87); -0,0084 (0,33) |
| Verbindung Nr. I-1-65, Lösungsmittel: [DMSO], Spektrometer: 399.95MHz |
| 11,2029 (3,03); 9,1267 (3,24); 9,1222 (3,11); 8,7373 (2,62); 8,7332 (2,83); 8,7253 (2,78); 8,7212 (2,76); 8,3453 (1,29); 8,3402 (1,9); 8,3355 (1,31); 8,3254 (1,4); 8,3202 (2,01); 8,3156 (1,32); 7,8435 (4,6); 7,8376 (4,56); 7,5419 (1,84); 7,5402 (1,98); 7,5298 (1,8); 7,5282 (1,95); 7,522 (1,78); 7,5203 (1,89); 7,51 (1,71); 7,5082 (1,82); 6,7181 (4); 6,7122 (3,94); 4,9978 (0,48); 4,9813 (0,41); 4,969 (0,55); 4,9652 (0,46); 4,953 (0,91); 4,941 (0,71); 4,9377 (0,75); 4,9265 (1,32); 4,9025 (3); 4,8871 (3,88); 4,8769 (2,1); 4,8644 (0,74); 4,8523 (0,43); 4,8079 (0,71); 4,7833 (2,88); 4,7711 (3,54); 4,7677 (3,15); 4,7594 (2,3); 4,7468 (0,74); 4,7346 (0,41); 3,5679 (0,59); 3,3237 (23,24); 2,5243 (1); 2,5108 (16,85); 2,5064 (33,75); 2,5018 (44,33); 2,4972 (31,57); 2,4927 (14,9); 2,0859 (16); 1,9889 (0,83); 1,1744 (0,45); 0,008 (0,59); -0,0002 (16,34); -0,0086 (0,47) |
| Verbindung Nr. I-1-l0, Lösungsmittel: [DMSO], Spektrometer: 399.95MHz |
| 11,2613 (10,17); 9,1245 (10,86); 9,12 (10,69); 8,7412 (8,57); 8,7372 (9,15); 8,7292 (9,01); 8,7251 (8,87); 8,3457 (4,57); 8,3408 (6,65); 8,3359 (4,59); 8,3258 (4,88); 8,3208 (6,94); 8,316 (4,58); 8,0063 (0,42); 7,9997 (0,43); 7,8191 (15,1); 7,8131 (15,08); 7,5443 (6,21); 7,5429 (6,33); 7,5322 (6,15); 7,5308 (6,26); 7,5244 (6,01); 7,523 (6,08); 7,5124 (5,74); 7,5109 (5,8); 6,7806 (14,07); 6,7746 (13,85); 6,6696 (0,49); 6,6632 (0,5); 5,7577 (0,36); 5,3111 (0,4); 5,2887 (0,43); 5,1141 (4,71); 5,0914 (15,23); 5,0686 (16); 5,0457 (5,43); 3,3215 (92,5); 2,6794 (0,64); 2,6751 (1,32); 2,6706 (1,81); 2,6661 (1,32); 2,6617 (0,62); 2,5238 (6,97); 2,5189 (10,67); 2,5105 (102,78); 2,5061 (203,49); 2,5015 (266,6); 2,497 (191,68); 2,4925 (92,04); 2,3373 (0,62); 2,3328 (1,29); 2,3283 (1,77); 2,3237 (1,28); 2,3193 (0,61); 2,1388 (0,34); 1,2333 (0,39); 0,1459 (0,79); 0,0079 (6,68); -0,0003 (182,05); -0,0086 (6,37); -0,1497 (0,82) |
| Verbindung Nr. I-1-2: Masse (m/z): 261,1 (M+H)⁺; 1H-NMR (CD₃CN) 1.10 - 1.16 (t, 3H), 2.33 (s, 3H), 3.49 - 3.54 (q, 2H), 5.29 (s, 2H), 6.54 (s, 1H), 7.41 - 7.45 (m, 1H), 8.17 - 8.20 (m, 1H), 8.70 - 8.71 (m, 1H), 8.91 (br. s, 1H), 9.05 (s, 1H). |
| Verbindung Nr. I-1-13, Lösungsmittel: [DMSO], Spektrometer: 399.95MHz |
| 11,2004 (9,57); 9,1215 (10,71); 9,1174 (10,62); 9,116 (9,9); 8,7389 (9,46); 8,7348 (10,03); 8,7269 (9,98); 8,7227 (9,7); 8,3403 (4,84); 8,3356 (6,51); 8,3304 (4,84); 8,3204 (5,17); 8,3152 (6,75); 8,3105 (4,75); 7,9274 (0,42); 7,921 (0,42); 7,7939 (0,36); 7,7689 (0,47); 7,763 (0,71); 7,7526 (16); 7,7467 (15,94); 7,5439 (6,8); 7,5419 (6,78); 7,5319 (6,64); 7,5299 (6,59); 7,524 (6,54); 7,522 (6,42); 7,512 (6,36); 7,51 (6,21); 6,7215 (14,18); 6,7157 (13,94); 6,6009 (0,51); 6,5946 (0,49); 6,4839 (1,78); 6,4745 (3,59); 6,4652 (1,56); 6,3466 (3,57); 6,3372 (7,81); 6,3278 (3,43); 6,275 (0,37); 6,269 (0,41); 6,2092 (1,73); 6,1998 (3,92); 6,1905 (1,78); 5,6649 (0,32); 4,997 (0,35); 4,6165 (5,54); 4,6072 (5,92); 4,5788 (12,33); 4,5694 (11,91); 4,5578 (0,63); 4,541 (6,32); 4,5317 (5,85); 4,113 (0,4); 4,0998 (1,17); 4,0867 (1,19); 4,0736 (0,42); 3,3224 (164,26); 3,1746 (5,69); 3,1615 (5,49); 2,6796 (0,62); 2,6752 (1,34); 2,6706 (1,87); 2,666 (1,35); 2,6613 (0,62); 2,5515 (0,45); 2,524 (6,02); 2,5193 (8,91); 2,5106 (103,82); 2,5061 (211,76); 2,5015 (279,15); 2,4969 (198,41); 2,4923 (93,16); 2,3373 (0,61); 2,3329 (1,33); 2,3283 (1,85); 2,3237 (1,32); 2,3191 (0,62); 1,9799 (0,87); 1,2982 (0,48); 1,2584 (0,74); 1,2337 (1,11); 1,2249 (0,45); 1,1336 (0,44); 1,1161 (0,89); 1,0985 (0,45); 0,146 (0,41); 0,008 (3,45); -0,0002 (110,9); -0,0086 (3,51); -0,1497 (0,43) |
| Verbindung Nr. I-1-48, Lösungsmittel: [CD3CN], Spektrometer: 399.95MHz |
| 9,4678 (3,5); 9,1159 (11,57); 9,1119 (11,83); 8,7531 (8,46); 8,7491 (8,63); 8,741 (8,89); 8,7371 (8,3); 8,5255 (0,82); 8,5177 (0,86); 8,5139 (0,84); 8,4673 (9,23); 8,4636 (9,13); 8,4557 (9,5); 8,4521 (8,87); 8,3522 (0,41); 8,2789 (5,68); 8,2746 (7,42); 8,2692 (5,11); 8,259 (6,14); 8,2535 (8,68); 8,2492 (5,65); 8,183 (0,35); 8,1155 (16); 8,1089 (15,75); 8,0367 (9,81); 8,0332 (9,12); 8,0166 (10,56); 8,013 (9,66); 7,9292 (0,56); 7,5173 (0,98); 7,5017 (7,24); 7,5 (6,38); 7,4895 (7,45); 7,4878 (6,84); 7,4817 (7,3); 7,4696 (6,67); 7,4242 (9,49); 7,4125 (9,51); 7,404 (9,12); 7,3925 (8,47); 7,3157 (0,54); 7,2973 (0,62); 7,2836 (0,76); 7,2637 (1,4); 7,2503 (1,46); 7,1911 (1,39); 7,1728 (1,04); 7,157 (0,91); 7,1353 (0,59); 7,0322 (14,15); 7,0257 (13,66); 6,9733 (0,67); 6,8915 (1,55); 6,8846 (1,47); 5,9789 (0,39); 5,9714 (0,37); 5,4473 (2,42); 5,2091 (0,32); 3,6001 (4,8); 2,6305 (0,67); 2,463 (1,52); 2,4582 (1,19); 2,3641 (0,6); 2,2216 (1,31); 2,1462 (455,25); 2,1198 (5,12); 2,1133 (4,96); 2,1071 (5,09); 2,101 (3,55); 2,095 (2,01); 1,9637 (33,68); 1,9575 (53,42); 1,9519 (256,12); 1,9457 (459,7); 1,9396 (599,75); 1,9335 (416,36); 1,9274 (217,09); 1,7803 (1,82); 1,7741 (2,95); 1,7681 (3,81); 1,7618 (2,69); 1,7557 (1,59); 1,629 (1,03); |
| 1,6178 (1,39); 1,5454 (0,59); 1,53 (0,56); 1,3875 (10,31); 1,2712 (10,53); 1,2553 (3,32); 1,2377 (2,63); 1,1903 (0,88); 1,1313 (0,83); 1,1139 (1,08); 1,0964 (0,9); 0,9339 (0,51); 0,8818 (2,77); 0,8767 (2,35); 0,8578 (2,77); 0,8393 (1,69); 0,2256 (2,42); 0,1462 (1,26); -0,0002 (259,77); -0,0079 (14,1); -0,1492 (1,21) |
| Verbindung Nr. I-1-23 (Synthesebeispiel 13): 1H-NMR (DMSO-D₆) 2.66 (s, 3H), 6.83 (s, 1H), 7.41 - 7.43 (m, 1H), 7.49 - 7.53 (m, 1H), 8.35 - 8.38 (m, 1H), 8.72 - 8.74 (m, 1H), 8.84 - 8.85 (m, 2H), 9.17 (s, 1H), 11.26 (s, 1H). |
| Verbindung Nr. I-1-16, Lösungsmittel: [DMSO], Spektrometer: 399.95MHz |
| 8,5322 (1,69); 8,5228 (1,74); 8,4772 (1,46); 8,3155 (0,5); 7,7336 (0,94); 7,7176 (1); 7,3603 (1,02); 7,3476 (1,25); 7,3306 (0,91); 6,4401 (0,41); 3,9021 (2,23); 3,6664 (2,4); 3,5739 (16); 3,5243 (0,37); 3,3306 (106,01); 3,2854 (41,42); 2,6752 (0,61); 2,6709 (0,84); 2,6662 (0,59); 2,541 (0,5); 2,5241 (2,12); 2,5107 (54,82); 2,5064 (108,01); 2,5018 (139,17); 2,4973 (99,74); 2,4929 (48,2); 2,3332 (0,63); 2,3286 (0,84); 2,324 (0,62); 2,2541 (1,72); 2,1307 (12,56); 1,235 (0,84); 0,008 (0,41); -0,0002 (12,78); -0,0085 (0,41) |
| Verbindung Nr. I-1-55, Lösungsmittel: [DMSO], Spektrometer: 399.95MHz |
| 11,4245 (2,93); 9,1654 (2,05); 9,1618 (2,03); 9,1126 (3,96); 9,1092 (3,91); 8,7665 (1,42); 8,7579 (1,43); 8,584 (3,21); 8,5775 (4,94); 8,5583 (3,39); 8,5547 (3,37); 8,5519 (2,3); 8,5483 (2,06); 8,3827 (1,21); 8,3781 (1,63); 8,3729 (1,19); 8,3628 (1,3); 8,3578 (1,71); 8,3529 (1,21); 8,315 (0,37); 7,572 (1,29); 7,5598 (1,31); 7,5531 (1,29); 7,5412 (1,21); 6,8889 (4,09); 3,9023 (2,09); 3,3269 (107,68); 2,671 (16); 2,6696 (15,46); 2,5416 (0,68); 2,5248 (2,16); 2,5115 (45,57); 2,507 (90,92); 2,5025 (117,85); 2,4979 (82,89); 2,4933 (38,32); 2,3338 (0,52); 2,3293 (0,69); 2,3246 (0,5); 1,2303 (0,87); 0,008 (0,42); -0,0002 (12,29); -0,0085 (0,32) |
| Verbindung Nr. I-1-51, Lösungsmittel: [DMSO], Spektrometer: 399.95MHz |
| 11,0667 (2,54); 9,1155 (1,81); 8,7289 (1,33); 8,7192 (1,34); 8,5513 (1,7); 8,3237 (1,45); 8,3194 (1,04); 8,3162 (0,85); 8,3085 (1,1); 8,3039 (1,49); 7,5298 (1,23); 7,518 (1,28); 7,5099 (1,23); 7,4979 (1,13); 7,0918 (2,86); 7,0791 (2,76); 6,5843 (4,12); 5,3112 (7,61); 3,9023 (2,39); 3,3341 (18); 3,1686 (1,07); 2,6756 (0,61); 2,6711 (0,78); 2,6667 (0,56); 2,5414 (0,45); 2,5244 (2,29); 2,5111 (52,85); 2,5067 (103,65); 2,5021 (133,21); 2,4976 (95,08); 2,4931 (45,4); 2,3335 (0,59); 2,3289 (0,8); 2,3243 (0,58); 2,2615 (16); 2,2365 (0,43); 0,0079 (0,44); - 0,0002 (12,74); -0,0085 (0,37) |
| Verbindung Nr. I-1-33, Lösungsmittel: [DMSO], Spektrometer: 399.95MHz |
| 11,0522 (2,23); 9,1158 (1,67); 8,7321 (1,21); 8,7219 (1,2); 8,3327 (0,97); 8,3279 (1,37); 8,3232 (0,91); 8,3128 (1,04); 8,3081 (1,43); 8,3033 (0,92); 7,5362 (1,15); 7,5242 (1,2); 7,5166 (1,16); 7,5044 (1,06); 6,4815 (3,86); 4,3253 (2,28); 4,3107 (5,04); 4,2963 (2,63); 3,9809 (2,67); 3,9665 (5,13); 3,9519 (2,27); 3,9023 (1,48); 3,3325 (63,82); 3,1685 (0,33); 2,6756 (0,43); 2,6713 (0,59); 2,6665 (0,43); 2,5244 (1,48); 2,511 (38,98); 2,5066 (77,58); 2,5021 (100,32); 2,4975 (71,24); 2,493 (33,84); 2,4654 (0,36); 2,3332 (0,49); 2,3287 (0,71); 2,3243 (0,59); 2,3063 (16); -0,0002 (9,05) |
| Verbindung Nr. I-1-5, Lösungsmittel: [DMSO], Spektrometer: 399.95MHz |
| 11,036 (2,16); 9,1258 (1,21); 8,7388 (0,93); 8,7315 (0,92); 8,3344 (0,91); 8,33 (1,33); 8,3255 (0,9); 8,3151 (1,14); 8,3101 (1,37); 8,3056 (0,87); 7,5401 (0,97); 7,5282 (1,04); 7,5206 (1,01); 7,5083 (0,89); 6,4831 (3,77); 4,7996 (1,26); 4,7879 (2,28); 4,7759 (1,37); 4,6815 (1,24); 4,6698 (2,29); 4,6577 (1,4); 4,3457 (1,27); 4,3338 (2,14); 4,322 (1,17); 4,278 (1,33); 4,2661 (2,15); 4,2544 (1,1); 3,9023 (1,97); 3,337 (52,28); 2,6762 (0,32); 2,6714 (0,47); 2,6668 (0,32); 2,5417 (0,36); 2,5248 (1,46); 2,5114 (31,22); 2,5069 (61,27); 2,5024 (78,95); 2,4978 (56,38); 2,4933 (26,93); 2,3336 (0,38); 2,3292 (0,49); 2,3246 (0,37); 2,2754 (16); -0,0002 (8,18) |
| Verbindung Nr. I-1-1, Lösungsmittel: [DMSO], Spektrometer: 600.13MHz |
| 10.990 (3.4); 9.102 (3.2); 9.099 (3.2); 8.794 (6.8); 8.786 (6.8); 8.714 (2.2); 8.712 (2.3); 8.706 (2.3); 8.704 (2.2); 8.313 (1.8); 8.300 (1.9); 7.513 (1.6); 7.505 (1.8); 7.500 (1.7); 7.492 (1.5); 7.453 (1.8); 7.444 (3.4); 7.436 (1.7); 6.532 (4.3); 5.431 (10.3); 3.346 (5.1); 2.507 (9.9); 2.324 (16.0) |
| Verbindung Nr. I-1-4, Lösungsmittel: [DMSO], Spektrometer: 399.95MHz |
| 11,0907 (2,57); 9,1104 (2,62); 9,106 (2,55); 8,7307 (1,9); 8,7268 (2); 8,7188 (1,98); 8,7149 (1,9); 8,3304 (1,16); 8,3257 (1,6); 8,3206 (1,12); 8,3148 (0,61); 8,3104 (1,26); 8,3054 (1,66); 8,3006 (1,1); 7,5346 (1,48); 7,533 (1,48); 7,5224 (1,49); 7,5209 (1,5); 7,5147 (1,46); 7,513 (1,42); 7,5026 (1,36); 7,501 (1,32); 6,5409 (4,18); 6,4718 (0,38); 6,4625 (0,76); 6,4533 (0,34); 6,3347 (0,78); 6,3253 (1,65); 6,3161 (0,74); 6,1976 (0,38); 6,188 (0,81); 6,1789 (0,39); 4,5348 (1,24); 4,5257 (1,31); 4,497 (2,71); 4,4877 (2,61); 4,4592 (1,39); 4,4498 (1,27); 3,9022 (1,26); 3,3281 (157,45); 2,6753 (0,54); 2,6708 (0,71); 2,6663 (0,52); 2,5411 (0,42); 2,5106 (47,54); 2,5063 (90,79); 2,5018 (115,85); 2,4972 (82,7); 2,4928 (39,52); 2,3332 (0,58); 2,3285 (0,79); 2,3236 (0,69); 2,2985 (16); 0,0079 (1,02); -0,0002 (21,85); -0,0085 (0,77) |
| Verbindung Nr. I-1-38, Lösungsmittel: [DMSO], Spektrometer: 399.95MHz |
| 10,9825 (2,06); 9,111 (1,89); 8,7207 (1,37); 8,7121 (1,36); 8,3266 (0,94); 8,3219 (1,34); 8,3169 (0,94); 8,3067 (1,03); 8,3019 (1,4); 8,2972 (0,91); 7,5278 (1,19); 7,5157 (1,21); 7,5079 (1,2); 7,4958 (1,1); 6,4383 (3,86); 4,0198 (1,43); 4,0018 (4,71); 3,9837 (4,76); 3,9657 (1,48); 3,9023 (1,26); 3,3269 (73,21); 2,6756 (0,34); 2,6711 (0,46); 2,6665 (0,33); 2,5245 (1,31); 2,5111 (32,01); 2,5066 (62,9); 2,5021 (80,69); 2,4975 (57,17); 2,493 (26,9); 2,3334 (0,38); 2,3287 (0,51); 2,3242 (0,37); 2,269 (16); 1,3213 (5,07); 1,3033 (11,2); 1,2852 (4,94); -0,0002 (7,72) |
| Verbindung Nr. I-1-15, Lösungsmittel: [DMSO], Spektrometer: 399.95MHz |
| 11,0372 (2,51); 9,1006 (1,99); 8,7207 (1,41); 8,7175 (1,48); 8,7089 (1,47); 8,508 (1,22); 8,4983 (1,23); 8,4588 (1,68); 8,3182 (1,04); 8,3144 (1,63); 8,3086 (0,97); 8,2984 (1,1); 8,2935 (1,5); 8,2887 (0,97); 7,5521 (1,21); 7,5324 (1,57); 7,5217 (1,38); 7,5086 (1,32); 7,5017 (1,27); 7,4897 (1,18); 7,4006 (1,23); 7,3888 (1,27); 7,3812 (1,06); 7,369 (0,92); 6,5412 (4,12); 5,2861 (8,02); 3,9022 (3); 3,331 (148,75); 3,1687 (0,64); 2,6759 (0,49); 2,6714 (0,67); 2,6668 (0,49); 2,5415 (0,37); 2,5246 (2,01); 2,5114 (45,24); 2,5069 (88,65); 2,5023 (114,2); 2,4977 (80,72); 2,4932 (37,85); 2,3335 (0,59); 2,329 (0,89); 2,3242 (0,87); 2,3125 (16); 0,0079 (0,39); -0,0002 (10,47) |
| Verbindung Nr. I-1-14, Lösungsmittel: [DMSO], Spektrometer: 399.95MHz |
| 11,0908 (2,63); 9,1089 (2,1); 8,7242 (1,55); 8,7151 (1,52); 8,3265 (1,07); 8,3218 (1,57); 8,3169 (1,12); 8,3065 (1,17); 8,3019 (1,61); 8,2972 (1,03); 7,7865 (3,78); 7,7784 (4,51); 7,7067 (4,83); 7,6986 (3,88); 7,5272 (1,42); 7,5147 (1,46); 7,5071 (1,39); 7,495 (1,31); 6,5593 (4,12); 5,5694 (10,83); 3,902 (2,03); 3,3251 (92,11); 2,6755 (0,59); 2,6711 (0,82); 2,6667 (0,59); 2,5414 (0,52); 2,5243 (2,38); 2,511 (53,83); 2,5066 (106,69); 2,502 (137,88); 2,4974 (97,26); 2,4929 (45,4); 2,3388 (16); 2,329 (1,45); 2,3244 (0,85); 1,2346 (0,56); 0,0079 (0,51); -0,0002 (14,27); -0,0085 (0,42) |
| Verbindung Nr. I-1-44, Lösungsmittel: [DMSO], Spektrometer: 399.95MHz |
| 11,3077 (3,35); 9,1414 (2,45); 9,1368 (2,41); 9,0294 (2,55); 9,0275 (2,92); 9,0244 (2,91); 8,8361 (3,14); 8,832 (2,96); 8,7539 (1,68); 8,7504 (1,78); 8,7421 (1,74); 8,7385 (1,69); 8,3608 (1,19); 8,3558 (1,66); 8,351 (1,17); 8,3408 (1,27); 8,3357 (1,72); 8,331 (1,13); 8,3153 (0,36); 7,5555 (1,45); 7,5435 (1,46); 7,5367 (1,42); 7,5234 (1,33); 6,8349 (4,36); 3,9022 (2,26); 3,3238 (106,68); 2,6754 (0,64); 2,6708 (0,89); 2,6663 (0,66); 2,541 (0,8); 2,5239 (3,19); 2,5107 (56,29); 2,5063 (109,93); 2,5018 (141,79); 2,4972 (100,86); 2,4927 (47,64); 2,333 (0,65); 2,3286 (0,86); 2,3239 (0,67); 2,3192 (0,41); 2,297 (16); 1,2983 (0,39); 1,2586 (0,53); 1,2327 (0,45); -0,0002 (7,49) |
| Verbindung Nr. I-1-34, Lösungsmittel: [DMSO], Spektrometer: 399.95MHz |
| 11,2383 (2,69); 9,1427 (1,93); 8,7474 (1,38); 8,7384 (1,36); 8,6105 (2,67); 8,6066 (2,86); 8,5988 (2,83); 8,5949 (2,78); 8,3592 (1,08); 8,3544 (1,52); 8,3495 (1,05); 8,3392 (1,15); 8,3342 (1,57); 8,3295 (1,05); 8,3153 (0,34); 8,286 (2,76); 8,2821 (2,78); 8,2658 (3,05); 8,2619 (2,84); 7,6715 (2,98); 7,6598 (2,82); 7,6513 (2,71); 7,6396 (2,69); 7,5532 (1,26); 7,5406 (1,27); 7,5336 (1,24); 7,5214 (1,16); 6,7727 (4,14); 3,9023 (2,38); 3,3258 (88,39); 2,6757 (0,56); 2,6711 (0,74); 2,6665 (0,55); 2,5414 (0,58); 2,5244 (2,47); 2,5111 (48,22); 2,5066 (94,42); 2,5021 (121,68); 2,4975 (85,81); 2,4929 (39,88); 2,3334 (0,48); 2,3288 (0,7); 2,3243 (0,47); 2,2048 (16); 2,2036 (15,87); 1,2829 (0,39); 1,2347 (0,5); 0,8758 (0,64); 0,8626 (0,46); 0,8569 (0,39); 0,008 (0,42); -0,0002 (11,59) |
| Verbindung Nr. I-1-45, Lösungsmittel: [DMSO], Spektrometer: 399.95MHz |
| 11,4154 (3,59); 9,1646 (2,46); 9,1602 (2,38); 8,886 (2,48); 8,8843 (2,46); 8,8803 (2,42); 8,7712 (1,66); 8,7676 (1,72); 8,7593 (1,74); 8,7557 (1,64); 8,3948 (1,52); 8,3885 (1,53); 8,3798 (1,46); 8,3738 (2,86); 8,3665 (1,87); 8,36 (1,49); 8,3552 (1,85); 8,3501 (1,24); 8,3154 (0,34); 8,0268 (2,68); 8,0049 (2,42); 7,5742 (1,46); 7,5616 (1,48); 7,5543 (1,43); 7,5423 (1,37); 6,887 (4,52); 3,9025 (1,8); 3,3257 (99,06); 2,7266 (16); 2,6761 (0,57); 2,6715 (0,77); 2,667 (0,57); 2,5418 (0,53); 2,5248 (2,29); 2,5115 (50,31); 2,5071 (98,93); 2,5026 (127,7); 2,498 (90,93); 2,4935 (43,5); 2,3338 (0,57); 2,3293 (0,76); 2,3247 (0,55); 1,3514 (0,34); 1,2982 (0,61); 1,2589 (0,86); 1,2313 (0,6); 0,0079 (0,48); -0,0002 (12,55); -0,0085 (0,44) |
| Verbindung Nr. I-1-43, Lösungsmittel: [DMSO], Spektrometer: 399.95MHz |
| 11,0618 (6,27); 9,1012 (4,76); 8,7281 (3,38); 8,7195 (3,37); 8,3152 (3,28); 8,3103 (3,61); 8,3054 (2,51); 8,2953 (2,79); 8,2902 (3,72); 8,2855 (2,45); 7,5334 (3,06); 7,5221 (3,12); 7,5138 (2,98); 7,5025 (2,76); 6,3135 (12,89); 4,9991 (16); 4,9928 (15,69); 3,9022 (6,47); 3,4126 (5,02); 3,4065 (11,29); 3,4003 (4,65); 3,3712 (0,46); 3,3243 (165,08); 3,1749 (0,33); 3,1619 (0,37); 2,6757 (1,25); 2,671 (1,68); 2,6664 (1,23); 2,6619 (0,62); 2,5681 (0,4); 2,5636 (0,51); 2,5413 (1,29); 2,5242 (5,35); 2,5109 (109,53); 2,5065 (213,36); 2,5019 (273); 2,4973 (191,54); 2,4928 (89,2); 2,3377 (0,54); 2,3332 (1,19); 2,3287 (1,59); 2,3241 (1,17); 1,9857 (0,79); 1,9728 (1,67); 1,9645 (1,89); 1,9522 (3,44); 1,9399 (2,01); 1,9317 (1,8); 1,9189 (0,88); 1,3363 (0,45); 1,2982 (0,92); 1,2587 (1,35); 1,2354 (1,09); 1,225 (0,74); 1,0196 (2,45); 1,0088 (6,64); 1,0033 (6,89); 0,9988 (3,53); 0,993 (3,67); 0,9879 (6,48); 0,9824 (6,65); 0,9723 (2,57); 0,9575 (0,41); 0,9355 (0,36); 0,8783 (0,41); 0,8556 (0,35); 0,6928 (2,85); 0,6826 (7,26); 0,6772 (7,23); 0,6701 (7); 0,6648 (7,34); 0,654 (2,26); 0,008 (1); -0,0002 (28,42); -0,0087 (0,78) |
| Verbindung Nr. I-1-40, Lösungsmittel: [DMSO], Spektrometer: 399.95MHz |
| 11,0644 (3,41); 9,116 (2,49); 8,7334 (1,77); 8,7244 (1,75); 8,3295 (1,35); 8,3246 (1,95); 8,3199 (1,36); 8,3152 (0,73); 8,3096 (1,48); 8,3045 (2,02); 8,3 (1,31); 7,5393 (1,63); 7,5272 (1,65); 7,5194 (1,61); 7,5074 (1,47); 6,5219 (6,07); 5,0353 (0,43); 5,0289 (0,42); 4,9031 (8,57); 4,8968 (8,46); 3,9022 (2,39); 3,4991 (0,35); 3,4128 (2,83); 3,4067 (6,51); 3,4005 (2,64); 3,3249 (89,02); 2,8388 (0,34); 2,8198 (0,33); 2,7138 (1,64); 2,6947 (5,09); 2,6758 (5,74); 2,6664 (0,87); 2,6572 (1,75); 2,6287 (0,49); 2,6096 (0,5); 2,5414 (0,59); 2,5243 (2,43); 2,5109 (52,34); 2,5065 (102,48); 2,502 (131,97); 2,4974 (93,85); 2,4929 (44,4); 2,4098 (0,38); 2,3333 (0,59); 2,3287 (0,79); 2,3242 (0,56); 1,2981 (0,46); 1,2583 (1,03); 1,251 (7,43); 1,2427 (1,44); 1,2322 (16); 1,2133 (7,22); 1,1974 (0,96); 1,1931 (0,37); 1,1783 (0,49); 0,0079 (0,42); -0,0002 (12,46); -0,0086 (0,38) |
| Verbindung Nr. I-1-54, Lösungsmittel: [DMSO], Spektrometer: 399.95MHz |
| 11,1808 (5,05); 9,1292 (3,95); 8,7395 (2,81); 8,7301 (2,77); 8,3444 (2,25); 8,3396 (3,24); 8,3348 (2,22); 8,3245 (2,42); 8,3195 (3,43); 8,3149 (2,66); 7,7397 (8,3); 7,7339 (8,23); 7,5451 (2,7); 7,5331 (2,75); 7,5257 (2,68); 7,5137 (2,47); 6,6748 (7,23); 6,6691 (7,09); 4,9883 (16); 4,982 (15,93); 3,9021 (2,7); 3,4869 (4,61); 3,4806 |
| (10,07); 3,4743 (4,34); 3,3254 (110,21); 3,1751 (1,31); 3,1622 (1,31); 2,6756 (0,78); 2,6712 (1,05); 2,6666 (0,77); 2,5413 (0,81); 2,5243 (2,85); 2,5109 (66,35); 2,5066 (132,31); 2,502 (171,79); 2,4975 (123,01); 2,4931 (58,76); 2,3332 (0,73); 2,3289 (0,99); 2,3244 (0,74); 0,0079 (0,53); -0,0002 (16,36); -0,0084 (0,53) |
| Verbindung Nr. I-1-79 (Synthesebeispiel 7), Lösungsmittel: [DMSO], Spektrometer: 399.95MHz |
| 11,4076 (2,83); 9,6377 (2,26); 9,6348 (2,69); 9,6323 (2,61); 9,6293 (2,16); 9,4434 (2,13); 9,4407 (2,08); 9,4302 (2,21); 9,4274 (2,11); 8,1351 (2,08); 8,1292 (2,08); 8,1218 (2,06); 8,1159 (1,97); 7,3666 (1,63); 7,3628 (0,71); 7,3492 (4,47); 7,3303 (3,48); 7,2953 (1,78); 7,2822 (0,71); 7,277 (2,17); 7,2708 (0,51); 7,2587 (0,65); 7,1551 (3,8); 7,1374 (3,23); 6,552 (4,86); 5,25 (8,33); 3,9022 (3,49); 3,3257 (84,46); 3,1676 (0,35); 2,6755 (0,53); 2,671 (0,7); 2,6664 (0,5); 2,5415 (0,54); 2,5243 (2,04); 2,5109 (45,85); 2,5065 (89,88); 2,502 (115,63); 2,4974 (82,09); 2,4929 (38,81); 2,3332 (0,51); 2,3288 (0,69); 2,3241 (0,49); 2,2757 (16); 1,2349 (0,37); 0,0081 (0,38); -0,0002 (11); -0,0086 (0,33) |
| Verbindung Nr. I-1-6 : Masse (m/z): 294,1 (M+H)⁺; 1H-NMR (CD₃CN) 2.28 (s, 3H), 5.27 (s, 2H), 6.54 (s, 1H), 6.98 - 7.00 (m, 1H), 7.21 - 7.24 (m, 1H), 7.40 - 7.44 (m, 1H), 7.67 - 7.71 (m, 1H), 8.17 - 8.20 (m, 1H), 8.50 - 8.51 (m, 1H), 8.69 - 8.70 (m, 1H), 8.94 (br. s, 1H), 9.04 (s, 1H). |
| Verbindung Nr. I-1-66, Lösungsmittel: [DMSO], Spektrometer: 399.95MHz |
| 11,2631 (2,78); 9,317 (6,75); 9,2585 (12,79); 8,315 (0,33); 7,3694 (1,11); 7,3665 (1,77); 7,3625 (0,8); 7,349 (4,74); 7,3456 (2,63); 7,3302 (3,8); 7,3135 (0,34); 7,2947 (1,92); 7,2915 (1,24); 7,2818 (0,85); 7,2765 (2,34); 7,2703 (0,69); 7,2581 (0,8); 7,155 (3,77); 7,1373 (3,19); 7,1137 (0,38); 7,0944 (0,35); 6,5403 (4,59); 5,2434 (8,43); 5,1764 (0,39); 5,155 (0,46); 3,9021 (4,04); 3,3309 (71,79); 3,3282 (71,38); 2,6758 (0,51); 2,6711 (0,69); 2,6665 (0,49); 2,5413 (0,49); 2,5244 (2,21); 2,5111 (44,79); 2,5067 (87,23); 2,5021 (111,67); 2,4975 (78,96); 2,493 (37,19); 2,3333 (0,49); 2,3288 (0,68); 2,3242 (0,48); 2,2735 (16); 2,204 (0,6); 2,1976 (0,33); 2,1847 (0,9); 1,8664 (0,35); 1,2983 (0,75); 1,2587 (1,06); 0,0079 (0,43); -0,0002 (11,6); -0,0086 (0,35) |
| Verbindung Nr. I-1-56, Lösungsmittel: [DMSO], Spektrometer: 399.95MHz |
| 11,0446 (5,25); 9,0931 (3,53); 8,7173 (2,57); 8,7078 (2,53); 8,3152 (0,82); 8,3076 (1,94); 8,3031 (2,84); 8,2982 (1,91); 8,2877 (2,08); 8,2829 (2,91); 8,2784 (1,87); 7,5194 (2,43); 7,5077 (2,47); 7,5002 (2,4); 7,4881 (2,23); 7,3701 (3,46); 7,3662 (1,56); 7,3525 (9,31); 7,3493 (5,09); 7,3336 (7,56); 7,3201 (1,94); 7,2961 (3,59); 7,293 (2,42); 7,2832 (1,47); 7,2778 (4,45); 7,2717 (1,13); 7,2595 (1,42); 7,1993 (7,64); 7,1817 (6,15); 6,339 (10,52); 5,3572 (16); 4,3241 (0,49); 4,3086 (0,46); 3,9021 (6,07); 3,3257 (166,84); 3,1752 (0,43); 3,1624 (0,42); 2,6799 (0,44); 2,6754 (0,9); 2,671 (1,22); 2,6664 (0,89); 2,5412 (1,03); 2,5242 (3,83); 2,5109 (81,5); 2,5065 (160,87); 2,5019 (207,93); 2,4973 (147,4); 2,4928 (69,4); 2,3333 (0,89); 2,3286 (1,19); 2,3242 (0,88); 1,9541 (0,63); 1,9416 (1,33); 1,933 (1,53); 1,9207 (2,73); 1,9084 (1,68); 1,9001 (1,41); 1,8875 (0,7); 1,2983 (0,79); 1,2586 (1,16); 1,2351 (0,76); 0,9784 (0,34); 0,9609 (1,97); 0,9501 (5,29); 0,9446 (5,46); 0,9402 (2,92); 0,9344 (2,9); 0,9293 (5,1); 0,9238 (5,35); 0,9138 (2,01); 0,8385 (0,38); 0,8321 (0,33); 0,8262 (0,37); 0,6498 (2,22); 0,6395 (5,82); 0,6343 (5,89); 0,6271 (5,65); 0,6219 (5,86); 0,611 (1,77); 0,008 (0,69); -0,0002 (21,48); -0,0085 (0,62) |
| Verbindung Nr. I-1-58, Lösungsmittel: [DMSO], Spektrometer: 399.95MHz |
| 11,0606 (3,98); 9,1004 (2,94); 8,7236 (2,12); 8,7141 (2,13); 8,3142 (2,26); 8,308 (1,53); 8,293 (6,09); 8,2877 (5,35); 7,6011 (1,76); 7,5951 (1,7); 7,5805 (3,02); 7,5744 (3); 7,527 (7,62); 7,5154 (2,1); 7,5064 (4,97); 7,4958 (1,74); 7,3915 (0,33); 6,5729 (6,61); 5,3012 (12,17); 3,9022 (1,06); 3,5113 (0,37); 3,4136 (0,34); 3,3956 (0,4); 3,3274 (248,86); 3,1752 (0,6); 3,1624 (0,52); 2,7009 (1,89); 2,6822 (6); 2,6713 (1,99); 2,6635 (6,27); 2,6445 (2,02); 2,6165 (0,32); 2,571 (0,34); 2,5415 (1,07); 2,5243 (4,49); 2,511 (93,98); 2,5068 (177,56); 2,5024 (223,43); 2,4979 (158,87); 2,4938 (76,61); 2,3335 (0,96); 2,3292 (1,23); 2,3245 (0,92); 1,2984 (3,97); 1,2588 (5,41); 1,2351 (2,63); 1,2057 (7,68); 1,1869 (16); 1,1682 (7,19); 1,1309 (0,42); 0,8541 (0,42); 0,0078 (0,63); -0,0002 (17,04); - 0,0081 (0,59) |
| Verbindung Nr. I-1-26: Masse (m/z): 280,1 (M+H)⁺; 1H-NMR (DMSO-D₆) 5.36 (s, 2H), 6.68 (s, 1H), 7.10 - 7.12 (m, 1H), 7.28 - 7.31 (m, 1H), 7.46 - 7.50 (m, 1H), 7.74 - 7.80 (m, 2H), 8.28 - 8.31 (m, 1H), 8.52 - 8.53 (m, 1H), 8.69 - 8.70 (m, 1H), 9.10 (s, 1H), 10.90 (s, 1H). |
| Verbindung Nr. I-1-72, Lösungsmittel: [DMSO], Spektrometer: 399.95MHz |
| 11,1127 (2,35); 9,1049 (2,26); 9,1005 (2,23); 8,7211 (1,61); 8,7174 (1,69); 8,7092 (1,68); 8,7055 (1,58); 8,3223 (1,02); 8,3176 (1,48); 8,313 (1,02); 8,3025 (1,09); 8,2976 (1,49); 8,2929 (0,95); 7,8201 (3,67); 7,8145 (3,59); 7,523 (1,34); 7,5111 (1,37); 7,5032 (1,31); 7,4911 (1,23); 7,2525 (1,23); 7,2337 (3,05); 7,2148 (1,99); 7,1144 (1,82); 7,0949 (1,38); 7,0722 (2,88); 7,0521 (1,74); 7,0338 (1,42); 6,6721 (3,21); 6,6666 (3,12); 5,2248 (9,11); 3,902 (1,57); 3,3284 (127,81); 3,1753 (0,52); 3,1625 (0,5); 2,6756 (0,45); 2,671 (0,62); 2,6666 (0,45); 2,5412 (0,4); 2,5243 (1,91); 2,511 (40,97); 2,5066 (79,71); 2,502 (102,01); 2,4975 (72,3); 2,4931 (34,21); 2,3332 (0,46); 2,3288 (0,61); 2,3243 (0,45); 2,2805 (16); 0,008 (0,34); -0,0002 (9,66) |
| Verbindung Nr. I-1-84, Lösungsmittel: [DMSO], Spektrometer: 399.95MHz |
| 11,1184 (4); 9,1045 (3,67); 9,1001 (3,64); 8,721 (2,58); 8,7174 (2,77); 8,7092 (2,76); 8,7055 (2,62); 8,3217 (1,66); 8,316 (2,57); 8,3122 (1,68); 8,3018 (1,84); 8,2968 (2,51); 8,2922 (1,65); 7,8373 (6,75); 7,8316 (6,69); 7,5217 (2,2); 7,5096 (2,28); 7,5031 (2,2); 7,4911 (2,09); 7,3746 (1,55); 7,371 (2,7); 7,3671 (1,19); 7,3583 (1,98); 7,3539 (7,29); 7,3501 (4,43); 7,3387 (2,93); 7,3354 (6,94); 7,3114 (1,63); 7,3077 (3,32); 7,3042 (2,36); 7,2957 (1,15); 7,2897 (3,88); 7,2825 (0,98); 7,275 (0,82); 7,2714 (1,24); 7,2678 (0,89); 7,2584 (5,94); 7,2547 (7,02); 7,2377 (5); |
| 6,6794 (5,7); 6,6738 (5,6); 5,2753 (16); 3,9021 (3,66); 3,3259 (181,81); 3,174 (0,8); 3,1627 (0,76); 2,7549 (0,54); 2,6754 (0,89); 2,6708 (1,2); 2,6662 (0,89); 2,6619 (0,42); 2,5411 (0,86); 2,5242 (3,57); 2,5108 (77,77); 2,5064 (153,77); 2,5018 (198,62); 2,4972 (141,08); 2,4927 (66,65); 2,3376 (0,42); 2,3331 (0,88); 2,3286 (1,17); 2,324 (0,87); 0,9869 (0,48); 0,0079 (0,78); -0,0002 (20,79); -0,0085 (0,68) |
| Verbindung Nr. I-1-9: Masse (m/z): 281,1 (M+H)⁺; 1H-NMR (DMSO-D₆) 5.46 (s, 2H), 6.67 (s, 1H), 7.41 - 7.49 (m, 2H), 7.78 (s, 1H), 8.28 - 8.31 (m, 1H), 8.61- 8.78 (m, 3H), 9.10 (s, 1H), 10.87 (s, 1H). |
| Verbindung Nr. I-1-21, Lösungsmittel: [DMSO], Spektrometer: 399.95MHz |
| 11,037 (2,51); 9,1001 (1,66); 8,7229 (1,2); 8,7131 (1,19); 8,3154 (1,41); 8,311 (1,58); 8,3036 (3,11); 8,2967 (3,42); 7,6155 (1,31); 7,6094 (1,27); 7,5949 (2,05); 7,5888 (2); 7,5293 (4,09); 7,5091 (3,21); 7,4916 (1,03); 6,5436 (4,09); 5,2895 (8,39); 3,9023 (3,01); 3,3244 (69,56); 2,6759 (0,65); 2,6712 (0,85); 2,6666 (0,62); 2,5414 (0,72); 2,5242 (2,98); 2,5111 (55,19); 2,5067 (106,8); 2,5022 (136,24); 2,4976 (96,59); 2,4932 (45,64); 2,3334 (0,69); 2,3288 (0,97); 2,3241 (0,84); 2,31 (16); 1,2983 (0,83); 1,2587 (1,2); 1,2352 (0,35); 0,008 (0,46); -0,0002 (13,73); - 0,0086 (0,4) |
| Verbindung Nr. I-1-49, Lösungsmittel: [DMSO], Spektrometer: 399.95MHz |
| 11,0029 (1,84); 9,1119 (2,05); 9,1073 (1,98); 8,7123 (1,46); 8,7085 (1,46); 8,7004 (1,51); 8,6966 (1,37); 8,3325 (0,88); 8,3276 (1,26); 8,3229 (0,85); 8,3125 (1); 8,3066 (2,24); 8,3032 (0,91); 7,5137 (1,13); 7,5016 (1,14); 7,4938 (1,11); 7,4818 (1,03); 6,4307 (3,61); 4,504 (0,42); 4,4876 (1,09); 4,4712 (1,49); 4,4548 (1,11); 4,4384 (0,42); 3,8966 (0,77); 3,3294 (112,76); 3,325 (112,9); 2,6771 (0,44); 2,6726 (0,59); 2,6681 (0,42); 2,5427 (0,46); 2,5257 (1,86); 2,5125 (40,15); 2,5081 (77,22); 2,5035 (98,5); 2,499 (69,54); 2,4945 (32,61); 2,3348 (0,46); 2,3303 (0,62); 2,3257 (0,45); 2,2748 (14,07); 1,3736 (16); 1,3572 (15,83); 0,0078 (1,06); -0,0002 (25,49); -0,0085 (0,79) |
| Verbindung Nr. I-1-110, Lösungsmittel: [DMSO], Spektrometer: 399.95MHz |
| 10,8332 (1,98); 8,8059 (1,92); 8,8003 (1,91); 8,2612 (1,19); 8,2551 (1,16); 8,2394 (1,24); 8,2332 (1,22); 7,1931 (8,25); 7,178 (3,53); 7,1722 (4,02); 7,1567 (0,34); 7,1504 (0,51); 6,8984 (2,08); 6,8766 (2,04); 6,5068 (2,91); 5,2152 (4,91); 3,913 (16); 3,9023 (2,4); 3,8931 (0,39); 3,326 (50,47); 2,676 (0,36); 2,6715 (0,49); 2,667 (0,37); 2,5416 (0,35); 2,5244 (1,58); 2,5112 (33,62); 2,5068 (65,38); 2,5023 (84,13); 2,4978 (60,68); 2,4934 (29,67); 2,3336 (0,38); 2,3289 (0,51); 2,3246 (0,39); 2,2595 (10,18); -0,0002 (8,02) |
| Verbindung Nr. I-1-97, Lösungsmittel: [DMSO], Spektrometer: 399.95MHz |
| 11,2093 (3,14); 8,8997 (4,1); 8,8944 (4,16); 8,6305 (4,44); 8,6252 (4,2); 8,3154 (0,34); 7,2215 (0,39); 7,1964 (8,21); 7,1842 (4,49); 7,1751 (5,62); 7,1682 (0,8); 7,1595 (0,48); 7,152 (0,82); 6,5224 (4,79); 5,2263 (7,99); 3,9024 (3,64); 3,3274 (128,85); 3,1747 (0,52); 3,1626 (0,51); 2,6759 (0,61); 2,6713 (0,82); 2,6669 (0,59); 2,5415 (0,67); 2,5245 (2,77); 2,5113 (56,37); 2,5068 (110,37); 2,5023 (141,89); 2,4977 (100,95); 2,4932 (47,93); 2,3381 (0,34); 2,3336 (0,62); 2,3292 (0,86); 2,3245 (0,64); 2,2721 (16); 0,0079 (0,53); -0,0002 (14,91); -0,0086 (0,42) |
| Verbindung Nr. I-1-109, Lösungsmittel: [DMSO], Spektrometer: 399.95MHz |
| 10,9441 (2,67); 8,9981 (2,68); 8,993 (2,6); 8,3158 (0,32); 8,2207 (1,57); 8,2149 (1,52); 8,2004 (1,66); 8,1947 (1,59); 7,3651 (2,61); 7,3449 (2,48); 7,2186 (0,36); 7,1942 (10,41); 7,1802 (5,03); 7,1766 (3,17); 7,1729 (5,88); 7,1655 (0,72); 7,1573 (0,49); 7,1498 (0,73); 6,5183 (4,35); 5,2185 (8,04); 3,9024 (3,14); 3,3293 (30,53); 3,1686 (1,16); 2,6806 (0,33); 2,6754 (0,55); 2,6709 (0,7); 2,6664 (0,51); 2,541 (0,93); 2,5245 (20,21); 2,5108 (49,12); 2,5065 (93,73); 2,5019 (119,35); 2,4974 (85,14); 2,4929 (40,77); 2,3332 (0,53); 2,3287 (0,7); 2,3242 (0,51); 2,2628 (16); 0,0079 (0,53); -0,0002 (13,22); -0,0085 (0,41) |
| Verbindung Nr. I-1-11: Masse (m/z): 242,1 (M+H)⁺; 1H-NMR (DMSO-D₆) 2.33 (s, 3H), 5.30 (s, 2H), 6.55 (s, 1H), 7.39 - 7.51 (m, 1H), 8.29 - 8.32 (m, 1H), 8.70 - 8.72 (m, 1H), 9.11 (s, 1H), 10.90 (s, 1H). |
| Verbindung Nr. I-1-83, Lösungsmittel: [DMSO], Spektrometer: 399.95MHz |
| 11,1519 (2,97); 9,0715 (2,85); 9,0674 (2,79); 8,8691 (3,07); 8,8639 (3,04); 8,5627 (2,08); 8,5578 (3,26); 8,5529 (1,82); 8,3156 (0,34); 7,2232 (0,42); 7,1971 (7,73); 7,1852 (4,3); 7,1756 (5,44); 7,1596 (0,49); 7,1527 (0,79); 6,5229 (4,58); 5,2252 (8,08); 3,9024 (2,68); 3,3252 (73,75); 2,6756 (0,62); 2,6712 (0,82); 2,6667 (0,6); 2,5414 (0,6); 2,5109 (57,73); 2,5067 (110,15); 2,5022 (140,04); 2,4977 (100,57); 2,4934 (49,11); 2,3334 (0,62); 2,3289 (0,83); 2,3244 (0,61); 2,2696 (16); 0,0079 (0,49); -0,0002 (13,21); -0,0084 (0,46) |
| Verbindung Nr. I-1-95, Lösungsmittel: [DMSO], Spektrometer: 399.95MHz |
| 11,1282 (2,97); 8,9443 (2,92); 8,9386 (2,87); 8,3634 (1,62); 8,3574 (1,58); 8,3426 (1,73); 8,3365 (1,65); 7,6565 (3,18); 7,6357 (3,01); 7,2195 (0,41); 7,1956 (9,77); 7,1815 (4,75); 7,1739 (5,42); 7,1584 (0,49); 7,1517 (0,75); 6,5231 (4,45); 5,2231 (7,89); 3,9022 (3,7); 3,3294 (84,6); 2,6716 (0,73); 2,6677 (0,56); 2,542 (0,87); 2,5071 (102,51); 2,5028 (127,82); 2,4985 (94,93); 2,3338 (0,61); 2,3296 (0,77); 2,2685 (16); -0,0002 (9,94); -0,0084 (0,46) |
| Verbindung Nr. I-1-62, Lösungsmittel: [DMSO], Spektrometer: 399.95MHz |
| 11,0412 (2,7); 9,106 (2,43); 8,7185 (1,75); 8,7095 (1,73); 8,3187 (1,57); 8,2986 (1,57); 7,5228 (1,39); 7,5109 (1,43); 7,5032 (1,39); 7,4912 (1,25); 7,2212 (0,43); 7,1971 (8,56); 7,1838 (4,47); 7,1753 (5,18); 7,1595 (0,47); 7,1523 (0,77); 6,5316 (4,21); 5,2246 (8,43); 3,9023 (1,31); 3,324 (63,08); 2,6752 (0,5); 2,6707 (0,61); 2,5059 (84,65); 2,5018 (105,45); 2,4975 (78,62); 2,328 (0,65); 2,2681 (16); -0,0002 (4,07) |
| Verbindung Nr. I-1-94, Lösungsmittel: [DMSO], Spektrometer: 399.95MHz |
| 10,8384 (1,81); 8,809 (1,8); 8,8034 (1,77); 8,2646 (1,21); 8,2584 (1,15); 8,2428 (1,25); 8,2366 (1,21); 7,4241 |
| (0,49); 7,4088 (0,65); 7,4041 (0,91); 7,3889 (1,01); 7,3846 (0,73); 7,3691 (0,66); 7,1384 (0,45); 7,1319 (0,47); 7,1173 (0,76); 7,111 (0,78); 7,0949 (0,4); 7,09 (0,39); 6,9745 (1,18); 6,9528 (1,49); 6,946 (0,79); 6,9268 (0,68); 6,921 (0,76); 6,9008 (1,97); 6,8789 (1,92); 6,5246 (2,75); 5,2564 (4,72); 3,9141 (16); 3,9025 (1,74); 3,8934 (0,43); 3,3254 (60,55); 2,6756 (0,37); 2,6711 (0,49); 2,6665 (0,37); 2,5412 (0,39); 2,5244 (1,73); 2,5111 (32,77); 2,5067 (64,28); 2,5021 (82,83); 2,4975 (58,95); 2,4931 (27,93); 2,3334 (0,37); 2,3288 (0,48); 2,3244 (0,36); 2,2605 (9,56); -0,0002 (8,37) |
| Verbindung Nr. I-1-96, Lösungsmittel: [DMSO], Spektrometer: 399.95MHz |
| 11,2124 (3,08); 8,9024 (3,94); 8,8972 (3,92); 8,6353 (4,23); 8,63 (3,93); 8,3157 (0,36); 7,4255 (0,76); 7,41 (1,07); 7,4055 (1,45); 7,3902 (1,65); 7,386 (1,17); 7,3704 (1,09); 7,1419 (0,79); 7,1367 (0,8); 7,1206 (1,33); 7,1147 (1,29); 7,0997 (0,67); 7,0938 (0,65); 6,9811 (1,9); 6,9601 (2,66); 6,9296 (1,22); 6,5407 (4,72); 5,2676 (8,13); 3,9023 (3,41); 3,3244 (84,01); 2,6756 (0,68); 2,6711 (0,87); 2,6667 (0,63); 2,5413 (0,66); 2,511 (61,26); 2,5066 (117,71); 2,5021 (149,82); 2,4975 (106,16); 2,4931 (50,3); 2,3376 (0,36); 2,3333 (0,65); 2,3288 (0,89); 2,3242 (0,62); 2,2727 (16); 0,9866 (0,33); 0,0079 (0,6); -0,0002 (16,72); -0,0086 (0,49) |
| Verbindung Nr. I-1-108, Lösungsmittel: [DMSO], Spektrometer: 399.95MHz |
| 10,9469 (2,69); 9,0002 (2,68); 8,9951 (2,58); 8,3156 (0,38); 8,2218 (1,7); 8,216 (1,61); 8,2015 (1,75); 8,1957 (1,69); 7,4244 (0,82); 7,4089 (1,12); 7,4043 (1,53); 7,389 (1,68); 7,3847 (1,22); 7,3652 (2,81); 7,3449 (2,57); 7,1388 (0,77); 7,1332 (0,79); 7,1175 (1,28); 7,1113 (1,27); 7,0954 (0,64); 7,0906 (0,63); 6,9766 (1,93); 6,9553 (2,57); 6,9485 (1,29); 6,9295 (1,09); 6,9252 (1,19); 6,5361 (4,26); 5,2595 (8,02); 3,9023 (2,53); 3,3264 (71,21); 2,6804 (0,34); 2,6756 (0,58); 2,671 (0,78); 2,6664 (0,57); 2,5413 (0,99); 2,5249 (20,48); 2,52 (6,48); 2,511 (53,03); 2,5065 (103,25); 2,502 (132,69); 2,4974 (94,31); 2,4929 (44,68); 2,3378 (0,32); 2,3333 (0,59); 2,3287 (0,8); 2,3241 (0,58); 2,264 (16); 0,008 (0,56); -0,0002 (15,52); -0,0085 (0,47) |
| Verbindung Nr. I-1-107, Lösungsmittel: [DMSO], Spektrometer: 399.95MHz |
| 11,1546 (2,87); 9,0735 (2,92); 9,0692 (2,85); 8,87 (3,2); 8,8646 (3,21); 8,5668 (2,11); 8,5619 (3,36); 8,5568 (1,85); 8,3148 (0,52); 7,4262 (0,8); 7,4108 (1,07); 7,4061 (1,52); 7,3909 (1,67); 7,3865 (1,17); 7,3711 (1,1); 7,1418 (0,73); 7,1367 (0,76); 7,1205 (1,28); 7,1143 (1,29); 7,0983 (0,66); 7,0933 (0,63); 6,9823 (1,86); 6,9608 (2,36); 6,954 (1,3); 6,9294 (1,18); 6,5406 (4,45); 5,2661 (8,13); 3,9021 (3,63); 3,3285 (158,06); 2,6758 (0,69); 2,6714 (0,95); 2,667 (0,69); 2,6626 (0,35); 2,5416 (0,7); 2,5247 (2,92); 2,5114 (61,69); 2,5069 (121); 2,5024 (155,49); 2,4978 (110,3); 2,4933 (52,34); 2,3377 (0,33); 2,3337 (0,67); 2,3291 (0,9); 2,3245 (0,66); 2,3201 (0,33); 2,2703 (16); 0,008 (0,46); -0,0002 (14,88); -0,0085 (0,44) |
| Verbindung Nr. I-1-106, Lösungsmittel: [DMSO], Spektrometer: 399.95MHz |
| 11,1321 (2,84); 8,9478 (2,82); 8,9422 (2,8); 8,3674 (1,76); 8,3612 (1,71); 8,3465 (1,87); 8,3403 (1,83); 8,3155 (0,36); 7,6585 (3,2); 7,6382 (3); 7,4247 (0,76); 7,4095 (1,05); 7,4048 (1,47); 7,3895 (1,65); 7,3853 (1,18); 7,3698 (1,05); 7,1403 (0,75); 7,1345 (0,79); 7,119 (1,28); 7,1129 (1,3); 7,0971 (0,63); 7,0921 (0,64); 6,9778 (1,88); 6,9572 (2,74); 6,9314 (1,06); 6,9261 (1,19); 6,541 (4,34); 5,2646 (8,07); 3,9023 (3,18); 3,3253 (80,71); 2,6757 (0,6); 2,6713 (0,8); 2,6666 (0,6); 2,5414 (0,74); 2,5244 (2,66); 2,5111 (52,56); 2,5067 (103,02); 2,5022 (132,9); 2,4976 (95,61); 2,4932 (46,43); 2,3335 (0,57); 2,329 (0,77); 2,3245 (0,56); 2,2693 (16); 0,008 (0,42); -0,0002 (13,86); -0,0085 (0,48) |
| Verbindung Nr. I-1-60, Lösungsmittel: [DMSO], Spektrometer: 399.95MHz |
| 11,0462 (2,49); 9,1286 (0,95); 8,738 (0,86); 8,3248 (1,23); 8,316 (0,77); 8,3043 (1,28); 7,5326 (0,82); 7,5208 (0,95); 7,5136 (0,94); 7,5023 (0,76); 7,4266 (0,71); 7,4068 (1,45); 7,3912 (1,6); 7,3874 (1,2); 7,3715 (0,99); 7,1406 (0,75); 7,1351 (0,82); 7,1194 (1,33); 7,1137 (1,32); 7,0979 (0,69); 7,092 (0,68); 6,9809 (1,95); 6,96 (2,73); 6,9287 (1,29); 6,55 (4); 5,2655 (7,52); 3,9023 (1,9); 3,3284 (81,27); 2,6718 (0,69); 2,6675 (0,55); 2,5417 (0,63); 2,5071 (93,68); 2,5027 (118,77); 2,4983 (87,57); 2,3336 (0,54); 2,3295 (0,69); 2,2694 (16); 0,0079 (0,37); -0,0002 (10,28) |
| Verbindung Nr. I-1-3: Masse (m/z): 217,1 (M+H)⁺; 1H-NMR (DMSO-D₆) 2.25 (s, 3H), 3.66 (s, 3H), 6.41 (s, 1H), 7.47 - 7.50 (m, 1H), 8.27 - 8.30 (m, 1H), 8.69 - 8.70 (m, 1H), 9.10 (s, 1H), 10.69 (s, 1H). |
| Verbindung Nr. I-1-105, Lösungsmittel: [DMSO], Spektrometer: 399.95MHz |
| 10,8212 (1,84); 8,7951 (1,8); 8,7899 (1,74); 8,2521 (1,21); 8,2458 (1,15); 8,2303 (1,25); 8,224 (1,2); 7,3731 (0,33); 7,3687 (0,62); 7,3483 (0,74); 7,3437 (0,44); 7,3342 (0,4); 7,3298 (0,38); 7,2488 (0,77); 7,2465 (0,84); 7,2282 (0,7); 7,2251 (0,8); 7,223 (0,87); 7,2202 (0,91); 7,2019 (0,71); 7,1981 (1,12); 7,1949 (0,74); 7,1788 (1,52); 7,1762 (1,35); 7,1602 (0,84); 7,1574 (0,74); 7,0453 (0,58); 7,0413 (0,57); 7,0261 (0,99); 7,0221 (0,96); 7,007 (0,47); 7,0031 (0,43); 6,891 (1,94); 6,89 (1,92); 6,8693 (1,87); 6,8681 (1,85); 6,5124 (2,75); 5,2628 (4,43); 3,9083 (16); 3,9024 (2,33); 3,8933 (0,39); 3,3267 (51,97); 2,6756 (0,35); 2,6709 (0,47); 2,6665 (0,34); 2,5413 (0,44); 2,5242 (1,74); 2,5109 (31,79); 2,5065 (61,6); 2,502 (78,77); 2,4974 (55,83); 2,4929 (26,27); 2,3332 (0,38); 2,3287 (0,5); 2,3241 (0,4); 2,3018 (9,31); -0,0002 (8,08) |
| Verbindung Nr. I-1-103, Lösungsmittel: [DMSO], Spektrometer: 399.95MHz |
| 11,1922 (3,14); 8,8872 (4,07); 8,8819 (4,06); 8,6202 (4,34); 8,6149 (4,11); 8,3153 (0,4); 7,3911 (0,41); 7,3866 (0,44); 7,3771 (0,5); 7,3728 (1,07); 7,3525 (1,24); 7,3481 (0,7); 7,3385 (0,65); 7,3342 (0,62); 7,2468 (1,39); 7,2233 (1,45); 7,2205 (1,47); 7,1997 (2,25); 7,1808 (2,45); 7,1783 (2,22); 7,1622 (1,35); 7,1596 (1,25); 7,0717 (0,94); 7,0682 (0,94); 7,0527 (1,6); 7,0492 (1,55); 7,0337 (0,71); 7,0301 (0,68); 6,5269 (4,84); 5,272 (7,79); |
| 3,9021 (2,28); 3,3246 (108,09); 3,1751 (0,8); 3,1621 (0,78); 2,6757 (0,64); 2,671 (0,87); 2,6665 (0,62); 2,5411 (0,6); 2,5244 (2,53); 2,511 (59,39); 2,5066 (116,61); 2,5021 (150,19); 2,4975 (107,66); 2,493 (51,85); 2,3333 (0,79); 2,3287 (1,19); 2,3159 (16); 0,008 (0,48); -0,0002 (15,2); -0,0085 (0,51) |
| Verbindung Nr. I-1-102, Lösungsmittel: [DMSO], Spektrometer: 399.95MHz |
| 10,9291 (2,71); 8,9851 (2,76); 8,9801 (2,64); 8,2075 (1,68); 8,2017 (1,57); 8,1872 (1,74); 8,1814 (1,65); 7,3876 (0,42); 7,3832 (0,49); 7,3689 (1,12); 7,3534 (3,55); 7,3442 (0,87); 7,3335 (3,06); 7,2487 (1,31); 7,2467 (1,35); 7,223 (1,49); 7,2206 (1,46); 7,1984 (1,84); 7,1789 (2,5); 7,1767 (2,21); 7,1604 (1,36); 7,1578 (1,2); 7,0497 (0,97); 7,0458 (0,95); 7,0305 (1,65); 7,0268 (1,56); 7,0113 (0,76); 7,0075 (0,69); 6,5239 (4,46); 5,2657 (7,8); 3,9021 (1,88); 3,3261 (86,91); 2,6755 (0,62); 2,671 (0,73); 2,6666 (0,52); 2,541 (0,65); 2,5182 (23,24); 2,5107 (52,33); 2,5064 (97,66); 2,5019 (122,92); 2,4974 (87,5); 2,4931 (42,05); 2,4654 (0,59); 2,3331 (0,64); 2,3286 (0,83); 2,3239 (0,73); 2,3056 (16); 0,0079 (0,42); -0,0002 (10,87); -0,0085 (0,34) |
| Verbindung Nr. I-1-101, Lösungsmittel: [DMSO], Spektrometer: 399.95MHz |
| 11,1372 (2,83); 9,0583 (2,95); 9,0541 (2,89); 8,861 (3,18); 8,8555 (3,16); 8,5523 (2,08); 8,5472 (3,33); 8,5422 (1,8); 7,3914 (0,42); 7,3873 (0,45); 7,3775 (0,5); 7,3727 (1,02); 7,3527 (1,21); 7,3481 (0,71); 7,3385 (0,63); 7,3343 (0,62); 7,2504 (1,3); 7,2482 (1,4); 7,2245 (1,43); 7,2219 (1,48); 7,2007 (2,16); 7,1815 (2,46); 7,179 (2,22); 7,163 (1,33); 7,1604 (1,21); 7,0682 (0,91); 7,0647 (0,91); 7,0492 (1,55); 7,0455 (1,52); 7,0298 (0,7); 6,5278 (4,59); 5,2714 (7,78); 3,9024 (2,89); 3,3248 (82,34); 3,1749 (0,6); 3,1619 (0,6); 2,6757 (0,62); 2,6711 (0,83); 2,6666 (0,62); 2,5412 (0,64); 2,5242 (2,67); 2,5109 (57,13); 2,5065 (112,07); 2,502 (144,38); 2,4974 (103,37); 2,493 (49,83); 2,3331 (0,74); 2,3287 (1,05); 2,3131 (16); 0,008 (0,44); -0,0002 (13,94); -0,0083 (0,45) |
| Verbindung Nr. I-1-93, Lösungsmittel: [DMSO], Spektrometer: 399.95MHz |
| 11,1147 (2,87); 8,932 (2,82); 8,9263 (2,82); 8,3529 (1,76); 8,3466 (1,71); 8,332 (1,84); 8,3257 (1,81); 8,3155 (0,42); 7,647 (3,19); 7,6261 (3,01); 7,3892 (0,41); 7,3847 (0,44); 7,3707 (1,03); 7,3506 (1,25); 7,346 (0,7); 7,3365 (0,64); 7,3323 (0,61); 7,2464 (1,4); 7,228 (1,1); 7,223 (1,42); 7,2203 (1,49); 7,1989 (2,16); 7,1798 (2,44); 7,1773 (2,25); 7,1612 (1,32); 7,1586 (1,24); 7,0601 (0,89); 7,0576 (0,91); 7,0418 (1,55); 7,0383 (1,52); 7,0225 (0,71); 7,019 (0,67); 6,5278 (4,49); 5,2698 (7,74); 3,9021 (2,23); 3,3243 (65,4); 3,1752 (0,53); 3,1621 (0,48); 2,6757 (0,61); 2,6711 (0,8); 2,6666 (0,59); 2,5412 (0,71); 2,5243 (2,55); 2,511 (51,35); 2,5066 (101,5); 2,5021 (131,31); 2,4975 (94,34); 2,4931 (45,52); 2,3332 (0,63); 2,3286 (0,9); 2,324 (0,89); 2,3119 (16); 0,008 (0,39); -0,0002 (13,77); -0,0085 (0,43) |
| Verbindung Nr. I-1-64, Lösungsmittel: [DMSO], Spektrometer: 399.95MHz |
| 11,0304 (2,52); 9,1001 (1,48); 8,7191 (1,12); 8,7096 (1,1); 8,3092 (1,35); 8,2893 (1,4); 7,517 (1,05); 7,5053 (1,11); 7,4975 (1,09); 7,4852 (0,96); 7,3899 (0,4); 7,3855 (0,42); 7,3711 (1,01); 7,3513 (1,24); 7,3465 (0,72); 7,3372 (0,64); 7,3328 (0,6); 7,2489 (1,34); 7,2253 (1,44); 7,2227 (1,48); 7,2009 (1,95); 7,1815 (2,41); 7,1792 (2,25); 7,1629 (1,32); 7,1606 (1,25); 7,0574 (0,9); 7,0538 (0,91); 7,038 (1,56); 7,0348 (1,52); 7,0194 (0,72); 6,5374 (4,24); 5,2717 (7,7); 3,9024 (1,78); 3,3264 (78,21); 2,6756 (0,5); 2,6713 (0,66); 2,6669 (0,48); 2,5416 (0,66); 2,5109 (45,32); 2,5066 (87,34); 2,5021 (111,86); 2,4976 (80,6); 2,4932 (39,32); 2,3334 (0,59); 2,3286 (0,8); 2,3241 (0,81); 2,3113 (16); 0,0078 (0,36); -0,0002 (9,58) |
| Verbindung Nr. I-1-104, Lösungsmittel: [DMSO], Spektrometer: 399.95MHz |
| 10,8316 (1,87); 8,8078 (1,93); 8,8021 (1,84); 8,2632 (1,22); 8,257 (1,14); 8,2414 (1,25); 8,2352 (1,21); 7,3631 (1,1); 7,3591 (0,52); 7,3455 (2,93); 7,3266 (2,26); 7,2903 (1,13); 7,2722 (1,38); 7,2659 (0,34); 7,2538 (0,43); 7,1473 (2,51); 7,1297 (2,11); 6,898 (2,04); 6,8762 (1,95); 6,5092 (2,89); 5,2287 (5,01); 3,9126 (16); 3,9022 (1,93); 3,8922 (0,35); 3,326 (66,93); 2,6756 (0,39); 2,6711 (0,51); 2,6665 (0,37); 2,541 (0,41); 2,5106 (35,11); 2,5064 (66,58); 2,5019 (84,65); 2,4974 (60,71); 2,4929 (29,41); 2,333 (0,38); 2,3287 (0,49); 2,324 (0,36); 2,2565 (10,02); -0,0002 (7,86) |
| Verbindung Nr. I-1-100, Lösungsmittel: [DMSO], Spektrometer: 399.95MHz |
| 11,209 (3,14); 8,9021 (4,1); 8,8968 (4,12); 8,6325 (4,43); 8,6272 (4,16); 8,315 (0,43); 7,3642 (1,79); 7,3603 (0,8); 7,3468 (4,66); 7,3434 (2,53); 7,328 (3,63); 7,2935 (1,82); 7,2903 (1,16); 7,281 (0,7); 7,2753 (2,24); 7,2692 (0,53); 7,257 (0,64); 7,1528 (3,8); 7,1352 (3,24); 6,5257 (4,74); 5,2395 (8,29); 3,902 (4,15); 3,3267 (93,65); 2,6758 (0,66); 2,6712 (0,88); 2,6667 (0,63); 2,5415 (0,59); 2,5244 (2,81); 2,5112 (59,24); 2,5068 (114,82); 2,5022 (147,01); 2,4976 (104,25); 2,4931 (49,44); 2,3333 (0,69); 2,329 (0,89); 2,3241 (0,63); 2,2686 (16); 1,2981 (1,02); 1,2587 (1,45); 1,2344 (0,53); 0,008 (0,57); -0,0002 (14,86); -0,0085 (0,45) |
| Verbindung Nr. I-1-80, Lösungsmittel: [DMSO], Spektrometer: 399.95MHz |
| 10,9408 (2,6); 8,999 (2,69); 8,994 (2,63); 8,2205 (1,63); 8,2148 (1,58); 8,2002 (1,7); 8,1944 (1,67); 7,363 (4,27); 7,3455 (5,41); 7,3424 (5,05); 7,3269 (3,5); 7,2909 (1,74); 7,2727 (2,15); 7,2665 (0,5); 7,2543 (0,63); 7,1494 (3,92); 7,1318 (3,28); 6,521 (4,31); 5,2319 (8,25); 3,9021 (2,14); 3,3271 (78,93); 2,6799 (0,36); 2,6755 (0,56); 2,671 (0,73); 2,6665 (0,52); 2,5411 (0,81); 2,5236 (20,2); 2,5108 (49,41); 2,5064 (95); 2,5019 (121,11); 2,4974 (86,34); 2,4929 (41,45); 2,3332 (0,53); 2,3288 (0,7); 2,324 (0,51); 2,2597 (16); 1,2982 (1,04); 1,2587 (1,44); 0,008 (0,4); -0,0002 (11,26); -0,0085 (0,35) |
| Verbindung Nr. I-1-99, Lösungsmittel: [DMSO], Spektrometer: 399.95MHz |
| 11,1514 (2,76); 9,0736 (2,9); 9,0694 (2,81); 8,8678 (3,14); 8,8624 (3,09); 8,5647 (2,08); 8,5597 (3,28); 8,5547 (1,78); 8,3153 (0,4); 7,3652 (1,71); 7,3615 (0,77); 7,3477 (4,6); 7,3288 (3,62); 7,2939 (1,8); 7,2758 (2,24); 7,2698 |
| (0,54); 7,2575 (0,71); 7,1542 (3,73); 7,1365 (3,22); 6,5259 (4,56); 5,2386 (8,28); 3,9022 (2,55); 3,3256 (104,59); 3,1752 (0,75); 3,1622 (0,75); 2,6802 (0,34); 2,6758 (0,67); 2,6711 (0,87); 2,6667 (0,65); 2,5414 (0,64); 2,5242 (2,8); 2,5109 (61,84); 2,5066 (119,12); 2,5021 (151,56); 2,4976 (107,87); 2,4932 (51,62); 2,3334 (0,65); 2,3288 (0,88); 2,3243 (0,65); 2,2662 (16); 1,2589 (0,49); 1,2346 (1,14); 0,0079 (0,43); -0,0002 (13,52); -0,0085 (0,41) |
| Verbindung Nr. I-1-98, Lösungsmittel: [DMSO], Spektrometer: 399.95MHz |
| 11,1275 (2,75); 8,9468 (2,8); 8,941 (2,82); 8,366 (1,62); 8,3598 (1,61); 8,345 (1,76); 8,3388 (1,72); 8,3152 (0,37); 7,6559 (3,14); 7,635 (2,99); 7,3638 (1,64); 7,3463 (4,52); 7,3274 (3,56); 7,2921 (1,78); 7,2791 (0,78); 7,2741 (2,21); 7,2557 (0,72); 7,1505 (3,77); 7,1328 (3,27); 6,5261 (4,44); 5,2368 (8,21); 4,02 (0,88); 3,902 (3,81); 3,5035 (0,43); 3,49 (0,53); 3,3265 (79,58); 2,6757 (0,5); 2,6713 (0,68); 2,6671 (0,48); 2,5415 (0,49); 2,5109 (49,41); 2,5067 (95,16); 2,5023 (121,54); 2,4978 (87,97); 2,4936 (43,42); 2,3333 (0,57); 2,3289 (0,75); 2,3245 (0,56); 2,2653 (16); 1,2587 (0,36); 0,0079 (0,37); -0,0002 (11,15); -0,0083 (0,33) |
| Verbindung Nr. I-1-46, Lösungsmittel: [DMSO], Spektrometer: 399.95MHz |
| 11,0396 (2,49); 9,1182 (1,29); 8,7288 (1,01); 8,3221 (1,31); 8,3155 (0,98); 8,3021 (1,36); 7,5267 (1,01); 7,5149 (1,07); 7,5072 (1,04); 7,4952 (0,91); 7,3656 (1,72); 7,3481 (4,56); 7,3292 (3,54); 7,293 (1,78); 7,2803 (0,78); 7,2748 (2,2); 7,2564 (0,7); 7,1534 (3,78); 7,1357 (3,23); 6,5348 (4,08); 5,2379 (7,99); 4,0197 (0,51); 3,902 (1,08); 3,4895 (0,34); 3,327 (117,37); 2,6803 (0,36); 2,6758 (0,62); 2,6713 (0,81); 2,6667 (0,6); 2,5415 (0,84); 2,5111 (56,65); 2,5068 (107,56); 2,5023 (136,51); 2,4977 (98,29); 2,4934 (47,9); 2,3334 (0,6); 2,329 (0,77); 2,3245 (0,57); 2,2653 (16); 1,2346 (1,2); -0,0002 (5,56) |
| Verbindung Nr. I-1-140 (Synthesebeispiel 9), Lösungsmittel: [DMSO], Spektrometer: 399.95MHz |
| 11,267 (4,96); 9,1112 (4,57); 9,1071 (4,48); 8,8097 (13,79); 8,7974 (13,97); 8,7378 (3,51); 8,7338 (3,65); 8,7258 (3,74); 8,7217 (3,58); 8,3298 (1,91); 8,3251 (2,64); 8,3199 (1,83); 8,3155 (0,52); 8,3098 (2,08); 8,3047 (2,78); 8,2999 (1,86); 7,5381 (2,49); 7,5365 (2,43); 7,5261 (2,45); 7,5244 (2,44); 7,5182 (2,44); 7,5166 (2,33); 7,5062 (2,35); 7,5045 (2,26); 7,478 (3,09); 7,4657 (5,94); 7,4535 (2,94); 6,8529 (10,98); 5,5438 (16); 3,322 (29,51); 3,1756 (0,32); 3,1625 (0,33); 2,671 (0,38); 2,5244 (0,9); 2,5109 (23,53); 2,5065 (46,44); 2,5019 (60,99); 2,4974 (44,43); 2,4929 (21,07); 2,3287 (0,41); 0,0079 (1,45); -0,0002 (38,86); -0,0085 (1,3) |
| Verbindung Nr. I-1-134 (Synthesebeispiel 2), Lösungsmittel: [DMSO], Spektrometer: 600.13MHz |
| 11,4298 (2,04); 9,0149 (2,36); 8,7884 (2,36); 8,7838 (2,4); 8,2606 (0,71); 8,2575 (0,9); 8,2562 (0,89); 8,2532 (0,71); 8,2446 (0,74); 8,2416 (0,93); 8,2403 (0,87); 8,2372 (0,69); 7,1952 (4,33); 4,067 (16); 3,8634 (15,57); 3,3277 (6,53); 2,5124 (2,52); 2,5095 (5,35); 2,5065 (7,33); 2,5036 (5,31); 2,5007 (2,49) |
| Verbindung Nr. I-1-138, Lösungsmittel: [DMSO], Spektrometer: 399.95MHz |
| 11,3355 (1,63); 9,129 (1,76); 9,125 (1,76); 8,7542 (1,4); 8,7501 (1,49); 8,7422 (1,49); 8,7381 (1,46); 8,3454 (0,77); 8,3408 (1,04); 8,3355 (0,76); 8,3255 (0,83); 8,3201 (1,08); 8,3156 (0,82); 7,5578 (0,98); 7,556 (0,98); 7,5457 (0,96); 7,5439 (0,97); 7,5379 (0,96); 7,5361 (0,95); 7,5259 (0,92); 7,524 (0,91); 7,2032 (4,63); 4,1001 (0,45); 4,0869 (0,49); 4,0639 (16); 3,8616 (15,2); 3,3238 (15,96); 3,1756 (2,79); 3,1625 (2,7); 2,5249 (0,58); 2,52 (0,91); 2,5115 (11,33); 2,507 (22,88); 2,5024 (30,11); 2,4978 (21,37); 2,4933 (10,01); 0,008 (0,98); -0,0002 (27,98); -0,0086 (0,94) |
| Verbindung Nr. I-1-135, Lösungsmittel: [DMSO], Spektrometer: 399.95MHz |
| 11,2558 (2,83); 9,0026 (2,12); 8,999 (3,62); 8,7683 (3,79); 8,7614 (3,84); 8,2628 (1,09); 8,2581 (1,35); 8,2562 (1,32); 8,2516 (1,07); 8,2386 (1,12); 8,2339 (1,37); 8,2274 (1,03); 6,602 (4,59); 5,0416 (1,13); 5,0188 (3,64); 4,9959 (3,81); 4,9729 (1,3); 3,3263 (17,64); 3,1767 (0,4); 3,1635 (0,4); 2,5255 (0,51); 2,5122 (9,6); 2,5078 (19,1); 2,5033 (24,92); 2,4987 (17,71); 2,4943 (8,46); 2,3253 (16); -0,0002 (4,52) |
| Verbindung Nr. I-1-133, Lösungsmittel: [DMSO], Spektrometer: 600.13MHz |
| 11,1513 (2,75); 9,1183 (2,84); 9,1154 (2,88); 8,7333 (2,19); 8,7306 (2,27); 8,7253 (2,3); 8,7227 (2,25); 8,3341 (1,09); 8,3311 (1,63); 8,3278 (1,1); 8,3209 (1,17); 8,3177 (1,69); 8,3145 (1,1); 7,5307 (1,62); 7,5297 (1,54); 7,5227 (1,62); 7,5217 (1,55); 7,5175 (1,64); 7,5165 (1,53); 7,5095 (1,57); 7,5084 (1,47); 6,6052 (4,19); 5,0229 (0,99); 5,0077 (3,17); 4,9925 (3,36); 4,9772 (1,17); 3,3306 (14,88); 2,5078 (8,17); 2,5049 (11,1); 2,5019 (8,09); 2,3249 (16) |
| Verbindung Nr. I-1-132, Lösungsmittel: [DMSO], Spektrometer: 600.13MHz |
| 11,6586 (1,39); 9,0707 (0,9); 9,0682 (1,5); 9,066 (0,86); 8,7889 (1,48); 8,7843 (1,49); 8,321 (0,46); 8,318 (0,56); 8,3165 (0,54); 8,3135 (0,44); 8,3049 (0,48); 8,3019 (0,58); 8,3004 (0,53); 8,2974 (0,43); 7,2082 (2,24); 6,8361 (1,7); 3,328 (5,08); 2,665 (6,28); 2,513 (2,02); 2,51 (4,26); 2,507 (5,74); 2,504 (4,15); 2,5011 (1,93); 2,4699 (16) |
| Verbindung Nr. I-1-12: Masse (m/z): 259,1 (M+H)⁺; 1H-NMR (DMSO-D₆) 0.87 - 0.89 (d, 6H), 2.10 - 2.16 (m, 1H), 2.26 (s, 3H), 3.75 - 3.76 (d, 2H), 6.42 (s, 1H), 7.46 - 7.49 (m, 1H), 8.28 - 8.31 (m, 1H), 8.68 - 8.70 (m, 1H), 9.10 (s, 1H), 10.74 (s, 1H). |
| Verbindung Nr. I-1-139, Lösungsmittel: [DMSO], Spektrometer: 399.95MHz |
| 11,5553 (1,44); 9,1799 (1,3); 9,1759 (1,29); 9,1744 (1,2); 8,753 (0,97); 8,749 (1,02); 8,741 (1,03); 8,737 (1); 8,4029 (0,55); 8,3983 (0,75); 8,393 (0,54); 8,3829 (0,6); 8,3776 (0,79); 8,373 (0,55); 7,5561 (0,68); 7,5544 (0,68); 7,544 (0,67); 7,5424 (0,68); 7,5362 (0,67); 7,5345 (0,66); 7,5242 (0,64); 7,5224 (0,64); 7,2068 (2,35); 6,8408 (1,87); 6,8394 (1,85); 3,3232 (1,88); 2,6635 (6,38); 2,6624 (6,3); 2,5125 (4,34); 2,5081 (8,58); 2,5035 (11,36); 2,499 (8,39); 2,4945 (4,03); 2,4697 (16); -0,0002 (7,34) |
| Verbindung Nr. I-1-137, Lösungsmittel: [DMSO], Spektrometer: 399.95MHz |
| 10,5164 (3,25); 9,1054 (2,76); 8,8206 (12,35); 8,8083 (12,48); 8,7684 (2,72); 8,7651 (2,84); 8,7565 (2,84); 8,7532 (2,68); 8,303 (1,48); 8,285 (1,52); 8,1711 (10,82); 7,567 (2,02); 7,5549 (2,08); 7,5471 (2,05); 7,535 (1,87); 7,4875 (3,02); 7,4752 (5,76); 7,4629 (2,9); 5,5272 (16); 4,038 (0,81); 4,0203 (0,8); 3,3238 (34,5); 2,6762 (0,4); 2,671 (0,52); 2,5409 (0,44); 2,5064 (67,46); 2,502 (86,29); 2,4977 (62,17); 2,3335 (0,41); 2,3288 (0,55); 2,3245 (0,39); 1,989 (3,48); 1,2362 (0,57); 1,1927 (0,9); 1,1749 (1,79); 1,1571 (0,89); 0,0082 (0,32); -0,0002 (8,46) |
| Verbindung Nr. I-1-136 (Synthesebeispiel 11), Lösungsmittel: [DMSO], Spektrometer: 399.95MHz |
| 10,3936 (2,65); 9,1082 (2,55); 8,8035 (11,47); 8,7912 (11,64); 8,7553 (0,37); 8,7425 (2,15); 8,7386 (2,28); 8,7303 (2,06); 8,7269 (1,99); 8,3158 (0,39); 8,3039 (1,4); 8,2846 (1,43); 7,6604 (5,74); 7,5436 (1,41); 7,5313 (1,52); 7,5239 (1,48); 7,5116 (1,33); 7,4638 (2,83); 7,4515 (5,35); 7,4392 (2,66); 5,4369 (12,53); 5,147 (0,35); 3,3264 (39,31); 3,1758 (1,05); 3,1627 (1,04); 2,5243 (0,91); 2,511 (18,16); 2,5066 (36,92); 2,502 (48,86); 2,4975 (34,95); 2,493 (16,73); 1,9163 (16); 1,8877 (0,56); 1,8169 (0,52); -0,0002 (8,8 |
| Verbindung Nr. I-1-125, Lösungsmittel: [DMSO], Spektrometer: 601.6MHz |
| 10,4392 (0,79); 9,1036 (0,65); 8,8124 (5,6); 8,8043 (5,77); 8,7631 (0,8); 8,7611 (0,84); 8,7555 (0,79); 8,7534 (0,75); 8,2959 (0,37); 8,2843 (0,39); 7,5591 (0,53); 7,551 (0,58); 7,5461 (0,58); 7,538 (0,5); 7,4756 (1,2); 7,4674 (2,32); 7,4592 (1,16); 5,7562 (16); 5,5703 (5,19); 4,289 (0,47); 3,4391 (0,32); 3,3247 (11,38); 2,5088 (3,98); 2,5058 (8,7); 2,5027 (12,01); 2,4996 (8,77); 2,4966 (4,01); 2,309 (8,98); 2,161 (0,46); 1,9897 (1,34); 1,8273 (2,25); 1,187 (0,37); 1,1751 (0,72); 1,1633 (0,37); -0,0002 (2,26) |
| Verbindung Nr. I-1-20, Lösungsmittel: [DMSO], Spektrometer: 399.95MHz |
| 10,9775 (2,88); 9,0736 (2,7); 9,0686 (2,8); 8,7994 (1,99); 8,7953 (2,12); 8,7871 (2,09); 8,783 (2,06); 8,7027 (1,96); 8,6989 (2,08); 8,6908 (2,06); 8,6869 (2); 8,3752 (2,01); 8,3711 (2,01); 8,3554 (2,15); 8,3513 (2,07); 8,294 (1,02); 8,2898 (1,54); 8,2742 (1,11); 8,2698 (1,61); 7,5839 (1,94); 7,5716 (1,91); 7,5642 (1,86); 7,5519 (1,78); 7,5007 (1,56); 7,4886 (1,54); 7,4808 (1,53); 7,4687 (1,44); 6,5362 (4,37); 5,5075 (9,9); 3,3237 (14,04); 2,5063 (37,65); 2,5019 (48); 2,4975 (34,73); 2,4027 (16); 1,9889 (0,36); -0,0002 (7,41) |
| Verbindung Nr. I-1-37, Lösungsmittel: [DMSO], Spektrometer: 399.95MHz |
| 10,9663 (2,74); 9,0819 (2,71); 9,0767 (2,66); 8,7049 (1,92); 8,7011 (2,1); 8,693 (2,02); 8,6891 (2,03); 8,4789 (2,05); 8,4757 (2,22); 8,4673 (2,13); 8,464 (2,19); 8,3012 (0,99); 8,2966 (1,53); 8,2919 (1,03); 8,2813 (1,08); 8,2765 (1,62); 8,272 (1,03); 7,9762 (2,03); 7,9729 (2,12); 7,9559 (2,22); 7,9526 (2,22); 7,5036 (1,53); 7,4916 (1,54); 7,4837 (1,49); 7,4716 (1,42); 7,4106 (1,92); 7,3989 (1,91); 7,3903 (1,81); 7,3787 (1,75); 6,519 (4,28); 5,4463 (10,01); 3,3236 (4,61); 2,5062 (25,75); 2,5019 (33,44); 2,4975 (24,42); 2,3391 (16); -0,0002 (6,15) |
| Verbindung Nr. I-1-123, Lösungsmittel: [CD3CN], Spektrometer: 601.6MHz |
| 9,0535 (0,94); 9,0296 (0,49); 9,0282 (0,56); 9,024 (1,44); 9,0198 (1,35); 8,7487 (0,48); 8,7451 (1,07); 8,7422 (1,07); 8,7371 (1,15); 8,7343 (1,11); 8,7196 (0,94); 8,7128 (6,78); 8,707 (6,36); 8,7047 (6,51); 8,6988 (5,84); 8,6383 (1,12); 8,6302 (1,13); 8,5925 (0,73); 8,5844 (0,83); 8,5741 (0,39); 8,1994 (0,5); 8,1884 (0,73); 8,1854 (0,8); 8,1819 (1,06); 8,1787 (0,99); 8,1753 (0,9); 8,1722 (0,52); 8,1717 (0,51); 8,1687 (0,86); 8,1655 (0,87); 8,1622 (0,6); 7,4821 (0,71); 7,4741 (0,81); 7,4699 (0,91); 7,4688 (0,93); 7,4609 (1,29); 7,457 (0,58); 7,4553 (0,63); 7,4533 (0,68); 7,4473 (0,9); 7,4458 (0,73); 7,4392 (0,7); 7,4378 (0,58); 7,434 (0,37); 7,4326 (0,37); 7,3182 (1,52); 7,3155 (1,5); 7,3101 (3,13); 7,3073 (2,74); 7,3019 (1,56); 7,2991 (1,45); 7,1971 (0,48); 5,4461 (7,81); 5,4001 (7,55); 5,348 (6,65); 4,8207 (1,66); 4,7621 (0,89); 4,5171 (0,34); 4,5081 (0,63); 4,4988 (0,37); 4,4202 (0,64); 4,4114 (1,02); 4,4022 (0,73); 3,8819 (0,52); 3,8116 (0,5); 3,8026 (0,84); 3,7937 (0,48); 2,1966 (12,66); 2,1741 (2,37); 2,1273 (10,18); 2,0495 (0,49); 2,0048 (4,32); 1,9833 (0,44); 1,9753 (0,59); 1,9711 (0,39); 1,9632 (3,05); 1,9573 (0,85); 1,9551 (4,38); 1,9511 (4,63); 1,9471 (22,58); 1,9431 (39,14); 1,939 (55,5); 1,9348 (35,72); 1,9307 (18,86); 1,9261 (0,51); 1,8998 (16); 1,868 (0,48); 1,8242 (0,35); 1,8098 (0,56); 0,0053 (1,44); -0,0002 (51,4); -0,0057 (1,35) |
| Verbindung Nr. I-1-36, Lösungsmittel: [DMSO], Spektrometer: 399.95MHz |
| 10,9929 (1,81); 9,0976 (1,9); 8,7122 (1,52); 8,3102 (1,26); 8,2921 (0,97); 7,5152 (1,24); 7,5036 (1,22); 7,4968 (1,04); 7,484 (0,68); 7,1712 (2,24); 6,5152 (2,02); 5,2989 (4,26); 3,3548 (0,67); 3,3249 (3,21); 2,7881 (1,14); 2,7481 (1,07); 2,7436 (1,07); 2,6584 (1,76); 2,5045 (19,91); 2,3751 (16); 2,3392 (6,89); 0,0066 (3,35); -0,0002 (4,77) |
| Verbindung Nr. I-1-53, Lösungsmittel: [DMSO], Spektrometer: 399.95MHz |
| 10,9859 (1,21); 9,0997 (1,07); 9,0947 (1,15); 8,7157 (0,7); 8,712 (0,84); 8,7038 (0,87); 8,7 (0,93); 8,3168 (0,39); 8,3121 (0,62); 8,3077 (0,47); 8,297 (0,46); 8,292 (0,67); 8,2877 (0,49); 7,5174 (0,6); 7,5054 (0,66); 7,4975 (0,64); 7,4855 (0,62); 6,5342 (1,69); 6,1575 (2,69); 6,1455 (0,34); 5,2292 (3,72); 3,8168 (16); 3,8048 (1,99); 3,3247 (4,68); 3,3127 (0,72); 2,5066 (9,78); 2,5023 (13,64); 2,4981 (11,21); 2,3738 (6,2); 2,363 (0,91); -0,0002 (7,35); - 0,0084 (0,68); -0,0123 (0,91) |
| Verbindung Nr. I-1-124, Lösungsmittel: [DMSO], Spektrometer: 399.95MHz |
| 10,986 (2,92); 9,108 (2,79); 9,1037 (2,77); 8,7265 (2,14); 8,7225 (2,32); 8,7145 (2,28); 8,7105 (2,28); 8,3246 (1,07); 8,3197 (1,59); 8,315 (1,16); 8,3047 (1,18); 8,2996 (1,68); 8,2949 (1,11); 7,9743 (1,11); 7,9629 (1,15); 7,5314 (1,53); 7,5298 (1,56); 7,5193 (1,54); 7,5178 (1,59); 7,5115 (1,52); 7,5099 (1,53); 7,4995 (1,49); 7,4978 (1,52); 6,4799 (4,32); 4,6267 (9,57); 3,3247 (17,09); 2,6224 (9,5); 2,6109 (9,47); 2,5243 (0,75); 2,5108 (14,68); |
| 2,5065 (29,66); 2,502 (39,13); 2,4974 (28,3); 2,493 (13,72); 2,2374 (16); 1,9888 (1,07); 1,1748 (0,56); -0,0002 (1,32) |
| Verbindung Nr. I-1-31, Lösungsmittel: [DMSO], Spektrometer: 399.95MHz |
| 10,9873 (1,26); 9,0963 (2,09); 9,0914 (2,14); 8,7135 (1,57); 8,7096 (1,72); 8,7016 (1,69); 8,6975 (1,69); 8,6073 (3,09); 8,5945 (3,16); 8,3136 (0,79); 8,309 (1,22); 8,304 (0,83); 8,2937 (0,87); 8,2889 (1,3); 8,2841 (0,85); 8,2547 (1,12); 7,5156 (1,18); 7,5035 (1,18); 7,4958 (1,17); 7,4837 (1,1); 7,3123 (2,36); 7,2995 (2,3); 6,5204 (3,37); 5,3607 (7,7); 2,9497 (0,83); 2,5246 (0,42); 2,5113 (8,85); 2,507 (17,66); 2,5025 (23,76); 2,4981 (17,08); 2,4938 (8,47); 2,4454 (16); 2,3249 (12,62); -0,0002 (0,67) |
| Verbindung Nr. I-1-57, Lösungsmittel: [DMSO], Spektrometer: 399.95MHz |
| 10,8674 (2,49); 9,0649 (1,92); 8,8583 (2,09); 8,8517 (2,11); 8,3162 (0,35); 8,2619 (0,96); 8,2394 (0,94); 3,969 (14,69); 3,3979 (0,46); 3,3248 (923,39); 2,9946 (0,82); 2,6747 (3,1); 2,6704 (4,08); 2,666 (3,03); 2,5845 (16); 2,5408 (23,83); 2,5058 (485,46); 2,5013 (615,9); 2,4969 (451,2); 2,3972 (12,1); 2,3326 (2,92); 2,3281 (3,88); 2,3237 (2,83); 2,2875 (0,37); 2,2392 (14,31); 2,0744 (0,49); 1,2583 (0,41); 1,2353 (2,82); -0,0002 (64,79); -0,0083 (2,95) |
| Verbindung Nr. I-1-119, Lösungsmittel: [DMSO], Spektrometer: 399.95MHz |
| 11,0434 (1,56); 9,2061 (1,44); 9,2021 (1,43); 8,7947 (1,23); 8,7907 (1,31); 8,7827 (1,3); 8,7786 (1,26); 8,4077 (0,59); 8,4031 (0,87); 8,3984 (0,59); 8,3877 (0,64); 8,3831 (0,91); 7,7767 (1,59); 7,7562 (1,7); 7,6322 (1,68); 7,6108 (2,12); 7,6043 (1,03); 7,5922 (0,94); 7,5908 (0,94); 7,5843 (0,93); 7,5723 (0,89); 7,4413 (0,92); 7,4388 (0,94); 7,4243 (1,1); 7,4214 (1,28); 7,4175 (0,82); 7,4029 (0,86); 7,4004 (0,84); 7,1357 (1,04); 7,1341 (1,06); 7,1161 (1,39); 7,0981 (0,9); 7,0965 (0,86); 4,0194 (16); 3,3245 (87,06); 2,6751 (0,43); 2,6704 (0,59); 2,666 (0,43); 2,5408 (15,15); 2,5238 (1,58); 2,5104 (34,23); 2,506 (69,29); 2,5015 (91,48); 2,4969 (65,92); 2,4924 (31,4); 2,3328 (0,43); 2,3283 (0,58); 2,3236 (0,41); 1,235 (0,41); 0,008 (0,55); -0,0002 (17,1); -0,0085 (0,52) |
| Verbindung Nr. I-1-18 (Synthesebeispiel 3), Lösungsmittel: [DMSO], Spektrometer: 399.95MHz |
| 10,8077 (0,94); 9,0862 (0,92); 9,0819 (0,9); 9,0805 (0,89); 8,8312 (0,66); 8,8273 (0,71); 8,8192 (0,71); 8,8152 (0,7); 8,279 (0,37); 8,2737 (0,51); 8,2691 (0,38); 8,2591 (0,41); 8,2536 (0,56); 8,2491 (0,4); 7,6518 (0,47); 7,6502 (0,49); 7,6397 (0,47); 7,6382 (0,49); 7,6319 (0,46); 7,6303 (0,47); 7,6198 (0,44); 7,6182 (0,45); 6,6089 (2,25); 3,3238 (4,68); 2,5114 (4); 2,507 (7,91); 2,5024 (10,35); 2,4979 (7,53); 2,4934 (3,68); 2,214 (6,07); 1,5586 (16); - 0,0002 (2,41) |
| Verbindung Nr. I-1-27: Masse (m/z): 314,1 (M+H)⁺; 1H-NMR (DMSO-D₆) 5.32 (s, 2H), 6.66 - 6.67 (d, 1H), 7.46 - 7.50 (m, 2H), 7.69 - 7.72 (m, 1H), 7.81 - 7.82 (d, 1H), 8.26 - 8.30 (m, 1H), 8.35 (s, 1H), 8.69 - 8.70 (m, 1H), 9.09 (s, 1H), 10.88 (s, 1H). |
| Verbindung Nr. I-1-17 (Synthesebeispiel 4), Lösungsmittel: [DMSO], Spektrometer: 399.95MHz |
| 11,5244 (2,09); 9,3343 (2,9); 9,3301 (2,79); 8,9696 (2,07); 8,9662 (2,16); 8,9562 (2,16); 8,9528 (2,07); 8,8224 (1,44); 8,8021 (1,46); 7,9797 (1,38); 7,9661 (1,42); 7,9596 (1,36); 7,9461 (1,24); 6,4467 (4,23); 2,5271 (0,9); 2,5138 (14,54); 2,5095 (28,07); 2,5049 (36,1); 2,5004 (25,87); 2,4959 (12,29); 2,2701 (16); -0,0002 (2,81) |
| Verbindung Nr. I-2-1, Lösungsmittel: [DMSO], Spektrometer: 399.95MHz |
| 13,2623 (0,61); 13,2552 (0,59); 9,1661 (1,61); 9,1319 (4,59); 8,776 (6,31); 8,7671 (6,28); 8,7644 (6,06); 8,5652 (0,57); 8,5502 (0,6); 8,342 (2,69); 8,3292 (2,73); 7,5779 (4,86); 7,5658 (5,17); 7,5582 (5,05); 7,5461 (4,54); 7,2679 (0,8); 7,253 (0,79); 3,3295 (4,56); 3,1701 (0,55); 2,7642 (5,84); 2,6769 (0,54); 2,6723 (0,74); 2,6678 (0,55); 2,5424 (0,54); 2,5255 (2,65); 2,5121 (42,88); 2,5078 (84,73); 2,5033 (110,99); 2,4987 (80,56); 2,4943 (39,26); 2,35 (0,72); 2,3341 (1,33); 2,3299 (1,64); 2,3253 (1,61); 2,3207 (1,52); 2,2744 (8,52); 2,0893 (16); 1,9895 (0,51); 0,0077 (1,85); -0,0002 (49,21); -0,0084 (1,81) |
| Verbindung Nr. I-1-35 (Synthesebeispiel 10): 1H-NMR (DMSO-D₆) 1.17 (t, 3H), 2.62 (q, 2H), 5.23 (s, 2H), 6.54 (s, 1H), 7.13 - 7.35 (m, 5H), 7.46 - 7.50 (m, 1H), 8.29 - 8.32 (m, 1H), 8.69 - 8.70 (m, 1H), 9.11 (s, 1H), 10.81 (s, 1H). |
| Verbindung Nr. I-1-52, Lösungsmittel: [DMSO], Spektrometer: 399.95MHz |
| 11,4819 (10,59); 9,179 (9,97); 9,1774 (10,3); 9,1734 (10,58); 9,1717 (9,58); 8,7727 (8,4); 8,7686 (8,98); 8,7607 (8,95); 8,7566 (8,75); 8,6099 (16); 8,6033 (15,93); 8,4887 (5,51); 8,4868 (6,16); 8,4842 (6,62); 8,4822 (5,71); 8,4766 (5,86); 8,4746 (6,49); 8,4721 (6,39); 8,4701 (5,52); 8,3946 (4,86); 8,3902 (6,53); 8,3846 (4,78); 8,3747 (5,24); 8,3702 (6,63); 8,3691 (6,5); 8,3647 (4,92); 8,3177 (0,32); 8,2591 (0,43); 8,2525 (0,4); 8,0305 (4,45); 8,0259 (4,41); 8,012 (5,85); 8,0098 (6,62); 8,0074 (6,02); 8,0053 (6,24); 7,9914 (5,9); 7,9868 (5,72); 7,8477 (0,38); 7,8368 (11,7); 7,8161 (9,56); 7,8141 (5,36); 7,5979 (0,34); 7,5785 (6,37); 7,5765 (6,2); 7,5664 (6,08); 7,5645 (5,92); 7,5586 (6,04); 7,5566 (5,78); 7,5465 (5,87); 7,5445 (5,61); 7,362 (5,87); 7,3596 (5,92); 7,3498 (5,61); 7,3474 (6,05); 7,3436 (6,08); 7,3412 (5,4); 7,3314 (5,67); 7,329 (5,34); 7,0181 (14,8); 7,0114 (14,62); 5,8 (0,48); 5,7934 (0,46); 5,2497 (0,43); 3,3283 (75,47); 2,6778 (0,52); 2,6733 (0,72); 2,6687 (0,51); 2,5266 (2,17); 2,5218 (3,38); 2,5133 (39,29); 2,5088 (78,93); 2,5042 (104,18); 2,4996 (75,12); 2,4951 (35,55); 2,3356 (0,49); 2,3309 (0,69); 2,3264 (0,48); 1,3368 (0,37); 1,25 (0,5); 0,0079 (2,47); -0,0002 (70,38); -0,0086 (2,23) |
| Verbindung Nr. I-1-30, Lösungsmittel: [DMSO], Spektrometer: 399.95MHz |
| 11,5021 (11,76); 9,1701 (11,28); 9,166 (11,23); 8,7641 (8,87); 8,76 (9,52); 8,752 (9,43); 8,7479 (9,28); 8,4344 (11,84); 8,4333 (12,02); 8,4277 (11,98); 8,4267 (11,77); 8,3935 (5,74); 8,3893 (12,03); 8,3869 (13,88); 8,3837 |
| (10,85); 8,378 (6,61); 8,3745 (13,07); 8,3687 (8,01); 8,3637 (5,35); 8,2362 (0,36); 8,233 (0,63); 8,2296 (0,36); 8,2246 (0,37); 8,2214 (0,62); 8,2183 (0,35); 8,1404 (0,85); 8,1338 (0,81); 8,04 (5,02); 8,0366 (5,07); 8,0194 (5,65); 8,016 (5,53); 8,0112 (5,26); 8,0077 (5,22); 7,9905 (5,5); 7,9871 (5,37); 7,8674 (0,34); 7,8638 (0,33); 7,8469 (0,35); 7,8434 (0,36); 7,8371 (0,35); 7,8337 (0,32); 7,8167 (0,34); 7,8132 (0,34); 7,5678 (6,46); 7,566 (6,51); 7,5557 (6,38); 7,554 (6,42); 7,5478 (6,29); 7,5461 (6,27); 7,5358 (6,19); 7,534 (6,32); 7,5294 (5,73); 7,5208 (6,09); 7,5178 (5,7); 7,509 (9,52); 7,5002 (5,04); 7,4972 (5,13); 7,4887 (4,55); 7,3063 (0,44); 7,2983 (0,44); 7,2948 (0,45); 7,2863 (0,74); 7,2779 (0,46); 7,2745 (0,4); 7,2662 (0,43); 7,0478 (16); 7,041 (15,84); 5,833 (0,96); 5,8263 (0,96); 5,2229 (1,09); 3,3267 (75,69); 3,1771 (0,55); 3,164 (0,55); 2,6772 (0,62); 2,6727 (0,87); 2,6682 (0,64); 2,6638 (0,32); 2,526 (2,75); 2,5211 (4,31); 2,5127 (47,37); 2,5082 (94,59); 2,5036 (124,74); 2,4991 (90,44); 2,4945 (43,34); 2,3349 (0,58); 2,3304 (0,81); 2,3259 (0,57); 1,259 (0,37); 1,25 (0,41); 1,2322 (0,32); 0,1459 (0,37); 0,0079 (2,96); -0,0002 (80,32); -0,0084 (2,67); -0,1497 (0,35) |
| Verbindung Nr. I-1-117, Lösungsmittel: [DMSO], Spektrometer: 399.95MHz |
| 11,475 (11,08); 9,1604 (9,76); 9,1572 (9,64); 8,7644 (6,68); 8,7553 (6,64); 8,3881 (12,97); 8,3819 (16); 8,3575 (6,41); 8,036 (7,02); 8,0168 (7,52); 7,8979 (2,86); 7,8953 (2,87); 7,8773 (7,15); 7,8591 (5,76); 7,8565 (5,62); 7,8247 (10,78); 7,8052 (5,6); 7,6024 (4,83); 7,5839 (8,48); 7,5725 (6,57); 7,5621 (7,36); 7,553 (5,76); 7,5408 (4,91); 7,4142 (0,32); 7,3931 (0,41); 7,3654 (0,44); 7,0541 (11,38); 7,0479 (11,15); 5,7567 (1,47); 3,7424 (0,36); 3,3278 (68,26); 2,6716 (1,06); 2,5027 (148,84); 2,3294 (1,05); 1,9911 (0,59); 1,4917 (0,4); 1,3367 (0,6); 1,2989 (4,37); 1,259 (5,39); 1,2335 (0,82); -0,0002 (51,23) |
| Verbindung Nr. I-1-39, Lösungsmittel: [DMSO], Spektrometer: 399.95MHz |
| 11,3674 (1,9); 9,2176 (1,89); 9,2132 (1,91); 8,8012 (1,46); 8,7972 (1,54); 8,7892 (1,54); 8,7851 (1,49); 8,5859 (1,66); 8,5819 (1,81); 8,5748 (1,77); 8,5708 (1,74); 8,4233 (0,83); 8,4186 (1,2); 8,4134 (0,91); 8,4076 (1,81); 8,4036 (2,45); 8,3987 (1,35); 8,3935 (0,93); 8,3872 (1,8); 8,3832 (1,64); 7,6086 (1,1); 7,607 (1,07); 7,5965 (1,1); 7,5949 (1,07); 7,5887 (1,09); 7,5871 (1,03); 7,5767 (1,03); 7,575 (0,98); 7,2298 (1,73); 7,2187 (1,72); 7,2093 (1,71); 7,1982 (1,67); 4,0402 (16); 3,75 (0,59); 3,3283 (7,67); 3,1777 (0,44); 3,1646 (0,43); 2,5261 (0,41); 2,5128 (6,6); 2,5085 (12,79); 2,5039 (16,63); 2,4994 (12,16); 2,495 (5,98); -0,0002 (0,93) |
| Verbindung Nr. I-1-63, Lösungsmittel: [DMSO], Spektrometer: 399.95MHz |
| 11,511 (9,56); 9,1672 (8,52); 9,1629 (8,54); 8,9527 (7,08); 8,9507 (8,31); 8,9478 (8,14); 8,7696 (6,84); 8,7655 (7,27); 8,7576 (7,34); 8,7535 (7,46); 8,7447 (8,67); 8,7403 (8,38); 8,4333 (15,96); 8,4264 (16); 8,3901 (3,79); 8,3854 (5,26); 8,3802 (3,7); 8,3702 (4,09); 8,3653 (5,49); 8,3603 (3,75); 8,3165 (0,35); 7,5733 (4,92); 7,5717 (4,78); 7,5613 (4,81); 7,5596 (4,72); 7,5534 (4,74); 7,5518 (4,57); 7,5414 (4,6); 7,5396 (4,43); 7,0696 (13,27); 7,0628 (13,12); 5,7569 (13,74); 4,1008 (0,98); 4,0876 (1); 4,0745 (0,34); 4,0207 (0,34); 3,3247 (94,14); 3,1761 (4,4); 3,163 (4,27); 2,6767 (0,66); 2,672 (0,9); 2,6675 (0,64); 2,5254 (2,78); 2,5205 (4,37); 2,512 (51,18); 2,5075 (102,26); 2,5029 (134,94); 2,4984 (97,18); 2,4939 (46,08); 2,3342 (0,64); 2,3298 (0,89); 2,3251 (0,65); 1,9894 (1,46); 1,1932 (0,39); 1,1753 (0,79); 1,1575 (0,39); 0,1461 (0,37); 0,008 (3,11); -0,0002 (86,72); -0,0085 (2,62); - 0,1494 (0,38) |
| Verbindung Nr. I-1-32, Lösungsmittel: [DMSO], Spektrometer: 399.95MHz |
| 10,9793 (1,72); 9,111 (1,5); 9,1062 (1,51); 8,7277 (1,14); 8,7245 (1,21); 8,7158 (1,21); 8,7125 (1,2); 8,55 (1,74); 8,3219 (0,89); 8,3172 (0,58); 8,3061 (0,67); 8,302 (0,93); 8,2973 (0,58); 7,533 (0,9); 7,5211 (0,94); 7,5131 (1,04); 7,5012 (0,89); 6,4696 (2,23); 5,7575 (0,45); 4,6696 (4,7); 4,5295 (0,37); 3,8833 (0,69); 3,7057 (1,43); 3,6082 (0,33); 3,5968 (2,19); 3,5641 (10,33); 3,5535 (1,5); 3,5465 (0,5); 3,3379 (1,67); 2,5075 (8,7); 2,5035 (10,77); 2,4992 (7,83); 2,2016 (8,09); 2,0567 (0,63); 1,9896 (0,69); 1,4754 (3,67); 1,3772 (16); 1,363 (3,13); 1,3502 (2,31); 1,1748 (0,42); 0,0004 (0,78); -0,0002 (0,79) |
| Verbindung Nr. I-1-115, Lösungsmittel: [DMSO], Spektrometer: 399.95MHz |
| 10,9991 (3,37); 9,1096 (3,07); 9,1053 (3,13); 8,7284 (2,1); 8,7249 (2,22); 8,7166 (2,21); 8,7131 (2,15); 8,3224 (1,7); 8,3023 (1,79); 7,5338 (1,6); 7,5219 (1,61); 7,5142 (1,61); 7,502 (1,52); 6,4914 (4,6); 4,8147 (10,11); 3,3329 (9,41); 2,5023 (38,1); 2,2254 (16); 2,0742 (0,34); 1,2351 (0,33); -0,0002 (1,84) |
| Verbindung Nr. I-1-24, Lösungsmittel: [DMSO], Spektrometer: 601.6MHz |
| 11,0424 (1,93); 9,1088 (1,64); 9,1059 (1,6); 8,7305 (1,4); 8,7279 (1,49); 8,7226 (1,45); 8,7199 (1,48); 8,323 (0,66); 8,3197 (0,95); 8,3165 (0,69); 8,3098 (0,71); 8,3063 (1); 8,3033 (0,69); 7,5336 (0,97); 7,5325 (1,08); 7,5257 (0,95); 7,5245 (1,01); 7,5205 (0,94); 7,5193 (0,99); 7,5125 (0,92); 7,5113 (0,97); 6,517 (2,49); 4,9386 (7,38); 4,6393 (0,77); 4,1777 (1,25); 4,1659 (3,99); 4,1541 (4,02); 4,1422 (1,29); 4,1179 (0,43); 4,1061 (0,43); 4,046 (1,2); 4,0342 (3,63); 4,0224 (3,66); 4,0105 (1,21); 3,3638 (26,71); 2,511 (3,92); 2,508 (8,39); 2,505 (11,54); 2,502 (8,39); 2,499 (3,84); 2,234 (10,13); 2,0586 (0,92); 2,0578 (0,96); 1,9957 (0,34); 1,9911 (16); 1,3963 (0,42); 1,225 (4,65); 1,2132 (9,8); 1,2014 (4,64); 1,1917 (0,95); 1,1865 (4,54); 1,1799 (0,54); 1,1746 (8,86); 1,1628 (4,5) |
| Verbindung Nr. I-1-41, Lösungsmittel: [DMSO], Spektrometer: 601.6MHz |
| 11,1038 (1,55); 8,9982 (0,86); 8,9956 (1,49); 8,9932 (0,84); 8,7657 (1,62); 8,7611 (1,61); 8,5651 (1,44); 8,243 (0,47); 8,2401 (0,54); 8,2385 (0,54); 8,2356 (0,45); 8,227 (0,49); 8,2241 (0,57); 8,2225 (0,53); 8,2195 (0,44); 6,4699 (1,85); 4,6717 (4,09); 3,6936 (0,46); 3,5603 (13,53); 3,3484 (9,25); 2,5424 (3,35); 2,509 (7,78); 2,506 (17,41); 2,503 (23,98); 2,4999 (17,12); 2,4969 (7,53); 2,2017 (6,79); 2,2012 (6,52); 2,077 (0,43); 1,3731 (16); - 0,0002 (6,15) |
| Verbindung Nr. I-1-7 (Synthesebeispiel 12), Lösungsmittel: [DMSO], Spektrometer: 399.95MHz |
| 11,0604 (2,71); 8,9829 (1,96); 8,9791 (3,18); 8,7886 (8,34); 8,7764 (8,39); 8,7417 (3,2); 8,7348 (3,23); 8,2252 (1,03); 8,2207 (1,24); 8,2185 (1,24); 8,2141 (1,01); 8,201 (1,05); 8,1965 (1,24); 8,1943 (1,18); 8,1898 (0,93); 7,4498 (1,94); 7,4375 (3,71); 7,4253 (1,83); 6,5223 (3,92); 5,4262 (10,31); 4,0391 (0,55); 4,0213 (0,54); 3,5678 (0,39); 3,4225 (0,4); 3,3117 (570,27); 2,6744 (0,43); 2,6697 (0,6); 2,6652 (0,44); 2,5397 (0,8); 2,5228 (2,6); 2,5095 (33,79); 2,5051 (63,13); 2,5007 (82,71); 2,4962 (57,45); 2,4919 (27,5); 2,3215 (16); 1,9868 (2,34); 1,2363 (0,64); 1,1928 (0,67); 1,1749 (1,3); 1,1572 (0,65); -0,0002 (0,58) |
| Verbindung Nr. I-1-29: Masse (m/z): 354,1 (M+H)⁺; 1H-NMR (DMSO-D₆) 0.60 - 0.64 (m, 2H), 0.94 - 0.98 (m, 2H), 1.90 - 2.00 (m, 1H), 5.40 (s, 2H), 6.33 (s, 1H), 7.46 - 7.50 (m, 2H), 7.62 - 7.65 (m, 1H), 8.26 - 8.32 (m, 2H), 8.68 - 8.70 (m, 1H), 9.08 (s, 1H), 10.80 (s, 1H). |
| Verbindung Nr. I-1-61: HPLC-MS: logP = 1,35; Masse (m/z): 378,1 (M+H)⁺; 1H-NMR (CD₃CN) 1.21- 1.28 (m, 3H), 2.18 (s, 3H), 2.89 und 3.09 (s, 3H), 4.05 (s, 2H), 4.12 - 4.23 (m, 2H), 4.78 und 4.93 (s, 2H), 6.53 (s, 1H), 7.98 - 8.00 (m, 1H), 8.61 -8.62 (m, 1H), 8.90 (s, 1H), 9.19 (s, 1H); 13C-NMR (CD₃CN) 14.4, 35.3, 36.1, 50.5, 51.1, 51.3, 61.8, 62.4, 98.6, 122.9, 123.0, 132.6, 141.4, 141.9, 145.7, 146.6, 159.2, 160.9, 162.8, 168.3, 168.4, 170.1. |
| Verbindung Nr. I-1-114, Lösungsmittel: [DMSO], Spektrometer: 399.95MHz |
| 11,079 (3,12); 8,9949 (3,4); 8,7564 (3,21); 8,7496 (3,28); 8,2412 (1,09); 8,235 (1,37); 8,2304 (1,09); 8,2171 (1,13); 8,2126 (1,38); 8,2062 (1,05); 6,487 (4,2); 4,8053 (10,5); 3,4508 (2,35); 3,4331 (2,9); 3,3269 (16,37); 3,0754 (0,46); 2,6749 (0,39); 2,6703 (0,46); 2,6657 (0,38); 2,54 (1,04); 2,5056 (41,86); 2,5013 (52,51); 2,497 (37,41); 2,3279 (0,37); 2,2264 (16); 2,0691 (0,57); 1,2372 (0,98); 1,1696 (0,38); 1,0745 (0,71); 1,0571 (1,35); 1,0396 (0,68); 0,8749 (0,46); -0,0002 (2,39) |
| Verbindung Nr. I-1-59, Lösungsmittel: [DMSO], Spektrometer: 399.95MHz |
| 11,0906 (2,78); 8,9963 (2,03); 8,9925 (3,29); 8,9362 (0,34); 8,7579 (3,28); 8,751 (3,32); 8,2406 (1,09); 8,2361 (1,3); 8,2339 (1,3); 8,2294 (1,07); 8,2164 (1,11); 8,212 (1,33); 8,2099 (1,25); 8,2053 (1,02); 6,5088 (3,89); 4,931 (11,68); 4,7731 (0,36); 4,4845 (0,49); 4,1869 (1,95); 4,1691 (6,04); 4,1514 (6,08); 4,1419 (0,5); 4,1336 (2,02); 3,376 (0,39); 3,3599 (0,69); 3,3019 (305,24); 2,6736 (0,56); 2,669 (0,74); 2,6645 (0,55); 2,539 (1,16); 2,5221 (3,62); 2,5088 (43,33); 2,5044 (80,04); 2,5 (104,25); 2,4956 (73,63); 2,4912 (36,16); 2,3313 (0,62); 2,3268 (0,78); 2,3221 (0,59); 2,3179 (0,34); 2,2358 (16); 2,1814 (0,38); 2,0695 (0,76); 2,0591 (0,71); 1,9913 (1,03); 1,4627 (0,34); 1,3561 (1,3); 1,3308 (0,42); 1,3118 (0,4); 1,2985 (0,34); 1,2591 (0,34); 1,2299 (6,95); 1,2121 (14,17); 1,1943 (6,7); 1,1811 (0,41); 1,1413 (0,35); 0,8932 (0,68); 0,8855 (0,7); 0,8787 (0,65); 0,8748 (1,28); 0,8691 (0,77); 0,8565 (0,62); 0,8507 (0,38); 0,845 (0,46); 0,8281 (0,41); 0,0079 (0,95); -0,0002 (17,7); -0,0084 (0,82) |
| Verbindung Nr. I-1-47, Lösungsmittel: [DMSO], Spektrometer: 601.6MHz |
| 10,5679 (1,36); 9,1121 (1,36); 8,7939 (1,41); 8,7913 (1,48); 8,7859 (1,48); 8,7833 (1,44); 8,3051 (0,76); 8,292 (0,79); 7,5925 (1,05); 7,5844 (1,05); 7,5793 (1,06); 7,5713 (0,99); 7,4676 (1,25); 7,3493 (2,86); 7,231 (1,42); 5,7622 (6,17); 3,7493 (16); 3,3451 (39,63); 3,3215 (0,52); 2,5214 (0,33); 2,5183 (0,32); 2,5094 (7,19); 2,5064 (15,55); 2,5034 (21,3); 2,5003 (15,57); 2,4974 (7,19); -0,0002 (2,4) |
| Verbindung Nr. I-1-113, Lösungsmittel: [DMSO], Spektrometer: 399.95MHz |
| 10,6385 (2,6); 8,9971 (2,07); 8,8308 (2,66); 8,8238 (2,62); 8,1996 (0,77); 8,1952 (0,96); 8,1891 (0,74); 8,1762 (0,8); 8,1717 (0,95); 7,5153 (1,6); 7,3372 (3,51); 7,1592 (1,74); 5,7471 (0,37); 3,7498 (16); 3,307 (150,9); 3,2851 (1,15); 2,5397 (0,41); 2,5228 (1,23); 2,5095 (13,33); 2,5052 (23,9); 2,5007 (30,52); 2,4963 (20,91); 2,4919 (9,89) |
| Verbindung Nr. I-1-112 (Synthesebeispiel 5), Lösungsmittel: [DMSO], Spektrometer: 399.95MHz |
| 11,2317 (1,77); 8,9941 (1,43); 8,9902 (2,41); 8,9863 (1,34); 8,7756 (2,19); 8,7687 (2,22); 8,2396 (0,82); 8,2352 (0,95); 8,2328 (0,94); 8,2283 (0,79); 8,2156 (0,84); 8,2111 (0,96); 8,2087 (0,9); 8,2043 (0,76); 7,5178 (1,32); 7,3367 (2,89); 7,1557 (1,42); 6,4514 (2,93); 6,2668 (0,34); 3,636 (16); 3,5669 (1,96); 3,3175 (165,02); 2,5243 (0,62); 2,511 (8,63); 2,5066 (16,04); 2,5021 (20,89); 2,4977 (14,52); 2,4933 (6,95); 2,0702 (0,72); -0,0002 (1,46) |
| Verbindung Nr. I-1-111, Lösungsmittel: [DMSO], Spektrometer: 399.95MHz |
| 11,1283 (2,55); 9,1083 (2,89); 9,1034 (2,46); 8,7443 (2,1); 8,7404 (2,08); 8,7324 (1,86); 8,7285 (1,56); 8,3235 (1,59); 8,3191 (1,86); 8,3143 (1,36); 8,3037 (1,45); 8,2992 (1,63); 8,2946 (0,98); 7,5475 (1,78); 7,5353 (1,9); 7,5276 (1,76); 7,5164 (2,41); 7,3354 (2,76); 7,1543 (1,37); 6,4533 (3,65); 3,6312 (16); 3,3104 (29,99); 2,5661 (1,17); 2,5054 (25,89); 2,5012 (27,09); -0,0002 (2,17) |
| Verbindung Nr. I-3-1 (Synthesebeispiel 15), Lösungsmittel: [DMSO], Spektrometer: 399.95MHz |
| 11,1114 (2,03); 9,1905 (1,95); 9,185 (1,88); 8,8172 (1,43); 8,8131 (1,59); 8,8051 (1,53); 8,8011 (1,55); 8,5571 (1,18); 8,5546 (1,25); 8,5526 (1,18); 8,545 (1,26); 8,5425 (1,22); 8,5405 (1,15); 8,3894 (0,76); 8,3841 (1,12); 8,3796 (0,79); 8,3695 (0,82); 8,3641 (1,19); 8,3596 (0,8); 8,0746 (0,68); 8,07 (0,69); 8,054 (1,22); 8,0518 (1,1); 8,0495 (1,19); 8,0355 (1,05); 8,0309 (1,04); 7,9562 (2,21); 7,9356 (1,54); 7,6206 (1,12); 7,6085 (1,12); 7,6007 (1,09); 7,5902 (0,93); 7,5886 (1,04); 7,4773 (0,96); 7,475 (1,09); 7,4652 (0,95); 7,4629 (1,12); 7,459 (1,03); 7,4566 (1,01); 7,4469 (0,92); 7,4445 (0,98); 3,3248 (20,52); 2,5113 (14,15); 2,507 (28,39); 2,5025 (37,64); 2,498 (27,43); 2,4935 (13,49); 2,3237 (16); -0,0002 (1,69) |
| Verbindung Nr. I-1-22, Lösungsmittel: [DMSO], Spektrometer: 399.95MHz |
| 8.968 (3.4); 8.962 (3.6); 8.806 (2.3); 8.803 (2.5); 8.794 (2.4); 8.790 (2.3); 8.177 (1.3); 8.173 (2.0); 8.168 (1.4); 8.158 (1.5); 8.153 (2.1); 8.148 (1.4); 7.907 (1.0); 7.903 (0.8); 7.862 (4.0); 7.858 (2.8); 7.841 (0.6); 7.822 (0.6); |
| 7.782 (2.2); 7.763 (2.6); 7.744 (1.7); 7.741 (1.7); 7.726 (2.1); 7.723 (2.1); 7.721 (2.2); 7.694 (0.4); 7.679 (1.9); 7.676 (1.9); 7.661 (1.9); 7.659 (2.0); 7.656 (2.2); 7.609 (2.3); 7.601 (3.1); 7.597 (2.6); 7.589 (2.5); 7.581 (4.2); 7.561 (1.7); 7.548 (0.4); 7.527 (0.6); 7.513 (0.8); 7.494 (0.9); 7.474 (0.4); 7.400 (2.5); 7.396 (3.9); 7.392 (2.6); 7.343 (2.1); 7.323 (3.9); 7.303 (2.0); 6.941 (2.4); 6.921 (2.2); 6.748 (5.2); 6.746 (4.8); 5.756 (1.6); 4.056 (0.8); 4.038 (2.4); 4.020 (2.4); 4.003 (0.8); 3.323 (64.9); 2.671 (1.0); 2.667 (0.8); 2.651 (0.8); 2.614 (16.0); 2.541 (1.1); 2.507 (110.7); 2.502 (141.1); 2.498 (105.0); 2.334 (0.7); 2.329 (0.9); 2.325 (0.7); 1.989 (10.1); 1.398 (0.9); 1.299 (0.6); 1.259 (0.9); 1.235 (2.4); 1.193 (2.9); 1.175 (5.7); 1.157 (2.8); 0.868 (0.3); 0.854 (0.4); 0.146 (0.7); 0.008 (7.7); 0.000 (145.2); -0.008 (6.0); -0.149 (0.7) |
| Verbindung Nr. I-1-120, Lösungsmittel: [DMSO], Spektrometer: 399.95MHz |
| 11.435 (1.5); 9.136 (1.7); 9.132 (1.7); 9.130 (1.6); 8.763 (1.3); 8.759 (1.4); 8.751 (1.3); 8.747 (1.3); 8.358 (0.8); 8.352 (1.0); 8.348 (0.8); 8.338 (0.9); 8.332 (1.1); 8.328 (0.8); 7.561 (0.9); 7.559 (1.0); 7.549 (0.9); 7.547 (1.0); 7.541 (0.9); 7.539 (0.9); 7.529 (0.9); 7.527 (0.9); 6.861 (2.4); 6.859 (2.3); 3.322 (11.6); 3.176 (0.6); 3.163 (0.6); 2.608 (16.0); 2.544 (8.9); 2.542 (8.9); 2.525 (0.5); 2.511 (10.1); 2.507 (20.0); 2.502 (25.9); 2.498 (18.3); 2.493 (8.5); 0.008 (1.2); 0.000 (28.9); -0.009 (0.9) |
| Verbindung Nr. I-1-42, Lösungsmittel: [DMSO], Spektrometer: 399.95MHz |
| 13.176 (0.6); 11.214 (1.5); 8.962 (3.1); 8.957 (3.1); 8.923 (1.3); 8.919 (1.2); 8.816 (2.1); 8.812 (2.3); 8.803 (2.3); 8.800 (2.2); 8.746 (0.9); 8.742 (1.0); 8.734 (1.0); 8.730 (0.9); 8.184 (1.4); 8.178 (2.0); 8.174 (1.5); 8.164 (1.6); 8.158 (2.2); 8.154 (1.5); 8.141 (0.6); 8.136 (0.9); 8.131 (0.6); 8.121 (0.7); 8.115 (0.9); 8.111 (0.6); 8.006 (0.4); 7.985 (0.4); 7.959 (0.5); 7.953 (3.4); 7.948 (1.5); 7.941 (1.6); 7.937 (2.0); 7.931 (4.1); 7.926 (0.7); 7.911 (1.4); 7.869 (1.2); 7.863 (8.8); 7.858 (3.0); 7.846 (3.5); 7.841 (11.2); 7.835 (1.5); 7.809 (0.4); 7.803 (2.7); 7.798 (1.0); 7.786 (1.1); 7.781 (3.4); 7.775 (0.5); 7.681 (1.2); 7.675 (8.2); 7.670 (2.5); 7.666 (0.9); 7.658 (2.5); 7.653 (7.1); 7.649 (4.3); 7.644 (1.7); 7.632 (1.2); 7.627 (4.6); 7.613 (2.0); 7.606 (2.2); 7.594 (1.8); 7.593 (1.9); 7.587 (0.7); 7.581 (3.9); 7.576 (1.7); 7.570 (3.5); 7.564 (2.0); 7.559 (3.6); 7.553 (1.4); 7.548 (3.0); 7.539 (1.1); 7.526 (1.0); 7.518 (1.0); 7.506 (0.8); 7.399 (0.9); 7.393 (6.7); 7.389 (2.3); 7.376 (2.4); 7.372 (8.2); 7.366 (1.1); 7.125 (1.0); 7.119 (7.1); 7.114 (2.3); 7.102 (2.0); 7.097 (6.2); 7.091 (0.8); 6.801 (0.4); 6.766 (3.8); 6.709 (5.4); 6.707 (5.5); 5.757 (13.3); 5.367 (1.4); 5.337 (1.4); 3.522 (1.4); 3.504 (4.2); 3.486 (4.3); 3.469 (1.4); 3.395 (1.3); 3.377 (3.9); 3.359 (4.0); 3.341 (1.7); 3.326 (28.8); 2.677 (0.4); 2.672 (0.5); 2.668 (0.4); 2.646 (0.8); 2.595 (16.0); 2.526 (1.5); 2.512 (28.6); 2.508 (57.0); 2.503 (74.5); 2.499 (53.1); 2.494 (25.1); 2.335 (0.4); 2.330 (0.5); 2.326 (0.4); 2.245 (1.2); 2.163 (8.7); 1.836 (0.4); 1.642 (0.4); 1.299 (0.4); 1.259 (1.0); 1.234 (3.3); 1.214 (0.8); 1.194 (5.7); 1.176 (11.6); 1.158 (5.5); 1.136 (5.1); 1.118 (10.8); 1.100 (4.9); 0.853 (0.4); 0.146 (0.4); 0.008 (3.9); 0.000 (99.1); -0.009 (3.4); -0.150 (0.5) |
| Verbindung Nr. I-1-19: Masse (m/z): 241,1 (M+H)⁺; 1H-NMR (DMSO-D₆) 2.31 (s, 3H), 3.27 - 3.28 (m, 1H), 4.86 (s, 2H), 6.47 (s, 1H), 7.47 - 7.51 (m, 1H), 8.28 - 8.31 (m, 1H), 8.69 - 8.71 (m, 1H), 9.10 (s, 1H), 10.80 (s, 1H). |
| Verbindung Nr. I-1-75, Lösungsmittel: [DMSO], Spektrometer: 399.95MHz |
| 11.105 (0.6); 9.100 (0.6); 9.096 (0.6); 8.719 (0.5); 8.715 (0.5); 8.707 (0.5); 8.703 (0.5); 8.314 (0.3); 8.294 (0.4); 7.822 (0.9); 7.816 (0.9); 7.523 (0.3); 7.521 (0.3); 7.511 (0.3); 7.509 (0.3); 7.503 (0.3); 7.501 (0.3); 7.489 (0.3); 7.375 (1.1); 7.371 (0.4); 7.359 (0.4); 7.354 (1.4); 7.198 (1.1); 7.177 (0.9); 6.661 (0.7); 6.655 (0.7); 5.220 (2.0); 3.331 (6.1); 2.541 (8.0); 2.520 (0.5); 2.511 (7.6); 2.507 (15.8); 2.502 (21.2); 2.497 (15.5); 2.493 (7.5); 1.254 (16.0); 0.000 (6.0) |
| Verbindung Nr. I-1-86, Lösungsmittel: [DMSO], Spektrometer: 399.95MHz |
| 11.192 (0.6); 8.940 (0.6); 8.934 (0.6); 8.359 (0.4); 8.352 (0.4); 8.338 (0.4); 8.331 (0.4); 7.829 (0.8); 7.823 (0.8); 7.659 (0.8); 7.638 (0.6); 7.373 (1.0); 7.357 (0.4); 7.352 (1.3); 7.194 (1.0); 7.174 (0.8); 6.651 (0.7); 6.646 (0.7); 5.219 (1.9); 3.330 (41.1); 2.542 (7.5); 2.524 (0.5); 2.511 (9.8); 2.507 (19.6); 2.502 (25.9); 2.498 (18.8); 2.493 (9.1); 1.290 (0.8); 1.273 (1.0); 1.267 (1.2); 1.253 (16.0); 0.000 (6.9) |
| Verbindung Nr. I-1-70, Lösungsmittel: [DMSO], Spektrometer: 399.95MHz |
| 11.129 (4.3); 9.105 (4.4); 9.101 (4.3); 8.724 (3.7); 8.720 (3.9); 8.712 (3.9); 8.708 (3.9); 8.324 (1.8); 8.320 (2.6); 8.315 (1.8); 8.304 (2.0); 8.299 (2.7); 8.295 (1.8); 7.865 (6.9); 7.859 (6.8); 7.528 (2.6); 7.526 (2.7); 7.516 (2.6); 7.514 (2.6); 7.508 (2.5); 7.506 (2.6); 7.496 (2.5); 7.494 (2.5); 7.432 (1.5); 7.416 (1.7); 7.411 (3.2); 7.395 (2.9); 7.391 (1.9); 7.376 (1.9); 7.156 (1.2); 7.149 (1.3); 7.133 (2.0); 7.128 (2.2); 7.112 (1.0); 7.108 (1.1); 7.106 (1.1); 7.084 (3.4); 7.075 (1.9); 7.065 (5.0); 7.049 (1.9); 7.044 (2.0); 6.696 (5.6); 6.690 (5.5); 5.307 (16.0); 3.330 (211.9); 2.996 (0.3); 2.676 (0.6); 2.671 (0.8); 2.667 (0.6); 2.541 (42.5); 2.524 (1.8); 2.520 (2.8); 2.511 (44.3); 2.507 (90.4); 2.502 (120.2); 2.497 (86.8); 2.493 (41.2); 2.333 (0.6); 2.329 (0.8); 2.324 (0.6); 1.273 (0.4); 1.258 (0.6); 1.242 (0.8); 0.008 (1.1); 0.000 (35.0); -0.009 (1.1) |
| Verbindung Nr. I-1-74, Lösungsmittel: [DMSO], Spektrometer: 399.95MHz |
| 11.118 (1.7); 9.105 (1.6); 9.100 (1.6); 8.722 (1.2); 8.718 (1.3); 8.710 (1.3); 8.706 (1.3); 8.324 (0.6); 8.319 (1.0); 8.314 (0.7); 8.304 (0.7); 8.299 (1.0); 8.294 (0.7); 7.831 (2.5); 7.825 (2.4); 7.524 (1.0); 7.512 (1.0); 7.505 (1.0); 7.492 (0.9); 7.283 (1.0); 7.264 (1.7); 7.244 (1.1); 6.876 (0.8); 6.870 (1.0); 6.856 (0.7); 6.849 (1.0); 6.818 (1.9); 6.813 (2.7); 6.794 (1.1); 6.676 (2.0); 6.671 (2.0); 5.239 (5.8); 3.728 (16.0); 3.330 (109.7); 3.308 (0.4); 2.541 (12.8); 2.524 (0.9); 2.511 (18.3); 2.506 (36.6); 2.502 (48.4); 2.497 (35.4); 2.493 (17.1); 0.000 (8.7) |
| Verbindung Nr. I-1-76, Lösungsmittel: [DMSO], Spektrometer: 399.95MHz |
| 11.125 (5.5); 9.095 (5.4); 9.091 (5.3); 9.076 (2.8); 9.074 (3.0); 9.071 (2.9); 9.069 (2.8); 8.800 (2.3); 8.796 (2.4); 8.788 (2.4); 8.784 (2.4); 8.720 (4.2); 8.716 (4.5); 8.708 (4.5); 8.704 (4.4); 8.317 (2.2); 8.312 (3.2); 8.307 (2.2); 8.297 (2.4); 8.292 (3.4); 8.287 (2.5); 8.285 (2.4); 8.280 (2.4); 8.275 (1.5); 8.265 (1.6); 8.260 (2.4); 8.255 (1.5); 7.823 (7.4); 7.817 (7.4); 7.567 (1.8); 7.565 (1.9); 7.554 (1.8); 7.553 (1.9); 7.547 (1.8); 7.545 (1.8); 7.535 (1.7); 7.533 (1.8); 7.522 (3.1); 7.521 (3.2); 7.509 (3.1); 7.503 (3.0); 7.501 (3.1); 7.490 (2.9); 7.489 (2.9); 7.359 (1.4); 7.342 (1.8); 7.338 (3.0); 7.321 (3.1); 7.316 (2.1); 7.311 (2.1); 7.304 (2.2); 7.300 (1.9); 7.287 (2.3); 7.285 (2.5); 7.281 (2.6); 7.279 (2.4); 7.261 (1.8); 7.255 (1.9); 7.127 (1.6); 7.121 (1.5); 7.106 (2.9); 7.101 (2.9); 7.085 (1.3); 7.080 (1.2); 6.677 (6.7); 6.671 (6.6); 5.315 (16.0); 3.621 (0.4); 3.611 (0.4); 3.333 (271.4); 3.153 (0.5); 3.143 (0.5); 3.135 (0.5); 3.124 (0.5); 2.676 (0.9); 2.671 (1.3); 2.667 (1.0); 2.662 (0.5); 2.542 (9.9); 2.525 (3.1); 2.511 (71.7); 2.507 (146.0); 2.502 (193.8); 2.498 (141.6); 2.493 (68.5); 2.338 (0.5); 2.333 (0.9); 2.329 (1.3); 2.324 (1.0); 1.278 (3.5); 1.261 (6.9); 1.245 (4.1); 1.230 (1.1); 1.215 (1.1); 1.199 (1.1); 1.179 (0.4); 0.008 (1.6); 0.000 (51.9); -0.009 (1.8) |
| Verbindung Nr. I-1-71, Lösungsmittel: [DMSO], Spektrometer: 399.95MHz |
| 11.133 (4.7); 9.099 (4.8); 9.095 (4.8); 8.722 (3.8); 8.718 (4.1); 8.710 (4.0); 8.706 (4.0); 8.319 (2.0); 8.314 (2.8); 8.309 (2.0); 8.299 (2.1); 8.294 (2.9); 8.289 (2.0); 7.908 (7.3); 7.902 (7.3); 7.687 (2.0); 7.668 (3.6); 7.628 (7.2); 7.609 (4.3); 7.590 (2.3); 7.546 (3.6); 7.524 (4.4); 7.522 (3.9); 7.512 (2.8); 7.510 (2.8); 7.504 (2.7); 7.502 (2.7); 7.492 (2.6); 7.490 (2.6); 6.708 (5.9); 6.702 (5.9); 5.399 (16.0); 3.390 (0.3); 3.331 (354.8); 2.996 (2.3); 2.676 (0.8); 2.671 (1.0); 2.667 (0.8); 2.662 (0.4); 2.541 (28.4); 2.524 (3.5); 2.511 (57.0); 2.507 (114.1); 2.502 (151.3); 2.498 (111.3); 2.493 (54.4); 2.338 (0.3); 2.333 (0.7); 2.329 (1.0); 2.324 (0.7); 1.266 (0.8); 1.259 (0.3); 1.237 (1.3); 0.008 (1.1); 0.000 (29.0); -0.009 (1.0) |
| Verbindung Nr. I-1-87, Lösungsmittel: [DMSO], Spektrometer: 399.95MHz |
| 11.216 (5.3); 8.938 (5.0); 8.933 (5.0); 8.890 (0.5); 8.885 (0.5); 8.359 (3.4); 8.353 (3.3); 8.338 (3.6); 8.332 (3.5); 8.302 (0.5); 8.296 (0.5); 8.281 (0.5); 8.275 (0.5); 7.913 (7.5); 7.908 (7.4); 7.687 (2.0); 7.682 (1.7); 7.668 (3.8); 7.660 (7.0); 7.639 (5.7); 7.638 (5.6); 7.627 (7.1); 7.607 (4.4); 7.589 (2.5); 7.544 (3.5); 7.524 (2.2); 6.698 (6.2); 6.692 (6.1); 5.399 (16.0); 5.318 (0.6); 3.642 (0.3); 3.636 (0.6); 3.626 (0.7); 3.620 (0.8); 3.610 (0.8); 3.604 (0.6); 3.593 (0.6); 3.390 (0.4); 3.332 (532.2); 3.292 (0.6); 3.282 (0.4); 3.169 (0.4); 3.159 (0.4); 3.151 (1.0); 3.141 (1.0); 3.133 (1.0); 3.122 (1.1); 3.115 (0.5); 3.104 (0.5); 2.996 (0.7); 2.922 (0.4); 2.676 (1.0); 2.671 (1.3); 2.667 (1.0); 2.662 (0.5); 2.561 (0.5); 2.542 (50.9); 2.525 (3.3); 2.520 (5.1); 2.511 (72.2); 2.507 (147.1); 2.502 (195.0); 2.498 (140.8); 2.493 (66.7); 2.338 (0.4); 2.334 (0.9); 2.329 (1.3); 2.324 (0.9); 1.427 (0.4); 1.281 (7.4); 1.263 (13.2); 1.251 (6.3); 1.246 (8.7); 1.233 (2.7); 1.218 (1.5); 1.211 (0.4); 1.201 (1.6); 1.180 (1.7); 1.162 (0.9); 1.036 (1.3); 1.019 (1.3); 0.008 (1.4); 0.000 (45.4); -0.009 (1.4) |
| Verbindung Nr. I-1-77, Lösungsmittel: [DMSO], Spektrometer: 399.95MHz |
| 11.134 (1.6); 9.099 (1.6); 9.095 (1.6); 8.720 (1.3); 8.716 (1.4); 8.708 (1.4); 8.704 (1.3); 8.319 (0.6); 8.314 (0.9); 8.309 (0.6); 8.299 (0.7); 8.294 (1.0); 8.289 (0.6); 7.905 (0.6); 7.901 (1.1); 7.893 (2.7); 7.888 (3.0); 7.879 (1.0); 7.869 (1.9); 7.556 (0.4); 7.542 (1.4); 7.538 (3.4); 7.520 (2.5); 7.510 (1.0); 7.509 (1.0); 7.502 (1.3); 7.501 (1.3); 7.490 (0.9); 7.489 (0.9); 6.698 (2.0); 6.692 (2.0); 5.369 (5.4); 3.846 (16.0); 3.327 (16.1); 2.671 (0.4); 2.541 (21.6); 2.524 (0.8); 2.520 (1.3); 2.511 (20.8); 2.507 (42.7); 2.502 (57.0); 2.497 (41.3); 2.493 (19.7); 2.329 (0.4); 0.008 (0.5); 0.000 (16.3); -0.009 (0.5) |
| Verbindung Nr. I-1-88, Lösungsmittel: [DMSO], Spektrometer: 399.95MHz |
| 11.127 (3.8); 9.097 (5.0); 9.093 (5.0); 8.717 (4.0); 8.713 (4.2); 8.705 (4.1); 8.701 (4.0); 8.318 (4.1); 8.312 (3.0); 8.307 (2.0); 8.297 (2.2); 8.292 (3.1); 8.287 (2.0); 7.826 (7.2); 7.820 (7.2); 7.519 (3.0); 7.517 (2.8); 7.507 (2.9); 7.505 (2.8); 7.499 (2.9); 7.487 (2.7); 7.485 (2.6); 7.406 (0.8); 7.401 (0.8); 7.392 (0.9); 7.386 (1.9); 7.381 (1.3); 7.376 (1.1); 7.367 (2.0); 7.363 (1.4); 7.354 (1.0); 7.349 (1.2); 7.256 (1.1); 7.251 (3.4); 7.238 (2.9); 7.231 (3.5); 7.227 (2.8); 7.222 (4.4); 7.212 (5.3); 7.202 (2.3); 7.195 (4.1); 7.192 (3.8); 7.176 (1.4); 7.173 (1.2); 6.683 (6.8); 6.678 (6.7); 5.341 (16.0); 3.327 (127.8); 3.304 (1.6); 2.675 (0.7); 2.671 (0.9); 2.666 (0.7); 2.541 (10.7); 2.524 (2.3); 2.519 (3.5); 2.511 (50.7); 2.506 (103.6); 2.502 (137.7); 2.497 (99.4); 2.493 (47.3); 2.333 (0.7); 2.329 (0.9); 2.324 (0.6); 1.239 (0.6); 0.008 (1.4); 0.000 (45.2); -0.009 (1.5) |
| Verbindung Nr. I-1-89, Lösungsmittel: [DMSO], Spektrometer: 399.95MHz |
| 11.143 (4.4); 9.095 (4.1); 9.090 (4.0); 8.719 (3.1); 8.715 (3.2); 8.707 (3.3); 8.703 (3.1); 8.316 (1.8); 8.311 (2.6); 8.306 (1.7); 8.296 (1.9); 8.291 (2.7); 8.286 (1.7); 7.898 (9.4); 7.892 (6.8); 7.879 (3.6); 7.876 (3.6); 7.732 (1.8); 7.729 (1.7); 7.713 (3.7); 7.710 (3.5); 7.694 (2.3); 7.691 (2.1); 7.547 (2.4); 7.545 (2.4); 7.528 (3.9); 7.526 (3.9); 7.520 (2.7); 7.507 (4.1); 7.500 (2.5); 7.488 (2.3); 7.305 (3.6); 7.285 (3.3); 6.727 (5.4); 6.722 (5.3); 5.501 (16.0); 3.328 (246.4); 2.995 (1.1); 2.675 (0.9); 2.671 (1.2); 2.666 (0.9); 2.560 (0.4); 2.541 (17.6); 2.524 (3.0); 2.511 (69.5); 2.506 (140.1); 2.502 (184.9); 2.497 (134.0); 2.493 (64.4); 2.338 (0.4); 2.333 (0.9); 2.329 (1.2); 2.324 (0.8); 2.320 (0.5); 1.388 (0.4); 1.276 (2.3); 1.266 (1.9); 1.260 (4.5); 1.244 (3.6); 1.237 (2.7); 1.228 (1.6); 1.213 (0.5); 1.196 (0.5); 1.177 (0.3); 1.031 (0.4); 1.014 (0.4); 0.853 (0.5); 0.008 (1.4); 0.000 (48.3); -0.009 (1.6) |
| Verbindung Nr. I-1-90, Lösungsmittel: [DMSO], Spektrometer: 399.95MHz |
| 11.128 (5.2); 9.100 (5.1); 9.095 (5.2); 9.074 (0.4); 9.071 (0.4); 8.796 (0.3); 8.788 (0.4); 8.784 (0.3); 8.723 (4.2); 8.719 (4.5); 8.711 (4.4); 8.707 (4.4); 8.321 (2.2); 8.316 (3.2); 8.311 (2.2); 8.301 (2.3); 8.296 (3.3); 8.291 (2.2); 8.285 (0.4); 8.280 (0.4); 8.260 (0.4); 7.852 (7.5); 7.846 (7.6); 7.533 (0.4); 7.526 (3.1); 7.524 (3.0); 7.514 (3.1); |
| 7.512 (3.0); 7.506 (3.0); 7.504 (2.9); 7.494 (2.9); 7.492 (2.8); 7.333 (1.3); 7.321 (1.4); 7.310 (3.5); 7.298 (3.5); 7.287 (2.5); 7.275 (2.4); 7.261 (1.3); 7.251 (1.8); 7.242 (2.0); 7.231 (2.4); 7.222 (1.7); 7.209 (1.0); 7.200 (0.7); 7.088 (1.5); 7.080 (1.5); 7.074 (1.7); 7.066 (2.8); 7.058 (1.5); 7.052 (1.6); 7.044 (1.3); 6.694 (6.6); 6.688 (6.6); 5.340 (16.0); 3.622 (0.4); 3.613 (0.4); 3.332 (259.5); 3.154 (0.5); 3.144 (0.5); 3.136 (0.5); 3.125 (0.5); 2.996 (0.4); 2.680 (0.4); 2.676 (0.8); 2.671 (1.2); 2.667 (0.8); 2.662 (0.4); 2.542 (27.4); 2.525 (2.8); 2.520 (4.3); 2.511 (62.8); 2.507 (129.4); 2.502 (172.8); 2.498 (125.1); 2.493 (59.7); 2.338 (0.4); 2.333 (0.8); 2.329 (1.2); 2.324 (0.8); 2.320 (0.4); 1.277 (3.5); 1.260 (7.0); 1.244 (4.5); 1.228 (1.1); 1.214 (0.4); 1.197 (0.4); 1.035 (0.6); 1.018 (0.6); 0.853 (0.3); 0.008 (1.4); 0.000 (46.9); -0.008 (1.5) |
| Verbindung Nr. I-1-78, Lösungsmittel: [DMSO], Spektrometer: 399.95MHz |
| 11.108 (1.8); 9.101 (1.7); 9.097 (1.8); 8.720 (1.2); 8.716 (1.3); 8.708 (1.3); 8.704 (1.2); 8.315 (1.1); 8.300 (0.7); 8.295 (1.1); 8.291 (0.7); 7.785 (2.4); 7.779 (2.4); 7.522 (1.0); 7.510 (1.1); 7.502 (1.0); 7.490 (0.9); 7.228 (2.9); 7.206 (3.3); 6.927 (0.5); 6.919 (3.7); 6.898 (3.4); 6.650 (2.1); 6.645 (2.1); 5.184 (6.3); 5.103 (0.4); 3.729 (16.0); 3.329 (111.3); 2.671 (0.4); 2.541 (3.9); 2.506 (47.6); 2.502 (62.0); 2.497 (46.9); 2.329 (0.4); 1.033 (0.7); 1.016 (0.7); 0.008 (0.4); 0.000 (9.0); -0.008 (0.5) |
| Verbindung Nr. I-1-91, Lösungsmittel: [DMSO], Spektrometer: 399.95MHz |
| 11.167 (6.9); 9.109 (6.2); 9.104 (6.2); 8.725 (4.9); 8.721 (5.1); 8.713 (5.1); 8.709 (5.0); 8.329 (2.8); 8.324 (4.0); 8.319 (2.8); 8.309 (3.0); 8.304 (4.2); 8.299 (2.8); 7.881 (10.0); 7.875 (10.0); 7.790 (4.7); 7.771 (5.5); 7.675 (2.2); 7.656 (4.9); 7.637 (3.1); 7.545 (3.1); 7.527 (8.5); 7.526 (8.4); 7.515 (4.3); 7.513 (4.4); 7.507 (5.9); 7.506 (5.5); 7.495 (3.9); 7.493 (3.7); 7.204 (0.3); 7.076 (0.4); 6.964 (4.7); 6.945 (4.5); 6.757 (8.6); 6.752 (8.5); 5.507 (16.0); 3.331 (565.4); 2.680 (0.5); 2.676 (1.2); 2.671 (1.6); 2.666 (1.2); 2.662 (0.5); 2.541 (25.4); 2.524 (3.8); 2.520 (6.1); 2.511 (90.5); 2.507 (186.3); 2.502 (248.0); 2.497 (180.2); 2.493 (86.2); 2.338 (0.6); 2.333 (1.2); 2.329 (1.7); 2.324 (1.2); 2.320 (0.6); 1.258 (0.4); 1.239 (1.4); 1.044 (0.4); 1.027 (0.4); 0.853 (0.4); 0.008 (1.9); 0.000 (65.0); -0.009 (2.1) |
| Verbindung Nr. I-1-73, Lösungsmittel: [DMSO], Spektrometer: 399.95MHz |
| 11.144 (4.7); 9.108 (4.3); 9.103 (4.3); 8.730 (3.4); 8.725 (3.6); 8.717 (3.6); 8.713 (3.5); 8.334 (1.9); 8.329 (2.7); 8.324 (1.9); 8.314 (2.0); 8.309 (2.8); 8.304 (1.9); 7.898 (0.3); 7.888 (7.5); 7.882 (7.6); 7.538 (2.8); 7.536 (2.8); 7.526 (2.9); 7.524 (3.2); 7.521 (3.1); 7.518 (3.4); 7.516 (2.9); 7.503 (4.6); 7.501 (6.0); 7.481 (4.1); 7.313 (2.4); 7.310 (2.3); 7.293 (2.1); 7.290 (2.0); 7.260 (3.6); 7.240 (7.0); 7.206 (0.3); 6.706 (6.1); 6.700 (6.0); 5.354 (16.0); 5.272 (0.5); 3.359 (227.0); 3.314 (1.2); 2.995 (0.4); 2.680 (0.4); 2.676 (0.8); 2.671 (1.2); 2.667 (0.8); 2.662 (0.4); 2.564 (0.5); 2.550 (1.0); 2.542 (35.8); 2.525 (2.8); 2.520 (4.1); 2.511 (61.4); 2.507 (128.1); 2.502 (172.3); 2.498 (124.3); 2.493 (58.8); 2.338 (0.4); 2.334 (0.8); 2.329 (1.1); 2.324 (0.8); 2.320 (0.4); 1.956 (0.4); 1.239 (1.2); 1.039 (0.8); 1.022 (0.8); 0.008 (1.3); 0.000 (44.9); -0.009 (1.4) |
| Verbindung Nr. I-1-92, Lösungsmittel: [DMSO], Spektrometer: 399.95MHz |
| 11.155 (0.8); 11.132 (4.1); 9.103 (5.6); 9.099 (5.5); 8.946 (2.9); 8.944 (3.1); 8.941 (3.1); 8.939 (2.7); 8.721 (1.3); 8.717 (4.8); 8.713 (4.2); 8.709 (1.6); 8.705 (5.0); 8.701 (4.0); 8.528 (0.8); 8.452 (2.7); 8.448 (2.8); 8.440 (2.8); 8.436 (2.7); 8.326 (2.1); 8.322 (3.0); 8.317 (2.2); 8.306 (2.2); 8.301 (3.1); 8.297 (2.2); 8.097 (1.8); 8.092 (3.1); 8.088 (1.7); 8.078 (1.8); 8.073 (3.2); 8.068 (1.7); 7.953 (2.4); 7.949 (0.8); 7.936 (1.0); 7.932 (2.6); 7.881 (2.0); 7.876 (2.0); 7.821 (9.3); 7.815 (16.0); 7.799 (3.8); 7.795 (10.2); 7.783 (1.8); 7.762 (1.7); 7.521 (3.0); 7.520 (2.7); 7.509 (3.0); 7.507 (2.8); 7.502 (2.9); 7.500 (2.6); 7.489 (2.8); 7.488 (2.5); 7.403 (1.2); 7.398 (1.2); 7.359 (2.0); 7.338 (1.9); 7.281 (2.1); 7.279 (2.1); 7.269 (2.1); 7.267 (2.0); 7.262 (2.0); 7.260 (1.9); 7.250 (1.9); 7.248 (1.8); 7.138 (8.5); 7.118 (7.8); 7.063 (1.5); 7.042 (1.4); 6.711 (1.5); 6.705 (1.5); 6.674 (6.1); 6.668 (5.9); 5.404 (1.4); 5.398 (1.4); 5.382 (3.5); 5.253 (14.1); 4.997 (2.8); 4.534 (1.7); 4.329 (0.8); 4.311 (2.7); 4.293 (2.8); 4.276 (0.9); 3.335 (328.2); 2.996 (3.0); 2.711 (0.5); 2.680 (0.6); 2.676 (1.3); 2.671 (1.8); 2.666 (1.3); 2.662 (0.6); 2.573 (0.4); 2.541 (94.5); 2.524 (4.1); 2.520 (6.3); 2.511 (92.4); 2.506 (190.1); 2.502 (252.4); 2.497 (181.2); 2.493 (85.0); 2.338 (0.5); 2.333 (1.2); 2.329 (1.6); 2.324 (1.2); 2.320 (0.5); 1.595 (0.6); 1.324 (3.2); 1.307 (6.7); 1.289 (3.1); 1.240 (1.2); 1.220 (0.4); 1.090 (2.1); 1.037 (0.4); 1.020 (0.4); 0.944 (1.9); 0.927 (1.8); 0.008 (2.2); 0.000 (79.0); - 0.009 (2.6); -0.012 (0.7); -0.150 (0.3) |
| Verbindung Nr. I-1-81, Lösungsmittel: [DMSO], Spektrometer: 399.95MHz |
| 11.112 (2.7); 9.109 (2.5); 9.104 (2.4); 8.728 (1.8); 8.724 (1.9); 8.716 (1.9); 8.712 (1.8); 8.338 (1.1); 8.333 (1.6); 8.329 (1.1); 8.318 (1.2); 8.313 (1.7); 8.309 (1.1); 7.730 (4.0); 7.724 (4.0); 7.537 (1.6); 7.525 (1.6); 7.517 (1.5); 7.505 (1.4); 7.206 (0.6); 7.087 (1.9); 7.078 (0.8); 7.068 (3.7); 7.028 (2.1); 7.025 (2.2); 7.006 (1.2); 6.950 (0.6); 6.879 (3.1); 6.674 (3.4); 6.668 (3.3); 5.237 (10.2); 3.398 (49.4); 2.995 (0.4); 2.675 (0.7); 2.671 (1.0); 2.666 (0.7); 2.662 (0.4); 2.563 (0.4); 2.541 (40.7); 2.524 (2.5); 2.519 (3.8); 2.510 (56.2); 2.506 (114.7); 2.501 (152.7); 2.497 (110.5); 2.492 (52.9); 2.337 (0.4); 2.333 (0.7); 2.328 (1.0); 2.324 (0.7); 2.320 (0.4); 2.233 (16.0); 2.224 (15.9); 1.634 (0.3); 1.257 (0.3); 1.239 (1.0); 1.036 (0.5); 1.019 (0.4); 0.008 (1.2); 0.000 (40.7); -0.009 (1.3) |
| Verbindung Nr. 1-1-141: ¹H-NMR(400,0 MHz, d₆-DMSO): 11,374(2,2);9,148(2,2);9,144(2,2);8,816(1,6);8,812(1,6 );8,804(1,7);8,800(1,6);8,359(0,9);8,354(1,3);8,349(0,9);8,339(1,0);8,334(1,4);8,329(0,9);7,613(1,2);7,601(1,2);7, 593(1,2);7,581(1,1);4,022(15,8);3,329(3,8);3,214(16,0);3,175(0,4);3,164(0,3);2,512(6,0);2,508(11,4);2,503(14,4);2 ,499(10,3);2,494(4,9);0,000(5,6) |
| Verbindung Nr. 1-1-142: ¹H-NMR(400,0 MHz, d₆-DMSO): 11,147(3,1);9,112(2,8);9,107(2,9);8,735(1,9);8,731(2,1 |
| );8,723(2,1);8,719(2,0);8,326(1,6);8,322(1,1);8,311(1,2);8,306(1,7);8,302(1,1);7,536(1,6);7,524(1,6);7,516(1,6);7, 504(1,5);6,562(4,4);5,589(9,9);3,958(2,2);3,939(4,4);3,920(2,6);3,510(2,9);3,491(4,9);3,472(2,4);3,331(12,3);3,17 6(0,6);3,163(0,6);2,507(18,0);2,503(23,2);2,499(17,4);2,358(16,0);2,335(0,4);0,000(2,3) |
| Verbindung Nr. I-1-143: ¹H-NMR(400,0 MHz, d₆-DMSO): 11,125(2,5);9,104(2,2);9,100(2,2);8,722(1,5);8,718(1,7 );8,710(1,7);8,706(1,7);8,326(0,9);8,321(1,3);8,316(1,0);8,306(0,9);8,301(1,3);8,296(0,9);7,522(1,2);7,510(1,2);7, 503(1,1);7,502(1,1);7,490(1,1);6,499(3,3);5,787(7,7);3,329(33,7);3,105(16,0);2,511(9,5);2,507(18,5);2,503(24,2);2 ,498(18,2);2,494(9,3);2,443(11,5);2,138(15,8);1,235(0,5);0,008(0,5);0,000(11,0);-0,008(0,5) |
| Verbindung Nr. I-1-144: ¹H-NMR(400,0 MHz, d₆-DMSO): 11,163(2,2);9,111(1,9);9,107(1,9);8,725(1,6);8,721(1,7 );8,713(1,6);8,709(1,6);8,332(0,8);8,328(1,1);8,323(0,8);8,312(0,9);8,307(1,2);8,303(0,8);7,527(1,1);7,525(1,1);7, 515(1,1);7,513(1,1);7,507(1,1);7,505(1,0);7,495(1,0);7,493(1,0);6,516(2,9);5,716(6,5);5,460(0,3);3,873(16,0);3,86 4(1,0);3,327(18,8);2,713(0,6);2,706(0,7);2,697(1,3);2,689(0,6);2,686(0,7);2,681(0,6);2,671(0,4);2,525(0,3);2,512( 7,4);2,508(15,0);2,503(19,8);2,498(14,2);2,494(6,7);2,435(10,1);2,261(0,5);1,235(0,4);0,870(1,4);0,864(2,2);0,860 (1,6);0,853(2,9);0,848(4,4);0,841(3,5);0,834(1,5);0,008(0,4);0,000(11,5);-0,009(0,4) |
| Verbindung Nr. I-1-145: ¹H-NMR(400,0 MHz, d₆-DMSO): 11,050(2,1);9,091(1,9);9,087(1,9);8,729(1,5);8,725(1,6 );8,717(1,6);8,713(1,6);8,314(0,8);8,309(1,1);8,304(0,8);8,294(0,8);8,289(1,2);8,284(0,8);7,530(1,1);7,528(1,1);7, 516(1,1);7,510(1,1);7,509(1,1);7,498(1,0);7,496(1,0);6,529(3,0);5,731(7,2);3,327(22,2);3,176(1,0);3,163(1,0);3,11 7(16,0);2,919(16,0);2,525(0,4);2,511(9,1);2,507(18,3);2,503(24,2);2,498(17,5);2,494(8,4);2,376(11,1);0,008(0,4);0 ,000(11,9);-0,009(0,4) |
| Verbindung Nr. I-1-146: ¹H-NMR(400,0 MHz, d₆-DMSO): 11,132(2,4);9,104(2,1);9,100(2,0);8,723(1,6);8,719(1,7 );8,711(1,7);8,707(1,7);8,326(0,9);8,322(1,2);8,317(0,9);8,306(0,9);8,301(1,3);8,297(0,9);7,525(1,1);7,523(1,2);7, 513(1,1);7,511(1,2);7,505(1,1);7,503(1,1);7,493(1,1);7,491(1,1);6,497(3,2);5,739(7,2);3,327(17,1);2,801(0,6);2,79 8(0,6);2,796(0,6);2,793(0,6);2,786(1,3);2,779(0,6);2,774(0,7);2,771(0,5);2,759(0,3);2,525(0,4);2,512(8,2);2,507(1 6,5);2,503(21,7);2,498(15,6);2,493(7,4);2,431(10,8);2,190(16,0);2,171(0,4);1,235(0,5);0,914(1,5);0,909(2,6);0,899 (3,4);0,891(6,0);0,008(0,5);0,000(12,5);-0,009(0,4) |
| Verbindung Nr. I-1-147: ¹H-NMR(400,0 MHz, d₆-DMSO): 11,150(3,6);9,104(3,1);9,100(3,1);8,723(2,5);8,719(2,7 );8,711(2,7);8,707(2,7);8,326(1,3);8,321(1,8);8,316(1,4);8,306(1,4);8,301(1,9);8,296(1,3);7,524(1,8);7,523(1,8);7, 512(1,7);7,511(1,7);7,505(1,7);7,503(1,7);7,493(1,7);7,491(1,7);7,250(0,7);7,232(0,7);7,182(0,8);7,164(0,7);7,162 (0,6);7,144(0,3);6,498(4,7);5,757(3,9);5,711(10,3);4,056(1,0);4,038(3,0);4,020(3,0);4,002(1,0);3,327(57,5);3,268( 0,5);2,897(0,4);2,891(0,6);2,886(0,9);2,878(1,1);2,870(1,9);2,859(1,0);2,853(0,9);2,843(0,5);2,525(0,6);2,520(1,0) ;2,512(16,4);2,507(33,8);2,503(44,9);2,498(32,2);2,493(15,4);2,440(0,6);2,425(16,0);2,300(3,5);1,989(13,4);1,981 (0,6);1,968(1,0);1,960(1,0);1,956(0,7);1,948(2,0);1,939(0,6);1,935(1,1);1,927(1,0);1,914(0,5);1,193(3,7);1,175(7,2 );1,157(3,6);0,969(2,3);0,963(3,6);0,959(2,6);0,944(8,4);0,935(5,9);0,930(4,7);0,924(3,6);0,915(1,5);0,906(0,4);0, 898(0,4);0,816(1,3);0,806(3,2);0,800(3,0);0,794(3,2);0,788(3,0);0,777(0,9);0,008(0,8);0,000(25,7);-0,009(0,8) |
| Verbindung Nr. I-1-148: ¹H-NMR(600,1 MHz, d₆-DMSO): 3,344(16,0);3,249(0,5);2,509(0,6);2,506(1,3);2,503(1,8 );2,500(1,3);2,497(0,6);2,468(0,4);2,409(0,4) |
| Verbindung Nr. I-1-149: ¹H-NMR(601,6 MHz, d₆-DMSO): 11,058(3,2);9,111(2,8);9,108(2,9);8,725(2,1);8,722(2,4 );8,717(2,3);8,714(2,5);8,324(1,6);8,310(1,7);7,525(1,5);7,517(1,6);7,511(1,6);7,503(1,5);6,543(2,3);6,484(4,2);5, 435(9,8);5,185(0,5);3,344(15,9);3,330(3,7);3,320(2,3);3,092(1,8);3,084(2,7);2,507(27,2);2,504(38,4);2,501(30,7);2 ,355(16,0);2,183(0,7);1,990(0,4);1,791(0,6);1,782(1,7);1,772(2,5);1,763(1,7);1,753(0,7);1,056(0,4);0,005(1,2);0,00 0(28,8) |
| Verbindung Nr. I-1-150: ¹H-NMR(400,0 MHz, d₆-DMSO): 11,514(4,1);9,633(2,4);9,630(3,1);9,628(3,1);9,625(2,7 );9,448(2,5);9,445(2,6);9,435(2,6);9,432(2,6);8,136(2,2);8,130(2,3);8,123(2,2);8,117(2,2);6,512(5,1);5,724(9,8);3, 341(158,3);3,250(0,5);2,897(0,4);2,886(0,9);2,879(1,2);2,870(1,8);2,861(1,2);2,854(1,0);2,844(0,5);2,543(13,7);2, 526(0,9);2,508(33,5);2,503(44,9);2,499(34,9);2,431(16,0);1,981(0,4);1,969(0,9);1,961(1,0);1,948(1,9);1,936(1,1);1 ,928(1,0);1,915(0,5);0,991(0,4);0,963(3,8);0,959(2,9);0,952(5,2);0,946(8,1);0,935(6,5);0,932(7,2);0,925(5,4);0,916 (1,9);0,811(1,2);0,802(3,1);0,796(3,3);0,790(3,4);0,784(3,2);0,773(1,0);0,000(0,9) |
| Verbindung Nr. I-1-151: ¹H-NMR(400,0 MHz, d₆-DMSO): 11,498(3,3);9,631(2,7);9,629(2,6);9,449(2,1);9,447(2,0 );9,436(2,2);9,434(2,1);8,138(1,7);8,132(1,8);8,124(1,8);8,119(1,7);6,513(4,2);5,752(8,1);3,348(65,8);3,095(0,5);2 ,813(0,3);2,801(0,8);2,795(0,9);2,787(1,5);2,777(0,9);2,771(0,8);2,760(0,4);2,544(51,7);2,508(13,9);2,504(17,8);2, 500(14,0);2,439(13,0);2,190(16,0);0,910(3,1);0,892(6,5);0,000(0,5) |
| Verbindung Nr. I-1-152: ¹H-NMR(400,0 MHz, d₆-DMSO): 11,527(2,8);9,636(2,2);9,633(2,3);9,630(1,9);9,450(1,7 );9,448(1,9);9,437(1,8);9,434(1,9);8,141(1,6);8,136(1,6);8,128(1,6);8,122(1,6);6,529(3,7);5,727(7,0);3,870(16,0);3 ,341(171,2);2,723(0,3);2,713(0,8);2,705(0,9);2,697(1,4);2,687(0,8);2,680(0,8);2,670(0,6);2,542(29,8);2,507(34,9); 2,503(45,9);2,499(35,3);2,441(11,7);2,330(0,4);0,864(2,5);0,860(1,9);0,846(5,1);0,839(4,0);0,819(0,4);0,000(1,7) |
| Verbindung Nr. I-1-153: ¹H-NMR(400,0 MHz, d₆-DMSO): 11,024(2,2);9,091(2,0);9,087(2,0);8,722(1,5);8,718(1,6 );8,710(1,6);8,706(1,5);8,311(0,8);8,307(1,2);8,302(0,8);8,291(0,9);8,287(1,2);8,282(0,8);7,521(1,2);7,509(1,2);7, 501(1,2);7,489(1,1);6,540(3,2);5,215(8,2);4,992(0,4);3,329(29,9);3,307(16,5);3,284(1,5);3,263(0,9);3,187(1,1);3,1 29(1,0);3,084(1,1);3,069(16,0);2,965(1,0);2,507(20,8);2,503(26,9);2,498(19,7);2,350(12,1);2,180(0,7);2,153(0,8);1 ,235(0,8);0,000(0,5) |
| Verbindung Nr. I-1-154: ¹H-NMR(400,0 MHz, d₆-DMSO): 11,077(3,2);9,107(3,0);9,102(3,0);8,724(2,0);8,720(2,2 );8,712(2,2);8,708(2,1);8,321(1,8);8,317(1,4);8,306(1,3);8,301(1,8);7,525(1,6);7,513(1,7);7,505(1,7);7,493(1,5);6, 561(4,5);6,087(5,5);5,756(1,9);5,087(8,8);3,326(9,2);2,507(30,1);2,503(38,0);2,499(29,8);2,336(16,0);2,118(14,2); 0,000(38,9) |
| Verbindung Nr. I-1-155: ¹H-NMR(400,0 MHz, d₆-DMSO): 11,036(3,3);9,200(2,9);9,099(2,9);9,094(2,9);8,718(2,1 );8,714(2,3);8,706(2,2);8,702(2,2);8,317(1,1);8,313(1,9);8,307(1,2);8,297(1,2);8,292(1,7);8,287(1,2);7,850(5,3);7, 721(2,0);7,697(2,5);7,521(1,6);7,509(1,6);7,501(1,6);7,489(1,6);7,475(2,1);7,471(2,2);7,452(1,8);7,447(1,8);6,525 (4,4);5,362(8,3);3,913(0,5);3,446(0,4);3,351(732,8);3,249(0,7);3,170(6,1);3,099(0,5);2,996(0,7);2,677(0,7);2,673( 0,9);2,668(0,7);2,543(19,5);2,526(2,2);2,513(51,4);2,508(104,6);2,504(139,9);2,499(105,0);2,495(54,0);2,376(16, 0);2,335(0,9);2,331(1,1);2,326(0,8);2,235(0,3);2,141(0,3);0,008(2,6);0,000(72,2);-0,008(3,3) |
| Verbindung Nr. I-1-156: ¹H-NMR(400,0 MHz, d₆-DMSO): 11,066(3,3);9,111(3,1);9,107(3,2);8,722(2,4);8,718(2,4 );8,710(2,6);8,706(2,3);8,531(2,2);8,527(2,3);8,519(2,4);8,516(2,3);8,331(1,1);8,327(1,7);8,322(1,3);8,316(0,7);8, 312(1,3);8,307(1,8);8,302(1,3);8,208(2,0);8,205(1,9);8,188(2,2);8,185(2,0);7,648(2,1);7,636(2,1);7,628(2,0);7,616 (1,9);7,525(1,7);7,513(1,8);7,505(1,7);7,493(1,7);6,734(2,6);6,703(0,3);6,522(4,3);6,408(0,4);5,323(0,4);5,289(8,0 );5,269(0,5);3,334(11,8);2,996(0,4);2,712(0,5);2,676(0,4);2,672(0,6);2,667(0,4);2,542(129,7);2,525(1,6);2,507(61, 5);2,502(79,6);2,498(58,0);2,494(28,5);2,368(0,7);2,333(16,0);2,235(2,1);2,170(0,7);1,121(1,0);1,106(1,0);0,008(1 ,6);0,000(44,1);-0,009(1,7) |
| Verbindung Nr. I-1-157: ¹H-NMR(400,0 MHz, d₆-DMSO): 11,064(3,3);10,367(0,4);9,112(2,8);9,107(2,9);8,982(0, 4);8,977(0,4);8,727(1,9);8,724(2,1);8,716(2,1);8,712(2,1);8,329(1,1);8,324(1,6);8,320(1,2);8,309(1,2);8,304(1,7);8 ,300(1,2);7,531(1,6);7,519(1,6);7,511(1,6);7,499(1,5);6,914(0,9);6,910(1,0);6,906(0,9);6,900(1,6);6,893(1,4);6,889 (1,9);6,858(2,5);6,852(12,8);6,845(4,8);6,838(2,4);6,827(1,0);6,820(0,4);6,799(0,4);6,707(0,8);6,697(0,4);6,501(4, 4);6,146(0,5);4,609(0,3);4,604(0,4);4,594(1,1);4,589(1,2);4,579(1,4);4,574(1,3);4,565(0,7);4,559(0,6);4,360(1,8);4 ,354(1,7);4,343(0,5);4,331(2,2);4,325(2,0);4,306(5,1);4,291(4,2);4,272(0,3);4,068(1,5);4,053(1,5);4,039(1,4);4,024 (1,3);4,016(0,4);3,326(17,1);2,507(34,3);2,502(45,4);2,498(35,3);2,329(0,3);2,253(16,0);2,175(2,3);1,235(1,3);0,0 07(1,3);0,000(29,0) |
| Verbindung Nr. I-1-158: ¹H-NMR(400,0 MHz, d₆-DMSO): 11,026(3,0);9,106(2,4);9,103(2,6);8,719(2,0);8,715(2,3 );8,707(2,2);8,703(2,2);8,325(0,9);8,320(1,4);8,316(1,4);8,306(1,0);8,300(1,5);8,296(1,1);7,520(1,7);7,508(1,6);7, 500(1,6);7,488(1,6);7,027(0,5);7,015(1,7);6,996(2,3);6,979(1,1);6,961(0,7);6,953(0,4);6,940(0,6);6,936(0,6);6,919 (0,3);6,860(4,7);6,841(1,5);6,566(1,1);6,512(3,1);6,347(0,4);6,329(0,4);6,104(0,3);5,756(16,0);5,170(2,2);5,130(5, 8);5,118(0,9);3,324(35,3);2,890(0,4);2,753(0,6);2,738(1,1);2,732(1,1);2,722(0,8);2,670(5,3);2,667(5,2);2,606(0,6); 2,595(0,6);2,524(1,2);2,519(1,7);2,511(27,5);2,506(58,4);2,502(80,3);2,497(61,0);2,493(31,8);2,333(0,5);2,329(0, 7);2,324(0,5);2,255(12,0);2,216(4,4);2,195(0,4);2,191(0,4);2,159(1,1);2,142(1,5);2,093(0,4);2,059(0,4);2,046(0,6); 1,803(0,4);1,791(0,6);1,776(0,7);1,760(0,5);1,719(3,0);1,703(6,7);1,695(4,5);1,687(3,1);1,675(1,9);1,234(1,6);0,00 8(1,7);0,000(61,8);-0,008(3,1) |
| Verbindung Nr. I-1-159: ¹H-NMR(400,0 MHz, d₆-DMSO): 11,024(0,6);9,102(0,5);9,098(0,6);8,718(0,7);8,713(0,7 );8,706(0,5);8,702(0,4);8,294(0,3);7,711(1,0);6,514(0,8);5,310(1,6);4,121(1,3);4,107(3,8);4,094(3,9);4,081(1,4);3, 339(9,5);3,178(16,0);3,165(15,6);2,508(4,9);2,504(6,5);2,500(5,1);2,374(2,9);0,000(4,6) |
| Verbindung Nr. I-1-160: ¹H-NMR(400,0 MHz, d₆-DMSO): 11,028(3,5);9,103(3,1);9,098(3,1);8,794(3,6);8,791(3,6 );8,718(2,0);8,714(2,2);8,706(2,2);8,702(2,2);8,315(1,7);8,295(1,8);7,699(5,5);7,568(2,7);7,544(3,2);7,519(1,6);7, 507(1,7);7,500(1,6);7,488(1,5);7,289(2,3);7,284(2,4);7,265(2,0);7,260(2,0);6,517(4,7);5,318(9,0);3,329(22,4);2,50 8(22,1);2,504(28,5);2,500(22,5);2,371(16,0);0,000(33,8) |
| Verbindung Nr. I-1-161: ¹H-NMR(400,0 MHz, d₆-DMSO): 11,126(2,9);9,119(2,8);9,114(2,9);8,757(2,1);8,753(2,3 );8,745(2,2);8,741(2,3);8,337(1,1);8,332(1,7);8,328(1,2);8,317(1,3);8,312(1,8);8,308(1,2);8,134(0,4);7,559(1,6);7, 547(1,6);7,539(1,6);7,527(1,5);6,665(4,1);5,756(7,0);3,670(1,2);3,609(0,4);3,592(0,4);3,575(0,3);3,492(1,4);3,355 (1,0);3,239(0,6);3,014(0,9);2,525(0,5);2,507(27,5);2,503(36,7);2,498(27,7);2,421(16,0);1,861(5,8);1,739(0,6);1,25 3(0,6);1,235(0,4);1,180(0,6);0,008(0,7);0,000(21,4);-0,008(1,0) |
| Verbindung Nr. I-1-162: ¹H-NMR(600,1 MHz, d₆-DMSO): 11,412(3,3);9,135(2,9);9,134(3,0);9,131(3,0);8,760(2,4 );8,757(2,5);8,752(2,5);8,749(2,5);8,351(1,3);8,349(1,8);8,345(1,2);8,338(1,3);8,335(1,8);8,332(1,3);7,555(1,7);7, 554(1,6);7,547(1,7);7,546(1,6);7,542(1,7);7,540(1,6);7,534(1,6);7,532(1,5);6,815(4,3);3,769(1,8);3,749(1,9);3,327 (25,2);2,835(1,1);2,831(1,3);2,814(2,4);2,810(2,4);2,793(1,3);2,789(1,1);2,511(4,7);2,508(10,2);2,505(14,0);2,502 (10,1);2,499(4,7);2,478(16,0);2,477(15,3);1,990(0,5);1,678(1,5);1,674(1,5);1,656(1,6);1,653(1,6);1,456(0,5);1,450 (0,6);1,445(0,5);1,439(0,6);1,432(0,5);1,426(0,4);1,420(0,3);1,134(0,6);1,127(0,6);1,113(1,3);1,107(1,3);1,093(1,2 );1,086(1,2);1,072(0,5);1,065(0,5);0,870(8,7);0,859(8,5) |
| Verbindung Nr. I-1-163: ¹H-NMR(601,6 MHz, d₆-DMSO): 19,977(0,5);11,275(12,2);9,126(12,4);8,762(8,4);8,754 (8,6);8,336(6,4);8,323(7,0);7,698(3,6);7,689(6,9);7,680(3,9);7,564(5,5);7,556(5,9);7,551(6,1);7,543(5,5);6,735(16, 0);5,974(0,5);5,762(1,0);3,484(5,6);3,475(15,7);3,467(13,6);3,447(7,4);3,439(12,7);3,429(10,5);3,393(0,6);3,348(8 4,9);3,296(1,1);3,279(70,5);3,237(5,8);3,159(0,4);3,147(0,6);3,137(1,3);3,129(1,2);2,617(0,5);2,537(54,5);2,505(9 0,0);2,427(0,4);2,390(0,6);0,000(44,0) |
| Verbindung Nr. I-1-164: ¹H-NMR(400,0 MHz, d₆-DMSO): 9,122(0,9);9,118(0,9);8,766(0,6);8,762(0,6);8,754(0,6) |
| ;8,750(0,6);8,337(0,3);8,332(0,5);8,327(0,4);8,317(0,5);8,312(0,6);8,307(0,4);7,783(0,4);7,569(0,5);7,557(0,5);7,5 50(0,5);7,538(0,4);6,732(1,3);4,252(0,3);4,237(0,5);4,223(0,4);4,028(0,6);4,012(0,6);4,006(0,8);3,990(1,4);3,974( 1,8);3,960(1,7);3,947(3,0);3,928(2,7);3,912(1,6);3,723(0,5);3,709(0,6);3,702(0,5);3,687(0,5);3,634(1,8);3,619(2,2) ;3,615(2,1);3,600(1,4);3,457(0,4);3,438(0,4);3,424(0,6);3,409(0,4);3,364(0,5);3,349(0,7);3,334(0,6);3,315(0,5);3,3 00(0,4);3,277(0,3);3,261(0,3);3,160(0,3);2,648(0,7);2,634(0,7);2,616(2,5);2,602(2,4);2,590(2,4);2,576(2,1);2,558( 0,9);2,537(4,3);2,507(10,2);2,503(13,2);2,499(10,2);1,362(4,3);1,328(1,4);1,305(14,7);1,270(4,5);1,250(16,0);1,11 2(0,4);0,008(0,7);0,000(10,7) |
| Verbindung Nr. I-1-165: ¹H-NMR(601,6 MHz, d₆-DMSO): 9,121(1,4);9,118(1,4);8,761(1,0);8,759(1,0);8,753(1,0) ;8,751(1,0);8,330(0,5);8,327(0,8);8,321(2,9);8,317(0,7);8,313(0,9);8,310(0,6);7,924(0,3);7,914(0,7);7,905(0,4);7,5 63(0,7);7,555(0,7);7,550(0,7);7,542(0,7);6,705(1,9);3,347(1,0);3,336(1,8);3,325(1,7);3,314(0,9);3,041(0,8);3,031( 0,9);3,020(0,4);2,590(1,6);2,579(3,2);2,568(1,7);2,529(6,8);2,507(14,6);2,504(20,6);2,501(16,6);2,490(2,0);2,485( 2,9);2,472(2,1);2,444(0,9);2,432(1,2);2,420(0,9);2,235(0,6);2,054(11,8);2,028(8,8);2,026(16,0);1,810(1,0);1,798(1, 5);1,787(1,0);1,621(0,6);1,609(0,8);1,601(0,7);1,597(0,7);1,590(1,5);1,578(1,9);1,566(1,5);1,555(0,5);0,000(13,3) |
| Verbindung Nr. I-1-166: ¹H-NMR(400,0 MHz, d₆-DMSO): 9,136(4,0);9,130(4,0);8,764(2,8);8,760(3,1);8,752(3,0) ;8,748(3,1);8,350(1,5);8,345(2,4);8,340(1,7);8,330(1,7);8,325(2,6);8,320(1,8);7,564(2,2);7,552(2,2);7,544(2,3);7,5 32(4,1);7,510(2,2);6,741(5,5);6,740(5,9);4,058(0,4);4,045(0,9);4,042(0,8);4,028(1,1);4,024(1,1);4,007(0,9);3,993( 0,5);3,464(0,5);3,440(1,2);3,426(1,2);3,416(3,7);3,402(6,2);3,389(3,7);3,377(1,5);3,364(1,6);3,335(3,2);3,290(34,1 );3,280(2,1);3,273(0,6);3,261(0,5);3,243(3,5);3,236(16,0);3,116(0,8);3,112(0,4);3,103(0,8);3,094(1,7);3,080(1,9);3 ,068(1,6);3,051(1,9);3,046(0,7);3,029(0,8);2,938(0,6);2,921(1,0);2,907(0,8);2,891(0,5);2,673(0,4);2,538(20,8);2,52 2(0,7);2,513(9,3);2,509(19,6);2,504(26,6);2,500(19,6);2,495(9,7);2,483(0,4);2,465(0,3);2,236(0,4);2,098(0,4);2,02 5(1,2);1,187(12,4);1,170(12,2);1,003(1,5);0,987(1,4);0,918(7,2);0,911(0,5);0,902(7,1);0,000(2,1) |
| Verbindung Nr. I-1-167: ¹H-NMR(400,0 MHz, d₆-DMSO): 9,123(2,3);9,118(2,5);8,765(1,4);8,762(1,7);8,753(1,6) ;8,750(1,7);8,337(0,9);8,332(1,4);8,328(1,0);8,317(1,3);8,313(1,5);8,308(1,1);7,941(0,7);7,926(1,3);7,911(0,7);7,5 68(1,2);7,556(1,3);7,548(1,3);7,536(1,2);6,718(3,5);3,484(1,2);3,467(2,8);3,451(2,9);3,435(1,5);3,339(1,2);3,326( 1,2);3,283(1,0);3,232(0,7);3,215(0,8);3,197(0,8);3,188(0,7);3,182(0,6);3,172(0,9);3,155(0,9);3,139(0,5);3,111(0,3) ;2,688(2,8);2,672(5,7);2,658(3,3);2,537(11,9);2,508(18,5);2,504(24,5);2,500(20,8);2,483(4,3);2,466(4,2);2,450(2,0 );2,097(16,0);2,076(0,7);2,068(0,8);2,062(1,0);2,054(3,9);2,049(5,5);2,026(12,9);1,795(2,3);0,000(1,3) |
| Verbindung Nr. I-1-168: ¹H-NMR(400,0 MHz, d₆-DMSO): 11,226(11,5);9,119(9,6);9,114(10,0);8,761(6,6);8,757( 7,4);8,749(7,2);8,745(7,4);8,332(3,8);8,327(5,7);8,322(4,2);8,316(1,3);8,312(4,2);8,307(6,0);8,302(4,3);7,921(2,8) ;7,907(5,9);7,892(3,0);7,562(5,0);7,561(5,1);7,549(5,1);7,542(5,1);7,541(5,1);7,530(4,8);7,529(4,8);7,319(8,0);7,3 14(3,5);7,307(1,9);7,301(12,1);7,298(13,9);7,284(4,2);7,279(12,2);7,274(5,2);7,261(1,1);7,255(2,9);6,976(16,0);6, 968(6,1);6,966(6,1);6,956(13,4);6,954(13,1);6,947(12,0);6,929(5,2);6,924(6,6);6,904(1,1);6,734(14,1);6,732(14,3); 6,253(0,7);6,239(1,5);6,224(0,8);5,756(1,6);4,148(7,1);4,133(15,9);4,119(8,2);3,958(2,2);3,944(4,9);3,930(2,5);3,6 75(4,0);3,661(11,0);3,647(10,7);3,633(3,7);3,397(1,2);3,383(3,4);3,369(3,3);3,354(1,2);3,331(54,7);2,677(0,4);2,6 72(0,5);2,668(0,4);2,550(46,9);2,526(1,3);2,512(28,3);2,508(58,2);2,503(79,0);2,499(61,1);2,335(0,4);2,330(0,5);2 ,326(0,4);0,008(0,7);0,000(21,2);-0,008(1,2) |
| Verbindung Nr. I-1-169: ¹H-NMR(400,0 MHz, d₆-DMSO): 10,933(2,0);9,035(1,8);9,031(1,8);8,812(1,1);8,808(1,3 );8,800(1,2);8,796(1,3);8,233(0,6);8,228(1,0);8,224(0,7);8,213 (0,7);8,208(1,0);8,204(0,7);7,628(0,9);7,616(0,9);7, 608(0,9);7,596(0,8);6,424(3,5);3,690(1,7);3,602(2,4);3,589(3,5);3,332(12,5);3,295(0,5);3,270(0,6);3,242(16,0);2,5 42(1,8);2,507(13,3);2,503(17,2);2,499(13,5);2,208(9,7);0,000(3,8) |
| Verbindung Nr. I-1-170: ¹H-NMR(601,6 MHz, d₆-DMSO): 9,121(1,8);9,118(1,8);8,765(1,4);8,763(1,4);8,757(1,4) ;8,755(1,4);8,333(0,7);8,330(1,0);8,327(0,7);8,320(0,9);8,317(1,1);8,314(0,7);7,630(0,5);7,621(0,7);7,566(1,0);7,5 58(1,0);7,553(1,0);7,545(0,9);6,741(2,7);4,660(0,9);4,652(2,0);4,643(0,9);4,487(0,4);4,385 (0,6);4,336(1,0);4,327( 2,2);4,318(1,1);3,671(0,5);3,659(1,6);3,655(0,7);3,647(1,6);3,643(2,0);3,636(0,6);3,631(2,0);3,620(0,7);3,617(0,7) ;3,610(0,4);3,605(1,8);3,601(1,2);3,594(2,0);3,589(2,6);3,585(0,8);3,582(1,0);3,578(2,3);3,574(0,7);3,566(0,7);3,5 32(0,6);3,520(1,9);3,516(0,6);3,508(2,0);3,504(1,6);3,497(0,7);3,492(1,6);3,481(0,7);3,478(0,9);3,466(2,5);3,462( 0,8);3,454(2,7);3,450(2,1);3,442(1,1);3,438(2,0);3,427(0,7);3,376(2,5);3,366(4,7);3,357(5,3);3,075(0,5);3,066(0,8) ;3,056(0,4);2,588(0,7);2,579(0,7);2,538(11,6);2,529(3,0);2,510(7,7);2,507(16,5);2,505(22,6);2,502(16,4);1,141(6,7 );1,129(14,3);1,125(8,4);1,118(9,0);1,113(16,0);1,107(3,7);1,104(3,1);1,101(7,8);1,095(1,7);0,005(0,5);0,000(16,2 );-0,006(0,6) |
| Verbindung Nr. I-1-171: ¹H-NMR(400,0 MHz, d₆-DMSO): 11,240(4,2);9,124(3,5);9,119(3,6);8,764(2,4);8,760(2,7 );8,752(2,6);8,748(2,7);8,339(1,3);8,334(2,0);8,329(1,5);8,319(1,5);8,314(2,2);8,309(1,6);7,942(1,0);7,928(2,1);7, 913(1,1);7,566(1,9);7,554(1,9);7,546(1,9);7,534(1,8);7,177(0,4);7,157(5,9);7,152(3,3);7,142(5,4);7,132(6,2);7,123 (2,3);7,118(2,6);7,106(0,7);6,738(5,0);6,736(5,2);6,284(0,4);6,270(0,7);6,255(0,4);5,758(16,0);4,259(2,4);4,246(5, 2);4,232(2,7);4,069(1,0);4,055(2,2);4,041(1,1);3,641(1,5);3,627(3,9);3,613(3,8);3,599(1,3);3,358(0,7);3,343(2,4);3 ,334(39,9);3,315(0,8);2,542(17,3);2,527(0,8);2,513(11,3);2,509(23,2);2,504(31,1);2,500(23,7);0,008(0,3);0,000(9, 5) |
| Verbindung Nr. I-1-172: ¹H-NMR(400,0 MHz, d₆-DMSO): 11,207(10,8);9,121(9,1);9,117(9,2);8,767(6,5);8,763(7, 1);8,755(7,0);8,751(7,1);8,336(3,7);8,331(5,4);8,326(3,9);8,316(4,2);8,311(5,7);8,306(4,0);8,041(2,5);8,026(5,2);8 ,012(2,6);7,571(4,8);7,570(4,9);7,559(4,8);7,558(4,9);7,551(4,8);7,550(4,8);7,539(4,5);7,538(4,6);6,727(13,2);4,24 |
| 1(7,8);4,227(16,0);4,214(8,6);3,613(3,5);3,599(9,2);3,585(8,9);3,572(3,3);3,330(64,6);2,677(0,4);2,672(0,6);2,668 (0,4);2,540(45,1);2,526(2,4);2,521(2,5);2,512(28,8);2,508(59,2);2,503(79,8);2,499(60,0);2,495(30,9);2,335(0,4);2, 330(0,5);2,326(0,4);1,769(0,8);1,234(1,0);1,173(0,5);1,152(0,4);1,133(0,4);0,991(0,5);0,146(0,4);0,008(3,4);0,000 (95,6);-0,008(4,7);-0,150(0,4) |
| Verbindung Nr. I-1-173: ¹H-NMR (DMSO-d6) 2.26 (s, 3H), 3.22 (s, 3H), 3.62 - 3.65 (t, 2H), 4.10 - 4.12 (t, 2H), 6.43 (s, 1H), 7.49 - 7.53 (m, 1H), 8.29 - 8.32 (m, 1H), 8.71 - 8.72 (m, 1H), 9.10 - 9.11 (m, 1H), 11.00 (s, 1H). |
| Verbindung Nr. I-1-174: ¹H-NMR (DMSO-d6) 0.32 - 0.35 (m, 2H), 0.48 - 0.52 (m, 2H), 1.15 - 1.23 (m, 1H), 2.27 (s, 3H), 3.84 - 3.86 (d, 2H), 6.45 (s, 1H), 7.49 - 7.52 (m, 1H), 8.30 - 8.32 (m, 1H), 8.71 - 8.72 (m, 1H), 9.10 (s, 1H), 11.00 (s, 1H). |
| Verbindung Nr. I-1-175: ¹H-NMR (DMSO-d6) 2.32 (s, 3H), 3.23 (2, 3H), 5.29 (s, 2H), 6.57 (s, 1H), 7.50 - 7.54 (m, 1H), 8.30 - 8.33 (m, 1H), 8.72 - 8.73 (m, 1H), 9.11 (s, 1H), 11.10 (s, 1H). |
| Verbindung Nr. I-1-176: ¹H-NMR (DMSO-d6) 1.64 - 1.70 (m, 1H), 1.75 - 1.82 (m, 2H), 1.88 - 1.95 (m, 1H), 2.27 (s, 3H), 3.59 - 3.64 (m, 1H), 3.70 - 3.75 (m, 1H), 3.99 - 4.00 (d, 2H), 4.11 - 4.16 (m, 1H), 6.44 (s, 1H), 7.49 - 7.53 (m, 1H), 8.29 - 8.32 (m, 1H), 8.71 - 8.72 (m, 1H), 9.11 (s, 1H), 11.00 (s, 1H). |
| Verbindung Nr. I-1-177: ¹H-NMR (DMSO-d6) 0.09 - 0.32 (m, 4H), 0,95 (m, 1H), 2.17 (s, 3H), 3.28 (s, 3H), 3.73 - 3.75 (d, 2H), 5.87 (s, 1H), 7.32 (m, 1H), 7.68 (m, 1H), 8.46 - 8.50 (m, 2H). |
| Verbindung Nr. I-1-178: ¹H-NMR (DMSO-d6) 2.19 (s, 3H), 2.87 (s, 3H), 3.30 (s, 3H), 5.17 (s, 2H), 6.03 (s, 1H), 7.32 - 7.35 (m, 1H), 7.72 - 7.73 (m, 1H), 8.48 - 8.50 (m, 2H). |
| Verbindung Nr. I-1-179: 1H-NMR (DMSO-d6) 2.15 (s, 3H), 3.09 (s, 3H), 3.21 - 3.46 (m, 5H), 3.97 - 3.99 (m, 2H), 5.84 (s, 1H), 7.32 (m, 1H), 7.68 (m, 1H), 8.46 - 8.51 (m, 2H). |
| Verbindung Nr. I-1-180: ¹H-NMR (DMSO-d6) 2.28 (s, 3H), 3.79 - 3.90 (m, 4H), 4.10 - 4.11 (d, 2H), 5.13 - 5.15 (t, 1H), 6.47 (s, 1H), 7.49 - 7.53 (m, 1H), 8.30 - 8.33 (m, 1H), 8.71 - 8.72 (m, 1H), 9.11 (s, 1H), 11.05 (s, 1H). |
| Verbindung Nr. I-1-181: ¹H-NMR (DMSO-d6) 1.58 - 1.78 (m, 4H), 2.16 (s, 3H), 3.28 (s, 3H), 3.54 - 3.59 (m, 2H), 3.86 - 3.91 (m, 3H), 5.87 (s, 1H), 7.32 (m, 1H), 7.69 (m, 1H), 8.46 - 8.51 (m, 2H). |
| Verbindung Nr. I-1-182: ¹H-NMR (DMSO-d6) 2.32 (s, 3H), 2.91 (s, 3H), 3.61 - 3.64 (t, 2H), 4.37 - 4.40 (t, 2H), 6.48 (s, 1H), 7.50 - 7.53 (m, 1H), 8.30 - 8.32 (m, 1H), 8.72 - 8.73 (m, 1H), 9.10 (s, 1H), 11.06 (s, 1H). |
| Verbindung Nr. I-1-183: ¹H-NMR (DMSO-d6) 2.31 (s, 3H), 2.56 (s, 3H), 3.04 - 3.10 (m, 1H), 3.22 - 3.29 (m, 1H), 4.33 - 4.37 (m, 2H), 6.48 (s, 1H), 7.50 - 7.53 (m, 1H), 8.29 - 8.32 (m, 1H), 8.71 - 8.73 (m, 1H), 9.10 (s, 1H), 11.05 (s, 1H). |
| Verbindung Nr. I-1-184: ¹H-NMR (DMSO-d6) 2.03 (s, 3H), 2.31 (s, 3H), 2.83 - 2.87 (t, 2H), 4.13 - 4.17 (t, 2H), 6.46 (s, 1H), 7.49 - 7.53 (m, 1H), 8.30 - 8.32 (m, 1H), 8.71 - 8.72 (m, 1H), 9.11 (s, 1H), 11.03 (s, 1H). |
| Verbindung Nr. I-1-185: ¹H-NMR (DMSO-d6) 2.32 (s, 3H), 2.97 - 3.01 (t, 2H), 4.25 - 4.28 (t, 2H), 6.50 (s, 1H), 7.50 - 7.53 (m, 1H), 8.30 - 8.32 (m, 1H), 8.72 - 8.73 (m, 1H), 9.11 (s, 1H), 11.07 (s, 1H). |
| Verbindung Nr. I-1-186: ¹H-NMR (DMSO-d6) 2.19 (s, 3H), 2.46 (s, 3H), 2.92 (m, 2H), 3.29 (s, 3H), 4.22 - 4.24 (m, 2H), 5.87 (s, 1H), 7.33 (m, 1H), 7.68 (m, 1H), 8.46 - 8.53 (m, 2H). |
| Verbindung Nr. I-1-187: ¹H-NMR (DMSO-d6) 1.93 (s, 3H), 2.19 (s, 3H), 2.60 (m, 2H), 3.28 (s, 3H), 4.02 - 4.05 (m, 2H), 5.83 (s, 1H), 7.31 (m, 1H), 7.68 (m, 1H), 8.47 - 8.51 (m, 2H). |
| Verbindung Nr. I-1-188: ¹H-NMR (DMSO-d6) 2.28 (s, 3H), 2.77 - 2.85 (m, 2H), 4.20 - 4.24 (t, 2H), 6.47 (s, 1H), 7.50 - 7.53 (m, 1H), 8.29 - 8.32 (m, 1H), 8.71 - 8.72 (m, 1H), 9.10 (s, 1H), 11.05 (s, 1H). |
| Verbindung Nr. I-1-189: ¹H-NMR (DMSO-d6) 1.15 - 1.18 (t, 3H), 2.30 (s, 3H), 2.46 - 2.52 (m, 2H), 2.86 - 2.90 (t, 2H), 4.12 - 4.15 (t, 2H), 6.45 (s, 1H), 7.50 - 7.53 (m, 1H), 8.30 - 8.32 (m, 1H), 8.71 - 8.72 (m, 1H), 9.11 (s, 1H), 11.03 (s, 1H). |
| Verbindung Nr. I-1-190: ¹H-NMR (DMSO-d6) 1.16 - 1.19 (t, 3H), 2.31 (s, 3H), 2.61 - 2.70 (m, 1H), 2.75 - 2.82 (m, 1H), 3.02 - 3.08 (m, 1H), 3.17 - 3.24 (m, 1H), 4.30 - 4.40 (m, 2H), 6.48 (s, 1H), 7.50 - 7.53 (m, 1H), 8.29 - 8.32 (m, 1H); 8.72 - 8.73 (m, 1H), 9.10 (s, 1H), 11.05 (s, 1H). |
| Verbindung Nr. I-1-191: ¹H-NMR (DMSO-d6) 2.26 (s, 3H), 3.68 (s, 3H), 4.24 - 4.27 (t, 2H), 4.38 - 4.40 (t, 2H), 6.47 (s, 1H), 7.50 - 7.53 (m, 1H), 8.30 - 8.32 (m, 1H), 8.72 (m, 1H), 9.11 (s, 1H), 11.04 (s, 1H). |
| Verbindung Nr. I-1-192: ¹H-NMR(400,0 MHz, d₆-DMSO): 11,078(2,6);9,094(2,4);9,090(2,4);8,723(1,6);8,719(1,7 );8,711(1,7);8,707(1,6);8,313(0,9);8,308(1,4);8,304(0,9);8,293(1,0);8,288(1,5);8,284(1,0);7,744(2,8);7,739(2,9);7, 522(1,3);7,509(1,3);7,502(1,3);7,490(1,2);6,506(3,6);6,173(8,4);6,066(2,9);6,061(2,9);5,756(2,9);3,327(15,2);2,52 5(0,6);2,511(9,0);2,507(17,8);2,502(23,3);2,498(17,0);2,464(13,7);2,135(16,0);0,008(1,1);0,000(28,7);-0,008(1,2) |
| Verbindung Nr. I-1-193: ¹H-NMR(400,0 MHz, d₆-DMSO): 11,221(3,0);9,145(1,4);8,742(1,1);8,733(1,1);8,362(1,0 );8,358(1,6);8,353(1,1);8,342(1,1);8,338(1,7);8,333(1,1);7,549(1,3);7,537(1,3);7,529(1,3);7,517(1,2);7,436(4,0);7, 415(6,2);7,336(4,9);7,315(3,4);6,732(4,3);3,328(23,9);3,176(0,3);3,163(0,3);2,525(0,6);2,511(14,1);2,507(27,9);2, 502(36,1);2,498(26,2);2,494(12,7);2,373(14,1);2,338(16,0);2,320(0,8);2,216(0,6);2,075(0,4);0,008(1,4);0,000(35,4 |
| );-0,008(1,4) |
| Verbindung Nr. I-1-194: ¹H-NMR(400,0 MHz, d₆-DMSO): 11,160(2,7);9,125(2,2);9,121(2,2);8,825(7,1);8,813(7,2 );8,779(1,8);8,775(1,8);8,767(1,9);8,763(1,8);8,338(0,9);8,334(1,3);8,328(0,9);8,318(1,0);8,313(1,3);8,308(0,9);7, 574(1,2);7,572(1,2);7,562(1,2);7,560(1,2);7,554(1,2);7,552(1,2);7,542(1,2);7,540(1,1);7,497(1,6);7,484(3,0);7,472 (1,5);5,596(7,3);3,325(13,6);2,524(0,6);2,511(12,4);2,507(24,2);2,502(31,2);2,498(22,2);2,493(10,5);2,475(16,0);0 ,008(0,4);0,000(9,9);-0,009(0,3) |
| Verbindung Nr. I-1-195: ¹H-NMR(400,0 MHz, d₆-DMSO): 10,993(3,2);9,094(3,9);8,933(8,4);8,928(11,5);8,715(2, 7);8,703(2,7);8,311(1,8);8,306(2,0);8,291(1,9);8,286(2,0);7,517(1,7);7,504(1,8);7,497(1,6);7,485(1,4);6,533(4,6);5 ,449(10,0);3,329(13,9);3,324(19,7);2,507(36,7);2,502(37,3);2,312(16,0);0,004(6,1);0,000(8,5) |
| Verbindung Nr. I-1-197: ¹H-NMR(601,6 MHz, d₆-DMSO): 11,263(4,2);9,110(3,8);9,107(3,8);8,808(12,6);8,800(1 2,9);8,770(2,1);8,761(2,2);8,736(3,2);8,733(3,3);8,728(3,4);8,725(3,3);8,324(1,6);8,321(2,3);8,318(1,6);8,311(1,7) ;8,307(2,4);8,304(1,7);7,533(2,2);7,531(2,2);7,525(2,2);7,523(2,2);7,519(2,2);7,518(2,2);7,511(2,2);7,510(2,1);7,4 75(2,8);7,467(5,5);7,459(2,7);7,434(0,5);7,425(1,0);7,417(0,5);6,817(8,5);5,577(2,2);5,534(16,0);5,247(3,3);4,796 (1,3);4,023(0,3);3,323(51,2);2,523(0,4);2,520(0,5);2,517(0,5);2,508(15,4);2,505(34,4);2,502(48,7);2,499(35,7);2,4 96(17,1);1,989(1,4);1,187(0,4);1,175(0,7);1,163(0,4);0,000(11,6);-0,006(0,5) |
| Verbindung Nr. I-1-198: ¹H-NMR(400,0 MHz, d₆-DMSO): 11,065(1,5);9,117(1,9);9,112(1,9);8,734(1,4);8,730(1,5 );8,722(1,5);8,718(1,5);8,332(0,8);8,327(1,2);8,322(0,8);8,312(0,8);8,307(1,2);8,302(0,8);7,631(2,5);7,625(2,6);7, 539(1,2);7,527(1,1);7,519(1,1);7,507(1,1);6,607(2,2);6,601(2,3);3,793(16,0);3,553(0,5);3,324(5,3);2,511(6,5);2,50 7(13,3);2,502(17,8);2,498(13,2);2,494(6,6);0,008(0,6);0,000(17,0);-0,009(0,7) |
| Verbindung Nr. I-1-199: ¹H-NMR(400,0 MHz, d₆-DMSO): 10,516(1,0);10,429(0,5);9,126(2,8);8,769(1,4);8,765(1, 6);8,757(2,3);8,753(2,5);8,744(1,2);8,325(1,5);8,316(1,4);8,305(1,7);8,270(0,4);7,640(3,6);7,571(1,2);7,559(2,0);7 ,551(2,3);7,541(2,5);7,527(0,8);7,502(0,5);7,490(0,7);7,412(0,5);5,399(9,6);5,344(0,4);4,981(1,3);4,903(0,6);4,879 (0,8);4,645(1,1);4,590(0,6);3,685(2,7);3,667(0,5);3,328(10,6);2,777(0,4);2,758(0,5);2,739(0,4);2,675(0,5);2,671(0, 7);2,667(0,5);2,506(81,4);2,502(106,4);2,497(77,9);2,333(0,5);2,329(0,7);2,324(0,5);1,942(0,6);1,899(16,0);1,858( 1,4);1,848(1,5);1,810(0,6);1,492(0,4);1,327(0,4);1,309(0,3);0,903(0,8);0,885(1,5);0,867(0,7);0,146(0,7);0,008(6,2) ;0,000(143,9);-0,009(6,6);-0,150(0,7) |
| Verbindung Nr. I-1-200: ¹H-NMR(400,0 MHz, d₆-DMSO): 10,478(1,8);8,995(1,1);8,761(1,0);8,757(1,0);8,749(1,0 );8,745(1,0);8,189(0,6);8,169(0,6);7,559(0,8);7,547(0,8);7,540(0,8);7,528(0,7);7,436(2,6);7,419(1,0);7,414(2,9);7, 406(0,4);7,021(0,4);7,012(4,0);7,006(1,2);6,995(1,2);6,989(3,6);6,981(0,4);6,282(3,0);5,755(2,2);3,823(1,1);3,797 (0,7);3,789(0,4);3,780(0,5);3,761(16,0);3,324(18,8);2,524(0,5);2,511(11,1);2,506(22,6);2,502(29,8);2,497(21,2);2, 493(10,0);2,240(10,5);2,210(0,7);1,989(0,7);1,719(0,3);1,429(0,3);1,175(0,4);0,008(0,6);0,000(19,3);-0,009(0,6) |
| Verbindung Nr. I-1-201: ¹H-NMR(400,0 MHz, d₆-DMSO): 10,541(1,8);9,114(1,3);9,111(1,3);8,791(1,3);8,787(1,4 );8,779(1,4);8,775(1,3);8,308(0,8);8,288(0,8);7,591(0,9);7,590(0,9);7,579(1,0);7,578(0,9);7,572(0,9);7,560(0,9);3, 919(14,7);3,326(6,4);2,512(4,9);2,507(9,9);2,503(12,9);2,498(9,2);2,494(4,4);2,402(16,0);0,000(4,9) |
| Verbindung Nr. I-1-202: ¹H-NMR(400,0 MHz, d₆-DMSO): 11,270(3,1);9,150(2,0);8,751(1,5);8,742(1,5);8,369(1,1 );8,365(1,7);8,360(1,1);8,349(1,2);8,344(1,7);8,340(1,1);7,603(0,8);7,583(1,9);7,566(1,5);7,561(2,2);7,546(2,1);7, 540(1,5);7,528(1,4);7,483(0,9);7,478(1,7);7,472(1,2);7,453(3,5);7,450(3,7);7,433(1,5);7,432(1,6);7,429(1,3);7,286 (0,8);7,285(0,8);7,280(0,8);7,265(1,4);7,260(1,4);7,257(1,1);7,244(0,7);7,242(0,7);7,238(0,7);6,785(4,4);3,359(2,1 );2,525(0,6);2,512(12,2);2,508(24,0);2,503(31,1);2,499(22,2);2,494(10,6);2,420(16,0);2,379(0,4);1,989(1,1);1,175( 0,6);0,008(1,8);0,000(40,6);-0,009(1,5) |
| Verbindung Nr. I-1-203: ¹H-NMR(400,0 MHz, d₆-DMSO): 11,232(3,0);9,147(2,4);9,143(2,5);8,750(1,7);8,746(1,9 );8,738(1,9);8,734(1,9);8,367(1,1);8,362(1,6);8,357(1,1);8,347(1,2);8,342(1,7);8,337(1,2);7,615(2,7);7,610(1,0);7, 603(2,9);7,598(1,7);7,593(3,5);7,586(1,2);7,581(3,4);7,572(0,4);7,556(1,6);7,544(1,5);7,536(1,5);7,526(1,4);7,524 (1,4);7,391(3,5);7,386(1,1);7,379(0,5);7,374(1,1);7,369(5,5);7,364(1,2);7,360(0,4);7,353(0,9);7,347(2,9);6,750(4,3 );3,389(0,6);2,525(0,6);2,520(0,9);2,512(14,1);2,507(29,4);2,503(39,1);2,498(28,2);2,494(13,5);2,347(16,0);2,330( 0,4);2,239(1,1);0,008(1,9);0,000(57,8);-0,009(2,2) |
| Verbindung Nr. I-1-204: ¹H-NMR(400,0 MHz, d₆-DMSO): 10,997(2,8);9,108(2,3);9,104(2,2);9,075(0,5);9,072(0,5 );8,714(1,6);8,711(1,7);8,703(1,6);8,328(1,1);8,324(1,6);8,319(1,1);8,308(1,2);8,304(1,6);8,299(1,0);8,279(0,4);8, 259(0,4);7,552(0,3);7,532(0,3);7,517(1,5);7,505(1,5);7,498(1,5);7,486(1,3);6,429(4,3);4,081(0,4);4,072(0,5);4,054 (0,9);4,044(1,0);4,038(0,7);4,026(0,6);4,016(0,6);3,329(27,8);2,507(29,6);2,503(36,7);2,498(26,3);2,276(16,0);2,2 05(0,4);1,989(0,6);1,826(4,1);1,805(3,1);1,779(2,2);1,770(1,5);1,750(1,5);1,742(1,3);1,718(0,7);1,694(0,9);1,661( 1,0);1,463(0,5);1,429(1,4);1,405(1,3);1,397(1,5);1,373(0,5);1,366(0,5);1,212(0,5);1,198(1,6);1,193(0,6);1,180(1,0) ;1,175(1,0);1,157(0,7);1,152(0,7);1,147(0,8);0,008(3,4);0,000(45,0);-0,008(1,9) |
| Verbindung Nr. I-1-205: ¹H-NMR(400,0 MHz, d₆-DMSO): 11,034(2,4);9,111(2,1);9,107(2,1);8,714(1,5);8,710(1,6 );8,702(1,6);8,699(1,5);8,333(1,0);8,328(1,4);8,324(1,0);8,313(1,1);8,309(1,5);8,304(0,9);7,516(1,4);7,504(1,4);7, 496(1,3);7,484(1,3);6,436(4,1);4,214(0,5);4,200(0,7);4,197(0,7);4,191(0,7);4,184(0,7);4,177(0,7);4,161(0,5);4,038 (1,0);4,020(1,0);4,002(0,3);3,328(24,2);2,671(0,4);2,524(1,0);2,511(24,4);2,506(49,8);2,502(65,8);2,497(47,2);2,4 93(22,5);2,329(0,4);2,324(0,3);2,267(16,0);1,989(4,3);1,859(0,4);1,841(0,6);1,837(0,5);1,825(0,8);1,819(0,6);1,80 7(0,8);1,803(0,8);1,784(0,7);1,725(0,6);1,712(0,8);1,707(0,8);1,694(0,9);1,678(0,5);1,673(0,6);1,660(0,4);1,363(8, |
| 8);1,347(8,8);1,193(1,2);1,175(2,3);1,157(1,1);0,729(4,1);0,710(9,0);0,692(3,9);0,146(0,4);0,008(3,0);0,000(88,3); -0,009(3,2);-0,014(0,6);-0,150(0,4) |
| Verbindung Nr. I-1-206: ¹H-NMR(400,0 MHz, d₆-DMSO): 11,285(3,4);9,148(3,2);9,144(3,1);8,754(2,1);8,750(2,2 );8,742(2,2);8,738(2,1);8,365(1,2);8,360(1,8);8,356(1,2);8,345(1,4);8,340(1,9);7,895(3,7);7,889(3,7);7,805(3,2);7, 783(4,0);7,623(2,3);7,617(2,1);7,601(1,9);7,595(1,7);7,558(1,6);7,546(1,7);7,538(1,6);7,526(1,5);6,797(4,7);3,569 (0,6);3,328(30,0);2,507(36,0);2,503(43,7);2,499(32,3);2,430(16,0);0,000(50,6) |
| Verbindung Nr. I-1-207: ¹H-NMR(400,0 MHz, d₆-DMSO): 11,223(3,1);9,135(2,8);9,131(2,8);8,744(2,0);8,740(2,2 );8,732(2,1);8,728(2,1);8,352(1,1);8,348(1,6);8,343(1,1);8,333(1,2);8,328(1,7);8,323(1,1);7,939(2,6);7,936(4,1);7, 933(2,5);7,633(9,0);7,630(9,4);7,549(1,6);7,537(1,6);7,529(1,6);7,517(1,5);6,760(4,3);5,757(2,2);3,328(25,3);2,51 2(13,7);2,507(26,5);2,503(34,1);2,498(24,4);2,494(11,7);2,126(16,0);1,234(0,5);0,008(1,5);0,000(33,4);-0,009(1,1) |
| Verbindung Nr. I-1-208: ¹H-NMR(400,0 MHz, d₆-DMSO): 11,200(1,6);9,145(1,4);9,140(1,4);8,745(1,1);8,741(1,2 );8,733(1,1);8,729(1,1);8,362(0,6);8,357(0,9);8,352(0,6);8,342(0,6);8,337(0,9);8,332(0,6);7,550(0,8);7,549(0,8);7, 537(0,8);7,530(0,8);7,529(0,8);7,518(0,7);7,517(0,7);7,158(3,5);7,039(1,6);6,727(2,2);5,756(1,2);3,325(20,6);2,52 5(0,5);2,511(9,9);2,507(19,9);2,502(26,0);2,498(18,6);2,493(8,7);2,361(8,5);2,342(16,0);1,989(0,4);0,008(2,5);0,0 00(56,5);-0,009(2,0) |
| Verbindung Nr. I-1-209: ¹H-NMR(400,0 MHz, d₆-DMSO): 11,264(2,3);9,146(2,2);9,142(2,2);8,749(1,6);8,745(1,7 );8,737(1,7);8,733(1,7);8,363(0,9);8,359(1,2);8,354(0,9);8,343(0,9);8,338(1,3);8,334(0,9);7,619(0,3);7,610(0,4);7, 596(16,0);7,581(0,5);7,573(0,5);7,553(1,2);7,552(1,2);7,541(1,2);7,540(1,2);7,534(1,2);7,532(1,2);7,522(1,1);7,52 0(1,1);7,445(0,3);6,773(3,2);3,325(15,2);2,525(0,6);2,507(31,2);2,502(40,2);2,498(29,3);2,385(11,6);2,384(11,4);1 ,989(0,5);0,146(0,4);0,008(3,8);0,000(80,5);-0,009(3,3);-0,150(0,4) |
| Verbindung Nr. I-1-210: ¹H-NMR(400,0 MHz, d₆-DMSO): 11,059(2,5);9,112(2,4);9,108(2,4);8,712(1,7);8,708(1,8 );8,700(1,8);8,697(1,8);8,336(1,0);8,331(1,4);8,326(1,0);8,316(1,1);8,311(1,5);8,307(1,0);7,514(1,4);7,502(1,4);7, 494(1,4);7,482(1,3);6,450(4,1);5,146(0,5);4,389(0,3);3,948(0,6);3,935(0,8);3,923(1,1);3,912(0,8);3,899(0,6);3,324 (26,1);2,511(17,0);2,507(33,3);2,502(43,1);2,498(31,5);2,263(15,2);2,084(1,9);1,989(0,9);1,850(0,6);1,832(0,9);1, 826(0,7);1,816(1,4);1,808(1,0);1,798(1,4);1,792(1,5);1,773(1,6);1,754(1,6);1,743(1,5);1,736(1,6);1,724(1,7);1,720 (1,4);1,708(1,0);1,702(1,0);1,691(0,7);1,684(0,3);1,175(0,5);0,699(7,5);0,681(16,0);0,662(7,1);0,652(1,4);0,634(2, 2);0,616(1,0);0,146(0,3);0,008(3,0);0,000(69,3);-0,008(2,9);-0,150(0,3) |
| Verbindung Nr. I-1-211: ¹H-NMR(600,1 MHz, d₆-DMSO): 10,607(3,0);10,491(0,8);9,112(3,2);9,099(0,5);9,095(0, 4);8,813(4,2);8,808(12,1);8,805(4,7);8,800(12,0);8,792(0,9);8,784(0,8);8,759(2,8);8,757(3,2);8,751(2,6);8,749(2,5 );8,311(1,7);8,298(1,9);7,560(0,6);7,554(2,1);7,546(2,2);7,541(2,1);7,533(1,7);7,476(0,9);7,469(3,9);7,461(5,8);7, 453(2,7);7,442(0,4);6,533(1,0);5,545(4,4);5,457(13,1);5,429(1,0);4,036(0,5);4,025(0,5);3,337(60,7);2,522(0,3);2,5 19(0,4);2,510(8,7);2,507(18,0);2,504(24,5);2,501(17,7);2,498(8,3);2,324(1,5);2,300(7,6);2,274(16,0);1,990(2,1);1, 187(0,5);1,175(1,1);1,164(0,5);0,000(0,9) |
| Verbindung Nr. I-1-212: ¹H-NMR(600,1 MHz, d₆-DMSO): 10,548(0,8);9,139(0,7);8,814(3,9);8,805(4,1);8,799(0,8 );8,791(0,8);8,380(0,4);8,369(0,4);7,628(0,5);7,620(0,5);7,615(0,5);7,607(0,4);7,477(0,9);7,469(1,8);7,460(0,9);5, 546(4,3);3,581(1,0);2,569(12,5);2,511(2,4);2,508(5,0);2,505(6,8);2,502(4,9);2,499(2,3);2,301(7,2);2,076(16,0) |
| Verbindung Nr. I-1-213: ¹H-NMR(400,0 MHz, d₆-DMSO): 11,195(0,6);9,138(0,7);9,133(0,7);8,744(0,5);8,740(0,5 );8,732(0,5);8,728(0,5);8,350(0,4);8,330(0,4);7,730(0,5);7,711(0,5);7,706(0,4);7,586(0,5);7,582(0,7);7,578(0,4);7, 572(0,8);7,568(0,9);7,565(1,0);7,558(0,7);7,553(0,7);7,549(0,5);7,541(0,4);7,537(0,7);7,529(0,4);7,528(0,4);7,517 (0,5);7,277(0,3);6,749(1,1);6,681(0,3);3,3311(10,0);2,512(3,6);2,508(6,9);2,503(8,9);2,499(6,3);2,494(2,9);2,115(3, 9);1,756(16,0);0,008(0,8);0,000(17,2);-0,009(0,6) |
| Verbindung Nr. I-1-214: ¹H-NMR(400,0 MHz, d₆-DMSO): 11,228(2,8);9,126(1,7);8,735(1,3);8,725(1,3);8,349(1,1 );8,344(1,6);8,340(1,1);8,329(1,2);8,324(1,7);8,320(1,1);7,537(1,4);7,526(1,4);7,518(1,4);7,506(1,3);6,578(4,3);5, 828(0,7);5,810(2,6);5,793(2,7);5,775(0,8);3,334(4,3);2,525(0,5);2,512(10,7);2,508(21,6);2,503(28,3);2,498(20,7);2 ,494(10,4);2,333(16,0);1,989(0,7);1,747(9,4);1,730(9,3);1,175(0,4); 0,008(2,0);0,000(51,6);-0,009(2,5) |
| Verbindung Nr. I-1-215: ¹H-NMR(400,0 MHz, d₆-DMSO): 11,128(4,8);9,201(4,1);9,197(4,1);8,781(3,3);8,777(3,5 );8,769(3,9);8,761(14,1);8,749(13,7);8,401(2,2);8,381(2,3);7,813(4,1);7,792(4,4);7,670(4,2);7,649(5,0);7,587(2,4); 7,574(2,4);7,567(2,4);7,555(2,2);7,443(3,3);7,431(6,7);7,419(3,4);7,410(2,9);7,407(3,4);7,389(2,2);7,387(2,1);7,15 0(2,7);7,132(3,7);7,114(2,3);5,845(16,0);3,327(39,2);2,671(0,4);2,506(50,9);2,502(64,8);2,497(46,9);2,328(0,4);0, 008(1,9);0,000(41,3);-0,008(1,7) |
| Verbindung Nr. I-1-216: ¹H-NMR(400,0 MHz, d₆-DMSO): 11,233(4,1);9,085(3,9);8,876(0,4);8,872(0,8);8,868(0,4 );8,827(0,9);8,819(4,8);8,812(4,6);8,760(15,2);8,748(15,5);8,735(1,0);8,729(0,9);8,310(1,9);8,286(2,0);7,826(4,1); 7,806(4,6);7,788(0,5);7,677(4,5);7,656(5,3);7,613(0,4);7,592(0,6);7,443(3,7);7,431(9,1);7,419(4,2);7,416(3,6);7,41 3(3,6);7,409(2,5);7,394(2,5);7,392(2,4);7,158(2,8);7,157(2,8);7,139(3,6);7,121(2,5);7,119(2,3);5,850(16,0);5,756( 2,9);5,059(1,4);3,350(0,4);3,326(62,7);3,188(0,4);3,172(0,4);2,675(0,4);2,671(0,6);2,667(0,4);2,541(0,4);2,524(1,4 );2,511(32,7);2,506(66,0);2,502(86,0);2,497(60,5);2,493(28,0);2,333(0,4);2,329(0,5);2,324(0,4);1,739(0,3);1,722(0 ,5);0,146(0,8);0,008(6,9);0,000(177,6);-0,009(6,0);-0,150(0,8) |
| Verbindung Nr. I-1-217: ¹H-NMR(400,0 MHz, d₆-DMSO): 11,290(3,6);9,148(3,3);9,144(3,3);8,753(2,3);8,744(2,2 |
| );8,361(1,9);8,341(2,0);8,086(3,6);7,952(1,7);7,931(2,1);7,892(2,0);7,872(2,6);7,766(2,2);7,746(3,1);7,726(1,3);7, 601(0,3);7,561(1,7);7,549(1,8);7,541(1,7);7,529(1,5);6,809(4,8);3,330(17,1);2,503(45,7);2,436(16,0);2,392(0,6);2, 330(0,4);1,990(0,7);1,233(0,4);1,193(0,4);1,175(0,5);0,000(2,8) |
| Verbindung Nr. I-1-218: ¹H-NMR(400,0 MHz, d₆-DMSO): 11,042(2,6);9,117(2,5);9,112(2,5);8,717(1,8);8,713(1,9 );8,705(1,9);8,701(1,9);8,338(1,0);8,333(1,5);8,328(1,0);8,318(1,1);8,313(1,6);8,308(1,0);7,519(1,4);7,507(1,4);7, 499(1,4);7,487(1,3);6,430(4,4);3,627(1,1);3,610(1,2);3,604(1,2);3,587(1,1);3,325(9,3);3,169(1,5);2,671(0,4);2,524 (1,0);2,511(22,0);2,506(44,0);2,502(57,8);2,498(41,8);2,493(20,5);2,329(0,4);2,231(16,0);2,089(0,9);1,463(8,1);1, 447(7,9);1,370(0,6);1,353(0,6);1,330(0,4);1,321(0,5);1,308(0,9);1,298(0,8);1,287(0,9);1,275(0,5);1,267(0,4);1,248 (0,4);0,606(0,3);0,597(0,5);0,593(0,5);0,584(1,1);0,577(0,9);0,572(0,9);0,563(1,1);0,551(0,6);0,541(0,5);0,412(0,4 );0,399(0,6);0,390(1,0);0,378(0,9);0,369(1,3);0,357(1,0);0,349(1,1);0,343(0,9);0,336(1,2);0,323(1,4);0,313(1,2);0, 300(0,5);0,285(0,6);0,272(1,0);0,262(1,2);0,256(0,7);0,249(1,1);0,240(0,7);0,229(0,4);0,146(0,3);0,008(3,0);0,000 (74,1);-0,009(3,0);-0,150(0,4) |
| Verbindung Nr. I-1-219: ¹H-NMR(400,0 MHz, d₆-DMSO): 11,128(1,2);9,109(1,2);9,104(1,2);8,734(0,9);8,730(0,9 );8,722(0,9);8,718(0,9);8,330(0,5);8,325(0,7);8,320(0,5);8,310(0,5);8,306(0,7);8,301(0,5);7,534(0,7);7,522(0,7);7, 514(0,7);7,502(0,7);6,653(1,9);3,329(8,3);2,515(8,9);2,507(10,4);2,503(13,0);2,499(9,5);1,989(0,7);1,974(16,0);0, 000(1,2) |
| Verbindung Nr. I-1-220: ¹H-NMR(400,0 MHz, d₆-DMSO): 10,969(4,1);9,083(3,9);9,079(3,9);8,725(2,8);8,721(3,1 );8,713(3,0);8,709(3,0);8,303(1,5);8,298(2,3);8,293(1,6);8,283(1,6);8,278(2,4);8,273(1,6);7,523(2,3);7,511(2,2);7, 503(2,2);7,491(2,1);7,367(5,4);7,364(5,5);6,484(6,1);6,202(16,0);6,049(5,1);6,047(5,1);5,756(0,5);4,047(0,6);3,32 4(36,3);2,671(0,4);2,667(0,3);2,511(25,6);2,506(51,6);2,502(68,2);2,497(50,5);2,488(48,9);2,458(0,4);2,324(0,6);0 ,146(0,4);0,008(3,1);0,000(85,5);-0,009(3,3);-0,150(0,4) |
| Verbindung Nr. I-1-221: ¹H-NMR(400,0 MHz, d₆-DMSO): 11,274(2,4);9,127(2,6);9,122(2,6);8,753(1,9);8,749(2,0 );8,741(2,0);8,737(2,0);8,342(1,1);8,337(1,6);8,332(1,0);8,322(1,1);8,317(1,7);8,312(1,0);7,557(1,5);7,545(1,5);7, 537(1,5);7,525(1,4);7,453(1,4);7,320(3,2);7,186(1,6);6,969(3,5);3,873(16,0);3,333(21,1);2,526(0,4);2,513(8,3);2,5 08(16,6);2,504(21,5);2,499(15,3);2,495(7,3);0,000(7,2) |
| Verbindung Nr. I-1-222: ¹H-NMR(400,0 MHz, d₆-DMSO): 11,377(2,5);9,011(3,3);8,789(3,1);8,783(3,2);8,265(1,0 );8,260(1,3);8,254(1,0);8,241(1,0);8,236(1,3);8,230(1,0);7,456(1,4);7,323(3,1);7,189(1,5);6,968(3,6);3,877(16,0);3 ,329(10,5);2,526(0,4);2,508(16,8);2,504(21,8);2,499(15,7);0,008(0,4);0,000(9,1);-0,008(0,4) |
| Verbindung Nr. I-1-223: ¹H-NMR(400,0 MHz, d₆-DMSO): 11,123(4,8);9,090(4,5);9,085(4,5);8,726(3,1);8,723(3,2 );8,714(3,2);8,711(3,1);8,309(1,8);8,305(2,6);8,300(1,8);8,289(1,9);8,285(2,7);8,280(1,7);8,210(7,8);7,782(7,5);7, 525(2,5);7,513(2,5);7,505(2,4);7,493(2,3);6,631(16,0);6,561(6,9);6,343(0,4);3,330(40,0);2,672(0,4);2,507(45,5);2, 503(57,0);2,498(44,1);2,493(38,4);2,329(0,5);2,307(0,5);0,000(52,9) |
| Verbindung Nr. I-1-224: ¹H-NMR(400,0 MHz, d₆-DMSO): 11,097(4,9);9,087(4,6);9,083(4,6);8,736(10,5);8,724(3, 3);8,720(3,5);8,712(3,2);8,709(3,1);8,302(2,8);8,286(2,0);8,282(2,8);8,037(9,7);7,522(2,6);7,510(2,6);7,502(2,5);7 ,490(2,3);6,539(7,3);6,417(16,0);3,332(41,9);3,176(0,4);3,163(0,4);2,672(0,3);2,507(40,7);2,503(51,7);2,499(42,0) ;2,493(39,1);2,329(0,5);0,000(3,7) |
| Verbindung Nr. I-1-225: ¹H-NMR(400,0 MHz, d₆-DMSO): 11,088(2,7);9,094(2,9);9,089(2,9);8,724(1,9);8,720(2,1 );8,712(2,1);8,708(2,1);8,313(1,1);8,308(1,7);8,304(1,2);8,293(1,2);8,288(1,8);8,284(1,2);7,876(3,3);7,870(3,4);7, 518(3,9);7,514(4,2);7,503(1,7);7,491(1,5);6,515(4,4);6,301(2,6);6,296(4,5);6,288(11,1);3,339(1,4);2,507(19,0);2,5 03(24,6);2,498(18,6);2,477(16,0);0,000(2,2) |
| Verbindung Nr. I-1-226: ¹H-NMR(400,0 MHz, d₆-DMSO): 11,074(2,7);9,094(2,5);9,090(2,6);8,725(1,9);8,721(2,0 );8,713(2,0);8,709(2,0);8,314(1,1);8,309(1,6);8,304(1,1);8,294(1,2);8,289(1,6);8,284(1,1);7,602(4,3);7,525(1,5);7, 524(1,5);7,513(1,5);7,512(1,5);7,506(1,5);7,504(1,5);7,493(1,4);7,492(1,4);7,310(5,1);6,500(4,1);6,190(10,2);3,35 3(1,3);2,525(0,7);2,511(14,1);2,507(28,7);2,502(37,9);2,498(27,4);2,493(13,3);2,455(15,5);1,991(16,0);0,000(4,0) |
| Verbindung Nr. I-1-227: ¹H-NMR(400,0 MHz, d₆-DMSO): 11,027(3,1);9,075(3,1);9,071(3,0);8,717(2,1);8,714(2,1 );8,706(2,3);8,702(2,0);8,295(1,7);8,275(1,8);7,757(3,4);7,515(1,6);7,503(1,6);7,495(1,6);7,483(1,5);6,979(3,6);6, 557(4,5);6,381(10,1);3,329(20,7);2,506(35,1);2,502(42,3);2,498(31,1);2,477(16,0);2,329(0,4);1,234(0,4);0,000(3,4 ) |
| Verbindung Nr. I-1-228: ¹H-NMR(400,0 MHz, d₆-DMSO): 11,125(3,3);9,094(2,8);9,090(2,8);8,727(1,9);8,723(2,0 );8,715(2,0);8,711(1,9);8,313(1,1);8,309(1,7);8,304(1,1);8,293(1,2);8,289(1,8);8,284(1,1);8,126(2,7);8,123(2,8);7, 526(1,6);7,514(1,6);7,506(1,6);7,494(1,5);6,796(3,2);6,790(3,2);6,560(4,6);6,419(9,8);3,336(6,4);2,508(23,3);2,50 3(29,9);2,499(21,8);2,474(16,0);0,000(2,8) |
| Verbindung Nr. I-1-229: ¹H-NMR(400,0 MHz, d₆-DMSO): 11,036(2,5);9,145(2,6);9,141(2,6);9,090(0,6);9,089(0,6 );9,085(0,7);8,816(0,5);8,812(0,6);8,804(0,6);8,800(0,6);8,761(2,0);8,757(2,1);8,748(2,1);8,744(2,0);8,421(1,0);8, 417(1,4);8,412(0,9);8,401(1,0);8,397(1,5);8,392(0,9);8,320(0,4);8,316(0,5);8,300(0,3);8,296(0,5);7,606(1,4);7,594 (1,5);7,586(1,7);7,574(1,4);7,567(0,4);6,441(4,3);4,651(1,0);4,632(1,5);4,614(1,1);4,594(0,4);2,526(0,6);2,512(13, 4);2,508(27,0);2,503(35,1);2,499(24,8);2,494(11,5);2,282(16,0);2,060(0,4);2,041(0,8);2,031(1,2);2,024(0,9);2,010( 1,4);2,000(1,1);1,938(0,4);1,921(1,1);1,902(1,4);1,889(1,1);1,884(1,0);1,871(0,9);1,853(0,9);1,836(1,1);1,821(1,4) ;1,813(1,4);1,803(1,0);1,780(0,3);1,659(0,3);1,650(0,6);1,640(1,2);1,631(1,4);1,625(1,6);1,611(1,3);1,604(0,8);0,0 |
| 00(7,3) |
| Verbindung Nr. 1-1-230: ¹H-NMR(400,0 MHz, d₆-DMSO): 13,055(2,3);9,154(2,4);9,150(2,3);8,857(1,7);8,854(1,8 );8,845(1,8);8,842(1,7);8,615(1,4);8,612(1,4);8,602(1,4);8,600(1,4);8,347(1,0);8,342(1,3);8,337(1,0);8,327(1,1);8, 321(1,4);8,317(1,1);8,084(0,7);8,080(0,7);8,066(1,0);8,063(1,4);8,059(1,3);8,045(1,2);8,040(1,2);7,988(2,5);7,967 (1,5);7,684(1,2);7,683(1,2);7,672(1,2);7,671(1,2);7,664(1,2);7,663(1,2);7,653(1,1);7,651(1,1);7,394(1,1);7,392(1,1 );7,382(1,1);7,379(1,3);7,376(1,3);7,374(1,1);7,364(1,1);7,361(1,0);6,784(5,2);3,327(45,5);2,525(0,8);2,512(14,4); 2,508(28,0);2,503(36,2);2,499(26,1);2,494(12,6);2,284(16,0);0,000(5,4) |
| Verbindung Nr. 1-1-231: ¹H-NMR(601,6 MHz, d₆-DMSO): 11,416(1,2);9,131(1,2);9,128(1,2);8,758(0,9);8,752(0,8 );8,750(0,9);8,347(0,7);8,333(0,7);7,556(0,6);7,549(0,6);7,544(0,7);7,535(0,6);6,816(1,7);5,414(0,8);3,345(32,9);2 ,911(16,0);2,615(0,4);2,602(0,3);2,504(25,1);2,478(6,3);2,391(1,2);1,178(0,3);1,166(0,7);1,154(0,3);0,000(13,4) |
| Verbindung Nr. 1-1-232: ¹H-NMR(400,0 MHz, d₆-DMSO): 9,926(0,8);8,494(2,9);8,485(2,9);8,482(2,8);8,422(2,5) ;7,640(1,4);7,621(1,6);7,371(5,3);7,365(5,5);7,352(2,4);7,340(2,4);7,333(2,2);7,320(1,9);7,070(2,5);7,064(2,5);7,0 49(2,8);7,043(2,7);6,662(4,2);6,641(3,8);6,491(0,5);6,443(0,4);5,756(10,0);4,055(0,3);4,038(1,0);4,020(1,0);4,002 (0,3);3,744(0,9);3,666(1,4);3,632(16,0);3,600(0,5);3,326(51,8);2,671(0,4);2,667(0,3);2,506(53,6);2,502(69,4);2,49 7(52,1);2,329(0,5);2,324(0,4);2,300(0,7);2,268(1,0);2,254(1,4);2,223(15,3);1,989(4,3);1,193(1,2);1,175(2,3);1,157 (1,1);0,146(0,4);0,008(3,6);0,000(74,7);-0,008(3,6);-0,150(0,4) |
| Verbindung Nr. 1-1-233: ¹H-NMR(400,0 MHz, d₆-DMSO): 11,659(4,5);9,174(3,4);9,170(3,6);8,770(2,4);8,766(2,7 );8,758(2,7);8,754(2,7);8,396(1,4);8,391(2,0);8,386(1,5);8,376(1,6);8,371(2,1);8,366(1,6);7,570(1,8);7,568(1,8);7, 557(1,8);7,556(1,8);7,550(1,8);7,548(1,8);7,537(1,8);7,536(1,8);7,524(2,3);7,519(1,2);7,506(4,0);7,503(4,6);7,490 (1,5);7,485(4,2);7,391(6,4);7,380(2,1);7,375(3,3);7,372(4,4);7,360(3,1);7,344(0,9);7,342(1,3);6,972(4,8);6,970(4,9 );6,757(0,4);6,755(0,4);6,736(0,4);6,616(0,4);3,324(30,4);2,671(0,4);2,587(15,9);2,585(16,0);2,525(0,8);2,511(20, 9);2,507(42,9);2,502(57,6);2,498(44,0);2,494(23,3);2,366(1,1);2,329(0,4);0,008(2,0);0,000(52,1);-0,008(2,8) |
| Verbindung Nr. 1-1-234: ¹H-NMR(400,0 MHz, d₆-DMSO): 11,113(3,1);9,100(2,8);9,096(2,8);8,723(11,5);8,719(3, 3);8,711(11,6);8,320(1,2);8,315(1,7);8,310(1,2);8,300(1,3);8,295(1,8);8,290(1,2);7,523(1,6);7,522(1,6);7,511(1,5); 7,510(1,6);7,503(1,5);7,502(1,5);7,491(1,4);7,490(1,5);7,317(2,7);7,305(5,0);7,292(2,5);6,508(4,4);5,893(11,7);3,3 30(28,3);2,512(11,0);2,508(21,5);2,503(28,1);2,499(20,4);2,495(10,0);2,411(16,0);0,008(0,6);0,000(14,2);-0,008(0,5) |
| Verbindung Nr. 1-1-235: ¹H-NMR(600,1 MHz, d₆-DMSO): 11,077(3,4);9,100(2,9);9,098(3,0);9,036(10,0);9,028(1 0,2);8,740(2,3);8,737(2,4);8,732(2,4);8,729(2,4);8,317(1,2);8,314(1,7);8,311(1,2);8,304(1,2);8,301(1,7);8,297(1,2) ;7,900(0,4);7,738(2,7);7,730(5,2);7,721(2,7);7,540(1,7);7,539(1,7);7,532(1,7);7,531(1,6);7,527(1,7);7,525(1,6);7,5 19(1,7);7,517(1,6);6,582(4,1);5,613(2,8);5,591(4,3);5,523(4,2);5,501(2,9);5,447(0,5);3,380(1,1);3,356(38,8);2,513 (5,2);2,510(11,1);2,507(15,3);2,504(11,2);2,501(5,3);2,377(0,7);2,313(16,0);1,059(0,4) |
| Verbindung Nr. 1-1-236: ¹H-NMR(601,6 MHz, d₆-DMSO): 11,031(3,5);9,141(9,9);9,133(10,0);9,049(2,8);9,045(2, 7);8,722(2,3);8,719(2,5);8,714(2,4);8,711(2,5);8,706(0,4);8,698(0,3);8,270(1,1);8,266(1,6);8,263(1,2);8,256(1,2);8 ,253(1,7);8,250(1,2);7,924(2,8);7,916(5,3);7,908(2,7);7,517(1,5);7,516(1,6);7,509(1,5);7,508(1,6);7,503(1,5);7,502 (1,6);7,495(1,4);7,494(1,6);6,609(3,9);6,061(11,3);5,503(0,4);3,373(0,5);3,358(2,8);3,349(346,5);3,336(0,9);3,332 (0,5);2,618(0,4);2,615(0,5);2,612(0,4);2,524(0,9);2,521(1,1);2,518(1,2);2,509(26,1);2,506(57,1);2,503(79,4);2,500 (58,7);2,497(27,7);2,391(0,4);2,388(0,5);2,385(0,4);2,360(16,0);2,276(0,5);0,000(5,5) |
| Verbindung Nr. 1-1-237: ¹H-NMR(601,6 MHz, d₆-DMSO): 11,068(3,0);9,115(2,8);9,112(2,8);8,726(2,0);8,724(2,2 );8,718(2,2);8,716(2,2);8,328(1,6);8,315(1,6);7,527(1,5);7,519(1,5);7,514(1,5);7,506(1,5);6,506(4,3);4,328(1,2);4, 316(1,2);4,303(1,4);4,291(1,4);4,065(1,6);4,054(1,7);4,040(1,4);4,029(1,4);3,347(11,7);3,344(25,7);2,507(21,0);2, 504(29,2);2,501(22,4);2,318(16,0);2,296(0,5);2,284(0,9);2,278(0,5);2,271(1,0);2,266(1,0);2,260(0,5);2,254(0,9);2, 242(0,4);1,990(0,7);1,863(1,6);1,851(1,8);1,845(1,6);1,833(1,5);1,590(1,6);1,577(2,8);1,564(1,4);1,175(0,3);0,000 (27,6) |
| Verbindung Nr. 1-1-238: ¹H-NMR(601,6 MHz, d₆-DMSO): 11,079(2,8);9,110(2,6);9,107(2,6);8,725(2,0);8,722(2,1 );8,717(2,2);8,714(2,2);8,326(0,9);8,323(1,5);8,320(1,0);8,313(1,0);8,309(1,5);8,306(1,0);7,526(1,5);7,518(1,5);7, 513(1,5);7,505(1,4);6,508(4,2);6,381(1,5);6,370(3,4);6,359(1,6);4,777(6,2);4,765(6,1);3,349(9,4);3,345(11,6);3,34 2(14,4);3,173(1,2);3,165(1,2);2,522(0,3);2,519(0,4);2,510(9,8);2,507(21,2);2,504(29,0);2,501(20,8);2,498(9,4);2,3 01(16,0);0,005(1,0);0,000(31,4);-0,006(1,0) |
| Verbindung Nr. 1-1-239: ¹H-NMR(601,6 MHz, d₆-DMSO): 11,011(2,9);9,108(2,7);9,105(2,8);8,719(1,9);8,717(2,1 );8,711(2,0);8,709(2,2);8,319(1,6);8,306(1,6);7,520(1,5);7,512(1,5);7,507(1,6);7,499(1,5);6,453(4,4);5,269(0,7);5, 258(1,4);5,246(0,7);4,600(3,2);4,589(3,1);3,352(8,7);3,348(11,0);3,343(11,2);2,507(20,9);2,504(28,7);2,501(21,6); 2,254(16,0);2,234(0,3);1,747(11,9);1,707(10,5);0,000(25,7);-0,006(0,9) |
| Verbindung Nr. 1-1-240: ¹H-NMR(400,0 MHz, d₆-DMSO): 11,417(0,4);9,618(3,9);9,616(3,9);9,613(3,3);9,445(3,2 );9,442(3,2);9,431(3,3);9,429(3,3);8,122(2,7);8,116(2,8);8,109(2,8);8,103(2,7);7,879(4,6);7,873(4,6);7,516(4,9);7, 513(4,9);6,528(6,5);6,301(16,0);6,295(7,1);6,290(4,1);3,330(12,6);2,891(0,4);2,731(0,4);2,671(0,4);2,507(44,4);2, 502(57,4);2,498(44,3);2,484(23,6);2,329(0,4);2,324(0,4);2,295(0,4);0,000(65,0) |
| Verbindung Nr. 1-1-241: ¹H-NMR(400,0 MHz, d₆-DMSO): 11,073(1,7);9,114(1,8);9,109(1,8);8,729(1,5);8,725(1,6 );8,717(1,6);8,713(1,6);8,333(0,7);8,328(1,1);8,323(0,8);8,313(0,8);8,308(1,1);8,303(0,8);7,532(1,1);7,531(1,1);7, |
| 520(1,1);7,519(1,1);7,512(1,1);7,511(1,1);7,500(1,0);7,499(1,0);6,542(2,8);5,157(8,4);4,915(0,4);3,331(21,5);2,52 1(0,4);2,512(5,6);2,508(11,9);2,503(15,9);2,499(11,7);2,494(5,8);2,320(11,3);2,143(16,0);2,100(0,8);0,000(1,2) |
| Verbindung Nr. 1-1-242: ¹H-NMR(400,0 MHz, d₆-DMSO): 11,059(2,5);9,111(2,5);9,107(2,6);8,728(2,1);8,724(2,3 );8,716(2,3);8,712(2,3);8,332(1,0);8,327(1,5);8,322(1,1);8,312(1,1);8,307(1,6);8,302(1,1);7,531(1,5);7,529(1,6);7, 519(1,5);7,517(1,6);7,511(1,5);7,509(1,6);7,499(1,4);7,497(1,5);6,538(3,9);5,286(0,4);5,194(12,0);4,953(1,4);4,51 3(0,4);3,331(31,7);2,653(1,6);2,635(5,1);2,616(5,4);2,598(2,3);2,580(0,7);2,521(0,4);2,512(7,3);2,508(15,5);2,503 (20,9);2,499(15,5);2,494(7,8);2,337(0,4);2,321(16,0);2,142(1,5);2,141(1,6);1,199(6,5);1,181(13,8);1,162(6,9);1,14 2(0,7);0,000(1,6) |
| Verbindung Nr. 1-1-243: ¹H-NMR(400,0 MHz, d₆-DMSO): 11,124(2,4);9,117(2,2);9,113(2,3);8,738(1,5);8,734(1,8 );8,726(1,7);8,722(1,7);8,336(0,9);8,331(1,3);8,327(1,0);8,316(1,0);8,311(1,4);8,307(1,0);7,540(1,3);7,528(1,3);7, 521(1,2);7,520(1,3);7,509(1,1);7,508(1,2);6,575(3,5);5,373(2,1);5,340(3,1);5,226(3,2);5,192(2,1);3,329(22,8);2,65 1(16,0);2,507(17,5);2,503(23,7);2,498(18,3);2,340(12,9);0,008(0,8);0,000(19,8) |
| Verbindung Nr. 1-1-244: ¹H-NMR(400,0 MHz, d₆-DMSO): 11,148(3,3);9,114(2,9);9,109(3,1);8,742(2,0);8,738(2,3 );8,730(2,2);8,726(2,2);8,332(1,1);8,328(1,7);8,323(1,3);8,312(1,2);8,308(1,8);8,303(1,3);7,543(1,6);7,531(1,6);7, 524(1,6);7,512(1,5);7,511(1,5);6,615(4,5);5,605(8,7);3,332(26,5);3,074(16,0);2,508(14,3);2,503(19,0);2,499(14,9); 2,352(15,9);1,990(0,8);1,175(0,4);0,008(0,6);0,000(15,6) |
| Verbindung Nr. 1-1-245: ¹H-NMR(400,0 MHz, d₆-DMSO): 11,119(3,1);9,117(2,9);9,112(2,9);8,738(2,1);8,734(2,3 );8,726(2,2);8,722(2,3);8,336(1,1);8,331(1,7);8,326(1,2);8,316(1,3);8,311(1,8);8,306(1,2);7,541(1,5);7,539(1,6);7, 527(1,6);7,521(1,5);7,520(1,6);7,509(1,4);7,507(1,5);6,572(4,3);5,367(2,6);5,333(4,0);5,321(0,3);5,227(3,9);5,193 (2,6);4,368(0,4);4,355(0,8);4,342(0,4);3,456(0,6);3,444(0,5);3,439(0,6);3,426(0,5);3,334(52,9);3,176(0,7);3,163(0, 7);2,960(1,0);2,945(0,6);2,941(1,2);2,927(1,7);2,908(1,7);2,889(0,6);2,847(0,5);2,829(1,6);2,810(1,7);2,795(1,1);2 ,792(0,8);2,777(1,0);2,525(0,4);2,512(8,5);2,508(17,3);2,503(23,1);2,499(17,6);2,339(16,0);2,168(0,9);1,253(5,4); 1,235(11,3);1,216(5,3);1,195(0,6);1,074(1,2);1,056(2,2);1,039(1,1);0,008(0,8);0,000(21,7);-0,008(1,1) |
| Verbindung Nr. 1-1-246: ¹H-NMR(400,0 MHz, d₆-DMSO): 11,129(3,1);9,110(2,8);9,105(2,9);8,741(2,1);8,737(2,3 );8,729(2,3);8,725(2,3);8,329(1,1);8,324(1,7);8,320(1,2);8,309(1,2);8,304(1,8);8,300(1,2);7,543(1,6);7,541(1,6);7, 531(1,6);7,529(1,6);7,523(1,6);7,522(1,6);7,511(1,5);7,509(1,5);6,607(4,4);5,601(9,9);4,038(0,8);4,020(0,8);3,332 (50,6);3,221(1,3);3,202(4,1);3,184(4,2);3,165(1,4);2,512(9,9);2,507(20,5);2,503(27,4);2,498(20,6);2,494(10,6);2,3 60(16,0);2,335(0,3);1,989(3,6);1,280(5,5);1,261(11,9);1,242(5,5);1,193(1,0);1,175(1,9);1,157(0,9) |
| Verbindung Nr. 1-1-247: ¹H-NMR(400,0 MHz, d₆-DMSO): 11,009(3,3);9,105(2,8);9,100(2,8);8,726(1,9);8,722(2,1 );8,714(2,1);8,710(2,1);8,322(1,1);8,317(1,7);8,312(1,2);8,302(1,2);8,297(1,7);8,292(1,2);7,528(1,6);7,516(1,6);7, 508(1,5);7,496(1,5);7,466(1,5);7,457(1,7);7,453(1,5);7,443(2,0);7,434(0,5);7,368(0,9);7,359(1,3);7,355(1,3);7,353 (1,2);7,344(2,8);7,336(1,8);7,327(5,2);7,318(3,6);7,312(3,3);7,303(2,6);7,294(0,4);6,521(4,3);5,174(9,7);4,017(10, 3);3,331(29,9);2,525(0,5);2,511(10,4);2,507(21,1);2,503(28,1);2,498(21,0);2,177(16,0) |
| Verbindung Nr. 1-1-248: ¹H-NMR(400,0 MHz, d₆-DMSO): 11,921(1,3);9,698(1,1);9,696(1,1);9,693(0,9);9,483(0,9 );9,481(0,9);9,470(0,9);9,467(1,0);8,211(0,8);8,205(0,8);8,197(0,8);8,191(0,8);7,226(2,1);6,990(0,4);6,844(1,7);5, 057(0,3);3,338(421,5);3,264(0,4);3,249(1,0);2,668(6,5);2,560(0,3);2,542(32,4);2,507(87,9);2,502(117,2);2,498(88, 7);2,474(16,0);2,443(1,0);2,410(1,5);2,390(2,9);2,334(0,6);2,329(0,8);2,325(0,6);1,235(0,7);0,000(0,9) |
| Verbindung Nr. 1-1-249: ¹H-NMR(400,0 MHz, d₆-DMSO): 13,185(2,1);9,661(2,0);9,658(2,5);9,655(2,5);9,652(2,2 );9,571(1,7);9,568(1,8);9,558(1,8);9,555(1,9);8,647(1,5);8,637(1,5);8,635(1,5);8,134(1,8);8,128(1,9);8,121(1,9);8, 115(1,8);8,093(0,8);8,089(0,8);8,072(1,5);8,068(1,4);8,054(1,2);8,049(1,2);7,986(2,5);7,965(1,6);7,412(1,2);7,399 (1,4);7,396(1,3);7,383(1,1);7,381(1,1);6,801(5,0);3,449(0,4);3,333(745,9);2,676(1,5);2,671(2,0);2,667(1,6);2,541( 5,8);2,511(120,4);2,507(232,3);2,502(306,6);2,498(235,3);2,333(1,5);2,329(2,0);2,324(1,6);2,295(16,0);2,074(0,9) ;1,258(0,4);1,234(1,4);0,000(36,1);-0,008(2,3) |
| Verbindung Nr. 1-1-250: ¹H-NMR(400,0 MHz, d₆-DMSO): 11,512(3,1);9,641(2,4);9,638(2,9);9,635(2,9);9,632(2,4 );9,457(2,5);9,454(2,5);9,443(2,6);9,441(2,5);8,145(2,2);8,139(2,2);8,132(2,2);8,126(2,1);6,615(4,8);5,054(1,1);5, 031(3,5);5,008(3,7);4,985(1,3);3,336(117,8);3,250(1,3);3,110(2,4);2,676(0,7);2,672(0,6);2,542(32,6);2,507(51,3);2 ,503(66,5);2,498(49,6);2,330(16,0);1,235(0,3);0,000(7,2) |
| Verbindung Nr. 1-1-251: ¹H-NMR(400,0 MHz, d₆-DMSO): 11,360(1,4);9,630(0,9);9,627(1,1);9,624(1,1);9,621(1,0 );9,439(0,9);9,436(1,0);9,425(1,0);9,423(1,0);8,123(0,9);8,118(0,9);8,110(0,9);8,104(0,9);7,173(2,0);6,532(2,0);5, 312(3,9);3,337(12,8);3,251(0,4);3,122(0,9);2,543(12,7);2,512(4,3);2,508(9,0);2,503(12,2);2,499(9,2);2,494(4,8);2, 373(16,0);2,344(6,4);0,000(2,6) |
| Verbindung Nr. 1-1-252: ¹H-NMR(400,0 MHz, d₆-DMSO): 11,352(1,2);9,632(0,8);9,629(1,0);9,627(1,0);9,624(0,9 );9,441(0,8);9,438(0,9);9,428(0,9);9,425(0,9);8,126(0,8);8,120(0,8);8,112(0,8);8,107(0,8);6,550(1,7);6,160(2,8);5, 244(3,3);3,814(16,0);3,342(41,5);2,543(20,1);2,521(0,4);2,512(4,5);2,508(9,4);2,504(12,8);2,499(9,6);2,495(5,0);2 ,378(5,3) |
| Verbindung Nr. 1-1-253: ¹H-NMR(400,0 MHz, d₆-DMSO): 11,354(2,6);9,629(1,7);9,626(2,2);9,623(2,2);9,620(2,0 );9,439(1,8);9,436(1,9);9,425(1,9);9,422(2,0);8,607(3,1);8,594(3,2);8,122(1,7);8,116(1,8);8,109(1,8);8,103(1,7);7, 314(2,4);7,302(2,3);6,537(3,8);5,374(7,5);3,349(58,8);3,116(1,1);2,544(10,2);2,527(0,3);2,513(6,7);2,509(13,9);2, 504(18,9);2,500(14,3);2,495(7,4);2,444(16,0);2,331(12,1) |
| Verbindung Nr. 1-1-254: ¹H-NMR(400,0 MHz, d₆-DMSO): 11,335(4,0);9,613(3,9);9,610(4,0);9,608(3,4);9,427(3,1 );9,424(2,0);9,413(3,2);9,411(1,9);8,479(3,0);8,475(2,1);8,467(3,1);8,464(1,9);8,112(1,9);8,109(2,5);8,103(2,2);8, 098(2,1);8,096(2,6);8,090(1,9);7,979(2,9);7,975(1,8);7,961(2,4);7,958(3,2);7,955(1,8);7,417(1,7);7,414(1,7);7,405 (1,8);7,402(1,9);7,396(1,8);7,394(1,7);7,385(1,7);7,382(1,6);6,539(5,4);5,460(10,2);5,383(0,5);3,345(157,6);3,310 (0,5);3,253(0,5);3,250(0,4);2,546(32,5);2,543(33,8);2,529(1,1);2,507(37,0);2,503(36,8);2,351(16,0);2,280(0,7);0,0 03(0,8);0,000(0,9) |
| Verbindung Nr. 1-1-255: ¹H-NMR(400,0 MHz, d₆-DMSO): 11,473(2,6);9,613(2,2);9,444(1,8);9,431(1,9);8,127(2,1 );8,119(1,8);8,113(1,6);8,106(1,5);8,100(1,5);6,796(2,3);6,790(2,5);6,569(3,5);6,430(7,1);3,337(776,7);2,671(1,5); 2,541(4,0);2,506(169,9);2,502(226,5);2,498(177,7);2,479(16,0);2,329(1,4);2,074(0,7);1,235(1,4);0,000(8,4) |
| Verbindung Nr. 1-1-256: ¹H-NMR(400,0 MHz, d₆-DMSO): 11,428(3,2);9,618(2,8);9,615(2,7);9,446(2,2);9,444(2,2 );9,433(2,4);9,431 (2,2);8,121(1,9);8,115(2,0);8,108(2,0);8,102(2,0);7,604(4,2);7,310(4,9);6,513(4,5);6,203(9,7);3, 339(569,5);3,249(0,4);3,100(1,0);2,676(1,0);2,671(1,3);2,667(1,0);2,542(25,8);2,507(143,0);2,502(185,9);2,498(1 42,6);2,462(16,0);2,333(1,0);2,329(1,2);2,325(1,0);2,074(0,5);1,989(15,9);1,235(1,1);0,000(7,9) |
| Verbindung Nr. 1-1-257: ¹H-NMR(400,0 MHz, d₆-DMSO): 9,116(0,4);9,082(2,5);9,077(2,7);8,775(2,2);8,763(2,3) ;8,316(0,4);8,307(1,6);8,273(1,5);8,253(1,6);7,588(1,4);7,576(1,6);7,567(1,7);7,556(1,6);5,756(1,1);3,371(0,7);3,3 37(0,7);3,267(0,5);3,169(3,0);3,072(1,3);2,621(0,4);2,506(32,6);2,502(43,4);2,451(16,0);2,432(0,9);2,419(0,7);2,3 96(15,2);2,365(0,4);2,330(0,4);2,315(0,5);2,234(1,1);2,223(3,6);0,008(1,7);0,000(38,4) |
| Verbindung Nr. 1-1-258: ¹H-NMR(400,0 MHz, d₆-DMSO): 10,311(4,3);9,082(3,9);9,078(3,9);8,794(2,5);8,790(2,8 );8,782(2,7);8,778(2,7);8,287(1,5);8,282(2,3);8,278(1,7);8,267(1,7);8,262(2,4);8,258(1,7);7,611(2,0);7,598(2,1);7, 591(2,1);7,579(1,9);6,988(0,8);3,635(0,3);3,616(0,4);3,399(3,7);3,186(0,3);2,891(1,8);2,767(2,1);2,750(4,9);2,733 (6,0);2,717(2,2);2,507(28,2);2,503(37,2);2,499(29,1);2,398(16,0);2,244(1,4);1,478(0,5);1,459(1,0);1,453(0,6);1,44 1(1,2);1,434(2,3);1,416(4,6);1,398(4,6);1,380(2,4);1,362(0,6);0,875(1,4);0,856(2,9);0,836(7,3);0,817(13,4);0,798( 6,1);0,007(2,3);0,000(42,1);-0,025(0,4) |
| Verbindung Nr. 1-1-259: ¹H-NMR(400,0 MHz, d₆-DMSO): 11,622(12,5);9,149(10,9);9,144(10,6);9,111(0,4);9,106 (0,4);8,771(1,3);8,763(8,1);8,759(8,3);8,751(7,9);8,747(7,3);8,374(4,8);8,369(6,5);8,364(4,7);8,354(5,1);8,349(6,6 );8,344(4,2);8,317(0,4);7,569(0,7);7,558(6,2);7,546(6,0);7,538(6,0);7,526(5,3);6,942(15,1);6,666(0,5);5,114(5,1);5 ,105(2,3);5,092(15,5);5,083(2,7);5,070(16,0);5,047(5,5);3,340(8,7);3,326(86,2);3,177(0,4);3,164(0,3);2,677(0,6);2, 673(0,8);2,553(7,4);2,542(51,5);2,512(51,7);2,508(83,6);2,504(100,8);2,499(72,1);2,376(0,3);2,335(0,6);2,330(0,7 );2,326(0,5);2,256(1,4);2,236(1,8);0,146(0,5);0,013(9,2);0,008(8,7);0,000(100,6);-0,008(4,3);-0,150(0,5) |
| Verbindung Nr. 1-1-260: ¹H-NMR(400,0 MHz, d₆-DMSO): 10,978(2,7);9,102(2,8);9,098(2,8);8,727(1,8);8,723(2,2 );8,715(2,2);8,711(2,3);8,319(1,2);8,314(1,8);8,309(1,4);8,299(1,3);8,294(1,8);8,289(1,3);7,530(1,6);7,518(1,7);7, 510(1,7);7,498(1,6);6,502(3,9);6,463(0,4);6,399(0,4);5,756(16,0);5,343(9,5);5,264(1,0);5,255(0,4);5,163(0,4);4,94 9(0,5);3,625(0,4);3,607(0,4);3,325(17,0);2,671(0,6);2,667(0,3);2,541(0,4);2,524(0,7);2,511(22,3);2,507(46,8);2,50 2(63,7);2,497(48,0);2,493(24,7);2,333(0,4);2,329(0,5);2,324(0,4);2,295(0,5);2,235(0,3);2,158(14,5);2,114(0,6);2,0 98(0,6);2,087(0,3);2,071(1,0);2,061(0,3);2,050(1,0);2,039(0,5);2,013(1,4);1,951(0,4);1,797(1,5);1,782(3,6);1,774( 3,9);1,762(2,3);1,596(1,9);1,584(3,7);1,575(3,9);1,561(1,5);1,551(0,3);1,368(0,4);1,360(0,4);1,314(0,5);1,307(0,3) ;1,278(0,4);1,214(0,5);1,196(0,8);1,178(0,4);1,156(0,4);1,128(0,3);1,121(0,4);0,932(0,4);0,921(0,3);0,912(0,3);0,8 88(0,4);0,146(0,4);0,008(2,6);0,000(78,1);-0,008(3,7);-0,150(0,4) |
| Verbindung Nr. 1-1-261: ¹H-NMR(601,6 MHz, d₆-DMSO): 11,470(3,4);9,130(3,3);9,127(3,3);8,764(2,3);8,761(2,4 );8,756(2,4);8,753(2,4);8,347(1,3);8,344(2,0);8,341(1,4);8,334(1,4);8,331(2,0);8,327(1,4);7,558(1,8);7,550(1,7);7, 546(1,8);7,545(1,7);7,538(1,7);7,537(1,7);6,884(4,3);6,883(4,3);4,777(13,9);3,412(27,6);3,339(18,3);2,571(16,0);2 ,570(16,0);2,508(10,2);2,505(13,8);2,502(10,3);2,237(0,4) |
| Verbindung Nr. 1-1-262: ¹H-NMR(400,0 MHz, d₆-DMSO): 10,927(0,4);9,108(0,7);9,103(0,7);8,793(0,5);8,781(0,5 );8,733(8,4);8,720(9,0);8,710(0,9);8,519(1,9);8,515(2,4);8,510(3,2);8,507(4,3);8,504(4,2);8,318(0,6);8,305(0,4);8, 300(0,5);8,295(0,4);7,700(1,0);7,695(1,5);7,690(1,1);7,680(1,1);7,675(1,7);7,670(1,2);7,532(0,4);7,519(0,4);7,512 (0,4);7,500(0,4);7,429(2,0);7,417(3,7);7,405(1,9);7,288(1,4);7,276(1,4);7,270(1,3);7,257(1,2);6,420(0,4);5,988(3,1 );5,426(0,5);5,370(9,7);4,809(12,0);3,331(203,8);3,175(0,9);3,162(0,8);2,677(0,4);2,672(0,6);2,668(0,5);2,542(0,3 );2,526(1,2);2,521(2,0);2,512(31,9);2,508(67,3);2,503(92,0);2,499(69,9);2,494(36,3);2,334(0,5);2,330(0,7);2,325(0 ,6);2,321(1,1);2,235(3,3);2,201(16,0);1,235(0,5);0,146(0,4);0,008(2,7);0,000(82,5);-0,008(4,0);-0,150(0,4) |
| Verbindung Nr. 1-1-263: ¹H-NMR(400,0 MHz, d₆-DMSO): 11,423(4,6);9,145(3,4);9,140(3,6);8,775(2,4);8,771(2,7 );8,763(2,6);8,759(2,7);8,367(1,3);8,362(2,0);8,357(1,5);8,347(1,5);8,342(2,2);8,337(1,5);8,114(4,6);8,096(5,5);8, 093(4,2);7,827(0,9);7,813(0,7);7,808(2,8);7,790(2,2);7,737(3,8);7,717(5,4);7,699(2,2);7,574(1,8);7,562(1,8);7,555 (1,8);7,542(1,7);6,848(4,8);6,846(5,0);3,384(23,4);3,327(28,6);3,176(0,9);3,163(0,9);2,672(0,3);2,525(0,8);2,512( 17,9);2,507(36,8);2,503(49,6);2,498(38,2);2,494(20,3);2,405(16,0);2,366(0,3);2,329(0,3);0,008(1,4);0,000(37,5);-0,008(2,0) |
| Verbindung Nr. 1-1-264: ¹H-NMR(400,0 MHz, d₆-DMSO): 8,793(0,4);8,780(0,4);8,733(8,2);8,721(8,4);8,462(3,2) ;8,456(3,2);7,631(1,0);7,612(1,0);7,425(2,1);7,413(4,0);7,400(2,0);7,241(0,9);7,229(1,1);7,223(1,0);7,210(0,8);5,9 61(1,0);5,757(9,3);5,427(0,5);5,340(8,9);4,518(6,3);4,512(6,6);3,328(24,1);3,184(1,9);3,178(4,4);3,172(2,0);2,891 (0,7);2,732(0,6);2,525(0,5);2,511(11,8);2,507(24,4);2,503(33,0);2,498(25,3);2,494(13,3);2,321(0,8);2,197(16,0);1, |
| 989(0,8);1,175(0,4);0,008(0,9);0,000(24,8);-0,008(1,3) |
| Verbindung Nr. 1-1-265: ¹H-NMR(400,0 MHz, d₆-DMSO): 8,780(0,3);8,735(7,8);8,723(8,0);8,478(1,6);8,474(1,8) ;8,462(3,7);8,456(2,8);7,627(1,2);7,607(1,4);7,427(1,9);7,415(3,7);7,402(1,9);7,252(1,1);7,240(1,2);7,233(1,2);7,2 21(1,0);6,417(0,4);6,406(0,8);6,396(0,4);6,277(0,7);6,267(1,7);6,256(0,8);6,138(0,4);6,127(0,8);6,116(0,4);5,905( 1,4);5,756(0,5);5,425(0,4);5,337(9,4);4,182(1,1);4,172(1,3);4,146(2,5);4,136(2,5);4,110(1,3);4,100(1,2);3,323(9,1) ;2,891(0,9);2,731(0,8);2,671(0,4);2,541(1,3);2,524(0,8);2,510(23,7);2,506(48,8);2,502(66,2);2,497(50,2);2,333(0,4 );2,328(0,5);2,320(0,8);2,299(0,4);2,176(16,0);0,146(0,4);0,008(3,2);0,000(86,2);-0,008(3,9);-0,150(0,4) |
| Verbindung Nr. 1-1-266: ¹H-NMR(600,1 MHz, d₆-DMSO): 11,411(1,9);9,138(1,6);9,135(1,7);8,766(1,2);8,763(1,3 );8,758(1,3);8,755(1,2);8,354(0,7);8,31(1,0);8,348(0,7);8,341(0,7);8,338(1,0);8,334(0,7);7,561(0,9);7,560(0,9);7, 553(0,9);7,552(0,9);7,547(0,9);7,546(0,9);7,539(0,9);7,538(0,8);6,872(2,2);6,871(2,2);3,389(1,7);3,377(3,5);3,365 (1,9);3,326(5,0);2,836(1,9);2,824(3,8);2,811(1,8);2,553(8,2);2,552(7,9);2,510(2,4);2,508(5,1);2,505(7,0);2,502(5,1 );2,499(2,5);2,103(16,0) |
| Verbindung Nr. 1-1-267: ¹H-NMR(400,0 MHz, d₆-DMSO): 10,642(1,8);9,114(0,8);8,817(6,9);8,804(7,1);8,786(0,7 );8,759(0,8);8,315(1,3);8,295(1,1);8,171(0,8);8,063(2,8);8,052(2,8);7,563(1,1);7,551(1,2);7,543(1,1);7,531(0,9);7, 487(0,4);7,481(1,8);7,475(0,7);7,469(3,3);7,457(1,6);5,527(1,0);5,455(8,1);5,284(4,8);3,427(0,3);3,406(0,5);3,382 (2,1);3,365(3,2);3,345(2,8);3,319(0,7);3,169(3,0);3,111(2,0);3,092(2,4);3,074(1,6);2,671(0,4);2,667(0,3);2,525(1,0 );2,511(27,6);2,507(57,8);2,502(77,9);2,498(58,6);2,494(30,3);2,333(0,4);2,329(0,7);2,325(0,4);2,075(16,0);0,907( 0,5);0,888(1,0);0,870(0,5);0,146(0,4);0,008(3,3);0,000(103,3);-0,009(5,0);-0,150(0,5) |
| Verbindung Nr. 1-1-268: ¹H-NMR(400,0 MHz, d₆-DMSO): 11,473(3,0);9,148(0,7);8,768(0,7);8,366(0,8);8,361(1,3 );8,357(0,9);8,346(0,9);8,341(1,4);8,337(0,9);7,570(0,9);7,558(1,0);7,550(1,0);7,538(0,9);6,904(3,3);6,902(3,4);3, 637(0,6);3,603(0,7);3,597(0,8);3,588(1,0);3,581(2,4);3,577(1,9);3,562(2,9);3,534(2,7);3,518(2,2);3,510(1,0);3,501 (0,7);3,495(0,6);3,325(12,2);3,063(16,0);3,010(0,5);2,557(11,6);2,525(0,5);2,511(10,3);2,507(20,9);2,503(27,9);2, 498(20,9);2,494(10,6);0,000(3,4) |
| Verbindung Nr. 1-1-269: ¹H-NMR(600,1 MHz, d₆-DMSO): 11,510(4,0);9,122(4,1);9,119(4,2);8,837(13,5);8,829(1 3,7);8,753(3,1);8,750(3,2);8,745(3,3);8,742(3,2);8,335(1,7);8,332(2,5);8,329(1,7);8,322(1,9);8,319(2,5);8,316(1,7) ;7,550(2,3);7,549(2,2);7,542(2,3);7,541(2,2);7,536(2,3);7,535(2,2);7,528(2,2);7,527(2,1);7,512(2,9);7,504(5,7);7,4 96(2,8);7,476(12,5);5,757(1,2);5,721(16,0);3,325(68,2);3,174(1,3);3,165(1,3);2,524(0,4);2,520(0,5);2,517(0,5);2,5 09(15,6);2,506(33,7);2,503(46,4);2,500(33,5);2,497(15,5) |
| Verbindung Nr. 1-1-270: ¹H-NMR(400,0 MHz, d₆-DMSO): 11,252(2,0);9,137(1,9);9,132(1,9);8,803(1,3);8,799(1,4 );8,791(1,4);8,787(1,4);8,347(0,8);8,342(1,2);8,337(0,8);8,327(0,8);8,322(1,2);8,317(0,8);7,954(1,4);7,600(1,0);7, 587(1,1);7,580(1,0);7,568(1,0);5,757(0,7);3,903(14,0);3,327(4,8);2,892(10,4);2,732(8,8);2,580(16,0);2,512(5,0);2, 508(9,6);2,503(12,6);2,499(9,4);0,000(1,1) |
| Verbindung Nr. I-1-271: Masse (m/z): 295,1 (M+H)⁺;¹H-NMR (DMSO-d6) 3.75 (s, 3H), 5.67 (d, 1H), 6.95 (m, 2H), 7.56 (m, 4H), 8.00 (d, 1H), 8.36 (d, 1H), 11.35 (s, 1H). |
| Verbindung Nr. I-1-272: Masse (m/z): 351,1 (M+H)⁺;¹H-NMR (DMSO-d6) 1.24 (t, 3H), 4.16 (q, 2H), 5.34 (s, 2H), 7.1 - 7.4 (m, 5H), 7.49 (s, 1H), 7.55 (m, 1H), 8.26 (m, 1H), 8.75 (m, 1H), 9.07 (m, 1H), 10.48 (s, 1H). |
| Verbindung Nr. I-1-273: Masse (m/z): 272,1 (M+H)⁺;¹H-NMR (DMSO-d6) 7.06 (d, 1H), 7.55 (d, 1H) 7.57 (m, 1H), 7.64 (d, 1H), 8.37 (m, 1H), 8.49 (d, 1H), 8.76 (m, 1H) 9.17 (m, 1H), 11.56 (s, 1H). |
| Verbindung Nr. I-1-274: Masse (m/z): 299,1/301,0 (M+H)⁺;¹H-NMR (DMSO-d6) 7.02 (s, 1H), 7.45 - 7.55 (m, 6H), 8.35 (m, 1H), 8.76 (m, 1H), 9.15 (m, 1H), 11.49 (s, 1H). |
| Verbindung Nr. I-1-275: Masse (m/z): 303,0/305,0 (M+H)⁺;¹H-NMR (DMSO-d6) 3.73 (s, 3H), 7.37 (t, 1H), 7.58 (m,1H), 8.30 (m, 1H), 8.79 (m, 1H), 9.11 (1H), 10.61 (s, 1H). |
| Verbindung Nr. 1-1-276: ¹H-NMR(400,0 MHz, d₆-DMSO): 11,105(1,0);9,037(1,2);9,032(1,1);8,793(2,7);8,785(1,5 );8,780(3,5);8,424(0,8);8,419(1,1);8,414(0,6);7,954(2,3);7,455(0,6);7,443(1,2);7,430(0,6);6,525(1,5);5,430(3,2);3, 330(19,2);2,892(16,0);2,733(14,4);2,512(4,5);2,508(7,9);2,504(9,5);2,499(6,5);2,323(5,4);0,000(2,5) |
| Verbindung Nr. 1-1-277: ¹H-NMR(601,6 MHz, DMF): 11,525(8,1);10,468(0,4);9,332(10,9);9,331(11,5);9,328(11, 7);9,327(10,6);9,155(1,3);9,154(1,4);9,152(1,4);9,151(1,3);8,862(1,0);8,860(1,1);8,855(1,1);8,852(1,0);8,828(8,3); 8,826(8,8);8,820(8,7);8,818(8,6);8,531(5,3);8,528(7,1);8,524(5,2);8,517(5,5);8,515(7,2);8,514(7,2);8,511(5,3);8,34 0(0,6);8,337(0,9);8,336(0,8);8,333(0,7);8,327(0,7);8,324(0,9);8,323(0,9);8,320(0,7);8,027(8,9);7,671(0,8);7,670(0, 8);7,663(0,8);7,662(0,8);7,658(0,8);7,657(0,8);7,650(0,7);7,649(0,7);7,627(6,3);7,626(6,2);7,619(6,3);7,618(6,3);7 ,614(6,4);7,612(6,2);7,606(6,1);7,604(5,9);6,925(16,0);6,923(15,8);6,659(3,3);5,817(0,8);4,071(0,8);4,059(0,8);3,6 69(0,5);3,622(12,5);3,555(97,7);3,513(0,7);3,487(49,6);3,436(0,5);3,232(0,6);2,953(7,6);2,931(0,4);2,922(4,1);2,9 19(8,3);2,916(11,4);2,913(8,1);2,910(4,0);2,785(7,3);2,752(4,5);2,749(8,9);2,745(12,5);2,742(8,6);2,739(4,4);2,67 4(0,4);2,673(0,4);2,568(63,2);2,566(62,3);2,505(0,4);2,504(0,4);2,457(0,4);2,456(0,4);2,295(11,3);2,014(3,7);1,41 8(0,3);1,380(0,6);1,297(0,9);1,278(0,9);1,211(1,1);1,200(2,0);1,188(1,1);0,005(0,4);0,000(9,9);-0,006(0,3) |
| Verbindung Nr. 1-1-278: ¹H-NMR(400,0 MHz, d₆-DMSO): 11,587(5,0);9,146(4,3);9,141(4,3);8,764(2,9);8,760(3,1 );8,752(3,1);8,748(3,0);8,367(1,8);8,363(2,6);8,357(1,8);8,347(2,0);8,343(2,7);8,337(1,8);7,560(2,4);7,548(2,4);7, 540(2,3);7,528(2,2);6,887(6,5);4,039(1,0);4,021(1,0);4,003(0,3);3,656(2,2);3,637(7,0);3,619(7,1);3,601(2,3);3,329 (14,3);2,509(36,2);2,505(24,0);2,500(16,5);2,264(0,3);1,990(4,2);1,194(1,1);1,176(2,3);1,159(8,3);1,141(16,0);1,1 |
| 22(7,2);0,008(1,8);0,000(30,7);-0,008(1,2) |
| Verbindung Nr. I-1-279: ¹H-NMR(600,1 MHz, d₆-DMSO): 3,502(16,0);2,667(0,4);2,666(0,4) |
| Verbindung Nr. 1-1-280: ¹H-NMR(400,0 MHz, d₆-DMSO): 11,578(3,9);10,750(0,3);9,129(3,3);9,124(3,2);9,096(3, 5);9,091(3,6);8,958(2,4);8,954(2,5);8,946(2,5);8,942(2,5);8,748(2,2);8,744(2,4);8,736(2,4);8,732(2,3);8,373(1,4);8 ,368(1,9);8,363(1,6);8,353(1,6);8,347(2,0);8,343(1,6);8,320(1,4);8,315(2,1);8,310(1,5);8,300(1,5);8,295(2,1);8,290 (1,4);7,748(1,8);7,736(1,9);7,729(1,9);7,717(1,8);7,536(1,8);7,524(1,8);7,517(1,7);7,504(1,6);6,908(4,8);6,907(4,8 );6,524(0,6);3,332(17,0);2,611(16,0);2,510(18,4);2,506(23,3);2,502(17,3);2,177(1,7);1,991(1,3);1,194(0,3);1,176(0 ,7);1,158(0,3);0,000(29,0) |
| Verbindung Nr. 1-1-281: ¹H-NMR(400,0 MHz, d₆-DMSO): 11,079(0,9);9,098(0,8);8,724(0,5);8,712(0,5);8,316(0,4 );8,312(0,5);8,296(0,4);8,292(0,5);7,525(0,5);7,513(0,5);7,506(0,4);7,493(0,4);6,563(1,2);5,535(2,8);3,327(8,6);2, 507(10,6);2,503(12,3);2,365(4,4);1,338(1,7);1,324(16,0);1,296(0,4);1,253(0,6);1,152(0,6);1,124(0,3);1,099(0,9);0, 000(7,6) |
| Verbindung Nr. 1-1-282: ¹H-NMR(400,0 MHz, d₆-DMSO): 11,046(2,1);9,098(2,1);9,094(2,1);8,722(1,6);8,718(1,5 );8,710(1,6);8,706(1,4);8,317(0,9);8,312(1,2);8,307(0,8);8,297(0,9);8,292(1,2);8,287(0,8);7,523(1,1);7,511(1,1);7, 504(1,1);7,492(1,0);6,530(3,2);5,358(7,5);5,234(0,4);5,025(0,4);3,334(13,6);2,572(16,0);2,543(7,9);2,508(11,9);2, 503(14,5);2,499(10,3);2,362(11,3);2,220(0,5);0,000(5,8) |
| Verbindung Nr. 1-1-283: ¹H-NMR(400,0 MHz, d₆-DMSO): 11,054(2,6);9,104(2,5);9,100(2,5);8,724(1,9);8,720(2,0 );8,712(2,0);8,708(1,9);8,322(1,0);8,318(1,5);8,313(1,0);8,302(1,1);8,298(1,5);8,293(1,0);7,526(1,5);7,514(1,5);7, 506(1,5);7,494(1,4);6,524(3,9);6,042(3,9);5,757(2,1);5,251(10,5);3,329(17,7);2,525(0,6);2,507(19,0);2,503(24,8);2 ,498(17,9);2,411(0,4);2,405(0,5);2,388(0,4);2,366(16,0);2,344(1,7);2,329(0,9);2,314(15,5);2,283(0,4);2,274(0,5);2, 216(0,4);2,144(0,8);0,000(0,5) |
| Verbindung Nr. 1-1-284: ¹H-NMR(600,1 MHz, d₆-DMSO): 11,061(2,2);9,107(2,1);9,104(2,1);8,727(1,5);8,725(1,6 );8,719(1,5);8,717(1,5);8,319(0,8);8,317(1,2);8,314(0,9);8,306(0,9);8,303(1,2);8,300(0,8);7,524(1,1);7,516(1,2);7, 511(1,2);7,503(1,1);6,575(3,0);5,759(1,2);5,628(8,0);3,337(5,9);2,509(3,6);2,506(4,7);2,503(3,6);2,350(11,9);2,33 7(16,0) |
| Verbindung Nr. 1-1-285: ¹H-NMR(400,0 MHz, d₆-DMSO): 11,062(1,0);9,096(1,0);8,720(0,7);8,708(0,7);8,314(0,5 );8,294(0,5);7,523(0,5);7,510(0,5);7,503(0,5);7,491(0,4);6,538(1,4);5,377(2,9);3,325(4,8);2,503(10,6);2,501(10,1); 2,362(4,6);2,145(0,3);1,362(16,0);1,305(1,2);1,069(0,5);1,068(0,5) |
| Verbindung Nr. 1-1-286: ¹H-NMR(600,1 MHz, CD3CN): 9,325(0,7);9,045(1,3);9,042(1,3);8,696(1,0);8,694(1,0);8 ,688(1,0);8,686(1,0);8,200(0,5);8,197(0,8);8,194(0,5);8,187(0,6);8,184(0,8);8,180(0,5);7,437(0,7);7,436(0,7);7,429 (0,8);7,428(0,7);7,424(0,8);7,423(0,7);7,416(0,7);7,415(0,7);6,583(1,6);5,547(0,5);5,364(8,1);3,140(0,8);3,098(16, 0);3,063(0,8);3,039(15,1);2,508(7,9);2,3553(9,4);2,3545(9,0);2,269(0,4);2,256(0,5);2,246(0,3);1,951(0,9);1,947(1, 5);1,943(2,2);1,939(1,5);1,935(0,8) |
| Verbindung Nr. I-1-287: Masse (m/z): 288,1 (M+H)⁺;¹H-NMR (DMSO-d6) 1.00 (dd, 6 H), 2.53 (s, 3H), 3.63 (m, 1 H), 5.48 (d, 1H), 6.72 (s, 1H), 7.56 (dd, 1H), 8.76 (m, 1H), 9.13 (m, 1H), 11.25 (s, 1H). |
| Verbindung Nr. 1-1-288: ¹H-NMR(400,0 MHz, d₆-DMSO): 11,189(2,0);9,121(4,4);9,117(4,3);8,765(3,0);8,761(3,2 );8,753(3,1);8,749(3,0);8,335(1,7);8,330(2,5);8,325(1,7);8,315(2,7);8,310(2,7);8,305(1,7);7,841(1,1);7,826(2,0);7, 812(1,1);7,568(2,3);7,556(2,3);7,548(2,2);7,536(2,1);6,698(6,2);3,337(231,3);3,311(2,3);3,293(4,0);3,277(4,5);3,2 75(4,5);3,260(3,8);3,242(1,2);2,676(0,4);2,672(0,5);2,668(0,4);2,531(22,9);2,507(57,4);2,503(73,7);2,499(53,6);2, 334(0,4);2,330(0,5);2,325(0,4);2,075(1,1);1,150(7,6);1,133(16,0);1,115(7,3);0,008(2,5);0,000(52,4);-0,008(2,1) |
| Verbindung Nr. 1-1-290: ¹H-NMR(400,0 MHz, d₆-DMSO): 10,968(2,7);9,032(2,5);9,028(2,5);8,806(1,8);8,802(1,9 );8,794(1,9);8,790(1,8);8,230(1,0);8,225(1,4);8,220(1,0);8,210(1,1);8,205(1,5);8,200(1,0);7,622(1,3);7,620(1,3);7, 609(1,3);7,608(1,3);7,602(1,3);7,600(1,2);7,590(1,2);7,588(1,2);6,389(5,5);3,445(1,7);3,338(30,2);2,543(19,8);2,5 13(6,4);2,508(12,6);2,504(16,2);2,499(11,6);2,495(5,6);2,209(16,0);1,195(4,5);0,000(4,4) |
| Verbindung Nr. 1-1-291: ¹H-NMR(400,0 MHz, d₆-DMSO): 11,203(1,5);9,120(1,2);8,750(0,9);8,741(0,9);8,341(0,6 );8,337(0,8);8,332(0,6);8,321(0,6);8,317(0,9);8,312(0,6);7,554(0,8);7,542(0,8);7,535(0,8);7,522(0,7);6,682(2,1);3, 329(7,9);3,021(16,0);2,543(0,9);2,533(0,5);2,508(8,8);2,504(11,2);2,499(8,7);2,365(7,7);0,000(8,4) |
| Verbindung Nr. 1-1-292: ¹H-NMR(400,0 MHz, d₆-DMSO): 11,157(4,6);9,113(4,4);9,109(4,4);9,107(4,1);8,766(3,4 );8,761(3,6);8,754(3,6);8,749(3,5);8,326(1,9);8,321(2,6);8,316(2,0);8,306(2,1);8,301(2,8);8,296(2,0);7,774(1,7);7, 763(1,6);7,570(2,5);7,568(2,4);7,558(2,4);7,556(2,4);7,550(2,4);7,548(2,3);7,538(2,3);7,536(2,3);6,689(6,7);6,687 (6,5);3,334(21,5);2,817(16,0);2,805(15,8);2,531(23,3);2,530(23,3);2,512(11,4);2,508(22,9);2,503(30,0);2,499(21,5 );2,494(10,2);0,008(1,8);0,000(45,9);-0,009(1,5) |
| Verbindung Nr. 1-1-293: ¹H-NMR(600,1 MHz, CD3CN): 9,143(1,1);9,044(2,8);8,708(2,1);8,701(2,1);8,197(1,6);8 ,184(1,6);7,453(1,4);7,445(1,6);7,440(1,5);7,432(1,3);6,949(3,9);6,846(3,6);6,497(3,1);5,211(9,4);3,810(3,0);3,691 (16,0);3,276(8,7);2,351(15,7);2,300(1,9);2,271(1,1);2,243(0,4);1,964(0,6);1,956(1,6);1,952(2,0);1,948(10,7);1,944 (18,5);1,939(27,1);1,935(18,5);1,931(9,5);0,000(0,4) |
| Verbindung Nr. 1-1-294: ¹H-NMR(400,0 MHz, d₆-DMSO): 11,239(3,3);9,133(3,0);9,129(3,0);8,789(2,1);8,779(2,1 );8,777(2,0);8,746(2,3);8,742(2,4);8,734(2,4);8,729(2,4);8,354(1,3);8,350(1,8);8,345(1,3);8,335(1,4);8,329(1,8);8, 325(1,3);8,077(2,3);8,073(2,3);8,057(2,7);8,053(2,5);7,733(2,6);7,721(2,5);7,713(2,4);7,701(2,3);7,550(1,7);7,548 |
| (1,7);7,538(1,7);7,536(1,6);7,530(1,6);7,528(1,6);7,518(1,6);7,516(1,6);6,789(4,6);6,687(4,0);6,627(4,0);3,345(21 7,5);3,176(0,5);3,163(0,5);2,543(1,5);2,526(0,8);2,513(18,7);2,508(38,0);2,504(50,2);2,499(36,4);2,495(17,6);2,33 1(0,3);2,157(16,0) |
| Verbindung Nr. 1-1-295: ¹H-NMR(400,0 MHz, d₆-DMSO): 11,021(2,8);9,097(2,7);9,093(2,7);9,074(4,7);9,069(4,5 );8,716(2,3);8,712(2,4);8,704(2,4);8,700(2,3);8,315(1,2);8,310(1,6);8,305(1,1);8,295(1,2);8,290(1,6);8,286(1,1);7, 518(1,6);7,516(1,7);7,506(1,6);7,504(1,6);7,498(1,6);7,496(1,6);7,486(1,5);7,484(1,5);7,463(3,1);7,458(3,0);6,505 (4,2);5,409(0,6);5,336(9,1);3,327(49,1);2,542(2,9);2,525(0,9);2,511(17,0);2,507(33,8);2,502(44,2);2,498(31,9);2,4 93(15,3);2,3664(16,0);2,3656(15,9);2,329(0,4);2,324(0,8);2,282(0,4);2,139(1,2);0,008(1,0);0,000(25,8);-0,009(1,0) |
| Verbindung Nr. 1-1-296: ¹H-NMR(400,0 MHz, d₆-DMSO): 11,105(2,2);9,105(2,1);9,100(2,1);8,728(1,6);8,724(1,7 );8,716(1,7);8,712(1,6);8,324(0,8);8,319(1,2);8,314(0,8);8,304(0,9);8,299(1,3);8,295(0,8);7,529(1,2);7,517(1,2);7, 509(1,2);7,497(1,2);6,554(3,2);6,185(0,3);5,757(1,3);5,662(8,6);4,195(0,5);3,874(1,1);3,859(1,0);3,329(16,8);2,72 7(2,7);2,687(16,0);2,668(1,8);2,632(5,3);2,542(1,3);2,507(20,1);2,503(26,1);2,498(19,2);2,335(12,4);2,161(1,3);0, 008(0,5);0,000(13,4);-0,008(0,6) |
| Verbindung Nr. 1-1-297: ¹H-NMR(400,0 MHz, d₆-DMSO): 11,074(2,6);9,110(2,6);9,106(2,6);8,727(2,3);8,723(2,5 );8,715(2,5);8,711(2,4);8,330(1,1);8,326(1,6);8,321(1,1);8,317(0,4);8,310(1,2);8,305(1,6);8,301(1,1);7,529(1,6);7, 527(1,6);7,517(1,6);7,515(1,6);7,509(1,6);7,507(1,6);7,497(1,5);7,495(1,5);7,001(4,2);6,992(6,5);6,959(3,8);6,950 (2,5);6,512(4,1);5,353(8,5);3,329(27,3);2,542(1,8);2,526(0,4);2,521(0,7);2,512(10,3);2,508(21,0);2,503(27,7);2,49 9(19,8);2,494(9,3);2,308(16,0);2,307(16,0);0,008(0,6);0,000(18,9);-0,009(0,6) |
| Verbindung Nr. 1-1-298: ¹H-NMR(400,0 MHz, d₆-DMSO): 11,075(2,0);9,101(1,8);9,096(1,8);8,723(1,3);8,719(1,4 );8,711(1,4);8,707(1,4);8,320(0,7);8,315(1,1);8,311(0,7);8,300(0,8);8,295(1,1);8,291(0,7);7,523(1,0);7,511(1,0);7, 503(1,0);7,491(0,9);6,639(3,8);6,552(2,8);5,499(5,8);5,230(0,4);3,333(31,0);3,063(1,0);3,049(16,0);3,007(1,0);2,9 95(14,0);2,542(25,0);2,512(6,8);2,508(13,6);2,503(17,8);2,499(13,0);2,494(6,4);2,369(10,1);0,008(0,4);0,000(10,9 );-0,008(0,4) |
| Verbindung Nr. 1-1-299: ¹H-NMR(400,0 MHz, d₆-DMSO): 11,082(3,2);9,092(2,7);9,087(2,7);8,717(2,0);8,713(2,2 );8,705(2,1);8,701(2,1);8,310(1,1);8,306(1,7);8,301(1,2);8,291(1,3);8,285(1,8);8,281(1,2);7,981(0,4);7,974(3,1);7, 971(3,9);7,967(1,9);7,960(1,2);7,955(4,1);7,950(3,6);7,667(0,5);7,663(0,4);7,658(0,3);7,648(1,8);7,641(0,8);7,635 (1,7);7,631(3,0);7,623(4,4);7,619(2,1);7,608(2,0);7,604(4,4);7,592(0,8);7,588(1,6);7,582(1,1);7,570(0,4);7,516(1,6 );7,515(1,6);7,503(1,7);7,496(1,6);7,495(1,6);7,484(1,5);7,483(1,6);6,594(4,4);5,758(0,7);5,659(11,0);4,780(0,6);4 ,104(0,4);4,091(0,4);3,330(23,5);3,177(2,1);3,164(2,1);2,543(1,5);2,526(0,6);2,513(14,4);2,508(29,3);2,504(38,8); 2,499(28,3);2,495(13,8);2,432(16,0);2,164(1,0);0,008(0,7);0,000(21,4);-0,009(0,8) |
| Verbindung Nr. 1-1-300: ¹H-NMR(400,0 MHz, d₆-DMSO): 11,088(3,3);9,101(2,9);9,096(2,9);8,722(2,1);8,718(2,3 );8,710(2,3);8,706(2,2);8,319(1,1);8,315(1,7);8,310(1,1);8,299(1,2);8,294(1,8);8,290(1,2);8,011(0,4);8,005(3,1);8, 001(3,9);7,990(1,3);7,985(4,0);7,981(3,6);7,631(0,5);7,628(0,3);7,613(1,7);7,606(0,8);7,600(1,7);7,596(3,2);7,589 (4,5);7,570(4,3);7,558(0,8);7,554(1,3);7,548(1,0);7,523(1,6);7,521(1,6);7,511(1,6);7,509(1,6);7,503(1,6);7,502(1,6 );7,491(1,5);7,489(1,5);6,608(4,5);5,754(10,9);4,105(0,5);4,092(0,5);3,331(40,4);3,177(2,1);3,164(2,0);2,543(0,4); 2,526(0,6);2,512(12,0);2,508(23,7);2,504(30,9);2,499(22,4);2,495(10,8);2,405(16,0);0,008(0,8);0,000(20,2);-0,009(0,7) |
| Verbindung Nr. 1-1-301: ¹H-NMR(400,0 MHz, d₆-DMSO): 11,064(3,3);11,035(1,2);9,101(3,9);9,098(4,1);8,721(2, 4);8,718(2,9);8,710(2,8);8,706(3,0);8,316(2,3);8,296(2,4);8,136(3,5);8,115(3,6);7,866(1,2);7,847(1,2);7,523(1,9);7 ,510(2,1);7,504(2,1);7,492(1,9);7,040(3,1);7,020(3,0);6,635(1,0);6,615(1,0);6,576(4,4);6,559(1,7);5,337(9,5);5,235 (3,0);3,895(6,9);3,328(73,5);2,902(0,6);2,672(0,4);2,542(19,6);2,507(55,2);2,503(68,9);2,499(54,8);2,355(5,4);2,3 29(0,8);2,309(16,0);0,000(30,3) |
| Verbindung Nr. 1-1-302: ¹H-NMR(400,0 MHz, d₆-DMSO): 11,055(2,9);9,101(2,7);9,097(2,7);8,721(2,2);8,716(2,3 );8,709(2,3);8,704(2,3);8,319(1,1);8,314(1,6);8,309(1,1);8,299(1,2);8,294(1,7);8,289(1,1);7,891(5,2);7,522(1,6);7, 520(1,6);7,510(1,6);7,508(1,5);7,502(1,5);7,500(1,5);7,490(1,5);7,488(1,5);6,542(4,4);5,421(9,1);3,333(23,9);2,54 3(35,1);2,526(0,4);2,521(0,6);2,513(7,3);2,509(15,0);2,504(19,8);2,500(14,3);2,495(6,8);2,363(16,0);0,008(0,5);0, 000(14,2);-0,009(0,5) |
| Verbindung Nr. 1-1-303: ¹H-NMR(400,0 MHz, d₆-DMSO): 11,002(2,3);9,087(2,0);9,083(2,0);8,712(1,6);8,708(1,7 );8,700(1,7);8,696(1,6);8,306(0,8);8,302(1,2);8,297(0,8);8,286(0,9);8,281(1,3);8,277(0,8);7,938(2,4);7,935(3,1);7, 916(3,2);7,914(3,0);7,560(1,9);7,555(0,8);7,541(3,2);7,520(2,3);7,511(1,2);7,510(1,2);7,499(1,1);7,498(1,2);7,491 (1,1);7,490(1,1);7,479(1,1);7,478(1,1);7,402(1,2);7,384(1,9);7,365(0,8);6,528(3,2);5,758(3,2);5,382(7,8);3,332(36, 7);2,526(0,4);2,512(8,5);2,508(17,0);2,503(22,4);2,499(16,4);2,495(8,1);2,410(12,0);2,257(16,0);0,008(0,5);0,000( 15,4);-0,009(0,7) |
| Verbindung Nr. 1-1-304: ¹H-NMR(400,0 MHz, d₆-DMSO): 10,946(3,2);9,074(2,8);9,070(2,8);8,713(2,1);8,709(2,2 );8,701(2,3);8,697(2,2);8,293(1,1);8,288(1,6);8,283(1,1);8,273(1,2);8,268(1,7);8,263(1,1);8,131(7,4);7,953(0,5);7, 640(1,5);7,635(2,2);7,629(1,1);7,618(4,7);7,614(5,4);7,609(2,7);7,606(2,7);7,599(1,1);7,589(5,0);7,584(1,8);7,574 (1,6);7,568(3,0);7,563(2,7);7,558(1,4);7,555(1,0);7,546(1,8);7,537(0,4);7,533(0,5);7,529(0,6);7,525(0,4);7,512(1,7 );7,499(1,7);7,492(1,6);7,480(1,5);7,479(1,5);6,445(4,4);5,436(10,0);3,328(38,2);2,891(3,5);2,732(2,9);2,511(14,3 |
| );2,507(27,9);2,502(36,1);2,498(26,4);2,493(13,1);2,246(16,0);0,008(0,7);0,000(17,6);-0,008(0,8) |
| Verbindung Nr. I-1-305: Masse (m/z): 519,2 (M+H)⁺; ¹H-NMR (DMSO-d6) 2.27 (s, 3H), 5.35 (s, 2H), 5.68 (s, 2H), 7.26 (m, 1H), 7.53 - 7.63 (m, 6H), 7.76 (m, 1H), 7.95 - 8.0 (m, 4H), 8.45 (m, 1H), 8.55 (m, 1H). |
| Verbindung Nr. 1-1-306: ¹H-NMR(600,1 MHz, d₆-DMSO): 11,340(3,6);9,154(2,8);9,151(2,8);8,890(2,9);8,886(2,9 );8,756(2,0);8,754(2,1);8,748(2,1);8,746(2,0);8,367(1,2);8,364(1,7);8,361(1,2);8,354(1,2);8,350(1,8);8,347(1,1);8, 174(2,0);8,170(1,9);8,160(2,2);8,155(2,2);7,796(2,2);7,782(2,2);7,558(1,5);7,550(1,5);7,545(1,5);7,537(1,5);7,213 (3,4);7,171(3,4);6,851(4,3);3,351(101,8);2,524(0,3);2,521(0,4);2,512(8,0);2,509(16,5);2,506(22,4);2,503(16,4);2,5 00(7,8);2,465(16,0);0,000(0,9) |
| Verbindung Nr. 1-1-307: ¹H-NMR(601,6 MHz, d₆-DMSO): 19,963(0,3);11,284(5,2);9,139(4,6);9,135(4,8);8,767(3, 3);8,765(3,6);8,759(3,8);8,757(3,6);8,348(1,7);8,344(2,6);8,341(1,9);8,334(1,9);8,331(2,8);8,328(2,0);8,322(0,8);8 ,230(2,7);8,215(2,8);7,568(2,4);7,560(2,4);7,554(2,5);7,546(2,3);6,785(6,1);6,724(0,5);6,566(1,8);6,551(1,9);6,525 (2,2);6,510(2,1);5,762(1,0);4,706(0,7);4,693(1,1);4,679(1,1);4,666(0,7);4,465(0,4);4,452(1,2);4,439(2,2);4,426(2,6 );4,412(2,2);4,400(1,3);4,388(0,4);3,343(60,8);2,615(0,6);2,544(22,3);2,533(2,1);2,522(1,2);2,519(1,2);2,507(63,1 );2,504(87,4);2,501(64,7);2,388(0,5);2,239(0,4);1,411(10,6);1,399(10,6);1,362(0,8);1,269(0,8);1,223(12,9);1,218(1 4,5);1,211(13,4);1,206(16,0);1,195(2,2);0,005(2,1);0,000(64,3);-0,006(2,5) |
| Verbindung Nr. 1-1-308: ¹H-NMR(400,0 MHz, d₆-DMSO): 11,229(2,4);9,118(2,1);9,113(2,2);8,764(1,4);8,760(1,5 );8,752(1,5);8,748(1,5);8,333(0,8);8,328(1,3);8,323(0,9);8,313(0,9);8,308(1,4);8,303(0,9);8,094(0,6);8,080(1,3);8, 065(0,6);7,566(1,2);7,554(1,1);7,546(1,1);7,534(1,1);6,722(3,2);5,757(4,3);4,038(0,4);4,020(0,4);3,708(0,5);3,690 (1,1);3,674(2,0);3,659(1,6);3,644(0,5);3,639(0,3);3,331(25,8);3,126(0,4);3,110(0,6);3,092(0,9);3,077(0,9);3,059(0, 6);2,944(0,6);2,929(1,3);2,912(0,8);2,896(1,0);2,881(0,4);2,612(16,0);2,540(10,9);2,526(0,5);2,507(17,8);2,503(23 ,7);2,498(17,8);2,236(0,5);1,989(1,8);1,193(0,5);1,175(1,0);1,157(0,5);0,008(0,4);0,000(11,5);-0,008(0,5) |
| Verbindung Nr. 1-1-309: ¹H-NMR(400,0 MHz, d₆-DMSO): 11,224(3,5);9,114(3,0);9,109(3,0);8,765(2,0);8,761(2,0 );8,753(2,1);8,749(1,9);8,329(1,2);8,324(1,7);8,319(1,3);8,309(1,3);8,304(1,9);8,299(1,3);8,044(0,8);8,029(1,7);8, 014(0,9);7,568(1,5);7,556(1,5);7,548(1,5);7,536(1,5);7,535(1,4);6,727(3,9);6,486(0,3);5,755(5,1);4,391(0,6);3,738 (1,0);3,721(2,8);3,706(3,0);3,690(1,3);3,642(0,3);3,630(0,5);3,613(0,4);3,568(6,0);3,435(2,1);3,419(4,0);3,402(1,8 );3,355(0,4);3,327(58,1);3,077(0,8);3,059(16,0);3,052(2,2);2,913(2,1);2,544(12,9);2,507(31,5);2,502(40,8);2,498(3 0,4);2,457(0,7);2,323(1,4);2,203(0,5);2,164(1,2);2,074(2,0);1,235(0,6);0,000(48,5);-0,008(2,5) |
| Verbindung Nr. 1-1-310: ¹H-NMR(400,0 MHz, d₆-DMSO): 9,127(7,4);9,123(7,4);8,764(5,1);8,760(5,6);8,752(5,5) ;8,748(5,5);8,342(2,9);8,337(4,2);8,332(3,1);8,322(3,3);8,316(7,1);8,313(3,4);7,725(1,7);7,711(3,2);7,696(1,8);7,5 65(3,9);7,553(3,9);7,547(3,8);7,545(3,9);7,535(3,6);7,533(3,8);6,737(10,7);6,735(10,9);6,030(0,8);6,015(1,5);6,00 0(0,8);5,756(4,2);3,771(3,3);3,759(3,9);3,750(11,6);3,744(12,3);3,730(5,0);3,722(13,1);3,716(14,8);3,708(7,9);3,7 01(3,5);3,686(11,0);3,679(11,7);3,658(6,2);3,630(12,3);3,627(12,9);3,625(12,6);3,602(11,0);3,596(11,7);3,582(4,2 );3,575(4,8);3,568(9,1);3,562(5,0);3,553(3,4);3,547(4,8);3,541(13,8);3,534(11,4);3,518(1,8);3,512(8,2);3,506(7,8); 3,493(3,4);3,486(3,4);3,465(4,8);3,459(4,8);3,444(11,4);3,437(11,0);3,416(14,0);3,410(12,2);3,396(4,4);3,389(7,7) ;3,382(10,7);3,376(7,7);3,372(6,9);3,366(8,7);3,361(7,5);3,357(10,1);3,351(10,8);3,342(9,6);3,336(9,3);3,317(6,5) ;3,293(5,0);3,281(8,3);3,257(7,4);3,253(6,6);3,245(2,7);3,229(5,1);3,198(13,7);3,190(2,7);3,173(12,0);3,170(14,6) ;3,145(9,4);3,136(1,9);3,092(0,7);3,078(0,9);3,063(0,8);3,058(1,1);3,043(1,5);3,030(1,0);2,984(0,9);2,970(1,5);2,9 54(1,0);2,936(0,9);2,922(0,6);2,676(0,6);2,672(0,9);2,667(0,7);2,534(37,9);2,523(7,6);2,507(92,0);2,503(111,4);2, 498(82,1);2,494(44,4);2,481(17,5);2,466(16,0);2,448(5,1);2,434(4,7);2,370(0,4);2,334(0,7);2,329(0,8);2,325(0,7);2 ,234(1,6);1,801(0,5);1,234(0,4);0,146(0,4);0,008(3,0);0,000(87,8);-0,008(4,4);-0,150(0,4) |
| Verbindung Nr. 1-1-311: ¹H-NMR(400,0 MHz, d₆-DMSO): 11,289(13,0);9,133(11,0);9,128(11,0);8,765(7,2);8,761 (7,0);8,753(7,6);8,750(6,7);8,346(4,4);8,342(6,3);8,326(4,8);8,322(6,7);7,660(3,4);7,645(5,9);7,631(3,4);7,567(5,9 );7,555(6,0);7,547(5,9);7,535(5,5);6,744(16,0);5,983(0,9);5,971(1,5);5,958(0,9);5,758(6,2);4,000(1,2);3,983(3,6);3 ,972(4,2);3,966(4,3);3,955(3,9);3,937(1,4);3,807(2,5);3,789(5,6);3,770(7,0);3,753(4,3);3,731(2,3);3,713(1,0);3,677 (3,1);3,659(6,5);3,642(5,7);3,622(3,3);3,604(2,2);3,587(1,7);3,569(0,7);3,439(2,1);3,428(2,5);3,423(2,6);3,411(2,7 );3,405(3,4);3,394(3,8);3,390(3,6);3,378(2,9);3,331(51,6);3,268(3,0);3,254(3,9);3,250(3,5);3,236(4,7);3,221(2,6);3 ,216(2,5);3,203(2,0);3,147(0,5);3,133(0,7);3,128(0,6);3,123(0,7);3,113(1,0);3,104(1,1);3,098(1,0);3,094(1,0);3,090 (1,0);3,079(0,6);3,024(0,6);3,015(0,8);3,009(1,1);3,001(1,1);2,994(0,8);2,985(0,7);2,975(0,7);2,967(0,6);2,952(0,3 );2,673(0,6);2,540(52,6);2,507(70,2);2,504(85,1);2,330(0,6);1,977(1,0);1,958(1,9);1,945(2,6);1,940(2,7);1,934(2,3 );1,925(3,4);1,912(3,0);1,895(2,7);1,879(2,2);1,863(4,5);1,850(6,8);1,832(6,7);1,829(6,7);1,813(5,8);1,803(3,7);1, 798(4,1);1,783(2,7);1,773(1,7);1,744(0,6);1,732(0,3);1,602(1,6);1,585(3,1);1,571(2,5);1,564(3,0);1,555(3,2);1,536 (2,6);1,518(1,0);1,500(0,6);1,484(0,8);1,471(0,8);1,461(0,8);1,454(1,3);1,445(0,6);1,436(0,9);0,000(58,8) |
| Verbindung Nr. 1-1-312: ¹H-NMR(601,6 MHz, d₆-DMSO): 19,978(0,4);11,198(12,3);9,118(11,3);9,115(11,4);8,76 0(8,0);8,757(8,5);8,752(8,6);8,749(8,5);8,324(6,4);8,313(4,3);8,310(6,6);8,307(4,4);7,866(6,1);7,860(5,4);7,560(6, 2);7,552(6,2);7,547(6,2);7,539(6,0);6,711(16,0);6,029(1,3);5,762(1,9);3,352(29,0);3,350(38,1);2,765(0,7);2,759(2, 3);2,753(3,6);2,747(5,2);2,741(5,3);2,735(3,8);2,729(2,3);2,723(0,9);2,617(0,5);2,532(59,2);2,508(63,5);2,505(86, 4);2,502(64,2);2,422(0,4);2,403(0,5);2,392(1,4);2,387(1,4);2,381(0,9);2,239(0,4);1,232(0,4);0,732(2,9);0,724(9,0); 0,720(12,0);0,712(12,0);0,709(9,6);0,701(3,9);0,686(0,6);0,675(0,5);0,645(0,5);0,638(0,5);0,618(4,0);0,611(11,5); 0,606(11,8);0,601(9,8);0,592(2,9);0,551(0,8);0,543(2,4);0,539(3,1);0,532(3,1);0,528(2,4);0,520(0,9);0,330(0,9);0,3 |
| 23(2,7);0,319(2,7);0,313(2,5);0,305(0,7);0,000(59,4);-0,006(2,5) |
| Verbindung Nr. 1-1-313: ¹H-NMR(400,0 MHz, d₆-DMSO): 11,283(5,9);9,130(5,1);9,125(5,1);9,124(5,1);8,764(3,6 );8,760(4,0);8,752(3,9);8,748(4,0);8,346(2,0);8,341(3,0);8,336(2,2);8,326(2,2);8,321(3,2);8,316(2,4);7,602(1,5);7, 587(2,6);7,573(1,5);7,565(2,9);7,564(3,1);7,552(2,9);7,545(2,6);7,544(2,8);7,533(2,4);7,532(2,7);6,744(7,2);6,743 (7,6);5,757(16,0);3,901(1,7);3,874(1,9);3,869(1,8);3,456(0,5);3,447(0,8);3,441(0,9);3,438(0,9);3,428(1,4);3,423(1, 6);3,414(1,0);3,402(2,5);3,390(1,7);3,380(1,8);3,373(2,5);3,363(2,0);3,356(3,7);3,347(3,0);3,333(55,2);3,238(1,0); 3,225(1,8);3,220(1,5);3,206(2,2);3,192(1,3);3,186(0,9);3,173(1,0);2,536(25,1);2,522(1,3);2,513(14,2);2,508(29,3); 2,504(39,8);2,499(30,3);2,495(15,6);1,794(1,0);1,769(2,0);1,600(1,5);1,567(1,7);1,519(0,3);1,488(1,7);1,464(5,2); 1,437(1,9);1,415(0,8);1,405(0,6);1,252(0,7);1,222(1,3);1,193(1,1);1,173(0,4);1,164(0,4);1,111(0,3);0,000(1,9) |
| Verbindung Nr. 1-1-314: ¹H-NMR(400,0 MHz, d₆-DMSO): 9,123(4,0);9,119(4,0);9,118(3,9);8,763(2,9);8,759(3,1) ;8,751(3,1);8,747(3,1);8,337(1,6);8,332(2,3);8,327(1,8);8,318(9,6);8,312(2,7);8,307(1,8);7,822(0,9);7,808(1,8);7,7 93(1,0);7,568(2,1);7,566(2,2);7,556(2,1);7,554(2,1);7,548(2,1);7,546(2,1);7,536(2,0);7,534(2,0);7,174(2,1);7,168( 0,7);7,155(2,1);7,152(3,1);7,140(0,7);7,134(2,3);6,775(1,0);6,772(0,9);6,758(4,2);6,755(4,7);6,736(5,4);6,706(5,8) ;6,704(5,9);5,757(1,1);3,573(1,5);3,559(1,5);3,537(1,5);3,531(1,5);3,458(1,2);3,433(1,2);3,417(1,0);3,321(0,7);3,2 36(2,1);3,220(4,0);3,202(4,1);3,186(2,1);3,169(0,4);2,944(0,8);2,927(1,4);2,912(1,4);2,895(1,1);2,863(0,7);2,672( 0,4);2,529(21,1);2,512(25,3);2,508(49,1);2,503(65,6);2,499(50,8);2,494(30,1);2,477(4,1);2,455(0,6);2,436(0,4);2,4 08(1,1);2,392(1,1);2,334(0,4);2,330(0,5);2,325(0,4);2,234(0,5);1,587(0,5);1,569(2,2);1,550(4,2);1,532(4,3);1,514( 2,4);1,496(0,6);1,393(0,9);1,375(3,2);1,358(5,8);1,340(5,7);1,322(3,1);1,303(0,7);0,907(7,5);0,889(15,6);0,870(7,2 );0,858(7,5);0,839(16,0);0,820(12,2);0,801(3,2);0,374(0,3);0,364(0,9);0,360(1,0);0,354(0,5);0,350(0,5);0,344(1,0); 0,340(0,9);0,330(0,4);0,091(0,8);0,081(0,8);0,000(5,3) |
| Verbindung Nr. 1-1-315: ¹H-NMR(400,0 MHz, d₆-DMSO): 9,124(4,2);9,120(4,1);9,118(4,0);8,764(2,9);8,760(3,2) ;8,752(3,1);8,748(3,1);8,337(1,7);8,332(2,4);8,327(1,7);8,317(2,1);8,312(2,5);8,307(1,7);7,800(1,1);7,785(2,2);7,7 70(1,1);7,567(2,1);7,566(2,2);7,555(2,1);7,554(2,2);7,547(2,1);7,546(2,1);7,535(2,0);7,534(2,1);6,704(5,9);6,703( 6,1);5,716(0,3);5,702(0,6);5,688(0,4);3,335(2,7);3,276(2,0);3,258(4,5);3,243(4,4);3,225(1,9);2,979(0,7);2,963(1,9) ;2,948(1,7);2,931(0,9);2,528(21,4);2,513(11,0);2,508(22,6);2,504(29,9);2,499(22,2);2,495(11,2);1,547(0,8);1,529( 2,5);1,511(3,7);1,493(2,8);1,474(1,1);1,367(0,7);1,349(2,5);1,329(4,1);1,310(4,4);1,293(2,9);1,277(1,3);1,260(1,3) ;1,249(0,7);1,242(1,0);1,223(0,6);0,921(8,0);0,903(16,0);0,885(7,0);0,879(3,7);0,860(5,9);0,842(2,4);0,000(2,4) |
| Verbindung Nr. 1-1-316: ¹H-NMR(400,0 MHz, d₆-DMSO): 9,131(1,6);9,127(1,6);8,765(1,1);8,761(1,2);8,753(1,2) ;8,749(1,1);8,345(0,6);8,340(0,9);8,335(0,7);8,325(0,8);8,319(6,8);7,833(0,5);7,568(0,8);7,556(0,8);7,548(0,8);7,5 36(0,8);7,172(0,8);7,150(1,1);7,132(0,9);6,772(0,4);6,770(0,4);6,755(1,7);6,735(2,1);6,721(2,3);6,720(2,3);3,480( 0,6);3,169(2,6);3,139(4,5);3,123(5,7);3,109(5,8);3,086(5,3);2,875(1,1);2,859(1,3);2,843(1,0);2,804(0,7);2,677(0,4) ;2,673(0,5);2,668(0,4);2,535(8,1);2,508(28,5);2,504(37,7);2,499(28,4);2,395(12,9);2,379(13,4);2,335(0,5);2,330(0, 5);2,231(0,4);1,070(0,4);1,064(0,3);1,052(0,6);1,040(0,4);1,032(0,4);0,867(0,6);0,848(1,6);0,835(2,9);0,819(3,9);0 ,803(2,9);0,791(1,4);0,788(1,4);0,771(0,5);0,479(0,4);0,469(1,4);0,464(1,6);0,454(0,8);0,449(1,5);0,444(1,5);0,434 (0,6);0,380(0,7);0,375(0,8);0,366(5,3);0,355(15,6);0,351(16,0);0,346(7,2);0,341(7,6);0,335(15,7);0,331(14,5);0,32 1(6,0);0,304(0,7);0,285(0,6);0,272(0,7);0,259(1,9);0,250(1,7);0,247(1,9);0,235(0,5);0,131(0,4);0,110(1,1);0,095(4, 6);0,082(14,2);0,071(13,4);0,059(3,6);0,000(3,3) |
| Verbindung Nr. 1-1-317: ¹H-NMR(400,0 MHz, d₆-DMSO): 11,223(0,5);9,134(4,5);9,130(4,7);8,763(3,3);8,759(3,6 );8,751(3,6);8,747(3,5);8,347(1,8);8,342(2,7);8,337(1,9);8,327(2,0);8,322(2,9);8,317(2,3);7,565(2,6);7,553(2,5);7, 546(2,5);7,534(2,4);7,532(2,3);7,464(2,4);7,443(2,4);7,153(0,4);6,758(0,6);6,755(0,6);6,736(0,7);6,726(6,8);6,725 (6,6);3,780(0,6);3,763(1,3);3,742(1,5);3,725(1,3);3,708(0,6);3,331(45,5);2,672(0,3);2,534(24,4);2,521(1,5);2,512( 19,1);2,508(39,2);2,503(52,0);2,499(38,2);2,495(19,1);2,330(0,4);1,579(0,6);1,570(0,6);1,563(2,2);1,552(2,0);1,54 4(3,3);1,535(2,6);1,526(2,3);1,517(2,3);1,507(0,7);1,499(1,0);1,324(0,4);1,183(15,1);1,166(14,9);0,981(1,2);0,964 (1,2);0,905(7,6);0,886(16,0);0,868(6,8);0,829(0,5);0,811(1,0);0,792(0,4);0,000(4,0) |
| Verbindung Nr. 1-1-318: ¹H-NMR(400,0 MHz, d₆-DMSO): 9,135(4,5);9,130(4,7);8,762(3,2);8,758(3,3);8,750(3,4) ;8,746(3,3);8,346(1,8);8,342(2,7);8,337(1,8);8,327(2,0);8,322(2,9);8,318(2,5);7,565(2,6);7,553(2,5);7,545(2,5);7,5 33(2,4);7,475(2,5);7,453(2,5);7,175(0,7);7,153(1,0);7,135(0,7);6,776(0,4);6,758(1,7);6,756(1,6);6,737(1,7);6,723( 6,8);3,874(0,5);3,855(1,0);3,838(1,5);3,822(1,1);3,802(0,5);3,334(6,4);2,751(0,6);2,735(0,6);2,532(24,1);2,512(16, 5);2,508(32,7);2,504(42,4);2,499(30,8);1,571(0,4);1,565(0,6);1,553(1,0);1,544(0,7);1,539(0,9);1,530(1,4);1,516(1, 2);1,510(1,1);1,496(1,1);1,483(1,2);1,479(1,0);1,467(1,4);1,464(1,5);1,447(1,5);1,432(0,6);1,429(0,7);1,414(0,6);1 ,374(0,5);1,365(0,5);1,355(1,4);1,348(1,4);1,336(2,1);1,330(2,3);1,323(2,1);1,316(2,1);1,312(2,1);1,307(2,1);1,301 (1,6);1,289(1,6);1,283(1,3);1,273(1,0);1,265(1,2);1,257(0,6);1,248(0,8);1,240(0,4);1,223(6,9);1,209(1,3);1,184(14, 0);1,167(13,6);0,980(0,8);0,964(0,8);0,950(4,8);0,934(5,0);0,909(8,2);0,890(16,0);0,872(8,6);0,855(4,2);0,837(2,4 );0,000(3,2) |
| Verbindung Nr. 1-1-319: ¹H-NMR(400,0 MHz, d₆-DMSO): 9,136(0,8);9,134(0,9);9,130(0,9);9,129(0,8);8,761(0,7) ;8,757(0,7);8,749(0,7);8,745(0,7);8,348(0,4);8,343(0,5);8,342(0,5);8,338(0,4);8,328(0,4);8,322(0,6);8,318(0,7);7,5 62(0,5);7,560(0,5);7,550(0,5);7,548(0,5);7,542(0,5);7,540(0,5);7,530(0,5);7,528(0,5);7,269(0,9);7,153(0,4);6,758( 0,6);6,755(0,6);6,740(1,4);6,738(1,5);3,332(5,4);2,532(4,3);2,530(4,4);2,521(0,3);2,512(3,4);2,508(7,3);2,503(9,9) ;2,499(7,2);2,494(3,5);1,390(16,0);1,188(2,3);1,054(1,4);0,000(0,8) |
| Verbindung Nr. 1-1-320: ¹H-NMR(400,0 MHz, d₆-DMSO): 11,201(0,5);9,127(2,1);9,126(2,2);9,122(2,3);8,764(1,7 );8,760(1,8);8,752(1,8);8,748(1,8);8,339(0,9);8,335(1,3);8,329(1,0);8,319(1,1);8,315(1,4);8,314(1,4);8,309(1,0);7, 795(0,6);7,780(1,2);7,765(0,6);7,567(1,2);7,565(1,2);7,555(1,2);7,553(1,2);7,547(1,2);7,545(1,2);7,535(1,2);7,533 (1,1);6,717(3,3);6,715(3,3);3,334(23,5);3,105(2,2);3,089(3,6);3,073(2,3);2,817(0,4);2,802(0,5);2,786(0,4);2,531(1 1,8);2,530(11,4);2,513(6,9);2,508(14,1);2,504(18,9);2,499(14,0);2,495(7,2);1,874(0,4);1,857(0,9);1,840(1,1);1,823 (0,9);1,807(0,5);0,903(16,0);0,886(15,4);0,824(2,5);0,808(2,4);0,000(1,4) |
| Verbindung Nr. 1-1-321: ¹H-NMR(400,0 MHz, d₆-DMSO): 9,130(0,5);9,126(0,5);9,125(0,5);8,764(0,4);8,760(0,4) ;8,752(0,4);8,748(0,4);8,319(0,6);6,745(0,7);6,743(0,8);3,109(0,9);3,092(0,9);2,536(2,6);2,535(2,6);2,513(1,1);2,5 09(2,4);2,504(3,2);2,500(2,4);2,495(1,2);2,258(3,9);0,914(8,9);0,814(1,8);0,807(16,0) |
| Verbindung Nr. 1-1-322: ¹H-NMR(400,0 MHz, d₆-DMSO): 11,188(1,5);9,117(3,3);9,113(3,4);8,760(2,3);8,756(2,5 );8,748(2,5);8,744(2,5);8,329(1,4);8,324(2,2);8,319(2,2);8,309(2,0);8,303(3,6);8,300(2,9);8,286(1,0);7,561(1,8);7, 549(1,8);7,541(1,8);7,530(1,6);7,529(1,7);7,358(13,3);7,347(16,0);7,326(0,5);7,312(0,7);7,303(0,4);7,299(0,4);7,2 93(1,7);7,283(1,7);7,271(1,8);7,259(1,7);7,250(0,5);7,241(0,6);6,724(4,7);6,722(4,9);4,457(5,4);4,441(5,4);4,242( 0,8);4,227(0,8);3,343(26,2);2,542(16,2);2,541(16,2);2,512(6,5);2,508(13,5);2,504(18,1);2,499(13,6);2,495(7,1);2,2 39(0,3) |
| Verbindung Nr. 1-1-323: ¹H-NMR(400,0 MHz, d₆-DMSO): 11,232(12,8);9,123(10,7);9,119(10,9);8,764(7,6);8,760 (8,5);8,752(8,2);8,748(8,5);8,337(4,3);8,333(6,3);8,327(4,7);8,317(5,2);8,312(6,9);8,307(5,1);8,295(3,3);8,279(6,9 );8,264(3,4);7,566(5,6);7,565(5,8);7,554(5,6);7,553(5,8);7,546(5,6);7,545(5,7);7,534(5,3);7,533(5,5);7,493(6,1);7, 489(5,1);7,475(8,3);7,470(8,4);7,455(0,4);7,431(5,4);7,426(5,1);7,412(8,3);7,408(8,5);7,381(2,6);7,376(3,6);7,362 (9,1);7,358(8,5);7,349(9,9);7,345(12,4);7,343(13,4);7,330(7,6);7,326(7,6);7,312(2,4);7,307(2,4);7,297(1,6);7,291( 1,3);7,278(1,4);7,273(1,3);7,261(0,7);7,255(0,6);6,750(15,5);6,748(16,0);6,635(0,8);6,620(1,6);6,605(0,8);5,757(2, 0);4,547(15,5);4,532(15,5);4,307(4,6);4,292(4,6);3,353(0,6);3,332(126,1);2,700(0,3);2,677(0,5);2,672(0,8);2,668(0 ,6);2,540(53,1);2,539(53,3);2,526(2,9);2,512(38,3);2,507(79,4);2,503(106,6);2,498(79,6);2,494(40,4);2,334(0,5);2, 330(0,7);2,325(0,5);1,233(0,6);0,008(1,1);0,000(31,3);-0,008(1,3) |
| Verbindung Nr. 1-1-324: ¹H-NMR(400,0 MHz, d₆-DMSO): 11,173(10,2);9,114(12,4);8,760(8,2);8,749(8,6);8,471( 3,4);8,456(7,0);8,440(3,8);8,327(6,4);8,307(7,0);7,566(5,5);7,554(5,9);7,547(6,1);7,534(5,5);7,406(13,8);7,386(8,6 );7,367(10,0);7,344(9,3);7,341(9,0);7,324(13,4);7,306(5,8);6,720(16,0);5,757(8,2);4,453(15,2);4,438(15,8);3,333(5 2,1);2,672(0,5);2,533(51,5);2,503(79,2);2,500(73,8);2,330(0,5);0,000(6,2) |
| Verbindung Nr. 1-1-325: ¹H-NMR(400,0 MHz, d₆-DMSO): 11,176(2,2);9,115(5,5);9,111(5,6);9,110(5,5);8,763(4,0 );8,759(4,6);8,751(4,3);8,747(4,5);8,427(1,6);8,412(3,4);8,396(1,7);8,329(2,2);8,323(3,2);8,319(2,5);8,309(2,5);8, 303(3,5);8,299(2,6);7,567(2,8);7,565(3,1);7,555(2,8);7,553(3,0);7,547(2,8);7,545(3,0);7,535(2,7);7,533(2,9);7,426 (5,6);7,420(2,6);7,410(3,4);7,404(16,0);7,378(14,2);7,357(6,2);7,271(1,8);7,250(1,3);6,717(7,9);6,715(8,4);6,532( 0,5);5,758(0,7);4,437(7,9);4,422(7,8);4,213(1,5);4,198(1,5);3,334(68,0);2,672(0,4);2,532(26,7);2,530(27,5);2,512( 19,7);2,508(42,4);2,503(58,0);2,499(43,6);2,494(22,2);2,330(0,4) |
| Verbindung Nr. 1-1-326: ¹H-NMR(400,0 MHz, d₆-DMSO): 11,213(9,2);9,121(10,9);9,117(10,9);9,115(10,9);8,763 (7,8);8,759(8,7);8,751(8,5);8,747(8,7);8,334(4,3);8,329(6,4);8,324(4,8);8,314(4,9);8,309(7,0);8,304(5,0);8,278(3,1 );8,263(6,5);8,247(3,2);7,564(5,6);7,563(6,0);7,551(6,0);7,545(5,6);7,543(5,9);7,533(5,3);7,531(5,7);7,436(2,7);7, 433(3,2);7,418(5,5);7,413(6,5);7,398(3,3);7,394(3,7);7,374(1,4);7,370(1,5);7,360(1,7);7,356(3,7);7,341(3,6);7,335 (4,7);7,331(3,1);7,322(2,7);7,317(2,8);7,304(0,4);7,298(0,9);7,293(0,8);7,285(0,6);7,279(0,7);7,274(0,4);7,265(0,4 );7,261(0,3);7,230(5,6);7,216(6,2);7,212(4,4);7,207(6,0);7,203(6,7);7,200(10,3);7,186(3,7);7,182(6,9);7,179(4,4);7 ,158(1,2);7,149(0,8);7,142(0,6);7,140(0,5);7,129(0,5);6,735(15,4);6,734(16,0);6,510(0,3);6,495(0,7);6,480(0,3);4,5 21(13,9);4,506(13,9);4,276(1,7);4,261(1,7);3,339(70,8);2,678(0,3);2,673(0,4);2,669(0,3);2,536(53,9);2,522(2,4);2, 513(23,0);2,509(48,5);2,504(65,8);2,499(48,9);2,495(24,7);2,331(0,4);2,240(0,6) |
| Verbindung Nr. 1-1-327: ¹H-NMR(400,0 MHz, d₆-DMSO): 11,186(2,6);9,126(10,7);9,121(11,0);8,771(6,4);8,767( 7,6);8,759(7,0);8,755(7,5);8,536(0,6);8,456(3,1);8,440(6,4);8,425(3,3);8,333(6,1);8,329(4,5);8,318(4,1);8,313(6,5) ;8,309(4,6);7,630(0,4);7,573(5,7);7,561(5,8);7,553(5,8);7,541(5,6);7,521(0,4);7,431(2,3);7,410(5,6);7,395(6,3);7,3 75(3,8);7,347(0,8);7,342(0,8);7,327(0,6);7,207(8,2);7,187(12,4);7,162(5,3);7,129(3,0);7,123(2,6);7,106(6,3);7,086 (3,4);7,066(1,2);7,043(1,1);6,729(16,0);6,611(0,4);6,596(0,7);6,581(0,4);4,813(1,0);4,474(14,9);4,459(14,9);4,260 (2,1);4,245(2,0);3,347(71,4);2,679(0,4);2,542(53,5);2,511(67,4);2,507(54,1);2,378(0,3);2,337(0,4);2,245(0,5) |
| Verbindung Nr. 1-1-328: ¹H-NMR(400,0 MHz, d₆-DMSO): 11,174(5,4);9,114(9,1);9,109(9,1);8,762(6,1);8,758(6,6 );8,750(6,5);8,746(6,5);8,376(2,6);8,361(5,5);8,346(2,7);8,327(3,6);8,322(5,5);8,317(3,8);8,307(3,8);8,302(5,7);8, 298(3,7);7,564(4,9);7,552(4,9);7,544(4,8);7,532(4,6);7,411(6,7);7,396(8,1);7,389(9,3);7,375(8,2);7,296(0,8);7,281 (1,0);7,274(1,2);7,260(1,1);7,205(1,2);7,197(9,4);7,192(3,2);7,181(3,7);7,175(16,0);7,170(3,8);7,158(2,9);7,153(8, 9);7,136(0,6);7,131(2,1);7,109(1,0);6,714(13,5);6,483(0,4);6,468(0,6);6,452(0,3);5,758(1,0);4,432(12,6);4,416(12, 5);4,208(1,8);4,193(1,8);3,335(77,3);2,672(0,6);2,668(0,4);2,535(47,3);2,508(62,3);2,503(81,7);2,499(60,2);2,335( 0,4);2,330(0,5);2,326(0,4) |
| Verbindung Nr. 1-1-329: ¹H-NMR(400,0 MHz, d₆-DMSO): 11,282(2,0);9,134(10,1);9,130(10,1);9,129(10,0);9,112 (0,4);8,768(7,2);8,764(8,0);8,756(7,8);8,752(7,9);8,549(5,8);8,538(5,9);8,537(6,0);8,510(0,9);8,503(1,4);8,498(1,2 );8,493(1,4);8,491(1,4);8,465(0,3);8,450(0,6);8,441(0,6);8,436(0,6);8,429(0,5);8,423(0,5);8,406(3,3);8,392(7,0);8, |
| 378(3,5);8,351(4,1);8,345(6,1);8,341(4,3);8,331(4,5);8,325(6,6);8,321(4,7);8,302(0,4);8,261(0,6);8,251(0,6);7,822 (3,7);7,817(3,8);7,802(7,4);7,798(7,6);7,783(4,6);7,779(4,9);7,758(1,1);7,753(1,1);7,746(0,7);7,741(0,7);7,739(0,8 );7,734(0,9);7,729(1,5);7,725(1,6);7,717(0,9);7,712(0,9);7,707(0,6);7,702(0,4);7,698(0,6);7,693(0,8);7,687(0,7);7, 682(0,6);7,676(0,5);7,669(0,8);7,656(1,4);7,641(0,8);7,636(1,2);7,631(0,9);7,623(0,5);7,617(0,6);7,611(0,7);7,606 (0,5);7,592(0,3);7,587(0,3);7,579(0,4);7,569(5,7);7,557(5,7);7,549(5,6);7,539(5,1);7,537(5,5);7,403(8,2);7,383(7,6 );7,358(1,1);7,353(0,9);7,346(0,6);7,339(0,7);7,334(0,7);7,324(4,7);7,311(5,3);7,307(5,4);7,293(4,5);7,281(0,9);7, 275(0,9);7,269(1,0);7,264(1,4);7,261(1,2);7,252(1,3);7,249(1,3);7,245(1,1);7,230(1,0);7,221(0,6);7,209(0,8);7,194 (0,9);7,190(0,9);7,179(1,7);7,173(1,0);7,161(1,6);7,149(0,8);7,131(0,4);7,112(0,5);7,104(0,3);7,092(0,5);7,051(0,4 );7,031(0,4);7,001(0,6);6,989(0,6);6,983(0,7);6,970(0,5);6,946(0,8);6,926(0,7);6,758(14,7);6,756(15,3);5,060(0,3); 4,656(0,4);4,636(0,4);4,620(0,4);4,599(15,8);4,585(16,0);4,565(0,5);4,368(1,1);4,352(1,0);4,340(2,0);4,325(2,0);4, 272(0,3);4,146(0,4);4,014(0,4);3,998(0,4);3,940(0,4);3,929(0,6);3,910(0,5);3,873(1,6);3,869(1,7);3,614(0,5);3,605 (0,5);3,546(0,5);3,521(0,5);3,484(0,4);3,336(185,5);2,677(0,5);2,673(0,7);2,668(0,5);2,550(51,4);2,526(1,5);2,521 (2,2);2,512(39,1);2,508(83,3);2,503(113,3);2,499(84,5);2,495(42,8);2,385(0,4);2,335(0,6);2,330(0,8);2,326(0,6);2, 237(1,1);1,233(0,4);0,000(0,4) |
| Verbindung Nr. 1-1-330: ¹H-NMR(400,0 MHz, d₆-DMSO): 12,153(0,6);11,164(2,5);10,930(0,7);9,113(3,3);9,107( 3,5);8,763(1,8);8,759(1,9);8,751(1,9);8,747(1,9);8,726(0,8);8,723(0,9);8,715(0,8);8,711(0,9);8,579(2,2);8,574(2,3) ;8,490(2,2);8,487(2,2);8,478(2,2);8,475(3,1);8,459(0,8);8,326(1,4);8,321(2,1);8,316(1,7);8,306(1,6);8,301(2,3);8,2 96(1,5);7,777(0,8);7,772(1,3);7,767(0,9);7,757(0,9);7,752(1,5);7,748(1,0);7,565(1,4);7,553(1,3);7,545(1,3);7,533( 1,8);7,519(0,6);7,511(0,7);7,499(0,6);7,394(1,3);7,382(1,3);7,374(1,3);7,362(1,2);6,713(3,6);6,712(3,7);6,419(0,5) ;4,477(3,6);4,462(3,6);4,119(1,6);4,106(4,8);4,092(4,9);4,079(1,7);3,334(57,2);3,177(16,0);3,164(15,6);2,532(12,8 );2,521(1,2);2,512(13,0);2,508(27,1);2,503(36,6);2,499(27,3);2,495(13,9);2,325(0,3);2,236(5,8);1,234(0,8);0,000(9 ,2);-0,008(0,4) |
| Verbindung Nr. 1-1-331: ¹H-NMR(400,0 MHz, d₆-DMSO): 11,265(5,4);9,136(6,2);9,131(6,4);8,763(4,2);8,759(4,6 );8,751(4,5);8,747(4,5);8,346(2,3);8,342(3,6);8,337(2,5);8,326(2,6);8,322(3,9);8,317(2,8);7,997(3,8);7,977(3,9);7, 563(3,4);7,551(3,4);7,543(3,4);7,531(3,2);7,434(6,0);7,415(10,6);7,382(6,1);7,364(9,9);7,345(5,0);7,292(3,5);7,27 4(4,9);7,256(1,7);6,740(9,1);5,011(0,6);4,993(2,3);4,975(3,3);4,956(2,3);4,939(0,6);3,335(43,5);2,673(0,5);2,542( 0,5);2,512(47,4);2,504(45,8);2,499(34,0);2,239(0,4);1,537(16,0);1,519(15,9);1,300(0,4);1,297(0,4);0,000(3,6) |
| Verbindung Nr. 1-1-332: ¹H-NMR(400,0 MHz, d₆-DMSO): 11,252(6,1);9,134(7,2);9,130(7,2);8,763(4,6);8,761(4,8 );8,752(4,9);8,749(4,7);8,341(4,1);8,320(4,4);8,069(4,4);8,050(4,5);7,566(3,6);7,553(3,8);7,546(3,8);7,534(3,4);7, 488(5,3);7,474(6,8);7,467(7,3);7,453(6,0);7,205(6,1);7,183(10,9);7,161(5,2);6,733(10,0);5,015(0,7);4,998(2,5);4,9 80(3,7);4,962(2,5);4,944(0,7);3,333(32,1);2,673(0,5);2,668(0,5);2,504(94,2);2,330(0,5);1,524(16,0);1,507(15,9);0, 000(15,0) |
| Verbindung Nr. 1-1-333: ¹H-NMR(400,0 MHz, d₆-DMSO): 11,380(6,0);9,157(5,2);9,152(5,3);8,858(15,5);8,846(1 6,0);8,834(0,5);8,796(0,9);8,783(1,0);8,773(4,2);8,769(4,3);8,761(4,4);8,757(4,2);8,373(2,1);8,369(3,1);8,364(2,2) ;8,354(2,3);8,348(3,3);8,344(2,3);8,250(3,5);8,230(3,6);8,052(0,3);7,573(2,9);7,561(2,8);7,554(2,7);7,553(2,8);7,5 42(2,6);7,541(2,7);7,493(3,9);7,481(7,6);7,468(3,8);7,410(0,5);6,785(7,5);6,783(7,7);5,758(4,4);5,130(0,6);5,113( 2,2);5,095(3,1);5,077(2,4);5,059(0,7);3,333(65,9);2,674(0,4);2,544(26,4);2,527(1,2);2,513(19,5);2,509(40,5);2,504 (54,3);2,500(40,3);2,496(20,4);2,331(0,4);2,227(0,8);1,852(1,2);1,575(0,7);1,561(15,2);1,544(15,0);1,412(1,2);1,3 94(1,2);1,382(0,6);1,365(0,6) |
| Verbindung Nr. 1-1-334: ¹H-NMR(400,0 MHz, d₆-DMSO): 11,269(1,3);9,141(1,5);9,137(1,9);9,130(1,1);8,771(2,1 );8,768(1,0);8,758(2,0);8,488(0,7);8,473(0,3);8,352(0,4);8,347(0,6);8,342(0,4);8,332(0,5);8,327(0,7);8,322(0,4);7, 576(0,5);7,564(0,6);7,557(0,5);7,545(0,5);7,543(0,5);7,508(0,7);7,506(0,8);7,495(0,7);7,493(0,7);6,755(1,5);4,596 (1,6);4,581(1,6);3,334(8,3);3,098(1,1);3,085(1,5);3,079(3,5);3,067(3,8);3,061(3,8);3,049(3,7);3,043(1,6);3,031(1,2 );2,539(5,1);2,526(0,5);2,512(6,5);2,508(12,6);2,503(16,3);2,499(12,2);2,495(6,3);1,213(8,1);1,195(16,0);1,177(7, 7) |
| Verbindung Nr. 1-1-335: ¹H-NMR(400,0 MHz, d₆-DMSO): 11,333(2,0);9,159(1,6);9,155(1,7);8,876(4,9);8,863(5,1 );8,773(1,2);8,769(1,3);8,761(1,3);8,757(1,3);8,666(2,3);8,376(0,6);8,371(1,0);8,366(0,7);8,356(0,7);8,351(1,0);8, 346(0,7);7,575(0,9);7,573(0,9);7,562(0,9);7,561(0,9);7,555(0,9);7,553(0,9);7,543(0,9);7,541(0,8);7,479(1,3);7,467 (2,5);7,455(1,3);6,757(2,4);3,327(5,8);2,530(0,5);2,526(0,5);2,508(18,3);2,504(16,3);2,499(11,8);2,495(6,2);1,781 (16,0);1,765(1,0);1,652(0,4);0,008(0,7);0,000(20,7);-0,008(1,2) |
| Verbindung Nr. 1-1-336: ¹H-NMR(400,0 MHz, d₆-DMSO): 11,274(0,4);9,137(0,6);9,130(0,4);8,772(0,5);8,759(0,6 );6,753(0,5);4,596(0,5);4,582(0,5);3,334(2,2);3,086(1,0);3,068(3,1);3,057(3,7);3,050(3,5);3,039(3,5);3,021(1,2);2, 538(1,6);2,508(5,8);2,504(7,7);2,500(6,2);1,219(8,5);1,201(16,0);1,182(8,1);0,000(6,5) |
| Verbindung Nr. 1-1-337: ¹H-NMR(400,0 MHz, d₆-DMSO): 11,015(3,2);9,090(2,8);9,086(2,9);8,715(2,2);8,711(2,4 );8,703(2,3);8,699(2,3);8,317(0,4);8,310(1,1);8,305(1,7);8,300(1,2);8,290(1,3);8,285(1,8);8,280(1,2);7,515(1,6);7, 503(1,6);7,496(1,6);7,484(1,5);7,483(1,5);7,293(2,6);7,274(2,6);7,235(2,6);7,207(2,6);6,524(4,5);5,757(0,7);5,223 (7,5);3,818(1,3);3,792(4,1);3,766(4,3);3,754(0,9);3,740(1,5);3,325(26,0);2,672(0,4);2,525(0,9);2,511(20,8);2,507( 43,3);2,502(58,6);2,498(44,3);2,494(23,0);2,460(0,3);2,394(15,6);2,333(0,5);2,329(0,6);2,325(0,5);2,320(0,5);2,30 1(16,0);2,279(0,7);2,247(0,6);0,008(1,3);0,000(42,0);-0,008(2,1) |
| Verbindung Nr. 1-1-338: ¹H-NMR(400,0 MHz, d₆-DMSO): 11,302(1,4);9,133(1,8);8,767(1,2);8,756(1,2);8,349(1,0 );8,329(1,0);8,255(0,6);8,242(1,1);8,229(0,6);7,574(0,8);7,562(0,9);7,555(0,9);7,542(0,8);7,179(2,5);6,762(2,4);4, 595(2,5);4,581(2,5);3,336(26,3);2,545(7,6);2,503(18,9);2,417(16,0);0,000(15,7) |
| Verbindung Nr. 1-1-339: ¹H-NMR(400,0 MHz, d₆-DMSO): 11,071(3,1);9,111(2,8);9,107(2,9);8,722(1,9);8,719(2,2 );8,710(2,1);8,707(2,2);8,325(1,6);8,310(1,2);8,305(1,7);8,108(2,9);8,085(3,1);7,727(3,6);7,553(2,1);7,550(2,2);7, 531(2,1);7,526(2,8);7,511(1,7);7,504(1,6);7,491(1,5);6,596(4,3);5,757(0,8);5,479(8,0);3,326(16,6);2,672(0,4);2,50 8(42,4);2,503(55,9);2,499(44,1);2,320(16,0);1,233(0,4);0,007(2,2);0,000(47,7) |
| Verbindung Nr. 1-1-340: ¹H-NMR(400,0 MHz, d₆-DMSO): 11,054(3,3);9,092(2,9);9,087(2,9);8,717(2,1);8,713(2,2 );8,705(2,2);8,701(2,2);8,310(1,1);8,305(1,7);8,301(1,2);8,290(1,2);8,285(1,8);8,281(1,2);7,897(3,6);7,892(3,7);7, 801(3,6);7,779(4,1);7,515(1,6);7,503(1,6);7,495(1,6);7,482(3,8);7,477(2,6);7,460(2,2);7,455(2,2);6,582(4,6);5,756 (0,8);5,640(10,5);3,328(35,5);2,508(35,0);2,503(46,1);2,499(34,5);2,391(16,0);2,160(0,6);0,008(2,3);0,000(53,8);-0,008(2,5) |
| Verbindung Nr. 1-1-341: ¹H-NMR(400,0 MHz, d₆-DMSO): 11,070(3,3);9,106(2,7);9,101(2,9);8,717(2,1);8,713(2,3 );8,705(2,3);8,701(2,3);8,370(2,9);8,349(3,0);8,324(1,1);8,319(1,7);8,314(1,2);8,304(1,2);8,299(1,7);8,294(1,2);8, 000(2,1);7,979(4,6);7,957(2,3);7,798(1,2);7,794(1,2);7,781(1,6);7,777(2,3);7,773(1,3);7,760(1,3);7,756(1,2);7,625 (1,4);7,622(1,5);7,605(2,3);7,588(1,1);7,585(1,2);7,519(1,6);7,506(1,6);7,499(1,7);7,487(1,6);7,122(3,1);7,101(3,1 );6,602(4,4);5,757(0,6);5,520(10,0);3,328(29,4);2,525(0,8);2,512(16,6);2,508(34,1);2,503(45,9);2,499(35,1);2,494( 18,6);2,346(16,0);2,330(0,8);1,990(0,4);0,008(1,3);0,000(34,4);-0,008(1,9) |
| Verbindung Nr. 1-1-342: ¹H-NMR(400,0 MHz, d₆-DMSO): 11,052(2,1);10,612(1,9);9,091(1,8);9,086(1,8);9,023(2, 3);9,019(2,4);8,772(1,8);8,768(1,9);8,760(1,9);8,756(1,9);8,717(1,3);8,713(1,4);8,705(1,4);8,701(1,4);8,309(0,7);8 ,305(1,1);8,300(0,8);8,290(0,8);8,284(1,2);8,280(0,8);8,234(0,9);8,229(1,4);8,224(1,0);8,214(1,0);8,209(1,5);8,204 (1,0);7,981(2,4);7,977(2,5);7,934(3,3);7,930(3,3);7,788(2,5);7,767(2,8);7,728(3,3);7,707(3,8);7,572(1,4);7,560(1,5 );7,554(1,4);7,552(1,4);7,542(1,3);7,540(1,3);7,515(1,1);7,503(1,1);7,495(1,1);7,485(1,0);7,483(1,0);7,462(1,9);7, 457(1,9);7,441(1,8);7,436(3,9);7,431(2,8);7,415(2,2);7,410(2,3);6,580(3,0);6,226(4,0);5,757(8,5);5,632(16,0);3,32 6(40,3);2,676(0,4);2,672(0,5);2,667(0,4);2,525(1,2);2,511(30,8);2,507(63,8);2,503(85,8);2,498(64,4);2,494(32,9);2 ,390(10,6);2,334(0,4);2,329(0,6);2,325(0,5);2,295(0,4);2,158(15,7);2,071(0,5);1,235(1,8) |
| Verbindung Nr. 1-1-343: ¹H-NMR(400,0 MHz, d₆-DMSO): 8,598(4,2);8,594(4,2);8,530(2,7);8,526(3,0);8,518(3,0) ;8,514(3,0);8,317(14,9);8,081(3,3);8,058(4,0);7,990(3,1);7,954(1,7);7,847(3,1);7,824(4,6);7,816(2,0);7,805(1,8);7, 800(2,8);7,796(1,9);7,763(2,0);7,741(1,8);7,701(0,4);7,358(2,2);7,346(2,3);7,331(5,3);7,329(5,2);7,019(2,0);6,997 (1,9);6,077(1,2);5,757(0,4);5,296(10,0);5,246(10,8);3,325(62,5);2,892(12,6);2,732(10,2);2,677(0,7);2,672(1,0);2,6 68(0,7);2,526(2,1);2,521(3,3);2,512(54,3);2,508(113,7);2,503(152,7);2,499(112,6);2,494(55,9);2,339(0,4);2,335(0, 8);2,330(1,1);2,325(0,8);2,093(16,0);1,976(0,4);1,910(0,5);1,234(1,6);0,146(0,8);0,008(5,6);0,000(178,1);-0,009(6,9);-0,021(0,4);-0,150(0,8) |
| Verbindung Nr. 1-1-344: ¹H-NMR(400,0 MHz, d₆-DMSO): 12,461(2,5);8,865(2,5);8,860(2,5);8,803(8,0);8,791(8,2 );8,625(1,4);8,615(1,3);8,134(1,3);8,094(1,4);8,090(1,0);8,078(1,1);8,074(1,5);7,606(0,3);7,597(0,4);7,467(1,8);7, 455(3,5);7,449(1,6);7,442(2,1);7,437(1,4);7,429(1,3);7,417(1,2);7,109(5,1);7,009(0,4);7,002(0,4);6,987(0,4);6,980 (0,4);5,755(13,3);5,498(9,9);3,822(0,9);3,810(0,6);3,798(1,9);3,790(2,6);2,524(0,6);2,510(15,4);2,506(30,8);2,502 (40,0);2,497(28,9);2,493(14,1);2,346(16,0);2,328(0,4);1,755(1,1);0,008(1,0);0,000(28,3);-0,009(1,0) |
| Verbindung Nr. 1-1-345: ¹H-NMR(400,0 MHz, d₆-DMSO): 11,372(3,5);10,925(1,0);9,108(1,3);9,104(1,3);9,061(3, 0);9,059(3,2);9,055(3,3);9,053(3,0);8,996(0,5);8,983(11,9);8,971(11,9);8,726(3,3);8,722(3,5);8,714(3,5);8,710(3,4 );8,324(0,5);8,319(0,8);8,315(1,0);8,304(0,6);8,299(0,8);8,295(0,6);8,287(1,5);8,282(1,9);8,277(1,4);8,267(1,6);8, 262(2,0);8,261(2,0);8,257(1,5);7,722(3,2);7,710(6,1);7,697(3,1);7,531(0,8);7,530(0,8);7,518(0,8);7,510(2,5);7,498 (2,4);7,490(1,8);7,488(1,7);7,478(1,7);7,476(1,7);7,047(4,4);7,045(4,3);6,410(0,6);5,756(2,0);3,911(1,3);3,324(4,8 );2,709(16,0);2,707(15,7);2,680(0,5);2,675(0,9);2,671(1,2);2,666(0,9);2,662(0,4);2,541(0,9);2,524(3,1);2,519(4,7); 2,511(64,0);2,506(131,2);2,502(172,7);2,497(122,4);2,493(57,0);2,337(0,3);2,333(0,8);2,328(1,1);2,324(0,8);2,23 5(7,9);1,909(1,7);1,298(2,1);1,259(3,1);1,235(2,5);1,187(0,4);1,175(0,4);0,854(0,4);0,146(0,3);0,008(2,9);0,000(8 8,2);-0,009(2,8);-0,150(0,3) |
| Verbindung Nr. 1-1-346: ¹H-NMR(400,0 MHz, d₆-DMSO): 8,744(9,7);8,732(9,8);8,464(3,0);8,317(0,9);7,630(0,9) ;7,430(3,0);7,418(5,7);7,406(2,9);7,248(1,2);5,902(0,4);5,889(0,3);5,335(8,5);3,326(169,8);3,294(1,0);3,269(34,2) ;2,675(1,1);2,671(1,4);2,666(1,0);2,541(0,7);2,506(167,9);2,502(204,8);2,497(151,1);2,333(1,1);2,329(1,4);2,324( 1,0);2,184(16,0);0,146(0,8);0,000(177,1);-0,008(10,4);-0,150(0,9) |
| Verbindung Nr. 1-1-347: ¹H-NMR(400,0 MHz, d₆-DMSO): 10,893(0,7);9,099(0,7);9,095(0,7);8,714(0,5);8,710(0,5 );8,702(0,5);8,698(0,5);8,316(0,4);8,296(0,4);7,517(0,4);7,515(0,4);7,503(0,4);7,497(0,4);7,496(0,4);7,485(0,4);7, 483(0,4);6,461(1,1);5,756(0,3);3,325(7,5);2,511(3,3);2,507(6,7);2,502(8,9);2,498(6,6);2,424(4,4);1,575(16,0);0,00 0(0,9) |
| Verbindung Nr. 1-1-348: ¹H-NMR(601,6 MHz, CD3CN): 8,896(0,6);8,625(0,7);8,621(0,7);6,468(0,8);2,446(4,5);2 ,445(4,5);1,956(0,4);1,952(0,5);1,948(2,8);1,944(4,9);1,940(7,0);1,935(4,6);1,931(2,3);1,621(0,5);1,586(16,0);1,49 3(0,3);1,250(0,5);0,000(1,6) |
| Verbindung Nr. 1-1-349: ¹H-NMR(400,0 MHz, d₆-DMSO): 11,237(3,0);9,150(2,7);9,145(2,9);8,747(1,9);8,744(2,1 |
| );8,735(2,0);8,732(2,0);8,367(1,0);8,362(1,6);8,357(1,1);8,347(1,1);8,342(1,7);8,337(1,1);7,575(0,8);7,569(1,5);7, 564(0,8);7,552(8,4);7,549(10,8);7,541(2,3);7,532(5,9);7,520(1,7);7,511(1,4);7,434(0,8);7,430(1,3);7,424(0,8);7,41 3(1,6);7,401(0,5);7,397(0,7);7,392(0,5);6,760(4,3);3,326(9,4);2,507(18,5);2,503(24,3);2,499(18,2);2,373(16,0);0,0 08(0,7);0,000(16,0) |
| Verbindung Nr. 1-1-350: ¹H-NMR(400,0 MHz, d₆-DMSO): 11,343(3,0);9,038(2,1);9,034(3,5);8,783(3,3);8,776(3,4 );8,288(1,1);8,284(1,3);8,282(1,4);8,277(1,1);8,264(1,1);8,260(1,3);8,258(1,3);8,253(1,0);7,575(0,7);7,570(1,3);7, 564(0,7);7,553(9,2);7,551(9,6);7,534(4,6);7,529(1,3);7,518(0,7);7,513(1,3);7,439(0,8);7,434(1,4);7,428(0,8);7,424 (0,9);7,418(1,5);7,414(0,9);7,412(0,7);7,406(0,5);7,402(0,8);7,396(0,5);6,759(4,3);3,325(16,1);2,525(0,7);2,512(1 2,3);2,507(24,3);2,503(31,7);2,498(22,9);2,494(11,0);2,375(16,0);1,989(0,4);1,397(1,2);0,000(1,2) |
| Verbindung Nr. I-1-351: Masse (m/z): 368,1 (M+H)⁺;¹H-NMR (DMSO-d6) 2.40 (s, 3H), 5.35 (s, 2H), 5.76 (s, 1H), 6.50 (s, 1H), 7.36 (d, 1H), 7.50 (dd, 1H), 8.17 (d, 1H), 8.21 (s, 1H), 8.30 (m, 1H), 8.70 (dd, 1H), 9.09 (m, 1H), 11.00 (s, 1H). |
| Verbindung Nr. 1-1-352: ¹H-NMR(400,0 MHz, d₆-DMSO): 11,068(3,3);10,525(0,7);9,111(2,6);9,107(2,7);9,034(0, 6);8,769(0,4);8,765(0,4);8,757(0,4);8,753(0,4);8,725(1,8);8,722(2,0);8,713(2,0);8,710(2,0);8,326(1,6);8,321(1,1);8 ,310(1,1);8,306(1,7);8,301(1,1);8,239(0,3);8,219(0,4);7,569(0,3);7,556(0,4);7,549(0,3);7,536(0,4);7,528(1,6);7,516 (1,6);7,509(1,6);7,496(1,5);6,977(0,7);6,576(4,4);6,233(0,9);5,791(5,7);5,759(1,2);5,180(1,8);5,152(8,2);3,324(98, 6);3,088(1,7);3,069(5,6);3,051(6,2);3,032(2,7);3,015(0,6);2,891(1,6);2,804(1,0);2,785(1,0);2,766(0,3);2,732(1,4);2 ,671(1,1);2,667(0,8);2,524(2,4);2,506(127,8);2,502(169,3);2,498(126,0);2,333(0,9);2,329(1,2);2,324(1,0);2,298(16 ,0);2,274(0,5);2,244(0,4);2,197(0,6);2,184(3,8);2,160(0,7);1,989(0,4);1,473(0,4);1,351(0,5);1,331(6,2);1,312(12,7) ;1,298(4,0);1,294(6,5);1,279(1,8);1,235(15,5);1,224(2,2);1,205(2,3);1,193(0,4);1,186(1,1);0,871(0,6);0,854(1,6);0, 837(0,7);0,146(1,7);0,008(12,3);0,000(329,7);-0,008(14,8);-0,021(0,9);-0,150(1,7) |
| Verbindung Nr. 1-1-353: ¹H-NMR(400,0 MHz, d₆-DMSO): 11,051(3,2);10,626(0,8);9,092(2,8);9,088(2,8);9,038(0, 7);9,034(0,7);8,775(0,6);8,771(0,6);8,763(0,6);8,758(0,6);8,715(2,1);8,711(2,3);8,703(2,3);8,699(2,2);8,310(1,1);8 ,306(1,6);8,301(1,1);8,290(1,2);8,286(1,7);8,281(1,1);8,240(0,4);8,219(0,4);7,596(3,2);7,575(3,9);7,565(0,5);7,555 (0,6);7,546(4,0);7,544(3,9);7,527(1,1);7,515(1,6);7,513(1,5);7,503(1,6);7,501(1,5);7,495(1,7);7,493(1,8);7,483(1,9 );7,229(1,9);7,227(1,9);7,206(2,2);7,185(0,5);7,182(0,5);6,575(4,4);6,218(1,1);5,757(6,6);5,592(10,9);3,328(22,5); 2,525(0,9);2,512(17,1);2,507(33,9);2,503(44,4);2,498(32,4);2,494(16,0);2,415(15,7);2,401(5,2);2,393(16,0);2,330( 0,3);2,155(4,6);1,234(0,6);0,146(0,5);0,008(4,3);0,000(97,7);-0,009(4,5);-0,018(0,4);-0,150(0,5) |
| Verbindung Nr. 1-2-2: ¹H-NMR(600,1 MHz, d₆-DMSO): 10,894(2,9);9,067(2,5);9,064(2,4);8,825(6,1);8,816(6,1); 8,803(0,5);8,795(0,4);8,786(0,4);8,750(1,8);8,748(1,9);8,742(1,9);8,740(1,8);8,273(1,4);8,259(1,4);7,549(1,4);7,54 1(1,4);7,536(1,4);7,528(1,3);7,497(1,6);7,489(2,9);7,481(1,6);7,263(4,7);7,178(4,9);7,093(4,7);5,581(8,7);5,516(0, 5);5,320(0,5);3,372(3,6);3,174(0,4);2,845(0,4);2,692(0,5);2,632(0,8);2,514(14,6);2,512(18,6);2,509(14,7);2,452(16 ,0);2,332(0,8);2,287(1,0) |
| Verbindung Nr. 1-2-3: ¹H-NMR(400,0 MHz, d₆-DMSO): 11,469(4,7);9,118(3,4);9,113(3,4);8,948(9,2);8,778(2,4); 8,775(2,6);8,766(2,6);8,762(2,5);8,650(14,2);8,333(1,3);8,328(2,0);8,323(1,3);8,313(1,5);8,308(2,1);8,303(1,3);7,5 77(1,9);7,565(1,9);7,558(1,8);7,546(1,7);4,055(1,2);4,038(3,7);4,020(3,8);4,002(1,3);3,324(73,0);2,675(0,6);2,671 (0,8);2,667(0,6);2,541(0,6);2,506(93,1);2,502(120,4);2,497(87,6);2,333(0,6);2,329(0,8);2,324(0,6);1,989(16,0);1,2 98(0,3);1,259(0,5);1,235(0,7);1,193(4,3);1,175(8,4);1,157(4,2);0,146(0,9);0,008(8,3);0,000(189,2);-0,008(7,8);-0,150(0,9) |
| Verbindung Nr. 1-2-4: ¹H-NMR(400,0 MHz, d₆-DMSO): 10,968(2,3);9,063(2,0);9,059(2,0);8,747(1,4);8,744(1,5); 8,735(1,5);8,732(1,5);8,581(5,2);8,310(1,4);8,303(1,5);8,287(1,6);8,280(1,8);8,274(1,1);8,270(1,3);8,250(1,3);8,14 7(2,7);8,140(2,5);8,081(0,4);7,543(1,1);7,531(1,1);7,523(1,1);7,512(1,0);7,303(2,7);7,280(2,6);7,271(0,3);5,757(2, 2);5,442(7,1);5,173(0,6);3,976(16,0);3,962(1,8);3,898(0,3);3,327(5,1);2,525(0,8);2,511(21,0);2,507(40,6);2,503(51 ,8);2,498(37,7);2,494(18,7);2,329(0,3);0,000(22,0);-0,008(0,9) |
| Verbindung Nr. 1-2-5: ¹H-NMR(400,0 MHz, d₆-DMSO): 11,257(1,4);9,264(3,4);9,139(1,2);9,134(1,2);8,788(0,8); 8,784(0,9);8,776(0,9);8,772(0,9);8,352(0,4);8,346(0,7);8,342(0,5);8,332(0,5);8,326(0,7);8,322(0,5);7,878(1,2);7,87 6(1,6);7,857(1,8);7,855(1,7);7,603(1,1);7,598(0,5);7,589(1,0);7,584(1,8);7,568(0,9);7,563(1,3);7,557(0,8);7,447(0, 7);7,428(1,0);7,410(0,4);7,279(0,3);6,683(0,3);3,339(28,7);2,512(5,3);2,508(10,7);2,504(14,2);2,499(10,5);2,495(5 ,3);1,756(16,0);0,008(0,8);0,000(20,3);-0,008(0,9) |
| Verbindung Nr. 1-2-6: ¹H-NMR(400,0 MHz, d₆-DMSO): 11,141(3,4);9,121(2,6);9,116(2,5);8,774(1,8);8,770(1,9); 8,762(1,9);8,758(1,8);8,334(1,1);8,329(1,6);8,325(1,1);8,314(1,2);8,309(1,7);8,305(1,1);7,601(9,4);7,590(16,0);7,5 78(2,2);7,566(1,7);7,558(1,9);7,547(2,4);7,537(1,7);7,526(1,8);7,519(0,6);7,514(1,0);7,505(0,6);7,489(0,4);7,481( 0,4);7,476(0,5);4,056(0,4);4,038(1,1);4,020(1,1);4,002(0,4);3,357(7,4);2,847(1,6);2,828(5,3);2,809(5,4);2,790(1,7) ;2,694(0,4);2,676(0,6);2,512(14,5);2,507(28,9);2,503(37,5);2,498(27,0);2,494(12,9);1,989(4,8);1,258(5,5);1,240(1 1,4);1,221(5,3);1,193(1,7);1,175(3,4);1,157(1,6);0,008(0,5);0,000(14,6);-0,009(0,5) |
| Verbindung Nr. 1-2-7: ¹H-NMR(400,0 MHz, d₆-DMSO): 10,991(5,0);9,070(4,2);9,065(4,2);8,752(2,9);8,748(3,3); 8,740(3,1);8,736(3,2);8,625(9,9);8,276(2,4);8,271(1,7);8,260(1,7);8,256(2,5);8,251(1,7);7,547(2,5);7,535(2,7);7,52 8(4,5);7,523(2,3);7,520(2,3);7,515(3,7);7,510(2,2);7,505(3,5);7,497(0,5);7,423(0,6);7,417(0,9);7,405(3,1);7,399(5, 1);7,391(4,9);7,381(5,3);7,377(3,9);7,364(1,0);7,359(0,6);7,303(3,0);7,299(1,9);7,294(1,5);7,288(1,8);7,280(1,9);5 |
| ,501(16,0);3,333(27,4);2,507(24,7);2,503(32,6);2,499(24,7);0,000(1,8) |
| Verbindung Nr. 1-2-8: ¹H-NMR(400,0 MHz, d₆-DMSO): 10,909(4,7);9,050(3,7);9,029(0,4);8,771(0,7);8,761(0,7); 8,739(2,8);8,730(2,6);8,658(0,6);8,634(8,7);8,316(0,6);8,258(2,3);8,238(2,3);8,092(0,5);7,925(0,5);7,647(0,3);7,61 4(0,4);7,595(0,7);7,569(6,6);7,550(12,0);7,535(3,4);7,523(3,0);7,516(2,7);7,503(2,3);7,474(4,1);7,455(3,4);7,452( 3,1);7,433(2,1);5,894(0,5);5,794(0,4);5,756(0,4);5,624(1,9);5,614(16,0);5,362(1,1);5,290(0,5);3,332(314,1);2,671( 1,4);2,667(1,1);2,507(167,3);2,502(217,8);2,498(164,2);2,329(1,4);2,075(0,8);1,234(0,5);0,008(0,4);0,000(11,5) |
| Verbindung Nr. 1-2-9: ¹H-NMR(400,0 MHz, d₆-DMSO): 9,173(0,5);9,109(1,3);9,104(1,3);8,817(0,8);8,813(0,9);8, 805(0,9);8,801(0,9);8,617(0,3);8,330(0,5);8,326(0,8);8,321(0,7);8,311(0,8);8,306(0,9);8,301(0,7);8,098(2,3);7,640 (0,7);7,604(0,6);7,591(0,7);7,584(0,7);7,572(0,6);7,279(0,4);6,680(0,4);3,333(39,9);3,148(0,5);3,127(2,1);3,049(5, 8);3,010(6,2);2,849(0,5);2,796(2,0);2,541(0,6);2,507(27,3);2,503(35,0);2,498(25,3);1,755(16,0);1,235(0,9);0,008(1 ,7);0,000(40,3);-0,009(1,7) |
| Verbindung Nr. 1-2-10: ¹H-NMR(400,0 MHz, d₆-DMSO): 11,523(2,3);9,519(5,0);9,452(0,9);9,140(1,9);8,796(1,3) ;8,787(1,3);8,359(0,8);8,354(1,3);8,350(0,9);8,339(0,9);8,334(1,3);8,330(0,9);7,598(1,1);7,586(1,1);7,578(1,1);7,5 66(1,0);4,549(2,2);4,532(7,1);4,514(7,4);4,496(2,6);4,492(2,5);4,474(0,7);4,112(0,6);4,098(0,6);3,336(50,0);3,182 (2,8);3,169(2,7);2,549(0,5);2,514(36,8);2,510(47,2);2,506(35,8);1,402(7,4);1,384(16,0);1,367(9,1);1,352(2,4);1,22 4(0,4) |
| Verbindung Nr. 1-2-11: ¹H-NMR(600,1 MHz, d₆-DMSO): 11,410(0,9);9,451(5,5);9,148(1,8);9,145(1,8);8,792(1,3) ;8,789(1,3);8,784(1,3);8,781(1,3);8,620(3,1);8,611(3,1);8,357(0,7);8,354(1,1);8,351(0,8);8,344(0,8);8,341(1,1);8,3 37(0,8);7,590(1,0);7,589(1,0);7,582(1,0);7,581(1,0);7,577(1,0);7,576(1,0);7,569(1,0);7,568(0,9);7,008(3,2);6,999( 3,2);4,061(16,0);3,348(64,6);2,513(3,8);2,510(8,0);2,507(10,9);2,504(7,9);2,501(3,8);1,761(7,0);0,000(1,3) |
| Verbindung Nr. 1-2-12: ¹H-NMR(400,0 MHz, d₆-DMSO): 10,993(2,0);9,086(1,2);8,763(0,8);8,753(0,8);8,299(0,7) ;8,295(1,0);8,290(0,7);8,280(0,8);8,275(1,1);8,270(0,7);7,566(1,0);7,554(1,0);7,546(1,0);7,534(0,9);4,073(1,1);4,0 55(3,4);4,037(3,4);4,019(1,1);3,826(0,4);3,808(0,4);3,368(1,1);3,169(3,1);2,653(16,0);2,547(0,4);2,511(8,5);2,507 (16,6);2,503(21,5);2,498(15,5);2,494(7,5);2,394(2,1);1,353(3,5);1,335(7,4);1,317(3,4);1,211(0,5);1,193(1,0);1,175 (0,5);0,007(1,3);0,000(28,5);-0,008(1,1) |
| Verbindung Nr. 1-2-13: ¹H-NMR(600,1 MHz, d₆-DMSO): 10,979(3,9);9,070(4,1);9,067(4,1);8,751(3,5);8,749(3,7) ;8,743(3,7);8,741(3,6);8,623(11,9);8,272(2,3);8,269(1,7);8,262(1,8);8,259(2,4);7,545(2,5);7,544(2,5);7,537(2,5);7, 536(2,5);7,532(2,5);7,531(2,4);7,524(2,4);7,523(2,3);7,469(1,1);7,465(8,2);7,462(3,0);7,454(3,6);7,451(10,9);7,44 7(1,6);7,365(9,0);7,351(6,8);5,395(16,0);3,337(36,9);2,525(0,3);2,522(0,4);2,519(0,5);2,510(8,5);2,507(17,2);2,50 4(23,1);2,501(16,8);2,498(8,1);1,759(7,2);0,000(5,8) |
| Verbindung Nr. 1-2-14: ¹H-NMR(400,0 MHz, d₆-DMSO): 9,178(0,4);8,370(0,4);8,350(0,4);7,277(0,4);6,680(0,4); 3,713(7,7);3,330(11,8);2,507(19,5);2,502(24,7);2,498(17,6);1,754(16,0);1,073(0,3);1,055(0,7);1,038(0,3);0,000(2, 2) |
| Verbindung Nr. 1-2-15: ¹H-NMR(400,0 MHz, d₆-DMSO): 11,305(7,2);11,201(0,4);9,130(6,2);9,126(6,1);9,074(0,8 );9,071(0,8);9,040(16,0);8,964(0,6);8,913(1,1);8,799(0,7);8,795(0,8);8,783(5,1);8,779(4,9);8,771(4,8);8,767(4,8);8 ,680(0,9);8,660(5,1);8,656(5,5);8,648(5,3);8,645(5,3);8,628(0,4);8,625(0,4);8,617(0,4); 8,613 (0,4); 8,567(0,4); 8,563 (0,4);8,554(2,6);8,547(0,9);8,544(0,9);8,536(0,8);8,532(0,8);8,466(5,2);8,462(5,1);8,445(5,6);8,442(5,3);8,435(0,6 );8,419(0,5);8,415(0,5);8,367(0,4);8,363(0,4);8,344(3,3);8,340(3,8);8,335(2,6);8,325(3,3);8,320(4,0);8,315(2,7);8, 284(0,4);8,279(0,6);8,274(0,4);8,264(0,4);8,259(0,6);8,254(0,4);8,208(0,4);8,069(0,3);7,627(5,9);7,615(5,4);7,607 (5,6);7,600(0,8);7,595(5,3);7,583(3,7);7,571(3,7);7,563(3,8);7,551(3,7);7,534(0,5);7,497(0,7);7,486(0,7);7,475(1,0 );7,463(1,0);7,454(0,9);7,443(0,8);7,202(0,3);7,074(0,3);6,946(0,5);5,687(1,0);3,328(116,4);2,676(0,7);2,671(1,0); 2,667(0,8);2,566(6,8);2,542(30,2);2,525(2,9);2,511(58,4);2,507(116,7);2,502(153,1);2,498(110,7);2,493(53,3);2,3 38(0,4);2,333(0,7);2,329(1,0);2,325(0,7);0,146(0,6);0,008(5,6);0,000(149,6);-0,009(5,6);-0,150(0,7) |
| Verbindung Nr. 1-2-16: ¹H-NMR(400,0 MHz, d₆-DMSO): 10,846(1,9);9,070(1,9);9,066(2,0);8,757(1,2);8,753(1,3) ;8,745(1,3);8,741(1,2);8,282(0,7);8,277(1,2);8,272(0,8);8,262(0,8);8,257(1,2);8,252(0,9);7,556(1,1);7,544(1,1);7,5 36(1,1);7,524(1,0);4,411(0,4);4,038(0,8);4,029(16,0);3,902(0,6);3,813(0,7);3,805(0,9);3,689(0,4);3,556(0,6);3,541 (15,0);3,354(0,8);3,334(20,4);3,290(0,6);2,508(18,4);2,503(23,9);2,499(17,4);2,415(0,7);0,000(5,7) |
| Verbindung Nr. 1-2-17: ¹H-NMR(400,0 MHz, d₆-DMSO): 10,851(1,9);9,080(1,2);8,755(0,9);8,745(0,9);8,293(0,8) ;8,288(1,1);8,283(0,8);8,273(0,8);8,268(1,2);8,263(0,8);7,558(1,0);7,546(1,1);7,538(1,0);7,526(0,9);3,757(16,0);3, 558(2,8);3,349(2,2);3,169(0,3);2,780(1,1);2,761(3,7);2,742(3,8);2,723(1,3);2,643(0,5);2,624(0,5);2,512(8,5);2,508 (16,8);2,503(21,8);2,499(15,7);2,494(7,7);1,255(4,1);1,236(8,5);1,217(4,0);1,186(0,7);1,168(1,4);1,149(0,6);0,000 (5,2) |
| Verbindung Nr. 1-2-18: ¹H-NMR(400,0 MHz, d₆-DMSO): 11,080(3,6);9,100(3,5);9,096(3,6);8,770(2,3);8,767(2,6) ;8,758(2,5);8,755(2,6);8,677(7,5);8,311(1,3);8,307(2,0);8,303(1,5);8,292(1,4);8,287(2,1);7,569(1,9);7,557(1,9);7,5 50(1,9);7,538(1,8);5,450(16,0);3,334(17,1);3,310(28,7);2,508(17,9);2,504(23,5);2,500(18,3);1,756(4,5);0,000(4,9) |
| Verbindung Nr. 1-2-19: ¹H-NMR(400,0 MHz, d₆-DMSO): 10,989(2,3);9,069(1,9);9,065(2,0);8,753(1,4);8,749(1,5) ;8,741(1,5);8,737(1,5);8,640(4,8);8,276(1,1);8,256(1,2);7,549(1,1);7,537(1,1);7,529(1,1);7,517(1,0);7,449(0,3);7,4 27(1,8);7,411(7,9);7,399(0,6);7,309(1,1);7,305(1,6);7,299(1,1);7,295(1,0);7,289(1,3);6,677(0,4);5,410(6,8);3,331( 84,4);2,671(0,4);2,507(45,7);2,502(59,0);2,498(44,4);2,329(0,4);1,989(1,1);1,754(16,0);1,175(0,6);0,000(10,9) |
| Verbindung Nr. 1-2-20: ¹H-NMR(400,0 MHz, d₆-DMSO): 10,807(2,1);9,075(1,9);9,071(2,0);8,753(1,4);8,749(1,5) ;8,741(1,5);8,737(1,5);8,284(0,8);8,279(1,2);8,275(0,8);8,264(0,9);8,259(1,2);8,255(0,8);7,553(1,2);7,542(1,2);7,5 34(1,2);7,522(1,1);4,620(0,4);4,604(1,0);4,587(1,4);4,571(1,1);4,554(0,4);3,332(1,6);2,507(16,4);2,502(21,2);2,49 8(15,4);2,411(16,0);2,234(1,2);1,390(13,8);1,374(13,6);1,293(1,6);1,276(1,6);0,008(0,7);0,000(16,6);-0,008(0,6) |
| Verbindung Nr. 1-2-21: ¹H-NMR(400,0 MHz, d₆-DMSO): 10,941(3,6);9,084(2,8);8,759(2,1);8,750(2,1);8,456(5,9) ;8,299(1,3);8,295(2,0);8,290(1,4);8,280(1,7);8,275(2,3);8,270(1,4);8,261(0,5);7,563(2,0);7,551(2,1);7,544(2,0);7,5 32(1,9);4,122(4,2);4,104(8,5);4,087(4,3);3,873(0,3);3,856(0,7);3,839(0,4);3,347(7,6);2,672(0,4);2,525(1,0);2,512( 22,9);2,507(45,9);2,503(60,1);2,498(43,6);2,494(21,4);2,329(0,4);1,852(0,5);1,834(2,4);1,816(4,8);1,798(4,9);1,78 0(2,6);1,762(0,6);1,719(0,4);1,701(0,4);0,870(7,7);0,852(16,0);0,833(7,3);0,821(0,9);0,803(1,3);0,784(0,6);0,008( 0,5);0,000(14,1);-0,008(0,6) |
| Verbindung Nr. 1-2-22: ¹H-NMR(400,0 MHz, d₆-DMSO): 11,396(7,8);9,343(16,0);9,264(0,3);9,146(6,2);9,141(6,2 );8,793(4,4);8,789(4,7);8,781(4,6);8,777(4,5);8,358(2,5);8,353(3,7);8,348(2,4);8,338(2,7);8,333(3,9);8,328(2,5);7, 594(3,4);7,582(3,4);7,575(3,4);7,563(3,2);7,354(11,7);7,324(0,4);3,332(47,1);2,683(0,6);2,674(0,4);2,544(6,0);2,5 25(80,0);2,513(20,1);2,509(37,7);2,505(48,9);2,500(37,3);2,468(0,5);2,447(1,0);2,388(0,4);2,363(0,5);2,331(0,3);0 ,008(1,7);0,000(39,3);-0,008(1,5) |
| Verbindung Nr. 1-2-23: ¹H-NMR(400,0 MHz, d₆-DMSO): 10,954(3,0);9,070(2,4);9,065(2,4);8,750(1,8);8,746(1,9) ;8,738(1,9);8,734(1,8);8,564(6,1);8,276(1,4);8,271(1,0);8,260(1,0);8,256(1,5);7,547(1,4);7,535(1,4);7,527(1,4);7,5 15(1,3);7,245(1,1);7,242(1,2);7,227(5,5);7,215(1,8);7,209(0,8);7,196(1,4);7,189(1,2);7,182(0,7);7,174(0,7);7,126( 2,1);7,108(1,4);5,397(9,2);3,324(37,3);2,675(0,3);2,671(0,4);2,541(4,3);2,506(50,8);2,502(65,5);2,497(48,4);2,347 (16,0);2,329(0,6);2,303(0,4);0,007(2,5);0,000(49,3);-0,008(2,2) |
| Verbindung Nr. 1-2-24: ¹H-NMR(400,0 MHz, d₆-DMSO): 11,423(1,9);9,468(2,7);9,145(1,5);8,791(1,0);8,779(1,1) ;8,354(0,9);8,334(0,9);7,592(0,8);7,579(0,8);7,572(0,8);7,560(0,7);6,318(3,0);4,007(16,0);3,332(11,2);2,504(20,9) ;0,000(5,3) |
| Verbindung Nr. 1-2-25: ¹H-NMR(400,0 MHz, d₆-DMSO): 11,367(3,8);9,307(8,3);9,142(2,8);9,137(2,8);8,790(1,9) ;8,787(2,0);8,778(1,9);8,775(1,9);8,354(1,3);8,349(1,9);8,344(1,3);8,334(1,4);8,329(2,0);8,324(1,3);7,592(1,7);7,5 80(1,7);7,572(1,7);7,560(1,6);3,333(83,5);2,672(0,4);2,533(41,0);2,511(25,0);2,507(48,6);2,503(63,0);2,499(45,8); 2,330(0,4);2,263(16,0);1,755(4,1);0,007(1,0);0,000(23,2);-0,008(1,0) |
| Verbindung Nr. 1-2-26: ¹H-NMR(400,0 MHz, d₆-DMSO): 10,989(2,0);9,085(1,9);9,080(1,9);8,764(1,3);8,760(1,4) ;8,752(1,4);8,748(1,4);8,297(0,8);8,292(1,1);8,287(0,8);8,277(0,9);8,272(1,2);8,267(0,8);7,563(1,1);7,551(1,1);7,5 43(1,1);7,531(1,0);3,715(14,9);3,474(5,1);3,337(16,8);2,644(16,0);2,539(5,4);2,508(19,7);2,503(25,8);2,499(19,1); 0,000(5,3) |
| Verbindung Nr. 1-2-27: ¹H-NMR(400,0 MHz, d₆-DMSO): 11,607(5,3);9,013(9,0);9,009(9,1);8,799(5,6);8,795(6,4) ;8,787(6,0);8,783(6,2);8,208(3,2);8,204(5,1);8,199(3,7);8,189(3,5);8,184(5,4);8,179(3,8);7,614(10,0);7,594(14,5); 7,579(4,8);7,567(4,7);7,559(4,5);7,547(4,4);7,530(6,9);7,512(16,0);7,492(7,8);7,459(5,5);7,441(6,2);7,423(2,2);7,4 11(0,4);6,818(0,9);3,327(94,7);2,996(0,4);2,712(0,4);2,671(0,9);2,542(76,8);2,506(107,2);2,502(138,2);2,498(110, 3);2,368(0,4);2,329(0,9);0,146(0,4);0,000(88,4);-0,150(0,4) |
| Verbindung Nr. I-2-28: Masse (m/z): 296,1 (M+H)⁺; ¹H-NMR (DMSO-d6) 3.95 (s, 3H), 7.4 - 7.5 (m, 5H), 8.20 (m, 1 H), 8.79 (m, 2 H), 9.02 (m, 1 H), 11.36 (s, 1 H). |
| Verbindung Nr. 1-3-2: ¹H-NMR(400,0 MHz, d₆-DMSO): 11,504(1,6);9,148(1,9);9,144(2,0);8,772(1,4);8,768(1,6); 8,760(1,5);8,756(1,5);8,361(0,8);8,357(1,2);8,352(0,9);8,342(0,9);8,336(1,2);8,332(0,9);8,003(4,5);7,577(1,1);7,57 6(1,1);7,565(1,1);7,563(1,1);7,557(1,1);7,556(1,1);7,545(1,0);7,544(1,0);4,117(16,0);3,325(4,1);2,512(5,4);2,508( 11,0);2,503(14,7);2,499(11,0);2,495(5,7);0,008(0,5);0,000(13,7);-0,008(0,6) |
| Verbindung Nr. 1-3-3: ¹H-NMR(400,0 MHz, d₆-DMSO): 11,107(8,3);9,182(9,3);9,178(9,3);8,840(6,8);8,837(7,1); 8,828(7,2);8,825(6,9);8,413(0,4);8,385(3,5);8,380(5,1);8,375(3,5);8,365(3,9);8,360(5,4);8,355(3,5);7,995(9,8);7,99 2(12,8);7,973(13,5);7,916(0,4);7,811(0,4);7,640(5,3);7,630(11,1);7,622(5,9);7,620(6,1);7,610(16,0);7,591(9,2);7,5 02(5,2);7,483(7,6);7,465(3,0);4,056(0,5);4,039(1,6);4,021(1,6);4,003(0,5);3,336(114,0);2,677(0,5);2,673(0,6);2,66 9(0,4);2,509(71,7);2,504(91,4);2,500(65,4);2,331(0,6);2,326(0,4);1,990(6,9);1,397(0,9);1,193(1,9);1,176(3,7);1,15 8(1,8);0,000(2,1) |
| Verbindung Nr. 1-3-4: ¹H-NMR(400,0 MHz, d₆-DMSO): 11,888(6,6);9,199(5,7);9,195(5,9);8,801(4,2);8,797(4,5); 8,789(4,5);8,785(4,4);8,434(16,0);8,414(2,5);8,410(3,5);8,405(2,4);8,395(2,6);8,390(3,7);8,385(2,5);8,318(0,7);8,0 09(1,1);8,001(11,6);7,996(3,6);7,984(3,8);7,979(13,2);7,971(1,3);7,675(1,5);7,668(13,5);7,662(3,9);7,650(3,7);7,6 45(11,6);7,638(1,2);7,608(3,4);7,596(3,3);7,588(3,3);7,576(3,1);7,536(0,4);7,473(0,6);4,020(0,4);3,363(0,5);3,330 (303,9);3,309(1,1);2,676(1,5);2,672(2,0);2,667(1,4);2,525(4,0);2,512(114,7);2,507(233,3);2,503(304,7);2,498(214, 8);2,494(100,3);2,334(1,3);2,329(1,9);2,325(1,3);1,989(1,5);1,259(0,4);1,232(0,8);1,193(0,4);1,175(0,8);1,157(0,5 );0,146(1,0);0,008(7,2);0,000(214,6);-0,009(7,2);-0,018(0,7);-0,022(0,5);-0,150(1,0) |
| Verbindung Nr. 1-3-5: ¹H-NMR(400,0 MHz, d₆-DMSO): 12,007(5,7);9,212(5,5);9,208(5,6);9,058(5,3);9,055(5,2); 8,893(5,5);8,888(5,2);8,808(4,0);8,804(4,2);8,796(4,2);8,792(4,1);8,578(16,0);8,429(2,3);8,424(3,3);8,419(2,2);8,4 09(2,5);8,404(3,4);8,399(2,3);7,613(3,0);7,601(2,9);7,593(2,9);7,581(2,8);5,759(2,1);3,332(48,8);2,678(0,4);2,673 (0,5);2,669(0,3);2,527(1,2);2,513(28,6);2,509(57,6);2,504(74,8);2,500(52,8);2,495(24,7);2,336(0,3);2,331(0,4);1,9 |
| 90(1,1);1,352(0,5);1,337(0,7);1,299(2,8);1,259(4,1);1,250(1,3);1,232(2,8);1,193(0,5);1,188(0,5);1,176(0,8);1,158( 0,4);0,868(0,4);0,853(0,5);0,008(2,1);0,000(58,6);-0,009(1,9) |
| Verbindung Nr. 1-3-6: ¹H-NMR(400,0 MHz, d₆-DMSO): 11,472(11,8);9,181(8,5);9,176(8,4);8,847(5,7);8,844(6,0) ;8,835(6,0);8,832(5,8);8,702(7,9);8,698(8,3);8,690(8,3);8,687(8,3);8,447(8,1);8,443(8,1);8,427(8,9);8,423(8,4);8,3 88(3,9);8,383(5,7);8,378(3,9);8,368(4,2);8,363(6,0);8,358(4,0);8,319(0,4);7,858(8,5);7,847(8,2);7,838(7,9);7,826( 7,8);7,643(5,1);7,631(5,1);7,623(5,0);7,612(4,7);7,611(4,7);5,760(16,0);4,057(0,4);4,039(1,1);4,022(1,1);4,004(0,4 );3,335(93,6);2,679(0,4);2,674(0,6);2,670(0,4);2,527(1,6);2,514(35,4);2,510(70,9);2,505(92,7);2,501(66,2);2,497(3 1,4);2,467(0,3);2,336(0,4);2,332(0,6);2,327(0,4);1,991(4,9);1,338(0,8);1,300(2,3);1,260(3,3);1,250(1,4);1,234(3,4) ;1,194(1,4);1,189(0,5);1,176(2,7);1,159(1,3);0,853(0,6);0,840(0,3);0,835(0,4);0,008(0,8);0,000(21,6);-0,008(0,8) |
| Verbindung Nr. 1-3-7: ¹H-NMR(400,0 MHz, d₆-DMSO): 11,881(5,0);9,201(5,1);9,197(5,1);8,800(3,6);8,796(3,8); 8,788(3,9);8,784(3,7);8,665(4,1);8,661(4,3);8,654(4,4);8,650(4,2);8,482(4,2);8,478(4,2);8,460(16,0);8,417(2,1);8,4 13(3,0);8,408(2,1);8,397(2,3);8,393(3,0);8,388(2,1);7,653(4,2);7,641(4,0);7,633(4,0);7,621(4,0);7,606(2,7);7,605( 2,6);7,594(2,7);7,593(2,6);7,586(2,7);7,585(2,5);7,574(2,5);7,573(2,4);7,527(0,4);4,038(0,8);4,020(0,8);3,333(67,0 );3,176(1,0);3,163(0,9);2,673(0,4);2,508(48,8);2,504(62,6);2,499(45,0);2,495(22,0);2,331(0,4);1,990(3,4);1,299(0, 5);1,259(0,7);1,233(0,5);1,193(1,0);1,175(1,9);1,157(0,9);0,008(1,0);0,000(24,3);-0,008(1,1) |
| Verbindung Nr. 1-3-8: ¹H-NMR(400,0 MHz, d₆-DMSO): 11,458(6,5);9,170(7,5);9,165(7,4);8,840(5,0);8,837(5,2); 8,828(5,3);8,825(5,1);8,378(2,8);8,373(4,2);8,368(2,7);8,358(3,0);8,353(4,5);8,348(2,7);8,066(8,9);8,061(9,0);7,96 1(8,6);7,939(10,5);7,757(5,9);7,752(5,6);7,736(4,9);7,730(4,7);7,636(4,0);7,624(4,0);7,616(3,9);7,604(3,7);4,121( 1,2);4,108(3,4);4,095(3,5);4,082(1,2);3,334(45,5);3,177(16,0);3,164(15,5);2,678(0,3);2,674(0,4);2,669(0,3);2,509( 52,1);2,505(66,4);2,501(47,8);2,331(0,4);1,234(0,9);0,008(1,2);0,000(25,3);-0,008(1,0) |
| Verbindung Nr. 1-3-9: ¹H-NMR(400,0 MHz, d₆-DMSO): 11,056(2,4);9,186(2,1);9,182(2,1);8,812(1,5);8,808(1,6); 8,800(1,6);8,796(1,6);8,612(1,8);8,609(1,8);8,601(1,8);8,597(1,8);8,381(1,2);8,361(1,3);8,327(1,7);8,324(1,7);8,30 7(1,9);8,303(1,8);7,708(1,8);7,696(1,8);7,687(1,7);7,676(1,6);7,616(1,2);7,604(1,3);7,596(1,2);7,584(1,1);3,330(4 8,2);2,507(23,4);2,503(29,7);2,499(22,2);2,308(16,0);1,299(0,4);1,259(0,6);0,008(0,5);0,000(11,1) |
| Verbindung Nr. 1-3-10: ¹H-NMR(400,0 MHz, d₆-DMSO): 11,565(1,7);9,167(0,7);8,774(0,6);8,385(0,7);8,381(1,0) ;8,376(0,7);8,365(0,7);8,361(1,1);8,356(0,7);8,023(4,6);7,585(0,8);7,573(0,9);7,565(0,9);7,553(0,8);4,804(0,4);4,7 87(1,1);4,770(1,6);4,754(1,2);4,737(0,5);3,523(0,3);2,514(5,1);2,509(10,4);2,505(13,7);2,500(9,7);2,496(4,6);1,50 7(16,0);1,491(15,7);0,000(3,0) |
| Verbindung Nr. 1-3-11: ¹H-NMR(400,0 MHz, d₆-DMSO): 11,023(2,2);9,188(1,8);9,184(1,9);8,815(1,4);8,811(1,5) ;8,803(1,6);8,799(1,6);8,387(0,7);8,382(1,1);8,377(0,7);8,367(0,8);8,362(1,2);8,357(0,7);7,963(2,2);7,960(2,9);7,9 41(3,0);7,743(0,3);7,741(0,4);7,722(0,4);7,619(1,1);7,607(1,1);7,600(1,1);7,584(2,2);7,579(0,7);7,565(3,2);7,554( 0,4);7,544(2,3);7,426(0,5);7,414(1,1);7,405(0,4);7,396(1,8);7,377(0,8);3,334(9,7);2,526(0,4);2,513(8,4);2,509(17,0 );2,504(22,2);2,500(15,7);2,495(7,3);2,303(16,0);2,187(2,5);0,000(4,5) |
| Verbindung Nr. 1-3-12: ¹H-NMR(400,0 MHz, d₆-DMSO): 11,411(11,9);9,176(10,6);9,172(10,7);9,171(10,0);8,845 (8,0);8,841(8,5);8,833(8,6);8,828(8,4);8,384(4,7);8,379(6,2);8,374(4,7);8,364(5,1);8,358(6,7);8,354(4,9);8,317(0,8 );8,067(10,8);8,063(16,0);8,059(4,2);8,048(8,5);8,045(15,1);8,042(15,0);8,036(1,8);8,004(0,3);7,675(7,7);7,670(3, 3);7,656(16,0);7,653(9,7);7,640(10,8);7,636(12,2);7,630(7,9);7,628(7,0);7,622(6,0);7,620(5,9);7,610(5,8);7,608(5, 8);7,583(4,0);7,580(7,0);7,578(4,2);7,566(3,0);7,562(9,4);7,557(2,6);7,546(2,2);7,543(3,4);7,541(2,0);3,327(208,7 );2,681(0,5);2,677(1,1);2,672(1,5);2,667(1,1);2,663(0,5);2,542(12,0);2,525(3,8);2,521(6,2);2,512(86,7);2,507(177, 0);2,503(234,1);2,498(168,3);2,494(80,0);2,339(0,6);2,334(1,1);2,330(1,6);2,325(1,1);2,104(1,1);0,146(0,9);0,008( 7,3);0,000(214,2);-0,009(7,5);-0,016(0,7);-0,150(0,9) |
| Verbindung Nr. 1-3-13: ¹H-NMR(400,0 MHz, d₆-DMSO): 11,182(11,9);9,180(8,6);9,176(8,6);8,834(7,0);8,830(7,5 );8,822(7,5);8,818(7,4);8,384(3,5);8,379(5,0);8,374(3,5);8,364(3,8);8,359(5,3);8,354(3,5);7,817(6,5);7,812(6,3);7, 801(7,2);7,797(16,0);7,793(8,7);7,782(7,7);7,778(8,8);7,683(1,9);7,677(4,0);7,673(4,4);7,667(1,6);7,658(9,4);7,65 4(6,8);7,639(6,0);7,634(9,7);7,628(7,7);7,624(7,6);7,621(6,5);7,619(7,0);7,611(7,6);7,609(9,0);7,605(8,5);7,601(6, 7);7,595(3,1);7,590(3,1);7,586(2,8);7,519(0,6);7,514(0,4);7,507(3,8);7,501(2,3);7,496(2,5);7,494(2,5);7,490(2,1);7 ,483(3,4);7,471(0,4);5,769(4,7);3,349(0,4);3,332(80,0);3,316(0,4);2,714(0,6);2,678(0,3);2,673(0,5);2,669(0,4);2,56 2(0,6);2,544(154,2);2,527(1,6);2,522(2,2);2,513(28,3);2,509(57,5);2,504(75,9);2,500(54,7);2,495(26,2);2,370(0,6); 2,336(0,4);2,331(0,5);2,327(0,4);0,146(0,3);0,008(2,7);0,000(74,6);-0,009(2,6);-0,150(0,3) |
| Verbindung Nr. 1-3-14: ¹H-NMR(400,0 MHz, d₆-DMSO): 12,080(4,6);9,223(5,7);9,219(5,7);8,997(4,7);8,996(4,8) ;8,993(4,7);8,812(4,1);8,808(4,3);8,800(4,3);8,796(4,2);8,580(16,0);8,492(2,7);8,486(2,7);8,470(3,2);8,465(3,1);8, 438(2,4);8,433(3,3);8,428(2,4);8,418(2,6);8,413(3,5);8,408(2,4);8,317(0,4);8,196(5,2);8,175(4,5);7,618(3,0);7,616 (3,0);7,606(3,0);7,604(3,0);7,598(3,0);7,596(2,9);7,586(2,8);7,584(2,8);3,326(107,7);2,677(0,6);2,672(0,8);2,668( 0,6);2,543(3,8);2,525(2,4);2,512(50,0);2,508(98,4);2,503(127,6);2,499(92,2);2,494(44,7);2,334(0,6);2,330(0,8);2,3 26(0,6);0,146(0,5);0,008(4,1);0,000(103,9);-0,009(4,0);-0,150(0,5) |
| Verbindung Nr. 1-3-15: ¹H-NMR(400,0 MHz, d₆-DMSO): 11,919(5,6);9,199(6,1);9,195(6,0);8,800(4,3);8,796(4,5) ;8,788(4,6);8,784(4,4);8,727(7,8);8,722(8,9);8,661(8,8);8,655(7,5);8,502(16,0);8,416(2,6);8,411(3,6);8,406(2,5);8, 396(2,8);8,391(3,8);8,386(2,6);8,316(0,4);7,606(3,2);7,604(3,2);7,593(3,2);7,592(3,2);7,586(3,1);7,584(3,1);7,574 (3,0);7,572(2,9);3,325(129,9);2,676(0,7);2,672(1,0);2,667(0,7);2,542(9,8);2,525(3,1);2,511(61,2);2,507(117,8);2,5 |
| 03(150,9);2,498(109,1);2,494(53,1);2,338(0,4);2,334(0,8);2,329(1,0);2,325(0,8);0,146(0,5);0,008(5,5);0,000(120,8 );-0,008(5,0);-0,150(0,6) |
| Verbindung Nr. 1-3-16: ¹H-NMR(400,0 MHz, d₆-DMSO): 11,889(6,3);9,202(6,4);9,197(6,5);8,800(4,4);8,796(4,8) ;8,788(4,7);8,784(4,7);8,635(4,9);8,632(5,2);8,624(5,2);8,620(5,1);8,475(16,0);8,418(2,6);8,413(3,9);8,408(2,6);8, 398(2,8);8,393(4,0);8,388(2,7);8,351(4,9);8,347(4,9);8,331(5,3);8,327(5,1);8,317(0,5);7,735(5,3);7,724(5,1);7,715 (5,0);7,703(4,9);7,605(3,4);7,593(3,4);7,586(3,4);7,573(3,2);3,326(123,3);2,676(0,7);2,672(1,0);2,667(0,8);2,542( 6,7);2,525(2,9);2,511(59,3);2,507(118,0);2,503(155,3);2,498(114,9);2,334(0,8);2,329(1,0);2,325(0,8);0,146(0,6);0, 008(4,6);0,000(119,9);-0,008(5,3);-0,150(0,6) |
| Verbindung Nr. 1-3-17: ¹H-NMR(400,0 MHz, d₆-DMSO): 11,089(2,3);9,182(1,9);9,178(1,9);8,813(1,5);8,809(1,6) ;8,801(1,6);8,797(1,6);8,703(3,0);8,697(3,5);8,635(3,4);8,630(2,9);8,382(0,8);8,377(1,1);8,372(0,8);8,362(0,8);8,3 57(1,2);8,352(0,8);7,616(1,1);7,615(1,1);7,604(1,1);7,603(1,1);7,596(1,1);7,595(1,1);7,584(1,0);7,583(1,0);3,327( 19,1);2,542(1,5);2,525(0,6);2,512(11,2);2,508(22,0);2,503(28,5);2,498(20,6);2,494(9,9);2,309(16,0);0,008(1,0);0,0 00(24,8);-0,009(0,9) |
| Verbindung Nr. 1-3-18: ¹H-NMR(400,0 MHz, d₆-DMSO): 11,099(3,1);9,185(2,1);8,818(1,5);8,806(1,6);8,383(1,4) ;8,363(1,5);8,256(1,3);8,236(1,4);8,183(2,5);7,846(0,8);7,826(2,0);7,807(1,5);7,783(2,1);7,764(1,0);7,625(1,3);7,6 13(1,3);7,605(1,3);7,593(1,2);3,340(20,0);2,672(0,3);2,507(38,2);2,503(50,5);2,499(39,9);2,333(16,0);2,210(0,7);0 ,000(8,5) |
| Verbindung Nr. 1-3-19: ¹H-NMR(400,0 MHz, d₆-DMSO): 11,030(2,7);9,173(2,1);9,074(0,5);8,804(1,7);8,795(1,9) ;8,370(1,2);8,350(1,3);8,279(0,4);8,259(0,4);7,954(2,7);7,948(2,8);7,784(0,4);7,754(2,3);7,732(3,6);7,664(2,2);7,6 59(2,1);7,643(1,4);7,637(1,4);7,610(1,3);7,598(1,4);7,591(1,3);7,579(1,3);7,564(0,4);7,551(0,5);7,545(0,5);7,532( 0,4);5,757(0,5);4,038(0,5);4,020(0,5);3,896(0,5);3,326(34,5);2,672(0,3);2,507(40,7);2,503(51,6);2,499(39,2);2,329 (0,4);2,293(16,0);2,224(0,3);2,175(1,3);2,072(0,4);1,989(2,1);1,193(0,6);1,175(1,1);1,157(0,6);0,000(9,2) |
| Verbindung Nr. 1-3-20: ¹H-NMR(400,0 MHz, d₆-DMSO): 11,438(4,7);9,171(7,3);9,167(7,0);8,843(5,0);8,839(5,4) ;8,831(5,3);8,827(5,3);8,379(2,9);8,374(4,2);8,369(2,9);8,359(3,2);8,353(4,5);8,349(3,0);8,317(0,4);8,086(1,5);8,0 78(13,8);8,073(4,6);8,061(4,7);8,056(15,9);8,048(1,7);7,734(1,8);7,727(16,0);7,722(5,0);7,710(4,5);7,704(14,2);7, 697(1,4);7,639(3,9);7,627(3,8);7,619(3,7);7,607(3,5);3,329(289,9);2,996(0,6);2,712(2,1);2,677(0,9);2,672(1,1);2,6 67(0,9);2,583(0,6);2,543(459,5);2,525(3,8);2,512(60,5);2,507(121,0);2,503(159,3);2,498(115,9);2,494(56,5);2,368 (2,0);2,334(0,8);2,330(1,0);2,325(0,8);0,146(0,6);0,008(5,4);0,000(137,2);-0,009(5,1);-0,150(0,6) |
| Verbindung Nr. 1-3-21: ¹H-NMR(400,0 MHz, d₆-DMSO): 11,229(16,0);9,186(11,6);9,181(11,6);8,840(9,0);8,836( 9,4);8,828(9,5);8,824(9,2);8,661(10,2);8,658(10,6);8,650(10,7);8,646(10,5);8,393(11,5);8,390(15,0);8,385(7,5);8,3 80(5,1);8,373(12,9);8,369(15,5);8,365(7,8);8,360(4,9);7,790(11,5);7,778(10,9);7,770(10,6);7,758(10,5);7,639(6,6); 7,638(6,4);7,627(6,5);7,626(6,4);7,619(6,4);7,607(6,1);7,606(5,9);3,333(133,0);2,678(0,4);2,674(0,6);2,669(0,4);2, 544(13,4);2,527(1,6);2,514(33,1);2,509(65,7);2,505(85,4);2,500(60,9);2,496(28,7);2,336(0,4);2,332(0,6);2,327(0,4 );0,146(0,4);0,008(3,0);0,000(80,0);-0,009(2,7);-0,150(0,4) |
| Verbindung Nr. 1-3-22: ¹H-NMR(400,0 MHz, d₆-DMSO): 11,050(2,3);9,177(1,8);9,173(1,8);8,810(1,4);8,806(1,5) ;8,798(1,5);8,794(1,5);8,378(0,7);8,373(1,1);8,369(0,7);8,358(0,8);8,353(1,1);8,349(0,7);7,858(2,5);7,852(2,7);7,7 92(2,6);7,771(3,7);7,676(2,2);7,670(2,0);7,654(1,5);7,648(1,4);7,613(1,2);7,601(1,2);7,593(1,1);7,581(1,1);3,328( 32,3);2,526(0,5);2,512(10,1);2,508(19,8);2,503(25,5);2,499(18,3);2,495(8,8);2,302(16,0);1,990(0,6);1,259(0,4);1,2 34(0,5);1,175(0,3);0,000(2,7) |
| Verbindung Nr. 1-3-23: ¹H-NMR(400,0 MHz, d₆-DMSO): 11,004(2,7);9,176(2,3);9,070(0,4);8,804(1,8);8,795(2,0) ;8,792(1,9);8,375(1,3);8,355(1,4);7,750(1,3);7,743(1,2);7,735(1,0);7,731(1,3);7,727(1,9);7,718(0,5);7,708(1,4);7,7 03(1,1);7,699(1,1);7,692(1,4);7,685(1,8);7,610(1,4);7,598(2,0);7,590(1,7);7,585(2,3);7,579(4,5);7,570(3,5);7,560( 3,0);7,556(1,8);7,542(0,8);7,531(0,4);3,327(19,0);2,503(32,1);2,293(16,0);2,224(0,5);2,072(0,5);0,000(5,5) |
| Verbindung Nr. 1-3-24: ¹H-NMR(400,0 MHz, d₆-DMSO): 11,847(6,7);9,191(4,7);9,187(4,8);8,798(3,1);8,794(3,4) ;8,786(3,4);8,782(3,3);8,500(16,0);8,408(2,5);8,403(3,5);8,398(2,6);8,388(2,7);8,382(3,8);8,378(2,7);8,317(0,4);7, 779(0,9);7,763(1,9);7,757(1,6);7,747(1,2);7,742(3,7);7,736(1,3);7,726(1,7);7,720(2,3);7,704(1,1);7,601(3,1);7,589 (3,1);7,583(3,0);7,581(3,1);7,571(2,9);7,569(3,0);7,501(3,0);7,497(5,6);7,476(9,5);7,455(4,5);7,452(2,8);3,326(10 0,2);2,676(0,6);2,672(0,8);2,667(0,6);2,542(10,4);2,525(2,1);2,512(46,5);2,507(94,5);2,503(125,0);2,498(90,7);2,4 94(44,1);2,334(0,6);2,330(0,8);2,325(0,6);0,146(0,5);0,008(4,0);0,000(110,6);-0,009(4,0);-0,150(0,5) |
| Verbindung Nr. 1-4-2: ¹H-NMR(400,0 MHz, d₆-DMSO): 11,606(2,0);9,140(2,1);9,135(2,1);8,805(1,4);8,801(1,5); 8,793(1,6);8,789(1,5);8,352(0,9);8,348(1,3);8,343(0,9);8,333(1,0);8,327(1,3);8,323(0,9);7,602(1,1);7,601(1,1);7,59 0(1,1);7,589(1,1);7,583(1,1);7,570(1,0);7,569(1,0);4,368(16,0);3,326(4,5);2,508(11,2);2,503(14,2);2,499(10,5);0,0 00(3,2) |
| Verbindung Nr. 1-4-3: ¹H-NMR(400,0 MHz, d₆-DMSO): 11,696(4,8);9,120(4,3);9,115(4,4);8,796(3,1);8,792(3,2); 8,784(3,3);8,780(3,1);8,563(4,2);8,557(4,3);8,332(1,8);8,328(2,6);8,322(1,7);8,312(1,9);8,308(2,7);8,302(1,8);7,93 6(2,6);7,930(2,5);7,915(2,9);7,909(2,9);7,616(5,1);7,595(4,8);7,580(2,4);7,572(2,4);7,560(2,3);6,030(16,0);3,330( 58,9);2,673(0,3);2,526(0,8);2,508(41,4);2,504(53,0);2,499(38,1);2,331(0,3);0,008(0,7);0,000(17,5);-0,008(0,7) |
| Verbindung Nr. 1-4-4: ¹H-NMR(400,0 MHz, d₆-DMSO): 12,471(0,9);9,118(0,8);8,848(0,6);8,837(0,6);8,326(0,5); 8,321(0,7);8,306(0,5);8,301(0,7);8,296(0,4);7,630(0,6);7,618(0,6);7,611(0,6);7,598(0,6);7,220(0,7);7,093(0,7);6,96 |
| 5(0,7);6,454(3,1);3,814(16,0);3,332(20,4);2,507(33,8);2,502(43,8);2,498(32,1);1,260(0,8);1,245(0,5);0,008(1,3);0, 000(32,0);-0,008(1,6) |
| Verbindung Nr. 1-4-5: ¹H-NMR(400,0 MHz, d₆-DMSO): 11,744(3,7);9,148(3,1);9,147(3,2);9,143(3,3);8,809(2,5); 8,805(2,6);8,797(2,6);8,793(2,5);8,361(1,4);8,356(1,9);8,351(1,4);8,341(1,5);8,335(2,0);8,331(1,5);7,606(1,9);7,60 5(1,8);7,594(1,8);7,593(1,8);7,587(1,8);7,585(1,7);7,574(1,7);7,573(1,7);5,829(14,9);4,251(2,2);4,233(7,0);4,215( 7,1);4,198(2,3);3,329(26,3);2,525(0,6);2,512(10,7);2,507(21,5);2,503(28,2);2,498(20,3);2,494(9,6);2,076(0,4);1,25 8(7,9);1,240(16,0);1,222(7,6);0,008(0,4);0,000(13,3);-0,009(0,4) |
| Verbindung Nr. 1-4-6: ¹H-NMR(400,0 MHz, d₆-DMSO): 11,716(5,7);9,156(4,0);9,151(4,1);9,030(2,2);9,011(2,3); 8,880(2,7);8,868(2,7);8,821(2,7);8,818(2,9);8,809(2,9);8,806(2,9);8,480(0,6);8,460(1,3);8,441(0,7);8,397(1,5);8,39 2(2,2);8,388(1,6);8,377(1,7);8,372(2,4);8,368(1,6);7,973(1,7);7,957(2,1);7,954(2,1);7,938(1,5);7,639(2,0);7,627(2, 1);7,619(2,0);7,607(2,0);7,361(16,0);7,354(8,6);7,347(8,2);7,331(1,8);7,286(0,5);7,274(1,2);7,267(1,6);7,260(1,7); 7,253(1,9);7,245(1,4);7,239(1,0);7,232(0,6);5,495(12,6);5,146(0,4);4,973(0,4);4,955(1,6);4,937(2,4);4,918(1,7);4,9 01(0,5);4,137(0,3);4,130(0,3);4,085(0,4);4,062(0,4);4,027(0,4);4,007(0,4);3,988(0,4);3,918(0,4);3,908(0,4);3,862( 0,3);3,843(0,3);3,837(0,3);3,820(0,3);3,793(0,4);2,507(35,7);2,503(45,5);2,499(36,0);2,330(0,3);1,425(9,9);1,407( 10,0);1,379(0,6);0,000(28,5) |
| Verbindung Nr. 1-4-7: ¹H-NMR(400,0 MHz, d₆-DMSO): 11,918(3,7);9,129(5,7);9,124(5,9);8,934(1,7);8,920(3,4); 8,907(1,8);8,848(3,7);8,844(3,9);8,836(4,0);8,832(3,9);8,310(2,2);8,306(3,3);8,301(2,3);8,290(2,4);8,286(3,5);8,28 1(2,4);7,977(3,7);7,957(4,1);7,934(3,8);7,915(4,1);7,839(3,8);7,819(4,2);7,619(2,9);7,607(3,0);7,599(3,0);7,587(2, 7);7,521(1,7);7,504(3,9);7,487(3,2);7,484(3,2);7,479(3,2);7,475(3,2);7,458(3,5);7,441(1,5);7,391(2,8);7,375(5,2);7 ,343(4,3);7,324(4,7);7,306(2,3);5,281(16,0);4,746(7,8);4,733(7,9);3,326(17,4);2,671(0,6);2,506(70,6);2,502(90,7); 2,498(70,6);2,329(0,6);2,074(0,5);0,146(0,4);0,000(73,7);-0,150(0,4) |
| Verbindung Nr. 1-4-8: ¹H-NMR(601,6 MHz, d₆-DMSO): 11,671(2,9);9,140(5,9);9,136(6,2);8,804(4,0);8,801(4,8); 8,796(4,2);8,793(4,8);8,349(2,0);8,345(3,5);8,342(2,6);8,336(2,3);8,332(3,7);8,329(2,7);7,599(3,5);7,591(3,5);7,58 6(3,5);7,578(3,4);6,148(0,9);6,137(1,6);6,131(1,1);6,126(0,6);6,121(2,7);6,110(2,1);6,103(1,0);6,100(1,2);6,093(2, 0);6,082(1,1);5,384(4,5);5,383(4,3);5,367(3,6);5,366(4,5);5,356(16,0);5,350(5,2);5,347(8,5);5,345(7,1);5,327(3,4); 5,325(3,6);3,356(7,6);3,352(15,0);3,347(18,9);3,344(20,3);3,343(20,3);2,523(0,6);2,507(34,7);2,504(49,0);2,502(3 6,9);0,005(1,4);0,000(43,7);-0,006(1,8) |
| Verbindung Nr. 1-4-9: ¹H-NMR(400,0 MHz, d₆-DMSO): 11,621(4,0);9,142(3,8);9,136(3,9);8,805(2,7);8,801(2,8); 8,793(3,0);8,789(2,7);8,353(1,6);8,349(2,3);8,343(1,5);8,333(1,7);8,329(2,4);8,323(1,5);7,603(2,3);7,591(2,2);7,58 3(2,2);7,571(2,1);5,758(1,1);4,654(5,1);4,637(10,4);4,620(5,0);3,333(30,8);3,082(0,7);2,966(0,6);2,527(0,4);2,513 (9,7);2,509(19,0);2,504(24,5);2,500(17,3);2,002(0,6);1,990(1,5);1,985(2,6);1,967(5,3);1,949(5,3);1,931(2,8);1,913 (0,6);1,193(0,3);1,176(0,7);1,158(0,3);0,909(7,9);0,890(16,0);0,872(7,4);0,861(0,4);0,840(0,4);0,594(0,5);0,008(0, 8);0,000(20,3);-0,009(0,7) |
| Verbindung Nr. 1-4-10: ¹H-NMR(400,0 MHz, d₆-DMSO): 11,701(1,6);9,042(4,5);9,037(4,6);8,831(3,1);8,827(3,3) ;8,819(3,3);8,815(3,2);8,270(1,7);8,265(2,6);8,260(1,8);8,250(1,9);8,245(2,8);8,240(1,8);7,621(2,5);7,609(2,5);7,6 01(2,4);7,589(2,3);7,371(0,5);7,366(0,4);7,359(1,2);7,348(4,7);7,343(4,6);7,335(9,7);7,331(10,1);7,254(5,1);7,247 (4,2);7,235(3,8);7,231(3,0);5,613(16,0);3,333(22,2);2,672(0,4);2,507(48,6);2,502(62,4);2,498(45,6);2,329(0,4);0,0 00(6,7) |
| Verbindung Nr. 1-4-11: ¹H-NMR(400,0 MHz, d₆-DMSO): 11,681(6,2);9,142(4,7);9,138(4,7);8,806(3,2);8,802(3,4) ;8,794(3,4);8,790(3,2);8,464(1,3);8,452(2,3);8,439(1,3);8,358(2,0);8,353(2,8);8,348(2,0);8,338(2,2);8,333(2,9);8,3 28(1,9);7,605(2,5);7,593(2,5);7,585(2,5);7,573(2,3);5,410(16,0);3,352(12,7);3,149(2,2);3,132(5,1);3,117(5,1);3,10 0(2,3);2,507(27,0);2,503(34,1);2,499(25,8);1,470(0,8);1,454(2,6);1,446(1,5);1,436(4,0);1,417(3,6);1,399(1,5);1,36 1(0,8);1,344(2,7);1,325(3,7);1,305(3,6);1,288(2,0);1,270(0,6);0,906(8,2);0,888(15,8);0,870(6,8);0,008(3,4);0,000( 38,9) |

| |
|---|
| ²) Die NMR-Daten ausgewählter Beispiele werden entweder in klassischer Form (δ-Werte, Anzahl der H-Atome, Multiplettaufspaltung) oder als NMR-Peak-Listen aufgeführt. |

### NMR-Peak-Listenverfahren

Wenn die 1H-NMR-Daten ausgewählter Beispiele in Form von 1H-NMR-Peaklisten notiert werden, wird zu jedem Signalpeak erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet. Die Peakliste eines Beispieles hat daher die Form: δ₁ (Intensität₁); δ₂ (Intensität₂);........; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht. Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden. Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen. Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen. Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation. Weitere Details zu 1H-NMR-Peaklisten können der Research Disclosure Database Number 564025 entnommen werden.

Die NMR-Spektren wurden mit einem Bruker Avance 400, ausgestattet mit einem Durchflussprobenkopf (60 µl Volumen), bestimmt. Als Lösungsmittel wurden CD₃CN, CDCl₃, DMF-D₇ oder DMSO-D₆ verwendet, wobei als Referenz Tetramethylsilan (0.00 ppm) eingesetzt wurde. In Einzelfällen wurden die NMR Spektren mit einem Bruker Avance II 600 bestimmt. Bei NMR-Daten in der klassischer Form wurde die Aufstpaltung der Signale wie folgt beschrieben: s (Singulett), d (Duplett), t (Triplett), q (Quartett), m (Multiplett).

### Biologische Beispiele

Die folgenden Beispiele zeigen die biologische Wirkung der erfindungsgemäßen Verbindungen. Dabei beziehen sich die genannten erfindungsgemäßen Verbindungen auf die in den Tabellen 1 bis 4 mit den entsprechenden Referenzzeichen, z. B. I-1-35, aufgeführten Verbindungen:

**Myzus persicae - Sprühtest**

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100% bei einer Aufwandmenge von 500 g/ha: I-1-2, I-1-4, I-1-9, I-1-18, I-1-19, I-1-22, I-1-23, I-1-25, I-1-33, I-1-34, I-1-38, I-1-44, I-1-46, I-1-60, I-1-63, I-1-147, I-1-153, I-1-163, I-1-166, I-1-169, I-1-173 ,I-1-174, I-1-175, I-1-176, I-1-178, I-1-179, I-1-180, I-1-182, I-1-185, I-1-186, I-1-190, I-1-192, I-1-205, I-1-214, I-1-218, I-1-223, I-1-224, I-1-225, I-1-226, I-1-242, I-1-243, I-1-245, I-1-246, I-1-261, I-1-268, I-1-277, I-1-283, I-1-286, I-1-292, I-1-295, I-1-304, I-1-310, I-1-321, I-1-344, I-1-346, I-2-7

Bei diesem Test zeigten z. B. die folgenden Verbindungen der Herstellungsbeispiele Wirkung von 90% bei einer Aufwandmenge von 500 g/ha: I-1-1, I-1-3, I-1-5, I-1-6, I-1-7, I-1-8, I-1-10, I-1-13, I-1-15, I-1-16, I-1-17, I-1-20, I-1-26, I-1-27, I-1-28, I-1-30, I-1-31, I-1-32, I-1-36, I-1-39, I-1-40, I-1-41, I-1-42, I-1-43, I-1-46, I-1-50, I-1-51, I-1-52, I-1-54, I-1-64, I-1-65, I-1-123, I-1-124, I-1-126, I-1-128, I-1-129, I-1-130, I-1-131, I-1-133, I-1-140, I-1-143, I-1-144, I-1-148, I-1-149, I-1-154, I-1-155, I-1-156, I-1-164, I-1-167, I-1-170, I-1-172, I-1-181, I-1-183, I-1-184, I-1-187, I-1-188, I-1-189, I-1-191, I-1-196, I-1-198, I-1-202, I-1-203, I-1-204, I-1-211, I-1-212, I-1-215, I-1-216, I-1-220, I-1-221, I-1-227, I-1-228, I-1-229, I-1-231, I-1-233, I-1-234, I-1-237, I-1-239, I-1-244, I-1-259, I-1-260, I-1-262, I-1-264, I-1-266, I-1-279, I-1-281, I-1-282, I-1-285, I-1-287, I-1-289, I-1-291, I-1-293, I-1-296, I-1-297, I-1-298, I-1-308, I-1-311, I-1-312, I-1-313, I-1-314, I-1-317, I-1-318, I-1-320, I-1-329, I-1-330, I-1-333, I-1-335, I-1-338, I-1-342, I-1-345, I-1-352, I-2-4, I-2-16

Bei diesem Test zeigten z. B. die folgenden Verbindungen der Herstellungsbeispiele Wirkung von 80% bei einer Aufwandmenge von 500 g/ha: I-1-11, I-1-12, I-1-14, I-1-49, I-1-55, I-1-61

**Phaedon cochleariae - Sprühtest (PHAECO)**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100% bei einer Aufwandmenge von 500 g/ha: I-1-48, I-1-130, I-1-131, I-1-346

Bei diesem Test zeigten z. B. die folgenden Verbindungen der Herstellungsbeispiele Wirkung von 83% bei einer Aufwandmenge von 500 g/ha: I-1-22

**Spodoptera frugiperda - Sprühtest (SPODFR)**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigten z. B. die folgenden Verbindungen der Herstellungsbeispiele Wirkung von 100% bei einer Aufwandmenge von 500 g/ha: I-1-48, I-1-130, I-1-131

Bei diesem Test zeigten z. B. die folgenden Verbindungen der Herstellungsbeispiele Wirkung von 83% bei einer Aufwandmenge von 500 g/ha: I-1-22

**Tetranychus urticae - Sprühtest, OP-resistent (TETRUR)**

| | | |
|---|---|---|
| Lösungsmittel: | 78,0 | GewichtsteileAceton |
| | 1,5 | Gewichtsteile Dimethylformamid |
| Emulgator : | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris)*, die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 90% bei einer Aufwandmenge von 500 g/ha: I-1-131, I-1-205, I-1-210, I-1-282

**Myzus persicae - Sachettest**

| | |
|---|---|
| Lösungsmittel: | 2 Vol % N-Methylpyrrolidon |
| | 2 Vol % Diacetonalkohol |

Zur Herstellung einer zweckmäßigen Wirkstoffzubereitung vermischt man den Wirkstoff mit den angegebenen Mengen Lösungsmittel und verdünnt das Konzentrat mit Wasser auf die gewünschte Konzentration.

Zu einem speziellen Nährmedium wird durch pipettieren die Wirkstoffzubereitung in der gewünschten Konzentration zugegeben. Diese Platte wird verschlossen und mit einer weiteren, die mit der Grünen Pfirsichblattlaus (*Myzus persicae*) bestückt ist, kombiniert.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigten z. B. die folgenden Verbindungen der Herstellungsbeispiele Wirkung von 100% bei einer Aufwandmenge von 30 ppm: I-1-1, I-1-2, I-1-3, I-1-4, I-1-5, I-1-6, I-1-7, I-1-8, I-1-9, I-1-10, I-1-11, I-1-12, I-1-13, I-1-14, I-1-15, I-1-16, I-1-17, I-1-18, I-1-19, I-1-20, I-1-21, I-1-22, I-1-23, I-1-24, I-1-25, I-1-26, I-1-27, I-28, I-1-29, I-1-30, I-1-31, I-1-32, I-1-33, I-1-34, I-1-35, I-1-36, I-1-37, I-1-38, I-1-39, I-1-40, I-1-42, I-1-43, I-1-44, I-1-45, I-1-46, I-1-48, I-1-49, I-1-50, I-1-51, I-1-52, I-1-53, I-1-54, I-1-55, I-1-56, I-1-57, I-1-58, I-1-60, I-1-61, I-1-62, I-1-63, I-1-64, I-1-65, I-1-66, I-1-67, I-1-68, I-1-69, I-1-70, I-1-71, I-1-72, I-1-73, I-1-74, I-1-75, I-1-76, I-1-77, I-1-78, I-1-79, I-1-80, I-1-81, I-1-82, I-1-83, I-1-84, I-1-85, I-1-86, I-1-87, I-1-88, I-1-89, I-1-90, I-1-91, I-1-92, I-1-93, I-1-95, I-1-99, I-1-100, I-1-108, I-1-111, I-1-116, I-1-119, I-1-120, I-1-122, I-1-123, I-1-124, I-1-125, I-1-126, I-1-128, I-1-129, I-1-130, I-1-131, I-1-132, I-1-133, I-1-135, I-1-136, I-1-138, I-1-139, I-1-140, I-1-142, I-1-143, I-1-144, I-1-145, I-1-146, I-1-147, I-1-148, I-1-149, I-1-153, I-1-154, I-1-155, I-1-156, I-1-157, I-1-158, I-1-159, I-1-160, I-1-161, I-1-162, I-1-163, I-1-164, I-1-165, I-1-166, I-1-167, I-1-168, I-1-169, I-1-170, I-1-171, I-1-172, I-1-174, I-1-175, I-1-177, I-1-180, I-1-182, I-1-183, I-1-184, I-1-185, I-1-186, I-1-187, I-1-188, I-1-189, I-1-190, I-1-191, I-1-192, I-1-194, I-1-195, I-1-196, I-1-197, I-1-198, I-1-199, I-1-202, I-1-203, I-1-205, I-1-207, I-1-208, I-1-209, I-1-211, I-1-212, I-1-213, I-1-214, I-1-215, I-1-216, I-1-217, I-1-218, I-1-219, I-1-220, I-1-221, I-1-223, I-1-224, I-1-225, I-1-226, I-1-227, I-1-228, I-1-229, I-1-231, I-1-232, I-1-233, I-1-234, I-1-235, I-1-236, I-1-237, I-1-238, I-1-239, I-1-241, I-1-242, I-1-243, I-1-244, I-1-245, I-1-246, I-1-247, I-1-249, I-1-257, I-1-259, I-1-260, I-1-261, I-1-262, I-1-263, I-1-264, I-1-265, I-1-266, I-1-267, I-1-268, I-1-269, I-1-274, I-1-276, I-1-277, I-1-278, I-1-279, I-1-280, I-1-281, I-1-282, I-1-283, I-1-284, I-1-285, I-1-286, I-1-287, I-1-288, I-1-289, I-1-290, I-1-291, I-1-292, I-1-293, I-1-294, I-1-295, I-1-296, I-1-297, I-1-298, I-1-299, I-1-300, I-1-301, I-1-302, I-1-304, I-1-305, I-1-306, I-1-307, I-1-308, I-1-309, I-1-310, I-1-311, I-1-312, I-1-313, I-1-314, I-1-315, I-1-316, I-1-317, I-1-318, I-1-319, I-1-320, I-1-321, I-1-322, I-1-323, I-1-324, I-1-325, I-1-326, I-1-327, I-1-328, I-1-329, I-1-330, I-1-331, I-1-332, I-1-333, I-1-334, I-1-335, I-1-336, I-1-338, I-1-339, I-1-340, I-1-341, I-1-342, I-1-344, I-1-345, I-1-346, I-1-347, I-1-349, I-1-351, I-1-352, I-1-353, I-2-1, I-2-4, I-2-6, I-2-14, I-2-16, I-2-17, I-2-19, I-2-23, I-2-26, I-3-1, I-3-2, I-3-5, I-3-6, I-3-7, I-3-9, I-3-10, I-3-14, I-3-16, I-4-1, I-4-2, I-4-3, I-4-6, I-4-8

Bei diesem Test zeigten z. B. die folgenden Verbindungen der Herstellungsbeispiele Wirkung von 80% bei einer Aufwandmenge von 30ppm: I-1-104, I-1-173, I-1-176, I-1-178, I-1-181, I-1-204, I-1-210, I-1-270, I-1-348, I-1-350, I-2-18, I-2-22

Bei diesem Test zeigten z. B. die folgenden Verbindungen der Herstellungsbeispiele Wirkung von 100% bei einer Aufwandmenge von 6 ppm: I-1-1, I-1-2, I-1-3, I-1-4, I-1-5, I-1-6, I-1-7, I-1-8, I-1-9, I-1-10, I-1-11, I-1-12, I-1-13, I-1-14, I-1-15, I-1-17, I-1-18, I-1-19, I-1-20, I-1-21, I-1-22, I-1-23, I-1-24, I-1-25, I-1-26, I-1-27, I-1-28, I-1-29, I-1-30, I-1-31, I-1-32, I-1-33, I-1-34, I-1-36, I-1-37, I-1-38, I-1-39, I-1-40, I-1-43, I-1-44, I-1-45, I-1-46, I-1-49, I-1-51, I-1-52, I-1-53, I-1-54, I-1-57, I-1-60, I-1-62, I-1-64, I-1-65, I-1-67, I-1-72, I-1-74, I-1-76, I-1-77, I-1-84, I-1-85, I-1-88, I-1-89, I-1-90, I-1-111, I-1-116, I-1-120, I-1-122, I-1-123, I-1-124, I-1-126, I-1-128, I-1-129, I-1-130, I-1-131, I-1-133, I-1-139, I-1-140, I-1-142, I-1-143, I-1-144, I-1-146, I-1-147, I-1-148, I-1-149, I-1-153, I-1-154, I-1-155, I-1-156, I-1-159, I-1-160, I-1-162, I-1-163, I-1-164, I-1-165, I-1-166, I-1-167, I-1-168, I-1-169, I-1-170, I-1-172, I-1-174, I-1-175, I-1-182, I-1-183, I-1-184, I-1-185, I-1-186, I-1-187, I-1-188, I-1-189, I-1-190, I-1-191, I-1-192, I-1-195, I-1-196, I-1-197, I-1-198, I-1-207, I-1-211, I-1-212, I-1-214, I-1-215, I-1-216, I-1-217, I-1-218, I-1-219, I-1-220, I-1-221, I-1-223, I-1-224, I-1-225, I-1-226, I-1-227, I-1-228, I-1-229, I-1-231, I-1-233, I-1-234, I-1-236, I-1-237, I-1-238, I-1-239, I-1-241, I-1-242, I-1-243, I-1-244, I-1-245, I-1-246, I-1-247, I-1-259, I-1-260, I-1-261, I-1-262, I-1-263, I-1-264, I-1-265, I-1-266, I-1-268, I-1-269, I-1-276, I-1-277, I-1-279, I-1-282, I-1-283, I-1-284, I-1-285, I-1-286, I-1-287, I-1-288, I-1-289, I-1-290, I-1-291, I-1-292, I-1-293, I-1-295, I-1-296, I-1-298, I-1-299, I-1-300, I-1-302, I-1-304, I-1-307, I-1-308, I-1-309, I-1-310, I-1-312, I-1-313, I-1-314, I-1-315, I-1-316, I-1-317, I-1-318, I-1-319, I-1-320, I-1-321, I-1-322, I-1-323, I-1-324, I-1-325, I-1-326, I-1-327, I-1-328, I-1-329, I-1-330, I-1-331, I-1-332, I-1-333, I-1-334, I-1-335, I-1-336, I-1-338, I-1-340, I-1-341, I-1-342, I-1-344, I-1-345, I-1-346, I-1-353, I-3-1, I-3-14, I-4-3, I-4-6, I-4-8

Bei diesem Test zeigten z. B. die folgenden Verbindungen der Herstellungsbeispiele Wirkung von 80% bei einer Aufwandmenge von 6 ppm: I-1-42, I-1-58, I-1-80, I-1-104, I-1-161, I-1-171, I-1-173, I-1-176, I-1-180, I-1-202, I-1-203, I-1-204, I-1-209, I-1-235, I-1-280, I-1-281, I-1-347, I-3-7

## Patentansprüche

1. Nicht-therapeutische Verwendung von Verbindungen der Formel (I) 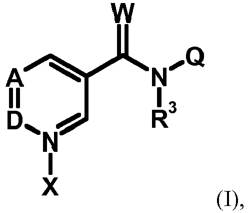 worin
A für N oder CR¹ steht,
D für N oder CR² steht, wobei A und D nicht gleichzeitig für N stehen,
R¹ für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
R² für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
X für ein freies Elektronenpaar oder Sauerstoff steht,
W für Sauerstoff, Schwefel oder NR⁴ steht,
R³ für einen Rest aus der Reihe Wasserstoff, Cyano, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl, C₁-C₆-Alkylcarbonyloxy, C₁-C₆-Alkoxycarbonyloxy, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-C₁-C₆-alkylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
R⁴ für einen Rest aus der Reihe Wasserstoff, Cyano, Nitro, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₆-Alkylsulfonyl, C₁-C₆-Halogenalkylsulfonyl, Aryl, Hetaryl, Aryl-C₁-C₆-alkyl, Hetaryl-C₁-C₆-alkyl, Arylcarbonyl, Hetarylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
Q für einen der Reste Q-1 bis Q-4 steht, 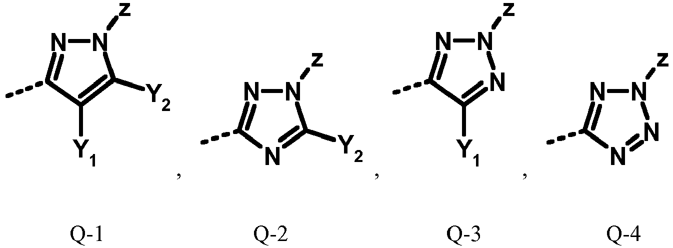 worin die gestrichelte Linie die Bindung zum Stickstoff in der Gruppe NR³ bedeutet,
Y₁ für einen Rest aus der Reihe Wasserstoff, Halogen, Cyano, Nitro, Amino, C₁-C₆-Alkylamino, Di-(C₁-C₆)-alkylamino, Hydroxy, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Y₂ für einen Rest aus der Reihe Wasserstoff, Halogen, Cyano, Nitro, Amino, C₁-C₆-Alkylamino, Di-(C₁-C₆)-alkylamino, Hydroxy, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht, oder
im Falle von Q = Q-1 Y₁ und Y₂ zusammen mit den Kohlenstoffatomen, an die sie gebunden sind, einen Phenylring oder einen 5- bis 7-gliedrigen heteroaromatischen Ring ausbilden, welcher gegebenenfalls durch einen oder mehrere, gleiche oder verschiedene Substituenten M¹ substituiert ist,
M¹ für Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl und C₁-C₆-Alkoxy-C₁-C₆-alkyl steht,
Z für einen Rest aus der Reihe Wasserstoff, Cyano, Nitro, L¹(=G)C*, L¹O(=G)C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach,
gleich oder verschieden durch M² substituiertes C₁-C₈-Alkyl, C₃-C₈-Cycloalkyl, C₂-C₈-Alkenyl, C₅-C₈-Cycloalkenyl, C₃-C₈-Alkinyl, für gegebenenfalls einfach oder mehrfach, gleich oder verschieden durch M⁶ substituiertes L⁵C(=E)L²N(=G)C-C₁-C₈-alkyl*, L⁵(=O)ₙSL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl* steht,
M² für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆-Alkyl, C₃-C₆-Cycloalkyl, C₂-C₆-Alkenyl, C₅-C₆-Cycloalkenyl, C₃-C₆-Alkinyl, C₁-C₆-Alkoxy steht,
M³ für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C* steht,
G und E unabhängig voneinander für Sauerstoff oder Schwefel stehen,
n für 1 oder 2 steht,
L¹ und L⁵ unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁴ für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹ steht,
L² für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl steht,
L³ und L⁴ unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁵ für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹ steht,
M⁶ für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₅-C₆-Cycloalkenyl, C₃-C₆-Alkinyl, C₁-C₆-Alkoxy-C₁-C₆-alkyl, C₁-C₆-Halogenalkoxy-C₁-C₆-alkyl steht,
L⁹ und L¹⁰ unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₆-Alkenyl, C₂-C₆-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl stehen,
worin die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₂-C₆-Alkenyl, C₃-C₆-Alkinyl, C₃-C₆-Cycloalkyl, C₁-C₆-Halogenalkyl, Cyano-C₁-C₆-alkyl, C₂-C₆-Halogenalkenyl, C₃-C₆-Halogenalkinyl, C₁-C₆-Alkoxy, C₂-C₆-Alkenyloxy, C₃-C₆-Alkinyloxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkylcarbonylamino, CO₂H, SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert sind,
und von deren Salzen und N-Oxiden zur Bekämpfung von tierischen Schädlingen.

2. Verbindungen der Formel (I) 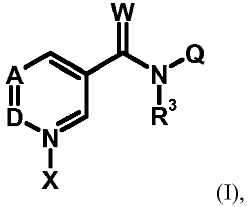 worin
A für N oder CR¹ steht,
D für N oder CR² steht, wobei A und D nicht gleichzeitig für N stehen,
R¹ für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
R² für einen Rest aus der Reihe Wasserstoff, Halogen, Cyano, Nitro, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl steht,
X für ein freies Elektronenpaar oder Sauerstoff steht,
W für Sauerstoff, Schwefel oder NR⁴ steht,
R³ für einen Rest aus der Reihe Wasserstoff, Cyano, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxy-C₁-C₆-alkylcarbonyl, C₁-C₆-Alkoxycarbonyl, C₁-C₆-Halogenalkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl, C₁-C₆-Alkylcarbonyloxy, C₁-C₆-Alkoxycarbonyloxy, Aryl, Hetaryl, Arylcarbonyl, Hetarylcarbonyl, Aryl-C₁-C₆-alkyl, Hetaryl-C₁-C₆-alkyl, Aryl-C₁-C₆-alkylcarbonyl, Hetaryl-C₁-C₆-alkylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl, Aryl-C₁-C₆-alkylcarbonyl, Hetaryl-C₁-C₆-alkylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
R⁴ für einen Rest aus der Reihe Wasserstoff, Cyano, Nitro, Hydroxy, C₁-C₆-Alkyl, C₃-C₆-Cycloalkyl, C₁-C₆-Halogenalkyl, C₃-C₆-Halogencycloalkyl, C₂-C₆-Alkenyl, C₂-C₆-Halogenalkenyl, C₃-C₆-Alkinyl, C₃-C₆-Halogenalkinyl, C₃-C₆-Cycloalkyl-C₁-C₆-alkyl, C₃-C₆-Halogencycloalkyl-C₁-C₆-alkyl, Cyano-C₁-C₆-alkyl, C₁-C₆-Alkoxy, C₁-C₆-Halogenalkoxy, C₁-C₆-Alkoxy-C₁-C₆-alkyl, C₁-C₆-Halogenalkoxy-C₁-C₆-alkyl, C₁-C₆-Alkylcarbonyl, C₁-C₆-Halogenalkylcarbonyl, C₁-C₆-Alkoxycarbonyl, C₁-C₆-Alkylcarbonyloxy, C₁-C₆-Alkylsulfonyl, C₁-C₆-Halogenalkylsulfonyl, Aryl, Hetaryl, Aryl-C₁-C₆-alkyl, Hetaryl-C₁-C₆-alkyl, Arylcarbonyl, Hetarylcarbonyl steht, wobei die Substituenten Aryl, Hetaryl, Aryl-C₁-C₆-alkyl, Hetaryl-C₁-C₆-alkyl, Arylcarbonyl, Hetarylcarbonyl gegebenenfalls einfach oder mehrfach, gleich oder unterschiedlich durch Halogen, Cyano, Nitro, Hydroxy, C₁-C₆-Alkyl, C₂-C₆-Alkenyl, C₃-C₆-Alkinyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₁-C₆-Alkoxy-C₁-C₆-alkyl substituiert sind,
Q für einen der Reste Q-1 bis Q-4 steht, 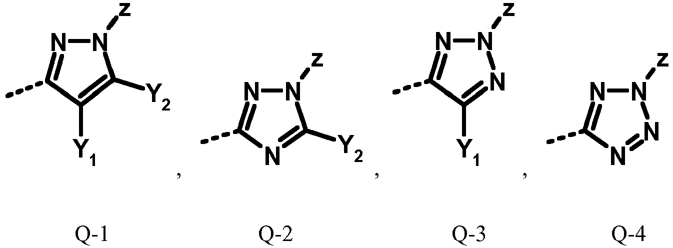 worin die gestrichelte Linie die Bindung zum Stickstoff in der Gruppe NR³ bedeutet,
Y₁ für einen Rest aus der Reihe Wasserstoff, Halogen, Cyano, Nitro, C₁-C₆-Alkylamino, Di-(C₁-C₆)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Y₂ für einen Rest aus der Reihe Wasserstoff, Halogen, Cyano, Nitro, C₁-C₆-Alkylamino, Di-(C₁-C₆)-alkylamino, COOH, C₁-C₆-Alkyl, C₃-C₆-Cycloalkyl, C₁-C₆-Halogenalkyl, C₁-C₆-Alkoxy, C₁-C₆-Halogenalkoxy, C₃-C₆-Halogencycloalkyl, C₃-C₆-Halogencycloalkyl-C₁-C₆-alkyl, C₁-C₆-Alkoxy-C₁-C₆-alkyl, C₁-C₆-Halogenalkoxy-C₁-C₆-alkyl, C₁-C₆-Alkoxy-C₁-C₆-alkyloxy, Cyano-C₁-C₆-alkyl, C₃-C₆-Cycloalkyl-C₁-C₆-alkyl, C₂-C₆-Alkenyl, C₃-C₆-Alkinyl, SH, C₁-C₆-Alkylthio, C₁-C₆-Alkylcarbonyl, C₃-C₆-Cycloalkylcarbonyl, C₂-C₆-Alkenylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, C₁-C₆-Alkylaminocarbonyl, Di-(C₁-C₆)-alkylaminocarbonyl steht,
Z für einen Rest aus der Reihe Wasserstoff, Cyano, Nitro, L¹(=G)C*, L¹O(=G)C*, L³L⁴N(=G)C*, L⁵(=E)CL²N(=G)C*, L⁵S(=O)ₙL²N(=G)C*, L⁵O(=E)CL²N(=G)C*, L¹L⁵N(=E)CL²N(=G)C*, L¹(=O)ₙS*, L³L⁴N(=O)ₙS*, Heterocyclyl, Aryl, Hetaryl, für gegebenenfalls einfach oder mehrfach, gleich oder verschieden durch M² substituiertes C₁-C₈-Alkyl, C₃-C₈-Cycloalkyl, C₂-C₈-Alkenyl, C₅-C₈-Cycloalkenyl, C₃-C₈-Alkinyl, für gegebenenfalls einfach oder mehrfach, gleich oder verschieden durch M⁶ substituiertes L⁵C(=E)L²N(=G)C-C₁-C₈-alkyl*, L⁵S(=O)ₙL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl* steht,
mit der Maßgabe, dass, wenn Q für Q-4 steht, Z nicht gleichzeitig für Wasserstoff oder unsubstituiertes C₁-C₈-Alkyl steht, und mit der Maßgabe, dass Z nicht für Wasserstoff steht, wenn W für NOH steht, und mit der Maßgabe, dass, wenn Q für Q-2 steht, Z nicht gleichzeitig für Wasserstoff steht,
M² für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, gegebenenfalls einfach oder mehrfach, gleich oder verschieden durch M³ substituiertes C₁-C₆-Alkyl, C₃-C₆-Cycloalkyl, C₂-C₆-Alkenyl, C₅-C₆-Cycloalkenyl, C₃-C₆-Alkinyl, C₁-C₆-Alkoxy steht,
M³ für Halogen, Cyano, Nitro, Heterocyclyl, Aryl, Hetaryl, NL⁹L¹⁰, OL⁹, SL⁹, C₁-C₆-Alkyl, C₁-C₆-Halogenalkyl, C₃-C₆-Cycloalkyl, C₃-C₆-Halogencycloalkyl, C₂-C₆-Alkenyl, C₃-C₆-Alkinyl, C₁-C₆-Alkoxy, C₁-C₆-Halogenalkoxy, L¹(=G)C*, L³O(=G)C*, L³L⁴N(=G)C* steht,
G und E unabhängig voneinander für Sauerstoff oder Schwefel stehen,
n für 1 oder 2 steht,
L¹ und L⁵ unabhängig voneinander für Heterocyclyl, Aryl, Hetaryl, gegebenenfalls einfach oder mehrfach, gleich oder verschieden durch M⁴ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁴ für Halogen, Cyano, Nitro, Heterocyclyl, Aryl, Hetaryl, C₁-C₆-Halogenalkyl, C₁-C₆-Alkoxy, C₁-C₆-Halogenalkoxy, NL⁹L¹⁰, OL⁹, SL⁹ steht,
L² für Wasserstoff, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl steht,
L³ und L⁴ unabhängig voneinander für Wasserstoff, Heterocyclyl, Aryl, Hetaryl, gegebenenfalls einfach oder mehrfach, gleich oder verschieden durch M⁵ substituiertes C₁-C₆-Alkyl, C₃-C₆-Cycloalkyl, C₃-C₆-Cycloalkyl-C₁-C₆-alkyl, C₂-C₆-Alkenyl, C₃-C₆-Alkinyl stehen,
M⁵ für Halogen, Cyano, Nitro, Heterocyclyl, Aryl, Hetaryl, C₁-C₆-Halogenalkyl, C₁-C₆-Alkoxy, C₁-C₆-Halogenalkoxy, C₁-C₆-Alkoxycarbonyl, NL⁹L¹⁰, OL⁹, SL⁹ steht,
M⁶ für Halogen, Cyano, Nitro, NL⁹L¹⁰, OL⁹, SL⁹, L¹(=G)C*, L³O(=G)C*, L³L⁴N(=G)C*, Heterocyclyl, Aryl, Hetaryl, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, C₂-C₆-Alkenyl, C₅-C₆-Cycloalkenyl, C₃-C₆-Alkinyl, C₁-C₆-Alkoxy-C₁-C₆-alkyl, C₁-C₆-Halogenalkoxy-C₁-C₆-alkyl steht,
L⁹ und L¹⁰ unabhängig voneinander für Wasserstoff, C₁-C₆-Alkyl, C₁-C₆-Halogenalkyl, C₃-C₆-Cycloalkyl, C₃-C₆-Halogencycloalkyl, C₃-C₆-Cycloalkyl-C₁-C₆-alkyl, C₃-C₆-Halogencycloalkyl-C₁-C₆-alkyl, Cyano-C₁-C₆-alkyl, C₂-C₆-Alkenyl, C₂-C₆-Halogenalkenyl, C₃-C₆-Alkinyl, C₁-C₆-Alkoxy-C₁-C₆-alkyl, C₁-C₆-Halogenalkoxy-C₁-C₆-alkyl, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, Aminocarbonyl, C₁-C₆-Alkylaminocarbonyl, Di-(C₁-C₆)-alkylaminocarbonyl, C₃-C₆-Cycloalkylaminocarbonyl, C₃-C₆-Cycloalkyl-C₁-C₆-alkylaminocarbonyl, Aryl, Hetaryl, Heterocyclyl, Aryl-C₁-C₆-alkyl, Hetaryl-C₁-C₆-alkyl stehen,
wobei, sofern nichts anderes angegeben ist, die Reste Heterocyclyl, Aryl und Hetaryl (auch als Teil einer größeren Einheit wie Aryloxy, Hetarylalkyl usw.) gegebenenfalls einfach oder mehrfach, gleich oder verschieden durch Halogen, Cyano, Nitro, SH, Hydroxy, C₁-C₆-Alkyl, C₂-C₆-Alkenyl, C₃-C₆-Alkinyl, C₃-C₆-Cycloalkyl, C₁-C₆-Halogenalkyl, Cyano-C₁-C₆-alkyl, C₂-C₆-Halogenalkenyl, C₃-C₆-Halogenalkinyl, C₁-C₆-Alkoxy, C₂-C₆-Alkenyloxy, C₃-C₆-Alkinyloxy, C₁-C₆-Halogenalkoxy, C₃-C₆-Halogencycloalkyl, C₁-C₆-Alkoxy-C₁-C₆-alkyl, C₁-C₆-Halogenalkoxy-C₁-C₆-alkyl, C₃-C₆-Cycloalkyl-C₁-C₆-alkyl, C₃-C₆-Halogencycloalkyl-C₁-C₆-alkyl, Amino, C₁-C₆-Alkylamino, Di-(C₁-C₆)-alkylamino, C₃-C₆-Cycloalkylamino, C₃-C₆-Cycloalkyl-C₁-C₆-alkylamino, C₁-C₆-Alkylcarbonylamino, C₁-C₆-Halogenalkylcarbonylamino, C₁-C₆-Cycloalkylcarbonylamino, CO₂H, SO₃H, CONH₂, SO₂NH₂, C₁-C₆-Alkylcarbonyl, C₂-C₆-Alkenylcarbonyl, C₃-C₆-Alkinylcarbonyl, C₁-C₆-Halogenalkylcarbonyl, C₃-C₆-Cycloalkylcarbonyl, C₃-C₆-Cycloalkyl-C₁-C₆-alkylcarbonyl, C₂-C₆-Halogenalkenylcarbonyl, C₃-C₆-Halogencycloalkylcarbonyl, C₁-C₆-Alkoxycarbonyl, C₁-C₆-Alkoxy-C₁-C₆-alkylcarbonyl, C₁-C₆-Halogenalkoxycarbonyl, C₃-C₆-Cycloalkoxycarbonyl, C₁-C₆-Alkylthio, C₁-C₆-Halogenalkylthio, C₁-C₆-Alkylsulfonyl, C₁-C₆-Halogenalkylsulfinyl, C₁-C₆-Alkylsulfonyl, C₁-C₆-Halogenalkylsulfonyl, C₁-C₆-Alkylaminosulfonyl, C₁-C₆-Halogenalkylaminosulfonyl, Di-(C₁-C₆)-alkylaminosulfonyl, C₁-C₆-Alkylaminocarbonyl, Di-(C₁-C₆)-alkylaminocarbonyl, C₁-C₆-Alkylcarbonyloxy, C₁-C₆-Halogenalkylcarbonyloxy, C₁-C₆-Alkoxycarbonyloxy, C₁-C₆-Halogenalkoxycarbonyloxy, Aryl, Hetaryl, Aryloxy, Hetaryloxy substituiert sind,
mit der Maßgabe, dass, wenn Y₂ für Methyl und Z für Phenyl-C₁-C₆-alkyl steht, der Phenylring in der ortho-Position nicht durch Alkoxy und nicht durch Arylalkoxy substituiert ist, mit der Maßgabe, dass Z nicht für 3,5-Dimethyl-1,2-oxazol-4-ylmethyl steht, wenn A und D jeweils für CH stehen und gleichzeitig X für ein freies Elektronenpaar steht, W für Sauerstoff steht, Q für Q-1 steht und R³, Y₁ und Y₂ für Wasserstoff stehen und mit der Maßgabe, dass Verbindungen der Formeln (G1), (G2) und (G4) ausgenommen sind.

3. Nicht-therapeutisches Verfahren zur Bekämpfung von Schädlingen, **dadurch gekennzeichnet, dass** man eine Verbindung der Formel (I) gemäß Anspruch 1 oder 2 auf die Schädlinge und/oder ihren Lebensraum einwirken lässt.

4. Nicht-therapeutische Verwendung von Verbindungen der Formel (I) gemäß Anspruch 2 zur Bekämpfung von Schädlingen.

## Claims

1. Non-therapeutic use of compounds of the formula (I) 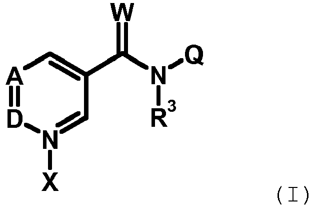 in which
A is N or CR¹,
D is N or CR², where A and D are not both N,
R¹ is a radical from the group of hydrogen, halogen, cyano, nitro, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
R² is a radical from the group of hydrogen, halogen, cyano, nitro, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
X is a free electron pair or oxygen,
W is oxygen, sulphur or NR⁴,
R³ is a radical from the group of hydrogen, cyano, hydroxyl, C₁-C₆-alkyl, C₃-C₆-cycloalkyl, C₁-C₆-haloalkyl, C₃-C₆-halocycloalkyl, C₂-C₆-alkenyl, C₂-C₆-haloalkenyl, C₃-C₆-alkynyl, C₃-C₆-haloalkynyl, C₃-C₆-cycloalkyl-C₁-C₆-alkyl, C₃-C₆-halocycloalkyl-C₁-C₆-alkyl, cyano-C₁-C₆-alkyl, C₁-C₆-alkoxy, C₁-C₆-haloalkoxy, C₁-C₆-alkoxy-C₁-C₆-alkyl, C₁-C₆-haloalkoxy-C₁-C₆-alkyl, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxy-C₁-C₆-alkylcarbonyl, C₁-C₆-alkoxycarbonyl, C₁-C₆-haloalkoxycarbonyl, C₁₋C₆-alkylsulphonyl, C₁-C₆-haloalkylsulphonyl, C₁-C₆-alkylaminocarbonyl, di(C₁-C₆)alkylaminocarbonyl, C₁-C₆-alkylcarbonyloxy, C₁-C₆-alkoxycarbonyloxy, aryl, hetaryl, arylcarbonyl, hetarylcarbonyl, aryl-C₁-C₆-alkyl, hetaryl-C₁-C₆-alkyl, aryl-C₁-C₆-alkylcarbonyl, hetaryl-C₁-C₆-alkylcarbonyl, where the aryl, hetaryl, aryl-C₁-C₆-alkyl, hetaryl-C₁-C₆-alkyl, arylcarbonyl, hetarylcarbonyl, aryl-C₁-C₆-alkylcarbonyl, hetaryl-C₁-C₆-alkylcarbonyl substituents are optionally mono- or polysubstituted, identically or differently, by halogen, cyano, nitro, hydroxyl, C₁-C₆-alkyl, C₂-C₆-alkenyl, C₃-C₆-alkynyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
R⁴ is a radical from the group of hydrogen, cyano, nitro, hydroxyl, C₁-C₆-alkyl, C₃-C₆-cycloalkyl, C₁-C₆-haloalkyl, C₃-C₆-halocycloalkyl, C₂-C₆-alkenyl, C₂-C₆-haloalkenyl, C₃-C₆-alkynyl, C₃-C₆-haloalkynyl, C₃-C₆-cycloalkyl-C₁-C₆-alkyl, C₃-C₆-halocycloalkyl-C₁-C₆-alkyl, cyano-C₁-C₆-alkyl, C₁-C₆-alkoxy, C₁-C₆-haloalkoxy, C₁-C₆-alkoxy-C₁-C₆-alkyl, C₁-C₆-haloalkoxy-C₁-C₆-alkyl, C₁-C₆-alkylcarbonyl, C₁-C₆-haloalkylcarbonyl, C₁-C₆-alkoxycarbonyl, C₁-C₆-alkylcarbonyloxy, C₁-C₆-alkylsulphonyl, C₁-C₆-haloalkylsulphonyl, aryl, hetaryl, aryl-C₁-C₆-alkyl, hetaryl-C₁-C₆-alkyl, arylcarbonyl, hetarylcarbonyl, where the aryl, hetaryl, aryl-C₁-C₆-alkyl, hetaryl-C₁-C₆-alkyl, arylcarbonyl, hetarylcarbonyl substituents are optionally mono- or polysubstituted, identically or differently, by halogen, cyano, nitro, hydroxyl, C₁-C₆-alkyl, C₂-C₆-alkenyl, C₃-C₆-alkynyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
Q is one of the Q-1 to Q-4 radicals: 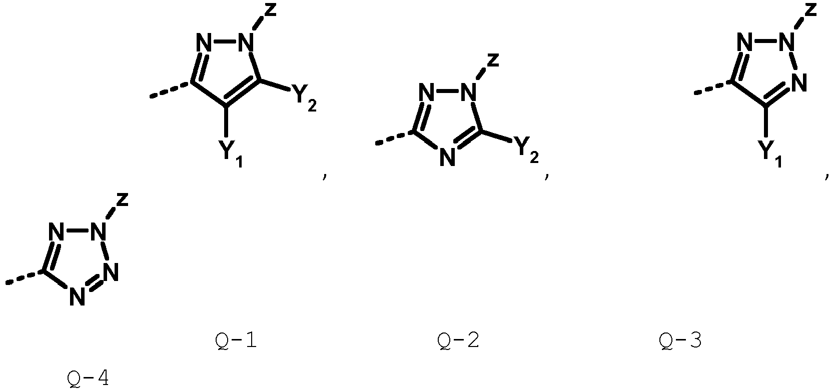 in which the dotted line is the bond to the nitrogen in the NR³ group,
Y₁ is a radical from the group of hydrogen, halogen, cyano, nitro, amino, C₁-C₆-alkylamino, di (C₁-C₆)alkylamino, hydroxyl, COOH, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₃-C₆-halocycloalkyl-C₁-C₆-alkyl, C₁-C₆-alkoxy-C₁-C₆-alkyl, C₁-C₆-haloalkoxy-C₁-C₆-alkyl, C₁-C₆-alkoxy-C₁-C₆-alkyloxy, cyano-C₁-C₆-alkyl, C₃-C₆-cycloalkyl-C₁-C₆-alkyl, C₂-C₆-alkenyl, C₃-C₆-alkynyl, SH, C₁-C₆-alkylthio, C₁-C₆-alkylcarbonyl, C₃-C₆-cycloalkylcarbonyl, C₂-C₆-alkenylcarbonyl, C₁-C₆-haloalkylcarbonyl, C₁-C₆-alkoxycarbonyl, C₁-C₆-alkylsulphonyl, C₁-C₆-haloalkylsulphonyl, C₁-C₆-alkylaminocarbonyl, di(C₁-C₆)alkylaminocarbonyl,
Y₂ is a radical from the group of hydrogen, halogen, cyano, nitro, amino, C₁-C₆-alkylamino, di (C₁-C₆)alkylamino, hydroxyl, COOH, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₃-C₆-halocycloalkyl-C₁-C₆-alkyl, C₁-C₆-alkoxy-C₁-C₆-alkyl, C₁-C₆-haloalkoxy-C₁-C₆-alkyl, C₁-C₆-alkoxy-C₁-C₆-alkyloxy, cyano-C₁-C₆-alkyl, C₃-C₆-cycloalkyl-C₁-C₆-alkyl, C₂-C₆-alkenyl, C₃-C₆-alkynyl, SH, C₁-C₆-alkylthio, C₁-C₆-alkylcarbonyl, C₃-C₆-cycloalkylcarbonyl, C₂-C₆-alkenylcarbonyl, C₁-C₆-haloalkylcarbonyl, C₁-C₆-alkoxycarbonyl, C₁-C₆-alkylsulphonyl, C₁-C₆-haloalkylsulphonyl, C₁-C₆-alkylaminocarbonyl, di(C₁-C₆)alkylaminocarbonyl, in the case that Q = Q-1, Y₁ and Y₂ together with the carbon atoms to which they are bonded form a phenyl ring or a 5- to 7-membered heteroaromatic ring which is optionally substituted by one or more identical or different substituents M¹,
M¹ is halogen, cyano, nitro, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl and C₁-C₆-alkoxy-C₁-C₆-alkyl,
Z is a radical from the group of hydrogen, cyano, nitro, L¹(=G)C*, L¹O(=G) C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, heterocyclyl, aryl, hetaryl, singly or multiply, identically or differently, M²-substituted C₁-C₈-alkyl, C₃-C₈-cycloalkyl, C₂-C₈-alkenyl, C₅-C₈-cycloalkenyl, C₃-C₈-alkynyl, singly or multiply, identically or differently, M⁶-substituted L⁵C(=E)L²N(=G)C-C₁-C₈-alkyl*, L⁵(=O)ₙSL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl*, M² is halogen, cyano, nitro, NL⁹L¹⁰, OL⁹, SL⁹, L¹(=G)C*, L³O(=G)C*, L³L⁴N(=G)C*, heterocyclyl, aryl, hetaryl, optionally singly or multiply, identically or differently, M³-substituted C₁-C₆-alkyl, C₃-C₆-cycloalkyl, C₂-C₆-alkenyl, C₅-C₆-cycloalkenyl, C₃-C₆-alkynyl, C₁-C₆-alkoxy,
M³ is halogen, cyano, nitro, heterocyclyl, aryl, hetaryl, NL⁹L¹⁰, OL⁹, SL⁹, C₁-C₆-alkyl, C₁-C₆-haloalkyl, C₃-C₆-cycloalkyl, C₃-C₆-halocycloalkyl, C₂-C₆-alkenyl, C₃-C₆-alkynyl, C₁-C₆-alkoxy, C₁-C₆-haloalkoxy, L¹(=G)C*, L³O(=G)C*, L³L⁴N(=G)C*,
G and E are each independently oxygen or sulphur,
n is 1 or 2,
L¹ and L⁵ are each independently heterocyclyl, aryl, hetaryl, optionally singly or multiply, identically or differently, M⁴-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
M⁴ is halogen, cyano, nitro, heterocyclyl, aryl, hetaryl, C₁-C₆-haloalkyl, C₁-C₆-alkoxy, C₁-C₆-haloalkoxy, NL⁹L¹⁰, OL⁹, SL⁹,
L² is hydrogen, aryl, hetaryl, optionally singly or multiply, identically or differently, M⁵-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
L³ and L⁴ are each independently hydrogen, heterocyclyl, aryl, hetaryl, optionally singly or multiply, identically or differently, M⁵-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
M⁵ is halogen, cyano, nitro, heterocyclyl, aryl, hetaryl, C₁-C₆-haloalkyl, C₁-C₆-alkoxy, C₁-C₆-haloalkoxy, C₁-C₆-alkoxycarbonyl, NL⁹L¹⁰, OL⁹, SL⁹,
M⁶ is halogen, cyano, nitro, NL⁹L¹⁰, OL⁹, SL⁹, L¹(=G)C*, L³O(=G)C*, L³L⁴N(=G)C*, heterocyclyl, aryl, hetaryl, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₃-C₆-halocycloalkyl-C₁-C₆-alkyl, C₂-C₆-alkenyl, C₅-C₆-cycloalkenyl, C₃-C₆-alkynyl, C₁-C₆-alkoxy-C₁-C₆-alkyl, C₁-C₆-haloalkoxy-C₁-C₆-alkyl,
L⁹ and L¹⁰ are each independently hydrogen, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₃-C₆-halocycloalkyl-C₁-C₆-alkyl, cyano-C₁-C₆-alkyl, C₂-C₆-alkenyl, C₂-C₆-haloalkenyl, C₃-C₆-alkynyl, C₁-C₆-alkoxy-C₁-C₆-alkyl, C₁-C₆-haloalkoxy-C₁-C₆-alkyl, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxycarbonyl, C₁-C₆-alkoxy-C₁-C₆-alkylcarbonyl, C₁-C₆-haloalkoxycarbonyl, C₃-C₆-cycloalkoxycarbonyl, aminocarbonyl, C₁-C₆-alkylaminocarbonyl, di(C₁-C₆)alkylaminocarbonyl, C₃-C₆-cycloalkylaminocarbonyl, C₃-C₆-cycloalkyl-C₁-C₆-alkylaminocarbonyl, aryl, hetaryl, heterocyclyl, aryl-C₁-C₆-alkyl, hetaryl-C₁-C₆-alkyl,
in which the heterocyclyl, aryl and hetaryl radicals (including as part of a larger unit such as aryloxy, hetarylalkyl etc.) are optionally mono- or polysubstituted, identically or differently, by halogen, cyano, nitro, SH, hydroxyl, C₁-C₆-alkyl, C₂-C₆-alkenyl, C₃-C₆-alkynyl, C₃-C₆-cycloalkyl, C₁-C₆-haloalkyl, cyano-C₁-C₆-alkyl, C₂-C₆-haloalkenyl, C₃-C₆-haloalkynyl, C₁-C₆-alkoxy, C₂-C₆-alkenyloxy, C₃-C₆-alkynyloxy, C₁-C₆-haloalkoxy, C₃-C₆-halocycloalkyl, C₁-C₆-alkoxy-C₁-C₆-alkyl, C₁-C₆-haloalkoxy-C₁-C₆-alkyl, C₃-C₆-cycloalkyl-C₁-C₆-alkyl, C₃-C₆-halocycloalkyl-C₁-C₆-alkyl, amino, C₁-C₆-alkylamino, di(C₁-C₆) alkylamino, C₃-C₆-cycloalkylamino, C₃-C₆-cycloalkyl-C₁-C₆-alkylamino, C₁-C₆-alkylcarbonylamino, C₁-C₆-haloalkylcarbonylamino, C₁-C₆-cycloalkylcarbonylamino, CO₂H, SO₃H, CONH₂, SO₂NH₂, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxycarbonyl, C₁-C₆-alkoxy-C₁-C₆-alkylcarbonyl, C₁-C₆-haloalkoxycarbonyl, C₃-C₆-cycloalkoxycarbonyl, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylaminosulphonyl, C₁-C₆-haloalkylaminosulphonyl, di(C₁-C₆)alkylaminosulphonyl, C₁-C₆-alkylaminocarbonyl, di(C₁-C₆)alkylaminocarbonyl, C₁-C₆-alkylcarbonyloxy, C₁-C₆-haloalkylcarbonyloxy, C₁-C₆-alkoxycarbonyloxy, C₁-C₆-haloalkoxycarbonyloxy, aryl, hetaryl, aryloxy, hetaryloxy,
and of the salts and N-oxides thereof for control of animal pests.

2. Compounds of the formula (I) 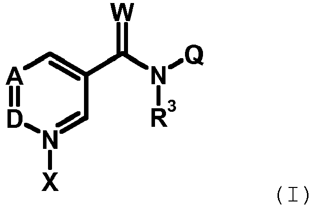 in which
A is N or CR¹,
D is N or CR², where A and D are not both N,
R¹ is a radical from the group of hydrogen, halogen, cyano, nitro, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
R² is a radical from the group of hydrogen, halogen, cyano, nitro, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
X is a free electron pair or oxygen,
W is oxygen, sulphur or NR⁴,
R³ is a radical from the group of hydrogen, cyano, hydroxyl, C₁-C₆-alkyl, C₃-C₆-cycloalkyl, C₁-C₆-haloalkyl, C₃-C₆-halocycloalkyl, C₂-C₆-alkenyl, C₂-C₆-haloalkenyl, C₃-C₆-alkynyl, C₃-C₆-haloalkynyl, C₃-C₆-_{C}y_{C}loalkyl-C₁-C₆-alkyl, C₃-C₆-halocycloalkyl-C₁-C₆-alkyl, cyano-C₁-C₆-alkyl, C₁-C₆-alkoxy, C₁-C₆-haloalkoxy, C₁-C₆-alkoxy-C₁-C₆-alkyl, C₁-C₆-haloalkoxy-C₁-C₆-alkyl, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxy-C₁-C₆-alkylcarbonyl, C₁-C₆-alkoxycarbonyl, C₁-C₆-haloalkoxycarbonyl, C₁-C₆-alkylsulphonyl, C₁-C₆-haloalkylsulphonyl, C₁-C₆-alkylaminocarbonyl, di(C₁-C₆)alkylaminocarbonyl, C₁-C₆-alkylcarbonyloxy, C₁-C₆-alkoxycarbonyloxy, aryl, hetaryl, arylcarbonyl, hetarylcarbonyl, aryl-C₁-C₆-alkyl, hetaryl-C₁-C₆-alkyl, aryl-C₁-C₆-alkylcarbonyl, hetaryl-C₁-C₆-alkylcarbonyl, where the aryl, hetaryl, aryl-C₁-C₆-alkyl, hetaryl-C₁-C₆-alkyl, arylcarbonyl, hetarylcarbonyl, aryl-C₁-C₆-alkylcarbonyl, hetaryl-C₁-C₆-alkylcarbonyl substituents are optionally mono- or polysubstituted, identically or differently, by halogen, cyano, nitro, hydroxyl, C₁-C₆-alkyl, C₂-C₆-alkenyl, C₃-C₆-alkynyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
R⁴ is a radical from the group of hydrogen, cyano, nitro, hydroxyl, C₁-C₆-alkyl, C₃-C₆-cycloalkyl, C₁-C₆-haloalkyl, C₃-C₆-halocycloalkyl, C₂-C₆-alkenyl, C₂-C₆-haloalkenyl, C₃-C₆-alkynyl, C₃-C₆-haloalkynyl, C₃-C₆-cycloalkyl-C₁-C₆-alkyl, C₃-C₆-halocycloalkyl-C₁-C₆-alkyl, cyano-C₁-C₆-alkyl, C₁-C₆-alkoxy, C₁-C₆-haloalkoxy, C₁-C₆-alkoxy-C₁-C₆-alkyl, C₁-C₆-haloalkoxy-C₁-C₆-alkyl, C₁-C₆-alkylcarbonyl, C₁-C₆-haloalkylcarbonyl, C₁-C₆-alkoxycarbonyl, C₁-C₆-alkylcarbonyloxy, C₁-C₆-alkylsulphonyl, C₁-C₆-haloalkylsulphonyl, aryl, hetaryl, aryl-C₁-C₆-alkyl, hetaryl-C₁-C₆-alkyl, arylcarbonyl, hetarylcarbonyl, where the aryl, hetaryl, aryl-C₁-C₆-alkyl, hetaryl-C₁-C₆-alkyl, arylcarbonyl, hetarylcarbonyl substituents are optionally mono- or polysubstituted, identically or differently, by halogen, cyano, nitro, hydroxyl, C₁-C₆-alkyl, C₂-C₆-alkenyl, C₃-C₆-alkynyl, C₃-C₆-cycloalkyl, C₁-C₆-haloalkyl, C₁-C₆-alkoxy, C₁-C₆-haloalkoxy, C₃-C₆-halocycloalkyl, C₁-C₆-alkoxy-C₁-C₆-alkyl,
Q is one of the Q-1 to Q-4 radicals: 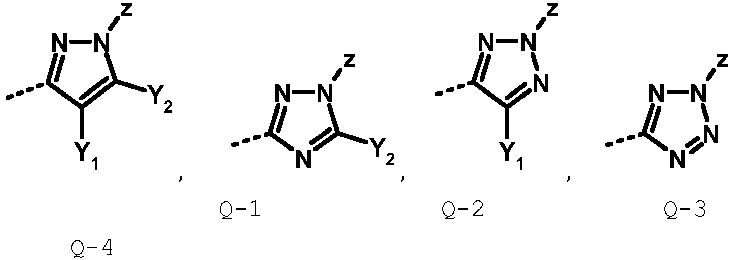 in which the dotted line is the bond to the nitrogen in the NR³ group,
Y₁ is a radical from the group of hydrogen, halogen, cyano, nitro, C₁-C₆-alkylamino, di(C₁-C₆)alkylamino, COOH, C₁-C₆-alkyl, C₃-C₆-cycloalkyl, C₁-C₆-haloalkyl, C₁-C₆-alkoxy, C₁-C₆-haloalkoxy, C₃-C₆-halocycloalkyl, C₃-C₆-halocycloalkyl-C₁-C₆-alkyl, C₁-C₆-alkoxy-C₁-C₆-alkyl, C₁-C₆-haloalkoxy-C₁-C₆-alkyl, C₁-C₆-alkoxy-C₁-C₆-alkyloxy, cyano-C₁-C₆-alkyl, C₃-C₆-cycloalkyl-C₁-C₆-alkyl, C₂-C₆-alkenyl, C₃-C₆-alkynyl, C₁-C₆-alkylthio, C₁-C₆-alkylcarbonyl, C₃-C₆-cycloalkylcarbonyl, C₂-C₆-alkenylcarbonyl, C₁-C₆-haloalkylcarbonyl, C₁-C₆-alkoxycarbonyl, C₁-C₆-alkylsulphonyl, C₁-C₆-haloalkylsulphonyl, C₁-C₆-alkylaminocarbonyl, di(C₁-C₆)alkylaminocarbonyl,
Y₂ is a radical from the group of hydrogen, halogen, cyano, nitro, C₁-C₆-alkylamino, di(C₁-C₆)alkylamino, COOH, C₁-C₆-alkyl, C₃-C₆-_{C}y_{C}loalkyl, C₁-C₆-haloalkyl, C₁-C₆-alkoxy, C₁-C₆-haloalkoxy, C₃-C₆-halocycloalkyl, C₃-C₆-halocycloalkyl-C₁-C₆-alkyl, C₁-C₆-alkoxy-C₁-C₆-alkyl, C₁-C₆-haloalkoxy-C₁-C₆-alkyl, C₁-C₆-alkoxy-C₁-C₆-alkyloxy, cyano-C₁-C₆-alkyl, C₃-C₆-_{C}y_{C}loalkyl-C₁-C₆-alkyl, C₂-C₆-alkenyl, C₃-C₆-alkynyl, SH, C₁-C₆-alkylthio, C₁-C₆-alkylcarbonyl, C₃-C₆-cycloalkylcarbonyl, C₂-C₆-alkenylcarbonyl, C₁-C₆-haloalkylcarbonyl, C₁-C₆-alkoxycarbonyl, C₁-C₆-alkylsulphonyl, C₁-C₆-haloalkylsulphonyl, C₁-C₆-alkylaminocarbonyl, di(C₁-C₆)alkylaminocarbonyl,
Z is a radical from the group of hydrogen, cyano, nitro, L¹(=G)C*, L¹O(=G)C*, L³L⁴N(=G)C*, L⁵(=E)CL²N(=G)C*, L⁵S(=O)ₙL²N(=G)C*, L⁵O(=E)CL²N(=G)C*, L¹L⁵N(=E)CL²N(=G)C*, L¹(=O)ₙS*, L³L⁴N(=O)ₙS*, heterocyclyl, aryl, hetaryl, singly or multiply, identically or differently, M²-substituted C₁-C₈-alkyl, C₃-C₈-cycloalkyl, C₂-C₈-alkenyl, C₅-C₈-cycloalkenyl, C₃-C₈-alkynyl, singly or multiply, identically or differently, M⁶-substituted L⁵C(=E)L²N(=G)C-C₁-C₈-alkyl*, L⁵S(=O)ₙL²N(=G)C-C₁-C₈-alkyl*, L⁵OC(=E)L²N(=G)C-C₁-C₈-alkyl*, L¹L⁵NC(=E)L²N(=G)C-C₁-C₈-alkyl*, L¹(=O)ₙS-C₁-C₈-alkyl*, L³O(=O)ₙS-C₁-C₈-alkyl*, L³L⁴N(=O)ₙS-C₁-C₈-alkyl*, with the proviso that, when Q is Q-4, Z is not simultaneously hydrogen or unsubstituted C₁-C₈-alkyl, and with the proviso that Z is not hydrogen when W is NOH, and with the proviso that, when Q is Q-2, Z is not simultaneously hydrogen,
M² is halogen, cyano, nitro, NL⁹L¹⁰, OL⁹, SL⁹, L¹(=G)C*, L³O(=G)C*, L³L⁴N(=G)C*, heterocyclyl, aryl, hetaryl, optionally singly or multiply, identically or differently, M³-substituted C₁-C₆-alkyl, C₃-C₆-cycloalkyl, C₂-C₆-alkenyl, C₅-C₆-cycloalkenyl, C₃-C₆-alkynyl, C₁-C₆-alkoxy,
M³ is halogen, cyano, nitro, heterocyclyl, aryl, hetaryl, NL⁹L¹⁰, OL⁹, SL⁹, C₁-C₆-alkyl, C₁-C₆-haloalkyl, C₃-C₆-cycloalkyl, C₃-C₆-halocycloalkyl, C₂-C₆-alkenyl, C₃-C₆-alkynyl, C₁-C₆-alkoxy, C₁-C₆-haloalkoxy, L¹(=G)C*, L³O(=G)C*, L³L⁴N(=G)C*,
G and E are each independently oxygen or sulphur,
n is 1 or 2,
L¹ and L⁵ are each independently heterocyclyl, aryl, hetaryl, optionally singly or multiply, identically or differently, M⁴-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
M⁴ is halogen, cyano, nitro, heterocyclyl, aryl, hetaryl, C₁-C₆-haloalkyl, C₁-C₆-alkoxy, C₁-C₆-haloalkoxy, NL⁹L¹⁰, OL⁹, SL⁹,
L² is hydrogen, aryl, hetaryl, optionally singly or multiply, identically or differently, M⁵-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
L³ and L⁴ are each independently hydrogen, heterocyclyl, aryl, hetaryl, optionally singly or multiply, identically or differently, M⁵-substituted C₁-C₆-alkyl, C₃-C₆-cycloalkyl, C₃-C₆-cycloalkyl-C₁-C₆-alkyl, C₂-C₆-alkenyl, C₃-C₆-alkynyl,
M⁵ is halogen, cyano, nitro, heterocyclyl, aryl, hetaryl, C₁-C₆-haloalkyl, C₁-C₆-alkoxy, C₁-C₆-haloalkoxy, C₁-C₆-alkoxycarbonyl, NL⁹L¹⁰, OL⁹, SL⁹,
M⁶ is halogen, cyano, nitro, NL⁹L¹⁰, OL⁹, SL⁹, L¹(=G)C*, L³O(=G)C*, L³L⁴N(=G)C*, heterocyclyl, aryl, hetaryl, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₃-C₆-halocycloalkyl-C₁-C₆-alkyl, C₂-C₆-alkenyl, C₅-C₆-cycloalkenyl, C₃-C₆-alkynyl, C₁-C₆-alkoxy-C₁-C₆-alkyl, C₁-C₆-haloalkoxy-C₁-C₆-alkyl,
L⁹ and L¹⁰ are each independently hydrogen, C₁-C₆-alkyl, C₁-C₆-haloalkyl, C₃-C₆-cycloalkyl, C₃-C₆-halocycloalkyl, C₃-C₆-cycloalkyl-C₁-C₆-alkyl, C₃-C₆-halocycloalkyl-C₁-C₆-alkyl, cyano-C₁-C₆-alkyl, C₂-C₆-alkenyl, C₂-C₆-haloalkenyl, C₃-C₆-alkynyl, C₁-C₆-alkoxy-C₁-C₆-alkyl, C₁-C₆-haloalkoxy-C₁-C₆-alkyl, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxycarbonyl, C₁-C₆-alkoxy-C₁-C₆-alkylcarbonyl, C₁-C₆-haloalkoxycarbonyl, C₃-C₆-cycloalkoxycarbonyl, aminocarbonyl, C₁-C₆-alkylaminocarbonyl, di(C₁-C₆)alkylaminocarbonyl, C₃-C₆-cycloalkylaminocarbonyl, C₃-C₆-cycloalkyl-C₁-C₆-alkylaminocarbonyl, aryl, hetaryl, heterocyclyl, aryl-C₁-C₆-alkyl, hetaryl-C₁-C₆-alkyl,
in which, unless stated otherwise, the heterocyclyl, aryl and hetaryl radicals (including as part of a larger unit such as aryloxy, hetarylalkyl etc.) are optionally mono- or polysubstituted, identically or differently, by halogen, cyano, nitro, SH, hydroxyl, C₁-C₆-alkyl, C₂-C₆-alkenyl, C₃-C₆-alkynyl, C₃-C₆-cycloalkyl, C₁-C₆-haloalkyl, cyano-C₁-C₆-alkyl, C₂-C₆-haloalkenyl, C₃-C₆-haloalkynyl, C₁-C₆-alkoxy, C₂-C₆-alkenyloxy, C₃-C₆-alkynyloxy, C₁-C₆-haloalkoxy, C₃-C₆-halocycloalkyl, C₁-C₆-alkoxy-C₁-C₆-alkyl, C₁-C₆-haloalkoxy-C₁-C₆-alkyl, C₃-C₆-cycloalkyl-C₁-C₆-alkyl, C₃-C₆-halocycloalkyl-C₁-C₆-alkyl, amino, C₁-C₆-alkylamino, di(C₁-C₆) alkylamino, C₃-C₆-cycloalkylamino, C₃-C₆-cycloalkyl-C₁-C₆-alkylamino, C₁-C₆-alkylcarbonylamino, C₁-C₆-haloalkylcarbonylamino, C₁-C₆-cycloalkylcarbonylamino, CO₂H, SO₃H, CONH₂, SO₂NH₂, C₁-C₆-alkylcarbonyl, C₂-C₆-alkenylcarbonyl, C₃-C₆-alkynylcarbonyl, C₁-C₆-haloalkylcarbonyl, C₃-C₆-cycloalkylcarbonyl, C₃-C₆-cycloalkyl-C₁-C₆-alkylcarbonyl, C₂-C₆-haloalkenylcarbonyl, C₃-C₆-halocycloalkylcarbonyl, C₁-C₆-alkoxycarbonyl, C₁-C₆-alkoxy-C₁-C₆-alkylcarbonyl, C₁-C₆-haloalkoxycarbonyl, C₃-C₆-cycloalkoxycarbonyl, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, C₁-C₆-alkylaminosulphonyl, C₁-C₆-haloalkylaminosulphonyl, di(C₁-C₆)alkylaminosulphonyl, C₁-C₆-alkylaminocarbonyl, di (C₁-C₆) alkylaminocarbonyl, C₁-C₆-alkylcarbonyloxy, C₁-C₆-haloalkylcarbonyloxy, C₁-C₆-alkoxycarbonyloxy, C₁-C₆-haloalkoxycarbonyloxy, aryl, hetaryl, aryloxy, hetaryloxy,
with the proviso that, when Y₂ is methyl and Z is phenyl-C₁-C₆-alkyl, the phenyl ring in the ortho position is not substituted by alkoxy or by arylalkoxy, with the proviso that Z is not 3,5-dimethyl-1,2-oxazol-4-ylmethyl when A and D are each CH and, at the same time, X is a free electron pair, W is oxygen, Q is Q-1 and R³, Y₁ and Y₂ are each hydrogen, and with the proviso that compounds of the formulae (G1), (G2) and (G4) are excluded.

3. Non-therapeutic method for controlling pests, **characterized in that** a compound of the formula (I) according to Claim 1 or 2 is allowed to act on the pests and/or their habitat.

4. Non-therapeutic use of compounds of the formula (I) according to Claim 2 for controlling pests.

## Revendications

1. Utilisation non thérapeutique de composés de formule (I) 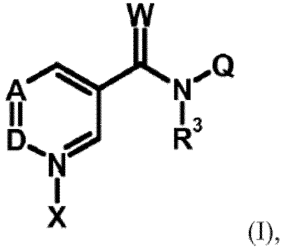 dans laquelle
A représente N ou CR¹,
D représente N ou CR², A et D ne représentant pas simultanément N,
R¹ représente un radical de la série constituée par hydrogène, halogène, cyano, nitro, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
R² représente un radical de la série constituée par hydrogène, halogène, cyano, nitro, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
X représente une paire d'électrons libres ou l'oxygène,
W représente oxygène, soufre ou NR⁴,
R³ représente un radical de la série constituée par hydrogène, cyano, hydroxy, alkyle en C₁-C₆, cycloalkyle en C₃-C₆, halogénoalkyle en C₁-C₆, halogénocycloalkyle en C₃-C₆, alcényle en C₂-C₆, halogénoalcényle en C₂-C₆, alcynyle en C₃-C₆, halogénoalcynyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcoxy en C₁-C₆, halogénoalcoxy en C₁-C₆, alcoxy en C₁-C₆-alkyle en C₁-C₆, halogénoalcoxy en C₁-C₆-alkyle en C₁-C₆, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogénocycloalkylcarbonyle en C₃-C₆, alcoxy en C₁-C₆-alkylcarbonyle en C₁-C₆, alcoxycarbonyle en C₁-C₆, halogénoalcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆, alkylcarbonyloxy en C₁-C₆, alcoxycarbonyloxy en C₁-C₆, aryle, hétaryle, arylcarbonyle, hétarylcarbonyle, aryl-alkyle en C₁-C₆, hétaryl-alkyle en C₁-C₆, aryl-alkylcarbonyle en C₁-C₆, hétaryl-alkylcarbonyle en C₁-C₆, les substituants aryle, hétaryle, aryl-alkyle en C₁-C₆, hétaryl-alkyle en C₁-C₆, arylcarbonyle, hétarylcarbonyle, aryl-alkylcarbonyle en C₁-C₆, hétaryl-alkylcarbonyle en C₁-C₆ étant éventuellement substitués une ou plusieurs fois, de manière identique ou différente, par halogène, cyano, nitro, hydroxy, alkyle en C₁-C₆, alcényle en C₂-C₆, alcynyle en C₃-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
R⁴ représente un radical de la série constituée par hydrogène, cyano, nitro, hydroxy, alkyle en C₁-C₆, cycloalkyle en C₃-C₆, halogénoalkyle en C₁-C₆, halogénocycloalkyle en C₃-C₆, alcényle en C₂-C₆, halogénoalcényle en C₂-C₆, alcynyle en C₃-C₆, halogénoalcynyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcoxy en C₁-C₆, halogénoalcoxy en C₁-C₆, alcoxy en C₁-C₆-alkyle en C₁-C₆, halogénoalcoxy en C₁-C₆-alkyle en C₁-C₆, alkylcarbonyle en C₁-C₆, halogénoalkylcarbonyle en C₁-C₆, alcoxycarbonyle en C₁-C₆, alkylcarbonyloxy en C₁-C₆, alkylsulfonyle en C₁-C₆, halogénoalkylsulfonyle en C₁-C₆, aryle, hétaryle, aryl-alkyle en C₁-C₆, hétaryl-alkyle en C₁-C₆, arylcarbonyle, hétarylcarbonyle, les substituants aryle, hétaryle, aryl-alkyle en C₁-C₆, hétaryl-alkyle en C₁-C₆, arylcarbonyle, hétarylcarbonyle étant éventuellement substitués une ou plusieurs fois, de manière identique ou différente, par halogène, cyano, nitro, hydroxy, alkyle en C₁-C₆, alcényle en C₂-C₆, alcynyle en C₃-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
Q représente un des radicaux Q-1 à Q-4 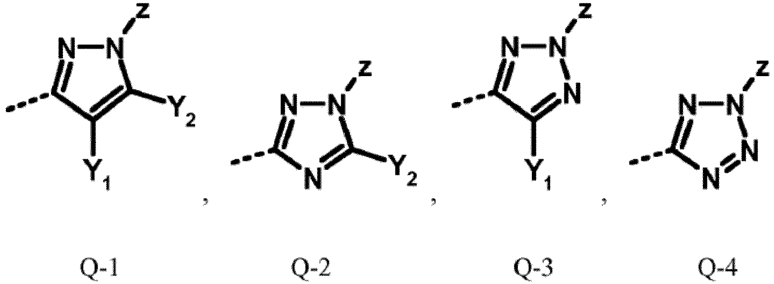 la ligne en pointillés signifiant la liaison à l'azote dans le groupe NR³,
Y₁ représente un radical de la série constituée par hydrogène, halogène, cyano, nitro, amino, alkylamino en C₁-C₆, di-alkylamino en C₁-C₆, hydroxy, COOH, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, halogénocycloalkyle en C₃-C₆-alkyle en C₁-C₆, alcoxy en C₁-C₆-alkyle en C₁-C₆, halogénoalcoxy en C₁-C₆-alkyle en C₁-C₆, alcoxy en C₁-C₆-alkyloxy en C₁-C₆, cyano-alkyle en C₁-C₆, cycloalkyle en C₃-C₆-alkyle en C₁-C₆, alcényle en C₂-C₆, alcynyle en C₃-C₆, SH, alkylthio en C₁-C₆, alkylcarbonyle en C₁-C₆, cycloalkylcarbonyle en C₃-C₆, alcénylcarbonyle en C₂-C₆, halogénoalkylcarbonyle en C₁-C₆, alcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆,
Y₂ représente un radical de la série constituée par hydrogène, halogène, cyano, nitro, amino, alkylamino en C₁-C₆, di-alkylamino en C₁-C₆, hydroxy, COOH, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, halogénocycloalkyle en C₃-C₆-alkyle en C₁-C₆, alcoxy en C₁-C₆-alkyle en C₁-C₆, halogénoalcoxy en C₁-C₆-alkyle en C₁-C₆, alcoxy en C₁-C₆-alkyloxy en C₁-C₆, cyano-alkyle en C₁-C₆, cycloalkyle en C₃-C₆-alkyle en C₁-C₆, alcényle en C₂-C₆, alcynyle en C₃-C₆, SH, alkylthio en C₁-C₆, alkylcarbonyle en C₁-C₆, cycloalkylcarbonyle en C₃-C₆, alcénylcarbonyle en C₂-C₆, halogénoalkylcarbonyle en C₁-C₆, alcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆, ou
lorsque Q = Q-1, Y₁ et Y₂ forment ensemble avec les atomes de carbone auxquels ils sont reliés un cycle phényle ou un cycle hétéroaromatique de 5 à 7 éléments, qui est éventuellement substitué par un ou plusieurs substituants M¹ identiques ou différents,
M¹ représente halogène, cyano, nitro, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆ et alcoxy en C₁-C₆-alkyle en C₁-C₆,
Z représente un radical de la série constituée par hydrogène, cyano, nitro, L¹(=G)C*, L¹O(=G)C*, L³L⁴N(=G)C*, L⁵(=E)CL²N(=G)C*, L⁵(=O)ₙSL²N(=G)C*, L⁵O(=E)CL²N(=G)C*, L¹L⁵N(=E)CL²N(=G)C*, L¹(=O)ₙS*, L³L⁴N(=O)ₙS*, hétérocyclyle, aryle, hétaryle, alkyle en C₁-C₈ éventuellement substitué une ou plusieurs fois, de manière identique ou différente, par M², cycloalkyle en C₃-C₈, alcényle en C₂-C₈, cycloalcényle en C₅-C₈, alcynyle en C₃-C₈, L⁵C(=E)L²N(=G)C-alkyle en C₁-C₈* éventuellement substitué une ou plusieurs fois, de manière identique ou différente, par M⁶, L⁵(=O)SL²N(=G)C-alkyle en C₁-C₈*, L⁵OC(=E)L²N(=G)C-alkyle en C₁-C₈*, L¹L⁵NC(=E)L²N(=G)C-alkyle en C₁-C₈*, L¹(=O)ₙS-alkyle en C₁-C₈*, L³O(=O)ₙS-alkyle en C₁-C₈*, L³L⁴N(=O)ₙS-alkyle en C₁-C₈*,
M² représente halogène, cyano, nitro, NL⁹L¹⁰, OL⁹, SL⁹, L¹(=G)C*, L³O(=G)C*, L³L⁴N(=G)C*, hétérocyclyle, aryle, hétaryle, alkyle en C₁-C₆ éventuellement substitué une ou plusieurs fois, de manière identique ou différente, par M³, cycloalkyle en C₃-C₆, alcényle en C₂-C₆, cycloalcényle en C₅-C₆, alcynyle en C₃-C₆, alcoxy en C₁-C₆,
M³ représente halogène, cyano, nitro, hétérocyclyle, aryle, hétaryle, NL⁹L¹⁰, OL⁹, SL⁹, alkyle en C₁-C₆, halogénoalkyle en C₁-C₆, cycloalkyle en C₃-C₆, halogénocycloalkyle en C₃-C₆, alcényle en C₂-C₆, alcynyle en C₃-C₆, alcoxy en C₁-C₆, halogénoalcoxy en C₁-C₆, L¹(=G)C*, L³O(=G)C*, L³L⁴N(=G)C*,
G et E représentent indépendamment l'un de l'autre oxygène ou soufre,
n représente 1 ou 2,
L¹ et L⁵ représentent indépendamment l'un de l'autre hétérocyclyle, aryle, hétaryle, alkyle en C₁-C₆ éventuellement substitué une ou plusieurs fois, de manière identique ou différente, par M⁴, cycloalkyle en C₃-C₆, cycloalkyle en C₃-C₆-alkyle en C₁-C₆, alcényle en C₂-C₆, alcynyle en C₃-C₆,
M⁴ représente halogène, cyano, nitro, hétérocyclyle, aryle, hétaryle, halogénoalkyle en C₁-C₆, alcoxy en C₁-C₆, halogénoalcoxy en C₁-C₆, NL⁹L¹⁰, OL⁹, SL⁹,
L² représente hydrogène, aryle, hétaryle, alkyle en C₁-C₆ éventuellement substitué une ou plusieurs fois, de manière identique ou différente, par M⁵, cycloalkyle en C₃-C₆, cycloalkyle en C₃-C₆-alkyle en C₁-C₆, alcényle en C₂-C₆, alcynyle en C₃-C₆,
L³ et L⁴ représentent indépendamment l'un de l'autre hydrogène, hétérocyclyle, aryle, hétaryle, alkyle en C₁-C₆ éventuellement substitué une ou plusieurs fois, de manière identique ou différente, par M⁵, cycloalkyle en C₃-C₆, cycloalkyle en C₃-C₆-alkyle en C₁-C₆, alcényle en C₂-C₆, alcynyle en C₃-C₆,
M⁵ représente halogène, cyano, nitro, hétérocyclyle, aryle, hétaryle, halogénoalkyle en C₁-C₆, alcoxy en C₁-C₆, halogénoalcoxy en C₁-C₆, alcoxycarbonyle en C₁-C₆, NL⁹L¹⁰, OL⁹, SL⁹,
M⁶ représente halogène, cyano, nitro, NL⁹L¹⁰, OL⁹, SL⁹, L¹(=G)C*, L³O(=G)C*, L³L⁴N(=G)C*, hétérocyclyle, aryle, hétaryle, alkyle en C₁-C₆, halogénoalkyle en C₁-C₆, cycloalkyle en C₃-C₆, halogénocycloalkyle en C₃-C₆, cycloalkyle en C₃-C₆-alkyle en C₁-C₆, halogénocycloalkyle en C₃-C₆-alkyle en C₁-C₆, alcényle en C₂-C₆, cycloalcényle en C₅-C₆, alcynyle en C₃-C₆, alcoxy en C₁-C₆-alkyle en C₁-C₆, halogénalcoxy en C₁-C₆-alkyle en C₁-C₆,
L⁹ et L¹⁰ représentent indépendamment l'un de l'autre hydrogène, alkyle en C₁-C₆, halogénoalkyle en C₁-C₆, cycloalkyle en C₃-C₆, halogénocycloalkyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcényle en C₂-C₆, halogénoalcényle en C₂-C₆, alcynyle en C₃-C₆, alcoxy en C₁-C₆-alkyle en C₁-C₆, halogénoalcoxy en C₁-C₆-alkyle en C₁-C₆, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogénocycloalkylcarbonyle en C₃-C₆, alcoxycarbonyle en C₁-C₆, alcoxy en C₁-C₆-alkylcarbonyle en C₁-C₆, halogénoalcoxycarbonyle en C₁-C₆, cycloalcoxycarbonyle en C₃-C₆, aminocarbonyle, alkylaminocarbonyle en C₁-C₆, di-alkylaminocarbonyle en C₁-C₆, cycloalkylaminocarbonyle en C₃-C₆, cycloalkyle en C₃-C₆-alkylaminocarbonyle en C₁-C₆, aryle, hétaryle, hétérocyclyle, aryl-alkyle en C₁-C₆, hétaryl-alkyle en C₁-C₆,
les radicaux hétérocyclyle, aryle et hétaryle (également en tant que partie d'une unité plus grande telle qu'aryloxy, hétarylalkyle, etc.) étant éventuellement substitués une ou plusieurs fois, de manière identique ou différente, par halogène, cyano, nitro, SH, hydroxy, alkyle en C₁-C₆, alcényle en C₂-C₆, alcynyle en C₃-C₆, cycloalkyle en C₃-C₆, halogénoalkyle en C₁-C₆, cyano-alkyle en C₁-C₆, halogénoalcényle en C₂-C₆, halogénoalcynyle en C₃-C₆, alcoxy en C₁-C₆, alcényloxy en C₂-C₆, alcynyloxy en C₃-C₆, halogénoalcoxy en C₁-C₆, halogénocycloalkyle en C₃-C₆, alcoxy en C₁-C₆-alkyle en C₁-C₆, halogénoalcoxy en C₁-C₆-alkyle en C₁-C₆, cycloalkyle en C₃-C₆-alkyle en C₁-C₆, halogénocycloalkyle en C₃-C₆-alkyle en C₁-C₆, amino, alkylamino en C₁-C₆, di-alkylamino en C₁-C₆, cycloalkylamino en C₃-C₆, cycloalkyle en C₃-C₆-alkylamino en C₁-C₆, alkylcarbonylamino en C₁-C₆, halogénoalkylcarbonylamino en C₁-C₆, cycloalkylcarbonylamino en C₁-C₆, CO₂H, SO₃H, CONH₂, SO₂NH₂, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogénocycloalkylcarbonyle en C₃-C₆, alcoxycarbonyle en C₁-C₆, alkoxy en C₁-C₆-alkylcarbonyle en C₁-C₆, halogénoalcoxycarbonyle en C₁-C₆, cycloalcoxycarbonyle en C₃-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylaminosulfonyle en C₁-C₆, halogénoalkylaminosulfonyle en C₁-C₆, di-alkylaminosulfonyle en C₁-C₆, alkylaminocarbonyle en C₁-C₆, di-alkylaminocarbonyle en C₁-C₆, alkylcarbonyloxy en C₁-C₆, halogénoalkylcarbonyloxy en C₁-C₆, alcoxycarbonyloxy en C₁-C₆, halogénoalcoxycarbonyloxy en C₁-C₆, aryle, hétaryle, aryloxy, hétaryloxy,
et leurs sels et N-oxydes pour lutter contre des animaux nuisibles.

2. Composés de formule (I) 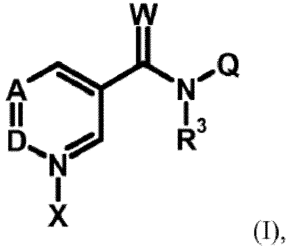 dans laquelle
A représente N ou CR¹,
D représente N ou CR², A et D ne représentant pas simultanément N,
R¹ représente un radical de la série constituée par hydrogène, halogène, cyano, nitro, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
R² représente un radical de la série constituée par hydrogène, halogène, cyano, nitro, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
X représente une paire d'électrons libres ou l'oxygène,
W représente oxygène, soufre ou NR⁴,
R³ représente un radical de la série constituée par hydrogène, cyano, hydroxy, alkyle en C₁-C₆, cycloalkyle en C₃-C₆, halogénoalkyle en C₁-C₆, halogénocycloalkyle en C₃-C₆, alcényle en C₂-C₆, halogénoalcényle en C₂-C₆, alcynyle en C₃-C₆, halogénoalcynyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcoxy en C₁-C₆, halogénoalcoxy en C₁-C₆, alcoxy en C₁-C₆-alkyle en C₁-C₆, halogénoalcoxy en C₁-C₆-alkyle en C₁-C₆, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogénocycloalkylcarbonyle en C₃-C₆, alcoxy en C₁-C₆-alkylcarbonyle en C₁-C₆, alcoxycarbonyle en C₁-C₆, halogénoalcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆, alkylcarbonyloxy en C₁-C₆, alcoxycarbonyloxy en C₁-C₆, aryle, hétaryle, arylcarbonyle, hétarylcarbonyle, aryl-alkyle en C₁-C₆, hétaryl-alkyle en C₁-C₆, aryl-alkylcarbonyle en C₁-C₆, hétaryl-alkylcarbonyle en C₁-C₆, les substituants aryle, hétaryle, aryl-alkyle en C₁-C₆, hétaryl-alkyle en C₁-C₆, arylcarbonyle, hétarylcarbonyle, aryl-alkylcarbonyle en C₁-C₆, hétaryl-alkylcarbonyle en C₁-C₆ étant éventuellement substitués une ou plusieurs fois, de manière identique ou différente, par halogène, cyano, nitro, hydroxy, alkyle en C₁-C₆, alcényle en C₂-C₆, alcynyle en C₃-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
R⁴ représente un radical de la série constituée par hydrogène, cyano, nitro, hydroxy, alkyle en C₁-C₆, cycloalkyle en C₃-C₆, halogénoalkyle en C₁-C₆, halogénocycloalkyle en C₃-C₆, alcényle en C₂-C₆, halogénoalcényle en C₂-C₆, alcynyle en C₃-C₆, halogénoalcynyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcoxy en C₁-C₆, halogénoalcoxy en C₁-C₆, alcoxy en C₁-C₆-alkyle en C₁-C₆, halogénoalcoxy en C₁-C₆-alkyle en C₁-C₆, alkylcarbonyle en C₁-C₆, halogénoalkylcarbonyle en C₁-C₆, alcoxycarbonyle en C₁-C₆, alkylcarbonyloxy en C₁-C₆, alkylsulfonyle en C₁-C₆, halogénoalkylsulfonyle en C₁-C₆, aryle, hétaryle, aryl-alkyle en C₁-C₆, hétaryl-alkyle en C₁-C₆, arylcarbonyle, hétarylcarbonyle, les substituants aryle, hétaryle, aryl-alkyle en C₁-C₆, hétaryl-alkyle en C₁-C₆, arylcarbonyle, hétarylcarbonyle étant éventuellement substitués une ou plusieurs fois, de manière identique ou différente, par halogène, cyano, nitro, hydroxy, alkyle en C₁-C₆, alcényle en C₂-C₆, alcynyle en C₃-C₆, cycloalkyle en C₃-C₆, halogénoalkyle en C₁-C₆, alcoxy en C₁-C₆, halogénoalcoxy en C₁-C₆, halogénocycloalkyle en C₃-C₆, alcoxy en C₁-C₆-alkyle en C₁-C₆,
Q représente un des radicaux Q-1 à Q-4 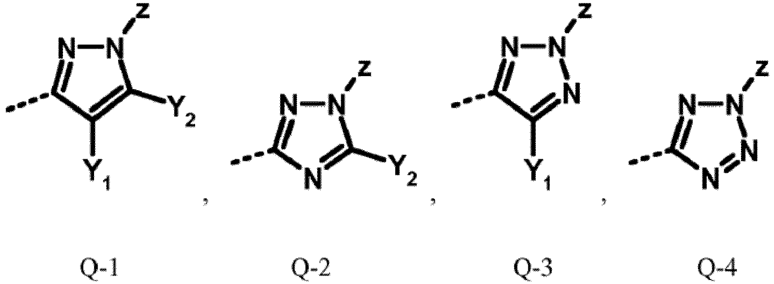 la ligne en pointillés signifiant la liaison à l'azote dans le groupe NR³,
Y₁ représente un radical de la série constituée par hydrogène, halogène, cyano, nitro, alkylamino en C₁-C₆, di-alkylamino en C₁-C₆, COOH, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, halogénocycloalkyle en C₃-C₆-alkyle en C₁-C₆, alcoxy en C₁-C₆-alkyle en C₁-C₆, halogénoalcoxy en C₁-C₆-alkyle en C₁-C₆, alcoxy en C₁-C₆-alkyloxy en C₁-C₆, cyano-alkyle en C₁-C₆, cycloalkyle en C₃-C₆-alkyle en C₁-C₆, alcényle en C₂-C₆, alcynyle en C₃-C₆, alkylthio en C₁-C₆, alkylcarbonyle en C₁-C₆, cycloalkylcarbonyle en C₃-C₆, alcénylcarbonyle en C₂-C₆, halogénoalkylcarbonyle en C₁-C₆, alcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆,
Y₂ représente un radical de la série constituée par hydrogène, halogène, cyano, nitro, alkylamino en C₁-C₆, di-alkylamino en C₁-C₆, COOH, alkyle en C₁-C₆, cycloalkyle en C₃-C₆, halogénoalkyle en C₁-C₆, alcoxy en C₁-C₆, halogénoalcoxy en C₁-C₆, halogénocycloalkyle en C₃-C₆, halogénocycloalkyle en C₃-C₆-alkyle en C₁-C₆, alcoxy en C₁-C₆-alkyle en C₁-C₆, halogénoalcoxy en C₁-C₆-alkyle en C₁-C₆, alcoxy en C₁-C₆-alkyloxy en C₁-C₆, cyano-alkyle en C₁-C₆, cycloalkyle en C₃-C₆-alkyle en C₁-C₆, alcényle en C₂-C₆, alcynyle en C₃-C₆, SH, alkylthio en C₁-C₆, alkylcarbonyle en C₁-C₆, cycloalkylcarbonyle en C₃-C₆, alcénylcarbonyle en C₂-C₆, halogénoalkylcarbonyle en C₁-C₆, alcoxycarbonyle en C₁-C₆, alkylsulfonyle en C₁-C₆, halogénoalkylsulfonyle en C₁-C₆, alkylaminocarbonyle en C₁-C₆, di-alkylaminocarbonyle en C₁-C₆,
Z représente un radical de la série constituée par hydrogène, cyano, nitro, L¹(=G)C*, L¹O(=G)C*, L³L⁴N(=G)C*, L⁵(=E)CL²N(=G)C*, L⁵S(=O)ₙL²N(=G)C*, L⁵O(=E)CL²N(=G)C*, L¹L⁵N(=E)CL²N(=G)C*, L¹(=O)ₙS*, L³L⁴N(=O)ₙS*, hétérocyclyle, aryle, hétaryle, alkyle en C₁-C₈ éventuellement substitué une ou plusieurs fois, de manière identique ou différente, par M², cycloalkyle en C₃-C₈, alcényle en C₂-C₈, cycloalcényle en C₅-C₈, alcynyle en C₃-C₈, L⁵C(=E)L²N (=G) C-alkyle en C₁-C₈* éventuellement substitué une ou plusieurs fois, de manière identique ou différente, par M⁶, L⁵S(=O)ₙL²N(=G)C-alkyle en C₁-C₈*, L⁵OC(=E)L²N(=G)C-alkyle en C₁-C₈*, L¹L⁵NC(=E)L²N(=G)C-alkyle en C₁-C₈*, L¹(=O)ₙS-alkyle en C₁-C₈*, L³O(=O)ₙS-alkyle en C₁-C₈*, L³L⁴N(=O)ₙS-alkyle en C₁-C₈*,
à condition que lorsque Q représente Q-4, Z ne représente pas simultanément hydrogène ou alkyle en C₁-C₈ non substitué, et à condition que Z ne représente pas hydrogène lorsque W représente NOH, et à condition que lorsque Q représente Q-2, Z ne représente pas simultanément hydrogène,
M² représente halogène, cyano, nitro, NL⁹L¹⁰, OL⁹, SL⁹, L¹(=G)C*, L³O(=G)C*, L³L⁴N(=G)C*, hétérocyclyle, aryle, hétaryle, alkyle en C₁-C₆ éventuellement substitué une ou plusieurs fois, de manière identique ou différente, par M³, cycloalkyle en C₃-C₆, alcényle en C₂-C₆, cycloalcényle en C₅-C₆, alcynyle en C₃-C₆, alcoxy en C₁-C₆,
M³ représente halogène, cyano, nitro, hétérocyclyle, aryle, hétaryle, NL⁹L¹⁰, OL⁹, SL⁹, alkyle en C₁-C₆, halogénoalkyle en C₁-C₆, cycloalkyle en C₃-C₆, halogénocycloalkyle en C₃-C₆, alcényle en C₂-C₆, alcynyle en C₃-C₆, alcoxy en C₁-C₆, halogénoalcoxy en C₁-C₆, L¹(=G)C*, L³O(=G)C*, L³L⁴N(=G)C*,
G et E représentent indépendamment l'un de l'autre oxygène ou soufre,
n représente 1 ou 2,
L¹ et L⁵ représentent indépendamment l'un de l'autre hétérocyclyle, aryle, hétaryle, alkyle en C₁-C₆ éventuellement substitué une ou plusieurs fois, de manière identique ou différente, par M⁴, cycloalkyle en C₃-C₆, cycloalkyle en C₃-C₆-alkyle en C₁-C₆, alcényle en C₂-C₆, alcynyle en C₃-C₆,
M⁴ représente halogène, cyano, nitro, hétérocyclyle, aryle, hétaryle, halogénoalkyle en C₁-C₆, alcoxy en C₁-C₆, halogénoalcoxy en C₁-C₆, NL⁹L¹⁰, OL⁹, SL⁹,
L² représente hydrogène, aryle, hétaryle, alkyle en C₁-C₆ éventuellement substitué une ou plusieurs fois, de manière identique ou différente, par M⁵, cycloalkyle en C₃-C₆, cycloalkyle en C₃-C₆-alkyle en C₁-C₆, alcényle en C₂-C₆, alcynyle en C₃-C₆,
L³ et L⁴ représentent indépendamment l'un de l'autre hydrogène, hétérocyclyle, aryle, hétaryle, alkyle en C₁-C₆ éventuellement substitué une ou plusieurs fois, de manière identique ou différente, par M⁵, cycloalkyle en C₃-C₆, cycloalkyle en C₃-C₆-alkyle en C₁-C₆, alcényle en C₂-C₆, alcynyle en C₃-C₆,
M⁵ représente halogène, cyano, nitro, hétérocyclyle, aryle, hétaryle, halogénoalkyle en C₁-C₆, alcoxy en C₁-C₆, halogénoalcoxy en C₁-C₆, alcoxycarbonyle en C₁-C₆, NL⁹L¹⁰, OL⁹, SL⁹,
M⁶ représente halogène, cyano, nitro, NL⁹L¹⁰, OL⁹, SL⁹, L¹(=G)C*, L³O(=G)C*, L³L⁴N(=G)C*, hétérocyclyle, aryle, hétaryle, alkyle en C₁-C₆, halogénoalkyle en C₁-C₆, cycloalkyle en C₃-C₆, halogénocycloalkyle en C₃-C₆, cycloalkyle en C₃-C₆-alkyle en C₁-C₆, halogénocycloalkyle en C₃-C₆-alkyle en C₁-C₆, alcényle en C₂-C₆, cycloalcényle en C₅-C₆, alcynyle en C₃-C₆, alcoxy en C₁-C₆-alkyle en C₁-C₆, halogénalcoxy en C₁-C₆-alkyle en C₁-C₆,
L⁹ et L¹⁰ représentent indépendamment l'un de l'autre hydrogène, alkyle en C₁-C₆, halogénoalkyle en C₁-C₆, cycloalkyle en C₃-C₆, halogénocycloalkyle en C₃-C₆, cycloalkyle en C₃-C₆-alkyle en C₁-C₆, halogénocycloalkyle en C₃-C₆-alkyle en C₁-C₆, cyano-alkyle en C₁-C₆, alcényle en C₂-C₆, halogénoalcényle en C₂-C₆, alcynyle en C₃-C₆, alcoxy en C₁-C₆-alkyle en C₁-C₆, halogénoalcoxy en C₁-C₆-alkyle en C₁-C₆, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogéncycloalkylcarbonyle en C₃-C₆, alcoxycarbonyle en C₁-C₆, alcoxy en C₁-C₆-alkylcarbonyle en C₁-C₆, halogénoalcoxycarbonyle en C₁-C₆, cycloalcoxycarbonyle en C₃-C₆, aminocarbonyle, alkylaminocarbonyle en C₁-C₆, di-alkylaminocarbonyle en C₁-C₆, cycloalkylaminocarbonyle en C₃-C₆, cycloalkyle en C₃-C₆-alkylaminocarbonyle en C₁-C₆, aryle, hétaryle, hétérocyclyle, aryl-alkyle en C₁-C₆, hétaryl-alkyle en C₁-C₆,
sauf indication contraire, les radicaux hétérocyclyle, aryle et hétaryle (également en tant que partie d'une unité plus grande telle qu'aryloxy, hétarylalkyle, etc.) étant éventuellement substitués une ou plusieurs fois, de manière identique ou différente, par halogène, cyano, nitro, SH, hydroxy, alkyle en C₁-C₆, alcényle en C₂-C₆, alcynyle en C₃-C₆, cycloalkyle en C₃-C₆, halogénoalkyle en C₁-C₆, cyano-alkyle en C₁-C₆, halogénoalcényle en C₂-C₆, halogénoalcynyle en C₃-C₆, alcoxy en C₁-C₆, alcényloxy en C₂-C₆, alcynyloxy en C₃-C₆, halogénoalcoxy en C₁-C₆, halogénocycloalkyle en C₃-C₆, alcoxy en C₁-C₆-alkyle en C₁-C₆, halogénoalcoxy en C₁-C₆-alkyle en C₁-C₆, cycloalkyle en C₃-C₆-alkyle en C₁-C₆, halogénocycloalkyle en C₃-C₆-alkyle en C₁-C₆, amino, alkylamino en C₁-C₆, di-alkylamino en C₁-C₆, cycloalkylamino en C₃-C₆, cycloalkyle en C₃-C₆-alkylamino en C₁-C₆, alkylcarbonylamino en C₁-C₆, halogénoalkylcarbonylamino en C₁-C₆, cycloalkylcarbonylamino en C₁-C₆, CO₂H, SO₃H, CONH₂, SO₂NH₂, alkylcarbonyle en C₁-C₆, alcénylcarbonyle en C₂-C₆, alcynylcarbonyle en C₃-C₆, halogénoalkylcarbonyle en C₁-C₆, cycloalkylcarbonyle en C₃-C₆, cycloalkyle en C₃-C₆-alkylcarbonyle en C₁-C₆, halogénoalcénylcarbonyle en C₂-C₆, halogénocycloalkylcarbonyle en C₃-C₆, alcoxycarbonyle en C₁-C₆, alkoxy en C₁-C₆-alkylcarbonyle en C₁-C₆, halogénoalcoxycarbonyle en C₁-C₆, cycloalcoxycarbonyle en C₃-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, alkylaminosulfonyle en C₁-C₆, halogénoalkylaminosulfonyle en C₁-C₆, di-alkylaminosulfonyle en C₁-C₆, alkylaminocarbonyle en C₁-C₆, di-alkylaminocarbonyle en C₁-C₆, alkylcarbonyloxy en C₁-C₆, halogénoalkylcarbonyloxy en C₁-C₆, alcoxycarbonyloxy en C₁-C₆, halogénoalcoxycarbonyloxy en C₁-C₆, aryle, hétaryle, aryloxy, hétaryloxy,
à condition que lorsqu'Y₂ représente méthyle et Z représente phényl-alkyle en C₁-C₆, le cycle phényle ne soit pas substitué en position ortho par alcoxy et par arylalcoxy, à condition que Z ne représente pas 3,5-diméthyl-1,2-oxazol-4-ylméthyle lorsqu'A et D représentent chacun CH et simultanément X représente une paire d'électrons libres, W représente oxygène, Q représente Q-1 et R³, Y₁ et Y₂ représentent hydrogène, et à condition que les composés de formule (G1), (G2) et (G4) soient exclus.

3. Procédé non thérapeutique de lutte contre des nuisibles, **caractérisé en ce qu'**un composé de formule (I) selon la revendication 1 ou 2 est laissé agir sur des nuisibles et/ou leur habitat.

4. Utilisation non thérapeutique de composés de formule (I) selon la revendication 2 pour lutter contre des nuisibles.
